# EUROPEAN PATENT APPLICATION

(11) **EP 3 851 453 A1**
(43) Date of publication of application: **21.07.2021**
(21) Application number: 21151830.3
(22) Date of filing: 01.05.2015
(51) Int. Cl.: C07K 16/18, C12N 5/00, C07K 16/22, C12N 5/10, C12N 15/00, C12N 15/09, C40B 40/02, C07K 16/00

(54) **CELLS FOR PRODUCING HUMAN ANTIBODY**

(30) Priority: 02.05.2014 JP 2014095236
(62) Divisional of application: 15786546.0
(71) Applicant: Chiome Bioscience Inc., Tokyo 151-0071 (JP)
(72) Inventor: HASHIMOTO, Shuichi, Tokyo, 151-0071 (JP); UCHIKI, Tomoaki, Tokyo, 151-0071 (JP); KAWATA, Shigehisa, Tokyo, 151-0071 (JP); ASAGOSHI, Kenjiro, Tokyo, 151-0071 (JP); YABUKI, Takashi, Tokyo, 151-0071 (JP); SANO, Hitomi, Tokyo, 151-0071 (JP); MIYAI, Shunsuke, Tokyo, 151-0071 (JP); TAKAHASHI, Naoki, Tokyo, 151-0071 (JP); TAKESUE, Aki, Tokyo, 151-0071 (JP); SAWADA, Atsushi, Tokyo, 151-0071 (JP)
(74) Representative: Vossius & Partner Patentanwälte Rechtsanwälte mbB

(57) **Abstract**

It is an object of the present invention to provide a chicken B cell that expresses a variety of human antibodies. The solution to the problem in the present invention is a chicken B cell, in which, in an antibody light chain gene locus thereof, all or a part of a DNA sequence derived from a human antibody light chain variable region and a human antibody light chain constant region are inserted, or the antibody light chain gene locus is replaced with all or a part of a DNA sequence derived from a human antibody light chain variable region and a human antibody light chain constant region, and in an antibody heavy chain gene locus thereof, all or a part of a DNA sequence derived from a human antibody heavy chain variable region and a human antibody heavy chain constant region are inserted, or the antibody heavy chain gene locus is replaced with all or a part of a DNA sequence derived from a human antibody heavy chain variable region and a human antibody heavy chain constant region, and in an antibody light chain pseudogene locus thereof, two or more DNA sequences derived from human antibody light chain variable regions are inserted, or the antibody light chain pseudogene locus is replaced with two or more DNA sequences derived from human antibody light chain variable regions, and/or in an antibody heavy chain pseudogene locus thereof, two or more DNA sequences derived from human antibody heavy chain variable regions are inserted, or the antibody heavy chain pseudogene locus is replaced with two or more DNA sequences derived from human antibody heavy chain variable regions.

SEQUENCE LISTING

<110>  Chiome Bioscience Inc.

<120>  Cells producing human antibodies

<130>  Y2872 EP/1 S3

<140>  EP 15 78 6546.0
<141>  2015-05-01

<150>  JP2014-095236
<151>  2014-05-02

<160>  183

<170>  PatentIn version 3.5

<210>  1
<211>  21
<212>  DNA
<213>  Artificial Sequence

<220>
<223>  Synthetic Primer

<400>  1
tccaccatgg cctgggctct g                                                21


<210>  2
<211>  33
<212>  DNA
<213>  Artificial Sequence

<220>
<223>  Synthetic Primer

<400>  2
gttgagaacc tatgaacatt ctgtaggggc cac                                   33


<210>  3
<211>  54
<212>  DNA
<213>  Artificial Sequence

<220>
<223>  Synthetic Primer

<400>  3
attggcgcgc ctctccaggt tccctggtgc aggcacagtc tgccctgact cagc             54


<210>  4
<211>  40
<212>  DNA
<213>  Artificial Sequence

<220>
<223>  Synthetic Primer

<400>  4

ttccatatga gcgactcacc taggacggtc agcttggtcc                    40


<210>  5
<211>  334
<212>  DNA
<213>  Homo sapiens

<400>  5
cagtctgccc tgactcagcc tgcctccgtg tctgggtctc ctggacagtc gatcaccatc      60

tcctgcactg gaaccagcag tgacgttggt ggttataact atgtctcctg gtaccaacaa     120

aacccaggca aagcccccaa actcatgatt tatgatgtca gtaatcggcc ctcagggatt     180

tctaatcgct tctctggctc caagtctggc aacacggcct ccctgaccat ctctgggctc     240

caggctgacg acgaggctga ttattactgc acctcatata caaacgacag caattctcag     300

gtattcggcg gagggaccaa gctgaccgtc ctag                                 334


<210>  6
<211>  51
<212>  DNA
<213>  Artificial Sequence

<220>
<223>  Synthetic Primer

<400>  6
attggcgcgc ctctgcctct ctcttgcagg tcagcccaag gctgccccct c              51


<210>  7
<211>  44
<212>  DNA
<213>  Artificial Sequence

<220>
<223>  Synthetic Primer

<400>  7
ggaattccat atggagtggg actactatga acattctgta gggg                      44


<210>  8
<211>  320
<212>  DNA
<213>  Homo sapiens

<400>  8
gtcagcccaa ggctgccccc tcggtcactc tgttcccgcc ctcctctgag gagcttcaag      60

ccaacaaggc cacactggtg tgtctcataa gtgacttcta cccgggagcc gtgacagtgg     120

cctggaaggc agatagcagc cccgtcaagg cgggagtgga gaccaccaca ccctccaaac     180

aaagcaacaa caagtacgcg gccagcagct acctgagcct gacgcctgag cagtggaagt     240

cccacagaag ctacagctgc caggtcacgc atgaagggag caccgtggag aagacagtgg     300

cccctacaga atgttcatag                                                 320

<210>  9
<211>  20
<212>  DNA
<213>  Artificial Sequence

<220>
<223>  Synthetic Primer

<400>  9
gagatctcct cctcccatcc                                                    20


<210>  10
<211>  20
<212>  DNA
<213>  Artificial Sequence

<220>
<223>  Synthetic Primer

<400>  10
caaaggacac gacagagcaa                                                    20


<210>  11
<211>  28
<212>  DNA
<213>  Artificial Sequence

<220>
<223>  Synthetic Primer

<400>  11
tgtctcgagt gaaggtcacc aaggatgg                                           28


<210>  12
<211>  28
<212>  DNA
<213>  Artificial Sequence

<220>
<223>  Synthetic Primer

<400>  12
ttaagcttgg agaggagaga ggggagaa                                           28


<210>  13
<211>  1491
<212>  DNA
<213>  Gallus gallus

<400>  13
   tcgagtgaag gtcaccaagg atggagactt cagagctatc tgggacacgt gttttaacgt      60
   ttgatcagcc ttgcagcaaa acagtgtttt gctctattga acagtgttca acaaaaacga     120
   tcagtggtct ggaacacatc tcctacgaag aaaggttgcg agagctgggt ctggagaaga     180
   ggaggctctg gggcaacctc cctgtggcct gtcagtgctt aaaggaggct caagagaaag     240
   atggagagag actttataca cggagctgta gcaatatgac aaggagaaat ggtttaaaat 300
   ggaaagaggg tagatttagg ttagctatca agaagaaatt cttcactcag aggtgatgaa 360
   gcactgtagc aggttgccca gagaagtatt ggatgcttcc ttcctggcag ggcccaaggc 420
   cagactggat gggttctggg cagcctgttc tggtgggaga tgtccctgcc cgtggcacgg 480
   cttggaaatg ggtagccttt gaggtccctc tcagcccaaa tcattctatg ttcttgtggt 540
   tccctgcatt tcagcctttg cctgttgact ctggtgccac tgttggacaa ttctactcag 600
   ctgtgtttca tccctgcccc aaagcagtaa tcccatggat aagcaatgct ctgaggcctt 660
   caccaccatc agggctcctg ctgaccctag cggcctccct tgcagcatga gccctttgtt 720
   gtccgcactg ctgctgctga gggtcctgcc agccccccag ggcctttctt gcccaggcac 780
   tgccacctgc taaaacccag cctgcccatg gcagggcatc gcatgtctga gggatggaac 840
   ggggcaaggg cagtgctgca ggcagtggtt caagaaacgt ttagatgttg tactgaggga 900
   tgtggtttag tgggaaatac tggtgatagg tggatggttg gactggagga tcttagaggt 960
   cttttccaac cttggtgatt atgtgattct atgaagggag ccatagcctg ctcccatcat 1020
   gccccgccag tccggctctc ccgagcactt tggttccgca gcatccaggc ccgctctggg 1080
   gccggtgtgg gcctgggtgc tgccacgtgt ccccgctgca aggcctggcc aaccccgctg 1140
   tgccacaatg caggggctgg gctaggagtg ggcagggagg tgtggggaga ggtgtaggac 1200
   gtggctggga gcgcagggag ttatttgcat aggggggcgt gctgcggaag gacgcgggta 1260
   taaaagggca tcgaggtccc cggcacagcc ccatcggcgt ggggacacac agctgctggg 1320
   attccgccat ggcctgggct cctctcctcc tggcggtgct cgcccacacc tcaggtactc 1380
   gttgcgcccg gtcggggact gtgggcacgg ggctctgtcc cactgctgcg cgggcagggc 1440
   tgtgcgtgcg gggccgtcac tgattgccgt tttctcccct ctctcctctc c 1491

<210> 14
<211> 1712
<212> DNA
<213> Gallus gallus

<400> 14
tctagagacc tcgtctcggt ctttcttccc ccatcgtgaa attgtgacat tttgtcgatt 60

tttggtgatt tgggggtttt tcttggactt ggcggcaggc tggggtctgc caccggcgca 120

gggccgggca ctcagcgcga cagcctgggc tgagtcttgt ccccaccgag ccggagggct 180

ccggtgtgcg ccatggagga cttagggtta ttttgtcaat ggaaagttct taaaatttga 240

ccagaaaatg tgcccgaggt ctgtctctgc cacacgattt cagaaattgt gtctaggtcg 300

atgagaagac agtttttgtc tttgtcagga aattagttgt gagttgttag tccttccctc 360

ttagtcctaa ggactaagac ctttgtcccc ggtctggtct ctcactgggg actcttggct 420

ccagtgccat ggggagccca agtgtcactg acacggtgtc cttgggggtg aaattcagtt 480

tttcagctgt ccaggtaagt gctgtcaaaa aatggaggtt attctgctga aaaagctgag 540

aggaatattt tgtcattttt ttcggaaata tatatatata tatatatata aatatataaa 600

ttatttatat attttatata tatatatata taaaatatat gtatttctct ctttcttttt 660

atatatatat ataaatatat atatatttct ccctctttct ttatatatat ttatagggag 720

agaatctata tttttggcca atttggccaa tttctctctc tctctcttta tatatatata 780

tatatgtata tgtaatttat atatatatat ataaatttat atttatttat ttatttattt 840

atatagatta tttttatata catatatata tataaatttg gccaatttag aagaaattgg 900

ccaaaaaaac ccaatctgaa ccaaattact tagggcataa gtccaaaaaa aaagtgcagt 960

tcaagaattc ctcgctggaa agtttttaat gggggcagtt cttccccatt ttctggtgta 1020

gatatgagtg aagatataga tatgtatgta gatatagatt tggctatagt ttgtttgatg 1080

aacagtctga ggaagagtaa atcttgcctt cctcatggca atttttaatt ctacttcttc 1140

tgagacaagt ctgaacatgc cttgaggtag aaagagcccg aaaattgagt gctttttctt 1200

tgaaatttta attaaaatta aaaaatagcc ttacagcagg aaagaaaggg ggtgatggca 1260

gctaatcgga gttccaaagc tgaaccacgt tcacccaaac acgtggttga aattttgtgt 1320

gtgtatatat ataaatatat atatgtatac aaaagaaaat gcctatatat aaaaaaattg 1380

ttatgtatat tatatagatt tattatagat atgtacataa gatataacac gtatacatat 1440

atatgtagtc tgctgtatgc tttgtataaa tttttataga tatgatgtat attatatgca 1500

tgggttatat atctataata tattttatct gtagaaggaa ctacagtata aaatgcatat 1560

atgcgtatat atacacatat ttatatatat gtgtatatat atgtatacgc acacgtataa 1620

catccatgaa aatagatatg gatgggtgga tgtgtttgtt ttacagaggt gcatgtgtgt 1680

ctgtacatac acagccatac atacgcgtgt gg 1712

<210> 15
<211> 1992
<212> DNA
<213> Gallus gallus

<400> 15
acgacactga actagtgctg actctgcatc catgtctctg tgtccttttg cgtgctgtct 60

gcatctcaca caggggggtc ggccccagta tggggaaggg ctggggggcg catacacaca 120

tattggtaat gttgggggcg gggggggggg gtgggggggt caacagatca gcactggaga 180

cactggtgta taccctggca ccaccaacat ctaaggcagg gtgctttggg gcaattttgg 240

ggcagtttaa ggtctgtgct ggcactgagc acgtggctgt ggccgtgctg tcctcatctc 300

ccacccacta cggtctgtgc gccaggtccc tagcagagat ttgctttatg ctgggaacag 360

ggggagttct ggctctgttc ccttgcattc agacaccctg gtgccccctg ggtgggatgt 420

cagtgtgaat actcctttgt gccctgtgcc tgcagcagcc tgaccctcca cacaccacac 480

gccttgtgtg caccccaccc ctgtcactat ccctctcccc gctccccagg gagattttgc 540

agtggcccct gtagggcagc ttttagcaca gcccccagca gcaagcaagc agaaagcact 600

gctgtgcaca gcttgtcagc tgtgtgtgtt tgctgaggag gatctgtctt ttgctgaggc 660

catcagtctt gtcctgctca acctccatcg atgctgccca cctcaacaca tctacccatc 720

tattccatct acaccaacat ctccattcat cccacccacc caaacatgtc catccatcac 780

aacacctcca tccaacccgc acactccagc acctccaatc attccatcta caccaccatg 840

ctgatctgct acagccactc caacgcaacc gtccattcca tctacaccaa tgtccatcca 900

tcccagccac tccagcacct ccagccatcc cacccaccct atgtctccat ccagccactg 960

gtggggtgca ggacatgggg ccagctctac tgtcaggact ggggtttttg catggcccca 1020

taccacttct gcagaagaga cgcactgaaa gtttggctga ccattttctc cacggtagag 1080

ttgtggcagt tctgtaattt agggtctttt atccagtttg gagatgggct gggatctccc 1140

agctccatgg caggcattca tgacactggg tttagtatct gatgggtggg atgtggctga 1200

acttcatttt ctttccccag tgacaaagtt tttgcagttg aatatgaatt cctgctttct 1260

gctctatgag ttgttttttt cccaggacgt acacagggaa tcagcagtct tcattctccc 1320

tctgccatgt gtagactctc tgccacacag gactgtgctg tcctcatgcc cctgcgccca 1380

aattgctgcc ctctgcccat gcctgccaag ctgagccctg caggctgcca tgctggattg 1440

acatgagccc tgagattggt acagaaatgg tgattttggg gttttctttg cactcaggaa 1500

gctgaaggct caatgctcag tgatggattt accaaactgt gccctgaggc agctgctcat 1560

gctggataaa gtcactggag cacagggaac caggcgctgg gcagggattt ctcatgggcc 1620

ccacttggaa agctgcaggc tgcaaacctg gctctgcctt cacgcctcac cctcatgagg 1680

acaacctcac taattattga ttaaaagatt ttgctaaacc atctccagaa gcaacaaccc 1740

actgaggagc atgtgctgaa ttatacatca cagctccacg gccctgccct catagcaggg 1800

ctgcatggca cccacagtgg cactcagagg gaccacaggg ctgagacagc cgggtctggt 1860

ggtggggaca cagctgagca taggatgagc cccccgggca gtgctgggct ttgctaatga 1920

gcagaagtat ggatagaaag caaccccagg gctccgtacc cagctgcagc tcttgctctg 1980

tcgtgtcctt tg 1992

<210> 16
<211> 11734
<212> DNA
<213> Artificial Sequence

<220>
<223>  Targeting vector

<400>  16
cacctgacgc gccctgtagc ggcgcattaa gcgcggcggg tgtggtggtt acgcgcagcg      60

tgaccgctac acttgccagc gccctagcgc ccgctccttt cgctttcttc ccttcctttc     120

tcgccacgtt cgccggcttt ccccgtcaag ctctaaatcg ggggctccct ttagggttcc     180

gatttagtgc tttacggcac ctcgacccca aaaaacttga ttagggtgat ggttcacgta     240

gtgggccatc gccctgatag acggtttttc gccctttgac gttggagtcc acgttcttta     300

atagtggact cttgttccaa actggaacaa cactcaaccc tatctcggtc tattcttttg     360

atttataagg gattttgccg atttcggcct attggttaaa aaatgagctg atttaacaaa     420

aatttaacgc gaattttaac aaaatattaa cgcttacaat ttccattcgc cattcaggct     480

gcgcaactgt tgggaagggc gatcggtgcg ggcctcttcg ctattacgcc agctggcgaa     540

agggggatgt gctgcaaggc gattaagttg ggtaacgcca gggttttccc agtcacgacg     600

ttgtaaaacg acggccagtg agcgcgcgta atacgactca ctatagggcg aattgggtac     660

cgggcccccc ctcgagtgaa ggtcaccaag gatggagact tcagagctat ctgggacacg     720

tgttttaacg tttgatcagc cttgcagcaa aacagtgttt tgctctattg aacagtgttc     780

aacaaaaacg atcagtggtc tggaacacat ctcctacgaa gaaaggttgc gagagctggg     840

tctggagaag aggaggctct ggggcaacct ccctgtggcc tgtcagtgct taaaggaggc     900

tcaagagaaa gatggagaga gactttatac acggagctgt agcaatatga caaggagaaa     960

tggtttaaaa tggaaagagg gtagatttag gttagctatc aagaagaaat tcttcactca    1020

gaggtgatga agcactgtag caggttgccc agagaagtat tggatgcttc cttcctggca    1080

gggcccaagg ccagactgga tgggttctgg gcagcctgtt ctggtgggag atgtccctgc    1140

ccgtggcacg gcttggaaat gggtagcctt tgaggtccct ctcagcccaa atcattctat    1200

gttcttgtgg ttccctgcat ttcagccttt gcctgttgac tctggtgcca ctgttggaca    1260

attctactca gctgtgtttc atccctgccc caaagcagta atcccatgga taagcaatgc    1320

tctgaggcct tcaccaccat cagggctcct gctgacccta gcggcctccc ttgcagcatg    1380

agccctttgt tgtccgcact gctgctgctg agggtcctgc cagcccccca gggccttttc    1440

tgcccaggca ctgccacctg ctaaacccca gcctgcccat ggcagggcat cgcatgtctg    1500

agggatggaa cggggcaagg gcagtgctgc aggcagtggt tcaagaaacg tttagatgtt    1560

gtactgaggg atgtggttta gtgggaaata ctggtgatag gtggatggtt ggactggagg    1620

atcttagagg tcttttccaa ccttggtgat tatgtgattc tatgaaggga gccatagcct    1680

gctcccatca tgccccgcca gtccggctct cccgagcact ttggttccgc agcatccagg    1740

cccgctctgg ggccggtgtg ggcctgggtg ctgccacgtg tccccgctgc aaggcctggc    1800

caaccccgct gtgccacaat gcaggggctg ggctaggagt gggcagggag gtgtggggag 1860

aggtgtagga cgtggctggg agcgcaggga gttatttgca taggggggcg tgctgcggaa 1920

ggacgcgggt ataaaagggc atcgaggtcc ccggcacagc cccatcggcg tggggacaca 1980

cagctgctgg gattccgcca tggcctgggc tcctctcctc ctggcggtgc tcgcccacac 2040

ctcaggtact cgttgcgccc ggtcggggac tgtgggcacg gggctctgtc ccactgctgc 2100

gcgggcaggg ctgtgcgtgc ggggccgtca ctgattgccg ttttctcccc tctctcctct 2160

ccggcgcgcc tctccaggtt ccctggtgca ggcacagtct gccctgactc agcctgcctc 2220

cgtgtctggg tctcctggac agtcgatcac catctcctgc actggaacca gcagtgacgt 2280

tggtggttat aactatgtct cctggtacca acaaaaccca ggcaaagccc ccaaactcat 2340

gatttatgat gtcagtaatc ggccctcagg gatttctaat cgcttctctg gctccaagtc 2400

tggcaacacg gcctccctga ccatctctgg gctccaggct gacgacgagg ctgattatta 2460

ctgcacctca tatacaaacg acagcaattc tcaggtattc ggcggaggga ccaagctgac 2520

cgtcctaggt gagtcgctca tatgggatcc ataacttcgt atagcataca ttatacgaag 2580

ttatcagaca tgataagata cattgatgag tttggacaaa ccacaactag aatgcagtga 2640

aaaaaatgct ttatttgtga aatttgtgat gctattgctt tatttgtaac cattataagc 2700

tgcaataaac aagttggggt gggcgaagaa ctccagcatg agatccccgc gctggaggat 2760

catccagccg gcgtcccgga aaacgattcc gaagcccaac ctttcataga aggcggcggt 2820

ggaatcgaaa tctcgtagca cgtgtcagtc ctgctcctcg gccacgaagt gcttagccct 2880

cccacacata accagagggc agcaattcac gaatcccaac tgccgtcggc tgtccatcac 2940

tgtccttcac tatggctttg atcccaggat gcagatcgag aagcacctgt cggcaccgtc 3000

cgcaggggct caagatgccc ctgttctcat ttccgatcgc gacgatacaa gtcaggttgc 3060

cagctgccgc agcagcagca gtgcccagca ccacgagttc tgcacaaggt cccccagtaa 3120

aatgatatac attgacacca gtgaagatgc ggccgtcgct agagagagct gcgctggcga 3180

cgctgtagtc ttcagagatg gggatgctgt tgattgtagc cgttgctctt tcaatgaggg 3240

tggattcttc ttgagacaaa ggcttggcca tggtttagtt cctcaccttg tcgtattata 3300

ctatgccgat atactatgcc gatgattaat tgtcaacacg tgctgatcag atccgaaaat 3360

ggatatacaa gctcccggga gctttttgca aaagcctagg cctccaaaaa agcctcctca 3420

ctacttctgg aatagctcag aggcagaggc ggcctcggcc tctgcataaa taaaaaaaat 3480

tagtcagcca tggggcggag aatgggcgga actgggcgga gttaggggcg ggatgggcgg 3540

agttaggggc gggactatgg ttgctgacta attgagatgc atgctttgca tacttctgcc 3600

tgctggggag cctggggact ttccacacct ggttgctgac taattgagat gcatgctttg 3660

catacttctg cctgctgggg agcctgggga ctttccacac cctaactgac acacattcca 3720

cagataactt cgtatagcat acattatacg aagttatgga tccactagtt ctagagacct 3780

cgtctcggtc tttcttcccc catcgtgaaa ttgtgacatt ttgtcgattt ttggtgattt 3840

gggggttttt cttggacttg gcggcaggct ggggtctgcc accggcgcag ggccgggcac 3900

tcagcgcgac agcctgggct gagtcttgtc cccaccgagc cggagggctc cggtgtgcgc 3960

catggaggac ttagggttat tttgtcaatg gaaagttctt aaaatttgac cagaaaatgt 4020

gcccgaggtc tgtctctgcc acacgatttc agaaattgtg tctaggtcga tgagaagaca 4080

gtttttgtct ttgtcaggaa attagttgtg agttgttagt ccttccctct tagtcctaag 4140

gactaagacc tttgtccccg gtctggtctc tcactgggga ctcttggctc cagtgccatg 4200

gggagcccaa gtgtcactga cacggtgtcc ttgggggtga aattcagttt ttcagctgtc 4260

caggtaagtg ctgtcaaaaa atggaggtta ttctgctgaa aaagctgaga ggaatatttt 4320

gtcatttttt tcggaaatat atatatatat atatatataa atatataaat tatttatata 4380

ttttatatat atatatatat aaaatatatg tatttctctc tttcttttta tatatatata 4440

taaatatata tatatttctc cctctttctt tatatatatt tatagggaga gaatctatat 4500

ttttggccaa tttggccaat ttctctctct ctctctttat atatatatat atatgtatat 4560

gtaatttata tatatatata taaatttata tttatttatt tatttattta tatagattat 4620

ttttatatac atatatatat ataaatttgg ccaatttaga agaaattggc caaaaaaacc 4680

caatctgaac caaattactt agggcataag tccaaaaaaa aagtgcagtt caagaattcc 4740

tcgctggaaa gtttttaatg gggcagttcc ttccccattt tctggtgtag atatgagtga 4800

agatatagat atgtatgtag atatagattt ggctatagtt tgtttgatga acagtctgag 4860

gaagagtaaa tcttgccttc ctcatggcaa tttttaattc tacttcttct gagacaagtc 4920

tgaacatgcc ttgaggtaga aagagcccga aaattgagtg ctttttcttt gaaattttaa 4980

ttaaaattaa aaaatagcct tacagcagga aagaaagggg gtgatggcag ctaatcggag 5040

ttccaaagct gaaccacgtt cacccaaaca cgtggttgaa attttgtgtg tgtatatata 5100

taaatatata tatgtataca aaagaaaatg cctatatata aaaaaattgt tatgtatatt 5160

atatagattt attatagata tgtacataag atataacacg tatacatata tatgtagtct 5220

gctgtatgct ttgtataaat ttttatagat atgatgtata ttatatgcat gggttatata 5280

tctataatat attttatctg tagaaggaac tacagtataa aatgcatata tgcgtatata 5340

tacacatatt tatatatatg tgtatatata tgtatacgca cacgtataac atccatgaaa 5400

atagatatgg atgggtggat gtgtttgttt tacagaggtg catgtgtgtc tgtacataca 5460

cagccataca tacgcgtgtg gcgcgcctct gcctctctct tgcaggtcag cccaaggctg 5520

ccccctcggt cactctgttc ccgccctcct ctgaggagct tcaagccaac aaggccacac 5580

tggtgtgtct cataagtgac ttctacccgg gagccgtgac agtggcctgg aaggcagata 5640

gcagccccgt caaggcggga gtggagacca ccacaccctc caaacaaagc aacaacaagt 5700

acgcggccag cagctacctg agcctgacgc ctgagcagtg gaagtcccac agaagctaca 5760

gctgccaggt cacgcatgaa gggagcaccg tggagaagac agtggcccct acagaatgtt 5820

catagtagtc ccactccata tgtcccactg gggatgcaat gtgaggacag tggttcctca 5880

ccctccctgt ccctctgggc cgctgctggt ggcagcagcc ctcacttccc actcagatgt 5940

cccccaccgt gcccccatca cccacctctg cctgtcgact cctcttgccc tcatctctcc 6000

aggtgtcaca ttaataaacg gatccataac ttcgtatagg atactttata cgaagttatt 6060

ttatttgtga aatttgtgat gctattgctt tatttgtaac cattataagc tgcaataaac 6120

aagttggggt gggcgaagaa ctccagcatg agatccccgc gctggaggat catccagccg 6180

gcgtcccgga aaacgattcc gaagcccaac ctttcataga aggcggcggt ggaatcgaaa 6240

tctcgtgatg gcaggttggg cgtcgcttgg tcggtcattt cgaaccccag agtcccgctc 6300

agaagaactc gtcaagaagg cgatagaagg cgatgcgctg cgaatcggga gcggcgatac 6360

cgtaaagcac gaggaagcgg tcagcccatt cgccgccaag ctcttcagca atatcacggg 6420

tagccaacgc tatgtcctga tagcggtccg ccacacccag ccggccacag tcgatgaatc 6480

cagaaaagcg gccattttcc accatgatat tcggcaagca ggcatcgcca tgggtcacga 6540

cgagatcctc gccgtcgggc atgcgcgcct tgagcctggc gaacagttcg gctggcgcga 6600

gcccctgatg ctcttcgtcc agatcatcct gatcgacaag accggcttcc atccgagtac 6660

gtgctcgctc gatgcgatgt ttcgcttggt ggtcgaatgg gcaggtagcc ggatcaagcg 6720

tatgcagccg ccgcattgca tcagccatga tggatacttt ctcggcagga gcaaggtgag 6780

atgacaggag atcctgcccc ggcacttcgc ccaatagcag ccagtccctt cccgcttcag 6840

tgacaacgtc gagcacagct gcgcaaggaa cgcccgtcgt ggccagccac gatagccgcg 6900

ctgcctcgtc ctgcagttca ttcagggcac cggacaggtc ggtcttgaca aaaagaaccg 6960

ggcgcccctg cgctgacagc cggaacacgg cggcatcaga gcagccgatt gtctgttgtg 7020

cccagtcata gccgaatagc ctctccaccc aagcggccgg agaacctgcg tgcaatccat 7080

cttgttcaat catgcgaaac gatcctcatc ctgtctcttg atcagatccg aaaatggata 7140

tacaagctcc cgggagcttt ttgcaaaagc ctaggcctcc aaaaaagcct cctcactact 7200

tctggaatag ctcagaggca gaggcggcct cggcctctgc ataaataaaa aaaattagtc 7260

agccatgggg cggagaatgg gcggaactgg gcggagttag gggcgggatg ggcggagtta 7320

ggggcgggac tatggttgct gactaattga gatgcatgct ttgcatactt ctgcctgctg 7380

gggagcctgg ggactttcca cacctggttg ctgactaatt gagatgcatg ctttgcatac 7440

ttctgcctgc tggggagcct ggggactttc cacaccctaa ataacttcgt ataggatact 7500

ttatacgaag ttatggatcc acgacactga actagtgctg actctgcatc catgtctctg 7560

tgtccttttg cgtgctgtct gcatctcaca caggggggtc ggccccagta tggggaaggg 7620

ctggggggcg catacacaca tattggtaat gttgggggcg gggggggggg gtgggggggt 7680

caacagatca gcactggaga cactggtgta taccctggca ccaccaacat ctaaggcagg 7740

gtgctttggg gcaattttgg ggcagtttaa ggtctgtgct ggcactgagc acgtggctgt 7800

ggccgtgctg tcctcatctc ccacccacta cggtctgtgc gccaggtccc tagcagagat 7860

ttgctttatg ctgggaacag ggggagttct ggctctgttc ccttgcattc agacaccctg 7920

gtgccccctg ggtgggatgt cagtgtgaat actcctttgt gccctgtgcc tgcagcagcc 7980

tgaccctcca cacaccacac gccttgtgtg caccccaccc ctgtcactat ccctctcccc 8040

gctccccagg gagattttgc agtggcccct gtagggcagc ttttagcaca gcccccagca 8100

gcaagcaagc agaaagcact gctgtgcaca gcttgtcagc tgtgtgtgtt tgctgaggag 8160

gatctgtctt ttgctgaggc catcagtctt gtcctgctca acctccatcg atgctgccca 8220

cctcaacaca tctacccatc tattccatct acaccaacat ctccattcat cccacccacc 8280

caaacatgtc catccatcac aacacctcca tccaacccgc acactccagc acctccaatc 8340

attccatcta caccaccatg ctgatctgct acagccactc caacgcaacc gtccattcca 8400

tctacaccaa tgtccatcca tcccagccac tccagcacct ccagccatcc cacccaccct 8460

atgtctccat ccagccactg gtggggtgca ggacatgggg ccagctctac tgtcaggact 8520

ggggtttttg catggcccca taccacttct gcagaagaga cgcactgaaa gtttggctga 8580

ccattttctc cacggtagag ttgtggcagt tctgtaattt agggtctttt atccagtttg 8640

gagatgggct gggatctccc agctccatgg caggcattca tgacactggg tttagtatct 8700

gatgggtggg atgtggctga acttcatttt ctttccccag tgacaaagtt tttgcagttg 8760

aatatgaatt cctgctttct gctctatgag ttgttttttt cccaggacgt acacagggaa 8820

tcagcagtct tcattctccc tctgccatgt gtagactctc tgccacacag gactgtgctg 8880

tcctcatgcc cctgcgccca aattgctgcc ctctgcccat gcctgccaag ctgagccctg 8940

caggctgcca tgctggattg acatgagccc tgagattggt acagaaatgg tgattttggg 9000

gttttctttg cactcaggaa gctgaaggct caatgctcag tgatggattt accaaactgt 9060

gccctgaggc agctgctcat gctggataaa gtcactggag cacagggaac caggcgctgg 9120

gcagggattt ctcatgggcc ccacttggaa agctgcaggc tgcaaacctg gctctgcctt 9180

cacgcctcac cctcatgagg acaacctcac taattattga ttaaaagatt ttgctaaacc 9240

atctccagaa gcaacaaccc actgaggagc atgtgctgaa ttatacatca cagctccacg 9300

gccctgccct catagcaggg ctgcatggca cccacagtgg cactcagagg gaccacaggg 9360

ctgagacagc cgggtctggt ggtggggaca cagctgagca taggatgagc cccccgggca 9420

gtgctgggct ttgctaatga gcagaagtat ggatagaaag caaccccagg gctccgtacc 9480

cagctgcagc tcttgctctg tcgtgtcctt tggcggccgc caccgcggtg gagctccagc 9540

ttttgttccc tttagtgagg gttaattgcg cgcttggcgt aatcatggtc atagctgttt 9600

cctgtgtgaa attgttatcc gctcacaatt ccacacaaca tacgagccgg aagcataaag 9660

tgtaaagcct ggggtgccta atgagtgagc taactcacat taattgcgtt gcgctcactg 9720

cccgctttcc agtcgggaaa cctgtcgtgc cagctgcatt aatgaatcgg ccaacgcgcg 9780

gggagaggcg gtttgcgtat tgggcgctct tccgcttcct cgctcactga ctcgctgcgc 9840

tcggtcgttc ggctgcggcg agcggtatca gctcactcaa aggcggtaat acggttatcc 9900

acagaatcag gggataacgc aggaaagaac atgtgagcaa aaggccagca aaaggccagg 9960

aaccgtaaaa aggccgcgtt gctggcgttt ttccataggc tccgcccccc tgacgagcat 10020

cacaaaaatc gacgctcaag tcagaggtgg cgaaacccga caggactata aagataccag 10080

gcgtttcccc ctggaagctc cctcgtgcgc tctcctgttc cgaccctgcc gcttaccgga 10140

tacctgtccg cctttctccc ttcgggaagc gtggcgcttt ctcatagctc acgctgtagg 10200

tatctcagtt cggtgtaggt cgttcgctcc aagctgggct gtgtgcacga accccccgtt 10260

cagcccgacc gctgcgcctt atccggtaac tatcgtcttg agtccaaccc ggtaagacac 10320

gacttatcgc cactggcagc agccactggt aacaggatta gcagagcgag gtatgtaggc 10380

ggtgctacag agttcttgaa gtggtggcct aactacggct acactagaag gacagtattt 10440

ggtatctgcg ctctgctgaa gccagttacc ttcggaaaaa gagttggtag ctcttgatcc 10500

ggcaaacaaa ccaccgctgg tagcggtggt ttttttgttt gcaagcagca gattacgcgc 10560

agaaaaaaag gatctcaaga agatcctttg atcttttcta cggggtctga cgctcagtgg 10620

aacgaaaact cacgttaagg gattttggtc atgagattat caaaaaggat cttcacctag 10680

atccttttaa attaaaaatg aagttttaaa tcaatctaaa gtatatatga gtaaacttgg 10740

tctgacagtt accaatgctt aatcagtgag gcacctatct cagcgatctg tctatttcgt 10800

tcatccatag ttgcctgact ccccgtcgtg tagataacta cgatacggga gggcttacca 10860

tctggcccca gtgctgcaat gataccgcga gacccacgct caccggctcc agatttatca 10920

gcaataaacc agccagccgg aagggccgag cgcagaagtg gtcctgcaac tttatccgcc 10980

tccatccagt ctattaattg ttgccgggaa gctagagtaa gtagttcgcc agttaatagt 11040

ttgcgcaacg ttgttgccat tgctacaggc atcgtggtgt cacgctcgtc gtttggtatg 11100

gcttcattca gctccggttc ccaacgatca aggcgagtta catgatcccc catgttgtgc 11160

aaaaaagcgg ttagctcctt cggtcctccg atcgttgtca gaagtaagtt ggccgcagtg 11220

ttatcactca tggttatggc agcactgcat aattctctta ctgtcatgcc atccgtaaga 11280

tgcttttctg tgactggtga gtactcaacc aagtcattct gagaatagtg tatgcggcga 11340

ccgagttgct cttgcccggc gtcaatacgg gataataccg cgccacatag cagaacttta 11400

aaagtgctca tcattggaaa acgttcttcg gggcgaaaac tctcaaggat cttaccgctg 11460

ttgagatcca gttcgatgta acccactcgt gcacccaact gatcttcagc atcttttact 11520

ttcaccagcg tttctgggtg agcaaaaaca ggaaggcaaa atgccgcaaa aaagggaata 11580

agggcgacac ggaaatgttg aatactcata ctcttccttt ttcaatatta ttgaagcatt 11640

tatcagggtt attgtctcat gagcggatac atatttgaat gtatttagaa aaataaacaa 11700

ataggggttc cgcgcacatt tccccgaaaa gtgc 11734

<210> 17
<211> 335
<212> DNA
<213> Artificial Sequence

<220>
<223> Synthetic DNA

<400> 17
cagtctgccc tgactcagcc tgcctccgtg tctgggtctc ctggacagtc gatcaccatc 60

tcctgcactg gaaccagcag tgacgttggt tataactatg tctcctggta ccaacaaaac 120

ccaggcaaag cccccaaact catgatttat gatggtaatc ggccctcagg gatttctaat 180

cgcttctctg gctccaagtc tggcaacacg gcctccctga ccatctctgg gctccaggct 240

gacgacgagg ctgattatta ctgcacctca tatacaaacg acagctctca ggtattcggc 300

ggagggacca agctgaccgt cctaggtggc gcgcc 335

<210> 18
<211> 26
<212> DNA
<213> Artificial Sequence

<220>
<223> Synthetic Primer

<400> 18
ttctgtgagc tgagaaaagg agtgta 26

<210> 19
<211> 24
<212> DNA
<213> Artificial Sequence

<220>
<223> Synthetic Primer

<400> 19
cctgcattgt ggcacagcgg ggtt 24

<210>  20
<211>  2387
<212>  DNA
<213>  Gallus gallus

<400>  20
   tagatgggag tttaagagtg aattgtggtc catggcatat gcctgtcagc atagggatgc      60
   tgtaggaatt gcaccttggc ttgagcccct ctcctcctga tgatgcttgc ccacagctct     120
   gatgtcctgg gaaggctcac tgcaccttgt tgtcagtgaa actgcaataa actatcaaga     180
   tttgatataa ttctgcccct ctgttctact tgtgagacca cacctggagt actgcatcca     240
   gttctggtac cactgcatcc agttctggta ccaccaacac aaaaaggaca tggagctgct     300
   ggagcagggc cagaggaggg ccatgaagat gaacagaggg ctgaagcacc tcccctatgg     360
   ggacaggctg agagacatgg ggcttttgag actggagaag agaaggctcc atgggacctt     420
   tcagtgtcct tccaatactt gaaagggtcc ttgaggaaag ctggggaggg acttcctata     480
   aggacatata gcaacaggac aaaaggaaag ggttttaaac tggaagaaag tagatttagt     540
   ctagatagta acaagaaatt ctttacagta aggatcacga gacactgaaa caggttgccc     600
   aaagcagctg tggatgcccc ctccctggaa gtgttccagg ccaggctgga tggggctttg     660
   agcaacctgt tctagtgaga ggtgaccctg cctacagcag gggagttgaa cctagatggt     720
   cttaaatgtc ccttccaacc caaatcattc tatggcaatt ctatagagaa cacctatgct     780
   gagagtagct acaagtatgg ctggttcctg gcagtgcccc tgtctctgtg atctatagca     840
   gcaccaacag accctcaaac atcccttcac gattctccgg ttccaaatcc ggctccacac     900
   acacattaac catcactggg gtccaagccg atgacaaggc tgtctattac tgtgggagca     960
   cagacagcag ctatgttggt atcacagtga catggagcaa ttaaaaatga tgcataacct    1020
   tttgcacatc tgtaatgcta taaggtgaga gaaagtaagc aataatagca gagcagcaca    1080
   atcctaggct acagcaaatg agaacaagcc cagatgcagc cacggaaata aagaaaaaaa    1140
   aactgcttac ctttagaaag cctcatatag acaggatctc ccagcaagat acccacacct    1200
   ccactgccaa cccttaaatg agggctggga agggctggat cctggctcca cccctttcaa    1260
   tcactcaggt acattgcaca cacctgagct cccctggctg gccctgcctt cccaccaggt    1320
   gctcaatcac tgctttgggc tgtgactcag catctccact acagcatcac agggagcggt    1380
   tgtcggtctc tgctgttcct atgtgggtgc cccgggacac ctgaaggcat gagtggcagt    1440
   gagggctccc agctctatcc atggtgaaca gacattatgt ttatgcttta cagcagcatg    1500
   aatctggcag tggtgttatt gctgtgatgc tgctgtctat gctcccacag taatagatag    1560
   cctcgtcctc ggcttggacc ccagtgatgg ttaatgtgtt tgtggagccg gatccagaac    1620
   cggagaatcg tgaagggatg tccgagggtc tgttattgcc attatagatc acagtgacag    1680
   gggcactgcc aggcatagtc gctgtaaccc ccggagcagg tgatctcgac ggttcctccc 1740
   aggtttgctg acaccgaggc cggctgagtc agcgctgcct gcaccaggga acctggagag 1800
   ggagaggaga gaggggagaa aatggatctg agcaattgcc tgacacacac agccctcccc 1860
   agactgtggg atgaggccct gtgcccgcag tcacatgtgg aatatcaaga cacacacatc 1920
   tatgacaatc acaatctgat tatcaaccac tatggctggt accagcagaa ggcacctagc 1980
   agtgcccctg tcactctgat ctactatgat gatgagagac cctcgaacat cccttcacga 2040
   ttctccggtt ccaaatccgg ctccacacac acattaacca tcactggggt ccaagccgac 2100
   gacgaggctg tctattactg tgggaatgaa gacagcagcg gtactggtgc cccagtgaca 2160
   cagagcaatg gagatatgat acacagacct gacatctcag ggagcagctg gtcattatcc 2220
   tggtgacaca agtcccaggc atggcccagc actgtgctgc aggtggctat gccgggtgcc 2280
   ctgctctgcc ctcctgtaga gcctcaggac tgtgcccatc actcagggag gagatggtcc 2340
   cagcagccct gtccctgccc tgcactgcac ttagctcctg gacccca 2387

<210> 21
<211> 1957
<212> DNA
<213> Gallus gallus

<400> 21
atattgcctc cctgtgttgc tgttgcaggg ttgcttctgc ctcatactgg tttctccctt 60

ctggaggtgg ccaaaagccg ggccctgtgc aaccctggtg cataaatacc ttatggcccc 120

taagtagggc aggtgtggga cacgctctgg cacctggggt gtgtgcaagt gctcaggaag 180

acctgcaggc acaggtggca gtgggggtct ctggctgtgc tcgagcagca gctgcctggg 240

gtaagggtag tactctgtgc atgaacaatg ctgcagggct cagctctgct cagaccacga 300

ccctggcacc aacagagacc tgcctggctc tgtggtcatg taaaccttta caggagctca 360

agacaaggct gtttattact gctctggcag gaaagaagca ctggccatgg tcatagagag 420

ttccagcaac aggaaagtga gagcccaagc tgctgaggta ccagggctcc tcaggtgcct 480

gctgcagcag cttggacaca gtcgaggaac agcaattgta cctgtgtggt ggatcaggct 540

gtgctgcctg tgaacctatt ctagcacatc tgtcacctct gtgccactca cagggatacc 600

acccctgaga cccctacccc atcagcctct gtgtgggata tggtgttggg cccaagggct 660

ctgttgcaca gggagataga ggcctgggga ggagggaaag cattgaggtg gtgttgatac 720

cagggatgtg agcccaagca agagatcagc agagcaagga ggaagaattg caggtgttgg 780

ggctggggaa agccccagat ggctggagct ggtgggccca ctggagatct cctcctccca 840

tcctgctcca tgctggggca gctgctgcag gctgaccagg gcctgcccgg gcacgttgtg 900

aaggtcacca aggatggaga cttcagagct atctgggaca cgtgttttaa cgtttgatca 960

gccttgcagc aaaacagtgt tttgctctat tgaacagtgt tcaacaaaaa cgatcagtgg 1020

cctggaacac atctcctacg aagaaaggtt gcgagagctg ggtctggaga agagaaggct 1080

ctggggcaac ctccctgtgg cctgtcagtg cttaaaggag gctcaagaga aagatggaga 1140

gagactttat acacggagct gtagcaatat gacaaggaga aatggtttaa aatggaaaga 1200

gggtagattt aggttagcta tcaagaagaa attcttcact cagaggtgat gaagcactgt 1260

agcaggttgc ccagagaagt attggatgct tccttcctgg cagggcccaa ggccagactg 1320

gatgggttct gggcagcctg ttctggtggg agatgtccct gcctgtggca tggcttggaa 1380

atgggtagcc tttgaggtcc ctctcagccc aaatcattct atgttcttgt ggttccctgc 1440

atttcagcct ttgcctgttg actctggtgc cactgttgga caattctact cagctgcgtt 1500

tcatccctgc cccaaagcag taatcccatg gataagcaat gctctgaggc cttcaccacc 1560

ctcagggctc ctgctgaccc tagcggcctc cctggcagca tgagcccttt gttgtccgca 1620

ctgctgctgc cgagggtcct gccagccccc cagggccttt tctgcccagg cactgccacc 1680

tgctaaaacc cagcctgccc atggcagggc atcgcatgtc tgagggatgg aacggggcaa 1740

gggcagtgct gcaggcagtg gttcaagaaa cgtttagatg ttgtactgag ggatgtggtt 1800

tagtgggaaa tactggtgat aggtggatgg ttggactgga ggatcttaga ggtcttttcc 1860

aaccttggtg attatgtgat tctatgaagg gagccatagc ctgctcccat catgccccgc 1920

cagtccggct ctcccgagca ctttggttcc gcagcat 1957

<210> 22
<211> 9897
<212> DNA
<213> Artificial Sequence

<220>
<223> Targeting vector

<400> 22
agcgcccaat acgcaaaccg cctctccccg cgcgttggcc gattcattaa tgcagctggc 60

acgacaggtt tcccgactgg aaagcgggca gtgagcgcaa cgcaattaat gtgagttagc 120

tcactcatta ggcaccccag gctttacact ttatgcttcc ggctcgtatg ttgtgtggaa 180

ttgtgagcgg ataacaattt cacacaggaa acagctatga ccatgattac gccaagctca 240

gaattaaccc tcactaaagg gactagtcct gcaggtttaa acgaattcgc ccttgtcgac 300

tagatgggag tttaagagtg aattgtggtc catggcatat gcctgtcagc atagggatgc 360

tgtaggaatt gcaccttggc ttgagcccct ctcctcctga tgatgcttgc ccacagctct 420

gatgtcctgg gaaggctcac tgcaccttgt tgtcagtgaa actgcaataa actatcaaga 480

tttgatataa ttctgcccct ctgttctact tgtgagacca cacctggagt actgcatcca 540

gttctggtac cactgcatcc agttctggta ccaccaacac aaaaaggaca tggagctgct 600

ggagcagggc cagaggaggg ccatgaagat gaacagaggg ctgaagcacc tcccctatgg 660

ggacaggctg agagacatgg ggcttttgag actggagaag agaaggctcc atggaccttt 720

tcagtgtcct tccaatactt gaaagggtcc ttgaggaaag ctggggaggg acttcctata 780

aggacatata gcaacaggac aaaaggaaag ggttttaaac tggaagaaag tagatttagt 840

ctagatagta acaagaaatt ctttacagta aggatcacga gacactgaaa caggttgccc 900

aaagcagctg tggatgcccc ctccctggaa gtgttccagg ccaggctgga tggggctttg 960

agcaacctgt tctagtgaga ggtgaccctg cctacagcag gggagttgaa cctagatggt 1020

cttaaatgtc ccttccaacc caaatcattc tatggcaatt ctatagagaa cacctatgct 1080

gagagtagct acaagtatgg ctggttcctg gcagtgcccc tgtctctgtg atctatagca 1140

gcaccaacag accctcaaac atcccttcac gattctccgg ttccaaatcc ggctccacac 1200

acacattaac catcactggg gtccaagccg atgacaaggc tgtctattac tgtgggagca 1260

cagacagcag ctatgttggt atcacagtga catggagcaa ttaaaaatga tgcataacct 1320

tttgcacatc tgtaatgcta taaggtgaga gaaagtaagc aataatagca gagcagcaca 1380

atcctaggct acagcaaatg agaacaagcc cagatgcagc cacggaaata aagaaaaaaa 1440

aactgcttac ctttagaaag cctcatatag acaggatctc ccagcaagat acccacacct 1500

ccactgccaa cccttaaatg agggctggga agggctggat cctggctcca cccctttcaa 1560

tcactcaggt acattgcaca cacctgagct cccctggctg gccctgcctt cccaccaggt 1620

gctcaatcac tgctttgggc tgtgactcag catctccact acagcatcac agggagcggt 1680

tgtcggtctc tgctgttcct atgtgggtgc cccgggacac ctgaaggcat gagtggcagt 1740

gagggctccc agctctatcc atggtgaaca gacattatgt ttatgcttta cagcagcatg 1800

aatctggcag tggtgttatt gctgtgatgc tgctgtctat gctcccacag taatagatag 1860

cctcgtcctc ggcttggacc ccagtgatgg ttaatgtgtt tgtggagccg gatccagaac 1920

cggagaatcg tgaagggatg tccgagggtc tgttattgcc attatagatc acagtgacag 1980

gggcactgcc aggcatagtc gctgtaaccc ccggagcagg tgatctcgac ggttcctccc 2040

aggtttgctg acaccgaggc cggctgagtc agcgctgcct gcaccaggga acctggagag 2100

ggagaggaga gaggggagaa aatggatctg agcaattgcc tgacacacac agccctcccc 2160

agactgtggg atgaggccct gtgcccgcag tcacatgtgg aatatcaaga cacacacatc 2220

tatgacaatc acaatctgat tatcaaccac tatggctggt accagcagaa ggcacctagc 2280

agtgcccctg tcactctgat ctactatgat gatgagagac cctcgaacat cccttcacga 2340

ttctccggtt ccaaatccgg ctccacacac acattaacca tcactggggt ccaagccgac 2400

gacgaggctg tctattactg tgggaatgaa gacagcagcg gtactggtgc cccagtgaca 2460

cagagcaatg gagatatgat acacagacct gacatctcag ggagcagctg gtcattatcc 2520

tggtgacaca agtcccaggc atggcccagc actgtgctgc aggtggctat gccgggtgcc 2580

ctgctctgcc ctcctgtaga gcctcaggac tgtgcccatc actcagggag gagatggtcc 2640

cagcagccct gtccctgccc tgcactgcac ttagctcctg gaccccatca atttctgaga 2700

actgtcattc tcggaatacc tcgatcgata acttcgtata atgtatacta tacgaagtta 2760

ttttatttgt gaaatttgtg atgctattgc tttatttgta accattataa gctgcaataa 2820

acaagttggg gtgggcgaag aactccagca tgagatcccc gcgctggagg atcatccagc 2880

cggcgtcccg gaaaacgatt ccgaagccca acctttcata gaaggcggcg gtggaatcga 2940

aatctcgtga tggcaggttg ggcgtcgctt ggtcggtcat ttcgaacccc agagtcccgc 3000

tcagaagaac tcgtcaagaa ggcgatagaa ggcgatgcgc tgcgaatcgg gagcggcgat 3060

accgtaaagc acgaggaagc ggtcagccca ttcgccgcca agctcttcag caatatcacg 3120

ggtagccaac gctatgtcct gatagcggtc cgccacaccc agccggccac agtcgatgaa 3180

tccagaaaag cggccatttt ccaccatgat attcggcaag caggcatcgc catgggtcac 3240

gacgagatcc tcgccgtcgg gcatgcgcgc cttgagcctg gcgaacagtt cggctggcgc 3300

gagcccctga tgctcttcgt ccagatcatc ctgatcgaca agaccggctt ccatccgagt 3360

acgtgctcgc tcgatgcgat gtttcgcttg gtggtcgaat gggcaggtag ccggatcaag 3420

cgtatgcagc cgccgcattg catcagccat gatggatact ttctcggcag gagcaaggtg 3480

agatgacagg agatcctgcc ccggcacttc gcccaatagc agccagtccc ttcccgcttc 3540

agtgacaacg tcgagcacag ctgcgcaagg aacgcccgtc gtggccagcc acgatagccg 3600

cgctgcctcg tcctgcagtt cattcagggc accggacagg tcggtcttga caaaaagaac 3660

cgggcgcccc tgcgctgaca gccggaacac ggcggcatca gagcagccga ttgtctgttg 3720

tgcccagtca tagccgaata gcctctccac ccaagcggcc ggagaacctg cgtgcaatcc 3780

atcttgttca atcatgcgaa acgatcctca tcctgtctct tgatcagatc cgaaaatgga 3840

tatacaagct cccgggagct ttttgcaaaa gcctaggcct ccaaaaaagc ctcctcacta 3900

cttctggaat agctcagagg cagaggcggc ctcggcctct gcataaataa aaaaaattag 3960

tcagccatgg ggcggagaat gggcggaact gggcggagtt aggggcggga tgggcggagt 4020

taggggcggg actatggttg ctgactaatt gagatgcatg ctttgcatac ttctgcctgc 4080

tggggagcct ggggactttc cacacctggt tgctgactaa ttgagatgca tgctttgcat 4140

acttctgcct gctggggagc ctggggactt tccacaccct aaataacttc gtataatgta 4200

tactatacga agttatctcg tgtccgataa ctgtaattat cggaccgtca atcgattcgc 4260

gaggcgcgcc atattgcctc cctgtgttgc tgttgcaggg ttgcttctgc ctcatactgg 4320

tttctccctt ctggaggtgg ccaaaagccg ggccctgtgc aaccctggtg cataaatacc 4380

ttatggcccc taagtagggc aggtgtggga cacgctctgg cacctggggt gtgtgcaagt 4440

gctcaggaag acctgcaggc acaggtggca gtgggggtct ctggctgtgc tcgagcagca 4500

gctgcctggg gtaagggtag tactctgtgc atgaacaatg ctgcagggct cagctctgct 4560

cagaccacga ccctggcacc aacagagacc tgcctggctc tgtggtcatg taaaccttta 4620

caggagctca agacaaggct gtttattact gctctggcag gaaagaagca ctggccatgg 4680

tcatagagag ttccagcaac aggaaagtga gagcccaagc tgctgaggta ccagggctcc 4740

tcaggtgcct gctgcagcag cttggacaca gtcgaggaac agcaattgta cctgtgtggt 4800

ggatcaggct gtgctgcctg tgaacctatt ctagcacatc tgtcacctct gtgccactca 4860

cagggatacc acccctgaga cccctacccc atcagcctct gtgtgggata tggtgttggg 4920

cccaagggct ctgttgcaca gggagataga ggcctgggga ggagggaaag cattgaggtg 4980

gtgttgatac cagggatgtg agcccaagca agagatcagc agagcaagga ggaagaattg 5040

caggtgttgg ggctggggaa agccccagat ggctggagct ggtggggcca ctggagatct 5100

cctcctccca tcctgctcca tgctggggca gctgctgcag gctgaccagg gcctgcccgg 5160

gcacgttgtg aaggtcacca aggatggaga cttcagagct atctgggaca cgtgttttaa 5220

cgtttgatca gccttgcagc aaaacagtgt tttgctctat tgaacagtgt tcaacaaaaa 5280

cgatcagtgg cctggaacac atctcctacg aagaaaggtt gcgagagctg ggtctggaga 5340

agagaaggct ctggggcaac ctccctgtgg cctgtcagtg cttaaaggag gctcaagaga 5400

aagatggaga gagactttat acacggagct gtagcaatat gacaaggaga aatggtttaa 5460

aatggaaaga gggtagattt aggttagcta tcaagaagaa attcttcact cagaggtgat 5520

gaagcactgt agcaggttgc ccagagaagt attggatgct tccttcctgg cagggcccaa 5580

ggccagactg gatgggttct gggcagcctg ttctggtggg agatgtccct gcctgtggca 5640

tggcttggaa atgggtagcc tttgaggtcc ctctcagccc aaatcattct atgttcttgt 5700

ggttccctgc atttcagcct ttgcctgttg actctggtgc cactgttgga caattctact 5760

cagctgcgtt tcatccctgc cccaaagcag taatcccatg gataagcaat gctctgaggc 5820

cttcaccacc ctcagggctc ctgctgaccc tagcggcctc cctggcagca tgagcccttt 5880

gttgtccgca ctgctgctgc cgagggtcct gccagccccc cagggccttt tctgcccagg 5940

cactgccacc tgctaaaacc cagcctgccc atggcagggc atcgcatgtc tgagggatgg 6000

aacggggcaa gggcagtgct gcaggcagtg gttcaagaaa cgtttagatg ttgtactgag 6060

ggatgtggtt tagtgggaaa tactggtgat aggtggatgg ttggactgga ggatcttaga 6120

ggtcttttcc aaccttggtg attatgtgat tctatgaagg gagccatagc ctgctcccat 6180

catgccccgc cagtccggct ctcccgagca ctttggttcc gcagcatgcg gccgcaaggg 6240

cgaattcgcg gccgctaaat tcaattcgcc ctatagtgag tcgtattaca attcactggc 6300

cgtcgtttta caacgtcgtg actgggaaaa ccctggcgtt acccaactta atcgccttgc 6360

agcacatccc cctttcgcca gctggcgtaa tagcgaagag gcccgcaccg atcgcccttc 6420

ccaacagttg cgcagcctat acgtacggca gtttaaggtt tacacctata aaagagagag 6480

ccgttatcgt ctgtttgtgg atgtacagag tgatattatt gacacgccgg ggcgacggat 6540

ggtgatcccc ctggccagtg cacgtctgct gtcagataaa gtctcccgtg aactttaccc 6600

ggtggtgcat atcggggatg aaagctggcg catgatgacc accgatatgg ccagtgtgcc 6660

ggtctccgtt atcggggaag aagtggctga tctcagccac cgcgaaaatg acatcaaaaa 6720

cgccattaac ctgatgttct ggggaatata aatgtcaggc atgagattat caaaaaggat 6780

cttcacctag atccttttca cgtagaaagc cagtccgcag aaacggtgct gaccccggat 6840

gaatgtcagc tactgggcta tctggacaag ggaaaacgca agcgcaaaga gaaagcaggt 6900

agcttgcagt gggcttacat ggcgatagct agactgggcg gttttatgga cagcaagcga 6960

accggaattg ccagctgggg cgccctctgg taaggttggg aagccctgca aagtaaactg 7020

gatggctttc ttgccgccaa ggatctgatg gcgcagggga tcaagctctg atcaagagac 7080

aggatgagga tcgtttcgca tgattgaaca agatggattg cacgcaggtt ctccggccgc 7140

ttgggtggag aggctattcg gctatgactg ggcacaacag acaatcggct gctctgatgc 7200

cgccgtgttc cggctgtcag cgcaggggcg cccggttctt tttgtcaaga ccgacctgtc 7260

cggtgccctg aatgaactgc aagacgaggc agcgcggcta tcgtggctgg ccacgacggg 7320

cgttccttgc gcagctgtgc tcgacgttgt cactgaagcg ggaagggact ggctgctatt 7380

gggcgaagtg ccggggcagg atctcctgtc atctcacctt gctcctgccg agaaagtatc 7440

catcatggct gatgcaatgc ggcggctgca tacgcttgat ccggctacct gcccattcga 7500

ccaccaagcg aaacatcgca tcgagcgagc acgtactcgg atggaagccg gtcttgtcga 7560

tcaggatgat ctggacgaag agcatcaggg gctcgcgcca gccgaactgt tcgccaggct 7620

caaggcgagc atgcccgacg gcgaggatct cgtcgtgacc catggcgatg cctgcttgcc 7680

gaatatcatg gtggaaaatg gccgcttttc tggattcatc gactgtggcc ggctgggtgt 7740

ggcggaccgc tatcaggaca tagcgttggc tacccgtgat attgctgaag agcttggcgg 7800

cgaatgggct gaccgcttcc tcgtgcttta cggtatcgcc gctcccgatt cgcagcgcat 7860

cgccttctat cgccttcttg acgagttctt ctgaattatt aacgcttaca atttcctgat 7920

gcggtatttt ctccttacgc atctgtgcgg tatttcacac cgcatcaggt ggcacttttc 7980

ggggaaatgt gcgcggaacc cctatttgtt tatttttcta aatacattca aatatgtatc 8040

cgctcatgag attatcaaaa aggatcttca cctagatcct tttaaattaa aaatgaagtt 8100

ttaaatcaat ctaaagtata tatgagtaaa cttggtctga cagttaccaa tgcttaatca 8160

gtgaggcacc tatctcagcg atctgtctat ttcgttcatc catagttgcc tgactccccg 8220

tcgtgtagat aactacgata cgggagggct taccatctgg ccccagtgct gcaatgatac 8280

cgcgagaccc acgctcaccg gctccagatt tatcagcaat aaaccagcca gccggaaggg 8340

ccgagcgcag aagtggtcct gcaactttat ccgcctccat ccagtctatt aattgttgcc 8400

gggaagctag agtaagtagt tcgccagtta atagtttgcg caacgttgtt gccattgcta 8460

caggcatcgt ggtgtcacgc tcgtcgtttg gtatggcttc attcagctcc ggttcccaac 8520

gatcaaggcg agttacatga tcccccatgt tgtgcaaaaa agcggttagc tccttcggtc 8580

ctccgatcgt tgtcagaagt aagttggccg cagtgttatc actcatggtt atggcagcac 8640

tgcataattc tcttactgtc atgccatccg taagatgctt ttctgtgact ggtgagtact 8700

caaccaagtc attctgagaa tagtgtatgc ggcgaccgag ttgctcttgc ccggcgtcaa 8760

tacgggataa taccgcgcca catagcagaa ctttaaaagt gctcatcatt ggaaaacgtt 8820

cttcggggcg aaaactctca aggatcttac cgctgttgag atccagttcg atgtaaccca 8880

ctcgtgcacc caactgatct tcagcatctt ttactttcac cagcgtttct gggtgagcaa 8940

aaacaggaag gcaaaatgcc gcaaaaaagg gaataagggc gacacggaaa tgttgaatac 9000

tcatactctt cctttttcaa tattattgaa gcatttatca gggttattgt ctcatgacca 9060

aaatccctta acgtgagttt tcgttccact gagcgtcaga ccccgtagaa aagatcaaag 9120

gatcttcttg agatcctttt tttctgcgcg taatctgctg cttgcaaaca aaaaaaccac 9180

cgctaccagc ggtggtttgt ttgccggatc aagagctacc aactcttttt ccgaaggtaa 9240

ctggcttcag cagagcgcag ataccaaata ctgttcttct agtgtagccg tagttaggcc 9300

accacttcaa gaactctgta gcaccgccta catacctcgc tctgctaatc ctgttaccag 9360

tggctgctgc cagtggcgat aagtcgtgtc ttaccgggtt ggactcaaga cgatagttac 9420

cggataaggc gcagcggtcg ggctgaacgg ggggttcgtg cacacagccc agcttggagc 9480

gaacgaccta caccgaactg agatacctac agcgtgagct atgagaaagc gccacgcttc 9540

ccgaagggag aaaggcggac aggtatccgg taagcggcag ggtcggaaca ggagagcgca 9600

cgagggagct tccaggggga aacgcctggt atctttatag tcctgtcggg tttcgccacc 9660

tctgacttga gcgtcgattt ttgtgatgct cgtcaggggg gcggagccta tggaaaaacg 9720

ccagcaacgc ggccttttta cggttcctgg ccttttgctg gccttttgct cacatgttct 9780

ttcctgcgtt atcccctgat tctgtggata accgtattac cgcctttgag tgagctgata 9840

ccgctcgccg cagccgaacg accgagcgca gcgagtcagt gagcgaggaa gcggaag 9897

<210> 23
<211> 10664

<212>  DNA
<213>  Artificial Sequence

<220>
<223>  Targeting vector

<400>  23
   agcgcccaat acgcaaaccg cctctccccg cgcgttggcc gattcattaa tgcagctggc      60
   acgacaggtt tcccgactgg aaagcgggca gtgagcgcaa cgcaattaat gtgagttagc     120
   tcactcatta ggcaccccag gctttacact ttatgcttcc ggctcgtatg ttgtgtggaa     180
   ttgtgagcgg ataacaattt cacacaggaa acagctatga ccatgattac gccaagctca     240
   gaattaaccc tcactaaagg gactagtcct gcaggtttaa acgaattcgc ccttgtcgac     300
   tagatgggag tttaagagtg aattgtggtc catggcatat gcctgtcagc atagggatgc     360
   tgtaggaatt gcaccttggc ttgagcccct ctcctcctga tgatgcttgc ccacagctct     420
   gatgtcctgg gaaggctcac tgcaccttgt tgtcagtgaa actgcaataa actatcaaga     480
   tttgatataa ttctgcccct ctgttctact tgtgagacca cacctggagt actgcatcca     540
   gttctggtac cactgcatcc agttctggta ccaccaacac aaaaaggaca tggagctgct     600
   ggagcagggc cagaggaggg ccatgaagat gaacagaggg ctgaagcacc tcccctatgg     660
   ggacaggctg agagacatgg ggcttttgag actggagaag agaaggctcc atgggacctt     720
   tcagtgtcct tccaatactt gaaagggtcc ttgaggaaag ctggggaggg acttcctata     780
   aggacatata gcaacaggac aaaaggaaag ggttttaaac tggaagaaag tagatttagt     840
   ctagatagta acaagaaatt ctttacagta aggatcacga gacactgaaa caggttgccc     900
   aaagcagctg tggatgcccc ctccctggaa gtgttccagg ccaggctgga tggggctttg     960
   agcaacctgt tctagtgaga ggtgaccctg cctacagcag gggagttgaa cctagatggt    1020
   cttaaatgtc ccttccaacc caaatcattc tatggcaatt ctatagagaa cacctatgct    1080
   gagagtagct acaagtatgg ctggttcctg gcagtgcccc tgtctctgtg atctatagca    1140
   gcaccaacag accctcaaac atcccttcac gattctccgg ttccaaatcc ggctccacac    1200
   acacattaac catcactggg gtccaagccg atgacaaggc tgtctattac tgtgggagca    1260
   cagacagcag ctatgttggt atcacagtga catggagcaa ttaaaaatga tgcataacct    1320
   tttgcacatc tgtaatgcta taaggtgaga gaaagtaagc aataatagca gagcagcaca    1380
   atcctaggct acagcaaatg agaacaagcc cagatgcagc cacggaaata aagaaaaaaa    1440
   aactgcttac ctttagaaag cctcatatag acaggatctc ccagcaagat acccacacct    1500
   ccactgccaa cccttaaatg agggctggga agggctggat cctggctcca cccctttcaa    1560
   tcactcaggt acattgcaca cacctgagct cccctggctg gccctgcctt cccaccaggt    1620
   gctcaatcac tgctttgggc tgtgactcag catctccact acagcatcac agggagcggt    1680
   tgtcggtctc tgctgttcct atgtgggtgc cccgggacac ctgaaggcat gagtggcagt 1740
   gagggctccc agctctatcc atggtgaaca gacattatgt ttatgcttta cagcagcatg 1800
   aatctggcag tggtgttatt gctgtgatgc tgctgtctat gctcccacag taatagatag 1860
   cctcgtcctc ggcttggacc ccagtgatgg ttaatgtgtt tgtggagccg gatccagaac 1920
   cggagaatcg tgaagggatg tccgagggtc tgttattgcc attatagatc acagtgacag 1980
   gggcactgcc aggcatagtc gctgtaaccc ccggagcagg tgatctcgac ggttcctccc 2040
   aggtttgctg acaccgaggc cggctgagtc agcgctgcct gcaccaggga acctggagag 2100
   ggagaggaga gaggggagaa aatggatctg agcaattgcc tgacacacac agccctcccc 2160
   agactgtggg atgaggccct gtgcccgcag tcacatgtgg aatatcaaga cacacacatc 2220
   tatgacaatc acaatctgat tatcaaccac tatggctggt accagcagaa ggcacctagc 2280
   agtgcccctg tcactctgat ctactatgat gatgagagac cctcgaacat cccttcacga 2340
   ttctccggtt ccaaatccgg ctccacacac acattaacca tcactggggt ccaagccgac 2400
   gacgaggctg tctattactg tgggaatgaa gacagcagcg gtactggtgc cccagtgaca 2460
   cagagcaatg gagatatgat acacagacct gacatctcag ggagcagctg gtcattatcc 2520
   tggtgacaca agtcccaggc atggcccagc actgtgctgc aggtggctat gccgggtgcc 2580
   ctgctctgcc ctcctgtaga gcctcaggac tgtgcccatc actcagggag gagatggtcc 2640
   cagcagccct gtccctgccc tgcactgcac ttagctcctg gaccccatca atttctgaga 2700
   actgtcattc tcggaatacc tcgatcgata acttcgtata atgtatacta tacgaagtta 2760
   ttttatttgt gaaatttgtg atgctattgc tttatttgta accattataa gctgcaataa 2820
   acaagttggg gtgggcgaag aactccagca tgagatcccc gcgctggagg atcatccagc 2880
   cggcgtcccg gaaaacgatt ccgaagccca acctttcata gaaggcggcg gtggaatcga 2940
   aatctcgtga tggcaggttg ggcgtcgctt ggtcggtcat ttcgaacccc agagtcccgc 3000
   tcagaagaac tcgtcaagaa ggcgatagaa ggcgatgcgc tgcgaatcgg gagcggcgat 3060
   accgtaaagc acgaggaagc ggtcagccca ttcgccgcca agctcttcag caatatcacg 3120
   ggtagccaac gctatgtcct gatagcggtc cgccacaccc agccggccac agtcgatgaa 3180
   tccagaaaag cggccatttt ccaccatgat attcggcaag caggcatcgc catgggtcac 3240
   gacgagatcc tcgccgtcgg gcatgcgcgc cttgagcctg gcgaacagtt cggctggcgc 3300
   gagcccctga tgctcttcgt ccagatcatc ctgatcgaca agaccggctt ccatccgagt 3360
   acgtgctcgc tcgatgcgat gtttcgcttg gtggtcgaat gggcaggtag ccggatcaag 3420
   cgtatgcagc cgccgcattg catcagccat gatggatact ttctcggcag gagcaaggtg 3480
   agatgacagg agatcctgcc ccggcacttc gcccaatagc agccagtccc ttcccgcttc 3540
   agtgacaacg tcgagcacag ctgcgcaagg aacgcccgtc gtggccagcc acgatagccg 3600
   cgctgcctcg tcctgcagtt cattcagggc accggacagg tcggtcttga caaaaagaac 3660
   cgggcgcccc tgcgctgaca gccggaacac ggcggcatca gagcagccga ttgtctgttg 3720
   tgcccagtca tagccgaata gcctctccac ccaagcggcc ggagaacctg cgtgcaatcc 3780
   atcttgttca atcatgcgaa acgatcctca tcctgtctct tgatcagatc cgaaaatgga 3840
   tatacaagct cccgggagct ttttgcaaaa gcctaggcct ccaaaaaagc ctcctcacta 3900
   cttctggaat agctcagagg cagaggcggc ctcggcctct gcataaataa aaaaaattag 3960
   tcagccatgg ggcggagaat gggcggaact gggcggagtt aggggcggga tgggcggagt 4020
   taggggcggg actatggttg ctgactaatt gagatgcatg ctttgcatac ttctgcctgc 4080
   tggggagcct ggggactttc cacacctggt tgctgactaa ttgagatgca tgctttgcat 4140
   acttctgcct gctggggagc ctggggactt tccacaccct aaataacttc gtataatgta 4200
   tactatacga agttatctcg tgtccgataa ctgtaattat cggaccgtca atcgattcgc 4260
   gacagtctgc cctgactcag cctgcctccg tgtctgggtc tcctggacag tcgatcacca 4320
   tctcctgcac tggaaccagc tcaagtgacg ttggtggtta taactatgtc tcctggtacc 4380
   aacaaaaccc aggcaaagcc cccaaactca tgatttatga tgtcagttca aatcggccct 4440
   cagggatttc taatcgcttc tctggctcca agtctggcaa cacggcctcc ctgaccatct 4500
   ctgggctcca ggctgacgac gaggctgatt attactgcac ctcatataca aacgacggta 4560
   gcaattctca ggtattcggc ggagggacca agctgaccgt cctaggtgcc tgacacacac 4620
   agccctcccc agactgtggg atgaggccct gtgcccgcag tcacatgtgg aatatcaaga 4680
   cacacacatc tatgacaatc accagtctgc cctgactcag cctgcctccg tgtctgggtc 4740
   tcctggacag tcgatcacca tctcctgcac tggaaccagc agtgacgttg gttataacta 4800
   tgtctcctgg taccaacaaa acccaggcaa agcccccaaa ctcatgattt atgatggtaa 4860
   tcggccctca gggatttcta atcgcttctc tggctccaag tctggcaaca cggcctccct 4920
   gaccatctct gggctccagg ctgacgacga ggctgattat tactgcacct catatacaaa 4980
   cgacagctct caggtattcg gcggagggac caagctgacc gtcctaggtg gcgcgccata 5040
   ttgcctccct gtgttgctgt tgcagggttg cttctgcctc atactggttt ctcccttctg 5100
   gaggtggcca aaagccgggc cctgtgcaac cctggtgcat aaatacctta tggcccctaa 5160
   gtagggcagg tgtgggacac gctctggcac ctggggtgtg tgcaagtgct caggaagacc 5220
   tgcaggcaca ggtggcagtg gggtctctgg gctgtgctcg agcagcagct gcctggggta 5280
   agggtagtac tctgtgcatg aacaatgctg cagggctcag ctctgctcag accacgaccc 5340
   tggcaccaac agagacctgc ctggctctgt ggtcatgtaa acctttacag gagctcaaga 5400
   caaggctgtt tattactgct ctggcaggaa agaagcactg gccatggtca tagagagttc 5460
   cagcaacagg aaagtgagag cccaagctgc tgaggtacca gggctcctca ggtgcctgct 5520
   gcagcagctt ggacacagtc gaggaacagc aattgtacct gtgtggtgga tcaggctgtg 5580
   ctgcctgtga acctattcta gcacatctgt cacctctgtg ccactcacag ggataccacc 5640
   cctgagaccc ctaccccatc agcctctgtg tgggatatgg tgttgggccc aagggctctg 5700
   ttgcacaggg agatagaggc ctggggagga gggaaagcat tgaggtggtg ttgataccag 5760
   ggatgtgagc ccaagcaaga gatcagcaga gcaaggagga agaattgcag gtgttggggc 5820
   tggggaaagc cccagatggc tggagctggt ggggccactg gagatctcct cctcccatcc 5880
   tgctccatgc tggggcagct gctgcaggct gaccaggccc tgcccgggca cgttgtgaag 5940
   gtcaccaagg atggagactt cagagctatc tgggacacgt gttttaacgt ttgatcagcc 6000
   ttgcagcaaa acagtgtttt gctctattga acagtgttca acaaaaacga tcagtggcct 6060
   ggaacacatc tcctacgaag aaaggttgcg agagctgggt ctggagaaga gaaggctctg 6120
   gggcaacctc cctgtggcct gtcagtgctt aaaggaggct caagagaaag atggagagag 6180
   actttataca cggagctgta gcaatatgac aaggagaaat ggtttaaaat ggaaagaggg 6240
   tagatttagg ttagctatca agaagaaatt cttcactcag aggtgatgaa gcactgtagc 6300
   aggttgccca gagaagtatt ggatgcttcc ttcctggcag ggcccaaggc cagactggat 6360
   gggttctggg cagcctgttc tggtgggaga tgtccctgcc tgtggcatgg cttggaaatg 6420
   ggtagccttt gaggtccctc tcagcccaaa tcattctatg ttcttgtggt tccctgcatt 6480
   tcagcctttg cctgttgact ctggtgccac tgttggacaa ttctactcag ctgcgtttca 6540
   tccctgcccc aaagcagtaa tcccatggat aagcaatgct ctgaggcctt caccaccctc 6600
   agggctcctg ctgaccctag cggcctccct ggcagcatga gccctttgtt gtccgcactg 6660
   ctgctgccga gggtcctgcc agccccccag ggcctttcct gcccaggcac tgccacctgc 6720
   taaaacccag cctgcccatg gcagggcatc gcatgtctga gggatggaac ggggcaaggg 6780
   cagtgctgca ggcagtggtt caagaaacgt ttagatgttg tactgaggga tgtggtttag 6840
   tgggaaatac tggtgatagg tggatggttg gactggagga tcttagaggt cttttccaac 6900
   cttggtgatt atgtgattct atgaagggag ccatagcctg ctcccatcat gccccgccag 6960
   tccggctctc ccgagcactt tggttccgca gcatgcggcc gcaagggcga attcgcggcc 7020
   gctaaattca attcgcccta tagtgagtcg tattacaatt cactggccgt cgttttacaa 7080
   cgtcgtgact gggaaaaccc tggcgttacc caacttaatc gccttgcagc acatccccct 7140
   ttcgccagct ggcgtaatag cgaagaggcc cgcaccgatc gcccttccca acagttgcgc 7200
   agcctatacg tacggcagtt taaggtttac acctataaaa gagagagccg ttatcgtctg 7260
   tttgtggatg tacagagtga tattattgac acgccggggc gacggatggt gatccccctg 7320
   gccagtgcac gtctgctgtc agataaagtc tcccgtgaac tttacccggt ggtgcatatc 7380
   ggggatgaaa gctggcgcat gatgaccacc gatatggcca gtgtgccggt ctccgttatc 7440
   ggggaagaag tggctgatct cagccaccgc gaaaatgaca tcaaaaacgc cattaacctg 7500
   atgttctggg gaatataaat gtcaggcatg agattatcaa aaaggatctt cacctagatc 7560
   cttttcacgt agaaagccag tccgcagaaa cggtgctgac cccggatgaa tgtcagctac 7620
   tgggctatct ggacaaggga aaacgcaagc gcaaagagaa agcaggtagc ttgcagtggg 7680
   cttacatggc gatagctaga ctgggcggtt ttatggacag caagcgaacc ggaattgcca 7740
   gctggggcgc cctctggtaa ggttgggaag ccctgcaaag taaactggat ggctttcttg 7800
   ccgccaagga tctgatggcg caggggatca agctctgatc aagagacagg atgaggatcg 7860
   tttcgcatga ttgaacaaga tggattgcac gcaggttctc cggccgcttg ggtggagagg 7920
   ctattcggct atgactgggc acaacagaca atcggctgct ctgatgccgc cgtgttccgg 7980
   ctgtcagcgc aggggcgccc ggttcttttt gtcaagaccg acctgtccgg tgccctgaat 8040
   gaactgcaag acgaggcagc gcggctatcg tggctggcca cgacgggcgt tccttgcgca 8100
   gctgtgctcg acgttgtcac tgaagcggga agggactggc tgctattggg cgaagtgccg 8160
   gggcaggatc tcctgtcatc tcaccttgct cctgccgaga aagtatccat catggctgat 8220
   gcaatgcggc ggctgcatac gcttgatccg gctacctgcc cattcgacca ccaagcgaaa 8280
   catcgcatcg agcgagcacg tactcggatg gaagccggtc ttgtcgatca ggatgatctg 8340
   gacgaagagc atcaggggct cgcgccagcc gaactgttcg ccaggctcaa ggcgagcatg 8400
   cccgacggcg aggatctcgt cgtgacccat ggcgatgcct gcttgccgaa tatcatggtg 8460
   gaaaatggcc gcttttctgg attcatcgac tgtggccggc tgggtgtggc ggaccgctat 8520
   caggacatag cgttggctac ccgtgatatt gctgaagagc ttggcggcga atgggctgac 8580
   cgcttcctcg tgctttacgg tatcgccgct cccgattcgc agcgcatcgc cttctatcgc 8640
   cttcttgacg agttcttctg aattattaac gcttacaatt tcctgatgcg gtattttctc 8700
   cttacgcatc tgtgcggtat ttcacaccgc atcaggtggc acttttcggg gaaatgtgcg 8760
   cggaacccct atttgtttat ttttctaaat acattcaaat atgtatccgc tcatgagatt 8820
   atcaaaaagg atcttcacct agatcctttt aaattaaaaa tgaagtttta aatcaatcta 8880
   aagtatatat gagtaaactt ggtctgacag ttaccaatgc ttaatcagtg aggcacctat 8940
   ctcagcgatc tgtctatttc gttcatccat agttgcctga ctccccgtcg tgtagataac 9000
   tacgatacgg gagggcttac catctggccc cagtgctgca atgataccgc gagacccacg 9060
   ctcaccggct ccagatttat cagcaataaa ccagccagcc ggaagggccg agcgcagaag 9120
   tggtcctgca actttatccg cctccatcca gtctattaat tgttgccggg aagctagagt 9180
   aagtagttcg ccagttaata gtttgcgcaa cgttgttgcc attgctacag gcatcgtggt 9240
   gtcacgctcg tcgtttggta tggcttcatt cagctccggt tcccaacgat caaggcgagt 9300
   tacatgatcc cccatgttgt gcaaaaaagc ggttagctcc ttcggtcctc cgatcgttgt 9360
   cagaagtaag ttggccgcag tgttatcact catggttatg gcagcactgc ataattctct 9420
   tactgtcatg ccatccgtaa gatgcttttc tgtgactggt gagtactcaa ccaagtcatt 9480
   ctgagaatag tgtatgcggc gaccgagttg ctcttgcccg gcgtcaatac gggataatac 9540
   cgcgccacat agcagaactt taaaagtgct catcattgga aaacgttctt cggggcgaaa 9600
   actctcaagg atcttaccgc tgttgagatc cagttcgatg taacccactc gtgcacccaa 9660
   ctgatcttca gcatctttta ctttcaccag cgtttctggg tgagcaaaaa caggaaggca 9720
   aaatgccgca aaaaagggaa taagggcgac acggaaatgt tgaatactca tactcttcct 9780
   ttttcaatat tattgaagca tttatcaggg ttattgtctc atgaccaaaa tcccttaacg 9840
   tgagttttcg ttccactgag cgtcagaccc cgtagaaaag atcaaaggat cttcttgaga 9900
   tccttttttt ctgcgcgtaa tctgctgctt gcaaacaaaa aaaccaccgc taccagcggt 9960
   ggtttgtttg ccggatcaag agctaccaac tctttttccg aaggtaactg gcttcagcag 10020
   agcgcagata ccaaatactg ttcttctagt gtagccgtag ttaggccacc acttcaagaa 10080
   ctctgtagca ccgcctacat acctcgctct gctaatcctg ttaccagtgg ctgctgccag 10140
   tggcgataag tcgtgtctta ccgggttgga ctcaagacga tagttaccgg ataaggcgca 10200
   gcggtcgggc tgaacggggg gttcgtgcac acagcccagc ttggagcgaa cgacctacac 10260
   cgaactgaga tacctacagc gtgagctatg agaaagcgcc acgcttcccg aagggagaaa 10320
   ggcggacagg tatccggtaa gcggcagggt cggaacagga gagcgcacga gggagcttcc 10380
   agggggaaac gcctggtatc tttatagtcc tgtcgggttt cgccacctct gacttgagcg 10440
   tcgatttttg tgatgctcgt caggggggcg gagcctatgg aaaaacgcca gcaacgcggc 10500
   ctttttacgg ttcctggcct tttgctggcc ttttgctcac atgttctttc ctgcgttatc 10560
   ccctgattct gtggataacc gtattaccgc ctttgagtga gctgataccg ctcgccgcag 10620
   ccgaacgacc gagcgcagcg agtcagtgag cgaggaagcg gaag 10664

<210> 24
<211> 24
<212> DNA
<213> Artificial Sequence

<220>
<223> Synthetic Primer

<400> 24
cgctttgtac gaacgttgtc acgt 24

<210> 25
<211> 25

<212>  DNA
<213>  Artificial Sequence

<220>
<223>  Synthetic Primer

<400>  25
tacctgaagg tctctttgtg ttttg                                              25


<210>  26
<211>  2794
<212>  DNA
<213>  Gallus gallus

<400>  26
gggtatcacg tggggcaaat cgagagctac cattggctat ggggaggaga aaatgaggcg        60

tgctacttcc tcgttcctgc agtgcgtgtc gaggtgccgt gttggtcgga tctcagtggc       120

agagcaggat ggtgagcggt ggccttgggg ccgctccctc actctgctgg gctggtgcgg       180

tggctccggg accggacttt cagtggtgtt tggtgtggtg ctcactgtcc tttggtgtca       240

ctgtgtggtg aattccctca gctgggcggt gagagcccat gctgtgtcct ggtaggagta       300

gtctgggctg cagtgcgggc tctttgtgcg tgttgggggc ggttccctgg aagctaattc       360

tggaagaaat cccggctgcc tctgtaacag ttgttggcga tgtcaaacac cctggagctg       420

ctgactggct ctgtaccatt gagtgatgca gtttaaacct ctctctttcc tcctgctgtg       480

tgtgaagctg gtgttagtgc taggagacct gcacatccct caccgatgca gtggtttacc       540

agcaaagttc aagagcctcc tggttccagg gaagattcag catattttgt gtacaggaaa       600

cctctgcacc aaggagagct atgactacct caggactttg gctggagatg ttcacgttgt       660

taagggggac tctgaggtaa tgttcttgct gctgtgaatt cctcagtcct ccatcttaca       720

agccgtagtt gtcccactga aagctgggaa acccttcggt gtttgcaatt cctgagcgct       780

aatgtgtggg ggaaggatgt aggaacaaat aaggctcaag agacaacaac ttcctaggct       840

cctcttattg cccttgtggt aagggaggac ttggtgtgta gtggtaatac atgcactcaa       900

cttattaagt tctcatagct gctgtgaagc tgctgcagtc cctcagatcc ctgcaggaag       960

aggggactga ctgactgttg aatagccttg aagctcctgc tctgcatgtg gttccggtca      1020

ggctcaagat cccagttctt agctcttcag ttgctcttct gggggtatag tactagcatc      1080

tgggtcactg cagatggaca gagactgtgc ttgggtagca aaaggcagac ttacgttgtg      1140

aaaccttcat tgtgttggtg aggttctttg atttctacct tgtgtatctc tcttcctcag      1200

agtctgaatt atcctgaaca gaaggttgta actgttgggc agttcagaat tgggttgatt      1260

catggccatc aagtcattcc ctggggtgat gtggcaagct tggcattgct gcagaggcag      1320

ctggatgtag acattcttat ctcaggacat acacacagat ttgaagcatt tgagcatgaa      1380

aataagttct acatcaaccc aggatcagct acaggagcct acactggttt acaaacgtga      1440

gtacaatgag ccttgtgttt gtctttaaga cagatgtctg tccttaagag tgcagcccat 1500

gtgttgaaga gcactggtga gatgcagtgc catgcaggcc atctctccct ttcccagaga 1560

acacggccta cctctctctg ttcataaggt cttggttaac ttatgagttg gccagcacag 1620

cctggcactg atgtgtcatg atcataaccc tgagcccacg ctctgctatg ctgttgcttc 1680

ttagagttgc attgccttga ggtgtattcc agaagctgtg tgctgagctc ttgttgggaa 1740

agaggccctc agggcactgc caacatctga ggcttgcagg agctggaaca ttcagccctg 1800

agggctgctc tggccaactg cagctctgag tctgcagtca gtgcctctga gctgtgatgc 1860

ttctcagata actgcagccc tttgcaatgt aatgttttat aatatatttc cataggacat 1920

cttggtaaga ggtgtaagct aatcagggtg attgacaaag tatgttttct ttccaggaac 1980

gtcattcctt catttgtact gatggatatc caggcttcca cagttgtgac ttatgtatac 2040

cagctaattg aggacgatgt gaaagtagaa agaattgagt tcaagaaata ctgacacgtg 2100

tttgaatctg cctgctgact tctcactgcc tctcattctt tctctcctag ttaaagctga 2160

atccggccac gtgggggtgc tgctgcagcg gcgtgtaact gtgagcttgg gaagttctca 2220

actagggctt agagcaggag aaactgatta acagaaattg ccttattaaa tgtatatatt 2280

tcctgtgtct aagaacgagg tgctgttgtt cctgacttgc ggagctgcag ctagtggtag 2340

cttggaattt aacctctcct gtaaaatgag catagaataa aacagtattt tcacatttct 2400

ggatcctgtt aattgtttgt taattaagag gacaggaatt cagtgtcagg gggctgctct 2460

ttgcttccca atgtgtacca ctgaagcctc tctggattta tcagagatac tcaatggact 2520

cttaacagca ctgtggcacc tgacctcctg ctgccttcag cctcccttcc tggctggtgt 2580

gcttatgttg aggagctgtg ggaggctggt gcagttctgt gggatgaggt aaggccagct 2640

gaatagcctg gggctggtc aggggcttta gtgagaggag caggctggg gaatgttgtg 2700

aacagcaggt ggtgccactg ctattctgag gaaggaggca gcttctgcaa atgctttgca 2760

atgcaggatg acaaagtgct tctgctagcc ctag 2794

<210> 27
<211> 10304
<212> DNA
<213> Artificial Sequence

<220>
<223> Targeting vector

<400> 27
agcgcccaat acgcaaaccg cctctccccg cgcgttggcc gattcattaa tgcagctggc 60

acgacaggtt tcccgactgg aaagcgggca gtgagcgcaa cgcaattaat gtgagttagc 120

tcactcatta ggcaccccag gctttacact ttatgcttcc ggctcgtatg ttgtgtggaa 180

ttgtgagcgg ataacaattt cacacaggaa acagctatga ccatgattac gccaagctca 240

gaattaaccc tcactaaagg gactagtcct gcaggtttaa acgaattcgc ccttgtcgac 300

gggtatcacg tggggcaaat cgagagctac cattggctat ggggaggaga aaatgaggcg 360

tgctacttcc tcgttcctgc agtgcgtgtc gaggtgccgt gttggtcgga tctcagtggc 420

agagcaggat ggtgagcggt ggccttgggg ccgctccctc actctgctgg gctggtgcgg 480

tggctccggg accggacttt cagtggtgtt tggtgtggtg ctcactgtcc tttggtgtca 540

ctgtgtggtg aattccctca gctgggcggt gagagcccat gctgtgtcct ggtaggagta 600

gtctgggctg cagtgcgggc tctttgtgcg tgttgggggc ggttccctgg aagctaattc 660

tggaagaaat cccggctgcc tctgtaacag ttgttggcga tgtcaaacac cctggagctg 720

ctgactggct ctgtaccatt gagtgatgca gtttaaacct ctctctttcc tcctgctgtg 780

tgtgaagctg gtgttagtgc taggagacct gcacatccct caccgatgca gtggtttacc 840

agcaaagttc aagagcctcc tggttccagg gaagattcag catattttgt gtacaggaaa 900

cctctgcacc aaggagagct atgactacct caggactttg gctggagatg ttcacgttgt 960

taagggggac tctgaggtaa tgttcttgct gctgtgaatt cctcagtcct ccatcttaca 1020

agccgtagtt gtcccactga aagctgggaa acccttcggt gtttgcaatt cctgagcgct 1080

aatgtgtggg ggaaggatgt aggaacaaat aaggctcaag agacaacaac ttcctaggct 1140

cctcttattg cccttgtggt aagggaggac ttggtgtgta gtggtaatac atgcactcaa 1200

cttattaagt tctcatagct gctgtgaagc tgctgcagtc cctcagatcc ctgcaggaag 1260

aggggactga ctgactgttg aatagccttg aagctcctgc tctgcatgtg gttccggtca 1320

ggctcaagat cccagttctt agctcttcag ttgctcttct gggggtatag tactagcatc 1380

tgggtcactg cagatggaca gagactgtgc ttgggtagca aaaggcagac ttacgttgtg 1440

aaaccttcat tgtgttggtg aggttctttg atttctacct tgtgtatctc tcttcctcag 1500

agtctgaatt atcctgaaca gaaggttgta actgttgggc agttcagaat tgggttgatt 1560

catggccatc aagtcattcc ctggggtgat gtggcaagct tggcattgct gcagaggcag 1620

ctggatgtag acattcttat ctcaggacat acacacagat ttgaagcatt tgagcatgaa 1680

aataagttct acatcaaccc aggatcagct acaggagcct acactggttt acaaacgtga 1740

gtacaatgag ccttgtgttt gtctttaaga cagatgtctg tccttaagag tgcagcccat 1800

gtgttgaaga gcactggtga gatgcagtgc catgcaggcc atctctccct ttcccagaga 1860

acacggccta cctctctctg ttcataaggt cttggttaac ttatgagttg gccagcacag 1920

cctggcactg atgtgtcatg atcataaccc tgagcccacg ctctgctatg ctgttgcttc 1980

ttagagttgc attgccttga ggtgtattcc agaagctgtg tgctgagctc ttgttgggaa 2040

agaggccctc agggcactgc caacatctga ggcttgcagg agctggaaca ttcagccctg 2100

agggctgctc tggccaactg cagctctgag tctgcagtca gtgcctctga gctgtgatgc 2160

ttctcagata actgcagccc tttgcaatgt aatgttttat aatatatttc cataggacat 2220

cttggtaaga ggtgtaagct aatcagggtg attgacaaag tatgttttct ttccaggaac 2280

gtcattcctt catttgtact gatggatatc caggcttcca cagttgtgac ttatgtatac 2340

cagctaattg aggacgatgt gaaagtagaa agaattgagt tcaagaaata ctgacacgtg 2400

tttgaatctg cctgctgact tctcactgcc tctcattctt tctctcctag ttaaagctga 2460

atccggccac gtgggggtgc tgctgcagcg gcgtgtaact gtgagcttgg gaagttctca 2520

actagggctt agagcaggag aaactgatta acagaaattg ccttattaaa tgtatatatt 2580

tcctgtgtct aagaacgagg tgctgttgtt cctgacttgc ggagctgcag ctagtggtag 2640

cttggaattt aacctctcct gtaaaatgag catagaataa aacagtattt tcacatttct 2700

ggatcctgtt aattgtttgt taattaagag gacaggaatt cagtgtcagg gggctgctct 2760

ttgcttccca atgtgtacca ctgaagcctc tctggattta tcagagatac tcaatggact 2820

cttaacagca ctgtggcacc tgacctcctg ctgccttcag cctcccttcc tggctggtgt 2880

gcttatgttg aggagctgtg ggaggctggt gcagttctgt gggatgaggt aaggccagct 2940

gaatagcctg gggctggtc aggggcttta gtgagaggag caggctggg gaatgttgtg 3000

aacagcaggt ggtgccactg ctattctgag gaaggaggca gcttctgcaa atgctttgca 3060

atgcaggatg acaaagtgct tctgctagcc ctagtcaatt tctgagaact gtcattctcg 3120

gaatacctcg atcgataact tcgtataatg tatactatac gaagttattt tatttgtgaa 3180

atttgtgatg ctattgcttt atttgtaacc attataagct gcaataaaca agttggggtg 3240

ggcgaagaac tccagcatga gatccccgcg ctggaggatc atccagccgg cgtcccggaa 3300

aacgattccg aagcccaacc tttcatagaa ggcggcggtg gaatcgaaat ctcgtgatgg 3360

caggttgggc gtcgcttggt cggtcatttc gaaccccaga gtcccgctca gaagaactcg 3420

tcaagaaggc gatagaaggc gatgcgctgc gaatcgggag cggcgatacc gtaaagcacg 3480

aggaagcggt cagcccattc gccgccaagc tcttcagcaa tatcacgggt agccaacgct 3540

atgtcctgat agcggtccgc cacacccagc cggccacagt cgatgaatcc agaaaagcgg 3600

ccattttcca ccatgatatt cggcaagcag gcatcgccat gggtcacgac gagatcctcg 3660

ccgtcgggca tgcgcgcctt gagcctggcg aacagttcgg ctggcgcgag cccctgatgc 3720

tcttcgtcca gatcatcctg atcgacaaga ccggcttcca tccgagtacg tgctcgctcg 3780

atgcgatgtt tcgcttggtg gtcgaatggg caggtagccg gatcaagcgt atgcagccgc 3840

cgcattgcat cagccatgat ggatactttc tcggcaggag caaggtgaga tgacaggaga 3900

tcctgccccg gcacttcgcc caatagcagc cagtcccttc ccgcttcagt gacaacgtcg 3960

agcacagctg cgcaaggaac gcccgtcgtg gccagccacg atagccgcgc tgcctcgtcc 4020

tgcagttcat tcagggcacc ggacaggtcg gtcttgacaa aaagaaccgg gcgcccctgc 4080

gctgacagcc ggaacacggc ggcatcagag cagccgattg tctgttgtgc ccagtcatag 4140

ccgaatagcc tctccaccca agcggccgga gaacctgcgt gcaatccatc ttgttcaatc 4200

atgcgaaacg atcctcatcc tgtctcttga tcagatccga aaatggatat acaagctccc 4260

gggagctttt tgcaaaagcc taggcctcca aaaaagcctc ctcactactt ctggaatagc 4320

tcagaggcag aggcggcctc ggcctctgca taaataaaaa aaattagtca gccatggggc 4380

ggagaatggg cggaactggg cggagttagg ggcgggatgg gcggagttag gggcgggact 4440

atggttgctg actaattgag atgcatgctt tgcatacttc tgcctgctgg ggagcctggg 4500

gactttccac acctggttgc tgactaattg agatgcatgc tttgcatact tctgcctgct 4560

ggggagcctg gggactttcc acaccctaaa taacttcgta taatgtatac tatacgaagt 4620

tatctcgtgt ccgataactg taattatcgg accgtcaatc gattcgcgag gcgcgccata 4680

ttgcctccct gtgttgctgt tgcagggttg cttctgcctc atactggttt ctcccttctg 4740

gaggtggcca aaagccgggc cctgtgcaac cctggtgcat aaatacctta tggcccctaa 4800

gtagggcagg tgtgggacac gctctggcac ctggggtgtg tgcaagtgct caggaagacc 4860

tgcaggcaca ggtggcagtg ggggtctctg gctgtgctcg agcagcagct gcctggggta 4920

agggtagtac tctgtgcatg aacaatgctg cagggctcag ctctgctcag accacgaccc 4980

tggcaccaac agagacctgc ctggctctgt ggtcatgtaa acctttacag gagctcaaga 5040

caaggctgtt tattactgct ctggcaggaa agaagcactg gccatggtca tagagagttc 5100

cagcaacagg aaagtgagag cccaagctgc tgaggtacca gggctcctca ggtgcctgct 5160

gcagcagctt ggacacagtc gaggaacagc aattgtacct gtgtggtgga tcaggctgtg 5220

ctgcctgtga acctattcta gcacatctgt cacctctgtg ccactcacag ggataccacc 5280

cctgagaccc ctaccccatc agcctctgtg tgggatatgg tgttgggccc aagggctctg 5340

ttgcacaggg agatagaggc ctggggagga gggaaagcat tgaggtggtg ttgataccag 5400

ggatgtgagc ccaagcaaga gatcagcaga gcaaggagga agaattgcag gtgttggggc 5460

tggggaaagc cccagatggc tggagctggt ggggccactg gagatctcct cctcccatcc 5520

tgctccatgc tggggcagct gctgcaggct gaccagggcc tgcccgggca cgttgtgaag 5580

gtcaccaagg atggagactt cagagctatc tgggacacgt gttttaacgt ttgatcagcc 5640

ttgcagcaaa acagtgtttt gctctattga acagtgttca acaaaaacga tcagtggcct 5700

ggaacacatc tcctacgaag aaaggttgcg agagctgggt ctggagaaga gaaggctctg 5760

gggcaacctc cctgtggcct gtcagtgctt aaaggaggct caagagaaag atggagagag 5820

actttataca cggagctgta gcaatatgac aaggagaaat ggtttaaaat ggaaagaggg 5880

tagatttagg ttagctatca agaagaaatt cttcactcag aggtgatgaa gcactgtagc 5940
aggttgccca gagaagtatt ggatgcttcc ttcctggcag ggcccaaggc cagactggat 6000
gggttctggg cagcctgttc tggtgggaga tgtccctgcc tgtggcatgg cttggaaatg 6060
ggtagccttt gaggtccctc tcagcccaaa tcattctatg ttcttgtggt tccctgcatt 6120
tcagcctttg cctgttgact ctggtgccac tgttggacaa ttctactcag ctgcgtttca 6180
tccctgcccc aaagcagtaa tcccatggat aagcaatgct ctgaggcctt caccaccctc 6240
agggctcctg ctgaccctag cggcctccct ggcagcatga gccctttgtt gtccgcactg 6300
ctgctgccga gggtcctgcc agccccccag ggccttttct gcccaggcac tgccacctgc 6360
taaaacccag cctgcccatg gcagggcatc gcatgtctga gggatggaac ggggcaaggg 6420
cagtgctgca ggcagtggtt caagaaacgt ttagatgttg tactgaggga tgtggtttag 6480
tgggaaatac tggtgatagg tggatggttg gactggagga tcttagaggt cttttccaac 6540
cttggtgatt atgtgattct atgaagggag ccatagcctg ctcccatcat gccccgccag 6600
tccggctctc ccgagcactt tggttccgca gcatgcggcc gcaagggcga attcgcggcc 6660
gctaaattca attcgcccta tagtgagtcg tattacaatt cactggccgt cgttttacaa 6720
cgtcgtgact gggaaaaccc tggcgttacc caacttaatc gccttgcagc acatccccct 6780
ttcgccagct ggcgtaatag cgaagaggcc cgcaccgatc gcccttccca acagttgcgc 6840
agcctatacg tacggcagtt taaggtttac acctataaaa gagagagccg ttatcgtctg 6900
tttgtggatg tacagagtga tattattgac acgccggggc gacggatggt gatccccctg 6960
gccagtgcac gtctgctgtc agataaagtc tcccgtgaac tttacccggt ggtgcatatc 7020
ggggatgaaa gctggcgcat gatgaccacc gatatggcca gtgtgccggt ctccgttatc 7080
ggggaagaag tggctgatct cagccaccgc gaaaatgaca tcaaaaacgc cattaacctg 7140
atgttctggg gaatataaat gtcaggcatg agattatcaa aaaggatctt cacctagatc 7200
cttttcacgt agaaagccag tccgcagaaa cggtgctgac cccggatgaa tgtcagctac 7260
tgggctatct ggacaaggga aaacgcaagc gcaaagagaa agcaggtagc ttgcagtggg 7320
cttacatggc gatagctaga ctgggcggtt ttatggacag caagcgaacc ggaattgcca 7380
gctggggcgc cctctggtaa ggttgggaag ccctgcaaag taaactggat ggctttcttg 7440
ccgccaagga tctgatggcg caggggatca agctctgatc aagagacagg atgaggatcg 7500
tttcgcatga ttgaacaaga tggattgcac gcaggttctc cggccgcttg ggtggagagg 7560
ctattcggct atgactgggc acaacagaca atcggctgct ctgatgccgc cgtgttccgg 7620
ctgtcagcgc aggggcgccc ggttcttttt gtcaagaccg acctgtccgg tgccctgaat 7680
gaactgcaag acgaggcagc gcggctatcg tggctggcca cgacgggcgt tccttgcgca 7740

gctgtgctcg acgttgtcac tgaagcggga agggactggc tgctattggg cgaagtgccg 7800

gggcaggatc tcctgtcatc tcaccttgct cctgccgaga aagtatccat catggctgat 7860

gcaatgcggc ggctgcatac gcttgatccg gctacctgcc cattcgacca ccaagcgaaa 7920

catcgcatcg agcgagcacg tactcggatg gaagccggtc ttgtcgatca ggatgatctg 7980

gacgaagagc atcaggggct cgcgccagcc gaactgttcg ccaggctcaa ggcgagcatg 8040

cccgacggcg aggatctcgt cgtgacccat ggcgatgcct gcttgccgaa tatcatggtg 8100

gaaaatggcc gcttttctgg attcatcgac tgtggccggc tgggtgtggc ggaccgctat 8160

caggacatag cgttggctac ccgtgatatt gctgaagagc ttggcggcga atgggctgac 8220

cgcttcctcg tgctttacgg tatcgccgct cccgattcgc agcgcatcgc cttctatcgc 8280

cttcttgacg agttcttctg aattattaac gcttacaatt tcctgatgcg gtattttctc 8340

cttacgcatc tgtgcggtat ttcacaccgc atcaggtggc acttttcggg gaaatgtgcg 8400

cggaacccct atttgtttat ttttctaaat acattcaaat atgtatccgc tcatgagatt 8460

atcaaaaagg atcttcacct agatcctttt aaattaaaaa tgaagtttta aatcaatcta 8520

aagtatatat gagtaaactt ggtctgacag ttaccaatgc ttaatcagtg aggcacctat 8580

ctcagcgatc tgtctatttc gttcatccat agttgcctga ctccccgtcg tgtagataac 8640

tacgatacgg gagggcttac catctggccc cagtgctgca atgataccgc gagacccacg 8700

ctcaccggct ccagatttat cagcaataaa ccagccagcc ggaagggccg agcgcagaag 8760

tggtcctgca actttatccg cctccatcca gtctattaat tgttgccggg aagctagagt 8820

aagtagttcg ccagttaata gtttgcgcaa cgttgttgcc attgctacag gcatcgtggt 8880

gtcacgctcg tcgtttggta tggcttcatt cagctccggt tcccaacgat caaggcgagt 8940

tacatgatcc cccatgttgt gcaaaaaagc ggttagctcc ttcggtcctc cgatcgttgt 9000

cagaagtaag ttggccgcag tgttatcact catggttatg gcagcactgc ataattctct 9060

tactgtcatg ccatccgtaa gatgcttttc tgtgactggt gagtactcaa ccaagtcatt 9120

ctgagaatag tgtatgcggc gaccgagttg ctcttgcccg gcgtcaatac gggataatac 9180

cgcgccacat agcagaactt taaaagtgct catcattgga aaacgttctt cggggcgaaa 9240

actctcaagg atcttaccgc tgttgagatc cagttcgatg taacccactc gtgcacccaa 9300

ctgatcttca gcatctttta ctttcaccag cgtttctggg tgagcaaaaa caggaaggca 9360

aaatgccgca aaaaagggaa taagggcgac acggaaatgt tgaatactca tactcttcct 9420

ttttcaatat tattgaagca tttatcaggg ttattgtctc atgaccaaaa tcccttaacg 9480

tgagttttcg ttccactgag cgtcagaccc cgtagaaaag atcaaaggat cttcttgaga 9540

tccttttttt ctgcgcgtaa tctgctgctt gcaaacaaaa aaaccaccgc taccagcggt 9600

ggtttgtttg ccggatcaag agctaccaac tctttttccg aaggtaactg gcttcagcag 9660

agcgcagata ccaaatactg ttcttctagt gtagccgtag ttaggccacc acttcaagaa 9720

ctctgtagca ccgcctacat acctcgctct gctaatcctg ttaccagtgg ctgctgccag 9780

tggcgataag tcgtgtctta ccgggttgga ctcaagacga tagttaccgg ataaggcgca 9840

gcggtcgggc tgaacggggg gttcgtgcac acagcccagc ttggagcgaa cgacctacac 9900

cgaactgaga tacctacagc gtgagctatg agaaagcgcc acgcttcccg aagggagaaa 9960

ggcggacagg tatccggtaa gcggcagggt cggaacagga gagcgcacga gggagcttcc 10020

agggggaaac gcctggtatc tttatagtcc tgtcgggttt cgccacctct gacttgagcg 10080

tcgatttttg tgatgctcgt caggggggcg gagcctatgg aaaaacgcca gcaacgcggc 10140

ctttttacgg ttcctggcct tttgctggcc ttttgctcac atgttctttc ctgcgttatc 10200

ccctgattct gtggataacc gtattaccgc ctttgagtga gctgataccg ctcgccgcag 10260

ccgaacgacc gagcgcagcg agtcagtgag cgaggaagcg gaag 10304

<210> 28
<211> 11071
<212> DNA
<213> Artificial Sequence

<220>
<223> Targeting vector

<400> 28
agcgcccaat acgcaaaccg cctctccccg cgcgttggcc gattcattaa tgcagctggc 60

acgacaggtt tcccgactgg aaagcgggca gtgagcgcaa cgcaattaat gtgagttagc 120

tcactcatta ggcaccccag gctttacact ttatgcttcc ggctcgtatg ttgtgtggaa 180

ttgtgagcgg ataacaattt cacacaggaa acagctatga ccatgattac gccaagctca 240

gaattaaccc tcactaaagg gactagtcct gcaggtttaa acgaattcgc ccttgtcgac 300

gggtatcacg tggggcaaat cgagagctac cattggctat ggggaggaga aaatgaggcg 360

tgctacttcc tcgttcctgc agtgcgtgtc gaggtgccgt gttggtcgga tctcagtggc 420

agagcaggat ggtgagcggt ggccttgggg ccgctccctc actctgctgg gctggtgcgg 480

tggctccggg accggacttt cagtggtgtt tggtgtggtg ctcactgtcc tttggtgtca 540

ctgtgtggtg aattccctca gctgggcggt gagagcccat gctgtgtcct ggtaggagta 600

gtctgggctg cagtgcgggc tctttgtgcg tgttgggggc ggttccctgg aagctaattc 660

tggaagaaat cccggctgcc tctgtaacag ttgttggcga tgtcaaacac cctggagctg 720

ctgactggct ctgtaccatt gagtgatgca gtttaaacct ctctctttcc tcctgctgtg 780

tgtgaagctg gtgttagtgc taggagacct gcacatccct caccgatgca gtggtttacc 840

agcaaagttc aagagcctcc tggttccagg gaagattcag catattttgt gtacaggaaa 900

cctctgcacc aaggagagct atgactacct caggactttg gctggagatg ttcacgttgt 960
taagggggac tctgaggtaa tgttcttgct gctgtgaatt cctcagtcct ccatcttaca 1020
agccgtagtt gtcccactga aagctgggaa acccttcggt gtttgcaatt cctgagcgct 1080
aatgtgtggg ggaaggatgt aggaacaaat aaggctcaag agacaacaac ttcctaggct 1140
cctcttattg cccttgtggt aagggaggac ttggtgtgta gtggtaatac atgcactcaa 1200
cttattaagt tctcatagct gctgtgaagc tgctgcagtc cctcagatcc ctgcaggaag 1260
aggggactga ctgactgttg aatagccttg aagctcctgc tctgcatgtg gttccggtca 1320
ggctcaagat cccagttctt agctcttcag ttgctcttct gggggtatag tactagcatc 1380
tgggtcactg cagatggaca gagactgtgc ttgggtagca aaaggcagac ttacgttgtg 1440
aaaccttcat tgtgttggtg aggttctttg atttctacct tgtgtatctc tcttcctcag 1500
agtctgaatt atcctgaaca gaaggttgta actgttgggc agttcagaat tgggttgatt 1560
catggccatc aagtcattcc ctggggtgat gtggcaagct tggcattgct gcagaggcag 1620
ctggatgtag acattcttat ctcaggacat acacacagat ttgaagcatt tgagcatgaa 1680
aataagttct acatcaaccc aggatcagct acaggagcct acactggttt acaaacgtga 1740
gtacaatgag ccttgtgttt gtctttaaga cagatgtctg tccttaagag tgcagcccat 1800
gtgttgaaga gcactggtga gatgcagtgc catgcaggcc atctctccct ttcccagaga 1860
acacggccta cctctctctg ttcataaggt cttggttaac ttatgagttg gccagcacag 1920
cctggcactg atgtgtcatg atcataaccc tgagcccacg ctctgctatg ctgttgcttc 1980
ttagagttgc attgccttga ggtgtattcc agaagctgtg tgctgagctc ttgttgggaa 2040
agaggccctc agggcactgc caacatctga ggcttgcagg agctggaaca ttcagccctg 2100
agggctgctc tggccaactg cagctctgag tctgcagtca gtgcctctga gctgtgatgc 2160
ttctcagata actgcagccc tttgcaatgt aatgttttat aatatatttc cataggacat 2220
cttggtaaga ggtgtaagct aatcagggtg attgacaaag tatgttttct ttccaggaac 2280
gtcattcctt catttgtact gatggatatc caggcttcca cagttgtgac ttatgtatac 2340
cagctaattg aggacgatgt gaaagtagaa agaattgagt tcaagaaata ctgacacgtg 2400
tttgaatctg cctgctgact tctcactgcc tctcattctt tctctcctag ttaaagctga 2460
atccggccac gtgggggtgc tgctgcagcg gcgtgtaact gtgagcttgg gaagttctca 2520
actagggctt agagcaggag aaactgatta acagaaattg ccttattaaa tgtatatatt 2580
tcctgtgtct aagaacgagg tgctgttgtt cctgacttgc ggagctgcag ctagtggtag 2640
cttggaattt aacctctcct gtaaaatgag catagaataa aacagtattt tcacatttct 2700
ggatcctgtt aattgtttgt taattaagag gacaggaatt cagtgtcagg gggctgctct 2760
ttgcttccca atgtgtacca ctgaagcctc tctggattta tcagagatac tcaatggact 2820

cttaacagca ctgtggcacc tgacctcctg ctgccttcag cctcccttcc tggctggtgt 2880

gcttatgttg aggagctgtg ggaggctggt gcagttctgt gggatgaggt aaggccagct 2940

gaatagcctg gggctggtc aggggcttta gtgagaggag caggctggg gaatgttgtg 3000

aacagcaggt ggtgccactg ctattctgag gaaggaggca gcttctgcaa atgctttgca 3060

atgcaggatg acaaagtgct tctgctagcc ctagtcaatt tctgagaact gtcattctcg 3120

gaatacctcg atcgataact tcgtataatg tatactatac gaagttattt tatttgtgaa 3180

atttgtgatg ctattgcttt atttgtaacc attataagct gcaataaaca agttggggtg 3240

ggcgaagaac tccagcatga gatccccgcg ctggaggatc atccagccgg cgtcccggaa 3300

aacgattccg aagcccaacc tttcatagaa ggcggcggtg gaatcgaaat ctcgtgatgg 3360

caggttgggc gtcgcttggt cggtcatttc gaaccccaga gtcccgctca gaagaactcg 3420

tcaagaaggc gatagaaggc gatgcgctgc gaatcgggag cggcgatacc gtaaagcacg 3480

aggaagcggt cagcccattc gccgccaagc tcttcagcaa tatcacgggt agccaacgct 3540

atgtcctgat agcggtccgc cacacccagc cggccacagt cgatgaatcc agaaaagcgg 3600

ccattttcca ccatgatatt cggcaagcag gcatcgccat gggtcacgac gagatcctcg 3660

ccgtcgggca tgcgcgcctt gagcctggcg aacagttcgg ctggcgcgag cccctgatgc 3720

tcttcgtcca gatcatcctg atcgacaaga ccggcttcca tccgagtacg tgctcgctcg 3780

atgcgatgtt tcgcttggtg gtcgaatggg caggtagccg gatcaagcgt atgcagccgc 3840

cgcattgcat cagccatgat ggatactttc tcggcaggag caaggtgaga tgacaggaga 3900

tcctgccccg gcacttcgcc caatagcagc cagtcccttc ccgcttcagt gacaacgtcg 3960

agcacagctg cgcaaggaac gcccgtcgtg gccagccacg atagccgcgc tgcctcgtcc 4020

tgcagttcat tcagggcacc ggacaggtcg gtcttgacaa aaagaaccgg gcgcccctgc 4080

gctgacagcc ggaacacggc ggcatcagag cagccgattg tctgttgtgc ccagtcatag 4140

ccgaatagcc tctccaccca agcggccgga gaacctgcgt gcaatccatc ttgttcaatc 4200

atgcgaaacg atcctcatcc tgtctcttga tcagatccga aaatggatat acaagctccc 4260

gggagctttt tgcaaaagcc taggcctcca aaaaagcctc ctcactactt ctggaatagc 4320

tcagaggcag aggcggcctc ggcctctgca taaataaaaa aaattagtca gccatggggc 4380

ggagaatggg cggaactggg cggagttagg ggcgggatgg gcggagttag gggcgggact 4440

atggttgctg actaattgag atgcatgctt tgcatacttc tgcctgctgg ggagcctggg 4500

gactttccac acctggttgc tgactaattg agatgcatgc tttgcatact tctgcctgct 4560

ggggagcctg gggactttcc acaccctaaa taacttcgta taatgtatac tatacgaagt 4620

tatctcgtgt ccgataactg taattatcgg accgtcaatc gattcgcgac agtctgccct 4680

gactcagcct gcctccgtgt ctgggtctcc tggacagtcg atcaccatct cctgcactgg 4740
aaccagctca agtgacgttg gtggttataa ctatgtctcc tggtaccaac aaaacccagg 4800
caaagccccc aaactcatga tttatgatgt cagttcaaat cggccctcag ggatttctaa 4860
tcgcttctct ggctccaagt ctggcaacac ggcctccctg accatctctg ggctccaggc 4920
tgacgacgag gctgattatt actgcacctc atatacaaac gacggtagca attctcaggt 4980
attcggcgga gggaccaagc tgaccgtcct aggtgcctga cacacacagc cctccccaga 5040
ctgtgggatg aggccctgtg cccgcagtca catgtggaat atcaagacac acacatctat 5100
gacaatcacc agtctgccct gactcagcct gcctccgtgt ctgggtctcc tggacagtcg 5160
atcaccatct cctgcactgg aaccagcagt gacgttggtt ataactatgt ctcctggtac 5220
caacaaaacc caggcaaagc ccccaaactc atgatttatg atggtaatcg gccctcaggg 5280
atttctaatc gcttctctgg ctccaagtct ggcaacacgg cctccctgac catctctggg 5340
ctccaggctg acgacgaggc tgattattac tgcacctcat atacaaacga cagctctcag 5400
gtattcggcg gagggaccaa gctgaccgtc ctaggtggcg cgccatattg cctccctgtg 5460
ttgctgttgc agggttgctt ctgcctcata ctggtttctc ccttctggag gtggccaaaa 5520
gccgggccct gtgcaaccct ggtgcataaa taccttatgg cccctaagta gggcaggtgt 5580
gggacacgct ctggcacctg gggtgtgtgc aagtgctcag gaagacctgc aggcacaggt 5640
ggcagtgggg gtctctggct gtgctcgagc agcagctgcc tggggtaagg gtagtactct 5700
gtgcatgaac aatgctgcag ggctcagctc tgctcagacc acgaccctgg caccaacaga 5760
gacctgcctg gctctgtggt catgtaaacc tttacaggag ctcaagacaa ggctgtttat 5820
tactgctctg gcaggaaaga agcactggcc atggtcatag agagttccag caacaggaaa 5880
gtgagagccc aagctgctga ggtaccaggg ctcctcaggt gcctgctgca gcagcttgga 5940
cacagtcgag gaacagcaat tgtacctgtg tggtggatca ggctgtgctg cctgtgaacc 6000
tattctagca catctgtcac ctctgtgcca ctcacaggga taccacccct gagaccccta 6060
ccccatcagc ctctgtgtgg gatatggtgt tgggcccaag ggctctgttg cacagggaga 6120
tagaggcctg gggaggaggg aaagcattga ggtggtgttg ataccaggga tgtgagccca 6180
agcaagagat cagcagagca aggaggaaga attgcaggtg ttggggctgg ggaaagcccc 6240
agatggctgg agctggtggg gccactggag atctcctcct cccatcctgc tccatgctgg 6300
ggcagctgct gcaggctgac cagggcctgc ccgggcacgt tgtgaaggtc accaaggatg 6360
gagacttcag agctatctgg gacacgtgtt ttaacgtttg atcagccttg cagcaaaaca 6420
gtgttttgct ctattgaaca gtgttcaaca aaaacgatca gtggcctgga acacatctcc 6480
tacgaagaaa ggttgcgaga gctgggtctg gagaagagaa ggctctgggg caacctccct 6540
gtggcctgtc agtgcttaaa ggaggctcaa gagaaagatg gagagagact ttatacacgg 6600

agctgtagca atatgacaag gagaaatggt ttaaaatgga aagagggtag atttaggtta 6660

gctatcaaga agaaattctt cactcagagg tgatgaagca ctgtagcagg ttgcccagag 6720

aagtattgga tgcttccttc ctggcagggc ccaaggccag actggatggg ttctgggcag 6780

cctgttctgg tgggagatgt ccctgcctgt ggcatggctt ggaaatgggt agcctttgag 6840

gtccctctca gcccaaatca ttctatgttc ttgtggttcc ctgcatttca gcctttgcct 6900

gttgactctg gtgccactgt tggacaattc tactcagctg cgtttcatcc ctgccccaaa 6960

gcagtaatcc catggataag caatgctctg aggccttcac caccctcagg gctcctgctg 7020

accctagcgg cctccctggc agcatgagcc ctttgttgtc cgcactgctg ctgccgaggg 7080

tcctgccagc cccccagggc cttttctgcc caggcactgc cacctgctaa aacccagcct 7140

gcccatggca gggcatcgca tgtctgaggg atggaacggg gcaagggcag tgctgcaggc 7200

agtggttcaa gaaacgttta gatgttgtac tgagggatgt ggtttagtgg gaaatactgg 7260

tgataggtgg atggttggac tggaggatct tagaggtctt ttccaacctt ggtgattatg 7320

tgattctatg aagggagcca tagcctgctc ccatcatgcc ccgccagtcc ggctctcccg 7380

agcactttgg ttccgcagca tgcggccgca agggcgaatt cgcggccgct aaattcaatt 7440

cgccctatag tgagtcgtat tacaattcac tggccgtcgt tttacaacgt cgtgactggg 7500

aaaaccctgg cgttacccaa cttaatcgcc ttgcagcaca tccccctttc gccagctggc 7560

gtaatagcga agaggcccgc accgatcgcc cttcccaaca gttgcgcagc ctatacgtac 7620

ggcagtttaa ggtttacacc tataaaagag agagccgtta tcgtctgttt gtggatgtac 7680

agagtgatat tattgacacg ccggggcgac ggatggtgat ccccctggcc agtgcacgtc 7740

tgctgtcaga taaagtctcc cgtgaacttt acccggtggt gcatatcggg gatgaaagct 7800

ggcgcatgat gaccaccgat atggccagtg tgccggtctc cgttatcggg gaagaagtgg 7860

ctgatctcag ccaccgcgaa aatgacatca aaaacgccat taacctgatg ttctggggaa 7920

tataaatgtc aggcatgaga ttatcaaaaa ggatcttcac ctagatcctt ttcacgtaga 7980

aagccagtcc gcagaaacgg tgctgacccc ggatgaatgt cagctactgg gctatctgga 8040

caagggaaaa cgcaagcgca aagagaaagc aggtagcttg cagtgggctt acatggcgat 8100

agctagactg ggcggtttta tggacagcaa gcgaaccgga attgccagct ggggcgccct 8160

ctggtaaggt tgggaagccc tgcaaagtaa actggatggc tttcttgccg ccaaggatct 8220

gatggcgcag gggatcaagc tctgatcaag agacaggatg aggatcgttt cgcatgattg 8280

aacaagatgg attgcacgca ggttctccgg ccgcttgggt ggagaggcta ttcggctatg 8340

actgggcaca acagacaatc ggctgctctg atgccgccgt gttccggctg tcagcgcagg 8400

ggcgcccggt tctttttgtc aagaccgacc tgtccggtgc cctgaatgaa ctgcaagacg 8460

aggcagcgcg gctatcgtgg ctggccacga cgggcgttcc ttgcgcagct gtgctcgacg 8520

ttgtcactga agcgggaagg gactggctgc tattgggcga agtgccgggg caggatctcc 8580

tgtcatctca ccttgctcct gccgagaaag tatccatcat ggctgatgca atgcggcggc 8640

tgcatacgct tgatccggct acctgcccat tcgaccacca agcgaaacat cgcatcgagc 8700

gagcacgtac tcggatggaa gccggtcttg tcgatcagga tgatctggac gaagagcatc 8760

aggggctcgc gccagccgaa ctgttcgcca ggctcaaggc gagcatgccc gacggcgagg 8820

atctcgtcgt gacccatggc gatgcctgct tgccgaatat catggtggaa aatggccgct 8880

tttctggatt catcgactgt ggccggctgg gtgtggcgga ccgctatcag gacatagcgt 8940

tggctacccg tgatattgct gaagagcttg gcggcgaatg ggctgaccgc ttcctcgtgc 9000

tttacggtat cgccgctccc gattcgcagc gcatcgcctt ctatcgcctt cttgacgagt 9060

tcttctgaat tattaacgct tacaatttcc tgatgcggta ttttctcctt acgcatctgt 9120

gcggtatttc acaccgcatc aggtggcact tttcggggaa atgtgcgcgg aacccctatt 9180

tgtttatttt tctaaataca ttcaaatatg tatccgctca tgagattatc aaaaaggatc 9240

ttcacctaga tccttttaaa ttaaaaatga agttttaaat caatctaaag tatatatgag 9300

taaacttggt ctgacagtta ccaatgctta atcagtgagg cacctatctc agcgatctgt 9360

ctatttcgtt catccatagt tgcctgactc cccgtcgtgt agataactac gatacgggag 9420

ggcttaccat ctggccccag tgctgcaatg ataccgcgag acccacgctc accggctcca 9480

gatttatcag caataaacca gccagccgga agggccgagc gcagaagtgg tcctgcaact 9540

ttatccgcct ccatccagtc tattaattgt tgccgggaag ctagagtaag tagttcgcca 9600

gttaatagtt tgcgcaacgt tgttgccatt gctacaggca tcgtggtgtc acgctcgtcg 9660

tttggtatgg cttcattcag ctccggttcc caacgatcaa ggcgagttac atgatccccc 9720

atgttgtgca aaaaagcggt tagctccttc ggtcctccga tcgttgtcag aagtaagttg 9780

gccgcagtgt tatcactcat ggttatggca gcactgcata attctcttac tgtcatgcca 9840

tccgtaagat gcttttctgt gactggtgag tactcaacca agtcattctg agaatagtgt 9900

atgcggcgac cgagttgctc ttgcccggcg tcaatacggg ataataccgc gccacatagc 9960

agaactttaa aagtgctcat cattggaaaa cgttcttcgg ggcgaaaact ctcaaggatc 10020

ttaccgctgt tgagatccag ttcgatgtaa cccactcgtg cacccaactg atcttcagca 10080

tcttttactt tcaccagcgt ttctgggtga gcaaaaacag gaaggcaaaa tgccgcaaaa 10140

aagggaataa gggcgacacg gaaatgttga atactcatac tcttcctttt tcaatattat 10200

tgaagcattt atcagggtta ttgtctcatg accaaaatcc cttaacgtga gttttcgttc 10260

cactgagcgt cagaccccgt agaaaagatc aaaggatctt cttgagatcc tttttttctg 10320

cgcgtaatct gctgcttgca aacaaaaaaa ccaccgctac cagcggtggt ttgtttgccg 10380

gatcaagagc taccaactct ttttccgaag gtaactggct tcagcagagc gcagatacca 10440

aatactgttc ttctagtgta gccgtagtta ggccaccact tcaagaactc tgtagcaccg 10500

cctacatacc tcgctctgct aatcctgtta ccagtggctg ctgccagtgg cgataagtcg 10560

tgtcttaccg ggttggactc aagacgatag ttaccggata aggcgcagcg gtcgggctga 10620

acggggggtt cgtgcacaca gcccagcttg gagcgaacga cctacaccga actgagatac 10680

ctacagcgtg agctatgaga aagcgccacg cttcccgaag ggagaaaggc ggacaggtat 10740

ccggtaagcg gcagggtcgg aacaggagag cgcacgaggg agcttccagg gggaaacgcc 10800

tggtatcttt atagtcctgt cgggtttcgc cacctctgac ttgagcgtcg atttttgtga 10860

tgctcgtcag gggggcggag cctatggaaa aacgccagca acgcggcctt tttacggttc 10920

ctggcctttt gctggccttt tgctcacatg ttctttcctg cgttatcccc tgattctgtg 10980

gataaccgta ttaccgcctt tgagtgagct gataccgctc gccgcagccg aacgaccgag 11040

cgcagcgagt cagtgagcga ggaagcggaa g 11071

<210> 29
<211> 2787
<212> DNA
<213> Homo sapiens

<400> 29
cctccaccaa gggcccatcg gtcttccccc tggcaccctc ctccaagagc acctctgggg 60

gcacagcggc cctgggctgc ctggtcaagg actacttccc cgaaccggtg acggtgtcgt 120

ggaactcagg cgccctgacc agcggcgtgc acaccttccc ggctgtccta cagtcctcag 180

gactctactc cctcagcagc gtggtgaccg tgccctccag cagcttgggc acccagacct 240

acatctgcaa cgtgaatcac aagcccagca acaccaaggt ggacaagaga gttgagccca 300

aatcttgtga caaaactcac acatgcccac cgtgcccagg taagtcacta gaccagagct 360

ccactcccgg gagaatggta agtgctgtaa acatccctgc actagaggat aagccatgta 420

cagatccatt tccatctctc ctcatcagca cctgaactcc tggggggacc gtcagtcttc 480

ctcttccccc caaaacccaa ggacaccctc atgatctccc ggacccctga ggtcacatgc 540

gtggtggtgg acgtgagcca cgaagaccct gaggtcaagt tcaactggta cgtggacggc 600

gtggaggtgc ataatgccaa gacaaagccg cgggaggagc agtacaacag cacgtaccgt 660

gtggtcagcg tcctcaccgt cctgcaccag gactggctga atggcaagga gtacaagtgc 720

aaggtctcca acaaagccct cccagccccc atcgagaaaa ccatctccaa agccaaaggt 780

gagagctgca gcctgactgc atgggggctg ggatgggcat aaggataaag gtctgtgtgg 840

acagccttct gcttcagcca tgacctttgt gtatgtttct accctcacag ggcagccccg 900

agaaccacag gtgtacaccc tgcccccatc ccgggaggag atgaccaaga accaggtcag 960

cctgacctgc ctggtcaaag gcttctatcc cagcgacatc gccgtggagt gggagagcaa 1020

tgggcagccg gagaacaact acaagaccac gcctcccgtg ctggactccg acggctcctt 1080

cttcctctat agcaagctca ccgtggacaa gagcaggtgg cagcagggga acgtcttctc 1140

atgctccgtg atgcatgagg ctctgcacaa ccactacacg cagaagagcc tctccctgtc 1200

cccgggtaaa tgagctcagc acccacaaaa ctctcaggtc caaagagaca cccacactca 1260

tctccatgct tcccttgtat aaataaagca cccagcaatg cctgggacca tgtaaaactg 1320

tcctggttct ttccaaggta tagagcatag ctcacaggct gatatttctg gccagggttg 1380

gaggacagcc ttgtctatag gaagagaatg aggtttttgc actgcaggac tcagagctca 1440

ttagttatcc tgccttggag tgttggggct tggctttagg cagtgccttt tccttgcctt 1500

cctacgaacc agcagctgcc atacatagag ataatcctag gaagcctcaa atggagaagg 1560

acacaaaccc acctccctca ggctgttcct ctatcccggc cccacttctt tacctagggg 1620

tttctctgag tctattgtgg agttacacat ggccaggggc attccagaga cccttgtcat 1680

ccatacactc aactcaggca gctttgcaca aacaaagtct gcacacccat acagatggct 1740

cactcttgcc tgtgccatgt agggctgagg cacatggctc ttgctgcccc aagggaggga 1800

ctattagata gccacactca tgctgaatcc tggcccattc aaattagcct gctgaacacc 1860

atccagtcca tatagcacat gtatccacat gcacgtgtgc acaaaacgca tttaacacac 1920

tgggacaaca attctgtgcc ctgcacagca cctatatcca gcaatgtatc accatacaca 1980

cgaccaaaaa ttcaatgccc acgtttctgc catcacaaac agacacatct ttcctctctg 2040

tggccactgc attatatgct caacacaaga cctctgaagc cagatccatc tctggcacct 2100

cggggtcatg cttcaacccc acatgaatta tgcaaaccat agccataatg gtctgaatca 2160

cttcacactg ggatgttccc aagttcaggc aagacgagcc acaggctctg ctgatgactg 2220

aaggacagca aagggtcagt ccagctgtat agccactgtt gacctgggtc acaggccctg 2280

ctgaccctcc accttctcct gtactgaagg aatgaaagat gagacaagca tagagggcac 2340

ttgaataatc caggtcactc tgaggtccac ccaaggcatt attggactca ggtgggaagc 2400

tgagactggt gtcccagagg gaaaggaagg aaagcaggcc ccggggaggg tctgctgtcc 2460

cagtcaggct ggagatctct cctctgaatc catgcagaca tgtctgcctc acagggaatc 2520

tctcccagca ccaaccatgt tgggacaaac actgactgtc ctctctgttc agagctgcaa 2580

ctggaggaga gctgtgcgga ggcgcaggac ggggagctgg acgggctgtg gacgaccatc 2640

accatcttca tcacactctt cctgttaagc gtgtgctaca gtgccaccgt caccttcttc 2700

aaggtgaagt ggatcttctc ctcggtggtg gacctgaagc agaccatcat ccccgactac 2760

aggaacatga tcggacaggg ggcctag 2787

<210> 30
<211> 361
<212> DNA
<213> Homo sapiens

<400> 30
gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcaaatga acagcctgag agccgaggac acggccgtat attactgtgc gaaaggatat 300

agtggctacg attactactt tgactactgg ggccagggca ccctggtcac cgtctcctca 360

g 361


<210> 31
<211> 21
<212> DNA
<213> Artificial Sequence

<220>
<223> Synthetic Primer

<400> 31
ttcccgaagc gaaagccgcg t 21


<210> 32
<211> 21
<212> DNA
<213> Artificial Sequence

<220>
<223> Synthetic Primer

<400> 32
actcaccgga ggagacgatg a 21


<210> 33
<211> 1823
<212> DNA
<213> Gallus gallus

<400> 33
ttcccgaagc gaaagccgcg tgttattttt gggtgggaaa gccccgattc gctccttttt 60

ttttatttat tatttttgtt gtttttcgtg tctttttctt ctccgttttg cagctgcggc 120

ctccaggtgt ccccggccgt ggtatttttg ggtcgaaaaa gccgcttttt ggctcacttg 180

gaaacctttc gggcgtctcc aagcggagcg ttggcgggaa atggtgtttt ttttgggttg 240

ttttcggtcc cttttttcac catttttgtg gtttttcggc ccattttcgg cctccccccc 300

cccccccccc ttcccgtggg gctgcgctcc gtggagcgga atggggtcgt taattaaggt 360

taattggttt aattaatggg ccgtgcaggg agaagagggc cgtgcggtgg ttgttagccc 420

tgattaatgg cttggttgag gggagagtgt aattaagtgc ttcgttagca ctaattaatg 480

gcttgattga gaggagagtg taattaaatg cttcataatc cctaattaat ggcttggttg 540

agacaagagt gtaattaagt gcttcattag ccctgattaa tggcttggtt gaggggagag 600

tgtaattaaa tgcttcgtta gcactaatta atggcttgat tgagaggaga gtgtaattaa 660

atgcttcata atccctaatt aatggcttgg ttgagacaag agtgtaatta agtgcttcat 720

tagccctgat taatggcttg gttgagagga gagtgtaatt aaatgcttca ttatccctaa 780

ttagggactg aattccaagg agagtgtaat taagtgcttc attatcccta attaatggct 840

tggttgagag gagagtgtaa ttaaatgctt tgttagccct aattaatggt ttgatggaga 900

ggagagtgta attaagtgct tcattatccc taattaatgg cttggttgag gggagagtgt 960

aattaaatgc ttagttagcc ctaattaatg gcttggttga gaggagagtg taattgaatg 1020

cttcattatc cctaattagg gattgaattg agaggagagt gtaattgaat gctttgttag 1080

ccctaattag ggattgaatt gagaggagag tgtaattaaa tgcctcatta tccctaatta 1140

gggattgaat tgagaggaga gtttaattaa atgcctcatt atccctaatt agggactgaa 1200

ttccgacgag agtgtaatta agtgcttcat ttagccctaa ttaatggctt gattgagagg 1260

agagtgtaat taaatgcttc attatcccta attagggatt gaattgagag gagagtgtaa 1320

ttaaatgctt cattatccct aattagggat tgaattgaga ggagagtgta attaaatgct 1380

tcattatccc taattaatgg cttggttgag aggggagtgt aattaagtgc ttcgttatcc 1440

ctaattaggg attgaattgt gaggagatgg caattaatac ttcattatcc ctaattaatt 1500

taattagtcc ctattatccc taatttaatt catggtttgg ttgatggaag aatgtaatta 1560

aatgctttgt tatccctaat taatggtttg attgagcgga gagtgtaatt aaatgcttca 1620

ttatccctta ttaatggttg tgtggaatgt agtgtaatta gaagtcccta attaatggtt 1680

taattgagag aagactgcaa ttagatgact catagcccct aataagtggt ttaattatga 1740

gataatcaca attacatgtt tcattgtccc taattaatcg tttaattgcg agaagatcac 1800

aattaattaa atgatcccta att 1823

<210> 34
<211> 1358
<212> DNA
<213> Gallus gallus

<400> 34
aatgttggcg cagaagggat tcatatcaat taattccctg tcctcaatta atgcataatt 60

gggagtgcga tggaatcatt caataagtaa tgaggacttg gtgtcaaaat gccaattaag 120

taattaatga cacccaatta atacacaatg gggattacag tggaataact cagtgaagtt 180

catatctaaa ttgcaattga ataattaatt acagctaatt aatggatggg agactggcag 240

tggtataact taatgaagga acacagtgaa ttcctgtctg aattgaaatt aattgattta 300

ttagggctaa ttaatgaatg gtggggttaa aaatagcatt actcagcgaa ggaacatgag 360

ttcatggcta aattgagatt gaattattaa ttacaactaa ttgattaatg actgatggga 420

gtaagaatgg aataactcag tgaaggaaca caatggattc atggcctaat tgaaattaaa 480

tggttaatta gagctaatta atgaatgatg gggggtggca atggaatgat acaagaaaga 540

aacacaatgg gttcgtgtct acattgaaat taaataatta attacagcta attaataaac 600

ggcgaggttg acaatggtat aactgtgaag agacagaatg agttcgtgac taaattggta 660

ttaaatgatt aattagagct aattaacagg tggtggggct gagaatggca taactcaatg 720

ggggatgatg agctgatggc ttaattggaa ttaataatta atcacaacca attaacaggt 780

gctgttaatg gggttacagg ggcatgactt caggaagaag cacaaagtgg ccattaaata 840

attaattgca cccaattaat tcctgatggg gattactgca gaggaactca gcgaaggatt 900

caagcagaaa tgacacagtt aggttgctca attgcgttaa ttaattaaaa tcgctaatta 960

ggtgagcttc attaaggcct gatggggttc atcccaacgc tgggatcaaa gaactcagcg 1020

cagagtggat aacgataatt agaaggggac aaattaatga ggaaacacga ctttgggcct 1080

agacggccat taaatcatta atcaacccga cgggcgccca attaatcata aagttattaa 1140

gcaattaagt cgaggctgac gagaaaccgc aacgagagcc ctccacgctc ctttgagttg 1200

gcgcaaacaa caaacaacgc gggctcgtta agcagcgacg acgccgagcg taacgaagca 1260

atccccgcgc ccgacaatta acgcgctaac gaagccggag ccctccttat gcaaattagc 1320

ccctccagag ggccataaaa gcgccggctc tccgacgg 1358

<210> 35
<211> 28
<212> DNA
<213> Artificial Sequence

<220>
<223> Synthetic Primer

<400> 35
ggggatcctg ggtcagtcga agggggcg 28

<210> 36
<211> 31
<212> DNA
<213> Artificial Sequence

<220>
<223> Synthetic Primer

<400> 36

gtgcggccgc caaaagcggt aaaatccacc c                                    31

<210>  37
<211>  4266
<212>  DNA
<213>  Gallus gallus

<220>
<221>  misc_feature
<222>  (688)..(688)
<223>  n is a, c, g, or t

<400>  37
tgggtcagtc gaagggggcg gtcggtccat catttgcggc cgggtgattt ttggggccga      60

aaagtgggaa tttggggccc aatttggggc ccaatttggg gcccaatttg ggttttcgag     120

gggggatttt tttaggggga gatttttggg tccccggaaa atggtgawtt tgggtcaaaa     180

atggggawtt tgggggttck ttttraggtg gggttttttg gggkagaaat ggcggtctga     240

ggggaaaatg tcgttttggg gccattttgg gccatttgag gggaaatttg ggtcaaaaaa     300

tgacgatttt gggtcgtttt agggaggaaa atgaatttag ggcgatttga gccaaatttg     360

ggtcaaaaat ggcgattttg agtcaaaaaa tgattatttt gggtcatttt agggaggaaa     420

atgaatttag ggagattgga cggcaaattt gggtcaaaaa atggtgattt tgggtcaaaa     480

aatgattatt ttgggtcatt ttagggagaa aaatgaattt agggagattt gagggcaaat     540

ttgggtcaaa aaatggtgat tttgggtcaa aaaatgatta ttttgggtca ttttagggag     600

aaaaatgaat ttagggagat ttgagggcaa atttgggtcg aaaaatggcg attttgggtg     660

aaaaatggac aattttgggt cattttangg ttaaaaatga atttagggag attggacggc     720

aaatttgggt craaaaatgg tgatttgggg tsaaaaatgr aywattttkg gtcattttag     780

ggataaaaaa tgaatttagg gcgatgtgag ccaattttgg gtcaaaaatg gcgattgtga     840

gtcaaaaaat gattattttg ggtcatttta gggttaaaaa tgaatttagg gagattggac     900

ggcaaatttg ggtcaaaaaa tggtgatttt cggtcaaaaa atgattattt tgggtcattt     960

tagggagaaa aatgaattta gggagattgg acggcaaatt tgggtcgaaa aatggtgatt    1020

ttgggtcaaa aatggacaat ttggggtcat tttagggata aaaatgaatt tagggagatt    1080

tgagagcaaa tttgggtcta aaaatgtcaa tttggggtca aaaatggtga ttttgggtca    1140

ttttagggat ataaatgaat tttgggagat ttgagggcaa atttgggtcg aaaaatggtg    1200

atttggggtc aatttaggga ggaaaatgaa tttaaggtga tttgacggca aatttgggtt    1260

gaaaaatgac aatttggggt cattttaaag ataaaatgaa tttagggcga tttgagggca    1320

aatttgggtc gaagaatggt gatttgggtc aaaaaatgac aattttgggt cattttaggg    1380

atataaatgg acttagagcg atttgagggc aaatttggtt gaaaaaatgg acaatttggg    1440

tcattttagg gatataaatg aatttaggga gatttgatgg caaatttggg tcgaaaaatg 1500

gtgatttggg tcaaaaatgg tgattttggt tgaaaaacag tcatttgggg tcattttagg 1560

gattaaaatg aatttcggga gatttgaggg caaatttggg tcaaaaaatg gtgattttgg 1620

gtcaattagt gataaaaaaa tgaatttagg gtgatttgag ggcaaatttg ggtcaaaaaa 1680

tgacaatttg gggttttttt agggaggaga atgaatttag ggagttttga gggcaaattt 1740

gggtcaaaaa atagtgattt gggtcaaaag tggtgatttt ggttgaaaaa cagtcatttt 1800

gggtcatttt agggagaaaa atgaatttcg ggagatttga gggcaaattt gggtcaaaaa 1860

atgacaattt tgggtcactt tacgaattaa aatgaattta gggagatttg agggcaaatt 1920

tgggtcaaaa tatggtgatt ttgggtcaat ttagggagga aaatgaattt agggcgattt 1980

gaaggcaaat ttgggtcgaa aatggtgatt ttgggtcaaa aaatgatgat tttgggtcgt 2040

tttagggaga aaatgaattt agggagattt gagggcaaat ttgggtcaaa aaatggtgat 2100

tttgggtcat tgtagggata aaaatgaatt tggggcgatt tgagggcaaa tttgggtgga 2160

aaatggacaa ttttgggtca ttttagggag aaaaatgaat ttagggagat ttgagggcaa 2220

atttgggtca aaaaatggtg atttggggtc attttcagaa gaaaaaatga ttattttccc 2280

cactaaaaat gtatattttg gggccaaatg gtgaaaaatg gtgattttta atcaaacgtc 2340

cccaaaattg gggaaatttc atcgatttga cccaaaattg agtttttttt ccctgttaaa 2400

aatgtacatt ttggggtcaa tcgctgaaat gttcccactt ttcacttctt tgcccccaaa 2460

ttttgctgtc cggtgagaaa ttaattaatt aatgatcatt aattgatcga caattaatgg 2520

ttattaatta attaataggt cctcttttgg tgactccttc gcttttgggg ccaaaagtcc 2580

ataaattggc cccaaaaaat taacgctgag taattggatt ccaaagtatt aatgataaac 2640

attaaaagtg tttaattaat aatgatatta aacataattt cgtttttatt atcgatttat 2700

caacaacgat gaacgataat actttacaac aatcgctaat aattaattaa ttatttaatt 2760

aattaatttc taataattac ttcgcattat cggacacgag atgttgtaat tattaataat 2820

ttaattccta ataattagaa gattcgttga aaatggtact tatctttaca aataatcact 2880

tctaataata atgattaata atagttaata acaacaatga cgataatgac gataatatta 2940

ataatatgtg atatatttaa tataaaattc gtattaatat attatatcta caaaatatga 3000

tataaaatat aatattttat ttatatataa cacaatttat tattattatc attattatca 3060

ttattaatat catcattatt aacgttatcg aaatacttat ttagaaataa taaaaacgga 3120

tttaatgatg gcaacaaaaa tattttatta atgttaaaaa aaaataatta ataatttcca 3180

aagattcgaa ttcggggcaa cgaacggcac tcgataattt ttaattaatt aatagtttga 3240

attaatcggt actttttaat cctccatttt gcccgaaatc gccgtttttt gccccaaatt 3300

ccccaccgcg gcgttaaaaa cataaagaaa ttaagcatca aaagtgccct tttttggggt 3360

tgttttgacc caaaaaaaaa atggccgaat tgggggcggc cgttttacgg ttgggttcat 3420

tttgggttca aaacagccaa aaatgggaac tttgggtttc gaaaacaaca acaacaaaaa 3480

aacgggttta ttttgggctc attttgggtg tttttgggtc aggaggagaa aaaataggaa 3540

gtttgagagc gaaacaacgg ccgcttttgg ggggaaaacg gccctttttg gtcaacggcg 3600

ggggaaaaaa aaaagcggag tttttggggt gaaaaagagc ggttttgggt aaatttgggt 3660

tttggggtaa aagtggagga ttttgggcaa tgggagttaa aaaatgggtg tttttatggg 3720

ggttcggtgc agtttttcct gtttgatggg gggtttatta atccgggggg ggggattaat 3780

gagaattaat aatgttaata gaaatatctg ggaaattaat agcaattatt aattgttaat 3840

agttattaat agttctatat atctcacatc tacgatacaa tataatatcg ttacaatcat 3900

atagtcgata tattacatat aattatcagt aataataata agtaacaata attagcagta 3960

attaataata ataattaata gtattcgtta ataagattat tgataataat taagtagtag 4020

cgattaatag agatgggatt tcgtgagaaa tggaccaaat ttgggccgtt ttgacccaaa 4080

tttttggtgg ttttttttcc gattctttgt gaatttcggg tcggattcat cagcaattaa 4140

ttacggttat taggggctat tagaggcttt taattgggat tattagagac ttttaagcgg 4200

atttggggac ttttaagtgg attttatgat tcttaagtgg attttgggtg gattttaccg 4260

cttttg 4266

<210> 38
<211> 21
<212> DNA
<213> Artificial Sequence

<220>
<223> Synthetic Primer

<400> 38
ccaaaccacc tcctggtgtc c 21

<210> 39
<211> 27
<212> DNA
<213> Artificial Sequence

<220>
<223> Synthetic Primer

<400> 39
caaaccaaaa ctacggattc tctgacc 27

<210> 40
<211> 866
<212> DNA
<213> Gallus gallus

<400>  40
   caaaccacct cctggtgtcc cccaaccctt catagccccc caccccgtct cggtcgccca      60
   cccaaccctc cacattaacc ccttcctccc cacgctcgtc ccccgattcc cacccccctt     120
   ttcgtcacca tgcacccccc gtcccgcgag gacttcgaag gccccttccg caacgcctcc     180
   atcctctgcc agacccgcgg gcgccgccgt cccgccgagg tcacgtggta caaaaatggc     240
   agccccgtcg ccgccgccgc cgccaccacc gccaccaccg tcggccccga agtggtggcc     300
   gagagccgca tcagcgtcac cgaaagcgaa tgggacaccg gggccacctt cagctgcgtc     360
   gtggaggggg agatgaggaa caccagcaag aggatggagt gcggatgtaa gtggaggggg     420
   ggtttttggg gggggatttt gggggaaaat tgggggcttt gggggcaaaa agtccgtcat     480
   tttggtttgg aaggggtcga tgtgggggaa aatgtgccaa aaatcccaat tttgggggca     540
   ctgaagtcgt aatttgaggt ggaggaaggt agacagaaag agggtggagt gcggatgtaa     600
   gtggaggggg atttggggca gatttggggg aaattggggg tttggggggc aaataatccg     660
   tagtttcggt ttggaagggg ttgatgtggt tttgtgctga atttcggggg aaaaactgcc     720
   aaaaatccca attttggggg cattgaagtc atagtttgag gttggaagga ggtagagagc     780
   aagaggatga agtgaggatg taagtcgggg gatttttggg gcagatttgg gggaaattgg     840
   gggttttggg gtcagagaat ccgtag                                          866

<210>  41
<211>  16786
<212>  DNA
<213>  Artificial Sequence

<220>
<223>  Targeting vector


<220>
<221>  misc_feature
<222>  (5126)..(5126)
<223>  n is a, c, g, or t

<400>  41
gcgccctagc gcccgctcct ttcgctttct tcccttcctt tctcgccacg ttcgccggct      60

ttccccgtca agctctaaat cgggggctcc ctttagggtt ccgatttagt gctttacggc     120

acctcgaccc caaaaaactt gattagggtg atggttcacg tagtgggcca tcgccctgat     180

agacggtttt tcgccctttg acgttggagt ccacgttctt taatagtgga ctcttgttcc     240

aaactggaac aacactcaac cctatctcgg tctattcttt tgatttataa gggattttgc     300

cgatttcggc ctattggtta aaaaatgagc tgatttaaca aaaatttaac gcgaatttta     360

acaaaatatt aacgcttaca atttccattc gccattcagg ctgcgcaact gttgggaagg     420

gcgatcgggt accataagaa tgcggccgct tcccgaagcg aaagccgcgt gttatttttg     480

ggtgggaaag ccccgattcg ctcctttttt tttatttatt atttttgttg tttttcgtgt 540

ctttttcttc tccgttttgc agctgcggcc tccaggtgtc cccggccgtg gtatttttgg 600

gtcgaaaaag ccgctttttg gctcacttgg aaacctttcg ggcgtctcca agcggagcgt 660

tggcgggaaa tggtgttttt tttgggttgt tttcggtccc ttttttcacc atttttgtgg 720

tttttcggcc cattttcggc ctcccccccc ccccccccct tcccgtgggg ctgcgctccg 780

tggagcggaa tggggtcgtt aattaaggtt aattggttta attaatgggc cgtgcaggga 840

gaagagggcc gtgcggtggt tgttagccct gattaatggc ttggttgagg ggagagtgta 900

attaagtgct tcgttagcac taattaatgg cttgattgag aggagagtgt aattaaatgc 960

ttcataatcc ctaattaatg gcttggttga gacaagagtg taattaagtg cttcattagc 1020

cctgattaat ggcttggttg aggggagagt gtaattaaat gcttcgttag cactaattaa 1080

tggcttgatt gagaggagag tgtaattaaa tgcttcataa tccctaatta atggcttggt 1140

tgagacaaga gtgtaattaa gtgcttcatt agccctgatt aatggcttgg ttgagaggag 1200

agtgtaatta aatgcttcat tatccctaat tagggactga attccaagga gagtgtaatt 1260

aagtgcttca ttatccctaa ttaatggctt ggttgagagg agagtgtaat taaatgcttt 1320

gttagcccta attaatggtt tgatggagag gagagtgtaa ttaagtgctt cattatccct 1380

aattaatggc ttggttgagg ggagagtgta attaaatgct tagttagccc taattaatgg 1440

cttggttgag aggagagtgt aattgaatgc ttcattatcc ctaattaggg attgaattga 1500

gaggagagtg taattgaatg ctttgttagc cctaattagg gattgaattg agaggagagt 1560

gtaattaaat gcctcattat ccctaattag ggattgaatt gagaggagag tttaattaaa 1620

tgcctcatta tccctaatta gggactgaat tccgacgaga gtgtaattaa gtgcttcatt 1680

tagccctaat taatggcttg attgagagga gagtgtaatt aaatgcttca ttatccctaa 1740

ttagggattg aattgagagg agagtgtaat taaatgcttc attatcccta attagggatt 1800

gaattgagag gagagtgtaa ttaaatgctt cattatccct aattaatggc ttggttgaga 1860

ggggagtgta attaagtgct tcgttatccc taattaggga ttgaattgtg aggagatggc 1920

aattaatact tcattatccc taattaattt aattagtccc tattatccct aatttaattc 1980

atggtttggt tgatggaaga atgtaattaa atgctttgtt atccctaatt aatggtttga 2040

ttgagcggag agtgtaatta aatgcttcat tatcccttat taatggttgt gtggaatgta 2100

gtgtaattag aagtccctaa ttaatggttt aattgagaga agactgcaat tagatgactc 2160

atagccccta ataagtggtt taattatgag ataatcacaa ttacatgttt cattgtccct 2220

aattaatcgt ttaattgcga gaagatcaca attaattaaa tgatccctaa tttcaatttc 2280

tgagaagtgt ctttctcgga aattgagata tcataagaat tcgcgatcaa tttctgagaa 2340

ctgtcattct cggaatacct gacgtcaatg ttggcgcaga agggattcat atcaattaat 2400

tccctgtcct caattaatgc ataattggga gtgcgatgga atcattcaat aagtaatgag 2460

gacttggtgt caaaatgcca attaagtaat taatgacacc caattaatac acaatgggga 2520

ttacagtgga ataactcagt gaagttcata tctaaattgc aattgaataa ttaattacag 2580

ctaattaatg gatgggagac tggcagtggt ataacttaat gaaggaacac agtgaattcc 2640

tgtctgaatt gaaattaatt gatttattag ggctaattaa tgaatggtgg ggttaaaaat 2700

agcattactc agcgaaggaa catgagttca tggctaaatt gagattgaat tattaattac 2760

aactaattga ttaatgactg atgggagtaa gaatggaata actcagtgaa ggaacacaat 2820

ggattcatgg cctaattgaa attaaatggt taattagagc taattaatga atgatggggg 2880

gtggcaatgg aatgatacaa gaaagaaaca caatgggttc gtgtctacat tgaaattaaa 2940

taattaatta cagctaatta ataaacggcg aggttgacaa tggtataact gtgaagagac 3000

agaatgagtt cgtgactaaa ttggtattaa atgattaatt agagctaatt aacaggtggt 3060

ggggctgaga atggcataac tcaatggggg atgatgagct gatggcttaa ttggaattaa 3120

taattaatca caaccaatta acaggtgctg ttaatggggt tacaggggca tgacttcagg 3180

aagaagcaca aagtggccat taaataatta attgcaccca attaattcct gatggggatt 3240

actgcagagg aactcagcga aggattcaag cagaaatgac acagttaggt tgctcaattg 3300

cgttaattaa ttaaaatcgc taattaggtg agcttcatta aggcctgatg gggttcatcc 3360

caacgctggg atcaaagaac tcagcgcaga gtggataacg ataattagaa ggggacaaat 3420

taatgaggaa acacgacttt gggcctagac ggccattaaa tcattaatca acccgacggg 3480

cgcccaatta atcataaagt tattaagcaa ttaagtcgag gctgacgaga aaccgcaacg 3540

agagccctcc acgctccttt gagttggcgc aaacaacaaa caacgcgggc tcgttaagca 3600

gcgacgacgc cgagcgtaac gaagcaatcc ccgcgcccga caattaacgc gctaacgaag 3660

ccggagccct ccttatgcaa attagcccct ccagagggcc ataaaagcgc cggctctccg 3720

acggcgcggg aggagcacca gtcggctccg caaccatgag cccactcgtc tcctccctcc 3780

tgctcctggc cgccctgcca ggtgagggcg ctgtggggct ctatggggct ctatggggtc 3840

tcagcggggc tctgcgggct caatggggcc caaagggggg gtctgcgggc tctatggggg 3900

ggtcaacggg gggtctcacg gggggccggc tccgcgaggc cgtgtggcgg cggctccgtc 3960

agcgctctct gggcgcgcct ccttccccac agggctgatg gcggaggtgc agctgttgga 4020

gtctggggga ggcttggtac agcctggggg gtccctgaga ctctcctgtg cagcctctgg 4080

attcaccttt agcagctatg ccatgagctg ggtccgccag gctccaggga aggggctgga 4140

gtgggtctca gctattagtg gtagtggtgg tagcacatac tacgcagact ccgtgaaggg 4200

ccggttcacc atctccagag acaattccaa gaacacgctg tatctgcaaa tgaacagcct 4260

gagagccgag gacacggccg tatattactg tgcgaaagga tatagtggct acgattacta 4320

ctttgactac tggggccagg gcaccctggt caccgtctcc tcaggtgagt cttcatatga 4380

acccccccca aaattgccgc ggcgattttg gggcaaaatc gggcgatttt atggatcctg 4440

ggtcagtcga agggggcggt cggtccatca tttgcggccg ggtgattttt ggggccgaaa 4500

agtgggaatt tggggcccaa tttggggccc aatttggggc ccaatttggg ttttcgaggg 4560

gggatttttt tagggggaga tttttgggtc cccggaaaat ggtgawtttg ggtcaaaaat 4620

ggggawtttg ggggttcktt ttraggtggg gttttttggg gkagaaatgg cggtctgagg 4680

ggaaaatgtc gttttggggc cattttgggc catttgaggg gaaatttggg tcaaaaaatg 4740

acgattttgg gtcgttttag ggaggaaaat gaatttaggg cgatttgagc caaatttggg 4800

tcaaaaatgg cgattttgag tcaaaaaatg attattttgg gtcattttag ggaggaaaat 4860

gaatttaggg agattggacg gcaaatttgg gtcaaaaaat ggtgattttg ggtcaaaaaa 4920

tgattatttt gggtcatttt agggagaaaa atgaatttag ggagatttga gggcaaattt 4980

gggtcaaaaa atggtgattt tgggtcaaaa aatgattatt ttgggtcatt ttagggagaa 5040

aaatgaattt agggagattt gagggcaaat ttgggtcgaa aaatggcgat tttgggtgaa 5100

aaatggacaa ttttgggtca ttttanggtt aaaaatgaat ttagggagat tggacggcaa 5160

atttgggtcr aaaaatggtg atttggggts aaaaatgray wattttkggt cattttaggg 5220

ataaaaaatg aatttagggc gatgtgagcc aattttgggt caaaaatggc gattgtgagt 5280

caaaaaatga ttattttggg tcattttagg gttaaaaatg aatttaggga gattggacgg 5340

caaatttggg tcaaaaaatg gtgattttcg gtcaaaaaat gattattttg ggtcatttta 5400

gggagaaaaa tgaatttagg gagattggac ggcaaatttg ggtcgaaaaa tggtgatttt 5460

gggtcaaaaa tggacaattt ggggtcattt tagggataaa aatgaattta gggagatttg 5520

agagcaaatt tgggtctaaa aatgtcaatt tggggtcaaa aatggtgatt ttgggtcatt 5580

ttagggatat aaatgaattt tgggagattt gagggcaaat ttgggtcgaa aaatggtgat 5640

ttggggtcaa tttagggagg aaaatgaatt taaggtgatt tgacggcaaa tttgggttga 5700

aaaatgacaa tttggggtca ttttaaagat aaaatgaatt tagggcgatt tgagggcaaa 5760

tttgggtcga agaatggtga tttgggtcaa aaaatgacaa ttttgggtca ttttagggat 5820

ataaatggac ttagagcgat ttgagggcaa atttggttga aaaaatggac aatttgggtc 5880

attttaggga tataaatgaa tttagggaga tttgatggca aatttgggtc gaaaaatggt 5940

gatttgggtc aaaaatggtg attttggttg aaaaacagtc atttggggtc attttaggga 6000

ttaaaatgaa tttcgggaga tttgagggca aatttgggtc aaaaaatggt gattttgggt 6060

caattagtga taaaaaaatg aatttagggt gatttgaggg caaatttggg tcaaaaaatg 6120

acaatttggg gtttttttag ggaggagaat gaatttaggg agttttgagg gcaaatttgg 6180

gtcaaaaaat agtgatttgg gtcaaaagtg gtgattttgg ttgaaaaaca gtcattttgg 6240

gtcattttag ggagaaaaat gaatttcggg agatttgagg gcaaatttgg gtcaaaaaat 6300

gacaattttg ggtcacttta cgaattaaaa tgaatttagg gagatttgag ggcaaatttg 6360

ggtcaaaata tggtgatttt gggtcaattt agggaggaaa atgaatttag ggcgatttga 6420

aggcaaattt gggtcgaaaa tggtgatttt gggtcaaaaa atgatgattt tgggtcgttt 6480

tagggagaaa atgaatttag ggagatttga gggcaaattt gggtcaaaaa atggtgattt 6540

tgggtcattg tagggataaa aatgaatttg gggcgatttg agggcaaatt tgggtggaaa 6600

atggacaatt ttgggtcatt ttagggagaa aaatgaattt agggagattt gagggcaaat 6660

ttgggtcaaa aaatggtgat ttggggtcat tttcagaaga aaaaatgatt attttcccca 6720

ctaaaaatgt atattttggg gccaaatggt gaaaaatggt gatttttaat caaacgtccc 6780

caaaattggg gaaatttcat cgatttgacc caaaattgag ttttttttcc ctgttaaaaa 6840

tgtacatttt ggggtcaatc gctgaaatgt tcccactttt cacttctttg cccccaaatt 6900

ttgctgtccg gtgagaaatt aattaattaa tgatcattaa ttgatcgaca attaatggtt 6960

attaattaat taataggtcc tcttttggtg actccttcgc ttttggggcc aaaagtccat 7020

aaattggccc caaaaaatta acgctgagta attggattcc aaagtattaa tgataaacat 7080

taaaagtgtt taattaataa tgatattaaa cataatttcg tttttattat cgatttatca 7140

acaacgatga acgataatac tttacaacaa tcgctaataa ttaattaatt atttaattaa 7200

ttaatttcta ataattactt cgcattatcg gacacgagat gttgtaatta ttaataattt 7260

aattcctaat aattagaaga ttcgttgaaa atggtactta tctttacaaa taatcacttc 7320

taataataat gattaataat agttaataac aacaatgacg ataatgacga taatattaat 7380

aatatgtgat atatttaata taaaattcgt attaatatat tatatctaca aaatatgata 7440

taaaatataa tattttattt atatataaca caatttatta ttattatcat tattatcatt 7500

attaatatca tcattattaa cgttatcgaa atacttattt agaaataata aaaacggatt 7560

taatgatggc aacaaaaata ttttattaat gttaaaaaaa aataattaat aatttccaaa 7620

gattcgaatt cggggcaacg aacggcactc gataattttt aattaattaa tagtttgaat 7680

taatcggtac tttttaatcc tccattttgc ccgaaatcgc cgttttttgc cccaaattcc 7740

ccaccgcggc gttaaaaaca taaagaaatt aagcatcaaa agtgcccttt tttggggttg 7800

ttttgaccca aaaaaaaaat ggccgaattg ggggcggccg ttttacggtt gggttcattt 7860

tgggttcaaa acagccaaaa atgggaactt tgggtttcga aaacaacaac aacaaaaaaa 7920

cgggtttatt ttgggctcat tttgggtgtt tttgggtcag gaggagaaaa aataggaagt 7980

ttgagagcga aacaacggcc gcttttgggg ggaaaacggc cctttttggt caacggcggg 8040

ggaaaaaaaa aagcggagtt tttggggtga aaaagagcgg ttttgggtaa atttgggttt 8100

tggggtaaaa gtggaggatt ttgggcaatg ggagttaaaa aatgggtgtt tttatggggg 8160

ttcggtgcag tttttcctgt ttgatggggg gtttattaat ccgggggggg ggattaatga 8220

gaattaataa tgttaataga aatatctggg aaattaatag caattattaa ttgttaatag 8280

ttattaatag ttctatatat ctcacatcta cgatacaata taatatcgtt acaatcatat 8340

agtcgatata ttacatataa ttatcagtaa taataataag taacaataat tagcagtaat 8400

taataataat aattaatagt attcgttaat aagattattg ataataatta agtagtagcg 8460

attaatagag atgggatttc gtgagaaatg gaccaaattt gggccgtttt gacccaaatt 8520

tttggtggtt ttttttccga ttctttgtga atttcgggtc ggattcatca gcaattaatt 8580

acggttatta ggggctatta gaggctttta attgggatta ttagagactt ttaagcggat 8640

ttggggactt ttaagtggat tttatgattc ttaagtggat tttgggtgga ttttaccgct 8700

tttgggccgc cgcccccctc taacccccca tctccccccc ttttctctct cccccccccc 8760

ccccaaaccc cacagcctcc accaagggcc catcggtctt ccccctggca ccctcctcca 8820

agagcacctc tgggggcaca gcggccctgg gctgcctggt caaggactac ttccccgaac 8880

cggtgacggt gtcgtggaac tcaggcgccc tgaccagcgg cgtgcacacc ttcccggctg 8940

tcctacagtc ctcaggactc tactccctca gcagcgtggt gaccgtgccc tccagcagct 9000

tgggcaccca gacctacatc tgcaacgtga atcacaagcc cagcaacacc aaggtggaca 9060

agagagttga gcccaaatct tgtgacaaaa ctcacacatg cccaccgtgc ccaggtaagt 9120

cactagacca gagctccact cccgggagaa tggtaagtgc tgtaaacatc cctgcactag 9180

aggataagcc atgtacagat ccatttccat ctctcctcat cagcacctga actcctgggg 9240

ggaccgtcag tcttcctctt ccccccaaaa cccaaggaca ccctcatgat ctcccggacc 9300

cctgaggtca catgcgtggt ggtggacgtg agccacgaag accctgaggt caagttcaac 9360

tggtacgtgg acggcgtgga ggtgcataat gccaagacaa agccgcggga ggagcagtac 9420

aacagcacgt accgtgtggt cagcgtcctc accgtcctgc accaggactg gctgaatggc 9480

aaggagtaca agtgcaaggt ctccaacaaa gccctcccag cccccatcga gaaaaccatc 9540

tccaaagcca aaggtgagag ctgcagcctg actgcatggg ggctgggatg ggcataagga 9600

taaaggtctg tgtggacagc cttctgcttc agccatgacc tttgtgtatg tttctaccct 9660

cacagggcag ccccgagaac cacaggtgta caccctgccc ccatcccggg aggagatgac 9720

caagaaccag gtcagcctga cctgcctggt caaaggcttc tatcccagcg acatcgccgt 9780

ggagtgggag agcaatgggc agccggagaa caactacaag accacgcctc ccgtgctgga 9840

ctccgacggc tccttcttcc tctatagcaa gctcaccgtg gacaagagca ggtggcagca 9900

ggggaacgtc ttctcatgct ccgtgatgca tgaggctctg cacaaccact acacgcagaa 9960

gagcctctcc ctgtccccgg gtaaatgagc tcagcaccca caaaactctc aggtccaaag 10020

agacacccac actcatctcc atgcttccct tgtataaata aagcacccag caatgcctgg 10080

gaccatgtaa aactgtcctg gttctttcca aggtatagag catagctcac aggctgatat 10140

ttctggccag ggttggagga cagccttgtc tataggaaga gaatgaggtt tttgcactgc 10200

aggactcaga gctcattagt tatcctgcct tggagtgttg gggcttggct ttaggcagtg 10260

ccttttcctt gccttcctac gaaccagcag ctgccataca tagagataat cctaggaagc 10320

ctcaaatgga gaaggacaca aacccacctc cctcaggctg ttcctctatc ccggccccac 10380

ttctttacct aggggtttct ctgagtctat tgtggagtta cacatggcca ggggcattcc 10440

agagaccctt gtcatccata cactcaactc aggcagcttt gcacaaacaa agtctgcaca 10500

cccatacaga tggctcactc ttgcctgtgc catgtagggc tgaggcacat ggctcttgct 10560

gccccaaggg agggactatt agatagccac actcatgctg aatcctggcc cattcaaatt 10620

agcctgctga acaccatcca gtccatatag cacatgtatc cacatgcacg tgtgcacaaa 10680

acgcatttaa cacactggga caacaattct gtgccctgca cagcacctat atccagcaat 10740

gtatcaccat acacacgacc aaaaattcaa tgcccacgtt tctgccatca caaacagaca 10800

catctttcct ctctgtggcc actgcattat atgctcaaca caagacctct gaagccagat 10860

ccatctctgg cacctcgggg tcatgcttca accccacatg aattatgcaa accatagcca 10920

taatggtctg aatcacttca cactgggatg ttcccaagtt caggcaagac gagccacagg 10980

ctctgctgat gactgaagga cagcaaaggg tcagtccagc tgtatagcca ctgttgacct 11040

gggtcacagg ccctgctgac cctccacctt ctcctgtact gaaggaatga aagatgagac 11100

aagcatagag ggcacttgaa taatccaggt cactctgagg tccacccaag gcattattgg 11160

actcaggtgg gaagctgaga ctggtgtccc agagggaaag gaaggaaagc aggccccggg 11220

gagggtctgc tgtcccagtc aggctggaga tctctcctct gaatccatgc agacatgtct 11280

gcctcacagg gaatctctcc cagcaccaac catgttggga caaacactga ctgtcctctc 11340

tgttcagagc tgcaactgga ggagagctgt gcggaggcgc aggacgggga gctggacggg 11400

ctgtggacga ccatcaccat cttcatcaca ctcttcctgt taagcgtgtg ctacagtgcc 11460

accgtcacct tcttcaaggt gaagtggatc ttctcctcgg tggtggacct gaagcagacc 11520

atcatccccg actacaggaa catgatcgga cagggggcct aggaattaat tcgctgtctg 11580

cgagggccag ctgttggggt gagtactccc tctcaaaagc gggcatgact tctgcgctaa 11640

gattgtcagt ttccaaaaac gaggaggatt tgatattcac ctggcccgcg gtgatgcctt 11700

tgagggtggc cgcgtccatc tggtcagaaa agacaatctt tttgttgtca agcttgaggt 11760

gtggcaggct tgagatctgg ccatacactt gagtgacaat gacatccact ttgcctttct 11820

ctccacaggt gtccactccc aggtccaact gcaggtcgag catgcatcta gggcggccaa 11880
ttggatccta ccgttcgtat aatgtatgct atacgaagtt atcgtacgct taattaactg 11940
atccggaatt ccgccccccc cccccccccc cctctccctc ccccccccct aacgttactg 12000
gccgaagccg cttggaataa ggccggtgtg cgtttgtcta tatgttattt tccaccatat 12060
tgccgtcttt tggcaatgtg agggcccgga aacctggccc tgtcttcttg acgagcattc 12120
ctaggggtct ttcccctctc gccaaaggaa tgcaaggtct gttgaatgtc gtgaaggaag 12180
cagttcctct ggaagcttct tgaagacaaa caacgtctgt agcgaccctt tgcaggcagc 12240
ggaacccccc acctggcgac aggtgcctct gcggccaaaa gccacgtgta taagatacac 12300
ctgcaaaggc ggcacaaccc cagtgccacg ttgtgagttg gatagttgtg gaaagagtca 12360
aatggctctc ctcaagcgta ttcaacaagg ggctgaagga tgcccagaag gtaccccatt 12420
gtatgggatc tgatctgggg cctcggtgca catgctttac atgtgtttag tcgaggttaa 12480
aaaaacgtct aggccccccg aaccacgggg acgtggtttt cctttgaaaa acacgatgat 12540
aagcttgcca caaccatggc tgaacaagat ggattgcacg caggttctcc ggccgcttgg 12600
gtggagaggc tattcggcta tgactgggca caacagacaa tcggctgctc tgatgccgcc 12660
gtgttccggc tgtcagcgca ggggcgcccg gttctttttg tcaagaccga cctgtccggt 12720
gccctgaatg aactgcagga cgaggcagcg cggctatcgt ggctggccac gacgggcgtt 12780
ccttgcgcag ctgtgctcga cgttgtcact gaagcgggaa gggactggct gctattgggc 12840
gaagtgccgg ggcaggatct cctgtcatct caccttgctc ctgccgagaa agtatccatc 12900
atggctgatg caatgcggcg gctgcatacg cttgatccgg ctacctgccc attcgaccac 12960
caagcgaaac atcgcatcga gcgagcacgt actcggatgg aagccggtct tgtcgatcag 13020
gatgatctgg acgaagagca tcaggggctc gcgccagccg aactgttcgc caggctcaag 13080
gcgcgcatgc ccgacggcga ggatctcgtc gtgacccatg gcgatgcctg cttgccgaat 13140
atcatggtgg aaaatggccg cttttctgga ttcatcgact gtggccggct gggtgtggcg 13200
gaccgctatc aggacatagc gttggctacc cgtgatattg ctgaagagct tggcggcgaa 13260
tgggctgacc gcttcctcgt gctttacggt atcgccgctc ccgattcgca gcgcatcgcc 13320
ttctatcgcc ttcttgacga gttcttctga ataacttcgt ataatgtatg ctatacgaac 13380
ggtaggatcc cgcccgcccc acgacccgca gcgcccgacc gaaaggagcg cacgacccca 13440
tggctccgac cgaagccacc cggggcggcc ccgccgaccc cgcacccgcc cccgaggccc 13500
accgactcta gataactgat cataatcagc cataccacat ttgtagaggt tttacttgct 13560
ttaaaaaacc tcccacacct ccccctgaac ctgaaacata aaatgaatgc aattgttgtt 13620
gttaacttgt ttattgcagc ttataatggt tacaaataaa gcaatagcat cacaaatttc 13680
acaaataaag catttttttc actgtatgca aaccacctcc tggtgtcccc caacccttca 13740

tagcccccca ccccgtctcg gtcgcccacc caaccctcca cattaacccc ttcctcccca 13800

cgctcgtccc ccgattccca cccccctttt cgtcaccatg caccccccgt cccgcgagga 13860

cttcgaaggc cccttccgca acgcctccat cctctgccag acccgcgggc gccgccgtcc 13920

cgccgaggtc acgtggtaca aaaatggcag ccccgtcgcc gccgccgccg ccaccaccgc 13980

caccaccgtc ggccccgaag tggtggccga gagccgcatc agcgtcaccg aaagcgaatg 14040

ggacaccggg gccaccttca gctgcgtcgt ggagggggag atgaggaaca ccagcaagag 14100

gatggagtgc ggatgtaagt ggaggggggg ttttttgggg ggggatttgg ggggaaattg 14160

ggggctttgg gggcaaaaag tccgtcattt tggtttggaa ggggtcgatg tgggggaaaa 14220

tgtgccaaaa atcccaattt tgggggcact gaagtcgtaa tttgaggtgg aggaaggtag 14280

acagaaagag ggtggagtgc ggatgtaagt ggagggggat ttggggcaga tttgggggaa 14340

attgggggtt tggggggcaa ataatccgta gtttcggttt ggaaggggtt gatgtggttt 14400

tgtgctgaat ttcgggggaa aaactgccaa aaatcccaat tttgggggca ttgaagtcat 14460

agtttgaggt tggaaggagg tagagagcaa gaggatgaag tgaggatgta agtcggggga 14520

tttttggggc agatttgggg gaaattgggg gttttggggt cagagaatcc gtaggtcgac 14580

cgcgccagct tttgttccct ttagtgaggg ttaattgcgc gcttggcgta atcatggtca 14640

tagctgtttc ctgtgtgaaa ttgttatccg ctcacaattc cacacaacat acgagccgga 14700

agcataaagt gtaaagcctg gggtgcctaa tgagtgagct aactcacatt aattgcgttg 14760

cgctcactgc ccgctttcca gtcgggaaac ctgtcgtgcc agctgcatta atgaatcggc 14820

caacgcgcgg ggagaggcgg tttgcgtatt gggcgctctt ccgcttcctc gctcactgac 14880

tcgctgcgct cggtcgttcg gctgcggcga gcggtatcag ctcactcaaa ggcggtaata 14940

cggttatcca cagaatcagg ggataacgca ggaaagaaca tgtgagcaaa aggccagcaa 15000

aaggccagga accgtaaaaa ggccgcgttg ctggcgtttt tccataggct ccgcccccct 15060

gacgagcatc acaaaaatcg acgctcaagt cagaggtggc gaaacccgac aggactataa 15120

agataccagg cgtttccccc tggaagctcc ctcgtgcgct ctcctgttcc gaccctgccg 15180

cttaccggat acctgtccgc ctttctccct tcgggaagcg tggcgctttc tcatagctca 15240

cgctgtaggt atctcagttc ggtgtaggtc gttcgctcca agctgggctg tgtgcacgaa 15300

ccccccgttc agcccgaccg ctgcgcctta tccggtaact atcgtcttga gtccaacccg 15360

gtaagacacg acttatcgcc actggcagca gccactggta acaggattag cagagcgagg 15420

tatgtaggcg gtgctacaga gttcttgaag tggtggccta actacggcta cactagaagg 15480

acagtatttg gtatctgcgc tctgctgaag ccagttacct tcggaaaaag agttggtagc 15540

tcttgatccg gcaaacaaac caccgctggt agcggtggtt tttttgtttg caagcagcag 15600

attacgcgca gaaaaaaagg atctcaagaa gatcctttga tcttttctac ggggtctgac 15660

gctcagtgga acgaaaactc acgttaaggg attttggtca tgagattatc aaaaaggatc 15720

ttcacctaga tccttttaaa ttaaaaatga agttttaaat caatctaaag tatatatgag 15780

taaacttggt ctgacagtta ccaatgctta atcagtgagg cacctatctc agcgatctgt 15840

ctatttcgtt catccatagt tgcctgactc cccgtcgtgt agataactac gatacgggag 15900

ggcttaccat ctggccccag tgctgcaatg ataccgcgag acccacgctc accggctcca 15960

gatttatcag caataaacca gccagccgga agggccgagc gcagaagtgg tcctgcaact 16020

ttatccgcct ccatccagtc tattaattgt tgccgggaag ctagagtaag tagttcgcca 16080

gttaatagtt tgcgcaacgt tgttgccatt gctacaggca tcgtggtgtc acgctcgtcg 16140

tttggtatgg cttcattcag ctccggttcc caacgatcaa ggcgagttac atgatccccc 16200

atgttgtgca aaaaagcggt tagctccttc ggtcctccga tcgttgtcag aagtaagttg 16260

gccgcagtgt tatcactcat ggttatggca gcactgcata attctcttac tgtcatgcca 16320

tccgtaagat gcttttctgt gactggtgag tactcaacca agtcattctg agaatagtgt 16380

atgcggcgac cgagttgctc ttgcccggcg tcaatacggg ataataccgc gccacatagc 16440

agaactttaa aagtgctcat cattggaaaa cgttcttcgg ggcgaaaact ctcaaggatc 16500

ttaccgctgt tgagatccag ttcgatgtaa cccactcgtg cacccaactg atcttcagca 16560

tcttttactt tcaccagcgt ttctgggtga gcaaaaacag gaaggcaaaa tgccgcaaaa 16620

aagggaataa gggcgacacg gaaatgttga atactcatac tcttcctttt tcaatattat 16680

tgaagcattt atcagggtta ttgtctcatg agcggataca tatttgaatg tatttagaaa 16740

aataaacaaa taggggttcc gcgcacattt ccccgaaaag tgccac 16786

<210> 42
<211> 814
<212> DNA
<213> Artificial Sequence

<220>
<223> Synthetic DNA

<400> 42
tgaggagacg gtgaccaggg tgccctggcc ccagtagtca aagtagtaat cgtaagccac 60

tatatccttt cgcacagtaa tatacggccg tgtcctcggc tctcaggctg ttcatttgca 120

gatacagcgt gttcttggaa ttgtctctgg agatggtgaa ccggcccttc acggagtctg 180

cgtagtatgt tgctaccacc actaccacta atagctgaga cccactccag ccccttccct 240

ggagcctggc ggacccagct catggcatat gctgctaaag gtgaatccag aggctgcaca 300

ggagagtctc agggaccccc caggctgtac caagcctccc ccagactcca acagctgcac 360

ctcgtgcctg gtgactcttc cgcaatcagt cctgggacag aaacctcagg gacagccttt 420

atgagttttg atcacatcta acagagagga tgggccagtg aggagacggt gaccagggtg 480

ccctggcccc agtagtcaaa gtagtaatcg tgccactata tcctttcgca cagtaatata 540

cggccgtgtc ctcggctctc aggctgttca tttgcagata cagcgtgttc ttggaattgt 600

ctctggagat ggtgaaccgg cccttcacgg agtctgcgta gtatgtgcta ccaccaacca 660

ctaatagctg agacccactc cagccccttc cctggagcct ggcggaccca gctcatggca 720

tgctgctaaa ggtgaatcca gaggctgcac aggagagtct cagggacccc ccaggctgta 780

ccaagcctcc cccagactcc aacagctgca cctc 814

<210> 43
<211> 17606
<212> DNA
<213> Artificial Sequence

<220>
<223> Targeting vector

<220>
<221> misc_feature
<222> (5946)..(5946)
<223> n is a, c, g, or t

<400> 43
   gcgccctagc gcccgctcct ttcgctttct tcccttcctt tctcgccacg ttcgccggct 60
   ttccccgtca agctctaaat cgggggctcc ctttagggtt ccgatttagt gctttacggc 120
   acctcgaccc caaaaaactt gattagggtg atggttcacg tagtgggcca tcgccctgat 180
   agacggtttt tcgccctttg acgttggagt ccacgttctt taatagtgga ctcttgttcc 240
   aaactggaac aacactcaac cctatctcgg tctattcttt tgatttataa gggattttgc 300
   cgatttcggc ctattggtta aaaaatgagc tgatttaaca aaaatttaac gcgaatttta 360
   acaaaatatt aacgcttaca atttccattc gccattcagg ctgcgcaact gttgggaagg 420
   gcgatcgggt accataagaa tgcggccgct tcccgaagcg aaagccgcgt gttatttttg 480
   ggtgggaaag ccccgattcg ctcctttttt tttatttatt atttttgttg tttttcgtgt 540
   ctttttcttc tccgttttgc agctgcggcc tccaggtgtc cccggccgtg gtatttttgg 600
   gtcgaaaaag ccgctttttg gctcacttgg aaacctttcg ggcgtctcca agcggagcgt 660
   tggcgggaaa tggtgttttt tttgggttgt tttcggtccc ttttttcacc atttttgtgg 720
   tttttcggcc cattttcggc ctcccccccc ccccccccct tcccgtgggg ctgcgctccg 780
   tggagcggaa tggggtcgtt aattaaggtt aattggttta attaatgggc cgtgcaggga 840
   gaagagggcc gtgcggtggt tgttagccct gattaatggc ttggttgagg ggagagtgta 900
   attaagtgct tcgttagcac taattaatgg cttgattgag aggagagtgt aattaaatgc 960
   ttcataatcc ctaattaatg gcttggttga gacaagagtg taattaagtg cttcattagc 1020
   cctgattaat ggcttggttg aggggagagt gtaattaaat gcttcgttag cactaattaa 1080
   tggcttgatt gagaggagag tgtaattaaa tgcttcataa tccctaatta atggcttggt 1140
   tgagacaaga gtgtaattaa gtgcttcatt agccctgatt aatggcttgg ttgagaggag 1200
   agtgtaatta aatgcttcat tatccctaat tagggactga attccaagga gagtgtaatt 1260
   aagtgcttca ttatccctaa ttaatggctt ggttgagagg agagtgtaat taaatgcttt 1320
   gttagcccta attaatggtt tgatggagag gagagtgtaa ttaagtgctt cattatccct 1380
   aattaatggc ttggttgagg ggagagtgta attaaatgct tagttagccc taattaatgg 1440
   cttggttgag aggagagtgt aattgaatgc ttcattatcc ctaattaggg attgaattga 1500
   gaggagagtg taattgaatg ctttgttagc cctaattagg gattgaattg agaggagagt 1560
   gtaattaaat gcctcattat ccctaattag ggattgaatt gagaggagag tttaattaaa 1620
   tgcctcatta tccctaatta gggactgaat tccgacgaga gtgtaattaa gtgcttcatt 1680
   tagccctaat taatggcttg attgagagga gagtgtaatt aaatgcttca ttatccctaa 1740
   ttagggattg aattgagagg agagtgtaat taaatgcttc attatcccta attagggatt 1800
   gaattgagag gagagtgtaa ttaaatgctt cattatccct aattaatggc ttggttgaga 1860
   ggggagtgta attaagtgct tcgttatccc taattaggga ttgaattgtg aggagatggc 1920
   aattaatact tcattatccc taattaattt aattagtccc tattatccct aatttaattc 1980
   atggtttggt tgatggaaga atgtaattaa atgctttgtt atccctaatt aatggtttga 2040
   ttgagcggag agtgtaatta aatgcttcat tatcccttat taatggttgt gtggaatgta 2100
   gtgtaattag aagtccctaa ttaatggttt aattgagaga agactgcaat tagatgactc 2160
   atagccccta ataagtggtt taattatgag ataatcacaa ttacatgttt cattgtccct 2220
   aattaatcgt ttaattgcga gaagatcaca attaattaaa tgatccctaa tttcaatttc 2280
   tgagaagtgt ctttctcgga aattgagata tcataagaat tcgcgatcaa tttctgagaa 2340
   ctgtcattct cggaatacct gacgtctgag gagacggtga ccagggtgcc ctggccccag 2400
   tagtcaaagt agtaatcgta agccactata tcctttcgca cagtaatata cggccgtgtc 2460
   ctcggctctc aggctgttca tttgcagata cagcgtgttc ttggaattgt ctctggagat 2520
   ggtgaaccgg cccttcacgg agtctgcgta gtatgttgct accaccacta ccactaatag 2580
   ctgagaccca ctccagcccc ttccctggag cctggcggac ccagctcatg gcatatgctg 2640
   ctaaaggtga atccagaggc tgcacaggag agtctcaggg accccccagg ctgtaccaag 2700
   cctcccccag actccaacag ctgcacctcg tgcctggtga ctcttccgca atcagtcctg 2760
   ggacagaaac ctcagggaca gcctttatga gttttgatca catctaacag agaggatggg 2820
   ccagtgagga gacggtgacc agggtgccct ggccccagta gtcaaagtag taatcgtgcc 2880
   actatatcct ttcgcacagt aatatacggc cgtgtcctcg gctctcaggc tgttcatttg 2940
   cagatacagc gtgttcttgg aattgtctct ggagatggtg aaccggccct tcacggagtc 3000
   tgcgtagtat gtgctaccac caaccactaa tagctgagac ccactccagc cccttccctg 3060
   gagcctggcg gacccagctc atggcatgct gctaaaggtg aatccagagg ctgcacagga 3120
   gagtctcagg gaccccccag gctgtaccaa gcctccccca gactccaaca gctgcacctc 3180
   gacgtcaatg ttggcgcaga agggattcat atcaattaat tccctgtcct caattaatgc 3240
   ataattggga gtgcgatgga atcattcaat aagtaatgag gacttggtgt caaaatgcca 3300
   attaagtaat taatgacacc caattaatac acaatgggga ttacagtgga ataactcagt 3360
   gaagttcata tctaaattgc aattgaataa ttaattacag ctaattaatg gatgggagac 3420
   tggcagtggt ataacttaat gaaggaacac agtgaattcc tgtctgaatt gaaattaatt 3480
   gatttattag ggctaattaa tgaatggtgg ggttaaaaat agcattactc agcgaaggaa 3540
   catgagttca tggctaaatt gagattgaat tattaattac aactaattga ttaatgactg 3600
   atgggagtaa gaatggaata actcagtgaa ggaacacaat ggattcatgg cctaattgaa 3660
   attaaatggt taattagagc taattaatga atgatggggg gtggcaatgg aatgatacaa 3720
   gaaagaaaca caatgggttc gtgtctacat tgaaattaaa taattaatta cagctaatta 3780
   ataaacggcg aggttgacaa tggtataact gtgaagagac agaatgagtt cgtgactaaa 3840
   ttggtattaa atgattaatt agagctaatt aacaggtggt ggggctgaga atggcataac 3900
   tcaatggggg atgatgagct gatggcttaa ttggaattaa taattaatca caaccaatta 3960
   acaggtgctg ttaatggggt tacaggggca tgacttcagg aagaagcaca aagtggccat 4020
   taaataatta attgcaccca attaattcct gatggggatt actgcagagg aactcagcga 4080
   aggattcaag cagaaatgac acagttaggt tgctcaattg cgttaattaa ttaaaatcgc 4140
   taattaggtg agcttcatta aggcctgatg gggttcatcc caacgctggg atcaaagaac 4200
   tcagcgcaga gtggataacg ataattagaa ggggacaaat taatgaggaa acacgacttt 4260
   gggcctagac ggccattaaa tcattaatca acccgacggg cgcccaatta atcataaagt 4320
   tattaagcaa ttaagtcgag gctgacgaga aaccgcaacg agagccctcc acgctccttt 4380
   gagttggcgc aaacaacaaa caacgcgggc tcgttaagca gcgacgacgc cgagcgtaac 4440
   gaagcaatcc ccgcgcccga caattaacgc gctaacgaag ccggagccct ccttatgcaa 4500
   attagcccct ccagagggcc ataaaagcgc cggctctccg acggcgcggg aggagcacca 4560
   gtcggctccg caaccatgag cccactcgtc tcctccctcc tgctcctggc cgccctgcca 4620
   ggtgagggcg ctgtggggct ctatggggct ctatggggtc tcagcggggc tctgcgggct 4680
   caatgggggc caaagggggg gtctgcgggc tctatggggg ggtcaacggg gggtctcacg 4740
   gggggccggc tccgcgaggc cgtgtggcgg cggctccgtc agcgctctct gggcgcgcct 4800
   ccttccccac agggctgatg gcggaggtgc agctgttgga gtctggggga ggcttggtac 4860
   agcctggggg gtccctgaga ctctcctgtg cagcctctgg attcaccttt agcagctatg 4920
   ccatgagctg ggtccgccag gctccaggga aggggctgga gtgggtctca gctattagtg 4980
   gtagtggtgg tagcacatac tacgcagact ccgtgaaggg ccggttcacc atctccagag 5040
   acaattccaa gaacacgctg tatctgcaaa tgaacagcct gagagccgag gacacggccg 5100
   tatattactg tgcgaaagga tatagtggct acgattacta ctttgactac tggggccagg 5160
   gcaccctggt caccgtctcc tcaggtgagt cttcatatga acccccccca aaattgccgc 5220
   ggcgattttg gggcaaaatc gggcgatttt atggatcctg ggtcagtcga agggggcggt 5280
   cggtccatca tttgcggccg ggtgattttt ggggccgaaa agtgggaatt tggggcccaa 5340
   tttggggccc aatttggggc ccaatttggg ttttcgaggg gggatttttt tagggggaga 5400
   tttttgggtc cccggaaaat ggtgawtttg ggtcaaaaat ggggawtttg ggggttcktt 5460
   ttraggtggg gttttttggg gkagaaatgg cggtctgagg ggaaaatgtc gttttggggc 5520
   cattttgggc catttgaggg gaaatttggg tcaaaaaatg acgattttgg gtcgttttag 5580
   ggaggaaaat gaatttaggg cgatttgagc caaatttggg tcaaaaatgg cgattttgag 5640
   tcaaaaaatg attattttgg gtcattttag ggaggaaaat gaatttaggg agattggacg 5700
   gcaaatttgg gtcaaaaaat ggtgattttg ggtcaaaaaa tgattatttt gggtcatttt 5760
   agggagaaaa atgaatttag ggagatttga gggcaaattt gggtcaaaaa atggtgattt 5820
   tgggtcaaaa aatgattatt ttgggtcatt ttagggagaa aaatgaattt agggagattt 5880
   gagggcaaat ttgggtcgaa aaatggcgat tttgggtgaa aaatggacaa ttttgggtca 5940
   ttttanggtt aaaaatgaat ttagggagat tggacggcaa atttgggtcr aaaaatggtg 6000
   atttggggts aaaaatgray wattttkggt cattttaggg ataaaaaatg aatttagggc 6060
   gatgtgagcc aattttgggt caaaaatggc gattgtgagt caaaaaatga ttattttggg 6120
   tcattttagg gttaaaaatg aatttaggga gattggacgg caaatttggg tcaaaaaatg 6180
   gtgattttcg gtcaaaaaat gattattttg ggtcatttta gggagaaaaa tgaatttagg 6240
   gagattggac ggcaaatttg ggtcgaaaaa tggtgatttt gggtcaaaaa tggacaattt 6300
   ggggtcattt tagggataaa aatgaattta gggagatttg agagcaaatt tgggtctaaa 6360
   aatgtcaatt tggggtcaaa aatggtgatt ttgggtcatt ttagggatat aaatgaattt 6420
   tgggagattt gagggcaaat ttgggtcgaa aaatggtgat ttggggtcaa tttagggagg 6480
   aaaatgaatt taaggtgatt tgacggcaaa tttgggttga aaaatgacaa tttggggtca 6540
   ttttaaagat aaaatgaatt tagggcgatt tgagggcaaa tttgggtcga agaatggtga 6600
   tttgggtcaa aaaatgacaa ttttgggtca ttttagggat ataaatggac ttagagcgat 6660
   ttgagggcaa atttggttga aaaaatggac aatttgggtc attttaggga tataaatgaa 6720
   tttagggaga tttgatggca aatttgggtc gaaaaatggt gatttgggtc aaaaatggtg 6780
   attttggttg aaaaacagtc atttggggtc attttaggga ttaaaatgaa tttcgggaga 6840
   tttgagggca aatttgggtc aaaaaatggt gattttgggt caattagtga taaaaaaatg 6900
   aatttagggt gatttgaggg caaatttggg tcaaaaaatg acaatttggg gtttttttag 6960
   ggaggagaat gaatttaggg agttttgagg gcaaatttgg gtcaaaaaat agtgatttgg 7020
   gtcaaaagtg gtgattttgg ttgaaaaaca gtcattttgg gtcattttag ggagaaaaat 7080
   gaatttcggg agatttgagg gcaaatttgg gtcaaaaaat gacaattttg ggtcacttta 7140
   cgaattaaaa tgaatttagg gagatttgag ggcaaatttg ggtcaaaata tggtgatttt 7200
   gggtcaattt agggaggaaa atgaatttag ggcgatttga aggcaaattt gggtcgaaaa 7260
   tggtgatttt gggtcaaaaa atgatgattt tgggtcgttt tagggagaaa atgaatttag 7320
   ggagatttga gggcaaattt gggtcaaaaa atggtgattt tgggtcattg tagggataaa 7380
   aatgaatttg gggcgatttg agggcaaatt tgggtggaaa atggacaatt ttgggtcatt 7440
   ttagggagaa aaatgaattt agggagattt gagggcaaat ttgggtcaaa aaatggtgat 7500
   ttggggtcat tttcagaaga aaaaatgatt attttcccca ctaaaaatgt atattttggg 7560
   gccaaatggt gaaaaatggt gatttttaat caaacgtccc caaaattggg gaaatttcat 7620
   cgatttgacc caaaattgag ttttttttcc ctgttaaaaa tgtacatttt ggggtcaatc 7680
   gctgaaatgt tcccactttt cacttctttg cccccaaatt ttgctgtccg gtgagaaatt 7740
   aattaattaa tgatcattaa ttgatcgaca attaatggtt attaattaat taataggtcc 7800
   tcttttggtg actccttcgc ttttggggcc aaaagtccat aaattggccc caaaaaatta 7860
   acgctgagta attggattcc aaagtattaa tgataaacat taaaagtgtt taattaataa 7920
   tgatattaaa cataatttcg tttttattat cgatttatca acaacgatga acgataatac 7980
   tttacaacaa tcgctaataa ttaattaatt atttaattaa ttaatttcta ataattactt 8040
   cgcattatcg gacacgagat gttgtaatta ttaataattt aattcctaat aattagaaga 8100
   ttcgttgaaa atggtactta tctttacaaa taatcacttc taataataat gattaataat 8160
   agttaataac aacaatgacg ataatgacga taatattaat aatatgtgat atatttaata 8220
   taaaattcgt attaatatat tatatctaca aaatatgata taaaatataa tattttattt 8280
   atatataaca caatttatta ttattatcat tattatcatt attaatatca tcattattaa 8340
   cgttatcgaa atacttattt agaaataata aaaacggatt taatgatggc aacaaaaata 8400
   ttttattaat gttaaaaaaa aataattaat aatttccaaa gattcgaatt cggggcaacg 8460
   aacggcactc gataattttt aattaattaa tagtttgaat taatcggtac tttttaatcc 8520
   tccattttgc ccgaaatcgc cgttttttgc cccaaattcc ccaccgcggc gttaaaaaca 8580
   taaagaaatt aagcatcaaa agtgcccttt tttggggttg ttttgaccca aaaaaaaaat 8640
   ggccgaattg ggggcggccg ttttacggtt gggttcattt tgggttcaaa acagccaaaa 8700
   atgggaactt tgggtttcga aaacaacaac aacaaaaaaa cgggtttatt ttgggctcat 8760
   tttgggtgtt tttgggtcag gaggagaaaa aataggaagt ttgagagcga aacaacggcc 8820
   gcttttgggg ggaaaacggc cctttttggt caacggcggg ggaaaaaaaa aagcggagtt 8880
   tttggggtga aaaagagcgg ttttgggtaa atttgggttt tggggtaaaa gtggaggatt 8940
   ttgggcaatg ggagttaaaa aatgggtgtt tttatggggg ttcggtgcag tttttcctgt 9000
   ttgatggggg gtttattaat ccgggggggg ggattaatga gaattaataa tgttaataga 9060
   aatatctggg aaattaatag caattattaa ttgttaatag ttattaatag ttctatatat 9120
   ctcacatcta cgatacaata taatatcgtt acaatcatat agtcgatata ttacatataa 9180
   ttatcagtaa taataataag taacaataat tagcagtaat taataataat aattaatagt 9240
   attcgttaat aagattattg ataataatta agtagtagcg attaatagag atgggatttc 9300
   gtgagaaatg gaccaaattt gggccgtttt gacccaaatt tttggtggtt ttttttccga 9360
   ttctttgtga atttcgggtc ggattcatca gcaattaatt acggttatta ggggctatta 9420
   gaggctttta attgggatta ttagagactt ttaagcggat ttggggactt ttaagtggat 9480
   tttatgattc ttaagtggat tttgggtgga ttttaccgct tttgggccgc cgcccccctc 9540
   taacccccca tctccccccc ttttctctct cccccccccc ccccaaaccc cacagcctcc 9600
   accaagggcc catcggtctt ccccctggca ccctcctcca agagcacctc tgggggcaca 9660
   gcggccctgg gctgcctggt caaggactac ttccccgaac cggtgacggt gtcgtggaac 9720
   tcaggcgccc tgaccagcgg cgtgcacacc ttcccggctg tcctacagtc ctcaggactc 9780
   tactccctca gcagcgtggt gaccgtgccc tccagcagct tgggcaccca gacctacatc 9840
   tgcaacgtga atcacaagcc cagcaacacc aaggtggaca agagagttga gcccaaatct 9900
   tgtgacaaaa ctcacacatg cccaccgtgc ccaggtaagt cactagacca gagctccact 9960
   cccgggagaa tggtaagtgc tgtaaacatc cctgcactag aggataagcc atgtacagat 10020
   ccatttccat ctctcctcat cagcacctga actcctgggg ggaccgtcag tcttcctctt 10080
   ccccccaaaa cccaaggaca ccctcatgat ctcccggacc cctgaggtca catgcgtggt 10140
   ggtggacgtg agccacgaag accctgaggt caagttcaac tggtacgtgg acggcgtgga 10200
   ggtgcataat gccaagacaa agccgcggga ggagcagtac aacagcacgt accgtgtggt 10260
   cagcgtcctc accgtcctgc accaggactg gctgaatggc aaggagtaca agtgcaaggt 10320
   ctccaacaaa gccctcccag cccccatcga gaaaaccatc tccaaagcca aaggtgagag 10380
   ctgcagcctg actgcatggg ggctgggatg ggcataagga taaaggtctg tgtggacagc 10440
   cttctgcttc agccatgacc tttgtgtatg tttctaccct cacagggcag ccccgagaac 10500
   cacaggtgta caccctgccc ccatcccggg aggagatgac caagaaccag gtcagcctga 10560
   cctgcctggt caaaggcttc tatcccagcg acatcgccgt ggagtgggag agcaatgggc 10620
   agccggagaa caactacaag accacgcctc ccgtgctgga ctccgacggc tccttcttcc 10680
   tctatagcaa gctcaccgtg gacaagagca ggtggcagca ggggaacgtc ttctcatgct 10740
   ccgtgatgca tgaggctctg cacaaccact acacgcagaa gagcctctcc ctgtccccgg 10800
   gtaaatgagc tcagcaccca caaaactctc aggtccaaag agacacccac actcatctcc 10860
   atgcttccct tgtataaata aagcacccag caatgcctgg gaccatgtaa aactgtcctg 10920
   gttctttcca aggtatagag catagctcac aggctgatat ttctggccag ggttggagga 10980
   cagccttgtc tataggaaga gaatgaggtt tttgcactgc aggactcaga gctcattagt 11040
   tatcctgcct tggagtgttg gggcttggct ttaggcagtg ccttttcctt gccttcctac 11100
   gaaccagcag ctgccataca tagagataat cctaggaagc ctcaaatgga gaaggacaca 11160
   aacccacctc cctcaggctg ttcctctatc ccggccccac ttctttacct aggggtttct 11220
   ctgagtctat tgtggagtta cacatggcca ggggcattcc agagaccctt gtcatccata 11280
   cactcaactc aggcagcttt gcacaaacaa agtctgcaca cccatacaga tggctcactc 11340
   ttgcctgtgc catgtagggc tgaggcacat ggctcttgct gccccaaggg agggactatt 11400
   agatagccac actcatgctg aatcctggcc cattcaaatt agcctgctga acaccatcca 11460
   gtccatatag cacatgtatc cacatgcacg tgtgcacaaa acgcatttaa cacactggga 11520
   caacaattct gtgccctgca cagcacctat atccagcaat gtatcaccat acacacgacc 11580
   aaaaattcaa tgcccacgtt tctgccatca caaacagaca catctttcct ctctgtggcc 11640
   actgcattat atgctcaaca caagacctct gaagccagat ccatctctgg cacctcgggg 11700
   tcatgcttca accccacatg aattatgcaa accatagcca taatggtctg aatcacttca 11760
   cactgggatg ttcccaagtt caggcaagac gagccacagg ctctgctgat gactgaagga 11820
   cagcaaaggg tcagtccagc tgtatagcca ctgttgacct gggtcacagg ccctgctgac 11880
   cctccacctt ctcctgtact gaaggaatga aagatgagac aagcatagag ggcacttgaa 11940
   taatccaggt cactctgagg tccacccaag gcattattgg actcaggtgg gaagctgaga 12000
   ctggtgtccc agagggaaag gaaggaaagc aggccccggg gagggtctgc tgtcccagtc 12060
   aggctggaga tctctcctct gaatccatgc agacatgtct gcctcacagg gaatctctcc 12120
   cagcaccaac catgttggga caaacactga ctgtcctctc tgttcagagc tgcaactgga 12180
   ggagagctgt gcggaggcgc aggacgggga gctggacggg ctgtggacga ccatcaccat 12240
   cttcatcaca ctcttcctgt taagcgtgtg ctacagtgcc accgtcacct tcttcaaggt 12300
   gaagtggatc ttctcctcgg tggtggacct gaagcagacc atcatccccg actacaggaa 12360
   catgatcgga cagggggcct aggaattaat tcgctgtctg cgagggccag ctgttggggt 12420
   gagtactccc tctcaaaagc gggcatgact tctgcgctaa gattgtcagt ttccaaaaac 12480
   gaggaggatt tgatattcac ctggcccgcg gtgatgcctt tgagggtggc cgcgtccatc 12540
   tggtcagaaa agacaatctt tttgttgtca agcttgaggt gtggcaggct tgagatctgg 12600
   ccatacactt gagtgacaat gacatccact ttgcctttct ctccacaggt gtccactccc 12660
   aggtccaact gcaggtcgag catgcatcta gggcggccaa ttggatccta ccgttcgtat 12720
   aatgtatgct atacgaagtt atcgtacgct taattaactg atccggaatt ccgccccccc 12780
   cccccccccc cctctccctc ccccccccct aacgttactg gccgaagccg cttggaataa 12840
   ggccggtgtg cgtttgtcta tatgttattt tccaccatat tgccgtcttt tggcaatgtg 12900
   agggcccgga aacctggccc tgtcttcttg acgagcattc ctaggggtct ttcccctctc 12960
   gccaaaggaa tgcaaggtct gttgaatgtc gtgaaggaag cagttcctct ggaagcttct 13020
   tgaagacaaa caacgtctgt agcgaccctt tgcaggcagc ggaacccccc acctggcgac 13080
   aggtgcctct gcggccaaaa gccacgtgta taagatacac ctgcaaaggc ggcacaaccc 13140
   cagtgccacg ttgtgagttg gatagttgtg gaaagagtca aatggctctc ctcaagcgta 13200
   ttcaacaagg ggctgaagga tgcccagaag gtaccccatt gtatgggatc tgatctgggg 13260
   cctcggtgca catgctttac atgtgtttag tcgaggttaa aaaaacgtct aggccccccg 13320
   aaccacgggg acgtggtttt cctttgaaaa acacgatgat aagcttgcca caaccatggc 13380
   tgaacaagat ggattgcacg caggttctcc ggccgcttgg gtggagaggc tattcggcta 13440
   tgactgggca caacagacaa tcggctgctc tgatgccgcc gtgttccggc tgtcagcgca 13500
   ggggcgcccg gttctttttg tcaagaccga cctgtccggt gccctgaatg aactgcagga 13560
   cgaggcagcg cggctatcgt ggctggccac gacgggcgtt ccttgcgcag ctgtgctcga 13620
   cgttgtcact gaagcgggaa gggactggct gctattgggc gaagtgccgg ggcaggatct 13680
   cctgtcatct caccttgctc ctgccgagaa agtatccatc atggctgatg caatgcggcg 13740
   gctgcatacg cttgatccgg ctacctgccc attcgaccac caagcgaaac atcgcatcga 13800
   gcgagcacgt actcggatgg aagccggtct tgtcgatcag gatgatctgg acgaagagca 13860
   tcaggggctc gcgccagccg aactgttcgc caggctcaag gcgcgcatgc ccgacggcga 13920
   ggatctcgtc gtgacccatg gcgatgcctg cttgccgaat atcatggtgg aaaatggccg 13980
   cttttctgga ttcatcgact gtggccggct gggtgtggcg gaccgctatc aggacatagc 14040
   gttggctacc cgtgatattg ctgaagagct tggcggcgaa tgggctgacc gcttcctcgt 14100
   gctttacggt atcgccgctc ccgattcgca gcgcatcgcc ttctatcgcc ttcttgacga 14160
   gttcttctga ataacttcgt ataatgtatg ctatacgaac ggtaggatcc cgcccgcccc 14220
   acgacccgca gcgcccgacc gaaaggagcg cacgacccca tggctccgac cgaagccacc 14280
   cggggcggcc ccgccgaccc cgcacccgcc cccgaggccc accgactcta gataactgat 14340
   cataatcagc cataccacat ttgtagaggt tttacttgct ttaaaaaacc tcccacacct 14400
   ccccctgaac ctgaaacata aaatgaatgc aattgttgtt gttaacttgt ttattgcagc 14460
   ttataatggt tacaaataaa gcaatagcat cacaaatttc acaaataaag catttttttc 14520
   actgtatgca aaccacctcc tggtgtcccc caacccttca tagcccccca ccccgtctcg 14580
   gtcgcccacc caaccctcca cattaacccc ttcctcccca cgctcgtccc ccgattccca 14640
   cccccctttt cgtcaccatg caccccccgt cccgcgagga cttcgaaggc cccttccgca 14700
   acgcctccat cctctgccag acccgcgggc gccgccgtcc cgccgaggtc acgtggtaca 14760
   aaaatggcag ccccgtcgcc gccgccgccg ccaccaccgc caccaccgtc ggccccgaag 14820
   tggtggccga gagccgcatc agcgtcaccg aaagcgaatg ggacaccggg gccaccttca 14880
   gctgcgtcgt ggagggggag atgaggaaca ccagcaagag gatggagtgc ggatgtaagt 14940
   ggaggggggg tttttggggg ggggatttgg ggggaaattg ggggctttgg gggcaaaaag 15000
   tccgtcattt tggtttggaa ggggtcgatg tgggggaaaa tgtgccaaaa atcccaattt 15060
   tgggggcact gaagtcgtaa tttgaggtgg aggaaggtag acagaaagag ggtggagtgc 15120
   ggatgtaagt ggagggggat ttggggcaga tttgggggaa attgggggtt tggggggcaa 15180
   ataatccgta gtttcggttt ggaaggggtt gatgtggttt tgtgctgaat ttcgggggaa 15240
   aaactgccaa aaatcccaat tttgggggca ttgaagtcat agtttgaggt tggaaggagg 15300
   tagagagcaa gaggatgaag tgaggatgta agtcggggga tttttggggc agatttgggg 15360
   gaaattgggg gttttggggt cagagaatcc gtaggtcgac cgcgccagct tttgttccct 15420
   ttagtgaggg ttaattgcgc gcttggcgta atcatggtca tagctgtttc ctgtgtgaaa 15480
   ttgttatccg ctcacaattc cacacaacat acgagccgga agcataaagt gtaaagcctg 15540
   gggtgcctaa tgagtgagct aactcacatt aattgcgttg cgctcactgc ccgctttcca 15600
   gtcgggaaac ctgtcgtgcc agctgcatta atgaatcggc caacgcgcgg ggagaggcgg 15660
   tttgcgtatt gggcgctctt ccgcttcctc gctcactgac tcgctgcgct cggtcgttcg 15720
   gctgcggcga gcggtatcag ctcactcaaa ggcggtaata cggttatcca cagaatcagg 15780
   ggataacgca ggaaagaaca tgtgagcaaa aggccagcaa aaggccagga accgtaaaaa 15840
   ggccgcgttg ctggcgtttt tccataggct ccgcccccct gacgagcatc acaaaaatcg 15900
   acgctcaagt cagaggtggc gaaacccgac aggactataa agataccagg cgtttccccc 15960
   tggaagctcc ctcgtgcgct ctcctgttcc gaccctgccg cttaccggat acctgtccgc 16020
   ctttctccct tcgggaagcg tggcgctttc tcatagctca cgctgtaggt atctcagttc 16080
   ggtgtaggtc gttcgctcca agctgggctg tgtgcacgaa ccccccgttc agcccgaccg 16140
   ctgcgcctta tccggtaact atcgtcttga gtccaacccg gtaagacacg acttatcgcc 16200
   actggcagca gccactggta acaggattag cagagcgagg tatgtaggcg gtgctacaga 16260
   gttcttgaag tggtggccta actacggcta cactagaagg acagtatttg gtatctgcgc 16320
   tctgctgaag ccagttacct tcggaaaaag agttggtagc tcttgatccg gcaaacaaac 16380
   caccgctggt agcggtggtt tttttgtttg caagcagcag attacgcgca gaaaaaaagg 16440
   atctcaagaa gatcctttga tcttttctac ggggtctgac gctcagtgga acgaaaactc 16500
   acgttaaggg attttggtca tgagattatc aaaaaggatc ttcacctaga tccttttaaa 16560
   ttaaaaatga agttttaaat caatctaaag tatatatgag taaacttggt ctgacagtta 16620
   ccaatgctta atcagtgagg cacctatctc agcgatctgt ctatttcgtt catccatagt 16680
   tgcctgactc cccgtcgtgt agataactac gatacgggag ggcttaccat ctggccccag 16740
   tgctgcaatg ataccgcgag acccacgctc accggctcca gatttatcag caataaacca 16800
   gccagccgga agggccgagc gcagaagtgg tcctgcaact ttatccgcct ccatccagtc 16860
   tattaattgt tgccgggaag ctagagtaag tagttcgcca gttaatagtt tgcgcaacgt 16920
   tgttgccatt gctacaggca tcgtggtgtc acgctcgtcg tttggtatgg cttcattcag 16980
   ctccggttcc caacgatcaa ggcgagttac atgatccccc atgttgtgca aaaaagcggt 17040
   tagctccttc ggtcctccga tcgttgtcag aagtaagttg gccgcagtgt tatcactcat 17100
   ggttatggca gcactgcata attctcttac tgtcatgcca tccgtaagat gcttttctgt 17160
   gactggtgag tactcaacca agtcattctg agaatagtgt atgcggcgac cgagttgctc 17220
   ttgcccggcg tcaatacggg ataataccgc gccacatagc agaactttaa aagtgctcat 17280
   cattggaaaa cgttcttcgg ggcgaaaact ctcaaggatc ttaccgctgt tgagatccag 17340
   ttcgatgtaa cccactcgtg cacccaactg atcttcagca tcttttactt tcaccagcgt 17400
   ttctgggtga gcaaaaacag gaaggcaaaa tgccgcaaaa aagggaataa gggcgacacg 17460
   gaaatgttga atactcatac tcttcctttt tcaatattat tgaagcattt atcagggtta 17520
   ttgtctcatg agcggataca tatttgaatg tatttagaaa aataaacaaa taggggttcc 17580
   gcgcacattt ccccgaaaag tgccac 17606

<210> 44
<211> 1032
<212> DNA
<213> Bacteriophage P1

<400> 44
atgtccaatt tactgaccgt acaccaaaat ttgcctgcat taccggtcga tgcaacgagt 60

gatgaggttc gcaagaacct gatggacatg ttcagggatc gccaggcgtt ttctgagcat 120

acctggaaaa tgcttctgtc cgtttgccgg tcgtgggcgg catggtgcaa gttgaataac 180

cggaaatggt ttcccgcaga acctgaagat gttcgcgatt atcttctata tcttcaggcg 240

cgcggtctgg cagtaaaaac tatccagcaa catttgggcc agctaaacat gcttcatcgt 300

cggtccgggc tgccacgacc aagtgacagc aatgctgttt cactggttat gcggcggatc 360

cgaaaagaaa acgttgatgc cggtgaacgt gcaaaacagg ctctagcgtt cgaacgcact 420

gatttcgacc aggttcgttc actcatggaa aatagcgatc gctgccagga tatacgtaat 480

ctggcatttc tggggattgc ttataacacc ctgttacgta tagccgaaat tgccaggatc 540

agggttaaag atatctcacg tactgacggt gggagaatgt taatccatat tggcagaacg 600

aaaacgctgg ttagcaccgc aggtgtagag aaggcactta gcctgggggt aactaaactg 660

gtcgagcgat ggatttccgt ctctggtgta gctgatgatc cgaataacta cctgttttgc 720

cgggtcagaa aaaatggtgt tgccgcgcca tctgccacca gccagctatc aactcgcgcc 780

ctggaaggga tttttgaagc aactcatcga ttgatttacg gcgctaagga tgactctggt 840

cagagatacc tggcctggtc tggacacagt gcccgtgtcg gagccgcgcg agatatggcc 900

cgcgctggag tttcaatacc ggagatcatg caagctggtg gctggaccaa tgtaaatatt 960

gtcatgaact atatccgtaa cctggatagt gaaacagggg caatggtgcg cctgctggaa 1020

gatggcgatt ag 1032

<210> 45
<211> 5739
<212> DNA
<213> Artificial Sequence

<220>
<223> Expression vector

<400> 45
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggtcgcca ccatggtgag caagggcgag gagctgttca ccggggtggt gcccatcctg 660

gtcgagctgg acggcgacgt aaacggccac aagttcagcg tgtccggcga gggcgagggc 720

gatgccacct acggcaagct gaccctgaag ttcatctgca ccaccggcaa gctgcccgtg 780

ccctggccca ccctcgtgac caccctgacc tacggcgtgc agtgcttcag ccgctacccc 840

gaccacatga agcagcacga cttcttcaag tccgccatgc ccgaaggcta cgtccaggag 900

cgcaccatct tcttcaagga cgacggcaac tacaagaccc gcgccgaggt gaagttcgag 960

ggcgacaccc tggtgaaccg catcgagctg aagggcatcg acttcaagga ggacggcaac 1020

atcctggggc acaagctgga gtacaactac aacagccaca acgtctatat catggccgac 1080

aagcagaaga acggcatcaa ggtgaacttc aagatccgcc acaacatcga ggacggcagc 1140

gtgcagctcg ccgaccacta ccagcagaac acccccatcg gcgacggccc cgtgctgctg 1200

cccgacaacc actacctgag cacccagtcc gccctgagca aagaccccaa cgagaagcgc 1260

gatcacatgg tcctgctgga gttcgtgacc gccgccggga tcactctcgg catggacgag 1320

ctgtacaagt ccggactcag atctatgtcc aatttactga ccgtacacca aaatttgcct 1380

gcattaccgg tcgatgcaac gagtgatgag gttcgcaaga acctgatgga catgttcagg 1440

gatcgccagg cgttttctga gcatacctgg aaaatgcttc tgtccgtttg ccggtcgtgg 1500

gcggcatggt gcaagttgaa taaccggaaa tggtttcccg cagaacctga agatgttcgc 1560

gattatcttc tatatcttca ggcgcgcggt ctggcagtaa aaactatcca gcaacatttg 1620

ggccagctaa acatgcttca tcgtcggtcc gggctgccac gaccaagtga cagcaatgct 1680

gtttcactgg ttatgcggcg gatccgaaaa gaaaacgttg atgccggtga acgtgcaaaa 1740

caggctctag cgttcgaacg cactgatttc gaccaggttc gttcactcat ggaaaatagc 1800

gatcgctgcc aggatatacg taatctggca tttctgggga ttgcttataa caccctgtta 1860

cgtatagccg aaattgccag gatcagggtt aaagatatct cacgtactga cggtgggaga 1920

atgttaatcc atattggcag aacgaaaacg ctggttagca ccgcaggtgt agagaaggca 1980

cttagcctgg gggtaactaa actggtcgag cgatggattt ccgtctctgg tgtagctgat 2040

gatccgaata actacctgtt ttgccgggtc agaaaaaatg gtgttgccgc gccatctgcc 2100

accagccagc tatcaactcg cgccctggaa gggatttttg aagcaactca tcgattgatt 2160

tacggcgcta aggatgactc tggtcagaga tacctggcct ggtctggaca cagtgcccgt 2220

gtcggagccg cgcgagatat ggcccgcgct ggagtttcaa taccggagat catgcaagct 2280

ggtggctgga ccaatgtaaa tattgtcatg aactatatcc gtaacctgga tagtgaaaca 2340

ggggcaatgg tgcgcctgct ggaagatggc gattaggtcg acggtaccgc gggcccggga 2400

tccaccggat ctagataact gatcataatc agccatacca catttgtaga ggttttactt 2460

gctttaaaaa acctcccaca cctccccctg aacctgaaac ataaaatgaa tgcaattgtt 2520

gttgttaact tgtttattgc agcttataat ggttacaaat aaagcaatag catcacaaat 2580

ttcacaaata aagcattttt ttcactgcat tctagttgtg gtttgtccaa actcatcaat 2640

gtatcttaac gcgtaaattg taagcgttaa tattttgtta aaattcgcgt taaatttttg 2700

ttaaatcagc tcatttttta accaataggc cgaaatcggc aaaatccctt ataaatcaaa 2760

agaatagacc gagatagggt tgagtgttgt tccagtttgg aacaagagtc cactattaaa 2820

gaacgtggac tccaacgtca aagggcgaaa aaccgtctat cagggcgatg gcccactacg 2880

tgaaccatca ccctaatcaa gttttttggg gtcgaggtgc cgtaaagcac taaatcggaa 2940

ccctaaaggg agcccccgat ttagagcttg acggggaaag ccggcgaacg tggcgagaaa 3000

ggaagggaag aaagcgaaag gagcgggcgc tagggcgctg gcaagtgtag cggtcacgct 3060

gcgcgtaacc accacacccg ccgcgcttaa tgcgccgcta cagggcgcgt caggtggcac 3120

ttttcgggga aatgtgcgcg gaacccctat ttgtttattt ttctaaatac attcaaatat 3180

gtatccgctc atgagacaat aaccctgata aatgcttcaa taatattgaa aaaggaagag 3240

tcctgaggcg gaaagaacca gctgtggaat gtgtgtcagt tagggtgtgg aaagtcccca 3300

ggctccccag caggcagaag tatgcaaagc atgcatctca attagtcagc aaccaggtgt 3360

ggaaagtccc caggctcccc agcaggcaga agtatgcaaa gcatgcatct caattagtca 3420

gcaaccatag tcccgcccct aactccgccc atcccgcccc taactccgcc cagttccgcc 3480

cattctccgc cccatggctg actaattttt tttatttatg cagaggccga ggccgcctcg 3540

gcctctgagc tattccagaa gtagtgagga ggcttttttg gaggcctagg cttttgcaaa 3600

gatcgatcaa gagacaggat gaggatcgtt tcgcatgatt gaacaagatg gattgcacgc 3660

aggttctccg gccgcttggg tggagaggct attcggctat gactgggcac aacagacaat 3720

cggctgctct gatgccgccg tgttccggct gtcagcgcag gggcgcccgg ttctttttgt 3780

caagaccgac ctgtccggtg ccctgaatga actgcaagac gaggcagcgc ggctatcgtg 3840

gctggccacg acgggcgttc cttgcgcagc tgtgctcgac gttgtcactg aagcgggaag 3900

ggactggctg ctattgggcg aagtgccggg gcaggatctc ctgtcatctc accttgctcc 3960

tgccgagaaa gtatccatca tggctgatgc aatgcggcgg ctgcatacgc ttgatccggc 4020

tacctgccca ttcgaccacc aagcgaaaca tcgcatcgag cgagcacgta ctcggatgga 4080

agccggtctt gtcgatcagg atgatctgga cgaagagcat caggggctcg cgccagccga 4140

actgttcgcc aggctcaagg cgagcatgcc cgacggcgag gatctcgtcg tgacccatgg 4200

cgatgcctgc ttgccgaata tcatggtgga aaatggccgc ttttctggat tcatcgactg 4260

tggccggctg ggtgtggcgg accgctatca ggacatagcg ttggctaccc gtgatattgc 4320

tgaagagctt ggcggcgaat gggctgaccg cttcctcgtg ctttacggta tcgccgctcc 4380

cgattcgcag cgcatcgcct tctatcgcct tcttgacgag ttcttctgag cgggactctg 4440

gggttcgaaa tgaccgacca agcgacgccc aacctgccat cacgagattt cgattccacc 4500

gccgccttct atgaaaggtt gggcttcgga atcgttttcc gggacgccgg ctggatgatc 4560

ctccagcgcg gggatctcat gctggagttc ttcgcccacc ctagggggag gctaactgaa 4620

acacggaagg agacaatacc ggaaggaacc cgcgctatga cggcaataaa aagacagaat 4680

aaaacgcacg gtgttgggtc gtttgttcat aaacgcgggg ttcggtccca gggctggcac 4740

tctgtcgata ccccaccgag accccattgg ggccaatacg cccgcgtttc ttccttttcc 4800

ccaccccacc ccccaagttc gggtgaaggc ccagggctcg cagccaacgt cggggcggca 4860

ggccctgcca tagcctcagg ttactcatat atactttaga ttgatttaaa acttcatttt 4920

taatttaaaa ggatctaggt gaagatcctt tttgataatc tcatgaccaa aatcccttaa 4980

cgtgagtttt cgttccactg agcgtcagac cccgtagaaa agatcaaagg atcttcttga 5040

gatccttttt ttctgcgcgt aatctgctgc ttgcaaacaa aaaaaccacc gctaccagcg 5100

gtggtttgtt tgccggatca agagctacca actctttttc cgaaggtaac tggcttcagc 5160

agagcgcaga taccaaatac tgtccttcta gtgtagccgt agttaggcca ccacttcaag 5220

aactctgtag caccgcctac atacctcgct ctgctaatcc tgttaccagt ggctgctgcc 5280

agtggcgata agtcgtgtct taccgggttg gactcaagac gatagttacc ggataaggcg 5340

cagcggtcgg gctgaacggg gggttcgtgc acacagccca gcttggagcg aacgacctac 5400

accgaactga gatacctaca gcgtgagcta tgagaaagcg ccacgcttcc cgaagggaga 5460

aaggcggaca ggtatccggt aagcggcagg gtcggaacag gagagcgcac gagggagctt 5520

ccagggggaa acgcctggta tctttatagt cctgtcgggt ttcgccacct ctgacttgag 5580

cgtcgatttt tgtgatgctc gtcagggggg cggagcctat ggaaaaacgc cagcaacgcg 5640

gcctttttac ggttcctggc cttttgctgg ccttttgctc acatgttctt tcctgcgtta 5700

tcccctgatt ctgtggataa ccgtattacc gccatgcat 5739

<210> 46
<211> 54
<212> DNA
<213> Artificial Sequence

<220>
<223> Synthetic Primer


<220>
<221> misc_feature
<222> (26)..(35)
<223> n is a, g, c or t.

<400> 46
cgtatcgcct ccctcgcgcc atcagnnnnn nnnnncaggt tccctggtgc aggc 54

<210>  47
<211>  44
<212>  DNA
<213>  Artificial Sequence

<220>
<223>  Synthetic Primer

<400>  47
ctatgcgcct tgccagcccg ctcaggcttg gtccctccgc cgaa                    44


<210>  48
<211>  41
<212>  DNA
<213>  Artificial Sequence

<220>
<223>  Synthetic Primer

<400>  48
ctatgcgcct tgccagcccg ctcagtccgt cagcgctctc t                       41


<210>  49
<211>  54
<212>  DNA
<213>  Artificial Sequence

<220>
<223>  Synthetic Primer


<220>
<221>  misc_feature
<222>  (26)..(35)
<223>  n is a, g, c or t.

<400>  49
cgtatcgcct ccctcgcgcc atcagnnnnn nnnnncggtg accagggtgc cctg          54


<210>  50
<211>  2503
<212>  DNA
<213>  Homo sapiens

<400>  50
tcgccccctc gacacgagtg cgtacgctcg acaagacgca ctccagtctg ccctgactca      60

gcctgcctcc gtgtctgggt ctcctggaca gtcgatcacc atctcctgct ctggaagcag     120

ctccaacatc ggaagtaatt atgtatactg gtaccaacag cacccaggca aagcccccaa     180

actcatgatt tataggaata atcagcggcc ctcaggggtt tctaatcgct tctctggctc     240

caagtctggc aacacggcct ccctgaccat ctctgggctc caggctgagg acgaggctga     300

ttattactgc gcagcatggg atgacagcct gagtggtcat tgggtgttcg gcggagggac     360

caagctgacc gtcctagcct gacacacaca gccctcccca gactgtggga tgaggccctg     420

tgcccgcagt cacatgtgga atatcaagac acacacatct atgacaatca cagcactgta 480

gatcagacac gatatcgcga gcagtctgcc ctgactcagc ctgcctccgt gtctgggtct 540

cctggacagt cgatcaccat ctcctgctct ggaagcagct ccaacatcgg aaataatgct 600

gtaaactggt accaacagca cccaggcaaa gcccccaaac tcatgattta ttatgatgat 660

ctgctgccct caggggtttc taatcgcttc tctggctcca agtctggcaa cacggcctcc 720

ctgaccatct ctgggctcca ggctgaggac gaggctgatt attactgcgg aacatgggat 780

ggcagcctga atggtgggag gctgttcggc ggagggacca agctgaccgt cctagcctga 840

cacacacagc cctccccaga ctgtgggatg aggccctgtg cccgcagtca catgtggaat 900

atcaagacac acacatctat gacaatcacc gtgtctctac tcgcgtgtct ctctatgcgc 960

tcgaggtcga cggtatcgat aagcttgata tcgaattcct gcagcccggg ggatcttacg 1020

ctgtcttagt gtagattcga tcacgtcagt ctgccctgac tcagcctgcc tccgtgtctg 1080

ggtctcctgg acagtcgatc accatctcct gctctggaga agcattgcca aaaaaatatg 1140

cttattggta ccaacagcac ccaggcaaag cccccaaact catgatttat aaagacagcg 1200

agaggccctc aggggtttct aatcgcttct ctggctccaa gtctggcaac acggcctccc 1260

tgaccatctc tgggctccag gctgaggacg aggctgatta ttactgcaac tcttttacaa 1320

gcaccaccaa ttatgtctta ttcggcggag ggaccaagct gaccgtccta gcctgacaca 1380

cacagccctc cccagactgt gggatgaggc cctgtgcccg cagtcacatg tggaatatca 1440

agacacacac atctatgaca atcactcgca ctagttctag cgacttctat actatggatc 1500

cactagacgt acagtcacgt actcagacta cgctctacag tctgccctga ctcagcctgc 1560

ctccgtgtct gggtctcctg gacagtcgat caccatctcc tgcaccctaa gcagtgagca 1620

cagcacctac accatcgaat ggtaccaaca gcacccaggc aaagccccca aactcatgat 1680

ttatgttaag agtgatggca gccacagcaa gggggacggg gtttctaatc gcttctctgg 1740

ctccaagtct ggcaacacgg cctccctgac catctctggg ctccaggctg aggacgaggc 1800

tgattattac tgcggagaga gccacacgat tgatggccaa gtcggtgtgg tattcggcgg 1860

agggaccaag ctgaccgtcc tagcctgaca cacacagccc tccccagact gtgggatgag 1920

gccctgtgcc cgcagtcaca tgtggaatat caagacacac acatctatga caatcaccta 1980

tagcgtatac gtcatcatag tcgcataact agacagtctg ccctgactca gcctgcctcc 2040

gtgtctgggt ctcctggaca gtcgatcacc atctcctgca ctctgagcag tgggcacagt 2100

agctacatca tcgcatggta ccaacagcac ccaggcaaag cccccaaact catgatttat 2160

cttgaaggta gtggaagcta caacaagggg agcggggttt ctaatcgctt ctctggctcc 2220

aagtctggca acacggcctc cctgaccatc tctgggctcc aggctgagga cgaggctgat 2280

tattactgcg agacctggga cagtaacact catgtggtat tcggcggagg gaccaagctg 2340

accgtcctag cctgacacac acagccctcc ccagactgtg ggatgaggcc ctgtgcccgc 2400

agtcacatgt ggaatatcaa gacacacaca tctatgacaa tcactatata tacatatgct 2460

agtatcacgc gagaactagt tctagagcgg ccgccaccgc cga 2503

<210> 51
<211> 12801
<212> DNA
<213> Artificial Sequence

<220>
<223> Targeting vector

<400> 51
agcgcccaat acgcaaaccg cctctccccg cgcgttggcc gattcattaa tgcagctggc 60

acgacaggtt tcccgactgg aaagcgggca gtgagcgcaa cgcaattaat gtgagttagc 120

tcactcatta ggcaccccag gctttacact ttatgcttcc ggctcgtatg ttgtgtggaa 180

ttgtgagcgg ataacaattt cacacaggaa acagctatga ccatgattac gccaagctca 240

gaattaaccc tcactaaagg gactagtcct gcaggtttaa acgaattcgc ccttgtcgac 300

gggtatcacg tggggcaaat cgagagctac cattggctat ggggaggaga aaatgaggcg 360

tgctacttcc tcgttcctgc agtgcgtgtc gaggtgccgt gttggtcgga tctcagtggc 420

agagcaggat ggtgagcggt ggccttgggg ccgctccctc actctgctgg gctggtgcgg 480

tggctccggg accggacttt cagtggtgtt tggtgtggtg ctcactgtcc tttggtgtca 540

ctgtgtggtg aattccctca gctgggcggt gagagcccat gctgtgtcct ggtaggagta 600

gtctgggctg cagtgcgggc tctttgtgcg tgttgggggc ggttccctgg aagctaattc 660

tggaagaaat cccggctgcc tctgtaacag ttgttggcga tgtcaaacac cctggagctg 720

ctgactggct ctgtaccatt gagtgatgca gtttaaacct ctctctttcc tcctgctgtg 780

tgtgaagctg gtgttagtgc taggagacct gcacatccct caccgatgca gtggtttacc 840

agcaaagttc aagagcctcc tggttccagg gaagattcag catattttgt gtacaggaaa 900

cctctgcacc aaggagagct atgactacct caggactttg gctggagatg ttcacgttgt 960

taagggggac tctgaggtaa tgttcttgct gctgtgaatt cctcagtcct ccatcttaca 1020

agccgtagtt gtcccactga aagctgggaa acccttcggt gtttgcaatt cctgagcgct 1080

aatgtgtggg ggaaggatgt aggaacaaat aaggctcaag agacaacaac ttcctaggct 1140

cctcttattg cccttgtggt aagggaggac ttggtgtgta gtggtaatac atgcactcaa 1200

cttattaagt tctcatagct gctgtgaagc tgctgcagtc cctcagatcc ctgcaggaag 1260

aggggactga ctgactgttg aatagccttg aagctcctgc tctgcatgtg gttccggtca 1320

ggctcaagat cccagttctt agctcttcag ttgctcttct gggggtatag tactagcatc 1380

tgggtcactg cagatggaca gagactgtgc ttgggtagca aaaggcagac ttacgttgtg 1440

aaaccttcat tgtgttggtg aggttctttg atttctacct tgtgtatctc tcttcctcag 1500

agtctgaatt atcctgaaca gaaggttgta actgttgggc agttcagaat tgggttgatt 1560

catggccatc aagtcattcc ctggggtgat gtggcaagct tggcattgct gcagaggcag 1620

ctggatgtag acattcttat ctcaggacat acacacagat ttgaagcatt tgagcatgaa 1680

aataagttct acatcaaccc aggatcagct acaggagcct acactggttt acaaacgtga 1740

gtacaatgag ccttgtgttt gtctttaaga cagatgtctg tccttaagag tgcagcccat 1800

gtgttgaaga gcactggtga gatgcagtgc catgcaggcc atctctccct ttcccagaga 1860

acacggccta cctctctctg ttcataaggt cttggttaac ttatgagttg gccagcacag 1920

cctggcactg atgtgtcatg atcataaccc tgagcccacg ctctgctatg ctgttgcttc 1980

ttagagttgc attgccttga ggtgtattcc agaagctgtg tgctgagctc ttgttgggaa 2040

agaggccctc agggcactgc caacatctga ggcttgcagg agctggaaca ttcagccctg 2100

agggctgctc tggccaactg cagctctgag tctgcagtca gtgcctctga gctgtgatgc 2160

ttctcagata actgcagccc tttgcaatgt aatgttttat aatatatttc cataggacat 2220

cttggtaaga ggtgtaagct aatcagggtg attgacaaag tatgttttct ttccaggaac 2280

gtcattcctt catttgtact gatggatatc caggcttcca cagttgtgac ttatgtatac 2340

cagctaattg aggacgatgt gaaagtagaa agaattgagt tcaagaaata ctgacacgtg 2400

tttgaatctg cctgctgact tctcactgcc tctcattctt tctctcctag ttaaagctga 2460

atccggccac gtgggggtgc tgctgcagcg gcgtgtaact gtgagcttgg gaagttctca 2520

actagggctt agagcaggag aaactgatta acagaaattg ccttattaaa tgtatatatt 2580

tcctgtgtct aagaacgagg tgctgttgtt cctgacttgc ggagctgcag ctagtggtag 2640

cttggaattt aacctctcct gtaaaatgag catagaataa aacagtattt tcacatttct 2700

ggatcctgtt aattgtttgt taattaagag gacaggaatt cagtgtcagg gggctgctct 2760

ttgcttccca atgtgtacca ctgaagcctc tctggattta tcagagatac tcaatggact 2820

cttaacagca ctgtggcacc tgacctcctg ctgccttcag cctcccttcc tggctggtgt 2880

gcttatgttg aggagctgtg ggaggctggt gcagttctgt gggatgaggt aaggccagct 2940

gaatagcctg gggctggtc aggggcttta gtgagaggag cagggctggg gaatgttgtg 3000

aacagcaggt ggtgccactg ctattctgag gaaggaggca gcttctgcaa atgctttgca 3060

atgcaggatg acaaagtgct tctgctagcc ctagtcaatt tctgagaact gtcattctcg 3120

gaatacctcg atcgataact tcgtataatg tatactatac gaagttattt tatttgtgaa 3180

atttgtgatg ctattgcttt atttgtaacc attataagct gcaataaaca agttggggtg 3240

ggcgaagaac tccagcatga gatccccgcg ctggaggatc atccagccgg cgtcccggaa 3300

aacgattccg aagcccaacc tttcatagaa ggcggcggtg gaatcgaaat ctcgtgatgg 3360

caggttgggc gtcgcttggt cggtcatttc gaaccccaga gtcccgctca gaagaactcg 3420

tcaagaaggc gatagaaggc gatgcgctgc gaatcgggag cggcgatacc gtaaagcacg 3480

aggaagcggt cagcccattc gccgccaagc tcttcagcaa tatcacgggt agccaacgct 3540

atgtcctgat agcggtccgc cacacccagc cggccacagt cgatgaatcc agaaaagcgg 3600

ccattttcca ccatgatatt cggcaagcag gcatcgccat gggtcacgac gagatcctcg 3660

ccgtcgggca tgcgcgcctt gagcctggcg aacagttcgg ctggcgcgag cccctgatgc 3720

tcttcgtcca gatcatcctg atcgacaaga ccggcttcca tccgagtacg tgctcgctcg 3780

atgcgatgtt tcgcttggtg gtcgaatggg caggtagccg gatcaagcgt atgcagccgc 3840

cgcattgcat cagccatgat ggatactttc tcggcaggag caaggtgaga tgacaggaga 3900

tcctgccccg gcacttcgcc caatagcagc cagtcccttc ccgcttcagt gacaacgtcg 3960

agcacagctg cgcaaggaac gcccgtcgtg gccagccacg atagccgcgc tgcctcgtcc 4020

tgcagttcat tcagggcacc ggacaggtcg gtcttgacaa aaagaaccgg gcgcccctgc 4080

gctgacagcc ggaacacggc ggcatcagag cagccgattg tctgttgtgc ccagtcatag 4140

ccgaatagcc tctccaccca agcggccgga gaacctgcgt gcaatccatc ttgttcaatc 4200

atgcgaaacg atcctcatcc tgtctcttga tcagatccga aaatggatat acaagctccc 4260

gggagctttt tgcaaaagcc taggcctcca aaaaagcctc ctcactactt ctggaatagc 4320

tcagaggcag aggcggcctc ggcctctgca taaataaaaa aaattagtca gccatggggc 4380

ggagaatggg cggaactggg cggagttagg ggcgggatgg gcggagttag gggcgggact 4440

atggttgctg actaattgag atgcatgctt tgcatacttc tgcctgctgg ggagcctggg 4500

gactttccac acctggttgc tgactaattg agatgcatgc tttgcatact tctgcctgct 4560

ggggagcctg gggactttcc acaccctaaa taacttcgta taatgtatac tatacgaagt 4620

tatctcgtgt ccgataactg taattatcgg accgtcaatc gattcgcccc ctcgacacga 4680

gtgcgtacgc tcgacaagac gcactccagt ctgccctgac tcagcctgcc tccgtgtctg 4740

ggtctcctgg acagtcgatc accatctcct gctctggaag cagctccaac atcggaagta 4800

attatgtata ctggtaccaa cagcacccag gcaaagcccc caaactcatg atttatagga 4860

ataatcagcg gccctcaggg gtttctaatc gcttctctgg ctccaagtct ggcaacacgg 4920

cctccctgac catctctggg ctccaggctg aggacgaggc tgattattac tgcgcagcat 4980

gggatgacag cctgagtggt cattgggtgt tcggcggagg gaccaagctg accgtcctag 5040

cctgacacac acagccctcc ccagactgtg ggatgaggcc ctgtgcccgc agtcacatgt 5100

ggaatatcaa gacacacaca tctatgacaa tcacagcact gtagatcaga cacgatatcg 5160

cgagcagtct gccctgactc agcctgcctc cgtgtctggg tctcctggac agtcgatcac 5220
catctcctgc tctggaagca gctccaacat cggaaataat gctgtaaact ggtaccaaca 5280
gcacccaggc aaagccccca aactcatgat ttattatgat gatctgctgc cctcaggggt 5340
ttctaatcgc ttctctggct ccaagtctgg caacacggcc tccctgacca tctctgggct 5400
ccaggctgag gacgaggctg attattactg cggaacatgg gatggcagcc tgaatggtgg 5460
gaggctgttc ggcggaggga ccaagctgac cgtcctagcc tgacacacac agccctcccc 5520
agactgtggg atgaggccct gtgcccgcag tcacatgtgg aatatcaaga cacacacatc 5580
tatgacaatc accgtgtctc tactcgcgtg tctctctatg cgctcgaggt cgacggtatc 5640
gataagcttg atatcgaatt cctgcagccc gggggatctt acgctgtctt agtgtagatt 5700
cgatcacgtc agtctgccct gactcagcct gcctccgtgt ctgggtctcc tggacagtcg 5760
atcaccatct cctgctctgg agaagcattg ccaaaaaaat atgcttattg gtaccaacag 5820
cacccaggca aagcccccaa actcatgatt tataaagaca gcgagaggcc ctcaggggtt 5880
tctaatcgct tctctggctc caagtctggc aacacggcct ccctgaccat ctctgggctc 5940
caggctgagg acgaggctga ttattactgc aactctttta caagcaccac caattatgtc 6000
ttattcggcg gagggaccaa gctgaccgtc ctagcctgac acacacagcc ctccccagac 6060
tgtgggatga ggccctgtgc ccgcagtcac atgtggaata tcaagacaca cacatctatg 6120
acaatcactc gcactagttc tagcgacttc tatactatgg atccactaga cgtacagtca 6180
cgtactcaga ctacgctcta cagtctgccc tgactcagcc tgcctccgtg tctgggtctc 6240
ctggacagtc gatcaccatc tcctgcaccc taagcagtga gcacagcacc tacaccatcg 6300
aatggtacca acagcaccca ggcaaagccc ccaaactcat gatttatgtt aagagtgatg 6360
gcagccacag caagggggac ggggtttcta atcgcttctc tggctccaag tctggcaaca 6420
cggcctccct gaccatctct gggctccagg ctgaggacga ggctgattat tactgcggag 6480
agagccacac gattgatggc caagtcggtg tggtattcgg cggagggacc aagctgaccg 6540
tcctagcctg acacacacag ccctccccag actgtgggat gaggccctgt gcccgcagtc 6600
acatgtggaa tatcaagaca cacacatcta tgacaatcac ctatagcgta tacgtcatca 6660
tagtcgcata actagacagt ctgccctgac tcagcctgcc tccgtgtctg ggtctcctgg 6720
acagtcgatc accatctcct gcactctgag cagtgggcac agtagctaca tcatcgcatg 6780
gtaccaacag cacccaggca aagcccccaa actcatgatt tatcttgaag gtagtggaag 6840
ctacaacaag gggagcgggg tttctaatcg cttctctggc tccaagtctg gcaacacggc 6900
ctccctgacc atctctgggc tccaggctga ggacgaggct gattattact gcgagacctg 6960
ggacagtaac actcatgtgg tattcggcgg agggaccaag ctgaccgtcc tagcctgaca 7020
cacacagccc tccccagact gtgggatgag gccctgtgcc cgcagtcaca tgtggaatat 7080

caagacacac acatctatga caatcactat atatacatat gctagtatca cgcgagaact 7140

agttctagag cggccgccac cgccgaggcg cgccatattg cctccctgtg ttgctgttgc 7200

agggttgctt ctgcctcata ctggtttctc ccttctggag gtggccaaaa gccgggccct 7260

gtgcaaccct ggtgcataaa taccttatgg cccctaagta gggcaggtgt gggacacgct 7320

ctggcacctg gggtgtgtgc aagtgctcag gaagacctgc aggcacaggt ggcagtgggg 7380

gtctctggct gtgctcgagc agcagctgcc tggggtaagg gtagtactct gtgcatgaac 7440

aatgctgcag ggctcagctc tgctcagacc acgaccctgg caccaacaga gacctgcctg 7500

gctctgtggt catgtaaacc tttacaggag ctcaagacaa ggctgtttat tactgctctg 7560

gcaggaaaga agcactggcc atggtcatag agagttccag caacaggaaa gtgagagccc 7620

aagctgctga ggtaccaggg ctcctcaggt gcctgctgca gcagcttgga cacagtcgag 7680

gaacagcaat tgtacctgtg tggtggatca ggctgtgctg cctgtgaacc tattctagca 7740

catctgtcac ctctgtgcca ctcacaggga taccacccct gagaccccta ccccatcagc 7800

ctctgtgtgg gatatggtgt tggcccaagg ggctctgttg cacagggaga tagaggcctg 7860

gggaggaggg aaagcattga ggtggtgttg ataccaggga tgtgagccca agcaagagat 7920

cagcagagca aggaggaaga attgcaggtg ttggggctgg ggaaagcccc agatggctgg 7980

agctggtggg gccactggag atctcctcct cccatcctgc tccatgctgg ggcagctgct 8040

gcaggctgac cagggcctgc ccgggcacgt tgtgaaggtc accaaggatg gagacttcag 8100

agctatctgg gacacgtgtt ttaacgtttg atcagccttg cagcaaaaca gtgttttgct 8160

ctattgaaca gtgttcaaca aaaacgatca gtggcctgga acacatctcc tacgaagaaa 8220

ggttgcgaga gctgggtctg gagaagagaa ggctctgggg caacctccct gtggcctgtc 8280

agtgcttaaa ggaggctcaa gagaaagatg gagagagact ttatacacgg agctgtagca 8340

atatgacaag gagaaatggt ttaaaatgga aagagggtag atttaggtta gctatcaaga 8400

agaaattctt cactcagagg tgatgaagca ctgtagcagg ttgcccagag aagtattgga 8460

tgcttccttc ctggcagggc ccaaggccag actggatggg ttctgggcag cctgttctgg 8520

tgggagatgt ccctgcctgt ggcatggctt ggaaatgggt agcctttgag gtccctctca 8580

gcccaaatca ttctatgttc ttgtggttcc ctgcatttca gcctttgcct gttgactctg 8640

gtgccactgt tggacaattc tactcagctg cgtttcatcc ctgccccaaa gcagtaatcc 8700

catggataag caatgctctg aggccttcac caccctcagg gctcctgctg accctagcgg 8760

cctccctggc agcatgagcc ctttgttgtc cgcactgctg ctgccgaggg tcctgccagc 8820

cccccagggc cttttctgcc caggcactgc cacctgctaa aacccagcct gcccatggca 8880

gggcatcgca tgtctgaggg atggaacggg gcaagggcag tgctgcaggc agtggttcaa 8940

gaaacgttta gatgttgtac tgagggatgt ggtttagtgg gaaatactgg tgataggtgg 9000

atggttggac tggaggatct tagaggtctt ttccaacctt ggtgattatg tgattctatg 9060

aagggagcca tagcctgctc ccatcatgcc ccgccagtcc ggctctcccg agcactttgg 9120

ttccgcagca tgcggccgca agggcgaatt cgcggccgct aaattcaatt cgccctatag 9180

tgagtcgtat tacaattcac tggccgtcgt tttacaacgt cgtgactggg aaaaccctgg 9240

cgttacccaa cttaatcgcc ttgcagcaca tccccctttc gccagctggc gtaatagcga 9300

agaggcccgc accgatcgcc cttcccaaca gttgcgcagc ctatacgtac ggcagtttaa 9360

ggtttacacc tataaaagag agagccgtta tcgtctgttt gtggatgtac agagtgatat 9420

tattgacacg ccggggcgac ggatggtgat ccccctggcc agtgcacgtc tgctgtcaga 9480

taaagtctcc cgtgaacttt acccggtggt gcatatcggg gatgaaagct ggcgcatgat 9540

gaccaccgat atggccagtg tgccggtctc cgttatcggg gaagaagtgg ctgatctcag 9600

ccaccgcgaa aatgacatca aaaacgccat taacctgatg ttctggggaa tataaatgtc 9660

aggcatgaga ttatcaaaaa ggatcttcac ctagatcctt ttcacgtaga aagccagtcc 9720

gcagaaacgg tgctgacccc ggatgaatgt cagctactgg gctatctgga caagggaaaa 9780

cgcaagcgca aagagaaagc aggtagcttg cagtgggctt acatggcgat agctagactg 9840

ggcggtttta tggacagcaa gcgaaccgga attgccagct ggggcgccct ctggtaaggt 9900

tgggaagccc tgcaaagtaa actggatggc tttcttgccg ccaaggatct gatggcgcag 9960

gggatcaagc tctgatcaag agacaggatg aggatcgttt cgcatgattg aacaagatgg 10020

attgcacgca ggttctccgg ccgcttgggt ggagaggcta ttcggctatg actgggcaca 10080

acagacaatc ggctgctctg atgccgccgt gttccggctg tcagcgcagg ggcgcccggt 10140

tctttttgtc aagaccgacc tgtccggtgc cctgaatgaa ctgcaagacg aggcagcgcg 10200

gctatcgtgg ctggccacga cgggcgttcc ttgcgcagct gtgctcgacg ttgtcactga 10260

agcgggaagg gactggctgc tattgggcga agtgccgggg caggatctcc tgtcatctca 10320

ccttgctcct gccgagaaag tatccatcat ggctgatgca atgcggcggc tgcatacgct 10380

tgatccggct acctgcccat tcgaccacca agcgaaacat cgcatcgagc gagcacgtac 10440

tcggatggaa gccggtcttg tcgatcagga tgatctggac gaagagcatc aggggctcgc 10500

gccagccgaa ctgttcgcca ggctcaaggc gagcatgccc gacggcgagg atctcgtcgt 10560

gacccatggc gatgcctgct tgccgaatat catggtggaa aatggccgct tttctggatt 10620

catcgactgt ggccggctgg gtgtggcgga ccgctatcag gacatagcgt tggctacccg 10680

tgatattgct gaagagcttg gcggcgaatg ggctgaccgc ttcctcgtgc tttacggtat 10740

cgccgctccc gattcgcagc gcatcgcctt ctatcgcctt cttgacgagt tcttctgaat 10800

tattaacgct tacaatttcc tgatgcggta ttttctcctt acgcatctgt gcggtatttc 10860

acaccgcatc aggtggcact tttcggggaa atgtgcgcgg aacccctatt tgtttatttt 10920

tctaaataca ttcaaatatg tatccgctca tgagattatc aaaaaggatc ttcacctaga 10980

tccttttaaa ttaaaaatga agttttaaat caatctaaag tatatatgag taaacttggt 11040

ctgacagtta ccaatgctta atcagtgagg cacctatctc agcgatctgt ctatttcgtt 11100

catccatagt tgcctgactc cccgtcgtgt agataactac gatacgggag ggcttaccat 11160

ctggccccag tgctgcaatg ataccgcgag acccacgctc accggctcca gatttatcag 11220

caataaacca gccagccgga agggccgagc gcagaagtgg tcctgcaact ttatccgcct 11280

ccatccagtc tattaattgt tgccgggaag ctagagtaag tagttcgcca gttaatagtt 11340

tgcgcaacgt tgttgccatt gctacaggca tcgtggtgtc acgctcgtcg tttggtatgg 11400

cttcattcag ctccggttcc caacgatcaa ggcgagttac atgatccccc atgttgtgca 11460

aaaaagcggt tagctccttc ggtcctccga tcgttgtcag aagtaagttg gccgcagtgt 11520

tatcactcat ggttatggca gcactgcata attctcttac tgtcatgcca tccgtaagat 11580

gcttttctgt gactggtgag tactcaacca agtcattctg agaatagtgt atgcggcgac 11640

cgagttgctc ttgcccggcg tcaatacggg ataataccgc gccacatagc agaactttaa 11700

aagtgctcat cattggaaaa cgttcttcgg ggcgaaaact ctcaaggatc ttaccgctgt 11760

tgagatccag ttcgatgtaa cccactcgtg cacccaactg atcttcagca tcttttactt 11820

tcaccagcgt ttctgggtga gcaaaaacag gaaggcaaaa tgccgcaaaa aagggaataa 11880

gggcgacacg gaaatgttga atactcatac tcttcctttt tcaatattat tgaagcattt 11940

atcagggtta ttgtctcatg accaaaatcc cttaacgtga gttttcgttc cactgagcgt 12000

cagaccccgt agaaaagatc aaaggatctt cttgagatcc tttttttctg cgcgtaatct 12060

gctgcttgca aacaaaaaaa ccaccgctac cagcggtggt ttgtttgccg gatcaagagc 12120

taccaactct ttttccgaag gtaactggct tcagcagagc gcagatacca aatactgttc 12180

ttctagtgta gccgtagtta ggccaccact tcaagaactc tgtagcaccg cctacatacc 12240

tcgctctgct aatcctgtta ccagtggctg ctgccagtgg cgataagtcg tgtcttaccg 12300

ggttggactc aagacgatag ttaccggata aggcgcagcg gtcgggctga acggggggtt 12360

cgtgcacaca gcccagcttg gagcgaacga cctacaccga actgagatac ctacagcgtg 12420

agctatgaga aagcgccacg cttcccgaag ggagaaaggc ggacaggtat ccggtaagcg 12480

gcagggtcgg aacaggagag cgcacgaggg agcttccagg gggaaacgcc tggtatcttt 12540

atagtcctgt cgggtttcgc cacctctgac ttgagcgtcg atttttgtga tgctcgtcag 12600

gggggcggag cctatggaaa aacgccagca acgcggcctt tttacggttc ctggcctttt 12660

gctggccttt tgctcacatg ttctttcctg cgttatcccc tgattctgtg gataaccgta 12720

ttaccgcctt tgagtgagct gataccgctc gccgcagccg aacgaccgag cgcagcgagt 12780

cagtgagcga ggaagcggaa g 12801

<210> 52
<211> 13120
<212> DNA
<213> Artificial Sequence

<220>
<223> Targeting vector

<400> 52
cacctgacgc gccctgtagc ggcgcattaa gcgcggcggg tgtggtggtt acgcgcagcg 60

tgaccgctac acttgccagc gccctagcgc ccgctccttt cgctttcttc ccttcctttc 120

tcgccacgtt cgccggcttt ccccgtcaag ctctaaatcg ggggctccct ttagggttcc 180

gatttagtgc tttacggcac ctcgacccca aaaaacttga ttagggtgat ggttcacgta 240

gtgggccatc gccctgatag acggtttttc gccctttgac gttggagtcc acgttcttta 300

atagtggact cttgttccaa actggaacaa cactcaaccc tatctcggtc tattcttttg 360

atttataagg gattttgccg atttcggcct attggttaaa aaatgagctg atttaacaaa 420

aatttaacgc gaattttaac aaaatattaa cgcttacaat ttccattcgc cattcaggct 480

gcgcaactgt tgggaagggc gatcggtgcg ggcctcttcg ctattacgcc agctggcgaa 540

agggggatgt gctgcaaggc gattaagttg ggtaacgcca gggttttccc agtcacgacg 600

ttgtaaaacg acggccagtg agcgcgcgta atacgactca ctatagggcg aattggctgg 660

gtcagtcgaa gggggcggtc ggtccatcat ttgcggccgg gtgatttttg ggccgaaaaa 720

gtgggaattt ggggcccaat ttggggccca atttggggcc caatttgggt tttcgagggg 780

ggattttttt agggggagat tttgggtccc cggaaaatgg tgattttggg tcaaaaatgg 840

ggattttggg gtcgttttga ggtggggttt tttggggtag aaatggcggt ctgaggggaa 900

aatgtcgttt tggggccatt ttgggccatt tgaggggaaa tttgggtcaa aaaatgacga 960

ttttgggtcg ttttagggag gaaaatgaat ttagggcgat ttgagccaaa tttgggtcaa 1020

aaatggcgat tttgagtcaa aaaatgatta ttttgggtca ttttagggag gaaaatgaat 1080

ttagggagat tggacggcaa atttgggtca aaaaatggtg attttgggtc aaaaaatgat 1140

tattttgggt cattttaggg agaaaaatga atttagggag atttgagggc aaatttgggt 1200

caaaaaatgg tgattttggg tcaaaaaatg attattttgg gtcattttag ggagaaaaat 1260

gaatttaggg agatttgagg gcaaatttgg gtcgaaaaat ggcgattttg ggtgaaaaat 1320

ggacaatttt gggtcatttt agggttaaaa atgaatttag ggagattgga cggcaaattt 1380

gggtcaaaaa atggtgattt ggggtcaaaa aataattatt ttgggtcatt ttagggataa 1440

aaaatgaatt tagggagatt tgagggcaaa tttgggtcga aaaatggtga tttggggtga 1500

aaaatggaca attttgggtc attttaggga taaaaaatga atttagggcg atttgagcca 1560

aatttgggtc aaaaatggcg attttgagtc aaaaaatgat tattttgggt cattttaggg 1620

ttaaaaatga atttagggag attggacggc aaatttgggt caaaaaatgg tgattttcgg 1680

tcaaaaaatg attattttgg gtcattttag ggagaaaaat gaatttaggg agattggacg 1740

gcaaatttgg gtcgaaaaat ggtgattttg ggtcaaaaat ggacaatttg gggtcatttt 1800

agggataaaa atgaatttag ggagatttga gagcaaattt gggtctaaaa atgtcaattt 1860

ggggtcaaaa atggtgattt tgggtcattt tagggatata aatgaatttt gggagatttg 1920

agggcaaatt tgggtcgaaa aatggtgatt tggggtcaat ttagggagga aaatgaattt 1980

aaggtgattt gacggcaaat ttgggttgaa aaatgacaat ttggggtcat tttaaagata 2040

aaatgaattt agggcgattt gagggcaaat ttgggtcgaa aaatggtgat ttgggtcaaa 2100

aaatgacaat tttgggtcat tttagggata taaatggact tagagcgatt tgagggcaaa 2160

tttggttgaa aaaatggaca atttgggtca ttttagggat ataaatgaat ttagggagat 2220

ttgatggcaa atttgggtcg aaaaatggtg atttgggtca aaaatggtga ttttggttga 2280

aaaacagtca tttggggtca ttttagggat taaaatgaat ttcgggagat ttgagggcaa 2340

atttgggtca aaaaatggtg attttgggtc aattagtgat aaaaaaatga atttagggtg 2400

atttgagggc aaatttgggt caaaaaatga caatttgggg tttttttagg gaggagaatg 2460

aatttaggga gttttgaggg caaatttggg tcaaaaaata gtgatttggg tcaaaagtgg 2520

tgattttggt tgaaaaacag tcattttggg tcattttagg gagaaaaatg aatttcggga 2580

gatttgaggg caaatttggg tcaaaaaatg acaattttgg gtcactttac gaattaaaat 2640

gaatttaggg agatttgagg gcaaatttgg gtcaaaatat ggtgattttg ggtcaattta 2700

gggaggaaaa tgaatttagg gcgatttgaa ggcaaatttg ggtcgaaaat ggtgattttg 2760

ggtcaaaaaa tgatgatttt gggtcgtttt agggagaaaa tgaatttagg gagatttgag 2820

ggcaaatttg ggtcaaaaaa tggtgatttt gggtcattgt agggataaaa atgaatttgg 2880

ggcgatttga gggcaaattt gggtggaaaa tggacaattt tgggtcattt tagggagaaa 2940

aatgaattta gggagatttg agggcaaatt tgggtcaaaa aatggtgatt tggggtcatt 3000

ttcagaagaa aaaatgatta ttttccccac taaaaatgta tattttgggg ccaaatggtg 3060

aaaaatggtg atttttaatc aaacgtcccc aaaattgggg aaatttcatc gatttgaccc 3120

aaaattgagt tttttttccc tgttaaaaat gtacattttg gggtcaatcg ctgaaatgtt 3180

cccacttttc acttctttgc ccccaaattt tgctgtccgg tgagaaatta attaattaat 3240

gatcattaat tgatcgacaa ttaatggtta ttaattaatt aataggtcct cttttggtga 3300

ctccttcgct tttggggcca aaagtccata aattggcccc aaaaaattaa cgctgagtaa 3360

ttggattcca aagtattaat gataaacatt aaaagtgttt aattaataat gatattaaac 3420

ataatttcgt ttttattatc gatttatcaa caacgatgaa cgataatact ttacaacaat 3480

cgctaataat taattaatta tttaattaat taatttctaa taattacttc gcattatcgg 3540

acacgagatg ttgtaattat taataattta attcctaata attagaagat tcgttgaaaa 3600

tggtacttat ctttacaaat aatcacttct aataataatg attaataata gttaataaca 3660

acaatgacga taatgacgat aatattaata atatgtgata tatttaatat aaaattcgta 3720

ttaatatatt atatctacaa aatatgatat aaaatataat attttattta tatataacac 3780

aatttattat tattatcatt attatcatta ttaatatcat cattattaac gttatcgaaa 3840

tacttattta gaaataataa aaacggattt aatgatggca acaaaaatat tttattaatg 3900

ttaaaaaaaa ataattaata atttccaaag attcgaattc ggggcaacga acggcactcg 3960

ataattttta attaattaat agtttgaatt aatcggtact ttttaatcct ccattttgcc 4020

cgaaatcgcc gttttttgcc ccaaattccc caccgcggcg ttaaaaacat aaagaaatta 4080

agcatcaaaa gtgccctttt ttggggttgt tttgacccaa aaaaaaaatg gccgaattgg 4140

gggcggccgt tttacggttg ggttcatttt gggttcaaaa cagccaaaaa tgggaacttt 4200

gggtttcgaa aacaacaaca acaaaaaaac gggtttattt tgggctcatt ttgggtgttt 4260

ttgggtcagg aggagaaaaa ataggaagtt tgagagcgaa acaacggccg cttttggggg 4320

gaaaacggcc ctttttggtc aacggcgggg gaaaaaaaaa agcggagttt ttggggtgaa 4380

aaagagcggt tttgggtaaa tttgggtttt ggggtaaaag tggaggattt tgggcaatgg 4440

gagttaaaaa atgggtgttt ttatgggggt tcggtgcagt ttttcctgtt tgatgggggg 4500

tttattaatc cggggggggg gattaatgag aattaataat gttaatagaa atatctggga 4560

aattaatagc aattattaat tgttaatagt tattaatagt tctatatatc tcacatctac 4620

gatacaatat aatatcgtta caatcatata gtcgatatat tacatataat tatcagtaat 4680

aataataagt aacaataatt agcagtaatt aataataata attaatagta ttcgttaata 4740

agattattga taataattaa gtagtagcga ttaatagaga tgggatttcg tgagaaatgg 4800

accaaatttg ggccgttttg acccaaattt ttggtggttt tttttccgat tctttgtgaa 4860

tttcgggtcg gattcatcag caattaatta cggttattag gggctattag aggcttttaa 4920

ttgggattat tagagacttt taagcggatt tggggacttt taagtggatt ttatgattct 4980

taagtggatt ttgggtggat tttaccgctt ttggacgtac gatcgggtac cataagaatg 5040

cggccgccgc ccccctctaa ccccccatct cccccccttt tctctctccc cccccccccc 5100

caaaccccac agcctccacc aagggcccat cggtcttccc cctggcaccc tcctccaaga 5160

gcacctctgg gggcacagcg gccctgggct gcctggtcaa ggactacttc cccgaaccgg 5220

tgacggtgtc gtggaactca ggcgccctga ccagcggcgt gcacaccttc ccggctgtcc 5280

tacagtcctc aggactctac tccctcagca gcgtggtgac cgtgccctcc agcagcttgg 5340

gcacccagac ctacatctgc aacgtgaatc acaagcccag caacaccaag gtggacaaga 5400

gagttgagcc caaatcttgt gacaaaactc acacatgccc accgtgccca ggtaagtcac 5460

tagaccagag ctccactccc gggagaatgg taagtgctgt aaacatccct gcactagagg 5520

ataagccatg tacagatcca tttccatctc tcctcatcag cacctgaact cctgggggga 5580

ccgtcagtct tcctcttccc cccaaaaccc aaggacaccc tcatgatctc ccggacccct 5640

gaggtcacat gcgtggtggt ggacgtgagc cacgaagacc ctgaggtcaa gttcaactgg 5700

tacgtggacg gcgtggaggt gcataatgcc aagacaaagc cgcgggagga gcagtacaac 5760

agcacgtacc gtgtggtcag cgtcctcacc gtcctgcacc aggactggct gaatggcaag 5820

gagtacaagt gcaaggtctc caacaaagcc ctcccagccc ccatcgagaa aaccatctcc 5880

aaagccaaag gtgagagctg cagcctgact gcatgggggc tgggatgggc ataaggataa 5940

aggtctgtgt ggacagcctt ctgcttcagc catgaccttt gtgtatgttt ctaccctcac 6000

agggcagccc cgagaaccac aggtgtacac cctgccccca tcccgggagg agatgaccaa 6060

gaaccaggtc agcctgacct gcctggtcaa aggcttctat cccagcgaca tcgccgtgga 6120

gtgggagagc aatgggcagc cggagaacaa ctacaagacc acgcctcccg tgctggactc 6180

cgacggctcc ttcttcctct atagcaagct caccgtggac aagagcaggt ggcagcaggg 6240

gaacgtcttc tcatgctccg tgatgcatga ggctctgcac aaccactaca cgcagaagag 6300

cctctccctg tccccgggta aatgagctca gcacccacaa aactctcagg tccaaagaga 6360

cacccacact catctccatg cttcccttgt ataaataaag cacccagcaa tgcctgggac 6420

catgtaaaac tgtcctggtt ctttccaagg tatagagcat agctcacagg ctgatatttc 6480

tggccagggt tggaggacag ccttgtctat aggaagagaa tgaggttttt gcactgcagg 6540

actcagagct cattagttat cctgccttgg agtgttgggg cttggcttta ggcagtgcct 6600

tttccttgcc ttcctacgaa ccagcagctg ccatacatag agataatcct aggaagcctc 6660

aaatggagaa ggacacaaac ccacctccct caggctgttc ctctatcccg gccccacttc 6720

tttacctagg ggtttctctg agtctattgt ggagttacac atggccaggg gcattccaga 6780

gacccttgtc atccatacac tcaactcagg cagctttgca caaacaaagt ctgcacaccc 6840

atacagatgg ctcactcttg cctgtgccat gtagggctga ggcacatggc tcttgctgcc 6900

ccaaggaggg gactattaga tagccacact catgctgaat cctggcccat tcaaattagc 6960

ctgctgaaca ccatccagtc catatagcac atgtatccac atgcacgtgt gcacaaaacg 7020

catttaacac actgggacaa caattctgtg ccctgcacag cacctatatc cagcaatgta 7080

tcaccataca cacgaccaaa aattcaatgc ccacgtttct gccatcacaa acagacacat 7140

ctttcctctc tgtggccact gcattatatg ctcaacacaa gacctctgaa gccagatcca 7200
tctctggcac ctcggggtca tgcttcaacc ccacatgaat tatgcaaacc atagccataa 7260
tggtctgaat cacttcacac tgggatgttc ccaagttcag gcaagacgag ccacaggctc 7320
tgctgatgac tgaaggacag caaagggtca gtccagctgt atagccactg ttgacctggg 7380
tcacaggccc tgctgaccct ccaccttctc ctgtactgaa ggaatgaaag atgagacaag 7440
catagagggc acttgaataa tccaggtcac tctgaggtcc acccaaggca ttattggact 7500
caggtgggaa gctgagactg gtgtcccaga gggaaaggaa ggaaagcagg ccccggggag 7560
ggtctgctgt cccagtcagg ctggagatct ctcctctgaa tccatgcaga catgtctgcc 7620
tcacagggaa tctctcccag caccaaccat gttgggacaa acactgactg tcctctctgt 7680
tcagagctgc aactggagga gagctgtgcg gaggcgcagg acggggagct ggacgggctg 7740
tggacgacca tcaccatctt catcacactc ttcctgttaa gcgtgtgcta cagtgccacc 7800
gtcaccttct tcaaggtgaa gtggatcttc tcctcggtgg tggacctgaa gcagaccatc 7860
atccccgact acaggaacat gatcggacag ggggcctagg aattaattcg ctgtctgcga 7920
gggccagctg ttggggtgag tactccctct caaaagcggg catgacttct gcgctaagat 7980
tgtcagtttc caaaaacgag gaggatttga tattcacctg gcccgcggtg atgcctttga 8040
gggtggccgc gtccatctgg tcagaaaaga caatcttttt gttgtcaagc ttgaggtgtg 8100
gcaggcttga gatctggcca tacacttgag tgacaatgac atccactttg cctttctctc 8160
cacaggtgtc cactcccagg tccaactgca ggtcgagcat gcatctaggg cggccaattg 8220
gatcctaccg ttcgtataat gtatgctata cgaagttatc gtacgcttaa ttaactgatc 8280
cggaattccg cccccccccc ccccccccct ctccctcccc cccccctaac gttactggcc 8340
gaagccgctt ggaataaggc cggtgtgcgt ttgtctatat gttattttcc accatattgc 8400
cgtcttttgg caatgtgagg gcccggaaac ctggccctgt cttcttgacg agcattccta 8460
ggggtctttc ccctctcgcc aaaggaatgc aaggtctgtt gaatgtcgtg aaggaagcag 8520
ttcctctgga agcttcttga agacaaacaa cgtctgtagc gaccctttgc aggcagcgga 8580
accccccacc tggcgacagg tgcctctgcg gccaaaagcc acgtgtataa gatacacctg 8640
caaaggcggc acaaccccag tgccacgttg tgagttggat agttgtggaa agagtcaaat 8700
ggctctcctc aagcgtattc aacaaggggc tgaaggatgc ccagaaggta ccccattgta 8760
tgggatctga tctggggcct cggtgcacat gctttacatg tgtttagtcg aggttaaaaa 8820
aacgtctagg ccccccgaac cacggggacg tggttttcct ttgaaaaaca cgatgataag 8880
cttgccacaa ccatggctga acaagatgga ttgcacgcag gttctccggc cgcttgggtg 8940
gagaggctat tcggctatga ctgggcacaa cagacaatcg gctgctctga tgccgccgtg 9000
ttccggctgt cagcgcaggg gcgcccggtt ctttttgtca agaccgacct gtccggtgcc 9060

ctgaatgaac tgcaggacga ggcagcgcgg ctatcgtggc tggccacgac gggcgttcct 9120

tgcgcagctg tgctcgacgt tgtcactgaa gcgggaaggg actggctgct attgggcgaa 9180

gtgccggggc aggatctcct gtcatctcac cttgctcctg ccgagaaagt atccatcatg 9240

gctgatgcaa tgcggcggct gcatacgctt gatccggcta cctgcccatt cgaccaccaa 9300

gcgaaacatc gcatcgagcg agcacgtact cggatggaag ccggtcttgt cgatcaggat 9360

gatctggacg aagagcatca ggggctcgcg ccagccgaac tgttcgccag gctcaaggcg 9420

cgcatgcccg acggcgagga tctcgtcgtg acccatggcg atgcctgctt gccgaatatc 9480

atggtggaaa atggccgctt ttctggattc atcgactgtg gccggctggg tgtggcggac 9540

cgctatcagg acatagcgtt ggctacccgt gatattgctg aagagcttgg cggcgaatgg 9600

gctgaccgct tcctcgtgct ttacggtatc gccgctcccg attcgcagcg catcgccttc 9660

tatcgccttc ttgacgagtt cttctgaata acttcgtata atgtatgcta tacgaacggt 9720

aggatcccgc ccgccccacg acccgcagcg cccgaccgaa aggagcgcac gaccccatgg 9780

ctccgaccga agccacccgg ggcggccccg ccgaccccgc acccgccccc gaggcccacc 9840

gactctagat aactgatcat aatcagccat accacatttg tagaggtttt acttgcttta 9900

aaaaacctcc cacacctccc cctgaacctg aaacataaaa tgaatgcaat tgttgttgtt 9960

aacttgttta ttgcagctta taatggttac aaataaagca atagcatcac aaatttcaca 10020

aataaagcat ttttttcact gtatgcaaac cacctcctgg tgtcccccaa cccttcatag 10080

ccccccaccc cgtctcggtc gcccacccaa ccctccacat taaccccttc ctccccacgc 10140

tcgtcccccg attcccaccc cccttttcgt caccatgcac cccccgtccc gcgaggactt 10200

cgaaggcccc ttccgcaacg cctccatcct ctgccagacc cgcgggcgcc gccgtcccgc 10260

cgaggtcacg tggtacaaaa atggcagccc cgtcgccgcc gccgccgcca ccaccgccac 10320

caccgtcggc cccgaagtgg tggccgagag ccgcatcagc gtcaccgaaa gcgaatggga 10380

caccggggcc accttcagct gcgtcgtgga gggggagatg aggaacacca gcaagaggat 10440

ggagtgcgga tgtaagtgga gggggggttt tttggggggg gatttggggg gaaattgggg 10500

gctttggggg caaaaagtcc gtcattttgg tttggaaggg gtcgatgtgg gggaaaatgt 10560

gccaaaaatc ccaattttgg gggcactgaa gtcgtaattt gaggtggagg aaggtagaca 10620

gaaagagggt ggagtgcgga tgtaagtgga gggggatttg gggcagattt gggggaaatt 10680

gggggtttgg ggggcaaata atccgtagtt tcggtttgga aggggttgat gtggttttgt 10740

gctgaatttc gggggaaaaa ctgccaaaaa tcccaatttt gggggcattg aagtcatagt 10800

ttgaggttgg aaggaggtag agagcaagag gatgaagtga ggatgtaagt cgggggattt 10860

ttggggcaga tttgggggaa attgggggtt ttggggtcag agaatccgta ggtcgaccgc 10920

gccagctttt gttcccttta gtgagggtta attgcgcgct tggcgtaatc atggtcatag 10980

ctgtttcctg tgtgaaattg ttatccgctc acaattccac acaacatacg agccggaagc 11040

ataaagtgta aagcctgggg tgcctaatga gtgagctaac tcacattaat tgcgttgcgc 11100

tcactgcccg ctttccagtc gggaaacctg tcgtgccagc tgcattaatg aatcggccaa 11160

cgcgcgggga gaggcggttt gcgtattggg cgctcttccg cttcctcgct cactgactcg 11220

ctgcgctcgg tcgttcggct gcggcgagcg gtatcagctc actcaaaggc ggtaatacgg 11280

ttatccacag aatcagggga taacgcagga aagaacatgt gagcaaaagg ccagcaaaag 11340

gccaggaacc gtaaaaaggc cgcgttgctg gcgtttttcc ataggctccg cccccctgac 11400

gagcatcaca aaaatcgacg ctcaagtcag aggtggcgaa acccgacagg actataaaga 11460

taccaggcgt ttccccctgg aagctccctc gtgcgctctc ctgttccgac cctgccgctt 11520

accggatacc tgtccgcctt tctcccttcg ggaagcgtgg cgctttctca tagctcacgc 11580

tgtaggtatc tcagttcggt gtaggtcgtt cgctccaagc tgggctgtgt gcacgaaccc 11640

cccgttcagc ccgaccgctg cgccttatcc ggtaactatc gtcttgagtc caacccggta 11700

agacacgact tatcgccact ggcagcagcc actggtaaca ggattagcag agcgaggtat 11760

gtaggcggtg ctacagagtt cttgaagtgg tggcctaact acggctacac tagaaggaca 11820

gtatttggta tctgcgctct gctgaagcca gttaccttcg gaaaaagagt tggtagctct 11880

tgatccggca aacaaaccac cgctggtagc ggtggttttt ttgtttgcaa gcagcagatt 11940

acgcgcagaa aaaaaggatc tcaagaagat cctttgatct tttctacggg gtctgacgct 12000

cagtggaacg aaaactcacg ttaagggatt ttggtcatga gattatcaaa aaggatcttc 12060

acctagatcc ttttaaatta aaaatgaagt tttaaatcaa tctaaagtat atatgagtaa 12120

acttggtctg acagttacca atgcttaatc agtgaggcac ctatctcagc gatctgtcta 12180

tttcgttcat ccatagttgc ctgactcccc gtcgtgtaga taactacgat acgggagggc 12240

ttaccatctg gccccagtgc tgcaatgata ccgcgagacc cacgctcacc ggctccagat 12300

ttatcagcaa taaaccagcc agccggaagg gccgagcgca gaagtggtcc tgcaacttta 12360

tccgcctcca tccagtctat taattgttgc cgggaagcta gagtaagtag ttcgccagtt 12420

aatagtttgc gcaacgttgt tgccattgct acaggcatcg tggtgtcacg ctcgtcgttt 12480

ggtatggctt cattcagctc cggttcccaa cgatcaaggc gagttacatg atcccccatg 12540

ttgtgcaaaa aagcggttag ctccttcggt cctccgatcg ttgtcagaag taagttggcc 12600

gcagtgttat cactcatggt tatggcagca ctgcataatt ctcttactgt catgccatcc 12660

gtaagatgct tttctgtgac tggtgagtac tcaaccaagt cattctgaga atagtgtatg 12720

cggcgaccga gttgctcttg cccggcgtca atacgggata ataccgcgcc acatagcaga 12780

actttaaaag tgctcatcat tggaaaacgt tcttcggggc gaaaactctc aaggatctta 12840

ccgctgttga gatccagttc gatgtaaccc actcgtgcac ccaactgatc ttcagcatct 12900

tttactttca ccagcgtttc tgggtgagca aaaacaggaa ggcaaaatgc cgcaaaaaag 12960

ggaataaggg cgacacggaa atgttgaata ctcatactct tcctttttca atattattga 13020

agcatttatc agggttattg tctcatgagc ggatacatat ttgaatgtat ttagaaaaat 13080

aaacaaatag gggttccgcg cacatttccc cgaaaagtgc 13120

<210> 53
<211> 2449
<212> DNA
<213> Homo sapiens

<400> 53
ctggcccatc ctctctgtta gatgtgatca aaactcataa aggctgtccc tgaggtttct 60

gtcccaggac tgattgcgga agagtcacca ggcactgagg agacggtgac cagggtgccc 120

tggccccaga cgtccatacc gtagtagtag tagtaggttc ctttcgcaca gtaatatacg 180

gccgtgtcct cggctctcag gctgttcatt tgcagataca gcgtgttctt ggaattgtct 240

ctggagatgg tgaaccggcc ctggaacttc tgtgcgtaga ttgtttcacc atcttcagga 300

tcaaaacctg agacccactc cagccccttc cctggagcct ggcggaccca gtgcatggat 360

aattcgctaa aggtgaatcc agaggctgca caggagagtc tcagggaccc cccaggctgt 420

accaagcctc ccccagactc caacagctgc acctcgtact agacgtagta cgtgatacgt 480

cgtatctggc ccatcctctc tgttagatgt gatcaaaact cataaaggct gtccctgagg 540

tttctgtccc aggactgatt gcggaagagt caccaggcac tgaggagacg gtgaccaggg 600

tgccctggcc ccagtagtca aagtagtaat aggataacct taaatctttc gcacagtaat 660

atacggccgt gtcctcggct ctcaggctgt tcatttgcag atacagcgtg ttcttggaat 720

tgtctctgga gatggtgaac cggccctgga gcttctgtgc atagtttgtg ttaccattgt 780

aagcgctgat ccatgagacc cactccagcc ccttccctgg agcctggcgg acccagctga 840

taccatagct gctaaaggtg aatccagagg ctgcacagga gagtctcagg gaccccccag 900

gctgtaccaa gcctccccca gactccaaca gctgcacctc gtatctctcg cactactatg 960

agacgtatca ctggcccatc ctctctgtta gatgtgatca aaactcataa aggctgtccc 1020

tgaggtttct gtcccaggac tgattgcgga agagtcacca ggcacgaggt gcagctgttg 1080

gagtctgggg gaggcttggt acagcctggg gggtccctga gactctcctg tgcagcctct 1140

ggattcacct ttagcagtta tgatatcaac tgggtccgcc aggctccagg gaaggggctg 1200

gagtgggtct catggatgaa ccctaacagt ggtaacacag gctatgcaca gaagttccag 1260

ggccggttca ccatctccag agacaattcc aagaacacgc tgtatctgca aatgaacagc 1320

ctgagagccg aggacacggc cgtatattac tgtgcgaaat ccagcagctg gcactactac 1380

tacggtatgg acgtctgggg ccagggcacc ctggtcaccg tctcctcacg catagagaga 1440

cacgcgagta gagacacgga attcctggcc catcctctct gttagatgtg atcaaaactc 1500

ataaaggctg tccctgaggt ttctgtccca ggactgattg cggaagagtc accaggcact 1560

gaggagacgg tgaccagggt gccctggccc cagtagtcaa agtggacgta gtaataacca 1620

ctactagacg ctttcgcaca gtaatatacg gccgtgtcct cggctctcag gctgttcatt 1680

tgcagataca gcgtgttctt ggaattgtct ctggagatgg tgaaccggcc ctggaacttc 1740

tgtgaatatt ttgtgttacc attgccagcg ttgatccatg agacccactc cagccccttc 1800

cctggagcct ggcggaccca atgcatagca tagctgctaa aggtgaatcc agaggctgca 1860

caggagagtc tcagggaccc cccaggctgt accaagcctc ccccagactc caacagctgc 1920

acctcctcgc gatatcgtgt ctgatctaca gtgctctggc ccatcctctc tgttagatgt 1980

gatcaaaact cataaaggct gtccctgagg tttctgtccc aggactgatt gcggaagagt 2040

caccaggcac tgaggagacg gtgaccaggg tgccctggcc ccaggggtcg aaccagttga 2100

gggtagtcac cgtatagccc caacctttcg cacagtaata tacggccgtg tcctcggctc 2160

tcaggctgtt catttgcaga tacagcgtgt tcttggaatt gtctctggag atggtgaacc 2220

ggccctgaaa cttctgtgca tagtttgtgc caccactgtt agggttgatc catgagaccc 2280

actccagccc cttccctgga gcctggcgga cccagtgcat atagtagccg ctaaaggtga 2340

atccagaggc tgcacaggag agtctcaggg accccccagg ctgtaccaag cctcccccag 2400

actccaacag ctgcacctcg tatgtacagg agcgtagagg agtgactag 2449

<210> 54
<211> 2581
<212> DNA
<213> Gallus gallus

<400> 54
tgggtcagtc gaagggggcg gtcggtccat catttgcggc cgggtgattt ttggggccga 60

aaagtgggaa tttggggccc aatttggggc ccaatttggg gcccaatttg ggttttcgag 120

gggggatttt tttaggggga gattttgggt ccccggaaaa tggtgatttt gggtcaaaaa 180

tggggatttt ggggtcgttt tgaggtgggg ttttttgggg tagaaatggc ggtctgaggg 240

gaaaatgtcg ttttggggcc attttgggcc atttgagggg aaatttgggt caaaaaatga 300

cgattttggg tcgttttagg gaggaaaatg aatttagggc gatttgagcc aaatttgggt 360

caaaaatggc gattttgagt caaaaaatga ttattttggg tcattttagg gaggaaaatg 420

aatttaggga gattggacgg caaatttggg tcaaaaaatg gtgattttgg gtcaaaaaat 480

gattattttg ggtcatttta gggagaaaaa tgaatttagg gagatttgag ggcaaatttg 540

ggtcaaaaaa tggtgatttg ggggtcattt cagaagaaaa aatgattatt ttccccacta 600

aaaatgtata ttttggggcc aaatggtgaa aaatggtgat ttttaatcaa acgtccccaa 660

aattggggaa atttcatcga tttgacccaa aattgagttt tttttccctg ttaaaaatgt 720

acattttggg gtcaatcgct gaaatgttcc cacttttcac ttctttgccc ccaaattttg 780

ctgtccggtg agaaattaat taattaatga tcattaattg atcgacaatt aatggttatt 840

aattaattaa taggtcctct tttggtgact ccttcgcttt tggggccaaa agtccataaa 900

ttggccccaa aaaattaacg ctgagtaatt ggattccaaa gtattaatga taaacattaa 960

aagtgtttaa ttaataatga tattaaacat aatttcgttt ttattatcga tttatcaaca 1020

acgatgaacg ataatacttt acaacaatcg ctaataatta attaattatt taattaatta 1080

atttctaata attacttcgc attatcggac acgagatgtt gtaattatta ataatttaat 1140

tcctaataat tagaagattc gttgaaaatg gtacttatct ttacaaataa tcacttctaa 1200

taataatgat taataatagt taataacaac aatgacgata atgacgataa tattaataat 1260

atgtgatata tttaatataa aattcgtatt aatatattat atctacaaaa tatgatataa 1320

aatataatat tttatttata tataacacaa tttattatta ttatcattat tatcattatt 1380

aatatcatca ttattaacgt tatcgaaata cttatttaga aataataaaa acggatttaa 1440

tgatggcaac aaaaatattt tattaatgtt aaaaaaaaat aattaataat ttccaaagat 1500

tcgaattcgg ggcaacgaac ggcactcgat aatttttaat taattaatag tttgaattaa 1560

tcggtacttt ttaatcctcc attttgcccg aaatcgccgt tttttgcccc aaattcccca 1620

ccgcggcgtt aaaaacataa agaaattaag catcaaaagt gccctttttt ggggttgttt 1680

tgacccaaaa aaaaaatggc cgaattgggg gcggccgttt tacggttggg ttcattttgg 1740

gttcaaaaca gccaaaaatg ggaactttgg gtttcgaaaa caacaacaac aaaaaaacgg 1800

gtttattttg ggctcatttt gggtgttttt gggtcaggag gagaaaaaat aggaagtttg 1860

agagcgaaac aacggccgct tttgggggga aaacggccct ttttggtcaa cggcggggga 1920

aaaaaaaaag cggagttttt ggggtgaaaa agagcggttt tgggtaaatt tgggttttgg 1980

ggtaaaagtg gaggattttg ggcaatggga gttaaaaaat gggtgttttt atgggggttc 2040

ggtgcagttt ttcctgtttg atggggggtt tattaatccg ggggggggga ttaatgagaa 2100

ttaataatgt taatagaaat atctgggaaa ttaatagcaa ttattaattg ttaatagtta 2160

ttaatagttc tatatatctc acatctacga tacaatataa tatcgttaca atcatatagt 2220

cgatatatta catataatta tcagtaataa taataagtaa caataattag cagtaattaa 2280

taataataat taatagtatt cgttaataag attattgata ataattaagt agtagcgatt 2340

aatagagatg ggatttcgtg agaaatggac caaatttggg ccgttttgac ccaaattttt 2400

ggtggttttt tttccgattc tttgtgaatt tcgggtcgga ttcatcagca attaattacg 2460

gttattaggg gctattagag gcttttaatt gggattatta gagactttta agcggatttg 2520

gggactttta agtggatttt atgattctta agtggatttt gggtggattt taccgctttt 2580

g 2581

<210> 55
<211> 13244
<212> DNA
<213> Artificial Sequence

<220>
<223> Targeting vector

<400> 55
   agcgtgaccg ctacacttgc cagcgcccta gcgcccgctc ctttcgcttt cttccttcct 60
   ttctcgccac gttcgccggc tttccccgtc aagctctaaa tcgggggctc cctttagggt 120
   tccgatttag tgctttacgg cacctcgacc ccaaaaaact tgattagggt gatggttcac 180
   gtagtgggcc atcgccctga tagacggttt ttcgcccttt gacgttggag tccacgttct 240
   ttaatagtgg actcttgttc caaactggaa caacactcaa ccctatctcg gtctattctt 300
   ttgatttata agggattttg ccgatttcgg cctattggtt aaaaaaatga gctgatttaa 360
   caaaaattta acgcgaattt taacaaaata ttaacgctta caatttccat tcgccattca 420
   ggctgcgcaa ctgttgggaa gggcgatcgg tgcgggcctc ttcgctatta cgccagctgg 480
   cgaaaggggg atgtgctgca aggcgattaa gttgggtaac gccagggttt tcccagtcac 540
   gacgttgtaa aacgacggcc agtgagcgcg cgtaatacga ctcactatag ggcgaattgg 600
   gtaccataag aatgcggccg cttcccgaag cgaaagccgc gtgttatttt tgggtgggaa 660
   agccccgatt cgctcctttt tttttattta ttatttttgt tgtttttcgt gtctttttct 720
   tctccgtttt gcagctgcgg cctccaggtg tccccggccg tggtattttt gggtcgaaaa 780
   agccgctttt tggctcactt ggaaaccttt cgggcgtctc caagcggagc gttggcggga 840
   aatggtgttt tttttgggtt gttttcggtc ccttttttca ccatttttgt ggtttttcgg 900
   cccattttcg gcctcccccc cccccccccc cttcccgtgg ggctgcgctc cgtggagcgg 960
   aatggggtcg ttaattaagg ttaattggtt taattaatgg gccgtgcagg gagaagaggg 1020
   ccgtgcggtg gttgttagcc ctgattaatg gcttggttga ggggagagtg taattaagtg 1080
   cttcgttagc actaattaat ggcttgattg agaggagagt gtaattaaat gcttcataat 1140
   ccctaattaa tggcttggtt gagacaagag tgtaattaag tgcttcatta gccctgatta 1200
   atggcttggt tgaggggaga gtgtaattaa atgcttcgtt agcactaatt aatggcttga 1260
   ttgagaggag agtgtaatta aatgcttcat aatccctaat taatggcttg gttgagacaa 1320
   gagtgtaatt aagtgcttca ttagccctga ttaatggctt ggttgagagg agagtgtaat 1380
   taaatgcttc attatcccta attagggact gaattccaag gagagtgtaa ttaagtgctt 1440
   cattatccct aattaatggc ttggttgaga ggagagtgta attaaatgct ttgttagccc 1500
   taattaatgg tttgatggag aggagagtgt aattaagtgc ttcattatcc ctaattaatg 1560
   gcttggttga ggggagagtg taattaaatg cttagttagc cctaattaat ggcttggttg 1620
   agaggagagt gtaattgaat gcttcattat ccctaattag ggattgaatt gagaggagag 1680
   tgtaattgaa tgctttgtta gccctaatta gggattgaat tgagaggaga gtgtaattaa 1740
   atgcctcatt atccctaatt agggattgaa ttgagaggag agtttaatta aatgcctcat 1800
   tatccctaat tagggactga attccgacga gagtgtaatt aagtgcttca tttagcccta 1860
   attaatggct tgattgagag gagagtgtaa ttaaatgctt cattatccct aattagggat 1920
   tgaattgaga ggagagtgta attaaatgct tcattatccc taattaggga ttgaattgag 1980
   aggagagtgt aattaaatgc ttcattatcc ctaattaatg gcttggttga gaggggagtg 2040
   taattaagtg cttcgttatc cctaattagg gattgaattg tgaggagatg gcaattaata 2100
   cttcattatc cctaattaat ttaattagtc cctattatcc ctaatttaat tcatggtttg 2160
   gttgatggaa gaatgtaatt aaatgctttg ttatccctaa ttaatggttt gattgagcgg 2220
   agagtgtaat taaatgcttc attatccctt attaatggtt gtgtggaatg tagtgtaatt 2280
   agaagtccct aattaatggt ttaattgaga gaagactgca attagatgac tcatagcccc 2340
   taataagtgg tttaattatg agataatcac aattacatgt ttcattgtcc ctaattaatc 2400
   gtttaattgc gagaagatca caattaatta aatgatccct aatttcaatt tctgagaagt 2460
   gtctttctcg gaaattgaga tatcataaga attcgcgatc aatttctgag aactgtcatt 2520
   ctcggaatac ctactagtat aagaatggtc atcatcagtc gacctactac tactcgagct 2580
   ggcccatcct ctctgttaga tgtgatcaaa actcataaag gctgtccctg aggtttctgt 2640
   cccaggactg attgcggaag agtcaccagg cactgaggag acggtgacca gggtgccctg 2700
   gccccagacg tccataccgt agtagtagta gtaggttcct ttcgcacagt aatatacggc 2760
   cgtgtcctcg gctctcaggc tgttcatttg cagatacagc gtgttcttgg aattgtctct 2820
   ggagatggtg aaccggccct ggaacttctg tgcgtagatt gtttcaccat cttcaggatc 2880
   aaaacctgag acccactcca gccccttccc tggagcctgg cggacccagt gcatggataa 2940
   ttcgctaaag gtgaatccag aggctgcaca ggagagtctc agggaccccc caggctgtac 3000
   caagcctccc ccagactcca acagctgcac ctcgtactag acgtagtacg tgatacgtcg 3060
   tatctggccc atcctctctg ttagatgtga tcaaaactca taaaggctgt ccctgaggtt 3120
   tctgtcccag gactgattgc ggaagagtca ccaggcactg aggagacggt gaccagggtg 3180
   ccctggcccc agtagtcaaa gtagtaatag gataacctta aatctttcgc acagtaatat 3240
   acggccgtgt cctcggctct caggctgttc atttgcagat acagcgtgtt cttggaattg 3300
   tctctggaga tggtgaaccg gccctggagc ttctgtgcat agtttgtgtt accattgtaa 3360
   gcgctgatcc atgagaccca ctccagcccc ttccctggag cctggcggac ccagctgata 3420
   ccatagctgc taaaggtgaa tccagaggct gcacaggaga gtctcaggga ccccccaggc 3480
   tgtaccaagc ctcccccaga ctccaacagc tgcacctcgt atctctcgca ctactatgag 3540
   acgtatcact ggcccatcct ctctgttaga tgtgatcaaa actcataaag gctgtccctg 3600
   aggtttctgt cccaggactg attgcggaag agtcaccagg cacgaggtgc agctgttgga 3660
   gtctggggga ggcttggtac agcctggggg gtccctgaga ctctcctgtg cagcctctgg 3720
   attcaccttt agcagttatg atatcaactg ggtccgccag gctccaggga aggggctgga 3780
   gtgggtctca tggatgaacc ctaacagtgg taacacaggc tatgcacaga agttccaggg 3840
   ccggttcacc atctccagag acaattccaa gaacacgctg tatctgcaaa tgaacagcct 3900
   gagagccgag gacacggccg tatattactg tgcgaaatcc agcagctggc actactacta 3960
   cggtatggac gtctggggcc agggcaccct ggtcaccgtc tcctcacgca tagagagaca 4020
   cgcgagtaga gacacggaat tcctggccca tcctctctgt tagatgtgat caaaactcat 4080
   aaaggctgtc cctgaggttt ctgtcccagg actgattgcg gaagagtcac caggcactga 4140
   ggagacggtg accagggtgc cctggcccca gtagtcaaag tggacgtagt aataaccact 4200
   actagacgct ttcgcacagt aatatacggc cgtgtcctcg gctctcaggc tgttcatttg 4260
   cagatacagc gtgttcttgg aattgtctct ggagatggtg aaccggccct ggaacttctg 4320
   tgaatatttt gtgttaccat tgccagcgtt gatccatgag acccactcca gccccttccc 4380
   tggagcctgg cggacccaat gcatagcata gctgctaaag gtgaatccag aggctgcaca 4440
   ggagagtctc agggaccccc caggctgtac caagcctccc ccagactcca acagctgcac 4500
   ctcctcgcga tatcgtgtct gatctacagt gctctggccc atcctctctg ttagatgtga 4560
   tcaaaactca taaaggctgt ccctgaggtt tctgtcccag gactgattgc ggaagagtca 4620
   ccaggcactg aggagacggt gaccagggtg ccctggcccc aggggtcgaa ccagttgagg 4680
   gtagtcaccg tatagcccca acctttcgca cagtaatata cggccgtgtc ctcggctctc 4740
   aggctgttca tttgcagata cagcgtgttc ttggaattgt ctctggagat ggtgaaccgg 4800
   ccctgaaact tctgtgcata gtttgtgcca ccactgttag ggttgatcca tgagacccac 4860
   tccagcccct tccctggagc ctggcggacc cagtgcatat agtagccgct aaaggtgaat 4920
   ccagaggctg cacaggagag tctcagggac cccccaggct gtaccaagcc tcccccagac 4980
   tccaacagct gcacctcgta tgtacaggag cgtagaggag tgactaggaa ttcctactac 5040
   tagcaatgtt ggcgcagaag ggattcatat caattaattc cctgtcctca attaatgcat 5100
   aattgggagt gcgatggaat cattcaataa gtaatgagga cttggtgtca aaatgccaat 5160
   taagtaatta atgacaccca attaatacac aatggggatt acagtggaat aactcagtga 5220
   agttcatatc taaattgcaa ttgaataatt aattacagct aattaatgga tgggagactg 5280
   gcagtggtat aacttaatga aggaacacag tgaattcctg tctgaattga aattaattga 5340
   tttattaggg ctaattaatg aatggtgggg ttaaaaatag cattactcag cgaaggaaca 5400
   tgagttcatg gctaaattga gattgaatta ttaattacaa ctaattgatt aatgactgat 5460
   gggagtaaga atggaataac tcagtgaagg aacacaatgg attcatggcc taattgaaat 5520
   taaatggtta attagagcta attaatgaat gatggggggt ggcaatggaa tgatacaaga 5580
   aagaaacaca atgggttcgt gtctacattg aaattaaata attaattaca gctaattaat 5640
   aaacggcgag gttgacaatg gtataactgt gaagagacag aatgagttcg tgactaaatt 5700
   ggtattaaat gattaattag agctaattaa caggtggtgg ggctgagaat ggcataactc 5760
   aatgggggat gatgagctga tggcttaatt ggaattaata attaatcaca accaattaac 5820
   aggtgctgtt aatggggtta caggggcatg acttcaggaa gaagcacaaa gtggccatta 5880
   aataattaat tgcacccaat taattcctga tggggattac tgcagaggaa ctcagcgaag 5940
   gattcaagca gaaatgacac agttaggttg ctcaattgcg ttaattaatt aaaatcgcta 6000
   attaggtgag cttcattaag gcctgatggg gttcatccca acgctgggat caaagaactc 6060
   agcgcagagt ggataacgat aattagaagg ggacaaatta atgaggaaac acgactttgg 6120
   gcctagacgg ccattaaatc attaatcaac ccgacgggcg cccaattaat cataaagtta 6180
   ttaagcaatt aagtcgaggc tgacgagaaa ccgcaacgag agccctccac gctcctttga 6240
   gttggcgcaa acaacaaaca acgcgggctc gttaagcagc gacgacgccg agcgtaacga 6300
   agcaatcccc gcgcccgaca attaacgcgc taacgaagcc ggagccctcc ttatgcaaat 6360
   tagcccctcc agagggccat aaaagcgccg gctctccgac ggcgcgggag gagcaccagt 6420
   cggctccgca accatgagcc cactcgtctc ctccctcctg ctcctggccg ccctgccagg 6480
   tgagggcgct gtggggctct atggggctct atggggtctc agcggggctc tgcgggctca 6540
   atgggggcca aggggggggt ctgcgggctc tatggggggg tcaacggggg gtctcacggg 6600
   gggccggctc cgcgaggccg tgtggcggcg gctccgtcag cgctctctgg gcgcgcctcc 6660
   ttccccacag ggctgatggc ggaggtgcag ctgttggagt ctgggggagg cttggtacag 6720
   cctggggggt ccctgagact ctcctgtgca gcctctggat tcacctttag cagctatgcc 6780
   atgagctggg tccgccaggc tccagggaag gggctggagt gggtctcagc tattagtggt 6840
   agtggtggta gcacatacta cgcagactcc gtgaagggcc ggttcaccat ctccagagac 6900
   aattccaaga acacgctgta tctgcaaatg aacagcctga gagccgagga cacggccgta 6960
   tattactgtg cgaaaggata tagtggctac gattactact ttgactactg gggccagggc 7020
   accctggtca ccgtctcctc aggtgagtct tcatatgaac cccccccaaa attgccgcgg 7080
   cgattttggg gcaaaatcgg gcgattttat ggatccccta ccgttcgtat aatgtatgct 7140
   atacgaagtt atagatccag acatgataag atacattgat gagtttggac aaaccacaac 7200
   tagaatgcag tgaaaaaaat gctttatttg tgaaatttgt gatgctattg ctttatttgt 7260
   aaccattata agctgcaata aacaagttaa caacaacaat tgcattcatt ttatgtttca 7320
   ggttcagggg gaggtgtggg aggtttttta aagcaagtaa aacctctaca aatgtggtat 7380
   ggctgattat gatctctagt caaggcacta tacatcaaat attccttatt aaccccttta 7440
   caaattaaaa agctaaaggt acacaatttt tgagcatagt tattaatagc agacactcta 7500
   tgcctgtgtg gagtaagaaa aaacagtatg ttatgattat aactgttatg cctacttata 7560
   aaggttacag aatatttttc cataattttc ttgtatagca gtgcagcttt ttcctttgtg 7620
   gtgtaaatag caaagcaagc aagagttcta ttactaaaca cagcatgact caaaaaactt 7680
   agcaattctg aaggaaagtc cttggggtct tctacctttc tcttcttttt tggaggagta 7740
   gaatgttgag agtcagcagt agcctcatca tcactagatg gcatttcttc tgagcaaaac 7800
   aggttttcct cattaaaggc attccaccac tgctcccatt catcagttcc ataggttgga 7860
   atctaaaata cacaaacaat tagaatcagt agtttaacac attatacact taaaaatttt 7920
   atatttacct tagagcttta aatctctgta ggtagtttgt ccaattatgt cacaccacag 7980
   aagtaaggtt ccttcacaaa gatccggacc aaagcggcca tcgtgcctcc ccactcctgc 8040
   agttcggggg catggatgcg cggatagccg ctgctggttt cctggatgcc gacggatttg 8100
   cactgccggt agaactccgc gaggtcgtcc agcctcaggc agcagccaag cttggtatgg 8160
   taaaactttt aatttcgggt atatttgagt ggaatgagtt cttcaatcgt agttttgact 8220
   aacttgccat tcatttctat taacacaaaa caatctggtg catagtctga aatcaactcc 8280
   ctacacatac cacaaggact taccactcga atacttctat ctacttcgtc agaataaggg 8340
   tgtctaacag ctacaatcgt gtcaaaatcc ttttgtccat tcgaaactgc actaccaatc 8400
   gcaatggctt ctgcacaaac agttactcgt cctatatacg cttcaatatg tactgccgaa 8460
   atgatttctc ctgttttcgt acgaattgcc gctcccacat gatgtttatt atcctcataa 8520
   agcattgtaa tcttctctgt cgctacttct actaattcta gatcttgttg agaaatgtta 8580
   aatgttttca tatattcttc ttcctccgat ttctgcaaaa atgaatccaa gcttgaatta 8640
   attctttgcc aaaatgatga gacagcacaa taaccagcac gttgcccagg agtaggaaaa 8700
   agaagaaggc atgaacatgg ttagcagagg ctctagagcc gccggtcaca cgccagaagc 8760
   cgaaccccgc cctgccccgt cccccccgaa ggcagccgtc cccccgcgga cagccccgag 8820
   gctggagagg gagaagggga cggcggcgcg gcgacgcacg aaggccctcc ccgcccattt 8880
   ccttcctgcc ggcgccgcac cgcttcgccc cgcgcccgct agagggggtg cggcggcgcc 8940
   tcccagattt cggctccgca cagatttggg acaaaggaag tccctgcgcc ctctcgcacg 9000
   attaccataa aaggcaatgg ctgcggctcg ccgcgcctcg acagccgccg gcgctccggg 9060
   ggccgccgcg cccctccccc gagccctccc cggcccgagg cggccccgcc ccgcccggca 9120
   cccccacctg ccgccacccc ccgcccggca cggcgagccc cgcgccacgc cccgtacgga 9180
   gccccgcacc cgaagccggg ccgtgctcag caactcgggg aggggggtgc acgggggggt 9240
   tgcagcccga ccgacgcgcc cacmccccct gctcaccccc ccacgcacac accccgcacg 9300
   cagcctttgt tcccctcgca gccccccccg caccgcgggg caccgccccc ggccgcgctc 9360
   cctcgcgcac actgcggagc gcacaaagcc ccgcgccgcg cccgcagcgc tcacagccgc 9420
   cgggcagcgc ggagccgcac gcggcgctcc ccacgcacac acacacgcac gcaccccccg 9480
   agccgctccc cccgcacaaa gggccctccc ggagcccctc aaggctttca cgcagccaca 9540
   gaaaagaaac aagccgtcaw waaaccaagc actrattaca gcccggaggt gaagggccgt 9600
   tyckcccgca tcaymtktgg gwktaaagcg rtcagtcaga rcckgggcgg smggcgygra 9660
   acgrcggcgg arcagggctc gggggdaagg cagsstgcaa cgaytmccgc cccccccacs 9720
   ctttgytttt taaagggccs ccgccgccgc cgcctcsccm taaaaggaaa ctttyggagc 9780
   gcgccgcttt gattggctgc cgccgcacct tttcgcctcg ccccgccccg cccctcgccc 9840
   cgccccgccc cgcctggcgc gcgccccccc cccccccccg cccccatcgc tgcacaaaat 9900
   aattaaaaaa taaataaata caaaattggg ggtggggagg ggggggagat ggggagagtg 9960
   aagcagaacg tggggctcac ctcgaggatc tgggctagcc ctgatcaata acttcgtata 10020
   atgtatgcta tacgaacggt aggggatcca ctagttgggt cagtcgaagg gggcggtcgg 10080
   tccatcattt gcggccgggt gatttttggg gccgaaaagt gggaatttgg ggcccaattt 10140
   ggggcccaat ttggggccca atttgggttt tcgagggggg atttttttag ggggagattt 10200
   tgggtccccg gaaaatggtg attttgggtc aaaaatgggg attttggggt cgttttgagg 10260
   tggggttttt tggggtagaa atggcggtct gaggggaaaa tgtcgttttg gggccatttt 10320
   gggccatttg aggggaaatt tgggtcaaaa aatgacgatt ttgggtcgtt ttagggagga 10380
   aaatgaattt agggcgattt gagccaaatt tgggtcaaaa atggcgattt tgagtcaaaa 10440
   aatgattatt ttgggtcatt ttagggagga aaatgaattt agggagattg gacggcaaat 10500
   ttgggtcaaa aaatggtgat tttgggtcaa aaaatgatta ttttgggtca ttttagggag 10560
   aaaaatgaat ttagggagat ttgagggcaa atttgggtca aaaaatggtg atttggggtc 10620
   attttcagaa gaaaaaatga ttattttccc cactaaaaat gtatattttg gggccaaatg 10680
   gtgaaaaatg gtgattttta atcaaacgtc cccaaaattg gggaaatttc atcgatttga 10740
   cccaaaattg agtttttttt ccctgttaaa aatgtacatt ttggggtcaa tcgctgaaat 10800
   gttcccactt ttcacttctt tgcccccaaa ttttgctgtc cggtgagaaa ttaattaatt 10860
   aatgatcatt aattgatcga caattaatgg ttattaatta attaataggt cctcttttgg 10920
   tgactccttc gcttttgggg ccaaaagtcc ataaattggc cccaaaaaat taacgctgag 10980
   taattggatt ccaaagtatt aatgataaac attaaaagtg tttaattaat aatgatatta 11040
   aacataattt cgtttttatt atcgatttat caacaacgat gaacgataat actttacaac 11100
   aatcgctaat aattaattaa ttatttaatt aattaatttc taataattac ttcgcattat 11160
   cggacacgag atgttgtaat tattaataat ttaattccta ataattagaa gattcgttga 11220
   aaatggtact tatctttaca aataatcact tctaataata atgattaata atagttaata 11280
   acaacaatga cgataatgac gataatatta ataatatgtg atatatttaa tataaaattc 11340
   gtattaatat attatatcta caaaatatga tataaaatat aatattttat ttatatataa 11400
   cacaatttat tattattatc attattatca ttattaatat catcattatt aacgttatcg 11460
   aaatacttat ttagaaataa taaaaacgga tttaatgatg gcaacaaaaa tattttatta 11520
   atgttaaaaa aaaataatta ataatttcca aagattcgaa ttcggggcaa cgaacggcac 11580
   tcgataattt ttaattaatt aatagtttga attaatcggt actttttaat cctccatttt 11640
   gcccgaaatc gccgtttttt gccccaaatt ccccaccgcg gcgttaaaaa cataaagaaa 11700
   ttaagcatca aaagtgccct tttttggggt tgttttgacc caaaaaaaaa atggccgaat 11760
   tgggggcggc cgttttacgg ttgggttcat tttgggttca aaacagccaa aaatgggaac 11820
   tttgggtttc gaaaacaaca acaacaaaaa aacgggttta ttttgggctc attttgggtg 11880
   tttttgggtc aggaggagaa aaaataggaa gtttgagagc gaaacaacgg ccgcttttgg 11940
   ggggaaaacg gccctttttg gtcaacggcg ggggaaaaaa aaaagcggag tttttggggt 12000
   gaaaaagagc ggttttgggt aaatttgggt tttggggtaa aagtggagga ttttgggcaa 12060
   tgggagttaa aaaatgggtg tttttatggg ggttcggtgc agtttttcct gtttgatggg 12120
   gggtttatta atccgggggg ggggattaat gagaattaat aatgttaata gaaatatctg 12180
   ggaaattaat agcaattatt aattgttaat agttattaat agttctatat atctcacatc 12240
   tacgatacaa tataatatcg ttacaatcat atagtcgata tattacatat aattatcagt 12300
   aataataata agtaacaata attagcagta attaataata ataattaata gtattcgtta 12360
   ataagattat tgataataat taagtagtag cgattaatag agatgggatt tcgtgagaaa 12420
   tggaccaaat ttgggccgtt ttgacccaaa tttttggtgg ttttttttcc gattctttgt 12480
   gaatttcggg tcggattcat cagcaattaa ttacggttat taggggctat tagaggcttt 12540
   taattgggat tattagagac ttttaagcgg atttggggac ttttaagtgg attttatgat 12600
   tcttaagtgg attttgggtg gattttaccg cttttggcgg ccgccaccgc ggtggagctc 12660
   cagcttttgt tccctttagt gagggttaat tgcgcgcttg gcgtaatcat ggtcatagct 12720
   gtttcctgtg tgaaattgtt atccgctcac aattccacac aacatacgag ccggaagcat 12780
   aaagtgtaaa gcctggggtg cctaatgagt gagctaactc acattaattg cgttgcgctc 12840
   actgcccgct ttccagtcgg gaaacctgtc gtgccagctg cattaatgaa tcggccaacg 12900
   cgcggggaga ggcggtttgc gtattgggcg ctcttccgct tcctcgctca ctgactcgct 12960
   gcgctcggtc gttcggctgc ggcgagcggt atcagctcac tcaaaggcgg taatacggtt 13020
   atccacagaa tcaggggata acgcaggaaa gaacatgtga gcaaaaggcc agcaaaaggc 13080
   caggaaccgt aaaaaggccg cgttgctggc gtttttccat aggctccgcc cccctgacga 13140
   gcatcacaaa aatcgacgct caagtcagag gtggcgaaac ccgacaggac tataaagata 13200
   ccaggcgttt ccccctgtaa gctccctcgt gcgctctcct gttc 13244

<210> 56
<211> 41
<212> DNA
<213> Artificial Sequence

<220>
<223> Synthetic Primer

<400> 56
ctatgcgcct tgccagcccg ctcagtccgt cagcgctctc t 41

<210> 57
<211> 54
<212> DNA
<213> Artificial Sequence

<220>
<223> Synthetic Primer

<220>
<221> misc_feature
<222> (26)..(35)
<223> n is a, c, g, or t

<400> 57
cgtatcgcct ccctcgcgcc atcagnnnnn nnnnnggggg gggttcatat gaag 54

<210> 58
<211> 10240
<212> DNA
<213> Artificial Sequence

<220>
<223> Targeting vector

<400> 58
cacctgacgc gccctgtagc ggcgcattaa gcgcggcggg tgtggtggtt acgcgcagcg 60

tgaccgctac acttgccagc gccctagcgc ccgctccttt cgctttcttc ccttcctttc 120

tcgccacgtt cgccggcttt ccccgtcaag ctctaaatcg ggggctccct ttagggttcc 180

gatttagtgc tttacggcac ctcgacccca aaaaacttga ttagggtgat ggttcacgta 240

gtgggccatc gccctgatag acggtttttc gccctttgac gttggagtcc acgttcttta 300

atagtggact cttgttccaa actggaacaa cactcaaccc tatctcggtc tattcttttg 360

atttataagg gattttgccg atttcggcct attggttaaa aaaatgagct gatttaacaa 420

aaatttaacg cgaattttaa caaaatatta acgcttacaa tttccattcg ccattcaggc 480

tgcgcaactg ttgggaaggg cgatcggtgc gggcctcttc gctattacgc cagctggcga 540

aagggggatg tgctgcaagg cgattaagtt gggtaacgcc agggttttcc cagtcacgac 600

gttgtaaaac gacggccagt gagcgcgcgt aatacgactc actatagggc gaattgggta 660

ccgggccccc cctcgagtga aggtcaccaa ggatggagac ttcagagcta tctgggacac 720

gtgttttaac gtttgatcag ccttgcagca aaacagtgtt ttgctctatt gaacagtgtt 780

caacaaaaac gatcagtggt ctggaacaca tctcctacga agaaaggttg cgagagctgg 840

gtctggagaa gaggaggctc tggggcaacc tccctgtggc ctgtcagtgc ttaaaggagg 900

ctcaagagaa agatggagag agactttata cacggagctg tagcaatatg acaaggagaa 960

atggtttaaa atggaaagag ggtagattta ggttagctat caagaagaaa ttcttcactc 1020

agaggtgatg aagcactgta gcaggttgcc cagagaagta ttggatgctt ccttcctggc 1080

agggcccaag gccagactgg atgggttctg ggcagcctgt tctggtggga gatgtccctg 1140

cccgtggcac ggcttggaaa tgggtagcct ttgaggtccc tctcagccca aatcattcta 1200

tgttcttgtg gttccctgca tttcagcctt tgcctgttga ctctggtgcc actgttggac 1260

aattctactc agctgtgttt catccctgcc ccaaagcagt aatcccatgg ataagcaatg 1320

ctctgaggcc ttcaccacca tcagggctcc tgctgaccct agcggcctcc cttgcagcat 1380

gagccctttg ttgtccgcac tgctgctgct gagggtcctg ccagcccccc agggcctttt 1440

ctgcccaggc actgccacct gctaaaaccc agcctgccca tggcagggca tcgcatgtct 1500

gagggatgga acggggcaag ggcagtgctg caggcagtgg ttcaagaaac gtttagatgt 1560

tgtactgagg gatgtggttt agtgggaaat actggtgata ggtggatggt tggactggag 1620

gatcttagag gtcttttcca accttggtga ttatgtgatt ctatgaaggg agccatagcc 1680

tgctcccatc atgccccgcc agtccggctc tcccgagcac tttggttccg cagcatccag 1740

gcccgctctg gggccggtgt gggcctgggt gctgccacgt gtccccgctg caaggcctgg 1800

ccaaccccgc tgtgccacaa tgcaggggct gggctaggag tgggcaggga ggtgtgggga 1860

gaggtgtagg acgtggctgg gagcgcaggg agttatttgc ataggggggc gtgctgcgga 1920

aggacgcggg tataaaaggg catcgaggtc cccggcacag ccccatcggc gtggggacac 1980

acagctgctg ggattccgcc atggcctggg ctcctctcct cctggcggtg ctcgcccaca 2040

cctcaggtac tcgttgcgcc cggtcgggga ctgtgggcac ggggctctgt cccactgctg 2100

cgcgggcagg gctgtgcgtg cggggccgtc actgattgcc gttttctccc ctctctcctc 2160

tccggcgcgc ctctccaggt tccctggtgc aggcacagtc tgccctgact cagcctgcct 2220

ccgtgtctgg gtctcctgga cagtcgatca ccatctcctg cactggaacc agcagtgacg 2280

ttggtggtta taactatgtc tcctggtacc aacaaaaccc aggcaaagcc cccaaactca 2340

tgatttatga tgtcagtaat cggccctcag ggatttctaa tcgcttctct ggctccaagt 2400

ctggcaacac ggcctccctg accatctctg ggctccaggc tgacgacgag gctgattatt 2460

actgcacctc atatacaaac gacagcaatt ctcaggtatt cggcggaggg accaagctga 2520

ccgtcctagg tgagtcgctc atatgggatc ctaccgttcg tatagcatac attatacgaa 2580

gttatcagac atgataagat acattgatga gtttggacaa accacaacta gaatgcagtg 2640

aaaaaaatgc tttatttgtg aaatttgtga tgctattgct ttatttgtaa ccattataag 2700

ctgcaataaa caagttgggg tgggcgaaga actccagcat gagatccccg cgctggagga 2760

tcatccagcc ggcgtcccgg aaaacgattc cgaagcccaa cctttcatag aaggcggcgg 2820

tggaatcgaa atctcgtagc acgtgtcagt cctgctcctc ggccacgaag tgcttagccc 2880

tcccacacat aaccagaggg cagcaattca cgaatcccaa ctgccgtcgg ctgtccatca 2940

ctgtccttca ctatggcttt gatcccagga tgcagatcga gaagcacctg tcggcaccgt 3000

ccgcaggggc tcaagatgcc cctgttctca tttccgatcg cgacgataca agtcaggttg 3060

ccagctgccg cagcagcagc agtgcccagc accacgagtt ctgcacaagg tcccccagta 3120

aaatgatata cattgacacc agtgaagatg cggccgtcgc tagagagagc tgcgctggcg 3180

acgctgtagt cttcagagat ggggatgctg ttgattgtag ccgttgctct ttcaatgagg 3240

gtggattctt cttgagacaa aggcttggcc atggtttagt tcctcacctt gtcgtattat 3300

actatgccga tatactatgc cgatgattaa ttgtcaacac gtgctgatca gatccgaaaa 3360

tggatataca agctcccggg agctttttgc aaaagcctag gcctccaaaa aagcctcctc 3420

actacttctg gaatagctca gaggcagagg cggcctcggc ctctgcataa ataaaaaaaa 3480

ttagtcagcc atggggcgga gaatgggcgg aactgggcgg agttaggggc gggatgggcg 3540

gagttagggg cgggactatg gttgctgact aattgagatg catgctttgc atacttctgc 3600

ctgctgggga gcctggggac tttccacacc tggttgctga ctaattgaga tgcatgcttt 3660

gcatacttct gcctgctggg gagcctgggg actttccaca ccctaactga cacacattcc 3720

acagataact tcgtatagca tacattatac gaaatggcgg atccactagt tctagagacc 3780

tcgtctcggt ctttcttccc ccatcgtgaa attgtgacat tttgtcgatt tttggtgatt 3840

tgggggtttt tcttggactt ggcggcaggc tggggtctgc caccggcgca gggccgggca 3900

ctcagcgcga cagcctgggc tgagtcttgt ccccaccgag ccggagggct ccggtgtgcg 3960

ccatggagga cttagggtta ttttgtcaat ggaaagttct taaaatttga ccagaaaatg 4020

tgcccgaggt ctgtctctgc cacacgattt cagaaattgt gtctaggtcg atgagaagac 4080

agtttttgtc tttgtcagga aattagttgt gagttgttag tccttccctc ttagtcctaa 4140

ggactaagac ctttgtcccc ggtctggtct ctcactgggg actcttggct ccagtgccat 4200

ggggagccca agtgtcactg acacggtgtc cttgggggtg aaattcagtt tttcagctgt 4260

ccaggtaagt gctgtcaaaa aatggaggtt attctgctga aaaagctgag aggaatattt 4320

tgtcattttt ttcggaaata tatatatata tatatatata aatatataaa ttatttatat 4380

attttatata tatatatata taaaatatat gtatttctct ctttcttttt atatatatat 4440

ataaatatat atatatttct ccctctttct ttatatatat ttatagggag agaatctata 4500

tttttggcca atttggccaa tttctctctc tctctcttta tatatatata tatatgtata 4560

tgtaatttat atatatatat ataaatttat atttatttat ttatttattt atatagatta 4620

tttttatata catatatata tataaatttg gccaatttag aagaaattgg ccaaaaaaac 4680

ccaatctgaa ccaaattact tagggcataa gtccaaaaaa aaagtgcagt tcaagaattc 4740

ctcgctggaa agtttttaat gggggcagtt cttccccatt ttctggtgta gatatgagtg 4800

aagatataga tatgtatgta gatatagatt tggctatagt ttgtttgatg aacagtctga 4860

ggaagagtaa atcttgcctt cctcatggca atttttaatt ctacttcttc tgagacaagt 4920

ctgaacatgc cttgaggtag aaagagcccg aaaattgagt gctttttctt tgaaatttta 4980

attaaaatta aaaaatagcc ttacagcagg aaagaaaggg ggtgatggca gctaatcgga 5040

gttccaaagc tgaaccacgt tcacccaaac acgtggttga aattttgtgt gtgtatatat 5100

ataaatatat atatgtatac aaaagaaaat gcctatatat aaaaaaattg ttatgtatat 5160

tatatagatt tattatagat atgtacataa gatataacac gtatacatat atatgtagtc 5220

tgctgtatgc tttgtataaa tttttataga tatgatgtat attatatgca tgggttatat 5280

atctataata tattttatct gtagaaggaa ctacagtata aaatgcatat atgcgtatat 5340

atacacatat ttatatatat gtgtatatat atgtatacgc acacgtataa catccatgaa 5400

aatagatatg gatgggtgga tgtgtttgtt ttacagaggt gcatgtgtgt ctgtacatac 5460

acagccatac atacgcgtgt ggcgcgcctc tgcctctctc ttgcaggtca gcccaaggct 5520

gccccctcgg tcactctgtt cccgccctcc tctgaggagc ttcaagccaa caaggccaca 5580

ctggtgtgtc tcataagtga cttctacccg ggagccgtga cagtggcctg gaaggcagat 5640

agcagccccg tcaaggcggg agtggagacc accacaccct ccaaacaaag caacaacaag 5700

tacgcggcca gcagctacct gagcctgacg cctgagcagt ggaagtccca cagaagctac 5760

agctgccagg tcacgcatga agggagcacc gtggagaaga cagtggcccc tacagaatgt 5820

tcatagtagt cccactccat atgtcccact ggggatgcaa tgtgaggaca gtggttcctc 5880

accctccctg tccctctggg ccgctgctgg tggcagcagc cctcacttcc cactcagatg 5940

tcccccaccg tgcccccatc acccacctct gcctgtcgac tcctcttgcc ctcatctctc 6000

caggtgtcac attaataaac ggatccacga cactgaacta gtgctgactc tgcatccatg 6060

tctctgtgtc cttttgcgtg ctgtctgcat ctcacacagg ggggtcggcc ccagtatggg 6120

gaagggctgg ggggcgcata cacacatatt ggtaatgttg ggggcggggg gggggggtgg 6180

gggggtcaac agatcagcac tggagacact ggtgtatacc ctggcaccac caacatctaa 6240

ggcagggtgc tttggggcaa ttttggggca gtttaaggtc tgtgctggca ctgagcacgt 6300

ggctgtggcc gtgctgtcct catctcccac ccactacggt ctgtgcgcca ggtccctagc 6360

agagatttgc tttatgctgg gaacaggggg agttctggct ctgttccctt gcattcagac 6420

accctggtgc cccctgggtg ggatgtcagt gtgaatactc ctttgtgccc tgtgcctgca 6480

gcagcctgac cctccacaca ccacacgcct tgtgtgcacc ccacccctgt cactatccct 6540

ctccccgctc cccagggaga ttttgcagtg gcccctgtag ggcagctttt agcacagccc 6600

ccagcagcaa gcaagcagaa agcactgctg tgcacagctt gtcagctgtg tgtgtttgct 6660

gaggaggatc tgtcttttgc tgaggccatc agtcttgtcc tgctcaacct ccatcgatgc 6720

tgcccacctc aacacatcta cccatctatt ccatctacac caacatctcc attcatccca 6780

cccacccaaa catgtccatc catcacaaca cctccatcca acccgcacac tccagcacct 6840

ccaatcattc catctacacc accatgctga tctgctacag ccactccaac gcaaccgtcc 6900

attccatcta caccaatgtc catccatccc agccactcca gcacctccag ccatcccacc 6960

caccctatgt ctccatccag ccactggtgg ggtgcaggac atggggccag ctctactgtc 7020

aggactgggg tttttgcatg gccccatacc acttctgcag aagagacgca ctgaaagttt 7080

ggctgaccat ttctccacgg gtagagttgt ggcagttctg taatttaggg tcttttatcc 7140

agtttggaga tgggctggga tctcccagct ccatggcagg cattcatgac actgggttta 7200

gtatctgatg ggtgggatgt ggctgaactt catttctttt ccccagtgac aaagtttttg 7260

cagttgaata tgaattcctg ctttctgctc tatgagttgt ttttttccca ggacgtacac 7320

agggaatcag cagtcttcat tctccctctg ccatgtgtag actctctgcc acacaggact 7380

gtgctgtcct catgcccctg cgcccaaatt gctgccctct gcccatgcct gccaagctga 7440

gccctgcagg ctgccatgct ggattgacat gagccctgag attggtacag aaatggtgat 7500

tttgggtttt tctttgcact caggaagctg aaggctcaat gctcagtgat ggatttacca 7560

aactgtgccc tgaggcagct gctcatgctg gataaagtca ctggagcaca gggaaccagg 7620

cgctgggcag ggatttctca tgggccccac ttggaaagct gcaggctgca aacctggctc 7680

tgccttcacg cctcaccctc atgaggacaa cctcactaat tattgattaa aagattttgc 7740

taaaccatct ccagaagcaa caacccactg aggagcatgt gctgaattat acatcacagc 7800

tccacggccc tgccctcata gcagggctgc atggcaccca cagtggcact cagagggacc 7860

acagggctga gacagccggg tctggtggtg gggacacagc tgagcatagg atgagccccc 7920

cgggcagtgc tgggctttgc taatgagcag aagtatggat agaaagcaac cccagggctc 7980

cgtacccagc tgcagctctt gctctgtcgt gtcctttggc ggccgccacc gcggtggagc 8040

tccagctttt gttcccttta gtgagggtta attgcgcgct tggcgtaatc atggtcatag 8100

ctgtttcctg tgtgaaattg ttatccgctc acaattccac acaacatacg agccggaagc 8160

ataaagtgta aagcctgggg tgcctaatga gtgagctaac tcacattaat tgcgttgcgc 8220

tcactgcccg ctttccagtc gggaaacctg tcgtgccagc tgcattaatg aatcggccaa 8280

cgcgcgggga gaggcggttt gcgtattggg cgctcttccg cttcctcgct cactgactcg 8340

ctgcgctcgg tcgttcggct gcggcgagcg gtatcagctc actcaaaggc ggtaatacgg 8400

ttatccacag aatcagggga taacgcagga aagaacatgt gagcaaaagg ccagcaaaag 8460

gccaggaacc gtaaaaaggc cgcgttgctg gcgtttttcc ataggctccg cccccctgac 8520

gagcatcaca aaaatcgacg ctcaagtcag aggtggcgaa acccgacagg actataaaga 8580

taccaggcgt ttccccctgg aagctccctc gtgcgctctc ctgttccgac cctgccgctt 8640

accggatacc tgtccgcctt tctcccttcg ggaagcgtgg cgctttctca tagctcacgc 8700

tgtaggtatc tcagttcggt gtaggtcgtt cgctccaagc tgggctgtgt gcacgaaccc 8760

cccgttcagc ccgaccgctg cgccttatcc ggtaactatc gtcttgagtc caacccggta 8820

agacacgact tatcgccact ggcagcagcc actggtaaca ggattagcag agcgaggtat 8880

gtaggcggtg ctacagagtt cttgaagtgg tggcctaact acggctacac tagaaggaca 8940

gtatttggta tctgcgctct gctgaagcca gttaccttcg gaaaaagagt tggtagctct 9000

tgatccggca aacaaaccac cgctggtagc ggtggttttt ttgtttgcaa gcagcagatt 9060

acgcgcagaa aaaaaggatc tcaagaagat cctttgatct tttctacggg gtctgacgct 9120

cagtggaacg aaaactcacg ttaagggatt ttggtcatga gattatcaaa aaggatcttc 9180

acctagatcc ttttaaatta aaaatgaagt tttaaatcaa tctaaagtat atatgagtaa 9240

acttggtctg acagttacca atgcttaatc agtgaggcac ctatctcagc gatctgtcta 9300

tttcgttcat ccatagttgc ctgactcccc gtcgtgtaga taactacgat acgggagggc 9360

ttaccatctg gccccagtgc tgcaatgata ccgcgagacc cacgctcacc ggctccagat 9420

ttatcagcaa taaaccagcc agccggaagg gccgagcgca gaagtggtcc tgcaacttta 9480

tccgcctcca tccagtctat taattgttgc cgggaagcta gagtaagtag ttcgccagtt 9540

aatagtttgc gcaacgttgt tgccattgct acaggcatcg tggtgtcacg ctcgtcgttt 9600

ggtatggctt cattcagctc cggttcccaa cgatcaaggc gagttacatg atcccccatg 9660

ttgtgcaaaa aagcggttag ctccttcggt cctccgatcg ttgtcagaag taagttggcc 9720

gcagtgttat cactcatggt tatggcagca ctgcataatt ctcttactgt catgccatcc 9780

gtaagatgct tttctgtgac tggtgagtac tcaaccaagt cattctgaga atagtgtatg 9840

cggcgaccga gttgctcttg cccggcgtca atacgggata ataccgcgcc acatagcaga 9900

actttaaaag tgctcatcat tggaaaacgt tcttcggggc gaaaactctc aaggatctta 9960

ccgctgttga gatccagttc gatgtaaccc actcgtgcac ccaactgatc ttcagcatct 10020

tttactttca ccagcgtttc tgggtgagca aaaacaggaa ggcaaaatgc cgcaaaaaag 10080

ggaataaggg cgacacggaa atgttgaata ctcatactct tcctttttca atattattga 10140

agcatttatc agggttattg tctcatgagc ggatacatat ttgaatgtat ttagaaaaat 10200

aaacaaatag gggttccgcg cacatttccc cgaaaagtgc 10240

<210> 59
<211> 26
<212> DNA
<213> Artificial Sequence

<220>
<223> Synthetic DNA

<400> 59
gttaaccggt atgcatcgat ttaaat 26

<210> 60
<211> 11743
<212> DNA
<213> Artificial Sequence

<220>
<223> Targeting vector

<400> 60
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgggta tcacgtgggg caaatcgaga 420

gctaccattg gctatgggga ggagaaaatg aggcgtgcta cttcctcgtt cctgcagtgc 480

gtgtcgaggt gccgtgttgg tcggatctca gtggcagagc aggatggtga gcggtggcct 540

tggggccgct ccctcactct gctgggctgg tgcggtggct ccgggaccgg actttcagtg 600

gtgtttggtg tggtgctcac tgtcctttgg tgtcactgtg tggtgaattc cctcagctgg 660

gcggtgagag cccatgctgt gtcctggtag gagtagtctg ggctgcagtg cgggctcttt 720

gtgcgtgttg ggggcggttc cctggaagct aattctggaa gaaatcccgg ctgcctctgt 780

aacagttgtt ggcgatgtca aacaccctgg agctgctgac tggctctgta ccattgagtg 840

atgcagttta aacctctctc tttcctcctg ctgtgtgtga agctggtgtt agtgctagga 900

gacctgcaca tccctcaccg atgcagtggt ttaccagcaa agttcaagag cctcctggtt 960

ccagggaaga ttcagcatat tttgtgtaca ggaaacctct gcaccaagga gagctatgac 1020

tacctcagga ctttggctgg agatgttcac gttgttaagg gggactctga ggtaatgttc 1080

ttgctgctgt gaattcctca gtcctccatc ttacaagccg tagttgtccc actgaaagct 1140

gggaaaccct tcggtgtttg caattcctga gcgctaatgt gtgggggaag gatgtaggaa 1200

caaataaggc tcaagagaca acaacttcct aggctcctct tattgccctt gtggtaaggg 1260

aggacttggt gtgtagtggt aatacatgca ctcaacttat taagttctca tagctgctgt 1320

gaagctgctg cagtccctca gatccctgca ggaagagggg actgactgac tgttgaatag 1380

ccttgaagct cctgctctgc atgtggttcc ggtcaggctc aagatcccag ttcttagctc 1440

ttcagttgct cttctggggg tatagtacta gcatctgggt cactgcagat ggacagagac 1500

tgtgcttggg tagcaaaagg cagacttacg ttgtgaaacc ttcattgtgt tggtgaggtt 1560

ctttgatttc taccttgtgt atctctcttc ctcagagtct gaattatcct gaacagaagg 1620

ttgtaactgt tgggcagttc agaattgggt tgattcatgg ccatcaagtc attccctggg 1680

gtgatgtggc aagcttggca ttgctgcaga ggcagctgga tgtagacatt cttatctcag 1740

gacatacaca cagatttgaa gcatttgagc atgaaaataa gttctacatc aacccaggat 1800

cagctacagg agcctacact ggtttacaaa cgtgagtaca atgagccttg tgtttgtctt 1860

taagacagat gtctgtcctt aagagtgcag cccatgtgtt gaagagcact ggtgagatgc 1920

agtgccatgc aggccatctc tccctttccc agagaacacg gcctacctct ctctgttcat 1980

aaggtcttgg ttaacttatg agttggccag cacagcctgg cactgatgtg tcatgatcat 2040

aaccctgagc ccacgctctg ctatgctgtt gcttcttaga gttgcattgc cttgaggtgt 2100

attccagaag ctgtgtgctg agctcttgtt gggaaagagg ccctcagggc actgccaaca 2160

tctgaggctt gcaggagctg gaacattcag ccctgagggc tgctctggcc aactgcagct 2220

ctgagtctgc agtcagtgcc tctgagctgt gatgcttctc agataactgc agccctttgc 2280

aatgtaatgt tttataatat atttccatag gacatcttgg taagaggtgt aagctaatca 2340

gggtgattga caaagtatgt tttctttcca ggaacgtcat tccttcattt gtactgatgg 2400

atatccaggc ttccacagtt gtgacttatg tataccagct aattgaggac gatgtgaaag 2460

tagaaagaat tgagttcaag aaatactgac acgtgtttga atctgcctgc tgacttctca 2520

ctgcctctca ttctttctct cctagttaaa gctgaatccg gccacgtggg ggtgctgctg 2580

cagcggcgtg taactgtgag cttgggaagt tctcaactag ggcttagagc aggagaaact 2640

gattaacaga aattgcctta ttaaatgtat atatttcctg tgtctaagaa cgaggtgctg 2700

ttgttcctga cttgcggagc tgcagctagt ggtagcttgg aatttaacct ctcctgtaaa 2760

atgagcatag aataaaacag tattttcaca tttctggatc ctgttaattg tttgttaatt 2820

aagaggacag gaattcagtg tcagggggct gctctttgct tcccaatgtg taccactgaa 2880

gcctctctgg atttatcaga gatactcaat ggactcttaa cagcactgtg gcacctgacc 2940

tcctgctgcc ttcagcctcc cttcctggct ggtgtgctta tgttgaggag ctgtgggagg 3000

ctggtgcagt tctgtgggat gaggtaaggc cagctgaata gcctgggggc tggtcagggg 3060

gcttagtgag aggagcaggg ctggggaatg ttgtgaacag caggtggtgc cactgctatt 3120

ctgaggaagg aggcagcttc tgcaaatgct ttgcaatgca ggatgacaaa gtgcttctgc 3180

tagccctagg ctaccgttcg tataatgtat actatacgaa gttattttat ttgtgaaatt 3240

tgtgatgcta ttgctttatt tgtaaccatt ataagctgca ataaacaagt tggggtgggc 3300

gaagaactcc agcatgagat ccccgcgctg gaggatcatc cagccggcgt cccggaaaac 3360

gattccgaag cccaaccttt catagaaggc ggcggtggaa tcgaaatctc gtgatggcag 3420

gttgggcgtc gcttggtcgg tcatttcgaa ccccagagtc ccgctcagaa gaactcgtca 3480

agaaggcgat agaaggcgat gcgctgcgaa tcgggagcgg cgataccgta aagcacgagg 3540

aagcggtcag cccattcgcc gccaagctct tcagcaatat cacgggtagc caacgctatg 3600

tcctgatagc ggtccgccac acccagccgg ccacagtcga tgaatccaga aaagcggcca 3660

ttttccacca tgatattcgg caagcaggca tcgccatggg tcacgacgag atcctcgccg 3720

tcgggcatgc gcgccttgag cctggcgaac agttcggctg gcgcgagccc ctgatgctct 3780

tcgtccagat catcctgatc gacaagaccg gcttccatcc gagtacgtgc tcgctcgatg 3840

cgatgtttcg cttggtggtc gaatgggcag gtagccggat caagcgtatg cagccgccgc 3900

attgcatcag ccatgatgga tactttctcg gcaggagcaa ggtgagatga caggagatcc 3960

tgccccggca cttcgcccaa tagcagccag tcccttcccg cttcagtgac aacgtcgagc 4020

acagctgcgc aaggaacgcc cgtcgtggcc agccacgata gccgcgctgc ctcgtcctgc 4080

agttcattca gggcaccgga caggtcggtc ttgacaaaaa gaaccgggcg cccctgcgct 4140

gacagccgga acacggcggc atcagagcag ccgattgtct gttgtgccca gtcatagccg 4200

aatagcctct ccacccaagc ggccggagaa cctgcgtgca atccatcttg ttcaatcatg 4260

cgaaacgatc ctcatcctgt ctcttgatca gatccgaaaa tggatataca agctcccggg 4320

agctttttgc aaaagcctag gcctccaaaa aagcctcctc actacttctg gaatagctca 4380

gaggcagagg cggcctcggc ctctgcataa ataaaaaaaa ttagtcagcc atggggcgga 4440

gaatgggcgg aactgggcgg agttaggggc gggatgggcg gagttagggg cgggactatg 4500

gttgctgact aattgagatg catgctttgc atacttctgc ctgctgggga gcctggggac 4560

tttccacacc tggttgctga ctaattgaga tgcatgcttt gcatacttct gcctgctggg 4620

gagcctgggg actttccaca ccctaaataa cttcgtataa tgtatactat acgaacggta 4680

gcgttaaccg gtatgcatcg atttaaatcg agtgaaggtc accaaggatg gagacttcag 4740

agctatctgg gacacgtgtt ttaacgtttg atcagccttg cagcaaaaca gtgttttgct 4800

ctattgaaca gtgttcaaca aaaacgatca gtggtctgga acacatctcc tacgaagaaa 4860

ggttgcgaga gctgggtctg gagaagagga ggctctgggg caacctccct gtggcctgtc 4920

agtgcttaaa ggaggctcaa gagaaagatg gagagagact ttatacacgg agctgtagca 4980

atatgacaag gagaaatggt ttaaaatgga aagagggtag atttaggtta gctatcaaga 5040

agaaattctt cactcagagg tgatgaagca ctgtagcagg ttgcccagag aagtattgga 5100

tgcttccttc ctggcagggc ccaaggccag actggatggg ttctgggcag cctgttctgg 5160

tgggagatgt ccctgcccgt ggcacggctt ggaaatgggt agcctttgag gtccctctca 5220

gcccaaatca ttctatgttc ttgtggttcc ctgcatttca gcctttgcct gttgactctg 5280

gtgccactgt tggacaattc tactcagctg tgtttcatcc ctgccccaaa gcagtaatcc 5340

catggataag caatgctctg aggccttcac caccatcagg gctcctgctg accctagcgg 5400

cctcccttgc agcatgagcc ctttgttgtc cgcactgctg ctgctgaggg tcctgccagc 5460

cccccagggc cttttctgcc caggcactgc cacctgctaa aacccagcct gcccatggca 5520

gggcatcgca tgtctgaggg atggaacggg gcaagggcag tgctgcaggc agtggttcaa 5580

gaaacgttta gatgttgtac tgagggatgt ggtttagtgg gaaatactgg tgataggtgg 5640

atggttggac tggaggatct tagaggtctt ttccaacctt ggtgattatg tgattctatg 5700

aagggagcca tagcctgctc ccatcatgcc ccgccagtcc ggctctcccg agcactttgg 5760

ttccgcagca tccaggcccg ctctgggccc ggtgtgggcc tgggtgctgc cacgtgtccc 5820

cgctgcaagg cctggccaac cccgctgtgc cacaatgcag gggctgggct aggagtgggc 5880

agggaggtgt ggggagaggt gtaggacgtg gctgggagcg cagggagtta tttgcatagg 5940

ggggcgtgct gcggaaggac gcgggtataa aagggcatcg aggtccccgg cacagcccca 6000

tcggcgtggg gacacacagc tgctgggatt ccgccatggc ctgggctcct ctcctcctgg 6060

cggtgctcgc ccacacctca ggtactcgtt gcgcccggtc ggggactgtg ggcacggggc 6120

tctgtcccac tgctgcgcgg gcagggctgt gcgtgcgggg ccgtcactga ttgccgtttt 6180

ctcccctctc tcctctccgg cgcgcctctc caggttccct ggtgcaggca cagtctgccc 6240

tgactcagcc tgcctccgtg tctgggtctc ctggacagtc gatcaccatc tcctgcactg 6300

gaaccagcag tgacgttggt ggttataact atgtctcctg gtaccaacaa aacccaggca 6360

aagcccccaa actcatgatt tatgatgtca gtaatcggcc ctcagggatt tctaatcgct 6420

tctctggctc caagtctggc aacacggcct ccctgaccat ctctgggctc caggctgacg 6480

acgaggctga ttattactgc acctcatata caaacgacag caattctcag gtattcggcg 6540

gagggaccaa gctgaccgtc ctaggtgagt cgctcatatg ctaccgttcg tatagcatac 6600

attatacgaa gttatcagac atgataagat acattgatga gtttggacaa accacaacta 6660

gaatgcagtg aaaaaaatgc tttatttgtg aaatttgtga tgctattgct ttatttgtaa 6720

ccattataag ctgcaataaa caagttgggg tgggcgaaga actccagcat gagatccccg 6780

cgctggagga tcatccagcc ggcgtcccgg aaaacgattc cgaagcccaa cctttcatag 6840

aaggcggcgg tggaatcgaa atctcgtagc acgtgtcagt cctgctcctc ggccacgaag 6900

tgcttagccc tcccacacat aaccagaggg cagcaattca cgaatcccaa ctgccgtcgg 6960

ctgtccatca ctgtccttca ctatggcttt gatcccagga tgcagatcga gaagcacctg 7020

tcggcaccgt ccgcaggggc tcaagatgcc cctgttctca tttccgatcg cgacgataca 7080

agtcaggttg ccagctgccg cagcagcagc agtgcccagc accacgagtt ctgcacaagg 7140

tcccccagta aaatgatata cattgacacc agtgaagatg cggccgtcgc tagagagagc 7200

tgcgctggcg acgctgtagt cttcagagat ggggatgctg ttgattgtag ccgttgctct 7260

ttcaatgagg gtggattctt cttgagacaa aggcttggcc atggtttagt tcctcacctt 7320

gtcgtattat actatgccga tatactatgc cgatgattaa ttgtcaacac gtgctgatca 7380

gatccgaaaa tggatataca agctcccggg agctttttgc aaaagcctag gcctccaaaa 7440

aagcctcctc actacttctg gaatagctca gaggcagagg cggcctcggc ctctgcataa 7500

ataaaaaaaa ttagtcagcc atggggcgga gaatgggcgg aactgggcgg agttaggggc 7560

gggatgggcg gagttagggg cgggactatg gttgctgact aattgagatg catgctttgc 7620

atacttctgc ctgctgggga gcctggggac tttccacacc tggttgctga ctaattgaga 7680

tgcatgcttt gcatacttct gcctgctggg gagcctgggg actttccaca ccctaactga 7740

cacacattcc acagataact tcgtatagca tacattatac gaacggtagc tctagagacc 7800

tcgtctcggt ctttcttccc ccatcgtgaa attgtgacat tttgtcgatt tttggtgatt 7860

tgggggtttt tcttggactt ggcggcaggc tggggtctgc caccggcgca gggccgggca 7920

ctcagcgcga cagcctgggc tgagtcttgt ccccaccgag ccggagggct ccggtgtgcg 7980

ccatggagga cttagggtta ttttgtcaat ggaaagttct taaaatttga ccagaaaatg 8040

tgcccgaggt ctgtctctgc cacacgattt cagaaattgt gtctaggtcg atgagaagac 8100

agtttttgtc tttgtcagga aattagttgt gagttgttag tccttccctc ttagtcctaa 8160

ggactaagac ctttgtcccc ggtctggtct ctcactgggg actcttggct ccagtgccat 8220

ggggagccca agtgtcactg acacggtgtc cttgggggtg aaattcagtt tttcagctgt 8280

ccaggtaagt gctgtcaaaa aatggaggtt attctgctga aaaagctgag aggaatattt 8340

tgtcattttt ttcggaaata tatatatata tatatatata aatatataaa ttatttatat 8400

attttatata tatatatata taaaatatat gtatttctct ctttcttttt atatatatat 8460

ataaatatat atatatttct ccctctttct ttatatatat ttatagggag agaatctata 8520

tttttggcca atttggccaa tttctctctc tctctcttta tatatatata tatatgtata 8580

tgtaatttat atatatatat ataaatttat atttatttat ttatttattt atatagatta 8640

tttttatata catatatata tataaatttg gccaatttag aagaaattgg ccaaaaaaac 8700

ccaatctgaa ccaaattact tagggcataa gtccaaaaaa aaagtgcagt tcaagaattc 8760

ctcgctggaa agtttttaat gggggcagtt cttccccatt ttctggtgta gatatgagtg 8820

aagatataga tatgtatgta gatatagatt tggctatagt ttgtttgatg aacagtctga 8880

ggaagagtaa atcttgcctt cctcatggca atttttaatt ctacttcttc tgagacaagt 8940

ctgaacatgc cttgaggtag aaagagcccg aaaattgagt gctttttctt tgaaatttta 9000

attaaaatta aaaaatagcc ttacagcagg aaagaaaggg ggtgatggca gctaatcgga 9060

gttccaaagc tgaaccacgt tcacccaaac acgtggttga aattttgtgt gtgtatatat 9120

ataaatatat atatgtatac aaaagaaaat gcctatatat aaaaaaattg ttatgtatat 9180

tatatagatt tattatagat atgtacataa gatataacac gtatacatat atatgtagtc 9240

tgctgtatgc tttgtataaa tttttataga tatgatgtat attatatgca tgggttatat 9300

atctataata tattttatct gtagaaggaa ctacagtata aaatgcatat atgcgtatat 9360

atacacatat ttatatatat gtgtatatat atgtatacgc acacgtataa catccatgaa 9420

aatagatatg gatgggtgga tgtgtttgtt ttacagaggt gcatgtgtgt ctgtacatac 9480

acagccatac atacgcgtgt gcggccgcag gcgtaatcat ggtcatagct gtttcctgtg 9540

tgaaattgtt atccgctcac aattccacac aacatacgag ccggaagcat aaagtgtaaa 9600

gcctggggtg cctaatgagt gagctaactc acattaattg cgttgcgctc actgcccgct 9660

ttccagtcgg gaaacctgtc gtgccagctg cattaatgaa tcggccaacg cgcggggaga 9720

ggcggtttgc gtattgggcg ctcttccgct tcctcgctca ctgactcgct gcgctcggtc 9780

gttcggctgc ggcgagcggt atcagctcac tcaaaggcgg taatacggtt atccacagaa 9840

tcaggggata acgcaggaaa gaacatgtga gcaaaaggcc agcaaaaggc caggaaccgt 9900

aaaaaggccg cgttgctggc gtttttccat aggctccgcc cccctgacga gcatcacaaa 9960

aatcgacgct caagtcagag gtggcgaaac ccgacaggac tataaagata ccaggcgttt 10020

ccccctggaa gctccctcgt gcgctctcct gttccgaccc tgccgcttac cggatacctg 10080

tccgcctttc tcccttcggg aagcgtggcg ctttctcata gctcacgctg taggtatctc 10140

agttcggtgt aggtcgttcg ctccaagctg ggctgtgtgc acgaaccccc cgttcagccc 10200

gaccgctgcg ccttatccgg taactatcgt cttgagtcca acccggtaag acacgactta 10260

tcgccactgg cagcagccac tggtaacagg attagcagag cgaggtatgt aggcggtgct 10320

acagagttct tgaagtggtg gcctaactac ggctacacta gaagaacagt atttggtatc 10380

tgcgctctgc tgaagccagt taccttcgga aaaagagttg gtagctcttg atccggcaaa 10440

caaaccaccg ctggtagcgg tggttttttt gtttgcaagc agcagattac gcgcagaaaa 10500

aaaggatctc aagaagatcc tttgatcttt tctacggggt ctgacgctca gtggaacgaa 10560

aactcacgtt aagggatttt ggtcatgaga ttatcaaaaa ggatcttcac ctagatcctt 10620

ttaaattaaa aatgaagttt taaatcaatc taaagtatat atgagtaaac ttggtctgac 10680

agttaccaat gcttaatcag tgaggcacct atctcagcga tctgtctatt tcgttcatcc 10740

atagttgcct gactccccgt cgtgtagata actacgatac gggagggctt accatctggc 10800

cccagtgctg caatgatacc gcgagaccca cgctcaccgg ctccagattt atcagcaata 10860

aaccagccag ccggaagggc cgagcgcaga agtggtcctg caactttatc cgcctccatc 10920

cagtctatta attgttgccg ggaagctaga gtaagtagtt cgccagttaa tagtttgcgc 10980

aacgttgttg ccattgctac aggcatcgtg gtgtcacgct cgtcgtttgg tatggcttca 11040

ttcagctccg gttcccaacg atcaaggcga gttacatgat cccccatgtt gtgcaaaaaa 11100

gcggttagct ccttcggtcc tccgatcgtt gtcagaagta agttggccgc agtgttatca 11160

ctcatggtta tggcagcact gcataattct cttactgtca tgccatccgt aagatgcttt 11220

tctgtgactg gtgagtactc aaccaagtca ttctgagaat agtgtatgcg gcgaccgagt 11280

tgctcttgcc cggcgtcaat acgggataat accgcgccac atagcagaac tttaaaagtg 11340

ctcatcattg gaaaacgttc ttcggggcga aaactctcaa ggatcttacc gctgttgaga 11400

tccagttcga tgtaacccac tcgtgcaccc aactgatctt cagcatcttt tactttcacc 11460

agcgtttctg ggtgagcaaa aacaggaagg caaaatgccg caaaaaaggg aataagggcg 11520

acacggaaat gttgaatact catactcttc ctttttcaat attattgaag catttatcag 11580

ggttattgtc tcatgagcgg atacatattt gaatgtattt agaaaaataa acaaataggg 11640

gttccgcgca catttccccg aaaagtgcca cctgacgtct aagaaaccat tattatcatg 11700

acattaacct ataaaaatag gcgtatcacg aggccctttc gtc 11743

<210> 61
<211> 7001
<212> DNA
<213> Homo sapiens

<400> 61
accggtcgac atgcaagctt gctagcgttt aaacacgagt gcgtacgctc gacaagacgc 60

actccagtct gccctgactc agcctgcctc cgtgtctggg tctcctggac agtcgatcac 120

catctcctgc tctggaagca gctccaacat cggaagtaat tatgtatact ggtaccaaca 180

gcacccaggc aaagccccca aactcatgat ttataggaat aatcagcggc cctcaggggt 240

ttctaatcgc ttctctggct ccaagtctgg caacacggcc tccctgacca tctctgggct 300

ccaggctgag gacgaggctg attattactg cgcagcatgg gatgacagcc tgagtggtca 360

ttgggtgttc ggcggaggga ccaagctgac cgtcctagcc tgacacacac agccctcccc 420

agactgtggg atgaggccct gtgcccgcag tcacatgtgg aatatcaaga cacacacatc 480

tatgacaatc accgtgtctc tactcgcgtg tctctctatg cgcagtctgc cctgactcag 540

cctgcctccg tgtctgggtc tcctggacag tcgatcacca tctcctgcac tgggagcagc 600

tccaacatcg gggcaggtta tgatgtacac tggtaccaac agcacccagg caaagccccc 660

aaactcatga tttatggtaa cagcaatcgg ccctcagggg tttctaatcg cttctctggc 720

tccaagtctg gcaacacggc ctccctgacc atctctgggc tccaggctga ggacgaggct 780

gattattact gccagtccta tgacagcagc ctgagtggtt ctgtggtatt cggcggaggg 840

accaagctga ccgtcctagc ctgacacaca cagccctccc cagactgtgg gatgaggccc 900

tgtgcccgca gtcacatgtg gaatatcaag acacacacat ctatgacaat cacatacgac 960

gtatcacgta ctacgtctag taccagtctg ccctgactca gcctgcctcc gtgtctgggt 1020

ctcctggaca gtcgatcacc atctcctgct ctggaagcag ctccaacatt gggaataatt 1080

atgtatcctg gtaccaacag cacccaggca aagcccccaa actcatgatt tatgacaata 1140

ataagcgacc ctcaggggtt tctaatcgct tctctggctc caagtctggc aacacggcct 1200

ccctgaccat ctctgggctc caggctgagg acgaggctga ttattactgc ggaacatggg 1260

atagcagcct gagtgctggt gctgtgttcg gcggagggac caagctgacc gtcctagcct 1320

gacacacaca gccctcccca gactgtggga tgaggccctg tgcccgcagt cacatgtgga 1380

atatcaagac acacacatct atgacaatca ccgtagacta gtacgagtat gtactctcgt 1440

gcagtctgcc ctgactcagc ctgcctccgt gtctgggtct cctggacagt cgatcaccat 1500

ctcctgcact gggagcagct ccaacattgg ggcgggttat gttgtacatt ggtaccaaca 1560

gcacccaggc aaagccccca aactcatgat ttatgaaaat aataagcgac cctcaggggt 1620

ttctaatcgc ttctctggct ccaagtctgg caacacggcc tccctgacca tctctgggct 1680

ccaggctgag gacgaggctg attattactg cagctcatat acaagcagcg gcactcctgt 1740

ggtattcggc ggagggacca agctgaccgt cctagcctga cacacacagc cctccccaga 1800

ctgtgggatg aggccctgtg cccgcagtca catgtggaat atcaagacac acacatctat 1860

gacaatcaca cgcgagtata ctactatgta ctgtacagtc agtctgccct gactcagcct 1920

gcctccgtgt ctgggtctcc tggacagtcg atcaccatct cctgcactgg aaccagcagt 1980

gatgttggga gttataacct tgtctcctgg taccaacagc acccaggcaa agcccccaaa 2040

ctcatgattt atgagggcag taagcggccc tcaggggttt ctaatcgctt ctctggctcc 2100

aagtctggca acacggcctc cctgaccatc tctgggctcc aggctgagga cgaggctgat 2160

tattactgcc agtcctatga cagcagcctg agtggttgcc agtcctatga cagcagcctg 2220

agtggtttgg tgttcggcgg agggaccaag ctgaccgtcc tagcctgaca cacacagccc 2280

tccccagact gtgggatgag gccctgtgcc cgcagtcaca tgtggaatat caagacacac 2340

acatctatga caatcacata gagtactcac gctacgtcag tagacgtcag tctgccctga 2400

ctcagcctgc ctccgtgtct gggtctcctg gacagtcgat caccatctcc tgcgggggaa 2460

acaacattgg aagtaaaaat gtgcactggt accaacagca cccaggcaaa gcccccaaac 2520

tcatgattta tagggatagc aaccggccct ctggggtttc taatcgcttc tctggctcca 2580

agtctggcaa cacggcctcc ctgaccatct ctgggctcca ggctgaggac gaggctgatt 2640

attactgcca gtcctatgac aggagtggtt gggtgttcgg cggagggacc aagctgaccg 2700

tcctagcctg acacacacag ccctccccag actgtgggat gaggccctgt gcccgcagtc 2760

acatgtggaa tatcaagaca cacacatcta tgacaatcac tacgctgtct tagtgtagat 2820

tcgatcacgt cagtctgccc tgactcagcc tgcctccgtg tctgggtctc ctggacagtc 2880

gatcaccatc tcctgctctg gagaagcatt gccaaaaaaa tatgcttatt ggtaccaaca 2940

gcacccaggc aaagccccca aactcatgat ttataaagac agcgagaggc cctcaggggt 3000

ttctaatcgc ttctctggct ccaagtctgg caacacggcc tccctgacca tctctgggct 3060

ccaggctgag gacgaggctg attattactg caactctttt acaagcacca ccaattatgt 3120

cttattcggc ggagggacca agctgaccgt cctagcctga cacacacagc cctccccaga 3180

ctgtgggatg aggccctgtg cccgcagtca catgtggaat atcaagacac acacatctat 3240

gacaatcact gacgtatgtt gtgagtagta cagtatatac agtctgccct gactcagcct 3300

gcctccgtgt ctgggtctcc tggacagtcg atcaccatct cctgcggggg aaacaacatt 3360

ggaagtaaaa gtgtgcactg gtaccaacag cacccaggca aagcccccaa actcatgatt 3420

tattatgata gcgaccggcc ctcaggggtt tctaatcgct tctctggctc caagtctggc 3480

aacacggcct ccctgaccat ctctgggctc caggctgagg acgaggctga ttattactgc 3540

caggtgtggg atgataatag tgatcatctt atattcggcg gagggaccaa gctgaccgtc 3600

ctagcctgac acacacagcc ctccccagac tgtgggatga ggccctgtgc ccgcagtcac 3660

atgtggaata tcaagacaca cacatctatg acaatcacag tatacataag tcgagagaag 3720

tgctacgaca gtctgccctg actcagcctg cctccgtgtc tgggtctcct ggacagtcga 3780

tcaccatctc ctgctctgga gatgcattgc caaagcaata tgcttattgg taccaacagc 3840

acccaggcaa agcccccaaa ctcatgattt atgatgatag cgaccggccc tcaggggttt 3900

ctaatcgctt ctctggctcc aagtctggca acacggcctc cctgaccatc tctgggctcc 3960

aggctgagga cgaggctgat tattactgca gctcatatgc aggcagcaac aattgggtgt 4020

tcggcggagg gaccaagctg accgtcctag cctgacacac acagccctcc ccagactgtg 4080

ggatgaggcc ctgtgcccgc agtcacatgt ggaatatcaa gacacacaca tctatgacaa 4140

tcaccgtaca gtcacgtact cagactacgc tctacagtct gccctgactc agcctgcctc 4200

cgtgtctggg tctcctggac agtcgatcac catctcctgc accctaagca gtgagcacag 4260

cacctacacc atcgaatggt accaacagca cccaggcaaa gcccccaaac tcatgattta 4320

tgttaagagt gatggcagcc acagcaaggg ggacggggtt tctaatcgct tctctggctc 4380

caagtctggc aacacggcct ccctgaccat ctctgggctc caggctgagg acgaggctga 4440

ttattactgc ggagagagcc acacgattga tggccaagtc ggtgtggtat tcggcggagg 4500

gaccaagctg accgtcctag cctgacacac acagccctcc ccagactgtg ggatgaggcc 4560

ctgtgcccgc agtcacatgt ggaatatcaa gacacacaca tctatgacaa tcactatata 4620

tacatatgct agtatcacgc gagacagtct gccctgactc agcctgcctc cgtgtctggg 4680

tctcctggac agtcgatcac catctcctgc actctgagca gtgggcacag cagctacgcc 4740

atcgcatggt accaacagca cccaggcaaa gcccccaaac tcatgattta tcttaacagt 4800

gatggcagcc acagcaaggg ggacggggtt tctaatcgct tctctggctc caagtctggc 4860

aacacggcct ccctgaccat ctctgggctc caggctgagg acgaggctga ttattactgc 4920

agctcatatg caggcaacaa caatttgggg gatgtgcttt tcggcggagg gaccaagctg 4980

accgtcctag cctgacacac acagccctcc ccagactgtg ggatgaggcc ctgtgcccgc 5040

agtcacatgt ggaatatcaa gacacacaca tctatgacaa tcactgagtc agtatgtagt 5100

gtgatgtcac acgacagtct gccctgactc agcctgcctc cgtgtctggg tctcctggac 5160

agtcgatcac catctcctgc accttgcgca gtggcatcaa tgttggtacc tacaggatat 5220

actggtacca acagcaccca ggcaaagccc ccaaactcat gatttattac aaatcagact 5280

cagataagca gcagggctct ggggtttcta atcgcttctc tggctccaag tctggcaaca 5340

cggcctccct gaccatctct gggctccagg ctgaggacga ggctgattat tactgcgcag 5400

catgggatga cagcctgaat gtggtattcg gcggagggac caagctgacc gtcctagcct 5460

gacacacaca gccctcccca gactgtggga tgaggccctg tgcccgcagt cacatgtgga 5520

atatcaagac acacacatct atgacaatca cacatgacga cacgacagct cacgtctcat 5580

ccagtctgcc ctgactcagc ctgcctccgt gtctgggtct cctggacagt cgatcaccat 5640

ctcctgcacc cgcagcagtg gcagcattgc cagcaactat gtgcagtggt accaacagca 5700

cccaggcaaa gcccccaaac tcatgattta tgaggataac caaagaccct ctggggtttc 5760

taatcgcttc tctggctcca agtctggcaa cacggcctcc ctgaccatct ctgggctcca 5820

ggctgaggac gaggctgatt attactgcca gtcctatgac agcagcctga ggagagtctt 5880

cggcggaggg accaagctga ccgtcctagc ctgacacaca cagccctccc cagactgtgg 5940

gatgaggccc tgtgcccgca gtcacatgtg gaatatcaag acacacacat ctatgacaat 6000

cacagcgact agcagtagtg atcagtgaca caccagtctg ccctgactca gcctgcctcc 6060

gtgtctgggt ctcctggaca gtcgatcacc atctcctgcg gctccagcac tggagctgtc 6120

accagtggtc attatcccta ctggtaccaa cagcacccag gcaaagcccc caaactcatg 6180

atttatgata caagcaacaa acactccggg gtttctaatc gcttctctgg ctccaagtct 6240

ggcaacacgg cctccctgac catctctggg ctccaggctg aggacgaggc tgattattac 6300

tgcgcaacat gggatgacag cctgaatgag ggagtgttcg gcggagggac caagctgacc 6360

gtcctagcct gacacacaca gccctcccca gactgtggga tgaggccctg tgcccgcagt 6420

cacatgtgga atatcaagac acacacatct atgacaatca catctactga ccacgtagat 6480

ccacgtgtcg ccagtctgcc ctgactcagc ctgcctccgt gtctgggtct cctggacagt 6540

cgatcaccat ctcctgcacc ctgagcagcg gctacagtaa ttataaagtg gactggtacc 6600

aacagcaccc aggcaaagcc cccaaactca tgatttatgt gggcactggt gggattgtgg 6660

gatccaaggg ggatggggtt tctaatcgct tctctggctc caagtctggc aacacggcct 6720

ccctgaccat ctctgggctc caggctgagg acgaggctga ttattactgc ggggcagacc 6780

atggcagtgg gagcaacttc gtggtggtat tcggcggagg gaccaagctg accgtcctag 6840

cctgacacac acagccctcc ccagactgtg ggatgaggcc ctgtgcccgc agtcacatgt 6900

ggaatatcaa gacacacaca tctatgacaa tcacgtttaa acaagcttcg aagggcccat 6960

gcgaattccg cggatgcggc gcgcctctag aatgcatcga t 7001

<210> 62
<211> 18736
<212> DNA
<213> Artificial Sequence

<220>
<223> Targeting vector

<400> 62
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgcggc cgcgggtatc acgtggggca 420

aatcgagagc taccattggc tatggggagg agaaaatgag gcgtgctact tcctcgttcc 480

tgcagtgcgt gtcgaggtgc cgtgttggtc ggatctcagt ggcagagcag gatggtgagc 540

ggtggccttg ggccgctccc ctcactctgc tgggctggtg cggtggctcc gggaccggac 600

tttcagtggt gtttggtgtg gtgctcactg tcctttggtg tcactgtgtg gtgaattccc 660

tcagctgggc ggtgagagcc catgctgtgt cctggtagga gtagtctggg ctgcagtgcg 720

ggctctttgt gcgtgttggg ggcggttccc tggaagctaa ttctggaaga aatcccggct 780

gcctctgtaa cagttgttgg cgatgtcaaa caccctggag ctgctgactg gctctgtacc 840

attgagtgat gcagtttaaa cctctctctt tcctcctgct gtgtgtgaag ctggtgttag 900

tgctaggaga cctgcacatc cctcaccgat gcagtggttt accagcaaag ttcaagagcc 960

tcctggttcc agggaagatt cagcatattt tgtgtacagg aaacctctgc accaaggaga 1020

gctatgacta cctcaggact ttggctggag atgttcacgt tgttaagggg gactctgagg 1080

taatgttctt gctgctgtga attcctcagt cctccatctt acaagccgta gttgtcccac 1140

tgaaagctgg gaaacccttc ggtgtttgca attcctgagc gctaatgtgt gggggaagga 1200

tgtaggaaca aataaggctc aagagacaac aacttcctag gctcctctta ttgcccttgt 1260

ggtaagggag gacttggtgt gtagtggtaa tacatgcact caacttatta agttctcata 1320

gctgctgtga agctgctgca gtccctcaga tccctgcagg aagaggggac tgactgactg 1380

ttgaatagcc ttgaagctcc tgctctgcat gtggttccgg tcaggctcaa gatcccagtt 1440

cttagctctt cagttgctct tctgggggta tagtactagc atctgggtca ctgcagatgg 1500

acagagactg tgcttgggta gcaaaaggca gacttacgtt gtgaaacctt cattgtgttg 1560

gtgaggttct ttgatttcta ccttgtgtat ctctcttcct cagagtctga attatcctga 1620

acagaaggtt gtaactgttg ggcagttcag aattgggttg attcatggcc atcaagtcat 1680

tccctggggt gatgtggcaa gcttggcatt gctgcagagg cagctggatg tagacattct 1740

tatctcagga catacacaca gatttgaagc atttgagcat gaaaataagt tctacatcaa 1800

cccaggatca gctacaggag cctacactgg tttacaaacg tgagtacaat gagccttgtg 1860

tttgtcttta agacagatgt ctgtccttaa gagtgcagcc catgtgttga agagcactgg 1920

tgagatgcag tgccatgcag gccatctctc cctttcccag agaacacggc ctacctctct 1980

ctgttcataa ggtcttggtt aacttatgag ttggccagca cagcctggca ctgatgtgtc 2040

atgatcataa ccctgagccc acgctctgct atgctgttgc ttcttagagt tgcattgcct 2100

tgaggtgtat tccagaagct gtgtgctgag ctcttgttgg gaaagaggcc ctcagggcac 2160

tgccaacatc tgaggcttgc aggagctgga acattcagcc ctgagggctg ctctggccaa 2220

ctgcagctct gagtctgcag tcagtgcctc tgagctgtga tgcttctcag ataactgcag 2280

ccctttgcaa tgtaatgttt tataatatat ttccatagga catcttggta agaggtgtaa 2340

gctaatcagg gtgattgaca aagtatgttt tctttccagg aacgtcattc cttcatttgt 2400

actgatggat atccaggctt ccacagttgt gacttatgta taccagctaa ttgaggacga 2460

tgtgaaagta gaaagaattg agttcaagaa atactgacac gtgtttgaat ctgcctgctg 2520

acttctcact gcctctcatt ctttctctcc tagttaaagc tgaatccggc cacgtggggg 2580

tgctgctgca gcggcgtgta actgtgagct tgggaagttc tcaactaggg cttagagcag 2640

gagaaactga ttaacagaaa ttgccttatt aaatgtatat atttcctgtg tctaagaacg 2700

aggtgctgtt gttcctgact tgcggagctg cagctagtgg tagcttggaa tttaacctct 2760

cctgtaaaat gagcatagaa taaaacagta ttttcacatt tctggatcct gttaattgtt 2820

tgttaattaa gaggacagga attcagtgtc agggggctgc tctttgcttc ccaatgtgta 2880

ccactgaagc ctctctggat ttatcagaga tactcaatgg actcttaaca gcactgtggc 2940

acctgacctc ctgctgcctt cagcctccct tcctggctgg tgtgcttatg ttgaggagct 3000

gtgggaggct ggtgcagttc tgtgggatga ggtaaggcca gctgaatagc ctgggggctg 3060

gtcagggggc ttagtgagag gagcagggct ggggaatgtt gtgaacagca ggtggtgcca 3120

ctgctattct gaggaaggag gcagcttctg caaatgcttt gcaatgcagg atgacaaagt 3180

gcttctgcta gccctaggct accgttcgta taatgtatac tatacgaagt tattttattt 3240

gtgaaatttg tgatgctatt gctttatttg taaccattat aagctgcaat aaacaagttg 3300

gggtgggcga agaactccag catgagatcc ccgcgctgga ggatcatcca gccggcgtcc 3360

cggaaaacga ttccgaagcc caacctttca tagaaggcgg cggtggaatc gaaatctcgt 3420

gatggcaggt tgggcgtcgc ttggtcggtc atttcgaacc ccagagtccc gctcagaaga 3480

actcgtcaag aaggcgatag aaggcgatgc gctgcgaatc gggagcggcg ataccgtaaa 3540

gcacgaggaa gcggtcagcc cattcgccgc caagctcttc agcaatatca cgggtagcca 3600

acgctatgtc ctgatagcgg tccgccacac ccagccggcc acagtcgatg aatccagaaa 3660

agcggccatt ttccaccatg atattcggca agcaggcatc gccatgggtc acgacgagat 3720

cctcgccgtc gggcatgcgc gccttgagcc tggcgaacag ttcggctggc gcgagcccct 3780

gatgctcttc gtccagatca tcctgatcga caagaccggc ttccatccga gtacgtgctc 3840

gctcgatgcg atgtttcgct tggtggtcga atgggcaggt agccggatca agcgtatgca 3900

gccgccgcat tgcatcagcc atgatggata ctttctcggc aggagcaagg tgagatgaca 3960

ggagatcctg ccccggcact tcgcccaata gcagccagtc ccttcccgct tcagtgacaa 4020

cgtcgagcac agctgcgcaa ggaacgcccg tcgtggccag ccacgatagc cgcgctgcct 4080

cgtcctgcag ttcattcagg gcaccggaca ggtcggtctt gacaaaaaga accgggcgcc 4140

cctgcgctga cagccggaac acggcggcat cagagcagcc gattgtctgt tgtgcccagt 4200

catagccgaa tagcctctcc acccaagcgg ccggagaacc tgcgtgcaat ccatcttgtt 4260

caatcatgcg aaacgatcct catcctgtct cttgatcaga tccgaaaatg gatatacaag 4320

ctcccgggag ctttttgcaa aagcctaggc ctccaaaaaa gcctcctcac tacttctgga 4380

atagctcaga ggcagaggcg gcctcggcct ctgcataaat aaaaaaaatt agtcagccat 4440

ggggcggaga atgggcggaa ctgggcggag ttaggggcgg gatgggcgga gttaggggcg 4500

ggactatggt tgctgactaa ttgagatgca tgctttgcat acttctgcct gctggggagc 4560

ctggggactt tccacacctg gttgctgact aattgagatg catgctttgc atacttctgc 4620

ctgctgggga gcctggggac tttccacacc ctaaataact tcgtataatg tatactatac 4680

gaacggtagc gttaaccggt cgacatgcaa gcttgctagc gtttaaacac gagtgcgtac 4740

gctcgacaag acgcactcca gtctgccctg actcagcctg cctccgtgtc tgggtctcct 4800

ggacagtcga tcaccatctc ctgctctgga agcagctcca acatcggaag taattatgta 4860

tactggtacc aacagcaccc aggcaaagcc cccaaactca tgatttatag gaataatcag 4920

cggccctcag gggtttctaa tcgcttctct ggctccaagt ctggcaacac ggcctccctg 4980

accatctctg ggctccaggc tgaggacgag gctgattatt actgcgcagc atgggatgac 5040

agcctgagtg gtcattgggt gttcggcgga gggaccaagc tgaccgtcct agcctgacac 5100

acacagccct ccccagactg tgggatgagg ccctgtgccc gcagtcacat gtggaatatc 5160

aagacacaca catctatgac aatcaccgtg tctctactcg cgtgtctctc tatgcgcagt 5220

ctgccctgac tcagcctgcc tccgtgtctg ggtctcctgg acagtcgatc accatctcct 5280

gcactgggag cagctccaac atcggggcag gttatgatgt acactggtac caacagcacc 5340

caggcaaagc ccccaaactc atgatttatg gtaacagcaa tcggccctca ggggtttcta 5400

atcgcttctc tggctccaag tctggcaaca cggcctccct gaccatctct gggctccagg 5460

ctgaggacga ggctgattat tactgccagt cctatgacag cagcctgagt ggttctgtgg 5520

tattcggcgg agggaccaag ctgaccgtcc tagcctgaca cacacagccc tccccagact 5580

gtgggatgag gccctgtgcc cgcagtcaca tgtggaatat caagacacac acatctatga 5640

caatcacata cgacgtatca cgtactacgt ctagtaccag tctgccctga ctcagcctgc 5700

ctccgtgtct gggtctcctg gacagtcgat caccatctcc tgctctggaa gcagctccaa 5760

cattgggaat aattatgtat cctggtacca acagcaccca ggcaaagccc ccaaactcat 5820

gatttatgac aataataagc gaccctcagg ggtttctaat cgcttctctg gctccaagtc 5880

tggcaacacg gcctccctga ccatctctgg gctccaggct gaggacgagg ctgattatta 5940

ctgcggaaca tgggatagca gcctgagtgc tggtgctgtg ttcggcggag ggaccaagct 6000

gaccgtccta gcctgacaca cacagccctc cccagactgt gggatgaggc cctgtgcccg 6060

cagtcacatg tggaatatca agacacacac atctatgaca atcaccgtag actagtacga 6120

gtatgtactc tcgtgcagtc tgccctgact cagcctgcct ccgtgtctgg gtctcctgga 6180

cagtcgatca ccatctcctg cactgggagc agctccaaca ttgggcgggt ttatgttgta 6240

cattggtacc aacagcaccc aggcaaagcc cccaaactca tgatttatga aaataataag 6300

cgaccctcag gggtttctaa tcgcttctct ggctccaagt ctggcaacac ggcctccctg 6360

accatctctg ggctccaggc tgaggacgag gctgattatt actgcagctc atatacaagc 6420

agcggcactc ctgtggtatt cggcggaggg accaagctga ccgtcctagc ctgacacaca 6480

cagccctccc cagactgtgg gatgaggccc tgtgcccgca gtcacatgtg gaatatcaag 6540

acacacacat ctatgacaat cacacgcgag tatactacta tgtactgtac agtcagtctg 6600

ccctgactca gcctgcctcc gtgtctgggt ctcctggaca gtcgatcacc atctcctgca 6660

ctggaaccag cagtgatgtt gggagttata accttgtctc ctggtaccaa cagcacccag 6720

gcaaagcccc caaactcatg atttatgagg gcagtaagcg gccctcaggg gtttctaatc 6780

gcttctctgg ctccaagtct ggcaacacgg cctccctgac catctctggg ctccaggctg 6840

aggacgaggc tgattattac tgccagtcct atgacagcag cctgagtggt tgccagtcct 6900

atgacagcag cctgagtggt ttggtgttcg gcggagggac caagctgacc gtcctagcct 6960

gacacacaca gccctcccca gactgtggga tgaggccctg tgcccgcagt cacatgtgga 7020

atatcaagac acacacatct atgacaatca catagagtac tcacgctacg tcagtagacg 7080

tcagtctgcc ctgactcagc ctgcctccgt gtctgggtct cctggacagt cgatcaccat 7140

ctcctgcggg ggaaacaaca ttggaagtaa aaatgtgcac tggtaccaac agcacccagg 7200

caaagccccc aaactcatga tttataggga tagcaaccgg ccctctgggg tttctaatcg 7260

cttctctggc tccaagtctg gcaacacggc ctccctgacc atctctgggc tccaggctga 7320

ggacgaggct gattattact gccagtccta tgacaggagt ggttgggtgt tcggcggagg 7380

gaccaagctg accgtcctag cctgacacac acagccctcc ccagactgtg ggatgaggcc 7440

ctgtgcccgc agtcacatgt ggaatatcaa gacacacaca tctatgacaa tcactacgct 7500

gtcttagtgt agattcgatc acgtcagtct gccctgactc agcctgcctc cgtgtctggg 7560

tctcctggac agtcgatcac catctcctgc tctggagaag cattgccaaa aaaatatgct 7620

tattggtacc aacagcaccc aggcaaagcc cccaaactca tgatttataa agacagcgag 7680

aggccctcag gggtttctaa tcgcttctct ggctccaagt ctggcaacac ggcctccctg 7740

accatctctg ggctccaggc tgaggacgag gctgattatt actgcaactc ttttacaagc 7800

accaccaatt atgtcttatt cggcggaggg accaagctga ccgtcctagc ctgacacaca 7860

cagccctccc cagactgtgg gatgaggccc tgtgcccgca gtcacatgtg gaatatcaag 7920

acacacacat ctatgacaat cactgacgta tgttgtgagt agtacagtat atacagtctg 7980

ccctgactca gcctgcctcc gtgtctgggt ctcctggaca gtcgatcacc atctcctgcg 8040

ggggaaacaa cattggaagt aaaagtgtgc actggtacca acagcaccca ggcaaagccc 8100

ccaaactcat gatttattat gatagcgacc ggccctcagg ggtttctaat cgcttctctg 8160

gctccaagtc tggcaacacg gcctccctga ccatctctgg gctccaggct gaggacgagg 8220

ctgattatta ctgccaggtg tgggatgata atagtgatca tcttatattc ggcggaggga 8280

ccaagctgac cgtcctagcc tgacacacac agccctcccc agactgtggg atgaggccct 8340

gtgcccgcag tcacatgtgg aatatcaaga cacacacatc tatgacaatc acagtataca 8400

taagtcgaga gaagtgctac gacagtctgc cctgactcag cctgcctccg tgtctgggtc 8460

tcctggacag tcgatcacca tctcctgctc tggagatgca ttgccaaagc aatatgctta 8520

ttggtaccaa cagcacccag gcaaagcccc caaactcatg atttatgatg atagcgaccg 8580

gccctcaggg gtttctaatc gcttctctgg ctccaagtct ggcaacacgg cctccctgac 8640

catctctggg ctccaggctg aggacgaggc tgattattac tgcagctcat atgcaggcag 8700

caacaattgg gtgttcggcg gagggaccaa gctgaccgtc ctagcctgac acacacagcc 8760

ctccccagac tgtgggatga ggccctgtgc ccgcagtcac atgtggaata tcaagacaca 8820

cacatctatg acaatcaccg tacagtcacg tactcagact acgctctaca gtctgccctg 8880

actcagcctg cctccgtgtc tgggtctcct ggacagtcga tcaccatctc ctgcacccta 8940

agcagtgagc acagcaccta caccatcgaa tggtaccaac agcacccagg caaagccccc 9000

aaactcatga tttatgttaa gagtgatggc agccacagca agggggacgg ggtttctaat 9060

cgcttctctg gctccaagtc tggcaacacg gcctccctga ccatctctgg gctccaggct 9120

gaggacgagg ctgattatta ctgcggagag agccacacga ttgatggcca agtcggtgtg 9180

gtattcggcg gagggaccaa gctgaccgtc ctagcctgac acacacagcc ctccccagac 9240

tgtgggatga ggccctgtgc ccgcagtcac atgtggaata tcaagacaca cacatctatg 9300

acaatcacta tatatacata tgctagtatc acgcgagaca gtctgccctg actcagcctg 9360

cctccgtgtc tgggtctcct ggacagtcga tcaccatctc ctgcactctg agcagtgggc 9420

acagcagcta cgccatcgca tggtaccaac agcacccagg caaagccccc aaactcatga 9480

tttatcttaa cagtgatggc agccacagca agggggacgg ggtttctaat cgcttctctg 9540

gctccaagtc tggcaacacg gcctccctga ccatctctgg gctccaggct gaggacgagg 9600

ctgattatta ctgcagctca tatgcaggca acaacaattt gggggatgtg cttttcggcg 9660

gagggaccaa gctgaccgtc ctagcctgac acacacagcc ctccccagac tgtgggatga 9720
ggccctgtgc ccgcagtcac atgtggaata tcaagacaca cacatctatg acaatcactg 9780
agtcagtatg tagtgtgatg tcacacgaca gtctgccctg actcagcctg cctccgtgtc 9840
tgggtctcct ggacagtcga tcaccatctc ctgcaccttg cgcagtggca tcaatgttgg 9900
tacctacagg atatactggt accaacagca cccaggcaaa gcccccaaac tcatgattta 9960
ttacaaatca gactcagata agcagcaggg ctctggggtt tctaatcgct tctctggctc 10020
caagtctggc aacacggcct ccctgaccat ctctgggctc caggctgagg acgaggctga 10080
ttattactgc gcagcatggg atgacagcct gaatggtgta ttcggcggag ggaccaagct 10140
gaccgtccta gcctgacaca cacagccctc cccagactgt gggatgaggc cctgtgcccg 10200
cagtcacatg tggaatatca agacacacac atctatgaca atcacacatg acgacacgac 10260
agctcacgtc tcatccagtc tgccctgact cagcctgcct ccgtgtctgg gtctcctgga 10320
cagtcgatca ccatctcctg cacccgcagc agtggcagca ttgccagcaa ctatgtgcag 10380
tggtaccaac agcacccagg caaagccccc aaactcatga tttatgagga taaccaaaga 10440
ccctctgggg tttctaatcg cttctctggc tccaagtctg gcaacacggc ctccctgacc 10500
atctctgggc tccaggctga ggacgaggct gattattact gccagtccta tgacagcagc 10560
ctgaggagag tcttcggcgg agggaccaag ctgaccgtcc tagcctgaca cacacagccc 10620
tccccagact gtgggatgag gccctgtgcc cgcagtcaca tgtggaatat caagacacac 10680
acatctatga caatcacagc gactagcagt agtgatcagt gacacaccag tctgccctga 10740
ctcagcctgc ctccgtgtct gggtctcctg gacagtcgat caccatctcc tgcggctcca 10800
gcactggagc tgtcaccagt ggtcattatc cctactggta ccaacagcac ccaggcaaag 10860
cccccaaact catgatttat gatacaagca acaaacactc cggggtttct aatcgcttct 10920
ctggctccaa gtctggcaac acggcctccc tgaccatctc tgggctccag gctgaggacg 10980
aggctgatta ttactgcgca acatgggatg acagcctgaa tgagggagtg ttcggcggag 11040
ggaccaagct gaccgtccta gcctgacaca cacagccctc cccagactgt gggatgaggc 11100
cctgtgcccg cagtcacatg tggaatatca agacacacac atctatgaca atcacatcta 11160
ctgaccacgt agatccacgt gtcgccagtc tgccctgact cagcctgcct ccgtgtctgg 11220
gtctcctgga cagtcgatca ccatctcctg caccctgagc agcggctaca gtaattataa 11280
agtggactgg taccaacagc acccaggcaa agcccccaaa ctcatgattt atgtgggcac 11340
tggtgggatt gtgggatcca agggggatgg ggtttctaat cgcttctctg gctccaagtc 11400
tggcaacacg gcctccctga ccatctctgg gctccaggct gaggacgagg ctgattatta 11460
ctgcggggca gaccatggca gtgggagcaa cttcgtggtg gtattcggcg gagggaccaa 11520
gctgaccgtc ctagcctgac acacacagcc ctccccagac tgtgggatga ggccctgtgc 11580

ccgcagtcac atgtggaata tcaagacaca cacatctatg acaatcacgt ttaaacaagc 11640

ttcgaagggc ccatgcgaat tccgcggatg cggcgcgcct ctagaatgca tcgatttaaa 11700

tcgagtgaag gtcaccaagg atggagactt cagagctatc tgggacacgt gttttaacgt 11760

ttgatcagcc ttgcagcaaa acagtgtttt gctctattga acagtgttca acaaaaacga 11820

tcagtggtct ggaacacatc tcctacgaag aaaggttgcg agagctgggt ctggagaaga 11880

ggaggctctg gggcaacctc cctgtggcct gtcagtgctt aaaggaggct caagagaaag 11940

atggagagag actttataca cggagctgta gcaatatgac aaggagaaat ggtttaaaat 12000

ggaaagaggg tagatttagg ttagctatca agaagaaatt cttcactcag aggtgatgaa 12060

gcactgtagc aggttgccca gagaagtatt ggatgcttcc ttcctggcag ggcccaaggc 12120

cagactggat gggttctggg cagcctgttc tggtgggaga tgtccctgcc cgtggcacgg 12180

cttggaaatg ggtagccttt gaggtccctc tcagcccaaa tcattctatg ttcttgtggt 12240

tccctgcatt tcagcctttg cctgttgact ctggtgccac tgttggacaa ttctactcag 12300

ctgtgtttca tccctgcccc aaagcagtaa tcccatggat aagcaatgct ctgaggcctt 12360

caccaccatc agggctcctg ctgaccctag cggcctccct tgcagcatga gccctttgtt 12420

gtccgcactg ctgctgctga gggtcctgcc agccccccag ggccttttct gcccaggcac 12480

tgccacctgc taaaacccag cctgcccatg gcagggcatc gcatgtctga gggatggaac 12540

ggggcaaggg cagtgctgca ggcagtggtt caagaaacgt ttagatgttg tactgaggga 12600

tgtggtttag tgggaaatac tggtgatagg tggatggttg gactggagga tcttagaggt 12660

cttttccaac cttggtgatt atgtgattct atgaagggag ccatagcctg ctcccatcat 12720

gccccgccag tccggctctc ccgagcactt tggttccgca gcatccaggc ccgctctggg 12780

gccggtgtgg gcctgggtgc tgccacgtgt ccccgctgca aggcctggcc aaccccgctg 12840

tgccacaatg caggggctgg gctaggagtg ggcagggagg tgtggggaga ggtgtaggac 12900

gtggctggga gcgcagggag ttatttgcat aggggggcgt gctgcggaag gacgcgggta 12960

taaaagggca tcgaggtccc cggcacagcc ccatcggcgt ggggacacac agctgctggg 13020

attccgccat ggcctgggct cctctcctcc tggcggtgct cgcccacacc tcaggtactc 13080

gttgcgcccg gtcggggact gtgggcacgg ggctctgtcc cactgctgcg cgggcagggc 13140

tgtgcgtgcg gggccgtcac tgattgccgt tttctcccct ctctcctctc cggcgcgcct 13200

ctccaggttc cctggtgcag gcacagtctg ccctgactca gcctgcctcc gtgtctgggt 13260

ctcctggaca gtcgatcacc atctcctgca ctggaaccag cagtgacgtt ggtggttata 13320

actatgtctc ctggtaccaa caaaacccag gcaaagcccc caaactcatg atttatgatg 13380

tcagtaatcg gccctcaggg atttctaatc gcttctctgg ctccaagtct ggcaacacgg 13440

cctccctgac catctctggg ctccaggctg acgacgaggc tgattattac tgcacctcat 13500

atacaaacga cagcaattct caggtattcg gcggagggac caagctgacc gtcctaggtg 13560

agtcgctcat atgctaccgt tcgtatagca tacattatac gaagttatca gacatgataa 13620

gatacattga tgagtttgga caaaccacaa ctagaatgca gtgaaaaaaa tgctttattt 13680

gtgaaatttg tgatgctatt gctttatttg taaccattat aagctgcaat aaacaagttg 13740

gggtgggcga agaactccag catgagatcc ccgcgctgga ggatcatcca gccggcgtcc 13800

cggaaaacga ttccgaagcc caacctttca tagaaggcgg cggtggaatc gaaatctcgt 13860

agcacgtgtc agtcctgctc ctcggccacg aagtgcttag ccctcccaca cataaccaga 13920

gggcagcaat tcacgaatcc caactgccgt cggctgtcca tcactgtcct tcactatggc 13980

tttgatccca ggatgcagat cgagaagcac ctgtcggcac cgtccgcagg ggctcaagat 14040

gcccctgttc tcatttccga tcgcgacgat acaagtcagg ttgccagctg ccgcagcagc 14100

agcagtgccc agcaccacga gttctgcaca aggtccccca gtaaaatgat atacattgac 14160

accagtgaag atgcggccgt cgctagagag agctgcgctg gcgacgctgt agtcttcaga 14220

gatggggatg ctgttgattg tagccgttgc tctttcaatg agggtggatt cttcttgaga 14280

caaaggcttg gccatggttt agttcctcac cttgtcgtat tatactatgc cgatatacta 14340

tgccgatgat taattgtcaa cacgtgctga tcagatccga aaatggatat acaagctccc 14400

gggagctttt tgcaaaagcc taggcctcca aaaaagcctc ctcactactt ctggaatagc 14460

tcagaggcag aggcggcctc ggcctctgca taaataaaaa aaattagtca gccatggggc 14520

ggagaatggg cggaactggg cggagttagg ggcgggatgg gcggagttag gggcgggact 14580

atggttgctg actaattgag atgcatgctt tgcatacttc tgcctgctgg ggagcctggg 14640

gactttccac acctggttgc tgactaattg agatgcatgc tttgcatact tctgcctgct 14700

ggggagcctg gggactttcc acaccctaac tgacacacat tccacagata acttcgtata 14760

gcatacatta tacgaacggt agctctagag acctcgtctc ggtctttctt cccccatcgt 14820

gaaattgtga cattttgtcg atttttggtg atttgggggt ttttcttgga cttggcggca 14880

ggctggggtc tgccaccggc gcagggccgg gcactcagcg cgacagcctg ggctgagtct 14940

tgtccccacc gagccggagg gctccggtgt gcgccatgga ggacttaggg ttattttgtc 15000

aatggaaagt tcttaaaatt tgaccagaaa atgtgcccga ggtctgtctc tgccacacga 15060

tttcagaaat tgtgtctagg tcgatgagaa gacagttttt gtctttgtca ggaaattagt 15120

tgtgagttgt tagtccttcc ctcttagtcc taaggactaa gacctttgtc cccggtctgg 15180

tctctcactg gggactcttg gctccagtgc catggggagc ccaagtgtca ctgacacggt 15240

gtccttgggg gtgaaattca gtttttcagc tgtccaggta agtgctgtca aaaaatggag 15300

gttattctgc tgaaaaagct gagaggaata ttttgtcatt tttttcggaa atatatatat 15360

atatatatat ataaatatat aaattattta tatattttat atatatatat atataaaata 15420

tatgtatttc tctctttctt tttatatata tatataaata tatatatatt tctccctctt 15480

tctttatata tatttatagg gagagaatct atatttttgg ccaatttggc caatttctct 15540

ctctctctct ttatatatat atatatatgt atatgtaatt tatatatata tatataaatt 15600

tatatttatt tatttattta tttatataga ttatttttat atacatatat atatataaat 15660

ttggccaatt tagaagaaat tggccaaaaa aacccaatct gaaccaaatt acttagggca 15720

taagtccaaa aaaaaagtgc agttcaagaa ttcctcgctg gaaagttttt aatgggggca 15780

gttcttcccc attttctggt gtagatatga gtgaagatat agatatgtat gtagatatag 15840

atttggctat agtttgtttg atgaacagtc tgaggaagag taaatcttgc cttcctcatg 15900

gcaattttta attctacttc ttctgagaca agtctgaaca tgccttgagg tagaaagagc 15960

ccgaaaattg agtgcttttt ctttgaaatt ttaattaaaa ttaaaaaata gccttacagc 16020

aggaaagaaa gggggtgatg gcagctaatc ggagttccaa agctgaacca cgttcaccca 16080

aacacgtggt tgaaattttg tgtgtgtata tatataaata tatatatgta tacaaaagaa 16140

aatgcctata tataaaaaaa ttgttatgta tattatatag atttattata gatatgtaca 16200

taagatataa cacgtataca tatatatgta gtctgctgta tgctttgtat aaatttttat 16260

agatatgatg tatattatat gcatgggtta tatatctata atatatttta tctgtagaag 16320

gaactacagt ataaaatgca tatatgcgta tatatacaca tatttatata tatgtgtata 16380

tatatgtata cgcacacgta taacatccat gaaaatagat atggatgggt ggatgtgttt 16440

gttttacaga ggtgcatgtg tgtctgtaca tacacagcca tacatacgcg tgtgcggccg 16500

caggcgtaat catggtcata gctgtttcct gtgtgaaatt gttatccgct cacaattcca 16560

cacaacatac gagccggaag cataaagtgt aaagcctggg gtgcctaatg agtgagctaa 16620

ctcacattaa ttgcgttgcg ctcactgccc gctttccagt cgggaaacct gtcgtgccag 16680

ctgcattaat gaatcggcca acgcgcgggg agaggcggtt tgcgtattgg gcgctcttcc 16740

gcttcctcgc tcactgactc gctgcgctcg gtcgttcggc tgcggcgagc ggtatcagct 16800

cactcaaagg cggtaatacg gttatccaca gaatcagggg ataacgcagg aaagaacatg 16860

tgagcaaaag gccagcaaaa ggccaggaac cgtaaaaagg ccgcgttgct ggcgtttttc 16920

cataggctcc gcccccctga cgagcatcac aaaaatcgac gctcaagtca gaggtggcga 16980

aacccgacag gactataaag ataccaggcg tttccccctg gaagctccct cgtgcgctct 17040

cctgttccga ccctgccgct taccggatac ctgtccgcct ttctcccttc gggaagcgtg 17100

gcgctttctc atagctcacg ctgtaggtat ctcagttcgg tgtaggtcgt tcgctccaag 17160

ctgggctgtg tgcacgaacc ccccgttcag cccgaccgct gcgccttatc cggtaactat 17220

cgtcttgagt ccaacccggt aagacacgac ttatcgccac tggcagcagc cactggtaac 17280

aggattagca gagcgaggta tgtaggcggt gctacagagt tcttgaagtg gtggcctaac 17340

tacggctaca ctagaagaac agtatttggt atctgcgctc tgctgaagcc agttaccttc 17400

ggaaaaagag ttggtagctc ttgatccggc aaacaaacca ccgctggtag cggtggtttt 17460

tttgtttgca agcagcagat tacgcgcaga aaaaaaggat ctcaagaaga tcctttgatc 17520

ttttctacgg ggtctgacgc tcagtggaac gaaaactcac gttaagggat tttggtcatg 17580

agattatcaa aaaggatctt cacctagatc cttttaaatt aaaaatgaag ttttaaatca 17640

atctaaagta tatatgagta aacttggtct gacagttacc aatgcttaat cagtgaggca 17700

cctatctcag cgatctgtct atttcgttca tccatagttg cctgactccc cgtcgtgtag 17760

ataactacga tacgggaggg cttaccatct ggccccagtg ctgcaatgat accgcgagac 17820

ccacgctcac cggctccaga tttatcagca ataaaccagc cagccggaag ggccgagcgc 17880

agaagtggtc ctgcaacttt atccgcctcc atccagtcta ttaattgttg ccgggaagct 17940

agagtaagta gttcgccagt taatagtttg cgcaacgttg ttgccattgc tacaggcatc 18000

gtggtgtcac gctcgtcgtt tggtatggct tcattcagct ccggttccca acgatcaagg 18060

cgagttacat gatcccccat gttgtgcaaa aaagcggtta gctccttcgg tcctccgatc 18120

gttgtcagaa gtaagttggc cgcagtgtta tcactcatgg ttatggcagc actgcataat 18180

tctcttactg tcatgccatc cgtaagatgc ttttctgtga ctggtgagta ctcaaccaag 18240

tcattctgag aatagtgtat gcggcgaccg agttgctctt gcccggcgtc aatacgggat 18300

aataccgcgc cacatagcag aactttaaaa gtgctcatca ttggaaaacg ttcttcgggg 18360

cgaaaactct caaggatctt accgctgttg agatccagtt cgatgtaacc cactcgtgca 18420

cccaactgat cttcagcatc ttttactttc accagcgttt ctgggtgagc aaaaacagga 18480

aggcaaaatg ccgcaaaaaa gggaataagg gcgacacgga aatgttgaat actcatactc 18540

ttcctttttc aatattattg aagcatttat cagggttatt gtctcatgag cggatacata 18600

tttgaatgta tttagaaaaa taaacaaata ggggttccgc gcacatttcc ccgaaaagtg 18660

ccacctgacg tctaagaaac cattattatc atgacattaa cctataaaaa taggcgtatc 18720

acgaggccct ttcgtc 18736

<210> 63
<211> 10494
<212> DNA
<213> Artificial Sequence

<220>
<223> Targeting vector

<400> 63
cacctgacgc gccctgtagc ggcgcattaa gcgcggcggg tgtggtggtt acgcgcagcg 60

tgaccgctac acttgccagc gccctagcgc ccgctccttt cgctttcttc ccttcctttc 120

tcgccacgtt cgccggcttt ccccgtcaag ctctaaatcg ggggctccct ttagggttcc 180

gatttagtgc tttacggcac ctcgacccca aaaaacttga ttagggtgat ggttcacgta 240

gtgggccatc gccctgatag acggtttttc gccctttgac gttggagtcc acgttcttta 300

atagtggact cttgttccaa actggaacaa cactcaaccc tatctcggtc tattcttttg 360

atttataagg gattttgccg atttcggcct attggttaaa aaatgagctg atttaacaaa 420

aatttaacgc gaattttaac aaaatattaa cgcttacaat ttccattcgc cattcaggct 480

gcgcaactgt tgggaagggc gatcggtgcg ggcctcttcg ctattacgcc agctggcgaa 540

agggggatgt gctgcaaggc gattaagttg ggtaacgcca gggttttccc agtcacgacg 600

ttgtaaaacg acggccagtg aattgtaata cgactcacta tagggcgaat tggaatgttg 660

gcgcagaagg gattcatatc aattaattcc ctgtcctcaa ttaatgcata attgggagtg 720

cgatggaatc attcaataag taatgaggac ttggtgtcaa aatgccaatt aagtaattaa 780

tgacacccaa ttaatacaca atggggatta cagtggaata actcagtgaa gttcatatct 840

aaattgcaat tgaataatta attacagcta attaatggat gggagactgg cagtggtata 900

acttaatgaa ggaacacagt gaattcctgt ctgaattgaa attaattgat ttattagggc 960

taattaatga atggtggggt taaaaatagc attactcagc gaaggaacat gagttcatgg 1020

ctaaattgag attgaattat taattacaac taattgatta atgactgatg ggagtaagaa 1080

tggaataact cagtgaagga acacaatgga ttcatggcct aattgaaatt aaatggttaa 1140

ttagagctaa ttaatgaatg atggggggtg gcaatggaat gatacaagaa agaaacacaa 1200

tgggttcgtg tctacattga aattaaataa ttaattacag ctaattaata aacggcgagg 1260

ttgacaatgg tataactgtg aagagacaga atgagttcgt gactaaattg gtattaaatg 1320

attaattaga gctaattaac aggtggtggg gctgagaatg gcataactca atgggggatg 1380

atgagctgat ggcttaattg gaattaataa ttaatcacaa ccaattaaca ggtgctgtta 1440

atggggttac aggggcatga cttcaggaag aagcacaaag tggccattaa ataattaatt 1500

gcacccaatt aattcctgat ggggattact gcagaggaac tcagcgaagg attcaagcag 1560

aaatgacaca gttaggttgc tcaattgcgt taattaatta aaatcgctaa ttaggtgagc 1620

ttcattaagg cctgatgggg ttcatcccaa cgctgggatc aaagaactca gcgcagagtg 1680

gataacgata attagaaggg gacaaattaa tgaggaaaca cgactttggg cctagacggc 1740

cattaaatca ttaatcaacc cgacgggcgc ccaattaatc ataaagttat taagcaatta 1800

agtcgaggct gacgagaaac cgcaacgaga gccctccacg ctcctttgag ttggcgcaaa 1860

caacaaacaa cgcgggctcg ttaagcagcg acgacgccga gcgtaacgaa gcaatccccg 1920

cgcccgacaa ttaacgcgct aacgaagccg gagccctcct tatgcaaatt agcccctcca 1980

gagggccata aaagcgccgg ctctccgacg gcgcgggagg agcaccagtc ggctccgcaa 2040

ccatgagccc actcgtctcc tccctcctgc tcctggccgc cctgccaggt gagggcgctg 2100

tggggctcta tggggctcta tggggtctca gcggggctct gcgggctcaa tgggggccaa 2160

agggggggtc tgcgggctct atgggggggt caacgggggg tctcacgggg ggccggctcc 2220

gcgaggccgt gtggcggcgg ctccgtcagc gctctctggg cgcgcctcct tccccacagg 2280

gctgatggcg gaggtgcagc tgttggagtc tgggggaggc ttggtacagc ctggggggtc 2340

cctgagactc tcctgtgcag cctctggatt cacctttagc agctatgcca tgagctgggt 2400

ccgccaggct ccagggaagg ggctggagtg ggtctcagct attagtggta gtggtggtag 2460

cacatactac gcagactccg tgaagggccg gttcaccatc tccagagaca attccaagaa 2520

cacgctgtat ctgcaaatga acagcctgag agccgaggac acggccgtat attactgtgc 2580

gaaaggatat agtggctacg attactactt tgactactgg ggccagggca ccctggtcac 2640

cgtctcctca ggtgagtctt catatgaacc ccccccaaaa ttgccgcggc gattttgggg 2700

caaaatcggg cgattttatg gatccccgct accgttcgta tagcatacat tatacgaagt 2760

tatcagacat gataagatac attgatgagt ttggacaaac cacaactaga atgcagtgaa 2820

aaaaatgctt tatttgtgaa atttgtgatg ctattgcttt atttgtaacc attataagct 2880

gcaataaaca agttggggtg ggcgaagaac tccagcatga gatccccgcg ctggaggatc 2940

atccagccgg cgtcccggaa aacgattccg aagcccaacc tttcatagaa ggcggcggtg 3000

gaatcgaaat ctcgtagcac gtgtcagtcc tgctcctcgg ccacgaagtg cttagccctc 3060

ccacacataa ccagagggca gcaattcacg aatcccaact gccgtcggct gtccatcact 3120

gtccttcact atggctttga tcccaggatg cagatcgaga agcacctgtc ggcaccgtcc 3180

gcaggggctc aagatgcccc tgttctcatt tccgatcgcg acgatacaag tcaggttgcc 3240

agctgccgca gcagcagcag tgcccagcac cacgagttct gcacaaggtc ccccagtaaa 3300

atgatataca ttgacaccag tgaagatgcg gccgtcgcta gagagagctg cgctggcgac 3360

gctgtagtct tcagagatgg ggatgctgtt gattgtagcc gttgctcttt caatgagggt 3420

ggattcttct tgagacaaag gcttggccat ggtttagttc ctcaccttgt cgtattatac 3480

tatgccgata tactatgccg atgattaatt gtcaacacgt gctgatcaga tccgaaaatg 3540

gatatacaag ctcccgggag ctttttgcaa aagcctaggc ctccaaaaaa gcctcctcac 3600

tacttctgga atagctcaga ggcagaggcg gcctcggcct ctgcataaat aaaaaaaatt 3660

agtcagccat ggggcggaga atgggcggaa ctgggcggag ttaggggcgg gatgggcgga 3720

gttaggggcg ggactatggt tgctgactaa ttgagatgca tgctttgcat acttctgcct 3780

gctggggagc ctggggactt tccacacctg gttgctgact aattgagatg catgctttgc 3840

atacttctgc ctgctgggga gcctggggac tttccacacc ctaactgaca cacattccac 3900

agataacttc gtatagcata cattatacga acggtagcct gggtcagtcg aagggggcgg 3960

tcggtccatc atttgcggcc gggtgatttt tggggccgaa aagtgggaat ttggggccca 4020

atttggggcc caatttgggg cccaatttgg gttttcgagg ggggattttt ttagggggag 4080

attttgggtc cccggaaaat ggtgattttg ggtcaaaaat ggggattttg gggtcgtttt 4140

gaggtggggt tttttggggt agaaatggcg gtctgagggg aaaatgtcgt tttggggcca 4200

ttttgggcca tttgagggga aatttgggtc aaaaaatgac gattttgggt cgttttaggg 4260

aggaaaatga atttagggcg atttgagcca aatttgggtc aaaaatggcg attttgagtc 4320

aaaaaatgat tattttgggt cattttaggg aggaaaatga atttagggag attggacggc 4380

aaatttgggt caaaaaatgg tgattttggg tcaaaaaatg attattttgg gtcattttag 4440

ggagaaaaat gaatttaggg agatttgagg gcaaatttgg gtcaaaaaat ggtgattttg 4500

ggtcaaaaaa tgattatttt gggtcatttt agggagaaaa atgaatttag ggagatttga 4560

gggcaaattt gggtcgaaaa atggcgattt tgggtgaaaa atggacaatt ttgggtcatt 4620

ttagggttaa aaatgaattt agggagattg gacggcaaat ttgggtcaaa aaatggtgat 4680

ttggggtcaa aaaataatta ttttgggtca ttttagggat aaaaaatgaa tttagggaga 4740

tttgagggca aatttgggtc gaaaaatggt gatttggggt gaaaaatgga caattttggg 4800

tcattttagg gataaaaaat gaatttaggg cgatttgagc caaatttggg tcaaaaatgg 4860

cgattttgag tcaaaaaatg attattttgg gtcattttag ggttaaaaat gaatttaggg 4920

agattggacg gcaaatttgg gtcaaaaaat ggtgattttc ggtcaaaaaa tgattatttt 4980

gggtcatttt agggagaaaa atgaatttag ggagattgga cggcaaattt gggtcgaaaa 5040

atggtgattt tgggtcaaaa atggacaatt tggggtcatt ttagggataa aaatgaattt 5100

agggagattt gagagcaaat ttgggtctaa aaatgtcaat ttggggtcaa aaatggtgat 5160

tttgggtcat tttagggata taaatgaatt ttgggagatt tgagggcaaa tttgggtcga 5220

aaaatggtga tttggggtca atttagggag gaaaatgaat ttaaggtgat ttgacggcaa 5280

atttgggttg aaaaatgaca atttggggtc attttaaaga taaaatgaat ttagggcgat 5340

ttgagggcaa atttgggtcg aaaaatggtg atttgggtca aaaaatgaca attttgggtc 5400

attttaggga tataaatgga cttagagcga tttgagggca aatttggttg aaaaaatgga 5460

caatttgggt cattttaggg atataaatga atttagggag atttgatggc aaatttgggt 5520

cgaaaaatgg tgatttgggt caaaaatggt gattttggtt gaaaaacagt catttggggt 5580

cattttaggg attaaaatga atttcgggag atttgagggc aaatttgggt caaaaaatgg 5640

tgattttggg tcaattagtg ataaaaaaat gaatttaggg tgatttgagg gcaaatttgg 5700

gtcaaaaaat gacaatttgg ggttttttta gggaggagaa tgaatttagg gagttttgag 5760

ggcaaatttg ggtcaaaaaa tagtgatttg ggtcaaaagt ggtgatttgg gttgaaaaac 5820

agtcattttg ggtcatttta gggagaaaaa tgaatttcgg gagatttgag ggcaaatttg 5880

ggtcaaaaaa tgacaatttt gggtcacttt acgaattaaa atgaatttag ggagatttga 5940

gggcaaattt gggtcaaaat atggtgattt tgggtcaatt tagggaggaa aatgaattta 6000

gggcgatttg aaggcaaatt tgggtcgaaa atggtgattt tgggtcaaaa aatgatgatt 6060

ttgggtcgtt ttagggagaa aatgaattta gggagatttg agggcaaatt tgggtcaaaa 6120

aatggtgatt ttgggtcatt gtagggataa aaatgaattt ggggcgattt gagggcaaat 6180

ttgggtggaa aatggacaat tttgggtcat tttagggaga aaaatgaatt tagggagatt 6240

tgagggcaaa tttgggtcaa aaaatggtga tttggggtca ttttcagaag aaaaaatgat 6300

tattttcccc actaaaaatg tatattttgg ggccaaatgg tgaaaaatgg tgatttttaa 6360

tcaaacgtcc ccaaaattgg ggaaatttca tcgatttgac ccaaaattga gttttttttc 6420

cctgttaaaa atgtacattt tggggtcaat cgctgaaatg ttcccacttt tcacttcttt 6480

gcccccaaat tttgctgtcc ggtgagaaat taattaatta atgatcatta attgatcgac 6540

aattaatggt tattaattaa ttaataggtc ctcttttggt gactccttcg cttttggggc 6600

caaaagtcca taaattggcc ccaaaaaatt aacgctgagt aattggattc caaagtatta 6660

atgataaaca ttaaaagtgt ttaattaata atgatattaa acataatttc gtttttatta 6720

tcgatttatc aacaacgatg aacgataata ctttacaaca atcgctaata attaattaat 6780

tatttaatta attaatttct aataattact tcgcattatc ggacacgaga tgttgtaatt 6840

attaataatt taattcctaa taattagaag attcgttgaa aatggtactt atctttacaa 6900

ataatcactt ctaataataa tgattaataa tagttaataa caacaatgac gataatgacg 6960

ataatattaa taatatgtga tatatttaat ataaaattcg tattaatata ttatatctac 7020

aaaatatgat ataaaatata atattttatt tatatataac acaatttatt attattatca 7080

ttattatcat tattaatatc atcattatta acgttatcga aatacttatt tagaaataat 7140

aaaaacggat ttaatgatgg caacaaaaat attttattaa tgttaaaaaa aaataattaa 7200

taatttccaa agattcgaat tcggggcaac gaacggcact cgataatttt taattaatta 7260

atagtttgaa ttaatcggta ctttttaatc ctccattttg cccgaaatcg ccgttttttg 7320

ccccaaattc cccaccgcgg cgttaaaaac ataaagaaat taagcatcaa aagtgccctt 7380

ttttggggtt gttttgaccc aaaaaaaaaa tggccgaatt gggggcggcc gttttacggt 7440

tgggttcatt ttgggttcaa aacagccaaa aatgggaact ttgggtttcg aaaacaacaa 7500

caacaaaaaa acgggtttat tttgggctca ttttgggtgt ttttgggtca ggaggagaaa 7560

aaataggaag tttgagagcg aaacaacggc cgcttttggg gggaaaacgg ccctttttgg 7620

tcaacggcgg gggaaaaaaa aaagcggagt ttttggggtg aaaaagagcg gttttgggta 7680

aatttgggtt ttggggtaaa agtggaggat tttgggcaat gggagttaaa aaatgggtgt 7740

ttttatgggg gttcggtgca gtttttcctg tttgatgggg ggtttattaa tccggggggg 7800

gggattaatg agaattaata atgttaatag aaatatctgg gaaattaata gcaattatta 7860

attgttaata gttattaata gttctatata tctcacatct acgatacaat ataatatcgt 7920

tacaatcata tagtcgatat attacatata attatcagta ataataataa gtaacaataa 7980

ttagcagtaa ttaataataa taattaatag tattcgttaa taagattatt gataataatt 8040

aagtagtagc gattaataga gatgggattt cgtgagaaat ggaccaaatt tgggccgttt 8100

tgacccaaat ttttggtggt tttttttccg attctttgtg aatttcgggt cggattcatc 8160

agcaattaat tacggttatt aggggctatt agaggctttt aattgggatt attagagact 8220

tttaagcgga tttggggact tttaagtgga ttttatgatt cttaagtgga ttttgggtgg 8280

attttaccgc ttttgccagc ttttgttccc tttagtgagg gttaatttcg agcttggcgt 8340

aatcatggtc atagctgttt cctgtgtgaa attgttatcc gctcacaatt ccacacaaca 8400

tacgagccgg aagcataaag tgtaaagcct ggggtgccta atgagtgagc taactcacat 8460

taattgcgtt gcgctcactg cccgctttcc agtcgggaaa cctgtcgtgc cagctgcatt 8520

aatgaatcgg ccaacgcgcg gggagaggcg gtttgcgtat tgggcgctct tccgcttcct 8580

cgctcactga ctcgctgcgc tcggtcgttc ggctgcggcg agcggtatca gctcactcaa 8640

aggcggtaat acggttatcc acagaatcag gggataacgc aggaaagaac atgtgagcaa 8700

aaggccagca aaaggccagg aaccgtaaaa aggccgcgtt gctggcgttt ttccataggc 8760

tccgcccccc tgacgagcat cacaaaaatc gacgctcaag tcagaggtgg cgaaacccga 8820

caggactata aagataccag gcgtttcccc ctggaagctc cctcgtgcgc tctcctgttc 8880

cgaccctgcc gcttaccgga tacctgtccg cctttctccc ttcgggaagc gtggcgcttt 8940

ctcatagctc acgctgtagg tatctcagtt cggtgtaggt cgttcgctcc aagctgggct 9000

gtgtgcacga accccccgtt cagcccgacc gctgcgcctt atccggtaac tatcgtcttg 9060

agtccaaccc ggtaagacac gacttatcgc cactggcagc agccactggt aacaggatta 9120

gcagagcgag gtatgtaggc ggtgctacag agttcttgaa gtggtggcct aactacggct 9180

acactagaag gacagtattt ggtatctgcg ctctgctgaa gccagttacc ttcggaaaaa 9240

gagttggtag ctcttgatcc ggcaaacaaa ccaccgctgg tagcggtggt ttttttgttt 9300

gcaagcagca gattacgcgc agaaaaaaag gatctcaaga agatcctttg atcttttcta 9360

cggggtctga cgctcagtgg aacgaaaact cacgttaagg gattttggtc atgagattat 9420

caaaaaggat cttcacctag atccttttaa attaaaaatg aagttttaaa tcaatctaaa 9480

gtatatatga gtaaacttgg tctgacagtt accaatgctt aatcagtgag gcacctatct 9540

cagcgatctg tctatttcgt tcatccatag ttgcctgact ccccgtcgtg tagataacta 9600

cgatacggga gggcttacca tctggcccca gtgctgcaat gataccgcga gacccacgct 9660

caccggctcc agatttatca gcaataaacc agccagccgg aagggccgag cgcagaagtg 9720

gtcctgcaac tttatccgcc tccatccagt ctattaattg ttgccgggaa gctagagtaa 9780

gtagttcgcc agttaatagt ttgcgcaacg ttgttgccat tgctacaggc atcgtggtgt 9840

cacgctcgtc gtttggtatg gcttcattca gctccggttc ccaacgatca aggcgagtta 9900

catgatcccc catgttgtgc aaaaaagcgg ttagctcctt cggtcctccg atcgttgtca 9960

gaagtaagtt ggccgcagtg ttatcactca tggttatggc agcactgcat aattctctta 10020

ctgtcatgcc atccgtaaga tgcttttctg tgactggtga gtactcaacc aagtcattct 10080

gagaatagtg tatgcggcga ccgagttgct cttgcccggc gtcaatacgg gataataccg 10140

cgccacatag cagaacttta aaagtgctca tcattggaaa acgttcttcg gggcgaaaac 10200

tctcaaggat cttaccgctg ttgagatcca gttcgatgta acccactcgt gcacccaact 10260

gatcttcagc atcttttact ttcaccagcg tttctgggtg agcaaaaaca ggaaggcaaa 10320

atgccgcaaa aaagggaata agggcgacac ggaaatgttg aatactcata ctcttccttt 10380

ttcaatatta ttgaagcatt tatcagggtt attgtctcat gagcggatac atatttgaat 10440

gtatttagaa aaataaacaa ataggggttc cgcgcacatt tccccgaaaa gtgc 10494

<210> 64
<211> 7433
<212> DNA
<213> Homo sapiens

<400> 64
   ctcgagagct catgcggtac cgctagcgtt taaacctagt cactcctcta cgctcctgta 60
   catacgaggt gcagctgttg gagtctgggg gaggcttggt acagcctggg gggtccctga 120
   gactctcctg tgcagcctct ggattcacct ttagcggcta ctatatgcac tgggtccgcc 180
   aggctccagg gaaggggctg gagtgggtct catggatcaa ccctaacagt ggtggcacaa 240
   actatgcaca gaagtttcag ggccggttca ccatctccag agacaattcc aagaacacgc 300
   tgtatctgca aatgaacagc ctgagagccg aggacacggc cgtatattac tgtgcgaaag 360
   gttggggcta tacggtgact accctcaact ggttcgaccc ctggggccag ggcaccctgg 420
   tcaccgtctc ctcagtgcct ggtgactctt ccgcaatcag tcctgggaca gaaacctcag 480
   ggacagcctt tatgagtttt gatcacatct aacagagagg atgggccagc gtgtctctac 540
   tcgcgtgtct ctctatgcgg aggtgcagct gttggagtct gggggaggct tggtacagcc 600
   tggggggtcc ctgagactct cctgtgcagc ctctggattc acctttagca gttatgatat 660
   caactgggtc cgccaggctc cagggaaggg gctggagtgg gtctcatgga tgaaccctaa 720
   cagtggtaac acaggctatg cacagaagtt ccagggccgg ttcaccatct ccagagacaa 780
   ttccaagaac acgctgtatc tgcaaatgaa cagcctgaga gccgaggaca cggccgtata 840
   ttactgtgcg aaatccagca gctggcacta ctactacggt atggacgtct ggggccaggg 900
   caccctggtc accgtctcct cagtgcctgg tgactcttcc gcaatcagtc ctgggacaga 960
   aacctcaggg acagccttta tgagttttga tcacatctaa cagagaggat gggccagata 1020
   cgacgtatca cgtactacgt ctagtacgag gtgcagctgt tggagtctgg gggaggcttg 1080
   gtacagcctg gggggtccct gagactctcc tgtgcagcct ctggattcac ctttagcgaa 1140
   ttatccatgc actgggtccg ccaggctcca gggaaggggc tggagtgggt ctcaggtttt 1200
   gatcctgaag atggtgaaac aatctacgca cagaagttcc agggccggtt caccatctcc 1260
   agagacaatt ccaagaacac gctgtatctg caaatgaaca gcctgagagc cgaggacacg 1320
   gccgtatatt actgtgcgaa aggaacctac tactactact acggtatgga cgtctggggc 1380
   cagggcaccc tggtcaccgt ctcctcagtg cctggtgact cttccgcaat cagtcctggg 1440
   acagaaacct cagggacagc ctttatgagt tttgatcaca tctaacagag aggatgggcc 1500
   agcgtagact agtacgagta tgtactctcg tggaggtgca gctgttggag tctgggggag 1560
   gcttggtaca gcctgggggg tccctgagac tctcctgtgc agcctctgga ttcaccttta 1620
   gcagctacta tatgcactgg gtccgccagg ctccagggaa ggggctggag tgggtctcaa 1680
   taatcaaccc tagtggtggt agcacaagct acgcacagaa gttccagggc cggttcacca 1740
   tctccagaga caattccaag aacacgctgt atctgcaaat gaacagcctg agagccgagg 1800
   acacggccgt atattactgt gcgaaaagtt tcagcagcag ctggtacaac tggttcgacc 1860
   cctggggcca gggcaccctg gtcaccgtct cctcagtgcc tggtgactct tccgcaatca 1920
   gtcctgggac agaaacctca gggacagcct ttatgagttt tgatcacatc taacagagag 1980
   gatgggccag acgcgagtat actactatgt actgtacagt gaggtgcagc tgttggagtc 2040
   tgggggaggc ttggtacagc ctggggggtc cctgagactc tcctgtgcag cctctggatt 2100
   cacctttagc agctatgcta tcagctgggt ccgccaggct ccagggaagg ggctggagtg 2160
   ggtctcaggg atcatcccta tctttggtac agcaaactac gcacagaagt tccagggccg 2220
   gttcaccatc tccagagaca attccaagaa cacgctgtat ctgcaaatga acagcctgag 2280
   agccgaggac acggccgtat attactgtgc gaaagggtcc gtcctcggtg atgcttttga 2340
   tatctggggc cagggcaccc tggtcaccgt ctcctcagtg cctggtgact cttccgcaat 2400
   cagtcctggg acagaaacct cagggacagc ctttatgagt tttgatcaca tctaacagag 2460
   aggatgggcc agatagagta ctcacgctac gtcagtagac gtgaggtgca gctgttggag 2520
   tctgggggag gcttggtaca gcctgggggg tccctgagac tctcctgtgc agcctctgga 2580
   ttcaccttta gcaatgctag aatgggtgtg agctgggtcc gccaggctcc agggaagggg 2640
   ctggagtggg tctcacacat tttttcgaat gacgaaaaat cctacagcac atctctgaag 2700
   agccggttca ccatctccag agacaattcc aagaacacgc tgtatctgca aatgaacagc 2760
   ctgagagccg aggacacggc cgtatattac tgtgcgaaaa tcccgtatga agcagtggct 2820
   ggctactttg actactgggg ccagggcacc ctggtcaccg tctcctcagt gcctggtgac 2880
   tcttccgcaa tcagtcctgg gacagaaacc tcaggacagc cctttatgag ttttgatcac 2940
   atctaacaga gaggatgggc cagtacgctg tcttagtgta gattcgatca cgtgaggtgc 3000
   agctgttgga gtctggggga ggcttggtac agcctggggg gtccctgaga ctctcctgtg 3060
   cagcctctgg attcaccttt agcagctatt ggatgagctg ggtccgccag gctccaggga 3120
   aggggctgga gtgggtctca aacataaagc aagatggaag tgagaaatac tatgtggact 3180
   ctgtgaaggg ccggttcacc atctccagag acaattccaa gaacacgctg tatctgcaaa 3240
   tgaacagcct gagagccgag gacacggccg tatattactg tgcgaaagtt acggtgaccg 3300
   ggacctggta cttcgatctc tggggccagg gcaccctggt caccgtctcc tcagtgcctg 3360
   gtgactcttc cgcaatcagt cctgggacag aaacctcagg gacagccttt atgagttttg 3420
   atcacatcta acagagagga tgggccagtg acgtatgttg tgagtagtac agtatataga 3480
   ggtgcagctg ttggagtctg ggggaggctt ggtacagcct ggggggtccc tgagactctc 3540
   ctgtgcagcc tctggattca cctttagcag ctacgacatg cactgggtcc gccaggctcc 3600
   agggaagggg ctggagtggg tctcagctat tggtactgct ggtgacacat actatccagg 3660
   ctccgtgaag ggccggttca ccatctccag agacaattcc aagaacacgc tgtatctgca 3720
   aatgaacagc ctgagagccg aggacacggc cgtatattac tgtgcgaaag cggatacagc 3780
   tatggtcatt tactactact actacggtat ggacgtctgg ggccagggca ccctggtcac 3840
   cgtctcctca gtgcctggtg actcttccgc aatcagtcct gggacagaaa cctcagggac 3900
   agcctttatg agttttgatc acatctaaca gagaggatgg gccagagtat acataagtcg 3960
   agagaagtgc tacgagaggt gcagctgttg gagtctgggg gaggcttggt acagcctggg 4020
   gggtccctga gactctcctg tgcagcctct ggattcacct ttagcagcta tagcatgaac 4080
   tgggtccgcc aggctccagg gaaggggctg gagtgggtct catccattag tagtagtagt 4140
   agttacatat actacgcaga ctcagtgaag ggccggttca ccatctccag agacaattcc 4200
   aagaacacgc tgtatctgca aatgaacagc ctgagagccg aggacacggc cgtatattac 4260
   tgtgcgaaag agaatagtgg ctatgattac tttgactact ggggccaggg caccctggtc 4320
   accgtctcct cagtgcctgg tgactcttcc gcaatcagtc ctgggacaga aacctcaggg 4380
   acagccttta tgagttttga tcacatctaa cagagaggat gggccagcgt acagtcacgt 4440
   actcagacta cgctctagag gtgcagctgt tggagtctgg gggaggcttg gtacagcctg 4500
   gggggtccct gagactctcc tgtgcagcct ctggattcac ctttagcgat tatgctatga 4560
   gctgggtccg ccaggctcca gggaaggggc tggagtgggt ctcattcatt agaagcaaag 4620
   cttatggtgg gacaacagaa tacaccgcgt ctgtgaaagg ccggttcacc atctccagag 4680
   acaattccaa gaacacgctg tatctgcaaa tgaacagcct gagagccgag gacacggccg 4740
   tatattactg tgcgaaagat ctaggtagtg gctttgacta ctggggccag ggcaccctgg 4800
   tcaccgtctc ctcagtgcct ggtgactctt ccgcaatcag tcctgggaca gaaacctcag 4860
   ggacagcctt tatgagtttt gatcacatct aacagagagg atgggccagt atatatacat 4920
   atgctagtat cacgcgagag aggtgcagct gttggagtct gggggaggct tggtacagcc 4980
   tggggggtcc ctgagactct cctgtgcagc ctctggattc acctttagcg accactacat 5040
   ggactgggtc cgccaggctc cagggaaggg gctggagtgg gtctcacgta ctagaaacaa 5100
   agctaacagt tacaccacag aatacgccgc gtctgtgaaa ggccggttca ccatctccag 5160
   agacaattcc aagaacacgc tgtatctgca aatgaacagc ctgagagccg aggacacggc 5220
   cgtatattac tgtgcgaaag gaggccaggg gagccgatac tactactact acggtatgga 5280
   cgtctggggc cagggcaccc tggtcaccgt ctcctcagtg cctggtgact cttccgcaat 5340
   cagtcctggg acagaaacct cagggacagc ctttatgagt tttgatcaca tctaacagag 5400
   aggatgggcc agtgagtcag tatgtagtgt gatgtcacac gagaggtgca gctgttggag 5460
   tctgggggag gcttggtaca gcctgggggg tccctgagac tctcctgtgc agcctctgga 5520
   ttcaccttta gcagcaatga gatgagctgg gtccgccagg ctccagggaa ggggctggag 5580
   tgggtctcat ccattagtgg tggtagcaca tactacgcag actccaggaa gggccggttc 5640
   accatctcca gagacaattc caagaacacg ctgtatctgc aaatgaacag cctgagagcc 5700
   gaggacacgg ccgtatatta ctgtgcgaaa gactcatatg atgcttttga tatctggggc 5760
   cagggcaccc tggtcaccgt ctcctcagtg cctggtgact cttccgcaat cagtcctggg 5820
   acagaaacct cagggacagc ctttatgagt tttgatcaca tctaacagag aggatgggcc 5880
   agacatgacg acacgacagc tcacgtctca tcgaggtgca gctgttggag tctgggggag 5940
   gcttggtaca gcctgggggg tccctgagac tctcctgtgc agcctctgga ttcaccttta 6000
   gcagtggtgg ttactcctgg agctgggtcc gccaggctcc agggaagggg ctggagtggg 6060
   tctcatacat ctatcatagt gggagcacct actacaaccc gtccctcaag agtcggttca 6120
   ccatctccag agacaattcc aagaacacgc tgtatctgca aatgaacagc ctgagagccg 6180
   aggacacggc cgtatattac tgtgcgaaag atgcgagtaa ccatttcttt gactactggg 6240
   gccagggcac cctggtcacc gtctcctcag tgcctggtga ctcttccgca atcagtcctg 6300
   ggacagaaac ctcagggaca gcctttatga gttttgatca catctaacag agaggatggg 6360
   ccagagcgac tagcagtagt gatcagtgac acacgaggtg cagctgttgg agtctggggg 6420
   aggcttggta cagcctgggg ggtccctgag actctcctgt gcagcctctg gattcacctt 6480
   tagcagtagt agttactact ggggctgggt ccgccaggct ccagggaagg ggctggagtg 6540
   ggtctcaagt atctattata gtgggagcac ctactacaac ccgtccctca agagtcggtt 6600
   caccatctcc agagacaatt ccaagaacac gctgtatctg caaatgaaca gcctgagagc 6660
   cgaggacacg gccgtatatt actgtgcgaa aaaggactgg aacgacaact ggttcgaccc 6720
   ctggggccag ggcaccctgg tcaccgtctc ctcagtgcct ggtgactctt ccgcaatcag 6780
   tcctgggaca gaaacctcag ggacagcctt tatgagtttt gatcacatct aacagagagg 6840
   atgggccaga tctactgacc acgtagatcc acgtgtcgcg aggtgcagct gttggagtct 6900
   gggggaggct tggtacagcc tggggggtcc ctgagactct cctgtgcagc ctctggattc 6960
   acctttagca gcaacagtgc tgcttggaac tgggtccgcc aggctccagg gaaggggctg 7020
   gagtgggtct caaggacata ctacaggtcc aagtggtata atgattatgc agtatctgtg 7080
   aaaagtcggt tcaccatctc cagagacaat tccaagaaca cgctgtatct gcaaatgaac 7140
   agcctgagag ccgaggacac ggccgtatat tactgtgcga aagatattgt agtagtacca 7200
   gctgctatat actggtactt cgatctctgg ggccagggca ccctggtcac cgtctcctca 7260
   gtgcctggtg actcttccgc aatcagtcct gggacagaaa cctcagggac agcctttatg 7320
   agttttgatc acatctaaca gagaggatgg gccaggttta aacggtacct ggccaggatc 7380
   catgcccatg gccgcggatg cggcgcgcca tcgatatgcg tcgacattta aat 7433

<210> 65
<211> 29
<212> DNA
<213> Artificial Sequence

<220>
<223> Synthetic DNA

<400> 65
gttaactcga gatgcgtcga catttaaat 29

<210> 66
<211> 16247
<212> DNA
<213> Artificial Sequence

<220>
<223> Targeting vector

<400> 66
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtttccc gaagcgaaag ccgcgtgtta 420

tttttgggtg ggaaagcccc gattcgctcc ttttttttta tttattattt ttgttgtttt 480

tcgtgtcttt ttcttctccg ttttgcagct gcggcctcca ggtgtccccg gccgtggtat 540

ttttgggtcg aaaaagccgc tttttggctc acttggaaac ctttcgggcg tctccaagcg 600

gagcgttggc gggaaatggt gttttttttg ggttgttttc ggtccctttt ttcaccattt 660

ttgtggtttt tcggcccatt ttcggcctcc cccccccccc ccccttcccc gtggggctgc 720

gctccgtgga gcggaatggg gtcgttaatt aaggttaatt ggtttaatta atgggccgtg 780

cagggagaag agggccgtgc ggtggttgtt agccctgatt aatggcttgg ttgaggggag 840

agtgtaatta agtgcttcgt tagcactaat taatggcttg attgagagga gagtgtaatt 900

aaatgcttca taatccctaa ttaatggctt ggttgagaca agagtgtaat taagtgcttc 960

attagccctg attaatggct tggttgaggg gagagtgtaa ttaaatgctt cgttagcact 1020

aattaatggc ttgattgaga ggagagtgta attaaatgct tcataatccc taattaatgg 1080

cttggttgag acaagagtgt aattaagtgc ttcattagcc ctgattaatg gcttggttga 1140

gaggagagtg taattaaatg cttcattatc cctaattagg gactgaattc caaggagagt 1200

gtaattaagt gcttcattat ccctaattaa tggcttggtt gagaggagag tgtaattaaa 1260

tgctttgtta gccctaatta atggtttgat ggagaggaga gtgtaattaa gtgcttcatt 1320

atccctaatt aatggcttgg ttgaggggag agtgtaatta aatgcttagt tagccctaat 1380

taatggcttg gttgagagga gagtgtaatt gaatgcttca ttatccctaa ttagggattg 1440

aattgagagg agagtgtaat tgaatgcttt gttagcccta attagggatt gaattgagag 1500

gagagtgtaa ttaaatgcct cattatccct aattagggat tgaattgaga ggagagttta 1560

attaaatgcc tcattatccc taattaggga ctgaattccg acgagagtgt aattaagtgc 1620

ttcatttagc cctaattaat ggcttgattg agaggagagt gtaattaaat gcttcattat 1680

ccctaattag ggattgaatt gagaggagag tgtaattaaa tgcttcatta tccctaatta 1740

gggattgaat tgagaggaga gtgtaattaa atgcttcatt atccctaatt aatggcttgg 1800

ttgagagggg agtgtaatta agtgcttcgt tatccctaat tagggattga attgtgagga 1860

gatggcaatt aatacttcat tatccctaat taatttaatt agtccctatt atccctaatt 1920

taattcatgg tttggttgat ggaagaatgt aattaaatgc tttgttatcc ctaattaatg 1980

gtttgattga gcggagagtg taattaaatg cttcattatc ccttattaat ggttgtgtgg 2040

aatgtagtgt aattagaagt ccctaattaa tggtttaatt gagagaagac tgcaattaga 2100

tgactcatag cccctaataa gtggtttaat tatgagataa tcacaattac atgtttcatt 2160

gtccctaatt aatcgtttaa ttgcgagaag atcacaatta attaaatgat ccctaattgc 2220

taccgttcgt ataatgtata ctatacgaag ttattttatt tgtgaaattt gtgatgctat 2280

tgctttattt gtaaccatta taagctgcaa taaacaagtt ggggtgggcg aagaactcca 2340

gcatgagatc cccgcgctgg aggatcatcc agccggcgtc ccggaaaacg attccgaagc 2400

ccaacctttc atagaaggcg gcggtggaat cgaaatctcg tgatggcagg ttgggcgtcg 2460

cttggtcggt catttcgaac cccagagtcc cgctcagaag aactcgtcaa gaaggcgata 2520

gaaggcgatg cgctgcgaat cgggagcggc gataccgtaa agcacgagga agcggtcagc 2580

ccattcgccg ccaagctctt cagcaatatc acgggtagcc aacgctatgt cctgatagcg 2640

gtccgccaca cccagccggc cacagtcgat gaatccagaa aagcggccat tttccaccat 2700

gatattcggc aagcaggcat cgccatgggt cacgacgaga tcctcgccgt cgggcatgcg 2760

cgccttgagc ctggcgaaca gttcggctgg cgcgagcccc tgatgctctt cgtccagatc 2820

atcctgatcg acaagaccgg cttccatccg agtacgtgct cgctcgatgc gatgtttcgc 2880

ttggtggtcg aatgggcagg tagccggatc aagcgtatgc agccgccgca ttgcatcagc 2940

catgatggat actttctcgg caggagcaag gtgagatgac aggagatcct gccccggcac 3000

ttcgcccaat agcagccagt cccttcccgc ttcagtgaca acgtcgagca cagctgcgca 3060

aggaacgccc gtcgtggcca gccacgatag ccgcgctgcc tcgtcctgca gttcattcag 3120

ggcaccggac aggtcggtct tgacaaaaag aaccgggcgc ccctgcgctg acagccggaa 3180

cacggcggca tcagagcagc cgattgtctg ttgtgcccag tcatagccga atagcctctc 3240

cacccaagcg gccggagaac ctgcgtgcaa tccatcttgt tcaatcatgc gaaacgatcc 3300

tcatcctgtc tcttgatcag atccgaaaat ggatatacaa gctcccggga gctttttgca 3360

aaagcctagg cctccaaaaa agcctcctca ctacttctgg aatagctcag aggcagaggc 3420

ggcctcggcc tctgcataaa taaaaaaaat tagtcagcca tggggcggag aatgggcgga 3480

actgggcgga gttaggggcg ggatgggcgg agttaggggc gggactatgg ttgctgacta 3540

attgagatgc atgctttgca tacttctgcc tgctggggag cctggggact ttccacacct 3600

ggttgctgac taattgagat gcatgctttg catacttctg cctgctgggg agcctgggga 3660

ctttccacac cctaaataac ttcgtataat gtatactata cgaacggtag cgttaacaag 3720

cttctgtgga atgtgtgtca gttagggtgt ggaaagtccc caggctcccc aggcaggcag 3780

aagtatgcaa agcatgcatc tcaattagtc agcaaccagg tgtggaaagt ccccaggctc 3840

cccagcaggc agaagtatgc aaagcatgca tctcaattag tcagcaacca tagtcccgcc 3900

cctaactccg cccatcccgc ccctaactcc gcccagttcc gcccattctc cgccccatgg 3960

ctgactaatt ttttttattt atgcagaggc cgaggccgcc tctgcctctg agctattcca 4020

gaagtagtga ggaggctttt ttggaggcct aggcttttgc aaaaagctat aacttcgtat 4080

atggtattat atacgaagtt atcccacccc aacttgttta ttgcagctta taatggttac 4140

aaataaagca atagcatcac aaatttcaca aataaagcat ttttttcact gcattctagt 4200

tgtggtttgt ccaaactcat caatgtatct tatcatgtct gtataccgtc gacctctagc 4260

tagagcttgg cgtaatcatg gtcatagctg tttcctgtgt gcccacccca acttgtttat 4320

tgcagcttat aatggttaca aataaagcaa tagcatcaca aatttcacaa ataaagcatt 4380

tttttcactg cattctagtt gtggtttgtc caaactcatc aatgtatctt atcatgtctg 4440

gagctcgcta gcgtttaaac ggtaccataa cttcgtatat tctatcttat acgaacggta 4500

ctcgagagct catgcggtac cgctagcgtt taaacctagt cactcctcta cgctcctgta 4560

catacgaggt gcagctgttg gagtctgggg gaggcttggt acagcctggg gggtccctga 4620

gactctcctg tgcagcctct ggattcacct ttagcggcta ctatatgcac tgggtccgcc 4680

aggctccagg gaaggggctg gagtgggtct catggatcaa ccctaacagt ggtggcacaa 4740

actatgcaca gaagtttcag ggccggttca ccatctccag agacaattcc aagaacacgc 4800

tgtatctgca aatgaacagc ctgagagccg aggacacggc cgtatattac tgtgcgaaag 4860

gttggggcta tacggtgact accctcaact ggttcgaccc ctggggccag ggcaccctgg 4920

tcaccgtctc ctcagtgcct ggtgactctt ccgcaatcag tcctgggaca gaaacctcag 4980

ggacagcctt tatgagtttt gatcacatct aacagagagg atgggccagc gtgtctctac 5040

tcgcgtgtct ctctatgcgg aggtgcagct gttggagtct gggggaggct tggtacagcc 5100

tggggggtcc ctgagactct cctgtgcagc ctctggattc acctttagca gttatgatat 5160

caactgggtc cgccaggctc cagggaaggg gctggagtgg gtctcatgga tgaaccctaa 5220

cagtggtaac acaggctatg cacagaagtt ccagggccgg ttcaccatct ccagagacaa 5280

ttccaagaac acgctgtatc tgcaaatgaa cagcctgaga gccgaggaca cggccgtata 5340

ttactgtgcg aaatccagca gctggcacta ctactacggt atggacgtct ggggccaggg 5400

caccctggtc accgtctcct cagtgcctgg tgactcttcc gcaatcagtc ctgggacaga 5460

aacctcaggg acagccttta tgagttttga tcacatctaa cagagaggat gggccagata 5520

cgacgtatca cgtactacgt ctagtacgag gtgcagctgt tggagtctgg gggaggcttg 5580

gtacagcctg gggggtccct gagactctcc tgtgcagcct ctggattcac ctttagcgaa 5640

ttatccatgc actgggtccg ccaggctcca gggaaggggc tggagtgggt ctcaggtttt 5700

gatcctgaag atggtgaaac aatctacgca cagaagttcc agggccggtt caccatctcc 5760

agagacaatt ccaagaacac gctgtatctg caaatgaaca gcctgagagc cgaggacacg 5820

gccgtatatt actgtgcgaa aggaacctac tactactact acggtatgga cgtctggggc 5880

cagggcaccc tggtcaccgt ctcctcagtg cctggtgact cttccgcaat cagtcctggg 5940

acagaaacct cagggacagc ctttatgagt tttgatcaca tctaacagag aggatgggcc 6000

agcgtagact agtacgagta tgtactctcg tggaggtgca gctgttggag tctgggggag 6060

gcttggtaca gcctgggggg tccctgagac tctcctgtgc agcctctgga ttcaccttta 6120

gcagctacta tatgcactgg gtccgccagg ctccagggaa ggggctggag tgggtctcaa 6180

taatcaaccc tagtggtggt agcacaagct acgcacagaa gttccagggc cggttcacca 6240

tctccagaga caattccaag aacacgctgt atctgcaaat gaacagcctg agagccgagg 6300

acacggccgt atattactgt gcgaaaagtt tcagcagcag ctggtacaac tggttcgacc 6360

cctggggcca gggcaccctg gtcaccgtct cctcagtgcc tggtgactct tccgcaatca 6420

gtcctgggac agaaacctca gggacagcct ttatgagttt tgatcacatc taacagagag 6480

gatgggccag acgcgagtat actactatgt actgtacagt gaggtgcagc tgttggagtc 6540

tgggggaggc ttggtacagc ctggggggtc cctgagactc tcctgtgcag cctctggatt 6600

cacctttagc agctatgcta tcagctgggt ccgccaggct ccagggaagg ggctggagtg 6660

ggtctcaggg atcatcccta tctttggtac agcaaactac gcacagaagt tccagggccg 6720

gttcaccatc tccagagaca attccaagaa cacgctgtat ctgcaaatga acagcctgag 6780

agccgaggac acggccgtat attactgtgc gaaagggtcc gtcctcggtg atgcttttga 6840

tatctggggc cagggcaccc tggtcaccgt ctcctcagtg cctggtgact cttccgcaat 6900

cagtcctggg acagaaacct cagggacagc ctttatgagt tttgatcaca tctaacagag 6960

aggatgggcc agatagagta ctcacgctac gtcagtagac gtgaggtgca gctgttggag 7020

tctgggggag gcttggtaca gcctgggggg tccctgagac tctcctgtgc agcctctgga 7080

ttcaccttta gcaatgctag aatgggtgtg agctgggtcc gccaggctcc agggaagggg 7140

ctggagtggg tctcacacat tttttcgaat gacgaaaaat cctacagcac atctctgaag 7200

agccggttca ccatctccag agacaattcc aagaacacgc tgtatctgca aatgaacagc 7260

ctgagagccg aggacacggc cgtatattac tgtgcgaaaa tcccgtatga agcagtggct 7320

ggctactttg actactgggg ccagggcacc ctggtcaccg tctcctcagt gcctggtgac 7380

tcttccgcaa tcagtcctgg gacagaaacc tcagggacag cctttatgag ttttgatcac 7440

atctaacaga gaggatgggc cagtacgctg tcttagtgta gattcgatca cgtgaggtgc 7500

agctgttgga gtctggggga ggcttggtac agcctggggg gtccctgaga ctctcctgtg 7560

cagcctctgg attcaccttt agcagctatt ggatgagctg ggtccgccag gctccaggga 7620

aggggctgga gtgggtctca aacataaagc aagatggaag tgagaaatac tatgtggact 7680

ctgtgaaggg ccggttcacc atctccagag acaattccaa gaacacgctg tatctgcaaa 7740

tgaacagcct gagagccgag gacacggccg tatattactg tgcgaaagtt acggtgaccg 7800

ggacctggta cttcgatctc tggggccagg gcaccctggt caccgtctcc tcagtgcctg 7860

gtgactcttc cgcaatcagt cctgggacag aaacctcagg gacagccttt atgagttttg 7920

atcacatcta acagagagga tgggccagtg acgtatgttg tgagtagtac agtatataga 7980

ggtgcagctg ttggagtctg ggggaggctt ggtacagcct ggggggtccc tgagactctc 8040

ctgtgcagcc tctggattca cctttagcag ctacgacatg cactgggtcc gccaggctcc 8100

agggaagggg ctggagtggg tctcagctat tggtactgct ggtgacacat actatccagg 8160

ctccgtgaag ggccggttca ccatctccag agacaattcc aagaacacgc tgtatctgca 8220

aatgaacagc ctgagagccg aggacacggc cgtatattac tgtgcgaaag cggatacagc 8280

tatggtcatt tactactact actacggtat ggacgtctgg ggccagggca ccctggtcac 8340

cgtctcctca gtgcctggtg actcttccgc aatcagtcct gggacagaaa cctcagggac 8400

agcctttatg agttttgatc acatctaaca gagaggatgg gccagagtat acataagtcg 8460

agagaagtgc tacgagaggt gcagctgttg gagtctgggg gaggcttggt acagcctggg 8520

gggtccctga gactctcctg tgcagcctct ggattcacct ttagcagcta tagcatgaac 8580

tgggtccgcc aggctccagg gaaggggctg gagtgggtct catccattag tagtagtagt 8640

agttacatat actacgcaga ctcagtgaag ggccggttca ccatctccag agacaattcc 8700

aagaacacgc tgtatctgca aatgaacagc ctgagagccg aggacacggc cgtatattac 8760

tgtgcgaaag agaatagtgg ctatgattac tttgactact ggggccaggg caccctggtc 8820

accgtctcct cagtgcctgg tgactcttcc gcaatcagtc ctgggacaga aacctcaggg 8880

acagccttta tgagttttga tcacatctaa cagagaggat gggccagcgt acagtcacgt 8940

actcagacta cgctctagag gtgcagctgt tggagtctgg gggaggcttg gtacagcctg 9000

gggggtccct gagactctcc tgtgcagcct ctggattcac ctttagcgat tatgctatga 9060

gctgggtccg ccaggctcca gggaaggggc tggagtgggt ctcattcatt agaagcaaag 9120

cttatggtgg gacaacagaa tacaccgcgt ctgtgaaagg ccggttcacc atctccagag 9180

acaattccaa gaacacgctg tatctgcaaa tgaacagcct gagagccgag gacacggccg 9240

tatattactg tgcgaaagat ctaggtagtg gctttgacta ctggggccag ggcaccctgg 9300

tcaccgtctc ctcagtgcct ggtgactctt ccgcaatcag tcctgggaca gaaacctcag 9360

ggacagcctt tatgagtttt gatcacatct aacagagagg atgggccagt atatatacat 9420

atgctagtat cacgcgagag aggtgcagct gttggagtct gggggaggct tggtacagcc 9480

tggggggtcc ctgagactct cctgtgcagc ctctggattc acctttagcg accactacat 9540

ggactgggtc cgccaggctc cagggaaggg gctggagtgg gtctcacgta ctagaaacaa 9600

agctaacagt tacaccacag aatacgccgc gtctgtgaaa ggccggttca ccatctccag 9660

agacaattcc aagaacacgc tgtatctgca aatgaacagc ctgagagccg aggacacggc 9720

cgtatattac tgtgcgaaag gaggccaggg gagccgatac tactactact acggtatgga 9780

cgtctggggc cagggcaccc tggtcaccgt ctcctcagtg cctggtgact cttccgcaat 9840

cagtcctggg acagaaacct cagggacagc ctttatgagt tttgatcaca tctaacagag 9900

aggatgggcc agtgagtcag tatgtagtgt gatgtcacac gagaggtgca gctgttggag 9960

tctgggggag gcttggtaca gcctgggggg tccctgagac tctcctgtgc agcctctgga 10020

ttcaccttta gcagcaatga gatgagctgg gtccgccagg ctccagggaa ggggctggag 10080

tgggtctcat ccattagtgg tggtagcaca tactacgcag actccaggaa gggccggttc 10140

accatctcca gagacaattc caagaacacg ctgtatctgc aaatgaacag cctgagagcc 10200

gaggacacgg ccgtatatta ctgtgcgaaa gactcatatg atgcttttga tatctggggc 10260

cagggcaccc tggtcaccgt ctcctcagtg cctggtgact cttccgcaat cagtcctggg 10320

acagaaacct cagggacagc ctttatgagt tttgatcaca tctaacagag aggatgggcc 10380

agacatgacg acacgacagc tcacgtctca tcgaggtgca gctgttggag tctgggggag 10440

gcttggtaca gcctgggggg tccctgagac tctcctgtgc agcctctgga ttcaccttta 10500

gcagtggtgg ttactcctgg agctgggtcc gccaggctcc agggaagggg ctggagtggg 10560

tctcatacat ctatcatagt gggagcacct actacaaccc gtccctcaag agtcggttca 10620

ccatctccag agacaattcc aagaacacgc tgtatctgca aatgaacagc ctgagagccg 10680

aggacacggc cgtatattac tgtgcgaaag atgcgagtaa ccatttcttt gactactggg 10740

gccagggcac cctggtcacc gtctcctcag tgcctggtga ctcttccgca atcagtcctg 10800

ggacagaaac ctcaggacaa gcctttatga gttttgatca catctaacag agaggatggg 10860

ccagagcgac tagcagtagt gatcagtgac acacgaggtg cagctgttgg agtctggggg 10920

aggcttggta cagcctgggg ggtccctgag actctcctgt gcagcctctg gattcacctt 10980

tagcagtagt agttactact ggggctgggt ccgccaggct ccagggaagg ggctggagtg 11040

ggtctcaagt atctattata gtgggagcac ctactacaac ccgtccctca agagtcggtt 11100

caccatctcc agagacaatt ccaagaacac gctgtatctg caaatgaaca gcctgagagc 11160

cgaggacacg gccgtatatt actgtgcgaa aaaggactgg aacgacaact ggttcgaccc 11220

ctggggccag ggcaccctgg tcaccgtctc ctcagtgcct ggtgactctt ccgcaatcag 11280

tcctgggaca gaaacctcag ggacagcctt tatgagtttt gatcacatct aacagagagg 11340

atgggccaga tctactgacc acgtagatcc acgtgtcgcg aggtgcagct gttggagtct 11400

gggggaggct tggtacagcc tggggggtcc ctgagactct cctgtgcagc ctctggattc 11460

acctttagca gcaacagtgc tgcttggaac tgggtccgcc aggctccagg gaaggggctg 11520

gagtgggtct caaggacata ctacaggtcc aagtggtata atgattatgc agtatctgtg 11580

aaaagtcggt tcaccatctc cagagacaat tccaagaaca cgctgtatct gcaaatgaac 11640

agcctgagag ccgaggacac ggccgtatat tactgtgcga aagatattgt agtagtacca 11700

gctgctatat actggtactt cgatctctgg ggccagggca ccctggtcac cgtctcctca 11760

gtgcctggtg actcttccgc aatcagtcct gggacagaaa cctcagggac agcctttatg 11820

agttttgatc acatctaaca gagaggatgg gccaggttta aacggtacct ggccaggatc 11880

catgcccatg gccgcggatg cggcgcgcca tcgatatgcg tcgacattta aatgttggcg 11940

cagaagggat tcatatcaat taattccctg tcctcaatta atgcataatt gggagtgcga 12000

tggaatcatt caataagtaa tgaggacttg gtgtcaaaat gccaattaag taattaatga 12060

cacccaatta atacacaatg gggattacag tggaataact cagtgaagtt catatctaaa 12120

ttgcaattga ataattaatt acagctaatt aatggatggg agactggcag tggtataact 12180

taatgaagga acacagtgaa ttcctgtctg aattgaaatt aattgattta ttagggctaa 12240

ttaatgaatg gtggggttaa aaatagcatt actcagcgaa ggaacatgag ttcatggcta 12300

aattgagatt gaattattaa ttacaactaa ttgattaatg actgatggga gtaagaatgg 12360

aataactcag tgaaggaaca caatggattc atggcctaat tgaaattaaa tggttaatta 12420

gagctaatta atgaatgatg gggggtggca atggaatgat acaagaaaga aacacaatgg 12480

gttcgtgtct acattgaaat taaataatta attacagcta attaataaac ggcgaggttg 12540

acaatggtat aactgtgaag agacagaatg agttcgtgac taaattggta ttaaatgatt 12600

aattagagct aattaacagg tggtggggct gagaatggca taactcaatg ggggatgatg 12660

agctgatggc ttaattggaa ttaataatta atcacaacca attaacaggt gctgttaatg 12720

gggttacagg ggcatgactt caggaagaag cacaaagtgg ccattaaata attaattgca 12780

cccaattaat tcctgatggg gattactgca gaggaactca gcgaaggatt caagcagaaa 12840

tgacacagtt aggttgctca attgcgttaa ttaattaaaa tcgctaatta ggtgagcttc 12900

attaaggcct gatggggttc atcccaacgc tgggatcaaa gaactcagcg cagagtggat 12960

aacgataatt agaaggggac aaattaatga ggaaacacga ctttgggcct agacggccat 13020

taaatcatta atcaacccga cgggcgccca attaatcata aagttattaa gcaattaagt 13080

cgaggctgac gagaaaccgc aacgagagcc ctccacgctc ctttgagttg gcgcaaacaa 13140

caaacaacgc gggctcgtta agcagcgacg acgccgagcg taacgaagca atccccgcgc 13200

ccgacaatta acgcgctaac gaagccggag ccctccttat gcaaattagc ccctccagag 13260

ggccataaaa gcgccggctc tccgacggcg cgggaggagc accagtcggc tccgcaacca 13320

tgagcccact cgtctcctcc ctcctgctcc tggccgccct gccaggtgag ggcgctgtgg 13380

ggctctatgg ggctctatgg ggtctcagcg gggctctgcg ggctcaatgg gggccaaagg 13440

gggggtctgc gggctctatg ggggggtcaa cggggggtct cacggggggc cggctccgcg 13500

aggccgtgtg gcggcggctc cgtcagcgct ctctgggcgc gcctccttcc ccacagggct 13560

gatggcggag gtgcagctgt tggagtctgg gggaggcttg gtacagcctg gggggtccct 13620

gagactctcc tgtgcagcct ctggattcac ctttagcagc tatgccatga gctgggtccg 13680

ccaggctcca gggaaggggc tggagtgggt ctcagctatt agtggtagtg gtggtagcac 13740

atactacgca gactccgtga agggccggtt caccatctcc agagacaatt ccaagaacac 13800

gctgtatctg caaatgaaca gcctgagagc cgaggacacg gccgtatatt actgtgcgaa 13860

aggatatagt ggctacgatt actactttga ctactggggc cagggcaccc tggtcaccgt 13920

ctcctcaggt gagtcttcat atgaaccccc cccaaaattg ccgcggcgat tttggggcaa 13980

aatcgggcga ttttatggat ccccgcggcc gcaggcgtaa tcatggtcat agctgtttcc 14040

tgtgtgaaat tgttatccgc tcacaattcc acacaacata cgagccggaa gcataaagtg 14100

taaagcctgg ggtgcctaat gagtgagcta actcacatta attgcgttgc gctcactgcc 14160

cgctttccag tcgggaaacc tgtcgtgcca gctgcattaa tgaatcggcc aacgcgcggg 14220

gagaggcggt ttgcgtattg ggcgctcttc cgcttcctcg ctcactgact cgctgcgctc 14280

ggtcgttcgg ctgcggcgag cggtatcagc tcactcaaag gcggtaatac ggttatccac 14340

agaatcaggg gataacgcag gaaagaacat gtgagcaaaa ggccagcaaa aggccaggaa 14400

ccgtaaaaag gccgcgttgc tggcgttttt ccataggctc cgcccccctg acgagcatca 14460

caaaaatcga cgctcaagtc agaggtggcg aaacccgaca ggactataaa gataccaggc 14520

gtttccccct ggaagctccc tcgtgcgctc tcctgttccg accctgccgc ttaccggata 14580

cctgtccgcc tttctccctt cgggaagcgt ggcgctttct catagctcac gctgtaggta 14640

tctcagttcg gtgtaggtcg ttcgctccaa gctgggctgt gtgcacgaac cccccgttca 14700

gcccgaccgc tgcgccttat ccggtaacta tcgtcttgag tccaacccgg taagacacga 14760

cttatcgcca ctggcagcag ccactggtaa caggattagc agagcgaggt atgtaggcgg 14820

tgctacagag ttcttgaagt ggtggcctaa ctacggctac actagaagaa cagtatttgg 14880

tatctgcgct ctgctgaagc cagttacctt cggaaaaaga gttggtagct cttgatccgg 14940

caaacaaacc accgctggta gcggtggttt ttttgtttgc aagcagcaga ttacgcgcag 15000

aaaaaaagga tctcaagaag atcctttgat cttttctacg gggtctgacg ctcagtggaa 15060

cgaaaactca cgttaaggga ttttggtcat gagattatca aaaaggatct tcacctagat 15120

ccttttaaat taaaaatgaa gttttaaatc aatctaaagt atatatgagt aaacttggtc 15180

tgacagttac caatgcttaa tcagtgaggc acctatctca gcgatctgtc tatttcgttc 15240

atccatagtt gcctgactcc ccgtcgtgta gataactacg atacgggagg gcttaccatc 15300

tggccccagt gctgcaatga taccgcgaga cccacgctca ccggctccag atttatcagc 15360

aataaaccag ccagccggaa gggccgagcg cagaagtggt cctgcaactt tatccgcctc 15420

catccagtct attaattgtt gccgggaagc tagagtaagt agttcgccag ttaatagttt 15480

gcgcaacgtt gttgccattg ctacaggcat cgtggtgtca cgctcgtcgt ttggtatggc 15540

ttcattcagc tccggttccc aacgatcaag gcgagttaca tgatccccca tgttgtgcaa 15600

aaaagcggtt agctccttcg gtcctccgat cgttgtcaga agtaagttgg ccgcagtgtt 15660

atcactcatg gttatggcag cactgcataa ttctcttact gtcatgccat ccgtaagatg 15720

cttttctgtg actggtgagt actcaaccaa gtcattctga gaatagtgta tgcggcgacc 15780

gagttgctct tgcccggcgt caatacggga taataccgcg ccacatagca gaactttaaa 15840

agtgctcatc attggaaaac gttcttcggg gcgaaaactc tcaaggatct taccgctgtt 15900

gagatccagt tcgatgtaac ccactcgtgc acccaactga tcttcagcat cttttacttt 15960

caccagcgtt tctgggtgag caaaaacagg aaggcaaaat gccgcaaaaa agggaataag 16020

ggcgacacgg aaatgttgaa tactcatact cttccttttt caatattatt gaagcattta 16080

tcagggttat tgtctcatga gcggatacat atttgaatgt atttagaaaa ataaacaaat 16140

aggggttccg cgcacatttc cccgaaaagt gccacctgac gtctaagaaa ccattattat 16200

catgacatta acctataaaa ataggcgtat cacgaggccc tttcgtc 16247

<210> 67
<211> 1674
<212> DNA
<213> Homo sapiens

<400> 67
atgaaagcca tgccctggaa ctggacctgc cttctctccc acctcctcat ggtgggcatg 60

ggctcctcca ctttgctcac ccggcagcca gccccgctgt cccagaagca gcggtcattt 120

gtcacattcc gaggagagcc cgccgagggt ttcaatcacc tggtggtgga tgagaggaca 180

ggacacattt acttgggggc cgtcaatcgg atttacaagc tctccagcga cctgaaggtc 240

ttggtgacgc atgagacagg gccggacgag gacaacccca agtgttaccc accccgcatc 300

gtccagacct gcaatgagcc cctgaccacc accaacaatg tcaacaagat gctcctcata 360

gactacaagg agaacaggct gattgcctgt gggagcctgt accaaggcat ctgcaagctg 420

ctgaggctgg aggacctctt caagctgggg gagccttatc ataagaagga gcactatctg 480

tcaggtgtca acgagagcgg ctcagtcttt ggagtgatcg tctcctacag caacctggat 540

gacaagctgt tcattgccac ggcagtggat gggaagcccg agtattttcc caccatctcc 600

agccggaaac tgaccaagaa ctctgaggcg gatggcatgt tcgcgtacgt cttccatgat 660

gagttcgtgg cctcgatgat taagatccct tcggacacct tcaccatcat ccctgacttt 720

gatatctact atgtctatgg ttttagcagt ggcaactttg tctacttttt gaccctccaa 780

cctgagatgg tgtctccacc aggctccacc accaaggagc aggtgtatac atccaagctc 840

gtgaggcttt gcaaggagga cacagccttc aactcctatg tagaggtgcc cattggctgt 900

gagcgcagtg gggtggagta ccgcctgctg caggctgcct acctgtccaa agcgggggcc 960

gtgcttggca ggacccttgg agtccatcca gatgatgacc tgctcttcac cgtcttctcc 1020

aagggccaga agcggaaaat gaaatccctg gatgagtcgg ccctgtgcat cttcatcttg 1080

aagcagataa atgaccgcat taaggagcgg ctgcagtctt gttaccgggg cgagggcacg 1140

ctggacctgg cctggctcaa ggtgaaggac atcccctgca gcagtgcgct cttaaccatt 1200

gacgataact tctgtggcct ggacatgaat gctcccctgg gagtgtccga catggtgcgt 1260

ggaattcccg tcttcacgga ggacagggac cgcatgacgt ctgtcatcgc atatgtctac 1320

aagaaccact ctctggcctt tgtgggcacc aaaagtggca agctgaagaa gatccgggtg 1380

gatggaccca ggggcaacgc cctccagtat gagacggtgc aggtggtgga ccccggccca 1440

gtcctccggg atatggcctt ctccaaggac cacgagcaac tctacatcat gtcagagagg 1500

cagctcacca gagtccctgt ggagtcctgt ggtcagtatc agagctgcgg cgagtgcctt 1560

ggctcaggcg acccccactg tggctggtgt gtgctgcaca acacttgcac ccggaaggag 1620

cggtgtgagc ggtccaagga gcccgactac aaggacgacg atgacaagtg atga 1674

<210> 68
<211> 556
<212> PRT
<213> Homo sapiens

<400> 68

Met Lys Ala Met Pro Trp Asn Trp Thr Cys Leu Leu Ser His Leu Leu
1               5                   10                  15

Met Val Gly Met Gly Ser Ser Thr Leu Leu Thr Arg Gln Pro Ala Pro
            20                  25                  30

Leu Ser Gln Lys Gln Arg Ser Phe Val Thr Phe Arg Gly Glu Pro Ala
        35                  40                  45

Glu Gly Phe Asn His Leu Val Val Asp Glu Arg Thr Gly His Ile Tyr
    50                  55                  60

Leu Gly Ala Val Asn Arg Ile Tyr Lys Leu Ser Ser Asp Leu Lys Val
65                  70                  75                  80

Leu Val Thr His Glu Thr Gly Pro Asp Glu Asp Asn Pro Lys Cys Tyr

                85                  90                  95

Pro Pro Arg Ile Val Gln Thr Cys Asn Glu Pro Leu Thr Thr Thr Asn
            100                 105                 110

Asn Val Asn Lys Met Leu Leu Ile Asp Tyr Lys Glu Asn Arg Leu Ile
        115                 120                 125

Ala Cys Gly Ser Leu Tyr Gln Gly Ile Cys Lys Leu Leu Arg Leu Glu
    130                 135                 140

Asp Leu Phe Lys Leu Gly Glu Pro Tyr His Lys Lys Glu His Tyr Leu
145                 150                 155                 160

Ser Gly Val Asn Glu Ser Gly Ser Val Phe Gly Val Ile Val Ser Tyr
                165                 170                 175

Ser Asn Leu Asp Asp Lys Leu Phe Ile Ala Thr Ala Val Asp Gly Lys
            180                 185                 190

Pro Glu Tyr Phe Pro Thr Ile Ser Ser Arg Lys Leu Thr Lys Asn Ser
        195                 200                 205

Glu Ala Asp Gly Met Phe Ala Tyr Val Phe His Asp Glu Phe Val Ala
    210                 215                 220

Ser Met Ile Lys Ile Pro Ser Asp Thr Phe Thr Ile Ile Pro Asp Phe
225                 230                 235                 240

Asp Ile Tyr Tyr Val Tyr Gly Phe Ser Ser Gly Asn Phe Val Tyr Phe
                245                 250                 255

Leu Thr Leu Gln Pro Glu Met Val Ser Pro Pro Gly Ser Thr Thr Lys
            260                 265                 270

Glu Gln Val Tyr Thr Ser Lys Leu Val Arg Leu Cys Lys Glu Asp Thr
        275                 280                 285

Ala Phe Asn Ser Tyr Val Glu Val Pro Ile Gly Cys Glu Arg Ser Gly
    290                 295                 300

Val Glu Tyr Arg Leu Leu Gln Ala Ala Tyr Leu Ser Lys Ala Gly Ala
305                 310                 315                 320

Val Leu Gly Arg Thr Leu Gly Val His Pro Asp Asp Asp Leu Leu Phe
                325                 330                 335

Thr Val Phe Ser Lys Gly Gln Lys Arg Lys Met Lys Ser Leu Asp Glu
            340                 345                 350

Ser Ala Leu Cys Ile Phe Ile Leu Lys Gln Ile Asn Asp Arg Ile Lys
        355                 360                 365

Glu Arg Leu Gln Ser Cys Tyr Arg Gly Glu Gly Thr Leu Asp Leu Ala
    370                 375                 380

Trp Leu Lys Val Lys Asp Ile Pro Cys Ser Ser Ala Leu Leu Thr Ile
385                 390                 395                 400

Asp Asp Asn Phe Cys Gly Leu Asp Met Asn Ala Pro Leu Gly Val Ser
                405                 410                 415

Asp Met Val Arg Gly Ile Pro Val Phe Thr Glu Asp Arg Asp Arg Met
            420                 425                 430

Thr Ser Val Ile Ala Tyr Val Tyr Lys Asn His Ser Leu Ala Phe Val
        435                 440                 445

Gly Thr Lys Ser Gly Lys Leu Lys Lys Ile Arg Val Asp Gly Pro Arg
    450                 455                 460

Gly Asn Ala Leu Gln Tyr Glu Thr Val Gln Val Val Asp Pro Gly Pro
465                 470                 475                 480

Val Leu Arg Asp Met Ala Phe Ser Lys Asp His Glu Gln Leu Tyr Ile
                485                 490                 495

Met Ser Glu Arg Gln Leu Thr Arg Val Pro Val Glu Ser Cys Gly Gln
            500                 505                 510

Tyr Gln Ser Cys Gly Glu Cys Leu Gly Ser Gly Asp Pro His Cys Gly
        515                 520                 525

Trp Cys Val Leu His Asn Thr Cys Thr Arg Lys Glu Arg Cys Glu Arg
    530                 535                 540

Ser Lys Glu Pro Asp Tyr Lys Asp Asp Asp Asp Lys
545                 550                 555

<210>  69
<211>  333
<212>  DNA
<213>  Artificial Sequence

<220>
<223>  Synthetic DNA

<400>  69
cagtctgccc tgactcagcc tgcctccgtg tctgggtctc ctggacagtc gatcaccatc      60

tcctgctctg gaagcagctc caacatcgga agtaattatg tatactggta ccaacagcac     120

ccaggcaaag cccccaaact catgatttat aggaataatc agcggccctc aggggtttct     180

aatcgcttct ctggctccaa gtctggcaac acggcctccc tgaccatctc tgggctccag     240

gctgaggacg aggctgatta ttactgcgca gcatgggatg acagcctgag tggtcattgg     300

gtgttcggcg gagggaccaa gctgaccgtc cta                                  333


<210>  70
<211>  333
<212>  DNA
<213>  Artificial Sequence

<220>
<223>  Synthetic DNA

<400>  70
cagtctgccc tgactcagcc tgcctccgtg tctgggtctc ctggacagtc gatcaccatc      60

tcctgctctg gaagcagctc caacatcgga aataatgctg taaactggta ccaacagcac     120

ccaggcaaag cccccaaact catgatttat tatgatgatc tgctgccctc aggggtttct     180

aatcgcttct ctggctccaa gtctggcaac acggcctccc tgaccatctc tgggctccag     240

gctgaggacg aggctgatta ttactgcgga acatgggatg gcagcctgaa tggtgggagg     300

ctgttcggcg gagggaccaa gctgaccgtc cta                                  333


<210>  71
<211>  324
<212>  DNA
<213>  Artificial Sequence

<220>
<223>  Synthetic DNA

<400>  71
cagtctgccc tgactcagcc tgcctccgtg tctgggtctc ctggacagtc gatcaccatc      60

tcctgctctg gagaagcatt gccaaaaaaa tatgcttatt ggtaccaaca gcacccaggc     120

aaagccccca aactcatgat ttataaagac agcgagaggc cctcaggggt ttctaatcgc     180

ttctctggct ccaagtctgg caacacggcc tccctgacca tctctgggct ccaggctgag     240

gacgaggctg attattactg caactctttt acaagcacca ccaattatgt cttattcggc     300

ggagggacca agctgaccgt ccta                                            324


<210>  72
<211>  345
<212>  DNA
<213>  Artificial Sequence

<220>
<223>  Synthetic DNA

<400>  72
cagtctgccc tgactcagcc tgcctccgtg tctgggtctc ctggacagtc gatcaccatc      60

tcctgcaccc taagcagtga gcacagcacc tacaccatcg aatggtacca acagcaccca     120

ggcaaagccc ccaaactcat gatttatgtt aagagtgatg gcagccacag caagggggac    180

ggggtttcta atcgcttctc tggctccaag tctggcaaca cggcctccct gaccatctct     240

gggctccagg ctgaggacga ggctgattat tactgcggag agagccacac gattgatggc     300

caagtcggtg tggtattcgg cggagggacc aagctgaccg tccta                     345


<210>  73
<211>  336
<212>  DNA
<213>  Artificial Sequence

<220>
<223>  Synthetic DNA

<400>  73
cagtctgccc tgactcagcc tgcctccgtg tctgggtctc ctggacagtc gatcaccatc      60

tcctgcactc tgagcagtgg gcacagtagc tacatcatcg catggtacca acagcaccca     120

ggcaaagccc ccaaactcat gatttatctt gaaggtagtg gaagctacaa caaggggagc     180

ggggtttcta atcgcttctc tggctccaag tctggcaaca cggcctccct gaccatctct     240

gggctccagg ctgaggacga ggctgattat tactgcgaga cctgggacag taacactcat     300

gtggtattcg gcggagggac caagctgacc gtccta                               336


<210>  74
<211>  333
<212>  DNA
<213>  Artificial Sequence

<220>
<223>  Synthetic DNA

<400>  74
cagtctgccc tgactcagcc tgcctccgtg tctgggtctc ctggacagtc gatcaccatc      60

tcctgctctg gaagcagctc caacatcgga agtaattatg tatactggta ccaacagcac     120

ccaggcaaag cccccaaact catgatttat aggaataatc agcggccctc aggggtttct     180

aatcgcttct ctggctccaa gtctggcaac acggcctccc tgaccatctc tgggctccag     240

gctgaggacg aggctgatta ttactgcgca gcatgggatg acagcctgag tggtcattgg     300

gtgttcggcg gagggaccaa gctgaccgtc cta                                  333


<210>  75

<211>  336
<212>  DNA
<213>  Artificial Sequence

<220>
<223>  Synthetic DNA

<400>  75
cagtctgccc tgactcagcc tgcctccgtg tctgggtctc ctggacagtc gatcaccatc      60

tcctgcactg ggagcagctc caacatcggg gcaggttatg atgtacactg gtaccaacag     120

cacccaggca aagcccccaa actcatgatt tatggtaaca gcaatcggcc ctcaggggtt     180

tctaatcgct tctctggctc caagtctggc aacacggcct ccctgaccat ctctgggctc     240

caggctgagg acgaggctga ttattactgc cagtcctatg acagcagcct gagtggttct     300

gtggtattcg gcggagggac caagctgacc gtccta                               336


<210>  76
<211>  333
<212>  DNA
<213>  Artificial Sequence

<220>
<223>  Synthetic DNA

<400>  76
cagtctgccc tgactcagcc tgcctccgtg tctgggtctc ctggacagtc gatcaccatc      60

tcctgctctg gaagcagctc caacattggg aataattatg tatcctggta ccaacagcac     120

ccaggcaaag cccccaaact catgatttat gacaataata agcgaccctc aggggtttct     180

aatcgcttct ctggctccaa gtctggcaac acggcctccc tgaccatctc tgggctccag     240

gctgaggacg aggctgatta ttactgcgga acatgggata gcagcctgag tgctggtgct     300

gtgttcggcg gagggaccaa gctgaccgtc cta                                  333


<210>  77
<211>  333
<212>  DNA
<213>  Artificial Sequence

<220>
<223>  Synthetic DNA

<400>  77
cagtctgccc tgactcagcc tgcctccgtg tctgggtctc ctggacagtc gatcaccatc      60

tcctgcactg ggagcagctc caacattggg gcgggttatg ttgtacattg gtaccaacag     120

cacccaggca aagcccccaa actcatgatt tatgaaaata ataagcgacc ctcaggggtt     180

tctaatcgct tctctggctc caagtctggc aacacggcct ccctgaccat ctctgggctc     240

caggctgagg acgaggctga ttattactgc agctcatata caagcagcgg cactcctgtg     300

gtattcggcg gagggaccaa gctgaccgtc cta                                  333

<210>  78
<211>  363
<212>  DNA
<213>  Artificial Sequence

<220>
<223>  Synthetic DNA

<400>  78
cagtctgccc tgactcagcc tgcctccgtg tctgggtctc ctggacagtc gatcaccatc      60

tcctgcactg gaaccagcag tgatgttggg agttataacc ttgtctcctg gtaccaacag     120

cacccaggca aagcccccaa actcatgatt tatgagggca gtaagcggcc ctcaggggtt     180

tctaatcgct tctctggctc caagtctggc aacacggcct ccctgaccat ctctgggctc     240

caggctgagg acgaggctga ttattactgc cagtcctatg acagcagcct gagtggttgc     300

cagtcctatg acagcagcct gagtggtttg gtgttcggcg gagggaccaa gctgaccgtc     360

cta                                                                   363


<210>  79
<211>  318
<212>  DNA
<213>  Artificial Sequence

<220>
<223>  Synthetic DNA

<400>  79
cagtctgccc tgactcagcc tgcctccgtg tctgggtctc ctggacagtc gatcaccatc      60

tcctgcgggg gaaacaacat tggaagtaaa aatgtgcact ggtaccaaca gcacccaggc     120

aaagccccca aactcatgat ttatagggat agcaaccggc cctctggggt ttctaatcgc     180

ttctctggct ccaagtctgg caacacggcc tccctgacca tctctgggct ccaggctgag     240

gacgaggctg attattactg ccagtcctat gacaggagtg gttgggtgtt cggcggaggg     300

accaagctga ccgtccta                                                   318


<210>  80
<211>  324
<212>  DNA
<213>  Artificial Sequence

<220>
<223>  Synthetic DNA

<400>  80
cagtctgccc tgactcagcc tgcctccgtg tctgggtctc ctggacagtc gatcaccatc      60

tcctgctctg gagaagcatt gccaaaaaaa tatgcttatt ggtaccaaca gcacccaggc     120

aaagccccca aactcatgat ttataaagac agcgagaggc cctcaggggt ttctaatcgc     180

ttctctggct ccaagtctgg caacacggcc tccctgacca tctctgggct ccaggctgag 240

gacgaggctg attattactg caactctttt acaagcacca ccaattatgt cttattcggc 300

ggagggacca agctgaccgt ccta 324

<210> 81
<211> 324
<212> DNA
<213> Artificial Sequence

<220>
<223> Synthetic DNA

<400> 81
cagtctgccc tgactcagcc tgcctccgtg tctgggtctc ctggacagtc gatcaccatc 60

tcctgcgggg gaaacaacat tggaagtaaa agtgtgcact ggtaccaaca gcacccaggc 120

aaagccccca aactcatgat ttattatgat agcgaccggc cctcaggggt ttctaatcgc 180

ttctctggct ccaagtctgg caacacggcc tccctgacca tctctgggct ccaggctgag 240

gacgaggctg attattactg ccaggtgtgg gatgataata gtgatcatct tatattcggc 300

ggagggacca agctgaccgt ccta 324

<210> 82
<211> 321
<212> DNA
<213> Artificial Sequence

<220>
<223> Synthetic DNA

<400> 82
cagtctgccc tgactcagcc tgcctccgtg tctgggtctc ctggacagtc gatcaccatc 60

tcctgctctg gagatgcatt gccaaagcaa tatgcttatt ggtaccaaca gcacccaggc 120

aaagccccca aactcatgat ttatgatgat agcgaccggc cctcaggggt ttctaatcgc 180

ttctctggct ccaagtctgg caacacggcc tccctgacca tctctgggct ccaggctgag 240

gacgaggctg attattactg cagctcatat gcaggcagca acaattgggt gttcggcgga 300

gggaccaagc tgaccgtcct a 321

<210> 83
<211> 345
<212> DNA
<213> Artificial Sequence

<220>
<223> Synthetic DNA

<400> 83
   cagtctgccc tgactcagcc tgcctccgtg tctgggtctc ctggacagtc gatcaccatc 60
   tcctgcaccc taagcagtga gcacagcacc tacaccatcg aatggtacca acagcaccca 120
   ggcaaagccc ccaaactcat gatttatgtt aagagtgatg gcagccacag caagggggac 180
   ggggtttcta atcgcttctc tggctccaag tctggcaaca cggcctccct gaccatctct 240
   gggctccagg ctgaggacga ggctgattat tactgcggag agagccacac gattgatggc 300
   caagtcggtg tggtattcgg cggagggacc aagctgaccg tccta 345

<210> 84
<211> 345
<212> DNA
<213> Artificial Sequence

<220>
<223> Synthetic DNA

<400> 84
   cagtctgccc tgactcagcc tgcctccgtg tctgggtctc ctggacagtc gatcaccatc 60
   tcctgcactc tgagcagtgg gcacagcagc tacgccatcg catggtacca acagcaccca 120
   ggcaaagccc ccaaactcat gatttatctt aacagtgatg gcagccacag caagggggac 180
   ggggtttcta atcgcttctc tggctccaag tctggcaaca cggcctccct gaccatctct 240
   gggctccagg ctgaggacga ggctgattat tactgcagct catatgcagg caacaacaat 300
   ttgggggatg tgcttttcgg cggagggacc aagctgaccg tccta 345

<210> 85
<211> 342
<212> DNA
<213> Artificial Sequence

<220>
<223> Synthetic DNA

<400> 85
cagtctgccc tgactcagcc tgcctccgtg tctgggtctc ctggacagtc gatcaccatc 60

tcctgcacct tgcgcagtgg catcaatgtt ggtacctaca ggatatactg gtaccaacag 120

cacccaggca aagcccccaa actcatgatt tattacaaat cagactcaga taagcagcag 180

ggctctgggg tttctaatcg cttctctggc tccaagtctg gcaacacggc ctccctgacc 240

atctctgggc tccaggctga ggacgaggct gattattact gcgcagcatg ggatgacagc 300

ctgaatgtgg tattcggcgg agggaccaag ctgaccgtcc ta 342

<210> 86
<211> 327
<212> DNA
<213> Artificial Sequence

<220>
<223> Synthetic DNA

<400> 86

cagtctgccc tgactcagcc tgcctccgtg tctgggtctc ctggacagtc gatcaccatc 60

tcctgcaccc gcagcagtgg cagcattgcc agcaactatg tgcagtggta ccaacagcac 120

ccaggcaaag cccccaaact catgatttat gaggataacc aaagaccctc tggggtttct 180

aatcgcttct ctggctccaa gtctggcaac acggcctccc tgaccatctc tgggctccag 240

gctgaggacg aggctgatta ttactgccag tcctatgaca gcagcctgag gagagtcttc 300

ggcggaggga ccaagctgac cgtccta 327

<210> 87
<211> 333
<212> DNA
<213> Artificial Sequence

<220>
<223> Synthetic DNA

<400> 87
cagtctgccc tgactcagcc tgcctccgtg tctgggtctc ctggacagtc gatcaccatc 60

tcctgcggct ccagcactgg agctgtcacc agtggtcatt atccctactg gtaccaacag 120

cacccaggca aagcccccaa actcatgatt tatgatacaa gcaacaaaca ctccggggtt 180

tctaatcgct tctctggctc caagtctggc aacacggcct ccctgaccat ctctgggctc 240

caggctgagg acgaggctga ttattactgc gcaacatggg atgacagcct gaatgaggga 300

gtgttcggcg gagggaccaa gctgaccgtc cta 333

<210> 88
<211> 348
<212> DNA
<213> Artificial Sequence

<220>
<223> Synthetic DNA

<400> 88
cagtctgccc tgactcagcc tgcctccgtg tctgggtctc ctggacagtc gatcaccatc 60

tcctgcaccc tgagcagcgg ctacagtaat tataaagtgg actggtacca acagcaccca 120

ggcaaagccc ccaaactcat gatttatgtg ggcactggtg ggattgtggg atccaagggg 180

gatggggttt ctaatcgctt ctctggctcc aagtctggca acacggcctc cctgaccatc 240

tctgggctcc aggctgagga cgaggctgat tattactgcg gggcagacca tggcagtggg 300

agcaacttcg tggtggtatt cggcggaggg accaagctga ccgtccta 348

<210> 89
<211> 369
<212> DNA
<213> Artificial Sequence

<220>

<223>  Synthetic DNA

<400>  89
gaggtgcagc tgttggagtc tgggggaggc ttggtacagc ctggggggtc cctgagactc      60

tcctgtgcag cctctggatt cacctttagc ggctactata tgcactgggt ccgccaggct     120

ccagggaagg ggctggagtg ggtctcatgg atcaacccta acagtggtgg cacaaactat     180

gcacagaagt ttcagggccg gttcaccatc tccagagaca attccaagaa cacgctgtat     240

ctgcaaatga acagcctgag agccgaggac acggccgtat attactgtgc gaaaggttgg     300

ggctatacgg tgactaccct caactggttc gacccctggg gccagggcac cctggtcacc     360

gtctcctca                                                             369


<210>  90
<211>  366
<212>  DNA
<213>  Artificial Sequence

<220>
<223>  Synthetic DNA

<400>  90
gaggtgcagc tgttggagtc tgggggaggc ttggtacagc ctggggggtc cctgagactc      60

tcctgtgcag cctctggatt cacctttagc agctatgcta tgcattgggt ccgccaggct     120

ccagggaagg ggctggagtg ggtctcatgg atcaacgctg gcaatggtaa cacaaaatat     180

tcacagaagt tccagggccg gttcaccatc tccagagaca attccaagaa cacgctgtat     240

ctgcaaatga acagcctgag agccgaggac acggccgtat attactgtgc gaaagcgtct     300

agtagtggtt attactacgt ccactttgac tactggggcc agggcaccct ggtcaccgtc     360

tcctca                                                                366


<210>  91
<211>  363
<212>  DNA
<213>  Artificial Sequence

<220>
<223>  Synthetic DNA

<400>  91
gaggtgcagc tgttggagtc tgggggaggc ttggtacagc ctggggggtc cctgagactc      60

tcctgtgcag cctctggatt cacctttagc agttatgata tcaactgggt ccgccaggct     120

ccagggaagg ggctggagtg ggtctcatgg atgaacccta acagtggtaa cacaggctat     180

gcacagaagt tccagggccg gttcaccatc tccagagaca attccaagaa cacgctgtat     240

ctgcaaatga acagcctgag agccgaggac acggccgtat attactgtgc gaaatccagc     300

agctggcact actactacgg tatggacgtc tggggccagg gcaccctggt caccgtctcc     360

tca                                                                 363

<210>  92
<211>  360
<212>  DNA
<213>  Artificial Sequence

<220>
<223>  Synthetic DNA

<400>  92
gaggtgcagc tgttggagtc tgggggaggc ttggtacagc ctggggggtc cctgagactc      60

tcctgtgcag cctctggatt cacctttagc agctatggta tcagctgggt ccgccaggct     120

ccagggaagg ggctggagtg ggtctcatgg atcagcgctt acaatggtaa cacaaactat     180

gcacagaagc tccagggccg gttcaccatc tccagagaca attccaagaa cacgctgtat     240

ctgcaaatga acagcctgag agccgaggac acggccgtat attactgtgc gaaagattta     300

aggttatcct attactactt tgactactgg ggccagggca ccctggtcac cgtctcctca     360

<210>  93
<211>  360
<212>  DNA
<213>  Artificial Sequence

<220>
<223>  Synthetic DNA

<400>  93
gaggtgcagc tgttggagtc tgggggaggc ttggtacagc ctggggggtc cctgagactc      60

tcctgtgcag cctctggatt cacctttagc gaattatcca tgcactgggt ccgccaggct     120

ccagggaagg ggctggagtg ggtctcaggt tttgatcctg aagatggtga aacaatctac     180

gcacagaagt tccagggccg gttcaccatc tccagagaca attccaagaa cacgctgtat     240

ctgcaaatga acagcctgag agccgaggac acggccgtat attactgtgc gaaaggaacc     300

tactactact actacggtat ggacgtctgg ggccagggca ccctggtcac cgtctcctca     360

<210>  94
<211>  369
<212>  DNA
<213>  Artificial Sequence

<220>
<223>  Synthetic DNA

<400>  94
gaggtgcagc tgttggagtc tgggggaggc ttggtacagc ctggggggtc cctgagactc      60

tcctgtgcag cctctggatt cacctttagc ggctactata tgcactgggt ccgccaggct     120

ccagggaagg ggctggagtg ggtctcatgg atcaacccta acagtggtgg cacaaactat     180

gcacagaagt ttcagggccg gttcaccatc tccagagaca attccaagaa cacgctgtat     240

ctgcaaatga acagcctgag agccgaggac acggccgtat attactgtgc gaaaggttgg 300

ggctatacgg tgactaccct caactggttc gacccctggg gccagggcac cctggtcacc 360

gtctcctca 369

<210> 95
<211> 363
<212> DNA
<213> Artificial Sequence

<220>
<223> Synthetic DNA

<400> 95
gaggtgcagc tgttggagtc tgggggaggc ttggtacagc ctggggggtc cctgagactc 60

tcctgtgcag cctctggatt cacctttagc agttatgata tcaactgggt ccgccaggct 120

ccagggaagg ggctggagtg ggtctcatgg atgaacccta acagtggtaa cacaggctat 180

gcacagaagt tccagggccg gttcaccatc tccagagaca attccaagaa cacgctgtat 240

ctgcaaatga acagcctgag agccgaggac acggccgtat attactgtgc gaaatccagc 300

agctggcact actactacgg tatggacgtc tggggccagg gcaccctggt caccgtctcc 360

tca 363

<210> 96
<211> 360
<212> DNA
<213> Artificial Sequence

<220>
<223> Synthetic DNA

<400> 96
gaggtgcagc tgttggagtc tgggggaggc ttggtacagc ctggggggtc cctgagactc 60

tcctgtgcag cctctggatt cacctttagc gaattatcca tgcactgggt ccgccaggct 120

ccagggaagg ggctggagtg ggtctcaggt tttgatcctg aagatggtga aacaatctac 180

gcacagaagt tccagggccg gttcaccatc tccagagaca attccaagaa cacgctgtat 240

ctgcaaatga acagcctgag agccgaggac acggccgtat attactgtgc gaaaggaacc 300

tactactact actacggtat ggacgtctgg ggccagggca ccctggtcac cgtctcctca 360

<210> 97
<211> 363
<212> DNA
<213> Artificial Sequence

<220>
<223> Synthetic DNA

<400> 97

gaggtgcagc tgttggagtc tgggggaggc ttggtacagc ctggggggtc cctgagactc 60

tcctgtgcag cctctggatt cacctttagc agctactata tgcactgggt ccgccaggct 120

ccagggaagg ggctggagtg ggtctcaata atcaacccta gtggtggtag cacaagctac 180

gcacagaagt tccagggccg gttcaccatc tccagagaca attccaagaa cacgctgtat 240

ctgcaaatga acagcctgag agccgaggac acggccgtat attactgtgc gaaaagtttc 300

agcagcagct ggtacaactg gttcgacccc tggggccagg gcaccctggt caccgtctcc 360

tca 363

<210> 98
<211> 357
<212> DNA
<213> Artificial Sequence

<220>
<223> Synthetic DNA

<400> 98
gaggtgcagc tgttggagtc tgggggaggc ttggtacagc ctggggggtc cctgagactc 60

tcctgtgcag cctctggatt cacctttagc agctatgcta tcagctgggt ccgccaggct 120

ccagggaagg ggctggagtg ggtctcaggg atcatcccta tctttggtac agcaaactac 180

gcacagaagt tccagggccg gttcaccatc tccagagaca attccaagaa cacgctgtat 240

ctgcaaatga acagcctgag agccgaggac acggccgtat attactgtgc gaaagggtcc 300

gtcctcggtg atgcttttga tatctggggc cagggcaccc tggtcaccgt ctcctca 357

<210> 99
<211> 366
<212> DNA
<213> Artificial Sequence

<220>
<223> Synthetic DNA

<400> 99
gaggtgcagc tgttggagtc tgggggaggc ttggtacagc ctggggggtc cctgagactc 60

tcctgtgcag cctctggatt cacctttagc aatgctagaa tgggtgtgag ctgggtccgc 120

caggctccag ggaaggggct ggagtgggtc tcacacattt tttcgaatga cgaaaaatcc 180

tacagcacat ctctgaagag ccggttcacc atctccagag acaattccaa gaacacgctg 240

tatctgcaaa tgaacagcct gagagccgag gacacggccg tatattactg tgcgaaaatc 300

ccgtatgaag cagtggctgg ctactttgac tactggggcc agggcaccct ggtcaccgtc 360

tcctca 366

<210> 100
<211> 360

<212>  DNA
<213>  Artificial Sequence

<220>
<223>  Synthetic DNA

<400>  100
gaggtgcagc tgttggagtc tgggggaggc ttggtacagc ctggggggtc cctgagactc      60

tcctgtgcag cctctggatt cacctttagc agctattgga tgagctgggt ccgccaggct     120

ccagggaagg ggctggagtg ggtctcaaac ataaagcaag atggaagtga gaaatactat     180

gtggactctg tgaagggccg gttcaccatc tccagagaca attccaagaa cacgctgtat     240

ctgcaaatga acagcctgag agccgaggac acggccgtat attactgtgc gaaagttacg     300

gtgaccggga cctggtactt cgatctctgg ggccagggca ccctggtcac cgtctcctca     360


<210>  101
<211>  372
<212>  DNA
<213>  Artificial Sequence

<220>
<223>  Synthetic DNA

<400>  101
gaggtgcagc tgttggagtc tgggggaggc ttggtacagc ctggggggtc cctgagactc      60

tcctgtgcag cctctggatt cacctttagc agctacgaca tgcactgggt ccgccaggct     120

ccagggaagg ggctggagtg ggtctcagct attggtactg ctggtgacac atactatcca     180

ggctccgtga agggccggtt caccatctcc agagacaatt ccaagaacac gctgtatctg     240

caaatgaaca gcctgagagc cgaggacacg gccgtatatt actgtgcgaa agcggataca     300

gctatggtca tttactacta ctactacggt atggacgtct ggggccaggg caccctggtc     360

accgtctcct ca                                                         372


<210>  102
<211>  357
<212>  DNA
<213>  Artificial Sequence

<220>
<223>  Synthetic DNA

<400>  102
gaggtgcagc tgttggagtc tgggggaggc ttggtacagc ctggggggtc cctgagactc      60

tcctgtgcag cctctggatt cacctttagc agctatagca tgaactgggt ccgccaggct     120

ccagggaagg ggctggagtg ggtctcatcc attagtagta gtagtagtta catatactac     180

gcagactcag tgaagggccg gttcaccatc tccagagaca attccaagaa cacgctgtat     240

ctgcaaatga acagcctgag agccgaggac acggccgtat attactgtgc gaaagagaat     300

agtggctatg attactttga ctactggggc cagggcaccc tggtcaccgt ctcctca 357

<210> 103
<211> 357
<212> DNA
<213> Artificial Sequence

<220>
<223> Synthetic DNA

<400> 103
gaggtgcagc tgttggagtc tgggggaggc ttggtacagc ctggggggtc cctgagactc 60

tcctgtgcag cctctggatt cacctttagc gattatgcta tgagctgggt ccgccaggct 120

ccagggaagg ggctggagtg ggtctcattc attagaagca aagcttatgg tgggacaaca 180

gaatacaccg cgtctgtgaa aggccggttc accatctcca gagacaattc caagaacacg 240

ctgtatctgc aaatgaacag cctgagagcc gaggacacgg ccgtatatta ctgtgcgaaa 300

gatctaggta gtggctttga ctactggggc cagggcaccc tggtcaccgt ctcctca 357

<210> 104
<211> 378
<212> DNA
<213> Artificial Sequence

<220>
<223> Synthetic DNA

<400> 104
gaggtgcagc tgttggagtc tgggggaggc ttggtacagc ctggggggtc cctgagactc 60

tcctgtgcag cctctggatt cacctttagc gaccactaca tggactgggt ccgccaggct 120

ccagggaagg ggctggagtg ggtctcacgt actagaaaca aagctaacag ttacaccaca 180

gaatacgccg cgtctgtgaa aggccggttc accatctcca gagacaattc caagaacacg 240

ctgtatctgc aaatgaacag cctgagagcc gaggacacgg ccgtatatta ctgtgcgaaa 300

ggaggccagg ggagccgata ctactactac tacggtatgg acgtctgggg ccagggcacc 360

ctggtcaccg tctcctca 378

<210> 105
<211> 345
<212> DNA
<213> Artificial Sequence

<220>
<223> Synthetic DNA

<400> 105
gaggtgcagc tgttggagtc tgggggaggc ttggtacagc ctggggggtc cctgagactc 60

tcctgtgcag cctctggatt cacctttagc agcaatgaga tgagctgggt ccgccaggct 120

ccagggaagg ggctggagtg ggtctcatcc attagtggtg gtagcacata ctacgcagac 180

tccaggaagg gccggttcac catctccaga gacaattcca agaacacgct gtatctgcaa 240

atgaacagcc tgagagccga ggacacggcc gtatattact gtgcgaaaga ctcatatgat 300

gcttttgata tctggggcca gggcaccctg gtcaccgtct cctca 345

<210> 106
<211> 357
<212> DNA
<213> Artificial Sequence

<220>
<223> Synthetic DNA

<400> 106
gaggtgcagc tgttggagtc tgggggaggc ttggtacagc ctggggggtc cctgagactc 60

tcctgtgcag cctctggatt cacctttagc agtggtggtt actcctggag ctgggtccgc 120

caggctccag ggaaggggct ggagtgggtc tcatacatct atcatagtgg gagcacctac 180

tacaacccgt ccctcaagag tcggttcacc atctccagag acaattccaa gaacacgctg 240

tatctgcaaa tgaacagcct gagagccgag gacacggccg tatattactg tgcgaaagat 300

gcgagtaacc atttctttga ctactggggc cagggcaccc tggtcaccgt ctcctca 357

<210> 107
<211> 360
<212> DNA
<213> Artificial Sequence

<220>
<223> Synthetic DNA

<400> 107
gaggtgcagc tgttggagtc tgggggaggc ttggtacagc ctggggggtc cctgagactc 60

tcctgtgcag cctctggatt cacctttagc agtagtagtt actactgggg ctgggtccgc 120

caggctccag ggaaggggct ggagtgggtc tcaagtatct attatagtgg gagcacctac 180

tacaacccgt ccctcaagag tcggttcacc atctccagag acaattccaa gaacacgctg 240

tatctgcaaa tgaacagcct gagagccgag gacacggccg tatattactg tgcgaaaaag 300

gactggaacg acaactggtt cgacccctgg ggccagggca ccctggtcac cgtctcctca 360

<210> 108
<211> 381
<212> DNA
<213> Artificial Sequence

<220>
<223> Synthetic DNA

<400> 108
gaggtgcagc tgttggagtc tgggggaggc ttggtacagc ctggggggtc cctgagactc 60

tcctgtgcag cctctggatt cacctttagc agcaacagtg ctgcttggaa ctgggtccgc 120

caggctccag ggaaggggct ggagtgggtc tcaaggacat actacaggtc caagtggtat 180

aatgattatg cagtatctgt gaaaagtcgg ttcaccatct ccagagacaa ttccaagaac 240

acgctgtatc tgcaaatgaa cagcctgaga gccgaggaca cggccgtata ttactgtgcg 300

aaagatattg tagtagtacc agctgctata tactggtact tcgatctctg gggccagggc 360

accctggtca ccgtctcctc a 381

<210> 109
<211> 14405
<212> DNA
<213> Artificial Sequence

<220>
<223> Synthetic DNA

<400> 109
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtttccc gaagcgaaag ccgcgtgtta 420
   tttttgggtg ggaaagcccc gattcgctcc ttttttttta tttattattt ttgttgtttt 480
   tcgtgtcttt ttcttctccg ttttgcagct gcggcctcca ggtgtccccg gccgtggtat 540
   ttttgggtcg aaaaagccgc tttttggctc acttggaaac ctttcgggcg tctccaagcg 600
   gagcgttggc gggaaatggt gttttttttg ggttgttttc ggtccctttt ttcaccattt 660
   ttgtggtttt tcggcccatt ttcggcctcc cccccccccc cccccttccc gtggggctgc 720
   gctccgtgga gcggaatggg gtcgttaatt aaggttaatt ggtttaatta atgggccgtg 780
   cagggagaag agggccgtgc ggtggttgtt agccctgatt aatggcttgg ttgaggggag 840
   agtgtaatta agtgcttcgt tagcactaat taatggcttg attgagagga gagtgtaatt 900
   aaatgcttca taatccctaa ttaatggctt ggttgagaca agagtgtaat taagtgcttc 960
   attagccctg attaatggct tggttgaggg gagagtgtaa ttaaatgctt cgttagcact 1020
   aattaatggc ttgattgaga ggagagtgta attaaatgct tcataatccc taattaatgg 1080
   cttggttgag acaagagtgt aattaagtgc ttcattagcc ctgattaatg gcttggttga 1140
   gaggagagtg taattaaatg cttcattatc cctaattagg gactgaattc caaggagagt 1200
   gtaattaagt gcttcattat ccctaattaa tggcttggtt gagaggagag tgtaattaaa 1260
   tgctttgtta gccctaatta atggtttgat ggagaggaga gtgtaattaa gtgcttcatt 1320
   atccctaatt aatggcttgg ttgaggggag agtgtaatta aatgcttagt tagccctaat 1380
   taatggcttg gttgagagga gagtgtaatt gaatgcttca ttatccctaa ttagggattg 1440
   aattgagagg agagtgtaat tgaatgcttt gttagcccta attagggatt gaattgagag 1500
   gagagtgtaa ttaaatgcct cattatccct aattagggat tgaattgaga ggagagttta 1560
   attaaatgcc tcattatccc taattaggga ctgaattccg acgagagtgt aattaagtgc 1620
   ttcatttagc cctaattaat ggcttgattg agaggagagt gtaattaaat gcttcattat 1680
   ccctaattag ggattgaatt gagaggagag tgtaattaaa tgcttcatta tccctaatta 1740
   gggattgaat tgagaggaga gtgtaattaa atgcttcatt atccctaatt aatggcttgg 1800
   ttgagagggg agtgtaatta agtgcttcgt tatccctaat tagggattga attgtgagga 1860
   gatggcaatt aatacttcat tatccctaat taatttaatt agtccctatt atccctaatt 1920
   taattcatgg tttggttgat ggaagaatgt aattaaatgc tttgttatcc ctaattaatg 1980
   gtttgattga gcggagagtg taattaaatg cttcattatc ccttattaat ggttgtgtgg 2040
   aatgtagtgt aattagaagt ccctaattaa tggtttaatt gagagaagac tgcaattaga 2100
   tgactcatag cccctaataa gtggtttaat tatgagataa tcacaattac atgtttcatt 2160
   gtccctaatt aatcgtttaa ttgcgagaag atcacaatta attaaatgat ccctaattgc 2220
   taccgttcgt ataatgtata ctatacgaag ttattttatt tgtgaaattt gtgatgctat 2280
   tgctttattt gtaaccatta taagctgcaa taaacaagtt ggggtgggcg aagaactcca 2340
   gcatgagatc cccgcgctgg aggatcatcc agccggcgtc ccggaaaacg attccgaagc 2400
   ccaacctttc atagaaggcg gcggtggaat cgaaatctcg tgatggcagg ttgggcgtcg 2460
   cttggtcggt catttcgaac cccagagtcc cgctcagaag aactcgtcaa gaaggcgata 2520
   gaaggcgatg cgctgcgaat cgggagcggc gataccgtaa agcacgagga agcggtcagc 2580
   ccattcgccg ccaagctctt cagcaatatc acgggtagcc aacgctatgt cctgatagcg 2640
   gtccgccaca cccagccggc cacagtcgat gaatccagaa aagcggccat tttccaccat 2700
   gatattcggc aagcaggcat cgccatgggt cacgacgaga tcctcgccgt cgggcatgcg 2760
   cgccttgagc ctggcgaaca gttcggctgg cgcgagcccc tgatgctctt cgtccagatc 2820
   atcctgatcg acaagaccgg cttccatccg agtacgtgct cgctcgatgc gatgtttcgc 2880
   ttggtggtcg aatgggcagg tagccggatc aagcgtatgc agccgccgca ttgcatcagc 2940
   catgatggat actttctcgg caggagcaag gtgagatgac aggagatcct gccccggcac 3000
   ttcgcccaat agcagccagt cccttcccgc ttcagtgaca acgtcgagca cagctgcgca 3060
   aggaacgccc gtcgtggcca gccacgatag ccgcgctgcc tcgtcctgca gttcattcag 3120
   ggcaccggac aggtcggtct tgacaaaaag aaccgggcgc ccctgcgctg acagccggaa 3180
   cacggcggca tcagagcagc cgattgtctg ttgtgcccag tcatagccga atagcctctc 3240
   cacccaagcg gccggagaac ctgcgtgcaa tccatcttgt tcaatcatgc gaaacgatcc 3300
   tcatcctgtc tcttgatcag atccgaaaat ggatatacaa gctcccggga gctttttgca 3360
   aaagcctagg cctccaaaaa agcctcctca ctacttctgg aatagctcag aggcagaggc 3420
   ggcctcggcc tctgcataaa taaaaaaaat tagtcagcca tggggcggag aatgggcgga 3480
   actgggcgga gttaggggcg ggatgggcgg agttaggggc gggactatgg ttgctgacta 3540
   attgagatgc atgctttgca tacttctgcc tgctggggag cctggggact ttccacacct 3600
   ggttgctgac taattgagat gcatgctttg catacttctg cctgctgggg agcctgggga 3660
   ctttccacac cctaaataac ttcgtataat gtatactata cgaacggtag cgttaacaag 3720
   cttctgtgga atgtgtgtca gttagggtgt ggaaagtccc caggctcccc aggcaggcag 3780
   aagtatgcaa agcatgcatc tcaattagtc agcaaccagg tgtggaaagt ccccaggctc 3840
   cccagcaggc agaagtatgc aaagcatgca tctcaattag tcagcaacca tagtcccgcc 3900
   cctaactccg cccatcccgc ccctaactcc gcccagttcc gcccattctc cgccccatgg 3960
   ctgactaatt ttttttattt atgcagaggc cgaggccgcc tctgcctctg agctattcca 4020
   gaagtagtga ggaggctttt ttggaggcct aggcttttgc aaaaagctat aacttcgtat 4080
   atggtattat atacgaagtt atcccacccc aacttgttta ttgcagctta taatggttac 4140
   aaataaagca atagcatcac aaatttcaca aataaagcat ttttttcact gcattctagt 4200
   tgtggtttgt ccaaactcat caatgtatct tatcatgtct gtataccgtc gacctctagc 4260
   tagagcttgg cgtaatcatg gtcatagctg tttcctgtgt gcccacccca acttgtttat 4320
   tgcagcttat aatggttaca aataaagcaa tagcatcaca aatttcacaa ataaagcatt 4380
   tttttcactg cattctagtt gtggtttgtc caaactcatc aatgtatctt atcatgtctg 4440
   gagctcgcta gcgtttaaac ggtaccataa cttcgtatat tctatcttat acgaacggta 4500
   ctcgagatgc gtcgacattt aaatgttggc gcagaaggga ttcatatcaa ttaattccct 4560
   gtcctcaatt aatgcataat tgggagtgcg atggaatcat tcaataagta atgaggactt 4620
   ggtgtcaaaa tgccaattaa gtaattaatg acacccaatt aatacacaat ggggattaca 4680
   gtggaataac tcagtgaagt tcatatctaa attgcaattg aataattaat tacagctaat 4740
   taatggatgg gagactggca gtggtataac ttaatgaagg aacacagtga attcctgtct 4800
   gaattgaaat taattgattt attagggcta attaatgaat ggtggggtta aaaatagcat 4860
   tactcagcga aggaacatga gttcatggct aaattgagat tgaattatta attacaacta 4920
   attgattaat gactgatggg agtaagaatg gaataactca gtgaaggaac acaatggatt 4980
   catggcctaa ttgaaattaa atggttaatt agagctaatt aatgaatgat ggggggtggc 5040
   aatggaatga tacaagaaag aaacacaatg ggttcgtgtc tacattgaaa ttaaataatt 5100
   aattacagct aattaataaa cggcgaggtt gacaatggta taactgtgaa gagacagaat 5160
   gagttcgtga ctaaattggt attaaatgat taattagagc taattaacag gtggtggggc 5220
   tgagaatggc ataactcaat gggggatgat gagctgatgg cttaattgga attaataatt 5280
   aatcacaacc aattaacagg tgctgttaat ggggttacag gggcatgact tcaggaagaa 5340
   gcacaaagtg gccattaaat aattaattgc acccaattaa ttcctgatgg ggattactgc 5400
   agaggaactc agcgaaggat tcaagcagaa atgacacagt taggttgctc aattgcgtta 5460
   attaattaaa atcgctaatt aggtgagctt cattaaggcc tgatggggtt catcccaacg 5520
   ctgggatcaa agaactcagc gcagagtgga taacgataat tagaagggga caaattaatg 5580
   aggaaacacg actttgggcc tagacggcca ttaaatcatt aatcaacccg acgggcgccc 5640
   aattaatcat aaagttatta agcaattaag tcgaggctga cgagaaaccg caacgagagc 5700
   cctccacgct cctttgagtt ggcgcaaaca acaaacaacg cgggctcgtt aagcagcgac 5760
   gacgccgagc gtaacgaagc aatccccgcg cccgacaatt aacgcgctaa cgaagccgga 5820
   gccctcctta tgcaaattag cccctccaga gggccataaa agcgccggct ctccgacggc 5880
   gcgggaggag caccagtcgg ctccgcaacc atgagcccac tcgtctcctc cctcctgctc 5940
   ctggccgccc tgccaggtga gggcgctgtg gggctctatg gggctctatg gggtctcagc 6000
   ggggctctgc gggctcaatg ggggccaaag ggggggtctg cgggctctat gggggggtca 6060
   acggggggtc tcacgggggg ccggctccgc gaggccgtgt ggcggcggct ccgtcagcgc 6120
   tctctgggcg cgcctccttc cccacagggc tgatggcgga ggtgcagctg ttggagtctg 6180
   ggggaggctt ggtacagcct ggggggtccc tgagactctc ctgtgcagcc tctggattca 6240
   cctttagcag ctatgccatg agctgggtcc gccaggctcc agggaagggg ctggagtggg 6300
   tctcagctat tagtggtagt ggtggtagca catactacgc agactccgtg aagggccggt 6360
   tcaccatctc cagagacaat tccaagaaca cgctgtatct gcaaatgaac agcctgagag 6420
   ccgaggacac ggccgtatat tactgtgcga aaggatatag tggctacgat tactactttg 6480
   actactgggg ccagggcacc ctggtcaccg tctcctcagg tgagtcttca tatgaacccc 6540
   ccccaaaatt gccgcggcga ttttggggca aaatcggcgg attttatgga tccccgctac 6600
   cgttcgtata gcatacatta tacgaagtta tcagacatga taagatacat tgatgagttt 6660
   ggacaaacca caactagaat gcagtgaaaa aaatgcttta tttgtgaaat ttgtgatgct 6720
   attgctttat ttgtaaccat tataagctgc aataaacaag ttggggtggg cgaagaactc 6780
   cagcatgaga tccccgcgct ggaggatcat ccagccggcg tcccggaaaa cgattccgaa 6840
   gcccaacctt tcatagaagg cggcggtgga atcgaaatct cgtagcacgt gtcagtcctg 6900
   ctcctcggcc acgaagtgct tagccctccc acacataacc agagggcagc aattcacgaa 6960
   tcccaactgc cgtcggctgt ccatcactgt ccttcactat ggctttgatc ccaggatgca 7020
   gatcgagaag cacctgtcgg caccgtccgc aggggctcaa gatgcccctg ttctcatttc 7080
   cgatcgcgac gatacaagtc aggttgccag ctgccgcagc agcagcagtg cccagcacca 7140
   cgagttctgc acaaggtccc ccagtaaaat gatatacatt gacaccagtg aagatgcggc 7200
   cgtcgctaga gagagctgcg ctggcgacgc tgtagtcttc agagatgggg atgctgttga 7260
   ttgtagccgt tgctctttca atgagggtgg attcttcttg agacaaaggc ttggccatgg 7320
   tttagttcct caccttgtcg tattatacta tgccgatata ctatgccgat gattaattgt 7380
   caacacgtgc tgatcagatc cgaaaatgga tatacaagct cccgggagct ttttgcaaaa 7440
   gcctaggcct ccaaaaaagc ctcctcacta cttctggaat agctcagagg cagaggcggc 7500
   ctcggcctct gcataaataa aaaaaattag tcagccatgg ggcggagaat gggcggaact 7560
   gggcggagtt aggggcggga tgggcggagt taggggcggg actatggttg ctgactaatt 7620
   gagatgcatg ctttgcatac ttctgcctgc tggggagcct ggggactttc cacacctggt 7680
   tgctgactaa ttgagatgca tgctttgcat acttctgcct gctggggagc ctggggactt 7740
   tccacaccct aactgacaca cattccacag ataacttcgt atagcataca ttatacgaac 7800
   ggtagcctgg gtcagtcgaa gggggcggtc ggtccatcat ttgcggccgg gtgatttttg 7860
   gggccgaaaa gtgggaattt ggggcccaat ttgggcccaa atttgggccc caatttgggt 7920
   tttcgagggg ggattttttt agggggagat tttgggtccc cggaaaatgg tgattttggg 7980
   tcaaaaatgg ggattttggg gtcgttttga ggtggggttt tttggggtag aaatggcggt 8040
   ctgaggggaa aatgtcgttt tggggccatt ttgggccatt tgaggggaaa tttgggtcaa 8100
   aaaatgacga ttttgggtcg ttttagggag gaaaatgaat ttagggcgat ttgagccaaa 8160
   tttgggtcaa aaatggcgat tttgagtcaa aaaatgatta ttttgggtca ttttagggag 8220
   gaaaatgaat ttagggagat tggacggcaa atttgggtca aaaaatggtg attttgggtc 8280
   aaaaaatgat tattttgggt cattttaggg agaaaaatga atttagggag atttgagggc 8340
   aaatttgggt caaaaaatgg tgattttggg tcaaaaaatg attattttgg gtcattttag 8400
   ggagaaaaat gaatttaggg agatttgagg gcaaatttgg gtcgaaaaat ggcgattttg 8460
   ggtgaaaaat ggacaatttt gggtcatttt agggttaaaa atgaatttag ggagattgga 8520
   cggcaaattt gggtcaaaaa atggtgattt ggggtcaaaa aataattatt ttgggtcatt 8580
   ttagggataa aaaatgaatt tagggagatt tgagggcaaa tttgggtcga aaaatggtga 8640
   tttggggtga aaaatggaca attttgggtc attttaggga taaaaaatga atttagggcg 8700
   atttgagcca aatttgggtc aaaaatggcg attttgagtc aaaaaatgat tattttgggt 8760
   cattttaggg ttaaaaatga atttagggag attggacggc aaatttgggt caaaaaatgg 8820
   tgattttcgg tcaaaaaatg attattttgg gtcattttag ggagaaaaat gaatttaggg 8880
   agattggacg gcaaatttgg gtcgaaaaat ggtgattttg ggtcaaaaat ggacaatttg 8940
   gggtcatttt agggataaaa atgaatttag ggagatttga gagcaaattt gggtctaaaa 9000
   atgtcaattt ggggtcaaaa atggtgattt tgggtcattt tagggatata aatgaatttt 9060
   gggagatttg agggcaaatt tgggtcgaaa aatggtgatt tggggtcaat ttagggagga 9120
   aaatgaattt aaggtgattt gacggcaaat ttgggttgaa aaatgacaat ttggggtcat 9180
   tttaaagata aaatgaattt agggcgattt gagggcaaat ttgggtcgaa aaatggtgat 9240
   ttgggtcaaa aaatgacaat tttgggtcat tttagggata taaatggact tagagcgatt 9300
   tgagggcaaa tttggttgaa aaaatggaca atttgggtca ttttagggat ataaatgaat 9360
   ttagggagat ttgatggcaa atttgggtcg aaaaatggtg atttgggtca aaaatggtga 9420
   ttttggttga aaaacagtca tttggggtca ttttagggat taaaatgaat ttcgggagat 9480
   ttgagggcaa atttgggtca aaaaatggtg attttgggtc aattagtgat aaaaaaatga 9540
   atttagggtg atttgagggc aaatttgggt caaaaaatga caatttgggg tttttttagg 9600
   gaggagaatg aatttaggga gttttgaggg caaatttggg tcaaaaaata gtgatttggg 9660
   tcaaaagtgg tgattttggt tgaaaaacag tcattttggg tcattttagg gagaaaaatg 9720
   aatttcggga gatttgaggg caaatttggg tcaaaaaatg acaattttgg gtcactttac 9780
   gaattaaaat gaatttaggg agatttgagg gcaaatttgg gtcaaaatat ggtgattttg 9840
   ggtcaattta gggaggaaaa tgaatttagg gcgatttgaa ggcaaatttg ggtcgaaaat 9900
   ggtgattttg ggtcaaaaaa tgatgatttt gggtcgtttt agggagaaaa tgaatttagg 9960
   gagatttgag ggcaaatttg ggtcaaaaaa tggtgatttt gggtcattgt agggataaaa 10020
   atgaatttgg ggcgatttga gggcaaattt gggtggaaaa tggacaattt tgggtcattt 10080
   tagggagaaa aatgaattta gggagatttg agggcaaatt tgggtcaaaa aatggtgatt 10140
   tggggtcatt ttcagaagaa aaaatgatta ttttccccac taaaaatgta tattttgggg 10200
   ccaaatggtg aaaaatggtg atttttaatc aaacgtcccc aaaattgggg aaatttcatc 10260
   gatttgaccc aaaattgagt tttttttccc tgttaaaaat gtacattttg gggtcaatcg 10320
   ctgaaatgtt cccacttttc acttctttgc ccccaaattt tgctgtccgg tgagaaatta 10380
   attaattaat gatcattaat tgatcgacaa ttaatggtta ttaattaatt aataggtcct 10440
   cttttggtga ctccttcgct tttgggccca aaagtccata aattggcccc aaaaaattaa 10500
   cgctgagtaa ttggattcca aagtattaat gataaacatt aaaagtgttt aattaataat 10560
   gatattaaac ataatttcgt ttttattatc gatttatcaa caacgatgaa cgataatact 10620
   ttacaacaat cgctaataat taattaatta tttaattaat taatttctaa taattacttc 10680
   gcattatcgg acacgagatg ttgtaattat taataattta attcctaata attagaagat 10740
   tcgttgaaaa tggtacttat ctttacaaat aatcacttct aataataatg attaataata 10800
   gttaataaca acaatgacga taatgacgat aatattaata atatgtgata tatttaatat 10860
   aaaattcgta ttaatatatt atatctacaa aatatgatat aaaatataat atttatttta 10920
   tatataacac aatttattat tattatcatt attatcatta ttaatatcat cattattaac 10980
   gttatcgaaa tacttattta gaaataataa aaacggattt aatgatggca acaaaaatat 11040
   tttattaatg ttaaaaaaaa ataattaata atttccaaag attcgaattc ggggcaacga 11100
   acggcactcg ataattttta attaattaat agtttgaatt aatcggtact ttttaatcct 11160
   ccattttgcc cgaaatcgcc gttttttgcc ccaaattccc caccgcggcg ttaaaaacat 11220
   aaagaaatta agcatcaaaa gtgccctttt ttggggttgt tttgacccaa aaaaaaaatg 11280
   gccgaattgg gggcggccgt tttacggttg ggttcatttt gggttcaaaa cagccaaaaa 11340
   tgggaacttt gggtttcgaa aacaacaaca acaaaaaaac gggtttattt tgggctcatt 11400
   ttgggtgttt ttgggtcagg aggagaaaaa ataggaagtt tgagagcgaa acaacggccg 11460
   cttttggggg gaaaacggcc ctttttggtc aacggcgggg gaaaaaaaaa agcggagttt 11520
   ttggggtgaa aaagagcggt tttgggtaaa tttgggtttt ggggtaaaag tggaggattt 11580
   tgggcaatgg gagttaaaaa atgggtgttt ttatgggggt tcggtgcagt ttttcctgtt 11640
   tgatgggggg tttattaatc cggggggggg gattaatgag aattaataat gttaatagaa 11700
   atatctggga aattaatagc aattattaat tgttaatagt tattaatagt tctatatatc 11760
   tcacatctac gatacaatat aatatcgtta caatcatata gtcgatatat tacatataat 11820
   tatcagtaat aataataagt aacaataatt agcagtaatt aataataata attaatagta 11880
   ttcgttaata agattattga taataattaa gtagtagcga ttaatagaga tgggatttcg 11940
   tgagaaatgg accaaatttg ggccgttttg acccaaattt ttggtggttt tttttccgat 12000
   tctttgtgaa tttcgggtcg gattcatcag caattaatta cggttattag gggctattag 12060
   aggcttttaa ttgggattat tagagacttt taagcggatt tggggacttt taagtggatt 12120
   ttatgattct taagtggatt ttgggtggat tttaccgctt ttgcggccgc aggcgtaatc 12180
   atggtcatag ctgtttcctg tgtgaaattg ttatccgctc acaattccac acaacatacg 12240
   agccggaagc ataaagtgta aagcctgggg tgcctaatga gtgagctaac tcacattaat 12300
   tgcgttgcgc tcactgcccg ctttccagtc gggaaacctg tcgtgccagc tgcattaatg 12360
   aatcggccaa cgcgcgggga gaggcggttt gcgtattggg cgctcttccg cttcctcgct 12420
   cactgactcg ctgcgctcgg tcgttcggct gcggcgagcg gtatcagctc actcaaaggc 12480
   ggtaatacgg ttatccacag aatcagggga taacgcagga aagaacatgt gagcaaaagg 12540
   ccagcaaaag gccaggaacc gtaaaaaggc cgcgttgctg gcgtttttcc ataggctccg 12600
   cccccctgac gagcatcaca aaaatcgacg ctcaagtcag aggtggcgaa acccgacagg 12660
   actataaaga taccaggcgt ttccccctgg aagctccctc gtgcgctctc ctgttccgac 12720
   cctgccgctt accggatacc tgtccgcctt tctcccttcg ggaagcgtgg cgctttctca 12780
   tagctcacgc tgtaggtatc tcagttcggt gtaggtcgtt cgctccaagc tgggctgtgt 12840
   gcacgaaccc cccgttcagc ccgaccgctg cgccttatcc ggtaactatc gtcttgagtc 12900
   caacccggta agacacgact tatcgccact ggcagcagcc actggtaaca ggattagcag 12960
   agcgaggtat gtaggcggtg ctacagagtt cttgaagtgg tggcctaact acggctacac 13020
   tagaagaaca gtatttggta tctgcgctct gctgaagcca gttaccttcg gaaaaagagt 13080
   tggtagctct tgatccggca aacaaaccac cgctggtagc ggtggttttt ttgtttgcaa 13140
   gcagcagatt acgcgcagaa aaaaaggatc tcaagaagat cctttgatct tttctacggg 13200
   gtctgacgct cagtggaacg aaaactcacg ttaagggatt ttggtcatga gattatcaaa 13260
   aaggatcttc acctagatcc ttttaaatta aaaatgaagt tttaaatcaa tctaaagtat 13320
   atatgagtaa acttggtctg acagttacca atgcttaatc agtgaggcac ctatctcagc 13380
   gatctgtcta tttcgttcat ccatagttgc ctgactcccc gtcgtgtaga taactacgat 13440
   acgggagggc ttaccatctg gccccagtgc tgcaatgata ccgcgagacc cacgctcacc 13500
   ggctccagat ttatcagcaa taaaccagcc agccggaagg gccgagcgca gaagtggtcc 13560
   tgcaacttta tccgcctcca tccagtctat taattgttgc cgggaagcta gagtaagtag 13620
   ttcgccagtt aatagtttgc gcaacgttgt tgccattgct acaggcatcg tggtgtcacg 13680
   ctcgtcgttt ggtatggctt cattcagctc cggttcccaa cgatcaaggc gagttacatg 13740
   atcccccatg ttgtgcaaaa aagcggttag ctccttcggt cctccgatcg ttgtcagaag 13800
   taagttggcc gcagtgttat cactcatggt tatggcagca ctgcataatt ctcttactgt 13860
   catgccatcc gtaagatgct tttctgtgac tggtgagtac tcaaccaagt cattctgaga 13920
   atagtgtatg cggcgaccga gttgctcttg cccggcgtca atacgggata ataccgcgcc 13980
   acatagcaga actttaaaag tgctcatcat tggaaaacgt tcttcggggc gaaaactctc 14040
   aaggatctta ccgctgttga gatccagttc gatgtaaccc actcgtgcac ccaactgatc 14100
   ttcagcatct tttactttca ccagcgtttc tgggtgagca aaaacaggaa ggcaaaatgc 14160
   cgcaaaaaag ggaataaggg cgacacggaa atgttgaata ctcatactct tcctttttca 14220
   atattattga agcatttatc agggttattg tctcatgagc ggatacatat ttgaatgtat 14280
   ttagaaaaat aaacaaatag gggttccgcg cacatttccc cgaaaagtgc cacctgacgt 14340
   ctaagaaacc attattatca tgacattaac ctataaaaat aggcgtatca cgaggccctt 14400
   tcgtc 14405

<210>  110
<211>  366
<212>  DNA
<213>  Artificial Sequence

<220>
<223>  Synthetic DNA

<400>  110
gaggtgcagc tgttggagtc tgggggaggc ttggtacagc ctggggggtc cctgagactc      60

tcctgtgcag cctctggatt cacctttagc agctatgcta tgcattgggt ccgccaggct     120

ccagggaagg ggctggagtg ggtctcatgg atcaacgctg gcaatggtaa cacaaaatat     180

tcacagaagt tccagggccg gttcaccatc tccagagaca attccaagaa cacgctgtat     240

ctgcaaatga acagcctgag agccgaggac acggccgtat attactgtgc gaaagcgtct     300

agtagtggtt attactacgt ccactttgac tactggggcc agggcaccct ggtcaccgtc     360

tcctca                                                                366


<210>  111
<211>  360
<212>  DNA
<213>  Artificial Sequence

<220>
<223>  Synthetic DNA

<400>  111
gaggtgcagc tgttggagtc tgggggaggc ttggtacagc ctggggggtc cctgagactc      60

tcctgtgcag cctctggatt cacctttagc agctatggta tcagctgggt ccgccaggct     120

ccagggaagg ggctggagtg ggtctcatgg atcagcgctt acaatggtaa cacaaactat     180

gcacagaagc tccagggccg gttcaccatc tccagagaca attccaagaa cacgctgtat     240

ctgcaaatga acagcctgag agccgaggac acggccgtat attactgtgc gaaagattta     300

aggttatcct attactactt tgactactgg ggccagggca ccctggtcac cgtctcctca     360


<210>  112
<211>  375
<212>  DNA
<213>  Artificial Sequence

<220>
<223>  Synthetic DNA

<400>  112
   gaggtgcagc tgttggagtc tgggggaggc ttggtacagc ctggggggtc cctgagactc      60
   tcctgtgcag cctctggatt cacctttagc ggctttgcca tgaactgggt ccgccaggct     120
   ccagggaagg ggctggagtg ggtctcaact attagtaata gtgctggtag cacatattac     180
   gcagactccg tgaagggccg gttcaccatc tccagagaca attccaagaa cacgctgtat     240
   ctgcaaatga acagcctgag agccgaggac acggccgtat attactgtgc gaaatatggg 300
   gaggatattg tcgttgtgcc agctcgctac ggtgtggacg tctgggccca gggcaccctg 360
   gtcaccgtct cctca 375

<210> 113
<211> 378
<212> DNA
<213> Artificial Sequence

<220>
<223> Synthetic DNA

<400> 113
gaggtgcagc tgttggagtc tgggggaggc ttggtacagc ctggggggtc cctgagactc 60

tcctgtgcag cctctggatt cacctttagc agctctgctg tgcagtgggt ccgccaggct 120

ccagggaagg ggctggagtg ggtctcatgg atcgtcgttg gcagtggtaa cacaaactac 180

gcacagaagt tccaggaacg gttcaccatc tccagagaca attccaagaa cacgctgtat 240

ctgcaaatga acagcctgag agccgaggac acggccgtat attactgtgc gaaagacgtc 300

cttgtagtac cagctgcccc ctactactac tacggtatgg acgtctgggg ccagggcacc 360

ctggtcaccg tctcctca 378

<210> 114
<211> 378
<212> DNA
<213> Artificial Sequence

<220>
<223> Synthetic DNA

<400> 114
gaggtgcagc tgttggagtc tgggggaggc ttggtacagc ctggggggtc cctgagactc 60

tcctgtgcag cctctggatt cacctttagc actagtggag tgggtgtggg ctgggtccgc 120

caggctccag ggaaggggct ggagtgggtc tcactcattt attggaatga tgataagcgc 180

tacagcccat ctctgaagag ccggttcacc atctccagag acaattccaa gaacacgctg 240

tatctgcaaa tgaacagcct gagagccgag gacacggccg tatattactg tgcgaaagac 300

gatggggcgt atgggccgta tagcagcgcc tggacctttg actactgggg ccagggcacc 360

ctggtcaccg tctcctca 378

<210> 115
<211> 369
<212> DNA
<213> Artificial Sequence

<220>
<223> Synthetic DNA

<400>  115
gaggtgcagc tgttggagtc tgggggaggc ttggtacagc ctggggggtc cctgagactc      60

tcctgtgcag cctctggatt cacctttagc actagtggaa tgcgtgtgag ctgggtccgc     120

caggctccag ggaaggggct ggagtgggtc tcacgcattg attgggatga tgataaattc     180

tacagcacat ctctgaagac ccggttcacc atctccagag acaattccaa gaacacgctg     240

tatctgcaaa tgaacagcct gagagccgag gacacggccg tatattactg tgcgaaagat     300

gacggtggca ccccgcccgg ggaattcttt gactactggg gccagggcac cctggtcacc     360

gtctcctca                                                             369


<210>  116
<211>  360
<212>  DNA
<213>  Artificial Sequence

<220>
<223>  Synthetic DNA

<400>  116
gaggtgcagc tgttggagtc tgggggaggc ttggtacagc ctggggggtc cctgagactc      60

tcctgtgcag cctctggatt cacctttagc gattatgcca tgcactgggt ccgccaggct     120

ccagggaagg ggctggagtg ggtctcaggt attagttgga atagtggtag cataggctat     180

gcggactctg tgaagggccg gttcaccatc tccagagaca attccaagaa cacgctgtat     240

ctgcaaatga acagcctgag agccgaggac acggccgtat attactgtgc gaaaatagtg     300

ggagctactt acaactggtt cgacccctgg ggccagggca ccctggtcac cgtctcctca     360


<210>  117
<211>  363
<212>  DNA
<213>  Artificial Sequence

<220>
<223>  Synthetic DNA

<400>  117
gaggtgcagc tgttggagtc tgggggaggc ttggtacagc ctggggggtc cctgagactc      60

tcctgtgcag cctctggatt cacctttagc aacgcctgga tgagctgggt ccgccaggct     120

ccagggaagg ggctggagtg ggtctcacgt attaaaagca aaactgatgg tgggacaaca     180

gactacgctg cacccgtgaa aggccggttc accatctcca gagacaattc caagaacacg     240

ctgtatctgc aaatgaacag cctgagagcc gaggacacgg ccgtatatta ctgtgcgaaa     300

gaaaggggta gcagctcgtc ctttgactac tggggccagg gcaccctggt caccgtctcc     360

tca                                                                   363

<210>  118
<211>  366
<212>  DNA
<213>  Artificial Sequence

<220>
<223>  Synthetic DNA

<400>  118
gaggtgcagc tgttggagtc tgggggaggc ttggtacagc ctggggggtc cctgagactc      60

tcctgtgcag cctctggatt cacctttagc agctatggca tgcactgggt ccgccaggct     120

ccagggaagg ggctggagtg ggtctcagtt atatggtatg atggaagtaa taaatactat     180

gcagactccg tgaagggccg gttcaccatc tccagagaca attccaagaa cacgctgtat     240

ctgcaaatga acagcctgag agccgaggac acggccgtat attactgtgc gaaagatctt     300

gtcttcgggc cagctgctac tccttttgac tactggggcc agggcaccct ggtcaccgtc     360

tcctca                                                                366


<210>  119
<211>  366
<212>  DNA
<213>  Artificial Sequence

<220>
<223>  Synthetic DNA

<400>  119
gaggtgcagc tgttggagtc tgggggaggc ttggtacagc ctggggggtc cctgagactc      60

tcctgtgcag cctctggatt cacctttagc agcaactaca tgagctgggt ccgccaggct     120

ccagggaagg ggctggagtg ggtctcagtt atttatagcg gtggtagcac atactacgca     180

gactccgtga agggccggtt caccatctcc agagacaatt ccaagaacac gctgtatctg     240

caaatgaaca gcctgagagc cgaggacacg gccgtatatt actgtgcgaa agcgaagttc     300

tcctatggtt cagggagtta ttactttgac tactggggcc agggcaccct ggtcaccgtc     360

tcctca                                                                366


<210>  120
<211>  372
<212>  DNA
<213>  Artificial Sequence

<220>
<223>  Synthetic DNA

<400>  120
gaggtgcagc tgttggagtc tgggggaggc ttggtacagc ctggggggtc cctgagactc      60

tcctgtgcag cctctggatt cacctttagc agctactgga tgcactgggt ccgccaggct     120

ccagggaagg ggctggagtg ggtctcacgt attaatagtg atgggagtag cacaagctac     180

gcggactccg tgaagggccg gttcaccatc tccagagaca attccaagaa cacgctgtat 240

ctgcaaatga acagcctgag agccgaggac acggccgtat attactgtgc gaaaggatat 300

tgtagtagta ccagctgcta tgctgatgct tttgatatct ggggccaggg caccctggtc 360

accgtctcct ca 372

<210> 121
<211> 354
<212> DNA
<213> Artificial Sequence

<220>
<223> Synthetic DNA

<400> 121
gaggtgcagc tgttggagtc tgggggaggc ttggtacagc ctggggggtc cctgagactc 60

tcctgtgcag cctctggatt cacctttagc agtagtaact ggtggggctg ggtccgccag 120

gctccaggga aggggctgga gtgggtctca tacatctatt atagtgggag cacctactac 180

aacccgtccc tcaagagtcg gttcaccatc tccagagaca attccaagaa cacgctgtat 240

ctgcaaatga acagcctgag agccgaggac acggccgtat attactgtgc gaaagatcga 300

agtagagggg gttttgacta ctggggccag ggcaccctgg tcaccgtctc ctca 354

<210> 122
<211> 363
<212> DNA
<213> Artificial Sequence

<220>
<223> Synthetic DNA

<400> 122
gaggtgcagc tgttggagtc tgggggaggc ttggtacagc ctggggggtc cctgagactc 60

tcctgtgcag cctctggatt cacctttagc ggttactact ggagctgggt ccgccaggct 120

ccagggaagg ggctggagtg ggtctcagaa atcaatcata gtggaagcac caactacaac 180

ccgtccctca agagtcggtt caccatctcc agagacaatt ccaagaacac gctgtatctg 240

caaatgaaca gcctgagagc cgaggacacg gccgtatatt actgtgcgaa agcccgtacc 300

agcgcgggcc cgggtggtgc ttttgatttc tggggccagg gcaccctggt caccgtctcc 360

tca 363

<210> 123
<211> 384
<212> DNA
<213> Artificial Sequence

<220>
<223> Synthetic DNA

<400>  123
gaggtgcagc tgttggagtc tgggggaggc ttggtacagc ctggggggtc cctgagactc      60

tcctgtgcag cctctggatt cacctttagc agctactgga tcggctgggt ccgccaggct     120

ccagggaagg ggctggagtg ggtctcaatc atctatcctg gtgactctga taccagatac     180

agcccgtcct tccaaggccg gttcaccatc tccagagaca attccaagaa cacgctgtat     240

ctgcaaatga acagcctgag agccgaggac acggccgtat attactgtgc gaaaggggtt     300

tgtagtacca gctcgtacgc cttttactac tactactacg gtatggacgt ctggggccag     360

ggcaccctgg tcaccgtctc ctca                                            384


<210>  124
<211>  363
<212>  DNA
<213>  Artificial Sequence

<220>
<223>  Synthetic DNA

<400>  124
gaggtgcagc tgttggagtc tgggggaggc ttggtacagc ctggggggtc cctgagactc      60

tcctgtgcag cctctggatt cacctttagc agctatgcta tgaattgggt ccgccaggct     120

ccagggaagg ggctggagtg ggtctcatgg atcaacacca acactgggaa cccaacgtat     180

gcccagggct tcacaggacg gttcaccatc tccagagaca attccaagaa cacgctgtat     240

ctgcaaatga acagcctgag agccgaggac acggccgtat attactgtgc gaaagactac     300

ggtggtaact cctactggta cttcgatctc tggggccagg gcaccctggt caccgtctcc     360

tca                                                                   363


<210>  125
<211>  14861
<212>  DNA
<213>  Artificial Sequence

<220>
<223>  Synthetic DNA

<400>  125
ctcgagagct catgcggtac cgctagcgtt taaacctagt cactcctcta cgctcctgta      60

catacgaggt gcagctgttg gagtctgggg gaggcttggt acagcctggg gggtccctga     120

gactctcctg tgcagcctct ggattcacct ttagcggcta ctatatgcac tgggtccgcc     180

aggctccagg gaaggggctg gagtgggtct catggatcaa ccctaacagt ggtggcacaa     240

actatgcaca gaagtttcag ggccggttca ccatctccag agacaattcc aagaacacgc     300

tgtatctgca aatgaacagc ctgagagccg aggacacggc cgtatattac tgtgcgaaag     360

gttggggcta tacggtgact accctcaact ggttcgaccc ctggggccag ggcaccctgg     420

tcaccgtctc ctcagtgcct ggtgactctt ccgcaatcag tcctgggaca gaaacctcag 480

ggacagcctt tatgagtttt gatcacatct aacagagagg atgggccagc gtgtctctac 540

tcgcgtgtct ctctatgcgg aggtgcagct gttggagtct gggggaggct tggtacagcc 600

tggggggtcc ctgagactct cctgtgcagc ctctggattc acctttagca gttatgatat 660

caactgggtc cgccaggctc cagggaaggg gctggagtgg gtctcatgga tgaaccctaa 720

cagtggtaac acaggctatg cacagaagtt ccagggccgg ttcaccatct ccagagacaa 780

ttccaagaac acgctgtatc tgcaaatgaa cagcctgaga gccgaggaca cggccgtata 840

ttactgtgcg aaatccagca gctggcacta ctactacggt atggacgtct ggggccaggg 900

caccctggtc accgtctcct cagtgcctgg tgactcttcc gcaatcagtc ctgggacaga 960

aacctcaggg acagccttta tgagttttga tcacatctaa cagagaggat gggccagata 1020

cgacgtatca cgtactacgt ctagtacgag gtgcagctgt tggagtctgg gggaggcttg 1080

gtacagcctg gggggtccct gagactctcc tgtgcagcct ctggattcac ctttagcgaa 1140

ttatccatgc actgggtccg ccaggctcca gggaaggggc tggagtgggt ctcaggtttt 1200

gatcctgaag atggtgaaac aatctacgca cagaagttcc agggccggtt caccatctcc 1260

agagacaatt ccaagaacac gctgtatctg caaatgaaca gcctgagagc cgaggacacg 1320

gccgtatatt actgtgcgaa aggaacctac tactactact acggtatgga cgtctggggc 1380

cagggcaccc tggtcaccgt ctcctcagtg cctggtgact cttccgcaat cagtcctggg 1440

acagaaacct cagggacagc ctttatgagt tttgatcaca tctaacagag aggatgggcc 1500

agcgtagact agtacgagta tgtactctcg tggaggtgca gctgttggag tctgggggag 1560

gcttggtaca gcctgggggg tccctgagac tctcctgtgc agcctctgga ttcaccttta 1620

gcagctacta tatgcactgg gtccgccagg ctccagggaa ggggctggag tgggtctcaa 1680

taatcaaccc tagtggtggt agcacaagct acgcacagaa gttccagggc cggttcacca 1740

tctccagaga caattccaag aacacgctgt atctgcaaat gaacagcctg agagccgagg 1800

acacggccgt atattactgt gcgaaaagtt tcagcagcag ctggtacaac tggttcgacc 1860

cctggggcca gggcaccctg gtcaccgtct cctcagtgcc tggtgactct tccgcaatca 1920

gtcctgggac agaaacctca gggacagcct ttatgagttt tgatcacatc taacagagag 1980

gatgggccag acgcgagtat actactatgt actgtacagt gaggtgcagc tgttggagtc 2040

tgggggaggc ttggtacagc ctggggggtc cctgagactc tcctgtgcag cctctggatt 2100

cacctttagc agctatgcta tcagctgggt ccgccaggct ccagggaagg ggctggagtg 2160

ggtctcaggg atcatcccta tctttggtac agcaaactac gcacagaagt tccagggccg 2220

gttcaccatc tccagagaca attccaagaa cacgctgtat ctgcaaatga acagcctgag 2280

agccgaggac acggccgtat attactgtgc gaaagggtcc gtcctcggtg atgcttttga 2340

tatctggggc cagggcaccc tggtcaccgt ctcctcagtg cctggtgact cttccgcaat 2400

cagtcctggg acagaaacct cagggacagc ctttatgagt tttgatcaca tctaacagag 2460

aggatgggcc agatagagta ctcacgctac gtcagtagac gtgaggtgca gctgttggag 2520

tctgggggag gcttggtaca gcctgggggg tccctgagac tctcctgtgc agcctctgga 2580

ttcaccttta gcaatgctag aatgggtgtg agctgggtcc gccaggctcc agggaagggg 2640

ctggagtggg tctcacacat tttttcgaat gacgaaaaat cctacagcac atctctgaag 2700

agccggttca ccatctccag agacaattcc aagaacacgc tgtatctgca aatgaacagc 2760

ctgagagccg aggacacggc cgtatattac tgtgcgaaaa tcccgtatga agcagtggct 2820

ggctactttg actactgggg ccagggcacc ctggtcaccg tctcctcagt gcctggtgac 2880

tcttccgcaa tcagtcctgg gacagaaacc tcagggacag cctttatgag ttttgatcac 2940

atctaacaga gaggatgggc cagtacgctg tcttagtgta gattcgatca cgtgaggtgc 3000

agctgttgga gtctggggga ggcttggtac agcctggggg gtccctgaga ctctcctgtg 3060

cagcctctgg attcaccttt agcagctatt ggatgagctg ggtccgccag gctccaggga 3120

aggggctgga gtgggtctca aacataaagc aagatggaag tgagaaatac tatgtggact 3180

ctgtgaaggg ccggttcacc atctccagag acaattccaa gaacacgctg tatctgcaaa 3240

tgaacagcct gagagccgag gacacggccg tatattactg tgcgaaagtt acggtgaccg 3300

ggacctggta cttcgatctc tggggccagg gcaccctggt caccgtctcc tcagtgcctg 3360

gtgactcttc cgcaatcagt cctgggacag aaacctcagg gacagccttt atgagttttg 3420

atcacatcta acagagagga tgggccagtg acgtatgttg tgagtagtac agtatataga 3480

ggtgcagctg ttggagtctg ggggaggctt ggtacagcct ggggggtccc tgagactctc 3540

ctgtgcagcc tctggattca cctttagcag ctacgacatg cactgggtcc gccaggctcc 3600

agggaagggg ctggagtggg tctcagctat tggtactgct ggtgacacat actatccagg 3660

ctccgtgaag ggccggttca ccatctccag agacaattcc aagaacacgc tgtatctgca 3720

aatgaacagc ctgagagccg aggacacggc cgtatattac tgtgcgaaag cggatacagc 3780

tatggtcatt tactactact actacggtat ggacgtctgg ggccagggca ccctggtcac 3840

cgtctcctca gtgcctggtg actcttccgc aatcagtcct gggacagaaa cctcagggac 3900

agcctttatg agttttgatc acatctaaca gagaggatgg gccagagtat acataagtcg 3960

agagaagtgc tacgagaggt gcagctgttg gagtctgggg gaggcttggt acagcctggg 4020

gggtccctga gactctcctg tgcagcctct ggattcacct ttagcagcta tagcatgaac 4080

tgggtccgcc aggctccagg gaaggggctg gagtgggtct catccattag tagtagtagt 4140

agttacatat actacgcaga ctcagtgaag ggccggttca ccatctccag agacaattcc 4200

aagaacacgc tgtatctgca aatgaacagc ctgagagccg aggacacggc cgtatattac 4260

tgtgcgaaag agaatagtgg ctatgattac tttgactact ggggccaggg caccctggtc 4320

accgtctcct cagtgcctgg tgactcttcc gcaatcagtc ctgggacaga aacctcaggg 4380

acagccttta tgagttttga tcacatctaa cagagaggat gggccagcgt acagtcacgt 4440

actcagacta cgctctagag gtgcagctgt tggagtctgg gggaggcttg gtacagcctg 4500

gggggtccct gagactctcc tgtgcagcct ctggattcac ctttagcgat tatgctatga 4560

gctgggtccg ccaggctcca gggaaggggc tggagtgggt ctcattcatt agaagcaaag 4620

cttatggtgg gacaacagaa tacaccgcgt ctgtgaaagg ccggttcacc atctccagag 4680

acaattccaa gaacacgctg tatctgcaaa tgaacagcct gagagccgag gacacggccg 4740

tatattactg tgcgaaagat ctaggtagtg gctttgacta ctggggccag ggcaccctgg 4800

tcaccgtctc ctcagtgcct ggtgactctt ccgcaatcag tcctgggaca gaaacctcag 4860

ggacagcctt tatgagtttt gatcacatct aacagagagg atgggccagt atatatacat 4920

atgctagtat cacgcgagag aggtgcagct gttggagtct gggggaggct tggtacagcc 4980

tggggggtcc ctgagactct cctgtgcagc ctctggattc acctttagcg accactacat 5040

ggactgggtc cgccaggctc cagggaaggg gctggagtgg gtctcacgta ctagaaacaa 5100

agctaacagt tacaccacag aatacgccgc gtctgtgaaa ggccggttca ccatctccag 5160

agacaattcc aagaacacgc tgtatctgca aatgaacagc ctgagagccg aggacacggc 5220

cgtatattac tgtgcgaaag gaggccaggg gagccgatac tactactact acggtatgga 5280

cgtctggggc cagggcaccc tggtcaccgt ctcctcagtg cctggtgact cttccgcaat 5340

cagtcctggg acagaaacct cagggacagc ctttatgagt tttgatcaca tctaacagag 5400

aggatgggcc agtgagtcag tatgtagtgt gatgtcacac gagaggtgca gctgttggag 5460

tctgggggag gcttggtaca gcctgggggg tccctgagac tctcctgtgc agcctctgga 5520

ttcaccttta gcagcaatga gatgagctgg gtccgccagg ctccagggaa ggggctggag 5580

tgggtctcat ccattagtgg tggtagcaca tactacgcag actccaggaa gggccggttc 5640

accatctcca gagacaattc caagaacacg ctgtatctgc aaatgaacag cctgagagcc 5700

gaggacacgg ccgtatatta ctgtgcgaaa gactcatatg atgcttttga tatctggggc 5760

cagggcaccc tggtcaccgt ctcctcagtg cctggtgact cttccgcaat cagtcctggg 5820

acagaaacct cagggacagc ctttatgagt tttgatcaca tctaacagag aggatgggcc 5880

agacatgacg acacgacagc tcacgtctca tcgaggtgca gctgttggag tctgggggag 5940

gcttggtaca gcctgggggg tccctgagac tctcctgtgc agcctctgga ttcaccttta 6000

gcagtggtgg ttactcctgg agctgggtcc gccaggctcc agggaagggg ctggagtggg 6060

tctcatacat ctatcatagt gggagcacct actacaaccc gtccctcaag agtcggttca 6120

ccatctccag agacaattcc aagaacacgc tgtatctgca aatgaacagc ctgagagccg 6180

aggacacggc cgtatattac tgtgcgaaag atgcgagtaa ccatttcttt gactactggg 6240

gccagggcac cctggtcacc gtctcctcag tgcctggtga ctcttccgca atcagtcctg 6300

ggacagaaac ctcagggaca gcctttatga gttttgatca catctaacag agaggatggg 6360

ccagagcgac tagcagtagt gatcagtgac acacgaggtg cagctgttgg agtctggggg 6420

aggcttggta cagcctgggg ggtccctgag actctcctgt gcagcctctg gattcacctt 6480

tagcagtagt agttactact ggggctgggt ccgccaggct ccagggaagg ggctggagtg 6540

ggtctcaagt atctattata gtgggagcac ctactacaac ccgtccctca agagtcggtt 6600

caccatctcc agagacaatt ccaagaacac gctgtatctg caaatgaaca gcctgagagc 6660

cgaggacacg gccgtatatt actgtgcgaa aaaggactgg aacgacaact ggttcgaccc 6720

ctggggccag ggcaccctgg tcaccgtctc ctcagtgcct ggtgactctt ccgcaatcag 6780

tcctgggaca gaaacctcag ggacagcctt tatgagtttt gatcacatct aacagagagg 6840

atgggccaga tctactgacc acgtagatcc acgtgtcgcg aggtgcagct gttggagtct 6900

gggggaggct tggtacagcc tggggggtcc ctgagactct cctgtgcagc ctctggattc 6960

acctttagca gcaacagtgc tgcttggaac tgggtccgcc aggctccagg gaaggggctg 7020

gagtgggtct caaggacata ctacaggtcc aagtggtata atgattatgc agtatctgtg 7080

aaaagtcggt tcaccatctc cagagacaat tccaagaaca cgctgtatct gcaaatgaac 7140

agcctgagag ccgaggacac ggccgtatat tactgtgcga aagatattgt agtagtacca 7200

gctgctatat actggtactt cgatctctgg ggccagggca ccctggtcac cgtctcctca 7260

gtgcctggtg actcttccgc aatcagtcct gggacagaaa cctcagggac agcctttatg 7320

agttttgatc acatctaaca gagaggatgg gccaggttta aacggtacct ggccaggatc 7380

cgctagcgag ctcgtttaaa cagcactgta gatcagacac gatatcgcga ggaggtgcag 7440

ctgttggagt ctgggggagg cttggtacag cctggggggt ccctgagact ctcctgtgca 7500

gcctctggat tcacctttag cagctatgct atgcattggg tccgccaggc tccagggaag 7560

gggctggagt gggtctcatg gatcaacgct ggcaatggta acacaaaata ttcacagaag 7620

ttccagggcc ggttcaccat ctccagagac aattccaaga acacgctgta tctgcaaatg 7680

aacagcctga gagccgagga cacggccgta tattactgtg cgaaagcgtc tagtagtggt 7740

tattactacg tccactttga ctactggggc cagggcaccc tggtcaccgt ctcctcagtg 7800

cctggtgact cttccgcaat cagtcctggg acagaaacct cagggacagc ctttatgagt 7860

tttgatcaca tctaacagag aggatgggcc agtgatacgt ctcatagtag tgcgagagat 7920

acgaggtgca gctgttggag tctgggggag gcttggtaca gcctgggggg tccctgagac 7980

tctcctgtgc agcctctgga ttcaccttta gcagctatgg tatcagctgg gtccgccagg 8040
ctccagggaa ggggctggag tgggtctcat ggatcagcgc ttacaatggt aacacaaact 8100
atgcacagaa gctccagggc cggttcacca tctccagaga caattccaag aacacgctgt 8160
atctgcaaat gaacagcctg agagccgagg acacggccgt atattactgt gcgaaagatt 8220
taaggttatc ctattactac tttgactact ggggccaggg caccctggtc accgtctcct 8280
cagtgcctgg tgactcttcc gcaatcagtc ctgggacaga aacctcaggg acagccttta 8340
tgagttttga tcacatctaa cagagaggat gggccagtct acgtagctgt actactcacg 8400
actacaggag gtgcagctgt tggagtctgg gggaggcttg gtacagcctg gggggtccct 8460
gagactctcc tgtgcagcct ctggattcac ctttagcggc tttgccatga actgggtccg 8520
ccaggctcca gggaaggggc tggagtgggt ctcaactatt agtaatagtg ctggtagcac 8580
atattacgca gactccgtga agggccggtt caccatctcc agagacaatt ccaagaacac 8640
gctgtatctg caaatgaaca gcctgagagc cgaggacacg gccgtatatt actgtgcgaa 8700
atatggggag gatattgtcg ttgtgccagc tcgctacggt gtggacgtct ggggccaggg 8760
caccctggtc accgtctcct cagtgcctgg tgactcttcc gcaatcagtc ctgggacaga 8820
aacctcaggg acagccttta tgagttttga tcacatctaa cagagaggat gggccagtag 8880
agacgagtcg tcgctcgaca tacgcgtgag gtgcagctgt tggagtctgg gggaggcttg 8940
gtacagcctg gggggtccct gagactctcc tgtgcagcct ctggattcac ctttagcagc 9000
tctgctgtgc agtgggtccg ccaggctcca gggaaggggc tggagtgggt ctcatggatc 9060
gtcgttggca gtggtaacac aaactacgca cagaagttcc aggaacggtt caccatctcc 9120
agagacaatt ccaagaacac gctgtatctg caaatgaaca gcctgagagc cgaggacacg 9180
gccgtatatt actgtgcgaa agacgtcctt gtagtaccag ctgcccccta ctactactac 9240
ggtatggacg tctggggcca gggcaccctg gtcaccgtct cctcagtgcc tggtgactct 9300
tccgcaatca gtcctgggac agaaacctca gggacagcct ttatgagttt tgatcacatc 9360
taacagagag gatgggccag agactatact agcgtcgtct agtacgctat gaggtgcagc 9420
tgttggagtc tgggggaggc ttggtacagc ctggggggtc cctgagactc tcctgtgcag 9480
cctctggatt cacctttagc actagtggag tgggtgtggg ctgggtccgc caggctccag 9540
ggaaggggct ggagtgggtc tcactcattt attggaatga tgataagcgc tacagcccat 9600
ctctgaagag ccggttcacc atctccagag acaattccaa gaacacgctg tatctgcaaa 9660
tgaacagcct gagagccgag gacacggccg tatattactg tgcgaaagac gatggggcgt 9720
atgggccgta tagcagcgcc tggacctttg actactgggg ccagggcacc ctggtcaccg 9780
tctcctcagt gcctggtgac tcttccgcaa tcagtcctgg gacagaaacc tcagggacag 9840
cctttatgag ttttgatcac atctaacaga gaggatgggc cagcgacgtg acttacacac 9900

acttacagat cgtgaggtgc agctgttgga gtctggggga ggcttggtac agcctggggg 9960

gtccctgaga ctctcctgtg cagcctctgg attcaccttt agcactagtg gaatgcgtgt 10020

gagctgggtc cgccaggctc cagggaaggg gctggagtgg gtctcacgca ttgattggga 10080

tgatgataaa ttctacagca catctctgaa gacccggttc accatctcca gagacaattc 10140

caagaacacg ctgtatctgc aaatgaacag cctgagagcc gaggacacgg ccgtatatta 10200

ctgtgcgaaa gatgacggtg gcaccccgcc cggggaattc tttgactact ggggccaggg 10260

caccctggtc accgtctcct cagtgcctgg tgactcttcc gcaatcagtc ctgggacaga 10320

aacctcaggg acagccttta tgagttttga tcacatctaa cagagaggat gggccagtcg 10380

cactagttct agcgacttct atactatgag gtgcagctgt tggagtctgg gggaggcttg 10440

gtacagcctg gggggtccct gagactctcc tgtgcagcct ctggattcac ctttagcgat 10500

tatgccatgc actgggtccg ccaggctcca gggaaggggc tggagtgggt ctcaggtatt 10560

agttggaata gtggtagcat aggctatgcg gactctgtga agggccggtt caccatctcc 10620

agagacaatt ccaagaacac gctgtatctg caaatgaaca gcctgagagc cgaggacacg 10680

gccgtatatt actgtgcgaa aatagtggga gctacttaca actggttcga cccctggggc 10740

cagggcaccc tggtcaccgt ctcctcagtg cctggtgact cttccgcaat cagtcctggg 10800

acagaaacct cagggacagc ctttatgagt tttgatcaca tctaacagag aggatgggcc 10860

agacgcgatc gaactagcag taagctcacg tagaggtgca gctgttggag tctgggggag 10920

gcttggtaca gcctgggggg tccctgagac tctcctgtgc agcctctgga ttcaccttta 10980

gcaacgcctg gatgagctgg gtccgccagg ctccagggaa ggggctggag tgggtctcac 11040

gtattaaaag caaaactgat ggtgggacaa cagactacgc tgcacccgtg aaaggccggt 11100

tcaccatctc cagagacaat tccaagaaca cgctgtatct gcaaatgaac agcctgagag 11160

ccgaggacac ggccgtatat tactgtgcga aagaaagggg tagcagctcg tcctttgact 11220

actggggcca gggcaccctg gtcaccgtct cctcagtgcc tggtgactct tccgcaatca 11280

gtcctgggac agaaacctca gggacagcct ttatgagttt tgatcacatc taacagagag 11340

gatgggccag cgatcgtata cgcagtacga cgcgtataca gaggtgcagc tgttggagtc 11400

tgggggaggc ttggtacagc ctggggggtc cctgagactc tcctgtgcag cctctggatt 11460

cacctttagc agctatggca tgcactgggt ccgccaggct ccagggaagg ggctggagtg 11520

ggtctcagtt atatggtatg atggaagtaa taaatactat gcagactccg tgaagggccg 11580

gttcaccatc tccagagaca attccaagaa cacgctgtat ctgcaaatga acagcctgag 11640

agccgaggac acggccgtat attactgtgc gaaagatctt gtcttcgggc cagctgctac 11700

tccttttgac tactggggcc agggcaccct ggtcaccgtc tcctcagtgc ctggtgactc 11760

ttccgcaatc agtcctggga cagaaacctc agggacagcc tttatgagtt ttgatcacat 11820

ctaacagaga ggatgggcca gctatagcgt atacgtcatc atagtcgcat agaggtgcag 11880

ctgttggagt ctgggggagg cttggtacag cctggggggt ccctgagact ctcctgtgca 11940

gcctctggat tcacctttag cagcaactac atgagctggg tccgccaggc tccagggaag 12000

gggctggagt gggtctcagt tatttatagc ggtggtagca catactacgc agactccgtg 12060

aagggccggt tcaccatctc cagagacaat tccaagaaca cgctgtatct gcaaatgaac 12120

agcctgagag ccgaggacac ggccgtatat tactgtgcga aagcgaagtt ctcctatggt 12180

tcagggagtt attactttga ctactggggc cagggcaccc tggtcaccgt ctcctcagtg 12240

cctggtgact cttccgcaat cagtcctggg acagaaacct cagggacagc ctttatgagt 12300

tttgatcaca tctaacagag aggatgggcc agtcgatagt gatcgctgcg tatctgacgt 12360

cagaggtgca gctgttggag tctgggggag gcttggtaca gcctgggggg tccctgagac 12420

tctcctgtgc agcctctgga ttcaccttta gcagctactg gatgcactgg gtccgccagg 12480

ctccagggaa ggggctggag tgggtctcac gtattaatag tgatgggagt agcacaagct 12540

acgcggactc cgtgaagggc cggttcacca tctccagaga caattccaag aacacgctgt 12600

atctgcaaat gaacagcctg agagccgagg acacggccgt atattactgt gcgaaaggat 12660

attgtagtag taccagctgc tatgctgatg cttttgatat ctggggccag ggcaccctgg 12720

tcaccgtctc ctcagtgcct ggtgactctt ccgcaatcag tcctgggaca gaaacctcag 12780

ggacagcctt tatgagtttt gatcacatct aacagagagg atgggccagt gtcgtcgcaa 12840

cacatacgca cagtcgtgcg aggtgcagct gttggagtct gggggaggct tggtacagcc 12900

tggggggtcc ctgagactct cctgtgcagc ctctggattc acctttagca gtagtaactg 12960

gtggggctgg gtccgccagg ctccagggaa ggggctggag tgggtctcat acatctatta 13020

tagtgggagc acctactaca acccgtccct caagagtcgg ttcaccatct ccagagacaa 13080

ttccaagaac acgctgtatc tgcaaatgaa cagcctgaga gccgaggaca cggccgtata 13140

ttactgtgcg aaagatcgaa gtagaggggg ttttgactac tggggccagg gcaccctggt 13200

caccgtctcc tcagtgcctg gtgactcttc cgcaatcagt cctgggacag aaacctcagg 13260

gacagccttt atgagttttg atcacatcta acagagagga tgggccagac tcatctacac 13320

tcgcgcacag agcgtcacga ggtgcagctg ttggagtctg ggggaggctt ggtacagcct 13380

ggggggtccc tgagactctc ctgtgcagcc tctggattca cctttagcgg ttactactgg 13440

agctgggtcc gccaggctcc agggaagggg ctggagtggg tctcagaaat caatcatagt 13500

ggaagcacca actacaaccc gtccctcaag agtcggttca ccatctccag agacaattcc 13560

aagaacacgc tgtatctgca aatgaacagc ctgagagccg aggacacggc cgtatattac 13620

tgtgcgaaag cccgtaccag cgcgggcccg ggtggtgctt ttgatttctg gggccagggc 13680

accctggtca ccgtctcctc agtgcctggt gactcttccg caatcagtcc tgggacagaa 13740

acctcaggga cagcctttat gagttttgat cacatctaac agagaggatg ggccagagtg 13800

tatgtcatag atagacatat agtcgcgagg tgcagctgtt ggagtctggg ggaggcttgg 13860

tacagcctgg ggggtccctg agactctcct gtgcagcctc tggattcacc tttagcagct 13920

actggatcgg ctgggtccgc caggctccag ggaaggggct ggagtgggtc tcaatcatct 13980

atcctggtga ctctgatacc agatacagcc cgtccttcca aggccggttc accatctcca 14040

gagacaattc caagaacacg ctgtatctgc aaatgaacag cctgagagcc gaggacacgg 14100

ccgtatatta ctgtgcgaaa ggggtttgta gtaccagctc gtacgccttt tactactact 14160

actacggtat ggacgtctgg ggccagggca ccctggtcac cgtctcctca gtgcctggtg 14220

actcttccgc aatcagtcct gggacagaaa cctcagggac agcctttatg agttttgatc 14280

acatctaaca gagaggatgg gccagcatac tctaccgaca ctatccgaga cgcgcgaggt 14340

gcagctgttg gagtctgggg gaggcttggt acagcctggg gggtccctga gactctcctg 14400

tgcagcctct ggattcacct ttagcagcta tgctatgaat tgggtccgcc aggctccagg 14460

gaaggggctg gagtgggtct catggatcaa caccaacact gggaacccaa cgtatgccca 14520

gggcttcaca ggacggttca ccatctccag agacaattcc aagaacacgc tgtatctgca 14580

aatgaacagc ctgagagccg aggacacggc cgtatattac tgtgcgaaag actacggtgg 14640

taactcctac tggtacttcg atctctgggg ccagggcacc ctggtcaccg tctcctcagt 14700

gcctggtgac tcttccgcaa tcagtcctgg gacagaaacc tcagggacag cctttatgag 14760

ttttgatcac atctaacaga gaggatgggc caggtttaaa cggtacctgg ccaccatggc 14820

cgcggatgcg gcgcgccatc gatatgcgtc gacatttaaa t 14861

<210> 126
<211> 22891
<212> DNA
<213> Artificial Sequence

<220>
<223> Synthetic DNA

<400> 126
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtttccc gaagcgaaag ccgcgtgtta 420

tttttgggtg ggaaagcccc gattcgctcc ttttttttta tttattattt ttgttgtttt 480

tcgtgtcttt ttcttctccg ttttgcagct gcggcctcca ggtgtccccg gccgtggtat 540

ttttgggtcg aaaaagccgc tttttggctc acttggaaac ctttcgggcg tctccaagcg 600

gagcgttggc gggaaatggt gttttttttg ggttgttttc ggtccctttt ttcaccattt 660

ttgtggtttt tcggcccatt ttcggcctcc cccccccccc cccccttccc gtggggctgc 720

gctccgtgga gcggaatggg gtcgttaatt aaggttaatt ggtttaatta atgggccgtg 780

cagggagaag agggccgtgc ggtggttgtt agccctgatt aatggcttgg ttgaggggag 840

agtgtaatta agtgcttcgt tagcactaat taatggcttg attgagagga gagtgtaatt 900

aaatgcttca taatccctaa ttaatggctt ggttgagaca agagtgtaat taagtgcttc 960

attagccctg attaatggct tggttgaggg gagagtgtaa ttaaatgctt cgttagcact 1020

aattaatggc ttgattgaga ggagagtgta attaaatgct tcataatccc taattaatgg 1080

cttggttgag acaagagtgt aattaagtgc ttcattagcc ctgattaatg gcttggttga 1140

gaggagagtg taattaaatg cttcattatc cctaattagg gactgaattc caaggagagt 1200

gtaattaagt gcttcattat ccctaattaa tggcttggtt gagaggagag tgtaattaaa 1260

tgctttgtta gccctaatta atggtttgat ggagaggaga gtgtaattaa gtgcttcatt 1320

atccctaatt aatggcttgg ttgaggggag agtgtaatta aatgcttagt tagccctaat 1380

taatggcttg gttgagagga gagtgtaatt gaatgcttca ttatccctaa ttagggattg 1440

aattgagagg agagtgtaat tgaatgcttt gttagcccta attagggatt gaattgagag 1500

gagagtgtaa ttaaatgcct cattatccct aattagggat tgaattgaga ggagagttta 1560

attaaatgcc tcattatccc taattaggga ctgaattccg acgagagtgt aattaagtgc 1620

ttcatttagc cctaattaat ggcttgattg agaggagagt gtaattaaat gcttcattat 1680

ccctaattag ggattgaatt gagaggagag tgtaattaaa tgcttcatta tccctaatta 1740

gggattgaat tgagaggaga gtgtaattaa atgcttcatt atccctaatt aatggcttgg 1800

ttgagagggg agtgtaatta agtgcttcgt tatccctaat tagggattga attgtgagga 1860

gatggcaatt aatacttcat tatccctaat taatttaatt agtccctatt atccctaatt 1920

taattcatgg tttggttgat ggaagaatgt aattaaatgc tttgttatcc ctaattaatg 1980

gtttgattga gcggagagtg taattaaatg cttcattatc ccttattaat ggttgtgtgg 2040

aatgtagtgt aattagaagt ccctaattaa tggtttaatt gagagaagac tgcaattaga 2100

tgactcatag cccctaataa gtggtttaat tatgagataa tcacaattac atgtttcatt 2160

gtccctaatt aatcgtttaa ttgcgagaag atcacaatta attaaatgat ccctaattgc 2220

taccgttcgt ataatgtata ctatacgaag ttattttatt tgtgaaattt gtgatgctat 2280

tgctttattt gtaaccatta taagctgcaa taaacaagtt ggggtgggcg aagaactcca 2340

gcatgagatc cccgcgctgg aggatcatcc agccggcgtc ccggaaaacg attccgaagc 2400

ccaacctttc atagaaggcg gcggtggaat cgaaatctcg tgatggcagg ttgggcgtcg 2460

cttggtcggt catttcgaac cccagagtcc cgctcagaag aactcgtcaa gaaggcgata 2520

gaaggcgatg cgctgcgaat cgggagcggc gataccgtaa agcacgagga agcggtcagc 2580

ccattcgccg ccaagctctt cagcaatatc acgggtagcc aacgctatgt cctgatagcg 2640

gtccgccaca cccagccggc cacagtcgat gaatccagaa aagcggccat tttccaccat 2700

gatattcggc aagcaggcat cgccatgggt cacgacgaga tcctcgccgt cgggcatgcg 2760

cgccttgagc ctggcgaaca gttcggctgg cgcgagcccc tgatgctctt cgtccagatc 2820

atcctgatcg acaagaccgg cttccatccg agtacgtgct cgctcgatgc gatgtttcgc 2880

ttggtggtcg aatgggcagg tagccggatc aagcgtatgc agccgccgca ttgcatcagc 2940

catgatggat actttctcgg caggagcaag gtgagatgac aggagatcct gccccggcac 3000

ttcgcccaat agcagccagt cccttcccgc ttcagtgaca acgtcgagca cagctgcgca 3060

aggaacgccc gtcgtggcca gccacgatag ccgcgctgcc tcgtcctgca gttcattcag 3120

ggcaccggac aggtcggtct tgacaaaaag aaccgggcgc ccctgcgctg acagccggaa 3180

cacggcggca tcagagcagc cgattgtctg ttgtgcccag tcatagccga atagcctctc 3240

cacccaagcg gccggagaac ctgcgtgcaa tccatcttgt tcaatcatgc gaaacgatcc 3300

tcatcctgtc tcttgatcag atccgaaaat ggatatacaa gctcccggga gctttttgca 3360

aaagcctagg cctccaaaaa agcctcctca ctacttctgg aatagctcag aggcagaggc 3420

ggcctcggcc tctgcataaa taaaaaaaat tagtcagcca tggggcggag aatgggcgga 3480

actgggcgga gttaggggcg ggatgggcgg agttaggggc gggactatgg ttgctgacta 3540

attgagatgc atgctttgca tacttctgcc tgctggggag cctggggact ttccacacct 3600

ggttgctgac taattgagat gcatgctttg catacttctg cctgctgggg agcctgggga 3660

ctttccacac cctaaataac ttcgtataat gtatactata cgaacggtag cgttaactcg 3720

agagctcatg cggtaccgct agcgtttaaa cctagtcact cctctacgct cctgtacata 3780

cgaggtgcag ctgttggagt ctgggggagg cttggtacag cctggggggt ccctgagact 3840

ctcctgtgca gcctctggat tcacctttag cggctactat atgcactggg tccgccaggc 3900

tccagggaag gggctggagt gggtctcatg gatcaaccct aacagtggtg gcacaaacta 3960

tgcacagaag tttcagggcc ggttcaccat ctccagagac aattccaaga acacgctgta 4020

tctgcaaatg aacagcctga gagccgagga cacggccgta tattactgtg cgaaaggttg 4080

gggctatacg gtgactaccc tcaactggtt cgacccctgg ggccagggca ccctggtcac 4140

cgtctcctca gtgcctggtg actcttccgc aatcagtcct gggacagaaa cctcagggac 4200
agcctttatg agttttgatc acatctaaca gagaggatgg gccagcgtgt ctctactcgc 4260
gtgtctctct atgcggaggt gcagctgttg gagtctgggg gaggcttggt acagcctggg 4320
gggtccctga gactctcctg tgcagcctct ggattcacct ttagcagtta tgatatcaac 4380
tgggtccgcc aggctccagg gaaggggctg gagtgggtct catggatgaa ccctaacagt 4440
ggtaacacag gctatgcaca gaagttccag ggccggttca ccatctccag agacaattcc 4500
aagaacacgc tgtatctgca aatgaacagc ctgagagccg aggacacggc cgtatattac 4560
tgtgcgaaat ccagcagctg gcactactac tacggtatgg acgtctgggg ccagggcacc 4620
ctggtcaccg tctcctcagt gcctggtgac tcttccgcaa tcagtcctgg gacagaaacc 4680
tcagggacag cctttatgag ttttgatcac atctaacaga gaggatgggc cagatacgac 4740
gtatcacgta ctacgtctag tacgaggtgc agctgttgga gtctggggga ggcttggtac 4800
agcctggggg gtccctgaga ctctcctgtg cagcctctgg attcaccttt agcgaattat 4860
ccatgcactg ggtccgccag gctccaggga aggggctgga gtgggtctca ggttttgatc 4920
ctgaagatgg tgaaacaatc tacgcacaga agttccaggg ccggttcacc atctccagag 4980
acaattccaa gaacacgctg tatctgcaaa tgaacagcct gagagccgag gacacggccg 5040
tatattactg tgcgaaagga acctactact actactacgg tatggacgtc tggggccagg 5100
gcaccctggt caccgtctcc tcagtgcctg gtgactcttc cgcaatcagt cctgggacag 5160
aaacctcagg gacagccttt atgagttttg atcacatcta acagagagga tgggccagcg 5220
tagactagta cgagtatgta ctctcgtgga ggtgcagctg ttggagtctg ggggaggctt 5280
ggtacagcct ggggggtccc tgagactctc ctgtgcagcc tctggattca cctttagcag 5340
ctactatatg cactgggtcc gccaggctcc agggaagggg ctggagtggg tctcaataat 5400
caaccctagt ggtggtagca caagctacgc acagaagttc cagggccggt tcaccatctc 5460
cagagacaat tccaagaaca cgctgtatct gcaaatgaac agcctgagag ccgaggacac 5520
ggccgtatat tactgtgcga aaagtttcag cagcagctgg tacaactggt tcgacccctg 5580
gggccagggc accctggtca ccgtctcctc agtgcctggt gactcttccg caatcagtcc 5640
tgggacagaa acctcaggga cagcctttat gagttttgat cacatctaac agagaggatg 5700
ggccagacgc gagtatacta ctatgtactg tacagtgagg tgcagctgtt ggagtctggg 5760
ggaggcttgg tacagcctgg ggggtccctg agactctcct gtgcagcctc tggattcacc 5820
tttagcagct atgctatcag ctgggtccgc caggctccag ggaaggggct ggagtgggtc 5880
tcagggatca tccctatctt tggtacagca aactacgcac agaagttcca gggccggttc 5940
accatctcca gagacaattc caagaacacg ctgtatctgc aaatgaacag cctgagagcc 6000
gaggacacgg ccgtatatta ctgtgcgaaa gggtccgtcc tcggtgatgc ttttgatatc 6060

tggggccagg gcaccctggt caccgtctcc tcagtgcctg gtgactcttc cgcaatcagt 6120

cctgggacag aaacctcagg gacagccttt atgagttttg atcacatcta acagagagga 6180

tgggccagat agagtactca cgctacgtca gtagacgtga ggtgcagctg ttggagtctg 6240

ggggaggctt ggtacagcct ggggggtccc tgagactctc ctgtgcagcc tctggattca 6300

cctttagcaa tgctagaatg ggtgtgagct gggtccgcca ggctccaggg aaggggctgg 6360

agtgggtctc acacattttt tcgaatgacg aaaaatccta cagcacatct ctgaagagcc 6420

ggttcaccat ctccagagac aattccaaga acacgctgta tctgcaaatg aacagcctga 6480

gagccgagga cacggccgta tattactgtg cgaaaatccc gtatgaagca gtggctggct 6540

actttgacta ctggggccag ggcaccctgg tcaccgtctc ctcagtgcct ggtgactctt 6600

ccgcaatcag tcctgggaca gaaacctcag ggacagcctt tatgagtttt gatcacatct 6660

aacagagagg atgggccagt acgctgtctt agtgtagatt cgatcacgtg aggtgcagct 6720

gttggagtct gggggaggct tggtacagcc tggggggtcc ctgagactct cctgtgcagc 6780

ctctggattc acctttagca gctattggat gagctgggtc cgccaggctc cagggaaggg 6840

gctggagtgg gtctcaaaca taaagcaaga tggaagtgag aaatactatg tggactctgt 6900

gaagggccgg ttcaccatct ccagagacaa ttccaagaac acgctgtatc tgcaaatgaa 6960

cagcctgaga gccgaggaca cggccgtata ttactgtgcg aaagttacgg tgaccgggac 7020

ctggtacttc gatctctggg gccagggcac cctggtcacc gtctcctcag tgcctggtga 7080

ctcttccgca atcagtcctg ggacagaaac ctcagggaca gcctttatga gttttgatca 7140

catctaacag agaggatggg ccagtgacgt atgttgtgag tagtacagta tatagaggtg 7200

cagctgttgg agtctggggg aggcttggta cagcctgggg ggtccctgag actctcctgt 7260

gcagcctctg gattcacctt tagcagctac gacatgcact gggtccgcca ggctccaggg 7320

aaggggctgg agtgggtctc agctattggt actgctggtg acacatacta tccaggctcc 7380

gtgaagggcc ggttcaccat ctccagagac aattccaaga acacgctgta tctgcaaatg 7440

aacagcctga gagccgagga cacggccgta tattactgtg cgaaagcgga tacagctatg 7500

gtcatttact actactacta cggtatggac gtctggggcc agggcaccct ggtcaccgtc 7560

tcctcagtgc ctggtgactc ttccgcaatc agtcctggga cagaaacctc agggacagcc 7620

tttatgagtt ttgatcacat ctaacagaga ggatgggcca gagtatacat aagtcgagag 7680

aagtgctacg agaggtgcag ctgttggagt ctgggggagg cttggtacag cctggggggt 7740

ccctgagact ctcctgtgca gcctctggat tcacctttag cagctatagc atgaactggg 7800

tccgccaggc tccagggaag gggctggagt gggtctcatc cattagtagt agtagtagtt 7860

acatatacta cgcagactca gtgaagggcc ggttcaccat ctccagagac aattccaaga 7920

acacgctgta tctgcaaatg aacagcctga gagccgagga cacggccgta tattactgtg 7980

cgaaagagaa tagtggctat gattactttg actactgggg ccagggcacc ctggtcaccg 8040

tctcctcagt gcctggtgac tcttccgcaa tcagtcctgg gacagaaacc tcagggacag 8100

cctttatgag ttttgatcac atctaacaga gaggatgggc cagcgtacag tcacgtactc 8160

agactacgct ctagaggtgc agctgttgga gtctggggga ggcttggtac agcctggggg 8220

gtccctgaga ctctcctgtg cagcctctgg attcaccttt agcgattatg ctatgagctg 8280

ggtccgccag gctccaggga aggggctgga gtgggtctca ttcattagaa gcaaagctta 8340

tggtgggaca acagaataca ccgcgtctgt gaaaggccgg ttcaccatct ccagagacaa 8400

ttccaagaac acgctgtatc tgcaaatgaa cagcctgaga gccgaggaca cggccgtata 8460

ttactgtgcg aaagatctag gtagtggctt tgactactgg ggccagggca ccctggtcac 8520

cgtctcctca gtgcctggtg actcttccgc aatcagtcct gggacagaaa cctcagggac 8580

agcctttatg agttttgatc acatctaaca gagaggatgg gccagtatat atacatatgc 8640

tagtatcacg cgagagaggt gcagctgttg gagtctgggg gaggcttggt acagcctggg 8700

gggtccctga gactctcctg tgcagcctct ggattcacct ttagcgacca ctacatggac 8760

tgggtccgcc aggctccagg gaaggggctg gagtgggtct cacgtactag aaacaaagct 8820

aacagttaca ccacagaata cgccgcgtct gtgaaaggcc ggttcaccat ctccagagac 8880

aattccaaga acacgctgta tctgcaaatg aacagcctga gagccgagga cacggccgta 8940

tattactgtg cgaaaggagg ccaggggagc cgatactact actactacgg tatggacgtc 9000

tggggccagg gcaccctggt caccgtctcc tcagtgcctg gtgactcttc cgcaatcagt 9060

cctgggacag aaacctcagg gacagccttt atgagttttg atcacatcta acagagagga 9120

tgggccagtg agtcagtatg tagtgtgatg tcacacgaga ggtgcagctg ttggagtctg 9180

ggggaggctt ggtacagcct ggggggtccc tgagactctc ctgtgcagcc tctggattca 9240

cctttagcag caatgagatg agctgggtcc gccaggctcc agggaagggg ctggagtggg 9300

tctcatccat tagtggtggt agcacatact acgcagactc caggaagggc cggttcacca 9360

tctccagaga caattccaag aacacgctgt atctgcaaat gaacagcctg agagccgagg 9420

acacggccgt atattactgt gcgaaagact catatgatgc ttttgatatc tggggccagg 9480

gcaccctggt caccgtctcc tcagtgcctg gtgactcttc cgcaatcagt cctgggacag 9540

aaacctcagg gacagccttt atgagttttg atcacatcta acagagagga tgggccagac 9600

atgacgacac gacagctcac gtctcatcga ggtgcagctg ttggagtctg ggggaggctt 9660

ggtacagcct ggggggtccc tgagactctc ctgtgcagcc tctggattca cctttagcag 9720

tggtggttac tcctggagct gggtccgcca ggctccaggg aaggggctgg agtgggtctc 9780

atacatctat catagtggga gcacctacta caacccgtcc ctcaagagtc ggttcaccat 9840

ctccagagac aattccaaga acacgctgta tctgcaaatg aacagcctga gagccgagga 9900

cacggccgta tattactgtg cgaaagatgc gagtaaccat ttctttgact actggggcca 9960

gggcaccctg gtcaccgtct cctcagtgcc tggtgactct tccgcaatca gtcctgggac 10020

agaaacctca gggacagcct ttatgagttt tgatcacatc taacagagag gatgggccag 10080

agcgactagc agtagtgatc agtgacacac gaggtgcagc tgttggagtc tgggggaggc 10140

ttggtacagc ctggggggtc cctgagactc tcctgtgcag cctctggatt cacctttagc 10200

agtagtagtt actactgggg ctgggtccgc caggctccag ggaaggggct ggagtgggtc 10260

tcaagtatct attatagtgg gagcacctac tacaacccgt ccctcaagag tcggttcacc 10320

atctccagag acaattccaa gaacacgctg tatctgcaaa tgaacagcct gagagccgag 10380

gacacggccg tatattactg tgcgaaaaag gactggaacg acaactggtt cgacccctgg 10440

ggccagggca ccctggtcac cgtctcctca gtgcctggtg actcttccgc aatcagtcct 10500

gggacagaaa cctcagggac agcctttatg agttttgatc acatctaaca gagaggatgg 10560

gccagatcta ctgaccacgt agatccacgt gtcgcgaggt gcagctgttg gagtctgggg 10620

gaggcttggt acagcctggg gggtccctga gactctcctg tgcagcctct ggattcacct 10680

ttagcagcaa cagtgctgct tggaactggg tccgccaggc tccagggaag gggctggagt 10740

gggtctcaag gacatactac aggtccaagt ggtataatga ttatgcagta tctgtgaaaa 10800

gtcggttcac catctccaga gacaattcca agaacacgct gtatctgcaa atgaacagcc 10860

tgagagccga ggacacggcc gtatattact gtgcgaaaga tattgtagta gtaccagctg 10920

ctatatactg gtacttcgat ctctggggcc agggcaccct ggtcaccgtc tcctcagtgc 10980

ctggtgactc ttccgcaatc agtcctggga cagaaacctc agggacagcc tttatgagtt 11040

ttgatcacat ctaacagaga ggatgggcca ggtttaaacg gtacctggcc aggatccgct 11100

agcgagctcg tttaaacagc actgtagatc agacacgata tcgcgaggag gtgcagctgt 11160

tggagtctgg gggaggcttg gtacagcctg gggggtccct gagactctcc tgtgcagcct 11220

ctggattcac ctttagcagc tatgctatgc attgggtccg ccaggctcca gggaaggggc 11280

tggagtgggt ctcatggatc aacgctggca atggtaacac aaaatattca cagaagttcc 11340

agggccggtt caccatctcc agagacaatt ccaagaacac gctgtatctg caaatgaaca 11400

gcctgagagc cgaggacacg gccgtatatt actgtgcgaa agcgtctagt agtggttatt 11460

actacgtcca ctttgactac tggggccagg gcaccctggt caccgtctcc tcagtgcctg 11520

gtgactcttc cgcaatcagt cctgggacag aaacctcagg gacagccttt atgagttttg 11580

atcacatcta acagagagga tgggccagtg atacgtctca tagtagtgcg agagatacga 11640

ggtgcagctg ttggagtctg ggggaggctt ggtacagcct ggggggtccc tgagactctc 11700

ctgtgcagcc tctggattca cctttagcag ctatggtatc agctgggtcc gccaggctcc 11760
agggaagggg ctggagtggg tctcatggat cagcgcttac aatggtaaca caaactatgc 11820
acagaagctc cagggccggt tcaccatctc cagagacaat tccaagaaca cgctgtatct 11880
gcaaatgaac agcctgagag ccgaggacac ggccgtatat tactgtgcga aagatttaag 11940
gttatcctat tactactttg actactgggg ccagggcacc ctggtcaccg tctcctcagt 12000
gcctggtgac tcttccgcaa tcagtcctgg gacagaaacc tcagggacag cctttatgag 12060
ttttgatcac atctaacaga gaggatgggc cagtctacgt agctgtacta ctcacgacta 12120
caggaggtgc agctgttgga gtctggggga ggcttggtac agcctggggg gtccctgaga 12180
ctctcctgtg cagcctctgg attcaccttt agcggctttg ccatgaactg ggtccgccag 12240
gctccaggga aggggctgga gtgggtctca actattagta atagtgctgg tagcacatat 12300
tacgcagact ccgtgaaggg ccggttcacc atctccagag acaattccaa gaacacgctg 12360
tatctgcaaa tgaacagcct gagagccgag gacacggccg tatattactg tgcgaaatat 12420
ggggaggata ttgtcgttgt gccagctcgc tacggtgtgg acgtctgggg ccagggcacc 12480
ctggtcaccg tctcctcagt gcctggtgac tcttccgcaa tcagtcctgg gacagaaacc 12540
tcagggacag cctttatgag ttttgatcac atctaacaga gaggatgggc cagtagagac 12600
gagtcgtcgc tcgacatacg cgtgaggtgc agctgttgga gtctggggga ggcttggtac 12660
agcctggggg gtccctgaga ctctcctgtg cagcctctgg attcaccttt agcagctctg 12720
ctgtgcagtg ggtccgccag gctccaggga aggggctgga gtgggtctca tggatcgtcg 12780
ttggcagtgg taacacaaac tacgcacaga agttccagga acggttcacc atctccagag 12840
acaattccaa gaacacgctg tatctgcaaa tgaacagcct gagagccgag gacacggccg 12900
tatattactg tgcgaaagac gtccttgtag taccagctgc cccctactac tactacggta 12960
tggacgtctg gggccagggc accctggtca ccgtctcctc agtgcctggt gactcttccg 13020
caatcagtcc tgggacagaa acctcaggga cagcctttat gagttttgat cacatctaac 13080
agagaggatg ggccagagac tatactagcg tcgtctagta cgctatgagg tgcagctgtt 13140
ggagtctggg ggaggcttgg tacagcctgg ggggtccctg agactctcct gtgcagcctc 13200
tggattcacc tttagcacta gtggagtggg tgtgggctgg gtccgccagg ctccagggaa 13260
ggggctggag tgggtctcac tcatttattg gaatgatgat aagcgctaca gcccatctct 13320
gaagagccgg ttcaccatct ccagagacaa ttccaagaac acgctgtatc tgcaaatgaa 13380
cagcctgaga gccgaggaca cggccgtata ttactgtgcg aaagacgatg gggcgtatgg 13440
gccgtatagc agcgcctgga cctttgacta ctggggccag ggcaccctgg tcaccgtctc 13500
ctcagtgcct ggtgactctt ccgcaatcag tcctgggaca gaaacctcag ggacagcctt 13560
tatgagtttt gatcacatct aacagagagg atgggccagc gacgtgactt acacacactt 13620

acagatcgtg aggtgcagct gttggagtct gggggaggct tggtacagcc tggggggtcc 13680

ctgagactct cctgtgcagc ctctggattc acctttagca ctagtggaat gcgtgtgagc 13740

tgggtccgcc aggctccagg gaaggggctg gagtgggtct cacgcattga ttgggatgat 13800

gataaattct acagcacatc tctgaagacc cggttcacca tctccagaga caattccaag 13860

aacacgctgt atctgcaaat gaacagcctg agagccgagg acacggccgt atattactgt 13920

gcgaaagatg acggtggcac cccgcccggg gaattctttg actactgggg ccagggcacc 13980

ctggtcaccg tctcctcagt gcctggtgac tcttccgcaa tcagtcctgg gacagaaacc 14040

tcagggacag cctttatgag ttttgatcac atctaacaga gaggatgggc cagtcgcact 14100

agttctagcg acttctatac tatgaggtgc agctgttgga gtctggggga ggcttggtac 14160

agcctggggg gtccctgaga ctctcctgtg cagcctctgg attcaccttt agcgattatg 14220

ccatgcactg ggtccgccag gctccaggga aggggctgga gtgggtctca ggtattagtt 14280

ggaatagtgg tagcataggc tatgcggact ctgtgaaggg ccggttcacc atctccagag 14340

acaattccaa gaacacgctg tatctgcaaa tgaacagcct gagagccgag gacacggccg 14400

tatattactg tgcgaaaata gtgggagcta cttacaactg gttcgacccc tggggccagg 14460

gcaccctggt caccgtctcc tcagtgcctg gtgactcttc cgcaatcagt cctgggacag 14520

aaacctcagg gacagccttt atgagttttg atcacatcta acagagagga tgggccagac 14580

gcgatcgaac tagcagtaag ctcacgtaga ggtgcagctg ttggagtctg ggggaggctt 14640

ggtacagcct ggggggtccc tgagactctc ctgtgcagcc tctggattca cctttagcaa 14700

cgcctggatg agctgggtcc gccaggctcc agggaagggg ctggagtggg tctcacgtat 14760

taaaagcaaa actgatggtg ggacaacaga ctacgctgca cccgtgaaag gccggttcac 14820

catctccaga gacaattcca agaacacgct gtatctgcaa atgaacagcc tgagagccga 14880

ggacacggcc gtatattact gtgcgaaaga aaggggtagc agctcgtcct ttgactactg 14940

gggccagggc accctggtca ccgtctcctc agtgcctggt gactcttccg caatcagtcc 15000

tgggacagaa acctcaggga cagcctttat gagttttgat cacatctaac agagaggatg 15060

ggccagcgat cgtatacgca gtacgacgcg tatacagagg tgcagctgtt ggagtctggg 15120

ggaggcttgg tacagcctgg ggggtccctg agactctcct gtgcagcctc tggattcacc 15180

tttagcagct atggcatgca ctgggtccgc caggctccag ggaaggggct ggagtgggtc 15240

tcagttatat ggtatgatgg aagtaataaa tactatgcag actccgtgaa gggccggttc 15300

accatctcca gagacaattc caagaacacg ctgtatctgc aaatgaacag cctgagagcc 15360

gaggacacgg ccgtatatta ctgtgcgaaa gatcttgtct tcggccagct tgctactcct 15420

tttgactact ggggccaggg caccctggtc accgtctcct cagtgcctgg tgactcttcc 15480

gcaatcagtc ctgggacaga aacctcaggg acagccttta tgagttttga tcacatctaa 15540

cagagaggat gggccagcta tagcgtatac gtcatcatag tcgcatagag gtgcagctgt 15600

tggagtctgg gggaggcttg gtacagcctg gggggtccct gagactctcc tgtgcagcct 15660

ctggattcac ctttagcagc aactacatga gctgggtccg ccaggctcca gggaaggggc 15720

tggagtgggt ctcagttatt tatagcggtg gtagcacata ctacgcagac tccgtgaagg 15780

gccggttcac catctccaga gacaattcca agaacacgct gtatctgcaa atgaacagcc 15840

tgagagccga ggacacggcc gtatattact gtgcgaaagc gaagttctcc tatggttcag 15900

ggagttatta ctttgactac tggggccagg gcaccctggt caccgtctcc tcagtgcctg 15960

gtgactcttc cgcaatcagt cctgggacag aaacctcagg gacagccttt atgagttttg 16020

atcacatcta acagagagga tgggccagtc gatagtgatc gctgcgtatc tgacgtcaga 16080

ggtgcagctg ttggagtctg ggggaggctt ggtacagcct ggggggtccc tgagactctc 16140

ctgtgcagcc tctggattca cctttagcag ctactggatg cactgggtcc gccaggctcc 16200

agggaagggg ctggagtggg tctcacgtat taatagtgat gggagtagca caagctacgc 16260

ggactccgtg aagggccggt tcaccatctc cagagacaat tccaagaaca cgctgtatct 16320

gcaaatgaac agcctgagag ccgaggacac ggccgtatat tactgtgcga aaggatattg 16380

tagtagtacc agctgctatg ctgatgcttt tgatatctgg ggccagggca ccctggtcac 16440

cgtctcctca gtgcctggtg actcttccgc aatcagtcct gggacagaaa cctcagggac 16500

agcctttatg agttttgatc acatctaaca gagaggatgg gccagtgtcg tcgcaacaca 16560

tacgcacagt cgtgcgaggt gcagctgttg gagtctgggg gaggcttggt acagcctggg 16620

gggtccctga gactctcctg tgcagcctct ggattcacct ttagcagtag taactggtgg 16680

ggctgggtcc gccaggctcc agggaagggg ctggagtggg tctcatacat ctattatagt 16740

gggagcacct actacaaccc gtccctcaag agtcggttca ccatctccag agacaattcc 16800

aagaacacgc tgtatctgca aatgaacagc ctgagagccg aggacacggc cgtatattac 16860

tgtgcgaaag atcgaagtag agggggtttt gactactggg gccagggcac cctggtcacc 16920

gtctcctcag tgcctggtga ctcttccgca atcagtcctg ggacagaaac ctcagggaca 16980

gcctttatga gttttgatca catctaacag agaggatggg ccagactcat ctacactcgc 17040

gcacagagcg tcacgaggtg cagctgttgg agtctggggg aggcttggta cagcctgggg 17100

ggtccctgag actctcctgt gcagcctctg gattcacctt tagcggttac tactggagct 17160

gggtccgcca ggctccaggg aaggggctgg agtgggtctc agaaatcaat catagtggaa 17220

gcaccaacta caacccgtcc ctcaagagtc ggttcaccat ctccagagac aattccaaga 17280

acacgctgta tctgcaaatg aacagcctga gagccgagga cacggccgta tattactgtg 17340

cgaaagcccg taccagcgcg ggcccgggtg gtgcttttga tttctggggc cagggcaccc 17400

tggtcaccgt ctcctcagtg cctggtgact cttccgcaat cagtcctggg acagaaacct 17460

cagggacagc ctttatgagt tttgatcaca tctaacagag aggatgggcc agagtgtatg 17520

tcatagatag acatatagtc gcgaggtgca gctgttggag tctgggggag gcttggtaca 17580

gcctgggggg tccctgagac tctcctgtgc agcctctgga ttcaccttta gcagctactg 17640

gatcggctgg gtccgccagg ctccagggaa ggggctggag tgggtctcaa tcatctatcc 17700

tggtgactct gataccagat acagcccgtc cttccaaggc cggttcacca tctccagaga 17760

caattccaag aacacgctgt atctgcaaat gaacagcctg agagccgagg acacggccgt 17820

atattactgt gcgaaagggg tttgtagtac cagctcgtac gccttttact actactacta 17880

cggtatggac gtctggggcc agggcaccct ggtcaccgtc tcctcagtgc ctggtgactc 17940

ttccgcaatc agtcctggga cagaaacctc agggacagcc tttatgagtt ttgatcacat 18000

ctaacagaga ggatgggcca gcatactcta ccgacactat ccgagacgcg cgaggtgcag 18060

ctgttggagt ctgggggagg cttggtacag cctggggggt ccctgagact ctcctgtgca 18120

gcctctggat tcacctttag cagctatgct atgaattggg tccgccaggc tccagggaag 18180

gggctggagt gggtctcatg gatcaacacc aacactggga acccaacgta tgcccagggc 18240

ttcacaggac ggttcaccat ctccagagac aattccaaga acacgctgta tctgcaaatg 18300

aacagcctga gagccgagga cacggccgta tattactgtg cgaaagacta cggtggtaac 18360

tcctactggt acttcgatct ctggggccag ggcaccctgg tcaccgtctc ctcagtgcct 18420

ggtgactctt ccgcaatcag tcctgggaca gaaacctcag ggacagcctt tatgagtttt 18480

gatcacatct aacagagagg atgggccagg tttaaacggt acctggccac catggccgcg 18540

gatgcggcgc gccatcgata tgcgtcgaca tttaaatgtt ggcgcagaag ggattcatat 18600

caattaattc cctgtcctca attaatgcat aattgggagt gcgatggaat cattcaataa 18660

gtaatgagga cttggtgtca aaatgccaat taagtaatta atgacaccca attaatacac 18720

aatggggatt acagtggaat aactcagtga agttcatatc taaattgcaa ttgaataatt 18780

aattacagct aattaatgga tgggagactg gcagtggtat aacttaatga aggaacacag 18840

tgaattcctg tctgaattga aattaattga tttattaggg ctaattaatg aatggtgggg 18900

ttaaaaatag cattactcag cgaaggaaca tgagttcatg gctaaattga gattgaatta 18960

ttaattacaa ctaattgatt aatgactgat gggagtaaga atggaataac tcagtgaagg 19020

aacacaatgg attcatggcc taattgaaat taaatggtta attagagcta attaatgaat 19080

gatggggggt ggcaatggaa tgatacaaga aagaaacaca atgggttcgt gtctacattg 19140

aaattaaata attaattaca gctaattaat aaacggcgag gttgacaatg gtataactgt 19200

gaagagacag aatgagttcg tgactaaatt ggtattaaat gattaattag agctaattaa 19260

caggtggtgg ggctgagaat ggcataactc aatgggggat gatgagctga tggcttaatt 19320
ggaattaata attaatcaca accaattaac aggtgctgtt aatggggtta caggggcatg 19380
acttcaggaa gaagcacaaa gtggccatta aataattaat tgcacccaat taattcctga 19440
tggggattac tgcagaggaa ctcagcgaag gattcaagca gaaatgacac agttaggttg 19500
ctcaattgcg ttaattaatt aaaatcgcta attaggtgag cttcattaag gcctgatggg 19560
gttcatccca acgctgggat caaagaactc agcgcagagt ggataacgat aattagaagg 19620
ggacaaatta atgaggaaac acgactttgg gcctagacgg ccattaaatc attaatcaac 19680
ccgacgggcg cccaattaat cataaagtta ttaagcaatt aagtcgaggc tgacgagaaa 19740
ccgcaacgag agccctccac gctcctttga gttggcgcaa acaacaaaca acgcgggctc 19800
gttaagcagc gacgacgccg agcgtaacga agcaatcccc gcgcccgaca attaacgcgc 19860
taacgaagcc ggagccctcc ttatgcaaat tagcccctcc agagggccat aaaagcgccg 19920
gctctccgac ggcgcgggag gagcaccagt cggctccgca accatgagcc cactcgtctc 19980
ctccctcctg ctcctggccg ccctgccagg tgagggcgct gtggggctct atggggctct 20040
atggggtctc agcggggctc tgcgggctca atgggggcca aggggggggt ctgcgggctc 20100
tatggggggg tcaacggggg gtctcacggg gggccggctc cgcgaggccg tgtggcggcg 20160
gctccgtcag cgctctctgg gcgcgcctcc ttccccacag ggctgatggc ggaggtgcag 20220
ctgttggagt ctgggggagg cttggtacag cctggggggt ccctgagact ctcctgtgca 20280
gcctctggat tcacctttag cagctatgcc atgagctggg tccgccaggc tccagggaag 20340
gggctggagt gggtctcagc tattagtggt agtggtggta gcacatacta cgcagactcc 20400
gtgaagggcc ggttcaccat ctccagagac aattccaaga acacgctgta tctgcaaatg 20460
aacagcctga gagccgagga cacggccgta tattactgtg cgaaaggata tagtggctac 20520
gattactact ttgactactg gggccagggc accctggtca ccgtctcctc aggtgagtct 20580
tcatatgaac cccccccaaa attgccgcgg cgattttggg gcaaaatcgg gcgattttat 20640
ggatccccgc ggccgcaggc gtaatcatgg tcatagctgt ttcctgtgtg aaattgttat 20700
ccgctcacaa ttccacacaa catacgagcc ggaagcataa agtgtaaagc ctggggtgcc 20760
taatgagtga gctaactcac attaattgcg ttgcgctcac tgcccgcttt ccagtcggga 20820
aacctgtcgt gccagctgca ttaatgaatc ggccaacgcg cggggagagg cggtttgcgt 20880
attgggcgct cttccgcttc ctcgctcact gactcgctgc gctcggtcgt tcggctgcgg 20940
cgagcggtat cagctcactc aaaggcggta atacggttat ccacagaatc aggggataac 21000
gcaggaaaga acatgtgagc aaaaggccag caaaaggcca ggaaccgtaa aaaggccgcg 21060
ttgctggcgt ttttccatag gctccgcccc cctgacgagc atcacaaaaa tcgacgctca 21120
agtcagaggt ggcgaaaccc gacaggacta taaagatacc aggcgtttcc ccctggaagc 21180

tccctcgtgc gctctcctgt tccgaccctg ccgcttaccg gatacctgtc cgcctttctc 21240

ccttcgggaa gcgtggcgct ttctcatagc tcacgctgta ggtatctcag ttcggtgtag 21300

gtcgttcgct ccaagctggg ctgtgtgcac gaaccccccg ttcagcccga ccgctgcgcc 21360

ttatccggta actatcgtct tgagtccaac ccggtaagac acgacttatc gccactggca 21420

gcagccactg gtaacaggat tagcagagcg aggtatgtag gcggtgctac agagttcttg 21480

aagtggtggc ctaactacgg ctacactaga agaacagtat ttggtatctg cgctctgctg 21540

aagccagtta ccttcggaaa aagagttggt agctcttgat ccggcaaaca aaccaccgct 21600

ggtagcggtg gtttttttgt ttgcaagcag cagattacgc gcagaaaaaa aggatctcaa 21660

gaagatcctt tgatcttttc tacggggtct gacgctcagt ggaacgaaaa ctcacgttaa 21720

gggattttgg tcatgagatt atcaaaaagg atcttcacct agatcctttt aaattaaaaa 21780

tgaagtttta aatcaatcta aagtatatat gagtaaactt ggtctgacag ttaccaatgc 21840

ttaatcagtg aggcacctat ctcagcgatc tgtctatttc gttcatccat agttgcctga 21900

ctccccgtcg tgtagataac tacgatacgg gagggcttac catctggccc cagtgctgca 21960

atgataccgc gagacccacg ctcaccggct ccagatttat cagcaataaa ccagccagcc 22020

ggaagggccg agcgcagaag tggtcctgca actttatccg cctccatcca gtctattaat 22080

tgttgccggg aagctagagt aagtagttcg ccagttaata gtttgcgcaa cgttgttgcc 22140

attgctacag gcatcgtggt gtcacgctcg tcgtttggta tggcttcatt cagctccggt 22200

tcccaacgat caaggcgagt tacatgatcc cccatgttgt gcaaaaaagc ggttagctcc 22260

ttcggtcctc cgatcgttgt cagaagtaag ttggccgcag tgttatcact catggttatg 22320

gcagcactgc ataattctct tactgtcatg ccatccgtaa gatgcttttc tgtgactggt 22380

gagtactcaa ccaagtcatt ctgagaatag tgtatgcggc gaccgagttg ctcttgcccg 22440

gcgtcaatac gggataatac cgcgccacat agcagaactt taaaagtgct catcattgga 22500

aaacgttctt cggggcgaaa actctcaagg atcttaccgc tgttgagatc cagttcgatg 22560

taacccactc gtgcacccaa ctgatcttca gcatctttta ctttcaccag cgtttctggg 22620

tgagcaaaaa caggaaggca aaatgccgca aaaaagggaa taagggcgac acggaaatgt 22680

tgaatactca tactcttcct ttttcaatat tattgaagca tttatcaggg ttattgtctc 22740

atgagcggat acatatttga atgtatttag aaaaataaac aaataggggt tccgcgcaca 22800

tttccccgaa aagtgccacc tgacgtctaa gaaaccatta ttatcatgac attaacctat 22860

aaaaataggc gtatcacgag gccctttcgt c 22891

<210> 127
<211> 333
<212> DNA

<213>  Artificial Sequence

<220>
<223>  Synthetic DNA

<400>  127
cagtctgccc tgactcagcc tgcctccgtg tctgggtctc ctggacagtc gatcaccatc      60

tcctgctctg gaagcagctc caacatcgga aataatgctg taaactggta ccaacagcac     120

ccaggcaaag cccccaaact catgatttat tatgatgatc tgctgccctc aggggtttct     180

aatcgcttct ctggctccaa gtctggcaac acggcctccc tgaccatctc tgggctccag     240

gctgaggacg aggctgatta ttactgcgga acatgggatg gcagcctgaa tggtgggagg     300

ctgttcggcg gagggaccaa gctgaccgtc cta                                  333


<210>  128
<211>  330
<212>  DNA
<213>  Artificial Sequence

<220>
<223>  Synthetic DNA

<400>  128
cagtctgccc tgactcagcc tgcctccgtg tctgggtctc ctggacagtc gatcaccatc      60

tcctgctctg gaagcagctc caacatcgga agtaatactg taaactggta ccaacagcac     120

ccaggcaaag cccccaaact catgatttat agtaataatc agcggccctc aggggtttct     180

aatcgcttct ctggctccaa gtctggcaac acggcctccc tgaccatctc tgggctccag     240

gctgaggacg aggctgatta ttactgcgca gcatgggatg acagcctgag tggtgtggtt     300

ttcggcggag ggaccaagct gaccgtccta                                      330


<210>  129
<211>  330
<212>  DNA
<213>  Artificial Sequence

<220>
<223>  Synthetic DNA

<400>  129
cagtctgccc tgactcagcc tgcctccgtg tctgggtctc ctggacagtc gatcaccatc      60

tcctgctctg gaagcagctc cgacatgggg aattatgcgg tatcctggta ccaacagcac     120

ccaggcaaag cccccaaact catgatttat gatgtcagta agcggccctc aggggtttct     180

aatcgcttct ctggctccaa gtctggcaac acggcctccc tgaccatctc tgggctccag     240

gctgaggacg aggctgatta ttactgcagc tcatatgaag acagcaacaa tttgggggta     300

ttcggcggag ggaccaagct gaccgtccta                                      330

<210>  130
<211>  342
<212>  DNA
<213>  Artificial Sequence

<220>
<223>  Synthetic DNA

<400>  130
cagtctgccc tgactcagcc tgcctccgtg tctgggtctc ctggacagtc gatcaccatc      60

tcctgcactg gaaccagcag tgacgttggt agttataacc gtgtctcctg gtaccaacag     120

cacccaggca aagcccccaa actcatgatt tatgaggtca gtaatcggcc ctcaggggtt     180

tctaatcgct tctctggctc caagtctggc aacacggcct ccctgaccat ctctgggctc     240

caggctgagg acgaggctga ttattactgc gcagcatggg atggcagccc ggccccgagt     300

gacttttatg tcttcggcgg agggaccaag ctgaccgtcc ta                        342


<210>  131
<211>  348
<212>  DNA
<213>  Artificial Sequence

<220>
<223>  Synthetic DNA

<400>  131
cagtctgccc tgactcagcc tgcctccgtg tctgggtctc ctggacagtc gatcaccatc      60

tcctgctctg gagataaatt gggggataaa tatgcttgct ggtaccaaca gcacccaggc     120

aaagccccca aactcatgat ttatcaagat agcaagcggc cctcaggggt ttctaatcgc     180

ttctctggct ccaagtctgg caacacggcc tccctgacca tctctgggct ccaggctgag     240

gacgaggctg attattactg ccagtccttt gacaacagcc tgtgccagtc ctttgacaac     300

agcctgaatt atgtgctttt cggcggaggg accaagctga ccgtccta                  348


<210>  132
<211>  330
<212>  DNA
<213>  Artificial Sequence

<220>
<223>  Synthetic DNA

<400>  132
cagtctgccc tgactcagcc tgcctccgtg tctgggtctc ctggacagtc gatcaccatc      60

tcctgctctg gagatgcatt gccaaaaaaa tatgcttatt ggtaccaaca gcacccaggc     120

aaagccccca aactcatgat ttatgaggac agcaaacgac cctccggggt ttctaatcgc     180

ttctctggct ccaagtctgg caacacggcc tccctgacca tctctgggct ccaggctgag     240

gacgaggctg attattactg cgcagcatgg gatgacagcc tgaatggtcc cttttatgtc     300

ttcggcggag ggaccaagct gaccgtccta 330

<210> 133
<211> 324
<212> DNA
<213> Artificial Sequence

<220>
<223> Synthetic DNA

<400> 133
cagtctgccc tgactcagcc tgcctccgtg tctgggtctc ctggacagtc gatcaccatc 60

tcctgccaag gagacagcct cagaagctat tatgcaagct ggtaccaaca gcacccaggc 120

aaagccccca aactcatgat ttatggtaaa aacaaccggc cctcaggggt ttctaatcgc 180

ttctctggct ccaagtctgg caacacggcc tccctgacca tctctgggct ccaggctgag 240

gacgaggctg attattactg ccagtcctat gacagcagcc tgagtggtgt ggtattcggc 300

ggagggacca agctgaccgt ccta 324

<210> 134
<211> 324
<212> DNA
<213> Artificial Sequence

<220>
<223> Synthetic DNA

<400> 134
cagtctgccc tgactcagcc tgcctccgtg tctgggtctc ctggacagtc gatcaccatc 60

tcctgctctg gagatgtact gggggaaaat tatgctgact ggtaccaaca gcacccaggc 120

aaagccccca aactcatgat ttatgaagat agtgagcggt accctggggt ttctaatcgc 180

ttctctggct ccaagtctgg caacacggcc tccctgacca tctctgggct ccaggctgag 240

gacgaggctg attattactg cggaacatgg gatagcagcc tgagtggtgt ggtattcggc 300

ggagggacca agctgaccgt ccta 324

<210> 135
<211> 321
<212> DNA
<213> Artificial Sequence

<220>
<223> Synthetic DNA

<400> 135
cagtctgccc tgactcagcc tgcctccgtg tctgggtctc ctggacagtc gatcaccatc 60

tcctgctcag gagatgtact ggcaaaaaaa tatgctcggt ggtaccaaca gcacccaggc 120

aaagccccca aactcatgat ttataggaaa aacagccaac tctcaggggt ttctaatcgc 180

ttctctggct ccaagtctgg caacacggcc tccctgacca tctctgggct ccaggctgag 240

gacgaggctg attattactg ctactctgcg gctgacaaca atcttgtggt attcggcgga 300

gggaccaagc tgaccgtcct a 321

<210> 136
<211> 336
<212> DNA
<213> Artificial Sequence

<220>
<223> Synthetic DNA

<400> 136
cagtctgccc tgactcagcc tgcctccgtg tctgggtctc ctggacagtc gatcaccatc 60

tcctgcactc tgagcagtgg gcacagtagc tacatcatcg catggtacca acagcaccca 120

ggcaaagccc ccaaactcat gatttatctt gaaggtagtg gaagctacaa caaggggagc 180

ggggtttcta atcgcttctc tggctccaag tctggcaaca cggcctccct gaccatctct 240

gggctccagg ctgaggacga ggctgattat tactgcgaga cctgggacag taacactcat 300

gtggtattcg gcggagggac caagctgacc gtccta 336

<210> 137
<211> 348
<212> DNA
<213> Artificial Sequence

<220>
<223> Synthetic DNA

<400> 137
cagtctgccc tgactcagcc tgcctccgtg tctgggtctc ctggacagtc gatcaccatc 60

tcctgcacct tgcccagtga catcaatgtt ggtagctaca acatatactg gtaccaacag 120

cacccaggca aagcccccaa actcatgatt tattactact cagactcaga taagggccag 180

ggctctgggg tttctaatcg cttctctggc tccaagtctg gcaacacggc ctccctgacc 240

atctctgggc tccaggctga ggacgaggct gattattact gccactcata tgcaggcggc 300

gacactttcg atgtggtgtt cggcggaggg accaagctga ccgtccta 348

<210> 138
<211> 348
<212> DNA
<213> Artificial Sequence

<220>
<223> Synthetic DNA

<400> 138
cagtctgccc tgactcagcc tgcctccgtg tctgggtctc ctggacagtc gatcaccatc 60

tcctgcatgc tgagcagtgg cttcagtgtt ggggacttct ggataaggtg gtaccaacag 120

cacccaggca aagcccccaa actcatgatt tattaccact cagactccaa taagggccaa 180

ggctctgggg tttctaatcg cttctctggc tccaagtctg gcaacacggc ctccctgacc 240

atctctgggc tccaggctga ggacgaggct gattattact gcggtacatg gcacagcaac 300

tctaagactc atgtggtatt cggcggaggg accaagctga ccgtccta 348

<210> 139
<211> 330
<212> DNA
<213> Artificial Sequence

<220>
<223> Synthetic DNA

<400> 139
cagtctgccc tgactcagcc tgcctccgtg tctgggtctc ctggacagtc gatcaccatc 60

tcctgcgctt ccagcactgg agcagtcacc agtggttact atccaaactg gtaccaacag 120

cacccaggca aagcccccaa actcatgatt tatagtacaa gcaacaaaca ctccggggtt 180

tctaatcgct tctctggctc caagtctggc aacacggcct ccctgaccat ctctgggctc 240

caggctgagg acgaggctga ttattactgc agctcatata caatcaccga catcgtggtc 300

ttcggcggag ggaccaagct gaccgtccta 330

<210> 140
<211> 333
<212> DNA
<213> Artificial Sequence

<220>
<223> Synthetic DNA

<400> 140
cagtctgccc tgactcagcc tgcctccgtg tctgggtctc ctggacagtc gatcaccatc 60

tcctgcggct tgagctctgg ctcagtctct actagttact accccagctg gtaccaacag 120

cacccaggca aagcccccaa actcatgatt tatagcacaa acactcgctc ttctggggtt 180

tctaatcgct tctctggctc caagtctggc aacacggcct ccctgaccat ctctgggctc 240

caggctgagg acgaggctga ttattactgc gtgctgtata tgggtagtgg catttctgtg 300

gtattcggcg gagggaccaa gctgaccgtc cta 333

<210> 141
<211> 333
<212> DNA
<213> Artificial Sequence

<220>
<223> Synthetic DNA

<400> 141
cagtctgccc tgactcagcc tgcctccgtg tctgggtctc ctggacagtc gatcaccatc 60

tcctgcactg ggaacagcaa caatgttggc aaccaaggag cagcttggta ccaacagcac 120

ccaggcaaag cccccaaact catgatttat aggaataaca accggccctc aggggtttct 180

aatcgcttct ctggctccaa gtctggcaac acggcctccc tgaccatctc tgggctccag 240

gctgaggacg aggctgatta ttactgcgca gcatgggatg acagcctgag tgcccatgtg 300

gtattcggcg gagggaccaa gctgaccgtc cta 333

<210> 142
<211> 13829
<212> DNA
<213> Artificial Sequence

<220>
<223> Synthetic DNA

<400> 142
acgagtgcgt acgctcgaca agacgcactc cagtctgccc tgactcagcc tgcctccgtg 60

tctgggtctc ctggacagtc gatcaccatc tcctgctctg gaagcagctc caacatcgga 120

agtaattatg tatactggta ccaacagcac ccaggcaaag cccccaaact catgatttat 180

aggaataatc agcggccctc aggggtttct aatcgcttct ctggctccaa gtctggcaac 240

acggcctccc tgaccatctc tgggctccag gctgaggacg aggctgatta ttactgcgca 300

gcatgggatg acagcctgag tggtcattgg gtgttcggcg gagggaccaa gctgaccgtc 360

ctagcctgac acacacagcc ctccccagac tgtgggatga ggccctgtgc ccgcagtcac 420

atgtggaata tcaagacaca cacatctatg acaatcaccg tgtctctact cgcgtgtctc 480

tctatgcgca gtctgccctg actcagcctg cctccgtgtc tgggtctcct ggacagtcga 540

tcaccatctc ctgcactggg agcagctcca acatcggggc aggttatgat gtacactggt 600

accaacagca cccaggcaaa gcccccaaac tcatgattta tggtaacagc aatcggccct 660

caggggtttc taatcgcttc tctggctcca agtctggcaa cacggcctcc ctgaccatct 720

ctgggctcca ggctgaggac gaggctgatt attactgcca gtcctatgac agcagcctga 780

gtggttctgt ggtattcggc ggagggacca agctgaccgt cctagcctga cacacacagc 840

cctccccaga ctgtgggatg aggccctgtg cccgcagtca catgtggaat atcaagacac 900

acacatctat gacaatcaca tacgacgtat cacgtactac gtctagtacc agtctgccct 960

gactcagcct gcctccgtgt ctgggtctcc tggacagtcg atcaccatct cctgctctgg 1020

aagcagctcc aacattggga ataattatgt atcctggtac caacagcacc caggcaaagc 1080

ccccaaactc atgatttatg acaataataa gcgaccctca ggggtttcta atcgcttctc 1140

tggctccaag tctggcaaca cggcctccct gaccatctct gggctccagg ctgaggacga 1200

ggctgattat tactgcggaa catgggatag cagcctgagt gctggtgctg tgttcggcgg 1260

agggaccaag ctgaccgtcc tagcctgaca cacacagccc tccccagact gtgggatgag 1320

gccctgtgcc cgcagtcaca tgtggaatat caagacacac acatctatga caatcaccgt 1380

agactagtac gagtatgtac tctcgtgcag tctgccctga ctcagcctgc ctccgtgtct 1440

gggtctcctg gacagtcgat caccatctcc tgcactggga gcagctccaa cattggggcg 1500

ggttatgttg tacattggta ccaacagcac ccaggcaaag cccccaaact catgatttat 1560

gaaaataata agcgaccctc aggggtttct aatcgcttct ctggctccaa gtctggcaac 1620

acggcctccc tgaccatctc tgggctccag gctgaggacg aggctgatta ttactgcagc 1680

tcatatacaa gcagcggcac tcctgtggta ttcggcggag ggaccaagct gaccgtccta 1740

gcctgacaca cacagccctc cccagactgt gggatgaggc cctgtgcccg cagtcacatg 1800

tggaatatca agacacacac atctatgaca atcacacgcg agtatactac tatgtactgt 1860

acagtcagtc tgccctgact cagcctgcct ccgtgtctgg gtctcctgga cagtcgatca 1920

ccatctcctg cactggaacc agcagtgatg ttgggagtta taaccttgtc tcctggtacc 1980

aacagcaccc aggcaaagcc cccaaactca tgatttatga gggcagtaag cggccctcag 2040

gggtttctaa tcgcttctct ggctccaagt ctggcaacac ggcctccctg accatctctg 2100

ggctccaggc tgaggacgag gctgattatt actgccagtc ctatgacagc agcctgagtg 2160

gttgccagtc ctatgacagc agcctgagtg gtttggtgtt cggcggaggg accaagctga 2220

ccgtcctagc ctgacacaca cagccctccc cagactgtgg gatgaggccc tgtgcccgca 2280

gtcacatgtg gaatatcaag acacacacat ctatgacaat cacatagagt actcacgcta 2340

cgtcagtaga cgtcagtctg ccctgactca gcctgcctcc gtgtctgggt ctcctggaca 2400

gtcgatcacc atctcctgcg ggggaaacaa cattggaagt aaaaatgtgc actggtacca 2460

acagcaccca ggcaaagccc ccaaactcat gatttatagg gatagcaacc ggccctctgg 2520

ggtttctaat cgcttctctg gctccaagtc tggcaacacg gcctccctga ccatctctgg 2580

gctccaggct gaggacgagg ctgattatta ctgccagtcc tatgacagga gtggttgggt 2640

gttcggcgga gggaccaagc tgaccgtcct agcctgacac acacagccct ccccagactg 2700

tgggatgagg ccctgtgccc gcagtcacat gtggaatatc aagacacaca catctatgac 2760

aatcactacg ctgtcttagt gtagattcga tcacgtcagt ctgccctgac tcagcctgcc 2820

tccgtgtctg ggtctcctgg acagtcgatc accatctcct gctctggaga agcattgcca 2880

aaaaaatatg cttattggta ccaacagcac ccaggcaaag cccccaaact catgatttat 2940

aaagacagcg agaggccctc aggggtttct aatcgcttct ctggctccaa gtctggcaac 3000

acggcctccc tgaccatctc tgggctccag gctgaggacg aggctgatta ttactgcaac 3060

tcttttacaa gcaccaccaa ttatgtctta ttcggcggag ggaccaagct gaccgtccta 3120

gcctgacaca cacagccctc cccagactgt gggatgaggc cctgtgcccg cagtcacatg 3180

tggaatatca agacacacac atctatgaca atcactgacg tatgttgtga gtagtacagt 3240

atatacagtc tgccctgact cagcctgcct ccgtgtctgg gtctcctgga cagtcgatca 3300

ccatctcctg cgggggaaac aacattggaa gtaaaagtgt gcactggtac caacagcacc 3360

caggcaaagc ccccaaactc atgatttatt atgatagcga ccggccctca ggggtttcta 3420

atcgcttctc tggctccaag tctggcaaca cggcctccct gaccatctct gggctccagg 3480

ctgaggacga ggctgattat tactgccagg tgtgggatga taatagtgat catcttatat 3540

tcggcggagg gaccaagctg accgtcctag cctgacacac acagccctcc ccagactgtg 3600

ggatgaggcc ctgtgcccgc agtcacatgt ggaatatcaa gacacacaca tctatgacaa 3660

tcacagtata cataagtcga gagaagtgct acgacagtct gccctgactc agcctgcctc 3720

cgtgtctggg tctcctggac agtcgatcac catctcctgc tctggagatg cattgccaaa 3780

gcaatatgct tattggtacc aacagcaccc aggcaaagcc cccaaactca tgatttatga 3840

tgatagcgac cggccctcag gggtttctaa tcgcttctct ggctccaagt ctggcaacac 3900

ggcctccctg accatctctg ggctccaggc tgaggacgag gctgattatt actgcagctc 3960

atatgcaggc agcaacaatt gggtgttcgg cggagggacc aagctgaccg tcctagcctg 4020

acacacacag ccctccccag actgtgggat gaggccctgt gcccgcagtc acatgtggaa 4080

tatcaagaca cacacatcta tgacaatcac cgtacagtca cgtactcaga ctacgctcta 4140

cagtctgccc tgactcagcc tgcctccgtg tctgggtctc ctggacagtc gatcaccatc 4200

tcctgcaccc taagcagtga gcacagcacc tacaccatcg aatggtacca acagcaccca 4260

ggcaaagccc ccaaactcat gatttatgtt aagagtgatg gcagccacag caagggggac 4320

ggggtttcta atcgcttctc tggctccaag tctggcaaca cggcctccct gaccatctct 4380

gggctccagg ctgaggacga ggctgattat tactgcggag agagccacac gattgatggc 4440

caagtcggtg tggtattcgg cggagggacc aagctgaccg tcctagcctg acacacacag 4500

ccctccccag actgtgggat gaggccctgt gcccgcagtc acatgtggaa tatcaagaca 4560

cacacatcta tgacaatcac tatatataca tatgctagta tcacgcgaga cagtctgccc 4620

tgactcagcc tgcctccgtg tctgggtctc ctggacagtc gatcaccatc tcctgcactc 4680

tgagcagtgg gcacagcagc tacgccatcg catggtacca acagcaccca ggcaaagccc 4740

ccaaactcat gatttatctt aacagtgatg gcagccacag caagggggac ggggtttcta 4800

atcgcttctc tggctccaag tctggcaaca cggcctccct gaccatctct gggctccagg 4860

ctgaggacga ggctgattat tactgcagct catatgcagg caacaacaat ttgggggatg 4920

tgcttttcgg cggagggacc aagctgaccg tcctagcctg acacacacag ccctccccag 4980

actgtgggat gaggccctgt gcccgcagtc acatgtggaa tatcaagaca cacacatcta 5040

tgacaatcac tgagtcagta tgtagtgtga tgtcacacga cagtctgccc tgactcagcc 5100

tgcctccgtg tctgggtctc ctggacagtc gatcaccatc tcctgcacct tgcgcagtgg 5160

catcaatgtt ggtacctaca ggatatactg gtaccaacag cacccaggca aagcccccaa 5220

actcatgatt tattacaaat cagactcaga taagcagcag ggctctgggg tttctaatcg 5280

cttctctggc tccaagtctg gcaacacggc ctccctgacc atctctgggc tccaggctga 5340

ggacgaggct gattattact gcgcagcatg ggatgacagc ctgaatgtgg tattcggcgg 5400

agggaccaag ctgaccgtcc tagcctgaca cacacagccc tccccagact gtgggatgag 5460

gccctgtgcc cgcagtcaca tgtggaatat caagacacac acatctatga caatcacaca 5520

tgacgacacg acagctcacg tctcatccag tctgccctga ctcagcctgc ctccgtgtct 5580

gggtctcctg gacagtcgat caccatctcc tgcacccgca gcagtggcag cattgccagc 5640

aactatgtgc agtggtacca acagcaccca ggcaaagccc ccaaactcat gatttatgag 5700

gataaccaaa gaccctctgg ggtttctaat cgcttctctg gctccaagtc tggcaacacg 5760

gcctccctga ccatctctgg gctccaggct gaggacgagg ctgattatta ctgccagtcc 5820

tatgacagca gcctgaggag agtcttcggc ggagggacca agctgaccgt cctagcctga 5880

cacacacagc cctccccaga ctgtgggatg aggccctgtg cccgcagtca catgtggaat 5940

atcaagacac acacatctat gacaatcaca gcgactagca gtagtgatca gtgacacacc 6000

agtctgccct gactcagcct gcctccgtgt ctgggtctcc tggacagtcg atcaccatct 6060

cctgcggctc cagcactgga gctgtcacca gtggtcatta tccctactgg taccaacagc 6120

acccaggcaa agcccccaaa ctcatgattt atgatacaag caacaaacac tccggggttt 6180

ctaatcgctt ctctggctcc aagtctggca acacggcctc cctgaccatc tctgggctcc 6240

aggctgagga cgaggctgat tattactgcg caacatggga tgacagcctg aatgagggag 6300

tgttcggcgg agggaccaag ctgaccgtcc tagcctgaca cacacagccc tccccagact 6360

gtgggatgag gccctgtgcc cgcagtcaca tgtggaatat caagacacac acatctatga 6420

caatcacatc tactgaccac gtagatccac gtgtcgccag tctgccctga ctcagcctgc 6480

ctccgtgtct gggtctcctg gacagtcgat caccatctcc tgcaccctga gcagcggcta 6540

cagtaattat aaagtggact ggtaccaaca gcacccaggc aaagccccca aactcatgat 6600

ttatgtgggc actggtggga ttgtgggatc caagggggat ggggtttcta atcgcttctc 6660

tggctccaag tctggcaaca cggcctccct gaccatctct gggctccagg ctgaggacga 6720

ggctgattat tactgcgggg cagaccatgg cagtgggagc aacttcgtgg tggtattcgg 6780

cggagggacc aagctgaccg tcctagcctg acacacacag ccctccccag actgtgggat 6840

gaggccctgt gcccgcagtc acatgtggaa tatcaagaca cacacatcta tgacaatcac 6900

gtttaaacaa gcttcgaagg gcccgctagc gtcgacgttt aaacagcact gtagatcaga 6960

cacgatatcg cgagcagtct gccctgactc agcctgcctc cgtgtctggg tctcctggac 7020

agtcgatcac catctcctgc tctggaagca gctccaacat cggaaataat gctgtaaact 7080

ggtaccaaca gcacccaggc aaagccccca aactcatgat ttattatgat gatctgctgc 7140

cctcaggggt ttctaatcgc ttctctggct ccaagtctgg caacacggcc tccctgacca 7200

tctctgggct ccaggctgag gacgaggctg attattactg cggaacatgg gatggcagcc 7260

tgaatggtgg gaggctgttc ggcggaggga ccaagctgac cgtcctagcc tgacacacac 7320

agccctcccc agactgtggg atgaggccct gtgcccgcag tcacatgtgg aatatcaaga 7380

cacacacatc tatgacaatc actgatacgt ctcatagtag tgcgagagat accagtctgc 7440

cctgactcag cctgcctccg tgtctgggtc tcctggacag tcgatcacca tctcctgctc 7500

tggaagcagc tccaacatcg gaagtaatac tgtaaactgg taccaacagc acccaggcaa 7560

agcccccaaa ctcatgattt atagtaataa tcagcggccc tcaggggttt ctaatcgctt 7620

ctctggctcc aagtctggca acacggcctc cctgaccatc tctgggctcc aggctgagga 7680

cgaggctgat tattactgcg cagcatggga tgacagcctg agtggtgtgg ttttcggcgg 7740

agggaccaag ctgaccgtcc tagcctgaca cacacagccc tccccagact gtgggatgag 7800

gccctgtgcc cgcagtcaca tgtggaatat caagacacac acatctatga caatcactct 7860

acgtagctgt actactcacg actacagcag tctgccctga ctcagcctgc ctccgtgtct 7920

gggtctcctg gacagtcgat caccatctcc tgctctggaa gcagctccga catggggaat 7980

tatgcggtat cctggtacca acagcaccca ggcaaagccc ccaaactcat gatttatgat 8040

gtcagtaagc ggccctcagg ggtttctaat cgcttctctg gctccaagtc tggcaacacg 8100

gcctccctga ccatctctgg gctccaggct gaggacgagg ctgattatta ctgcagctca 8160

tatgaagaca gcaacaattt gggggtattc ggcggaggga ccaagctgac cgtcctagcc 8220

tgacacacac agccctcccc agactgtggg atgaggccct gtgcccgcag tcacatgtgg 8280

aatatcaaga cacacacatc tatgacaatc actagagacg agtcgtcgct cgacatacgc 8340

gtcagtctgc cctgactcag cctgcctccg tgtctgggtc tcctggacag tcgatcacca 8400

tctcctgcac tggaaccagc agtgacgttg gtagttataa ccgtgtctcc tggtaccaac 8460

agcacccagg caaagccccc aaactcatga tttatgaggt cagtaatcgg ccctcagggg 8520

tttctaatcg cttctctggc tccaagtctg gcaacacggc ctccctgacc atctctgggc 8580

tccaggctga ggacgaggct gattattact gcgcagcatg ggatggcagc ccggccccga 8640

gtgactttta tgtcttcggc ggagggacca agctgaccgt cctagcctga cacacacagc 8700

cctccccaga ctgtgggatg aggccctgtg cccgcagtca catgtggaat atcaagacac 8760

acacatctat gacaatcaca gactatacta gcgtcgtcta gtacgctatc agtctgccct 8820

gactcagcct gcctccgtgt ctgggtctcc tggacagtcg atcaccatct cctgctctgg 8880

agataaattg ggggataaat atgcttgctg gtaccaacag cacccaggca aagcccccaa 8940

actcatgatt tatcaagata gcaagcggcc ctcaggggtt tctaatcgct tctctggctc 9000

caagtctggc aacacggcct ccctgaccat ctctgggctc caggctgagg acgaggctga 9060

ttattactgc cagtcctttg acaacagcct gtgccagtcc tttgacaaca gcctgaatta 9120

tgtgcttttc ggcggaggga ccaagctgac cgtcctagcc tgacacacac agccctcccc 9180

agactgtggg atgaggccct gtgcccgcag tcacatgtgg aatatcaaga cacacacatc 9240

tatgacaatc accgacgtga cttacacaca cttacagatc gtcagtctgc cctgactcag 9300

cctgcctccg tgtctgggtc tcctggacag tcgatcacca tctcctgctc tggagatgca 9360

ttgccaaaaa aatatgctta ttggtaccaa cagcacccag gcaaagcccc caaactcatg 9420

atttatgagg acagcaaacg accctccggg gtttctaatc gcttctctgg ctccaagtct 9480

ggcaacacgg cctccctgac catctctggg ctccaggctg aggacgaggc tgattattac 9540

tgcgcagcat gggatgacag cctgaatggt cccttttatg tcttcggcgg agggaccaag 9600

ctgaccgtcc tagcctgaca cacacagccc tccccagact gtgggatgag gccctgtgcc 9660

cgcagtcaca tgtggaatat caagacacac acatctatga caatcactcg cactagttct 9720

agcgacttct atactatcag tctgccctga ctcagcctgc ctccgtgtct gggtctcctg 9780

gacagtcgat caccatctcc tgccaaggag acagcctcag aagctattat gcaagctggt 9840

accaacagca cccaggcaaa gcccccaaac tcatgattta tggtaaaaac aaccggccct 9900

caggggtttc taatcgcttc tctggctcca agtctggcaa cacggcctcc ctgaccatct 9960

ctgggctcca ggctgaggac gaggctgatt attactgcca gtcctatgac agcagcctga 10020

gtggtgtggt attcggcgga gggaccaagc tgaccgtcct agcctgacac acacagccct 10080

ccccagactg tgggatgagg ccctgtgccc gcagtcacat gtggaatatc aagacacaca 10140

catctatgac aatcacacgc gatcgaacta gcagtaagct cacgtacagt ctgccctgac 10200

tcagcctgcc tccgtgtctg ggtctcctgg acagtcgatc accatctcct gctctggaga 10260

tgtactgggg gaaaattatg ctgactggta ccaacagcac ccaggcaaag cccccaaact 10320

catgatttat gaagatagtg agcggtaccc tggggtttct aatcgcttct ctggctccaa 10380

gtctggcaac acggcctccc tgaccatctc tgggctccag gctgaggacg aggctgatta 10440

ttactgcgga acatgggata gcagcctgag tggtgtggta ttcggcggag ggaccaagct 10500

gaccgtccta gcctgacaca cacagccctc cccagactgt gggatgaggc cctgtgcccg 10560

cagtcacatg tggaatatca agacacacac atctatgaca atcaccgatc gtatacgcag 10620

tacgacgcgt atacacagtc tgccctgact cagcctgcct ccgtgtctgg gtctcctgga 10680

cagtcgatca ccatctcctg ctcaggagat gtactggcaa aaaaatatgc tcggtggtac 10740

caacagcacc caggcaaagc ccccaaactc atgatttata ggaaaaacag ccaactctca 10800

ggggtttcta atcgcttctc tggctccaag tctggcaaca cggcctccct gaccatctct 10860

gggctccagg ctgaggacga ggctgattat tactgctact ctgcggctga caacaatctt 10920

gtggtattcg gcggagggac caagctgacc gtcctagcct gacacacaca gccctcccca 10980

gactgtggga tgaggccctg tgcccgcagt cacatgtgga atatcaagac acacacatct 11040

atgacaatca cctatagcgt atacgtcatc atagtcgcat acagtctgcc ctgactcagc 11100

ctgcctccgt gtctgggtct cctggacagt cgatcaccat ctcctgcact ctgagcagtg 11160

ggcacagtag ctacatcatc gcatggtacc aacagcaccc aggcaaagcc cccaaactca 11220

tgatttatct tgaaggtagt ggaagctaca acaaggggag cggggtttct aatcgcttct 11280

ctggctccaa gtctggcaac acggcctccc tgaccatctc tgggctccag gctgaggacg 11340

aggctgatta ttactgcgag acctgggaca gtaacactca tgtggtattc ggcggaggga 11400

ccaagctgac cgtcctagcc tgacacacac agccctcccc agactgtggg atgaggccct 11460

gtgcccgcag tcacatgtgg aatatcaaga cacacacatc tatgacaatc actcgatagt 11520

gatcgctgcg tatctgacgt cacagtctgc cctgactcag cctgcctccg tgtctgggtc 11580

tcctggacag tcgatcacca tctcctgcac cttgcccagt gacatcaatg ttggtagcta 11640

caacatatac tggtaccaac agcacccagg caaagccccc aaactcatga tttattacta 11700

ctcagactca gataagggcc agggctctgg ggtttctaat cgcttctctg gctccaagtc 11760

tggcaacacg gcctccctga ccatctctgg gctccaggct gaggacgagg ctgattatta 11820

ctgccactca tatgcaggcg gcgacacttt cgatgtggtg ttcggcggag ggaccaagct 11880

gaccgtccta gcctgacaca cacagccctc cccagactgt gggatgaggc cctgtgcccg 11940

cagtcacatg tggaatatca agacacacac atctatgaca atcactgtcg tcgcaacaca 12000

tacgcacagt cgtgccagtc tgccctgact cagcctgcct ccgtgtctgg gtctcctgga 12060

cagtcgatca ccatctcctg catgctgagc agtggcttca gtgttgggga cttctggata 12120

aggtggtacc aacagcaccc aggcaaagcc cccaaactca tgatttatta ccactcagac 12180

tccaataagg gccaaggctc tggggtttct aatcgcttct ctggctccaa gtctggcaac 12240

acggcctccc tgaccatctc tgggctccag gctgaggacg aggctgatta ttactgcggt 12300

acatggcaca gcaactctaa gactcatgtg gtattcggcg gagggaccaa gctgaccgtc 12360

ctagcctgac acacacagcc ctccccagac tgtgggatga ggccctgtgc ccgcagtcac 12420

atgtggaata tcaagacaca cacatctatg acaatcacac tcatctacac tcgcgcacag 12480

agcgtcacca gtctgccctg actcagcctg cctccgtgtc tgggtctcct ggacagtcga 12540

tcaccatctc ctgcgcttcc agcactggag cagtcaccag tggttactat ccaaactggt 12600

accaacagca cccaggcaaa gcccccaaac tcatgattta tagtacaagc aacaaacact 12660

ccggggtttc taatcgcttc tctggctcca agtctggcaa cacggcctcc ctgaccatct 12720

ctgggctcca ggctgaggac gaggctgatt attactgcag ctcatataca atcaccgaca 12780

tcgtggtctt cggcggaggg accaagctga ccgtcctagc ctgacacaca cagccctccc 12840

cagactgtgg gatgaggccc tgtgcccgca gtcacatgtg gaatatcaag acacacacat 12900

ctatgacaat cacagtgtat gtcatagata gacatatagt cgccagtctg ccctgactca 12960

gcctgcctcc gtgtctgggt ctcctggaca gtcgatcacc atctcctgcg gcttgagctc 13020

tggctcagtc tctactagtt actaccccag ctggtaccaa cagcacccag gcaaagcccc 13080

caaactcatg atttatagca caaacactcg ctcttctggg gtttctaatc gcttctctgg 13140

ctccaagtct ggcaacacgg cctccctgac catctctggg ctccaggctg aggacgaggc 13200

tgattattac tgcgtgctgt atatgggtag tggcatttct gtggtattcg gcggagggac 13260

caagctgacc gtcctagcct gacacacaca gccctcccca gactgtggga tgaggccctg 13320

tgcccgcagt cacatgtgga atatcaagac acacacatct atgacaatca ccatactcta 13380

ccgacactat ccgagacgcg ccagtctgcc ctgactcagc ctgcctccgt gtctgggtct 13440

cctggacagt cgatcaccat ctcctgcact gggaacagca acaatgttgg caaccaagga 13500

gcagcttggt accaacagca cccaggcaaa gcccccaaac tcatgattta taggaataac 13560

aaccggccct caggggtttc taatcgcttc tctggctcca agtctggcaa cacggcctcc 13620

ctgaccatct ctgggctcca ggctgaggac gaggctgatt attactgcgc agcatgggat 13680

gacagcctga gtgcccatgt ggtattcggc ggagggacca agctgaccgt cctagcctga 13740

cacacacagc cctccccaga ctgtgggatg aggccctgtg cccgcagtca catgtggaat 13800

atcaagacac acacatctat gacaatcac 13829

<210> 143
<211> 25655
<212> DNA
<213> Artificial Sequence

<220>
<223> Synthetic DNA

<400> 143
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgcggc cgcgggtatc acgtggggca 420

aatcgagagc taccattggc tatgggaggg agaaaatgag gcgtgctact tcctcgttcc 480

tgcagtgcgt gtcgaggtgc cgtgttggtc ggatctcagt ggcagagcag gatggtgagc 540

ggtggccttg gggccgctcc ctcactctgc tgggctggtg cggtggctcc gggaccggac 600

tttcagtggt gtttggtgtg gtgctcactg tcctttggtg tcactgtgtg gtgaattccc 660

tcagctgggc ggtgagagcc catgctgtgt cctggtagga gtagtctggg ctgcagtgcg 720

ggctctttgt gcgtgttggg ggcggttccc tggaagctaa ttctggaaga aatcccggct 780

gcctctgtaa cagttgttgg cgatgtcaaa caccctggag ctgctgactg gctctgtacc 840

attgagtgat gcagtttaaa cctctctctt tcctcctgct gtgtgtgaag ctggtgttag 900

tgctaggaga cctgcacatc cctcaccgat gcagtggttt accagcaaag ttcaagagcc 960

tcctggttcc agggaagatt cagcatattt tgtgtacagg aaacctctgc accaaggaga 1020

gctatgacta cctcaggact ttggctggag atgttcacgt tgttaagggg gactctgagg 1080

taatgttctt gctgctgtga attcctcagt cctccatctt acaagccgta gttgtcccac 1140

tgaaagctgg gaaacccttc ggtgtttgca attcctgagc gctaatgtgt gggggaagga 1200

tgtaggaaca aataaggctc aagagacaac aacttcctag gctcctctta ttgcccttgt 1260

ggtaagggag gacttggtgt gtagtggtaa tacatgcact caacttatta agttctcata 1320

gctgctgtga agctgctgca gtccctcaga tccctgcagg aagaggggac tgactgactg 1380

ttgaatagcc ttgaagctcc tgctctgcat gtggttccgg tcaggctcaa gatcccagtt 1440

cttagctctt cagttgctct tctgggggta tagtactagc atctgggtca ctgcagatgg 1500

acagagactg tgcttgggta gcaaaaggca gacttacgtt gtgaaacctt cattgtgttg 1560

gtgaggttct ttgatttcta ccttgtgtat ctctcttcct cagagtctga attatcctga 1620

acagaaggtt gtaactgttg ggcagttcag aattgggttg attcatggcc atcaagtcat 1680

tccctggggt gatgtggcaa gcttggcatt gctgcagagg cagctggatg tagacattct 1740

tatctcagga catacacaca gatttgaagc atttgagcat gaaaataagt tctacatcaa 1800

cccaggatca gctacaggag cctacactgg tttacaaacg tgagtacaat gagccttgtg 1860

tttgtcttta agacagatgt ctgtccttaa gagtgcagcc catgtgttga agagcactgg 1920

tgagatgcag tgccatgcag gccatctctc cctttcccag agaacacggc ctacctctct 1980

ctgttcataa ggtcttggtt aacttatgag ttggccagca cagcctggca ctgatgtgtc 2040

atgatcataa ccctgagccc acgctctgct atgctgttgc ttcttagagt tgcattgcct 2100

tgaggtgtat tccagaagct gtgtgctgag ctcttgttgg gaaagaggcc ctcagggcac 2160

tgccaacatc tgaggcttgc aggagctgga acattcagcc ctgaggctgg ctctggccaa 2220

ctgcagctct gagtctgcag tcagtgcctc tgagctgtga tgcttctcag ataactgcag 2280

ccctttgcaa tgtaatgttt tataatatat ttccatagga catcttggta agaggtgtaa 2340

gctaatcagg gtgattgaca aagtatgttt tctttccagg aacgtcattc cttcatttgt 2400

actgatggat atccaggctt ccacagttgt gacttatgta taccagctaa ttgaggacga 2460

tgtgaaagta gaaagaattg agttcaagaa atactgacac gtgtttgaat ctgcctgctg 2520

acttctcact gcctctcatt ctttctctcc tagttaaagc tgaatccggc cacgtggggg 2580

tgctgctgca gcggcgtgta actgtgagct tgggaagttc tcaactaggg cttagagcag 2640

gagaaactga ttaacagaaa ttgccttatt aaatgtatat atttcctgtg tctaagaacg 2700

aggtgctgtt gttcctgact tgcggagctg cagctagtgg tagcttggaa tttaacctct 2760

cctgtaaaat gagcatagaa taaaacagta ttttcacatt tctggatcct gttaattgtt 2820

tgttaattaa gaggacagga attcagtgtc agggggctgc tctttgcttc ccaatgtgta 2880

ccactgaagc ctctctggat ttatcagaga tactcaatgg actcttaaca gcactgtggc 2940

acctgacctc ctgctgcctt cagcctccct tcctggctgg tgtgcttatg ttgaggagct 3000

gtgggaggct ggtgcagttc tgtgggatga ggtaaggcca gctgaatagc ctgggggctg 3060

gtcagggggc ttagtgagag gagcagggct ggggaatgtt gtgaacagca ggtggtgcca 3120

ctgctattct gaggaaggag gcagcttctg caaatgcttt gcaatgcagg atgacaaagt 3180

gcttctgcta gccctaggct accgttcgta taatgtatac tatacgaagt tattttattt 3240

gtgaaatttg tgatgctatt gctttatttg taaccattat aagctgcaat aaacaagttg 3300

gggtgggcga agaactccag catgagatcc ccgcgctgga ggatcatcca gccggcgtcc 3360

cggaaaacga ttccgaagcc caacctttca tagaaggcgg cggtggaatc gaaatctcgt 3420

gatggcaggt tgggcgtcgc ttggtcggtc atttcgaacc ccagagtccc gctcagaaga 3480

actcgtcaag aaggcgatag aaggcgatgc gctgcgaatc gggagcggcg ataccgtaaa 3540

gcacgaggaa gcggtcagcc cattcgccgc caagctcttc agcaatatca cgggtagcca 3600

acgctatgtc ctgatagcgg tccgccacac ccagccggcc acagtcgatg aatccagaaa 3660

agcggccatt ttccaccatg atattcggca agcaggcatc gccatgggtc acgacgagat 3720

cctcgccgtc gggcatgcgc gccttgagcc tggcgaacag ttcggctggc gcgagcccct 3780

gatgctcttc gtccagatca tcctgatcga caagaccggc ttccatccga gtacgtgctc 3840

gctcgatgcg atgtttcgct tggtggtcga atgggcaggt agccggatca agcgtatgca 3900

gccgccgcat tgcatcagcc atgatggata ctttctcggc aggagcaagg tgagatgaca 3960

ggagatcctg ccccggcact tcgcccaata gcagccagtc ccttcccgct tcagtgacaa 4020

cgtcgagcac agctgcgcaa ggaacgcccg tcgtggccag ccacgatagc cgcgctgcct 4080

cgtcctgcag ttcattcagg gcaccggaca ggtcggtctt gacaaaaaga accgggcgcc 4140

cctgcgctga cagccggaac acggcggcat cagagcagcc gattgtctgt tgtgcccagt 4200

catagccgaa tagcctctcc acccaagcgg ccggagaacc tgcgtgcaat ccatcttgtt 4260

caatcatgcg aaacgatcct catcctgtct cttgatcaga tccgaaaatg gatatacaag 4320

ctcccgggag ctttttgcaa aagcctaggc ctccaaaaaa gcctcctcac tacttctgga 4380

atagctcaga ggcagaggcg gcctcggcct ctgcataaat aaaaaaaatt agtcagccat 4440

ggggcggaga atgggcggaa ctgggcggag ttaggggcgg gatgggcgga gttaggggcg 4500

ggactatggt tgctgactaa ttgagatgca tgctttgcat acttctgcct gctggggagc 4560

ctggggactt tccacacctg gttgctgact aattgagatg catgctttgc atacttctgc 4620

ctgctgggga gcctggggac tttccacacc ctaaataact tcgtataatg tatactatac 4680

gaacggtagc gttaaccggt cgacatgcaa gcttgctagc gtttaaacac gagtgcgtac 4740

gctcgacaag acgcactcca gtctgccctg actcagcctg cctccgtgtc tgggtctcct 4800

ggacagtcga tcaccatctc ctgctctgga agcagctcca acatcggaag taattatgta 4860

tactggtacc aacagcaccc aggcaaagcc cccaaactca tgatttatag gaataatcag 4920

cggccctcag gggtttctaa tcgcttctct ggctccaagt ctggcaacac ggcctccctg 4980

accatctctg ggctccaggc tgaggacgag gctgattatt actgcgcagc atgggatgac 5040

agcctgagtg gtcattgggt gttcggcgga gggaccaagc tgaccgtcct agcctgacac 5100

acacagccct ccccagactg tgggatgagg ccctgtgccc gcagtcacat gtggaatatc 5160

aagacacaca catctatgac aatcaccgtg tctctactcg cgtgtctctc tatgcgcagt 5220

ctgccctgac tcagcctgcc tccgtgtctg ggtctcctgg acagtcgatc accatctcct 5280

gcactgggag cagctccaac atcggggcag gttatgatgt acactggtac caacagcacc 5340

caggcaaagc ccccaaactc atgatttatg gtaacagcaa tcggccctca ggggtttcta 5400

atcgcttctc tggctccaag tctggcaaca cggcctccct gaccatctct gggctccagg 5460

ctgaggacga ggctgattat tactgccagt cctatgacag cagcctgagt ggttctgtgg 5520

tattcggcgg agggaccaag ctgaccgtcc tagcctgaca cacacagccc tccccagact 5580

gtgggatgag gccctgtgcc cgcagtcaca tgtggaatat caagacacac acatctatga 5640

caatcacata cgacgtatca cgtactacgt ctagtaccag tctgccctga ctcagcctgc 5700

ctccgtgtct gggtctcctg gacagtcgat caccatctcc tgctctggaa gcagctccaa 5760

cattgggaat aattatgtat cctggtacca acagcaccca ggcaaagccc ccaaactcat 5820

gatttatgac aataataagc gaccctcagg ggtttctaat cgcttctctg gctccaagtc 5880

tggcaacacg gcctccctga ccatctctgg gctccaggct gaggacgagg ctgattatta 5940

ctgcggaaca tgggatagca gcctgagtgc tggtgctgtg ttcggcggag ggaccaagct 6000

gaccgtccta gcctgacaca cacagccctc cccagactgt gggatgaggc cctgtgcccg 6060

cagtcacatg tggaatatca agacacacac atctatgaca atcaccgtag actagtacga 6120

gtatgtactc tcgtgcagtc tgccctgact cagcctgcct ccgtgtctgg gtctcctgga 6180

cagtcgatca ccatctcctg cactgggagc agctccaaca ttgggcgggg ttatgttgta 6240

cattggtacc aacagcaccc aggcaaagcc cccaaactca tgatttatga aaataataag 6300

cgaccctcag gggtttctaa tcgcttctct ggctccaagt ctggcaacac ggcctccctg 6360

accatctctg ggctccaggc tgaggacgag gctgattatt actgcagctc atatacaagc 6420

agcggcactc ctgtggtatt cggcggaggg accaagctga ccgtcctagc ctgacacaca 6480

cagccctccc cagactgtgg gatgaggccc tgtgcccgca gtcacatgtg gaatatcaag 6540

acacacacat ctatgacaat cacacgcgag tatactacta tgtactgtac agtcagtctg 6600

ccctgactca gcctgcctcc gtgtctgggt ctcctggaca gtcgatcacc atctcctgca 6660

ctggaaccag cagtgatgtt gggagttata accttgtctc ctggtaccaa cagcacccag 6720

gcaaagcccc caaactcatg atttatgagg gcagtaagcg gccctcaggg gtttctaatc 6780

gcttctctgg ctccaagtct ggcaacacgg cctccctgac catctctggg ctccaggctg 6840

aggacgaggc tgattattac tgccagtcct atgacagcag cctgagtggt tgccagtcct 6900

atgacagcag cctgagtggt ttggtgttcg gcggagggac caagctgacc gtcctagcct 6960

gacacacaca gccctcccca gactgtggga tgaggccctg tgcccgcagt cacatgtgga 7020

atatcaagac acacacatct atgacaatca catagagtac tcacgctacg tcagtagacg 7080

tcagtctgcc ctgactcagc ctgcctccgt gtctgggtct cctggacagt cgatcaccat 7140

ctcctgcggg ggaaacaaca ttggaagtaa aaatgtgcac tggtaccaac agcacccagg 7200

caaagccccc aaactcatga tttataggga tagcaaccgg ccctctgggg tttctaatcg 7260

cttctctggc tccaagtctg gcaacacggc ctccctgacc atctctgggc tccaggctga 7320

ggacgaggct gattattact gccagtccta tgacaggagt ggttgggtgt tcggcggagg 7380

gaccaagctg accgtcctag cctgacacac acagccctcc ccagactgtg ggatgaggcc 7440

ctgtgcccgc agtcacatgt ggaatatcaa gacacacaca tctatgacaa tcactacgct 7500

gtcttagtgt agattcgatc acgtcagtct gccctgactc agcctgcctc cgtgtctggg 7560

tctcctggac agtcgatcac catctcctgc tctggagaag cattgccaaa aaaatatgct 7620

tattggtacc aacagcaccc aggcaaagcc cccaaactca tgatttataa agacagcgag 7680

aggccctcag gggtttctaa tcgcttctct ggctccaagt ctggcaacac ggcctccctg 7740

accatctctg ggctccaggc tgaggacgag gctgattatt actgcaactc ttttacaagc 7800

accaccaatt atgtcttatt cggcggaggg accaagctga ccgtcctagc ctgacacaca 7860

cagccctccc cagactgtgg gatgaggccc tgtgcccgca gtcacatgtg gaatatcaag 7920

acacacacat ctatgacaat cactgacgta tgttgtgagt agtacagtat atacagtctg 7980

ccctgactca gcctgcctcc gtgtctgggt ctcctggaca gtcgatcacc atctcctgcg 8040

ggggaaacaa cattggaagt aaaagtgtgc actggtacca acagcaccca ggcaaagccc 8100

ccaaactcat gatttattat gatagcgacc ggccctcagg ggtttctaat cgcttctctg 8160

gctccaagtc tggcaacacg gcctccctga ccatctctgg gctccaggct gaggacgagg 8220

ctgattatta ctgccaggtg tgggatgata atagtgatca tcttatattc ggcggaggga 8280

ccaagctgac cgtcctagcc tgacacacac agccctcccc agactgtggg atgaggccct 8340

gtgcccgcag tcacatgtgg aatatcaaga cacacacatc tatgacaatc acagtataca 8400

taagtcgaga gaagtgctac gacagtctgc cctgactcag cctgcctccg tgtctgggtc 8460

tcctggacag tcgatcacca tctcctgctc tggagatgca ttgccaaagc aatatgctta 8520

ttggtaccaa cagcacccag gcaaagcccc caaactcatg atttatgatg atagcgaccg 8580

gccctcaggg gtttctaatc gcttctctgg ctccaagtct ggcaacacgg cctccctgac 8640

catctctggg ctccaggctg aggacgaggc tgattattac tgcagctcat atgcaggcag 8700

caacaattgg gtgttcggcg gagggaccaa gctgaccgtc ctagcctgac acacacagcc 8760

ctccccagac tgtgggatga ggccctgtgc ccgcagtcac atgtggaata tcaagacaca 8820

cacatctatg acaatcaccg tacagtcacg tactcagact acgctctaca gtctgccctg 8880

actcagcctg cctccgtgtc tgggtctcct ggacagtcga tcaccatctc ctgcacccta 8940

agcagtgagc acagcaccta caccatcgaa tggtaccaac agcacccagg caaagccccc 9000

aaactcatga tttatgttaa gagtgatggc agccacagca agggggacgg ggtttctaat 9060

cgcttctctg gctccaagtc tggcaacacg gcctccctga ccatctctgg gctccaggct 9120

gaggacgagg ctgattatta ctgcggagag agccacacga ttgatggcca agtcggtgtg 9180

gtattcggcg gagggaccaa gctgaccgtc ctagcctgac acacacagcc ctccccagac 9240

tgtgggatga ggccctgtgc ccgcagtcac atgtggaata tcaagacaca cacatctatg 9300

acaatcacta tatatacata tgctagtatc acgcgagaca gtctgccctg actcagcctg 9360

cctccgtgtc tgggtctcct ggacagtcga tcaccatctc ctgcactctg agcagtgggc 9420

acagcagcta cgccatcgca tggtaccaac agcacccagg caaagccccc aaactcatga 9480

tttatcttaa cagtgatggc agccacagca agggggacgg ggtttctaat cgcttctctg 9540

gctccaagtc tggcaacacg gcctccctga ccatctctgg gctccaggct gaggacgagg 9600

ctgattatta ctgcagctca tatgcaggca acaacaattt gggggatgtg cttttcggcg 9660

gagggaccaa gctgaccgtc ctagcctgac acacacagcc ctccccagac tgtgggatga 9720

ggccctgtgc ccgcagtcac atgtggaata tcaagacaca cacatctatg acaatcactg 9780

agtcagtatg tagtgtgatg tcacacgaca gtctgccctg actcagcctg cctccgtgtc 9840

tgggtctcct ggacagtcga tcaccatctc ctgcaccttg cgcagtggca tcaatgttgg 9900

tacctacagg atatactggt accaacagca cccaggcaaa gcccccaaac tcatgattta 9960

ttacaaatca gactcagata agcagcaggg ctctggggtt tctaatcgct tctctggctc 10020

caagtctggc aacacggcct ccctgaccat ctctgggctc caggctgagg acgaggctga 10080

ttattactgc gcagcatggg atgacagcct gaatgtggta ttcggcggag ggaccaagct 10140

gaccgtccta gcctgacaca cacagccctc cccagactgt gggatgaggc cctgtgcccg 10200

cagtcacatg tggaatatca agacacacac atctatgaca atcacacatg acgacacgac 10260

agctcacgtc tcatccagtc tgccctgact cagcctgcct ccgtgtctgg gtctcctgga 10320

cagtcgatca ccatctcctg cacccgcagc agtggcagca ttgccagcaa ctatgtgcag 10380

tggtaccaac agcacccagg caaagccccc aaactcatga tttatgagga taaccaaaga 10440

ccctctgggg tttctaatcg cttctctggc tccaagtctg gcaacacggc ctccctgacc 10500

atctctgggc tccaggctga ggacgaggct gattattact gccagtccta tgacagcagc 10560

ctgaggagag tcttcggcgg agggaccaag ctgaccgtcc tagcctgaca cacacagccc 10620

tccccagact gtgggatgag gccctgtgcc cgcagtcaca tgtggaatat caagacacac 10680

acatctatga caatcacagc gactagcagt agtgatcagt gacacaccag tctgccctga 10740

ctcagcctgc ctccgtgtct gggtctcctg gacagtcgat caccatctcc tgcggctcca 10800

gcactggagc tgtcaccagt ggtcattatc cctactggta ccaacagcac ccaggcaaag 10860

cccccaaact catgatttat gatacaagca acaaacactc cggggtttct aatcgcttct 10920

ctggctccaa gtctggcaac acggcctccc tgaccatctc tgggctccag gctgaggacg 10980

aggctgatta ttactgcgca acatgggatg acagcctgaa tgagggagtg ttcggcggag 11040

ggaccaagct gaccgtccta gcctgacaca cacagccctc cccagactgt gggatgaggc 11100

cctgtgcccg cagtcacatg tggaatatca agacacacac atctatgaca atcacatcta 11160

ctgaccacgt agatccacgt gtcgccagtc tgccctgact cagcctgcct ccgtgtctgg 11220

gtctcctgga cagtcgatca ccatctcctg caccctgagc agcggctaca gtaattataa 11280

agtggactgg taccaacagc acccaggcaa agcccccaaa ctcatgattt atgtgggcac 11340

tggtgggatt gtgggatcca agggggatgg ggtttctaat cgcttctctg gctccaagtc 11400

tggcaacacg gcctccctga ccatctctgg gctccaggct gaggacgagg ctgattatta 11460

ctgcggggca gaccatggca gtgggagcaa cttcgtggtg gtattcggcg gagggaccaa 11520

gctgaccgtc ctagcctgac acacacagcc ctccccagac tgtgggatga ggccctgtgc 11580

ccgcagtcac atgtggaata tcaagacaca cacatctatg acaatcacgt ttaaacaagc 11640

ttcgaagggc ccgctagcgt cgacgtttaa acagcactgt agatcagaca cgatatcgcg 11700

agcagtctgc cctgactcag cctgcctccg tgtctgggtc tcctggacag tcgatcacca 11760

tctcctgctc tggaagcagc tccaacatcg gaaataatgc tgtaaactgg taccaacagc 11820

acccaggcaa agcccccaaa ctcatgattt attatgatga tctgctgccc tcaggggttt 11880

ctaatcgctt ctctggctcc aagtctggca acacggcctc cctgaccatc tctgggctcc 11940

aggctgagga cgaggctgat tattactgcg gaacatggga tggcagcctg aatggtggga 12000

ggctgttcgg cggagggacc aagctgaccg tcctagcctg acacacacag ccctccccag 12060

actgtgggat gaggccctgt gcccgcagtc acatgtggaa tatcaagaca cacacatcta 12120

tgacaatcac tgatacgtct catagtagtg cgagagatac cagtctgccc tgactcagcc 12180

tgcctccgtg tctgggtctc ctggacagtc gatcaccatc tcctgctctg gaagcagctc 12240

caacatcgga agtaatactg taaactggta ccaacagcac ccaggcaaag cccccaaact 12300

catgatttat agtaataatc agcggccctc aggggtttct aatcgcttct ctggctccaa 12360

gtctggcaac acggcctccc tgaccatctc tgggctccag gctgaggacg aggctgatta 12420

ttactgcgca gcatgggatg acagcctgag tggtgtggtt ttcggcggag ggaccaagct 12480

gaccgtccta gcctgacaca cacagccctc cccagactgt gggatgaggc cctgtgcccg 12540

cagtcacatg tggaatatca agacacacac atctatgaca atcactctac gtagctgtac 12600

tactcacgac tacagcagtc tgccctgact cagcctgcct ccgtgtctgg gtctcctgga 12660

cagtcgatca ccatctcctg ctctggaagc agctccgaca tggggaatta tgcggtatcc 12720

tggtaccaac agcacccagg caaagccccc aaactcatga tttatgatgt cagtaagcgg 12780

ccctcagggg tttctaatcg cttctctggc tccaagtctg gcaacacggc ctccctgacc 12840

atctctgggc tccaggctga ggacgaggct gattattact gcagctcata tgaagacagc 12900

aacaatttgg gggtattcgg cggagggacc aagctgaccg tcctagcctg acacacacag 12960

ccctccccag actgtgggat gaggccctgt gcccgcagtc acatgtggaa tatcaagaca 13020

cacacatcta tgacaatcac tagagacgag tcgtcgctcg acatacgcgt cagtctgccc 13080

tgactcagcc tgcctccgtg tctgggtctc ctggacagtc gatcaccatc tcctgcactg 13140

gaaccagcag tgacgttggt agttataacc gtgtctcctg gtaccaacag cacccaggca 13200

aagcccccaa actcatgatt tatgaggtca gtaatcggcc ctcaggggtt tctaatcgct 13260

tctctggctc caagtctggc aacacggcct ccctgaccat ctctgggctc caggctgagg 13320

acgaggctga ttattactgc gcagcatggg atggcagccc ggccccgagt gacttttatg 13380

tcttcggcgg agggaccaag ctgaccgtcc tagcctgaca cacacagccc tccccagact 13440

gtgggatgag gccctgtgcc cgcagtcaca tgtggaatat caagacacac acatctatga 13500

caatcacaga ctatactagc gtcgtctagt acgctatcag tctgccctga ctcagcctgc 13560

ctccgtgtct gggtctcctg gacagtcgat caccatctcc tgctctggag ataaattggg 13620

ggataaatat gcttgctggt accaacagca cccaggcaaa gcccccaaac tcatgattta 13680

tcaagatagc aagcggccct caggggtttc taatcgcttc tctggctcca agtctggcaa 13740

cacggcctcc ctgaccatct ctgggctcca ggctgaggac gaggctgatt attactgcca 13800

gtcctttgac aacagcctgt gccagtcctt tgacaacagc ctgaattatg tgcttttcgg 13860

cggagggacc aagctgaccg tcctagcctg acacacacag ccctccccag actgtgggat 13920

gaggccctgt gcccgcagtc acatgtggaa tatcaagaca cacacatcta tgacaatcac 13980

cgacgtgact tacacacact tacagatcgt cagtctgccc tgactcagcc tgcctccgtg 14040

tctgggtctc ctggacagtc gatcaccatc tcctgctctg gagatgcatt gccaaaaaaa 14100

tatgcttatt ggtaccaaca gcacccaggc aaagccccca aactcatgat ttatgaggac 14160

agcaaacgac cctccggggt ttctaatcgc ttctctggct ccaagtctgg caacacggcc 14220

tccctgacca tctctgggct ccaggctgag gacgaggctg attattactg cgcagcatgg 14280

gatgacagcc tgaatggtcc cttttatgtc ttcggcggag ggaccaagct gaccgtccta 14340

gcctgacaca cacagccctc cccagactgt gggatgaggc cctgtgcccg cagtcacatg 14400

tggaatatca agacacacac atctatgaca atcactcgca ctagttctag cgacttctat 14460

actatcagtc tgccctgact cagcctgcct ccgtgtctgg gtctcctgga cagtcgatca 14520

ccatctcctg ccaaggagac agcctcagaa gctattatgc aagctggtac caacagcacc 14580

caggcaaagc ccccaaactc atgatttatg gtaaaaacaa ccggccctca ggggtttcta 14640

atcgcttctc tggctccaag tctggcaaca cggcctccct gaccatctct gggctccagg 14700

ctgaggacga ggctgattat tactgccagt cctatgacag cagcctgagt ggtgtggtat 14760

tcggcggagg gaccaagctg accgtcctag cctgacacac acagccctcc ccagactgtg 14820

ggatgaggcc ctgtgcccgc agtcacatgt ggaatatcaa gacacacaca tctatgacaa 14880

tcacacgcga tcgaactagc agtaagctca cgtacagtct gccctgactc agcctgcctc 14940

cgtgtctggg tctcctggac agtcgatcac catctcctgc tctggagatg tactggggga 15000

aaattatgct gactggtacc aacagcaccc aggcaaagcc cccaaactca tgatttatga 15060

agatagtgag cggtaccctg gggtttctaa tcgcttctct ggctccaagt ctggcaacac 15120

ggcctccctg accatctctg ggctccaggc tgaggacgag gctgattatt actgcggaac 15180

atgggatagc agcctgagtg gtgtggtatt cggcggaggg accaagctga ccgtcctagc 15240

ctgacacaca cagccctccc cagactgtgg gatgaggccc tgtgcccgca gtcacatgtg 15300

gaatatcaag acacacacat ctatgacaat caccgatcgt atacgcagta cgacgcgtat 15360

acacagtctg ccctgactca gcctgcctcc gtgtctgggt ctcctggaca gtcgatcacc 15420

atctcctgct caggagatgt actggcaaaa aaatatgctc ggtggtacca acagcaccca 15480

ggcaaagccc ccaaactcat gatttatagg aaaaacagcc aactctcagg ggtttctaat 15540
cgcttctctg gctccaagtc tggcaacacg gcctccctga ccatctctgg gctccaggct 15600
gaggacgagg ctgattatta ctgctactct gcggctgaca acaatcttgt ggtattcggc 15660
ggagggacca agctgaccgt cctagcctga cacacacagc cctccccaga ctgtgggatg 15720
aggccctgtg cccgcagtca catgtggaat atcaagacac acacatctat gacaatcacc 15780
tatagcgtat acgtcatcat agtcgcatac agtctgccct gactcagcct gcctccgtgt 15840
ctgggtctcc tggacagtcg atcaccatct cctgcactct gagcagtggg cacagtagct 15900
acatcatcgc atggtaccaa cagcacccag gcaaagcccc caaactcatg atttatcttg 15960
aaggtagtgg aagctacaac aaggggagcg gggtttctaa tcgcttctct ggctccaagt 16020
ctggcaacac ggcctccctg accatctctg ggctccaggc tgaggacgag gctgattatt 16080
actgcgagac ctgggacagt aacactcatg tggtattcgg cggagggacc aagctgaccg 16140
tcctagcctg acacacacag ccctccccag actgtgggat gaggccctgt gcccgcagtc 16200
acatgtggaa tatcaagaca cacacatcta tgacaatcac tcgatagtga tcgctgcgta 16260
tctgacgtca cagtctgccc tgactcagcc tgcctccgtg tctgggtctc ctggacagtc 16320
gatcaccatc tcctgcacct tgcccagtga catcaatgtt ggtagctaca acatatactg 16380
gtaccaacag cacccaggca aagcccccaa actcatgatt tattactact cagactcaga 16440
taagggccag ggctctgggg tttctaatcg cttctctggc tccaagtctg gcaacacggc 16500
ctccctgacc atctctgggc tccaggctga ggacgaggct gattattact gccactcata 16560
tgcaggcggc gacactttcg atgtggtgtt cggcggaggg accaagctga ccgtcctagc 16620
ctgacacaca cagccctccc cagactgtgg gatgaggccc tgtgcccgca gtcacatgtg 16680
gaatatcaag acacacacat ctatgacaat cactgtcgtc gcaacacata cgcacagtcg 16740
tgccagtctg ccctgactca gcctgcctcc gtgtctgggt ctcctggaca gtcgatcacc 16800
atctcctgca tgctgagcag tggcttcagt gttggggact tctggataag gtggtaccaa 16860
cagcacccag gcaaagcccc caaactcatg atttattacc actcagactc caataagggc 16920
caaggctctg gggtttctaa tcgcttctct ggctccaagt ctggcaacac ggcctccctg 16980
accatctctg ggctccaggc tgaggacgag gctgattatt actgcggtac atggcacagc 17040
aactctaaga ctcatgtggt attcggcgga gggaccaagc tgaccgtcct agcctgacac 17100
acacagccct ccccagactg tgggatgagg ccctgtgccc gcagtcacat gtggaatatc 17160
aagacacaca catctatgac aatcacactc atctacactc gcgcacagag cgtcaccagt 17220
ctgccctgac tcagcctgcc tccgtgtctg ggtctcctgg acagtcgatc accatctcct 17280
gcgcttccag cactggagca gtcaccagtg gttactatcc aaactggtac caacagcacc 17340

caggcaaagc ccccaaactc atgatttata gtacaagcaa caaacactcc ggggtttcta 17400

atcgcttctc tggctccaag tctggcaaca cggcctccct gaccatctct gggctccagg 17460

ctgaggacga ggctgattat tactgcagct catatacaat caccgacatc gtggtcttcg 17520

gcggagggac caagctgacc gtcctagcct gacacacaca gccctcccca gactgtggga 17580

tgaggccctg tgcccgcagt cacatgtgga atatcaagac acacacatct atgacaatca 17640

cagtgtatgt catagataga catatagtcg ccagtctgcc ctgactcagc ctgcctccgt 17700

gtctgggtct cctggacagt cgatcaccat ctcctgcggc ttgagctctg gctcagtctc 17760

tactagttac taccccagct ggtaccaaca gcacccaggc aaagccccca aactcatgat 17820

ttatagcaca aacactcgct cttctggggt ttctaatcgc ttctctggct ccaagtctgg 17880

caacacggcc tccctgacca tctctgggct ccaggctgag gacgaggctg attattactg 17940

cgtgctgtat atgggtagtg gcatttctgt ggtattcggc ggagggacca agctgaccgt 18000

cctagcctga cacacacagc cctccccaga ctgtgggatg aggccctgtg cccgcagtca 18060

catgtggaat atcaagacac acacatctat gacaatcacc atactctacc gacactatcc 18120

gagacgcgcc agtctgccct gactcagcct gcctccgtgt ctgggtctcc tggacagtcg 18180

atcaccatct cctgcactgg gaacagcaac aatgttggca accaaggagc agcttggtac 18240

caacagcacc caggcaaagc ccccaaactc atgatttata ggaataacaa ccggccctca 18300

ggggtttcta atcgcttctc tggctccaag tctggcaaca cggcctccct gaccatctct 18360

gggctccagg ctgaggacga ggctgattat tactgcgcag catgggatga cagcctgagt 18420

gcccatgtgg tattcggcgg agggaccaag ctgaccgtcc tagcctgaca cacacagccc 18480

tccccagact gtgggatgag gccctgtgcc cgcagtcaca tgtggaatat caagacacac 18540

acatctatga caatcacgtt taaacaagct tcgaagaatt ccgcggatgc ggcgcgcctc 18600

tagaatgcat cgatttaaat cgagtgaagg tcaccaagga tggagacttc agagctatct 18660

gggacacgtg ttttaacgtt tgatcagcct tgcagcaaaa cagtgttttg ctctattgaa 18720

cagtgttcaa caaaaacgat cagtggtctg gaacacatct cctacgaaga aaggttgcga 18780

gagctgggtc tggagaagag gaggctctgg ggcaacctcc ctgtggcctg tcagtgctta 18840

aaggaggctc aagagaaaga tggagagaga ctttatacac ggagctgtag caatatgaca 18900

aggagaaatg gtttaaaatg gaaagagggt agatttaggt tagctatcaa gaagaaattc 18960

ttcactcaga ggtgatgaag cactgtagca ggttgcccag agaagtattg gatgcttcct 19020

tcctggcagg gcccaaggcc agactggatg ggttctgggc agcctgttct ggtgggagat 19080

gtccctgccc gtggcacggc ttggaaatgg gtagcctttg aggtccctct cagcccaaat 19140

cattctatgt tcttgtggtt ccctgcattt cagcctttgc ctgttgactc tggtgccact 19200

gttggacaat tctactcagc tgtgtttcat ccctgcccca aagcagtaat cccatggata 19260

agcaatgctc tgaggccttc accaccatca gggctcctgc tgaccctagc ggcctccctt 19320

gcagcatgag ccctttgttg tccgcactgc tgctgctgag ggtcctgcca gccccccagg 19380

gccttttctg cccaggcact gccacctgct aaaacccagc ctgcccatgg cagggcatcg 19440

catgtctgag ggatggaacg gggcaagggc agtgctgcag gcagtggttc aagaaacgtt 19500

tagatgttgt actgagggat gtggtttagt gggaaatact ggtgataggt ggatggttgg 19560

actggaggat cttagaggtc ttttccaacc ttggtgatta tgtgattcta tgaagggagc 19620

catagcctgc tcccatcatg ccccgccagt ccggctctcc cgagcacttt ggttccgcag 19680

catccaggcc cgctctgggg ccggtgtggg cctgggtgct gccacgtgtc cccgctgcaa 19740

ggcctggcca accccgctgt gccacaatgc aggggctggg ctaggagtgg gcagggaggt 19800

gtggggagag gtgtaggacg tggctgggag cgcagggagt tatttgcata ggggggcgtg 19860

ctgcggaagg acgcgggtat aaaagggcat cgaggtcccc ggcacagccc catcggcgtg 19920

gggacacaca gctgctggga ttccgccatg gcctgggctc ctctcctcct ggcggtgctc 19980

gcccacacct caggtactcg ttgcgcccgg tcggggactg tgggcacggg gctctgtccc 20040

actgctgcgc gggcagggct gtgcgtgcgg ggccgtcact gattgccgtt ttctcccctc 20100

tctcctctcc ggcgcgcctc tccaggttcc ctggtgcagg cacagtctgc cctgactcag 20160

cctgcctccg tgtctgggtc tcctggacag tcgatcacca tctcctgcac tggaaccagc 20220

agtgacgttg gtggttataa ctatgtctcc tggtaccaac aaaacccagg caaagccccc 20280

aaactcatga tttatgatgt cagtaatcgg ccctcaggga tttctaatcg cttctctggc 20340

tccaagtctg gcaacacggc ctccctgacc atctctgggc tccaggctga cgacgaggct 20400

gattattact gcacctcata tacaaacgac agcaattctc aggtattcgg cggagggacc 20460

aagctgaccg tcctaggtga gtcgctcata tgctaccgtt cgtatagcat acattatacg 20520

aagttatcag acatgataag atacattgat gagtttggac aaaccacaac tagaatgcag 20580

tgaaaaaaat gctttatttg tgaaatttgt gatgctattg ctttatttgt aaccattata 20640

agctgcaata aacaagttgg ggtgggcgaa gaactccagc atgagatccc cgcgctggag 20700

gatcatccag ccggcgtccc ggaaaacgat tccgaagccc aacctttcat agaaggcggc 20760

ggtggaatcg aaatctcgta gcacgtgtca gtcctgctcc tcggccacga agtgcttagc 20820

cctcccacac ataaccagag ggcagcaatt cacgaatccc aactgccgtc ggctgtccat 20880

cactgtcctt cactatggct ttgatcccag gatgcagatc gagaagcacc tgtcggcacc 20940

gtccgcaggg gctcaagatg cccctgttct catttccgat cgcgacgata caagtcaggt 21000

tgccagctgc cgcagcagca gcagtgccca gcaccacgag ttctgcacaa ggtcccccag 21060

taaaatgata tacattgaca ccagtgaaga tgcggccgtc gctagagaga gctgcgctgg 21120

cgacgctgta gtcttcagag atggggatgc tgttgattgt agccgttgct ctttcaatga 21180

gggtggattc ttcttgagac aaaggcttgg ccatggttta gttcctcacc ttgtcgtatt 21240

atactatgcc gatatactat gccgatgatt aattgtcaac acgtgctgat cagatccgaa 21300

aatggatata caagctcccg ggagcttttt gcaaaagcct aggcctccaa aaaagcctcc 21360

tcactacttc tggaatagct cagaggcaga ggcggcctcg gcctctgcat aaataaaaaa 21420

aattagtcag ccatggggcg gagaatgggc ggaactgggc ggagttaggg gcgggatggg 21480

cggagttagg ggcgggacta tggttgctga ctaattgaga tgcatgcttt gcatacttct 21540

gcctgctggg gagcctgggg actttccaca cctggttgct gactaattga gatgcatgct 21600

ttgcatactt ctgcctgctg gggagcctgg ggactttcca caccctaact gacacacatt 21660

ccacagataa cttcgtatag catacattat acgaacggta gctctagaga cctcgtctcg 21720

gtctttcttc ccccatcgtg aaattgtgac attttgtcga tttttggtga tttgggggtt 21780

tttcttggac ttggcggcag gctggggtct gccaccggcg cagggccggg cactcagcgc 21840

gacagcctgg gctgagtctt gtccccaccg agccggaggg ctccggtgtg cgccatggag 21900

gacttagggt tattttgtca atggaaagtt cttaaaattt gaccagaaaa tgtgcccgag 21960

gtctgtctct gccacacgat ttcagaaatt gtgtctaggt cgatgagaag acagtttttg 22020

tctttgtcag gaaattagtt gtgagttgtt agtccttccc tcttagtcct aaggactaag 22080

acctttgtcc ccggtctggt ctctcactgg ggactcttgg ctccagtgcc atggggagcc 22140

caagtgtcac tgacacggtg tccttggggg tgaaattcag tttttcagct gtccaggtaa 22200

gtgctgtcaa aaaatggagg ttattctgct gaaaaagctg agaggaatat tttgtcattt 22260

ttttcggaaa tatatatata tatatatata taaatatata aattatttat atattttata 22320

tatatatata tataaaatat atgtatttct ctctttcttt ttatatatat atataaatat 22380

atatatattt ctccctcttt ctttatatat atttataggg agagaatcta tatttttggc 22440

caatttggcc aatttctctc tctctctctt tatatatata tatatatgta tatgtaattt 22500

atatatatat atataaattt atatttattt atttatttat ttatatagat tatttttata 22560

tacatatata tatataaatt tggccaattt agaagaaatt ggccaaaaaa acccaatctg 22620

aaccaaatta cttagggcat aagtccaaaa aaaaagtgca gttcaagaat tcctcgctgg 22680

aaagttttta atgggggcag ttcttcccca ttttctggtg tagatatgag tgaagatata 22740

gatatgtatg tagatataga tttggctata gtttgtttga tgaacagtct gaggaagagt 22800

aaatcttgcc ttcctcatgg caatttttaa ttctacttct tctgagacaa gtctgaacat 22860

gccttgaggt agaaagagcc cgaaaattga gtgctttttc tttgaaattt taattaaaat 22920

taaaaaatag ccttacagca ggaaagaaag ggggtgatgg cagctaatcg gagttccaaa 22980

gctgaaccac gttcacccaa acacgtggtt gaaattttgt gtgtgtatat atataaatat 23040

atatatgtat acaaaagaaa atgcctatat ataaaaaaat tgttatgtat attatataga 23100

tttattatag atatgtacat aagatataac acgtatacat atatatgtag tctgctgtat 23160

gctttgtata aatttttata gatatgatgt atattatatg catgggttat atatctataa 23220

tatattttat ctgtagaagg aactacagta taaaatgcat atatgcgtat atatacacat 23280

atttatatat atgtgtatat atatgtatac gcacacgtat aacatccatg aaaatagata 23340

tggatgggtg gatgtgtttg ttttacagag gtgcatgtgt gtctgtacat acacagccat 23400

acatacgcgt gtgcggccgc aggcgtaatc atggtcatag ctgtttcctg tgtgaaattg 23460

ttatccgctc acaattccac acaacatacg agccggaagc ataaagtgta aagcctgggg 23520

tgcctaatga gtgagctaac tcacattaat tgcgttgcgc tcactgcccg ctttccagtc 23580

gggaaacctg tcgtgccagc tgcattaatg aatcggccaa cgcgcgggga gaggcggttt 23640

gcgtattggg cgctcttccg cttcctcgct cactgactcg ctgcgctcgg tcgttcggct 23700

gcggcgagcg gtatcagctc actcaaaggc ggtaatacgg ttatccacag aatcagggga 23760

taacgcagga aagaacatgt gagcaaaagg ccagcaaaag gccaggaacc gtaaaaaggc 23820

cgcgttgctg gcgtttttcc ataggctccg cccccctgac gagcatcaca aaaatcgacg 23880

ctcaagtcag aggtggcgaa acccgacagg actataaaga taccaggcgt ttccccctgg 23940

aagctccctc gtgcgctctc ctgttccgac cctgccgctt accggatacc tgtccgcctt 24000

tctcccttcg ggaagcgtgg cgctttctca tagctcacgc tgtaggtatc tcagttcggt 24060

gtaggtcgtt cgctccaagc tgggctgtgt gcacgaaccc cccgttcagc ccgaccgctg 24120

cgccttatcc ggtaactatc gtcttgagtc caacccggta agacacgact tatcgccact 24180

ggcagcagcc actggtaaca ggattagcag agcgaggtat gtaggcggtg ctacagagtt 24240

cttgaagtgg tggcctaact acggctacac tagaagaaca gtatttggta tctgcgctct 24300

gctgaagcca gttaccttcg gaaaaagagt tggtagctct tgatccggca aacaaaccac 24360

cgctggtagc ggtggttttt ttgtttgcaa gcagcagatt acgcgcagaa aaaaaggatc 24420

tcaagaagat cctttgatct tttctacggg gtctgacgct cagtggaacg aaaactcacg 24480

ttaagggatt ttggtcatga gattatcaaa aaggatcttc acctagatcc ttttaaatta 24540

aaaatgaagt tttaaatcaa tctaaagtat atatgagtaa acttggtctg acagttacca 24600

atgcttaatc agtgaggcac ctatctcagc gatctgtcta tttcgttcat ccatagttgc 24660

ctgactcccc gtcgtgtaga taactacgat acgggagggc ttaccatctg gccccagtgc 24720

tgcaatgata ccgcgagacc cacgctcacc ggctccagat ttatcagcaa taaaccagcc 24780

agccggaagg gccgagcgca gaagtggtcc tgcaacttta tccgcctcca tccagtctat 24840

taattgttgc cgggaagcta gagtaagtag ttcgccagtt aatagtttgc gcaacgttgt 24900

tgccattgct acaggcatcg tggtgtcacg ctcgtcgttt ggtatggctt cattcagctc 24960

cggttcccaa cgatcaaggc gagttacatg atcccccatg ttgtgcaaaa aagcggttag 25020

ctccttcggt cctccgatcg ttgtcagaag taagttggcc gcagtgttat cactcatggt 25080

tatggcagca ctgcataatt ctcttactgt catgccatcc gtaagatgct tttctgtgac 25140

tggtgagtac tcaaccaagt cattctgaga atagtgtatg cggcgaccga gttgctcttg 25200

cccggcgtca atacgggata ataccgcgcc acatagcaga actttaaaag tgctcatcat 25260

tggaaaacgt tcttcggggc gaaaactctc aaggatctta ccgctgttga gatccagttc 25320

gatgtaaccc actcgtgcac ccaactgatc ttcagcatct tttactttca ccagcgtttc 25380

tgggtgagca aaaacaggaa ggcaaaatgc cgcaaaaaag ggaataaggg cgacacggaa 25440

atgttgaata ctcatactct tcctttttca atattattga agcatttatc agggttattg 25500

tctcatgagc ggatacatat ttgaatgtat ttagaaaaat aaacaaatag gggttccgcg 25560

cacatttccc cgaaaagtgc cacctgacgt ctaagaaacc attattatca tgacattaac 25620

ctataaaaat aggcgtatca cgaggccctt tcgtc 25655

<210> 144
<211> 7420
<212> DNA
<213> Artificial Sequence

<220>
<223> Synthetic DNA

<400> 144
gagctcgttt aaacagcact gtagatcaga cacgatatcg cgaggaggtg cagctgttgg 60

agtctggggg aggcttggta cagcctgggg ggtccctgag actctcctgt gcagcctctg 120

gattcacctt tagcagctat gctatgcatt gggtccgcca ggctccaggg aaggggctgg 180

agtgggtctc atggatcaac gctggcaatg gtaacacaaa atattcacag aagttccagg 240

gccggttcac catctccaga gacaattcca agaacacgct gtatctgcaa atgaacagcc 300

tgagagccga ggacacggcc gtatattact gtgcgaaagc gtctagtagt ggttattact 360

acgtccactt tgactactgg ggccagggca ccctggtcac cgtctcctca gtgcctggtg 420

actcttccgc aatcagtcct gggacagaaa cctcagggac agcctttatg agttttgatc 480

acatctaaca gagaggatgg gccagtgata cgtctcatag tagtgcgaga gatacgaggt 540

gcagctgttg gagtctgggg gaggcttggt acagcctggg gggtccctga gactctcctg 600

tgcagcctct ggattcacct ttagcagcta tggtatcagc tgggtccgcc aggctccagg 660

gaaggggctg gagtgggtct catggatcag cgcttacaat ggtaacacaa actatgcaca 720

gaagctccag ggccggttca ccatctccag agacaattcc aagaacacgc tgtatctgca 780

aatgaacagc ctgagagccg aggacacggc cgtatattac tgtgcgaaag atttaaggtt 840

atcctattac tactttgact actggggcca gggcaccctg gtcaccgtct cctcagtgcc 900

tggtgactct tccgcaatca gtcctgggac agaaacctca gggacagcct ttatgagttt 960

tgatcacatc taacagagag gatgggccag tctacgtagc tgtactactc acgactacag 1020

gaggtgcagc tgttggagtc tgggggaggc ttggtacagc ctggggggtc cctgagactc 1080

tcctgtgcag cctctggatt cacctttagc ggctttgcca tgaactgggt ccgccaggct 1140

ccagggaagg ggctggagtg ggtctcaact attagtaata gtgctggtag cacatattac 1200

gcagactccg tgaagggccg gttcaccatc tccagagaca attccaagaa cacgctgtat 1260

ctgcaaatga acagcctgag agccgaggac acggccgtat attactgtgc gaaatatggg 1320

gaggatattg tcgttgtgcc agctcgctac ggtgtggacg tctggggcca gggcaccctg 1380

gtcaccgtct cctcagtgcc tggtgactct tccgcaatca gtcctgggac agaaacctca 1440

gggacagcct ttatgagttt tgatcacatc taacagagag gatgggccag tagagacgag 1500

tcgtcgctcg acatacgcgt gaggtgcagc tgttggagtc tgggggaggc ttggtacagc 1560

ctggggggtc cctgagactc tcctgtgcag cctctggatt cacctttagc agctctgctg 1620

tgcagtgggt ccgccaggct ccagggaagg ggctggagtg ggtctcatgg atcgtcgttg 1680

gcagtggtaa cacaaactac gcacagaagt tccaggaacg gttcaccatc tccagagaca 1740

attccaagaa cacgctgtat ctgcaaatga acagcctgag agccgaggac acggccgtat 1800

attactgtgc gaaagacgtc cttgtagtac cagctgcccc ctactactac tacggtatgg 1860

acgtctgggg ccagggcacc ctggtcaccg tctcctcagt gcctggtgac tcttccgcaa 1920

tcagtcctgg gacagaaacc tcagggacag cctttatgag ttttgatcac atctaacaga 1980

gaggatgggc cagagactat actagcgtcg tctagtacgc tatgaggtgc agctgttgga 2040

gtctggggga ggcttggtac agcctggggg gtccctgaga ctctcctgtg cagcctctgg 2100

attcaccttt agcactagtg gagtgggtgt gggctgggtc cgccaggctc cagggaaggg 2160

gctggagtgg gtctcactca tttattggaa tgatgataag cgctacagcc catctctgaa 2220

gagccggttc accatctcca gagacaattc caagaacacg ctgtatctgc aaatgaacag 2280

cctgagagcc gaggacacgg ccgtatatta ctgtgcgaaa gacgatgggg cgtatggccc 2340

gtatagcagc gcctggacct ttgactactg gggccagggc accctggtca ccgtctcctc 2400

agtgcctggt gactcttccg caatcagtcc tgggacagaa acctcaggga cagcctttat 2460

gagttttgat cacatctaac agagaggatg ggccagcgac gtgacttaca cacacttaca 2520

gatcgtgagg tgcagctgtt ggagtctggg ggaggcttgg tacagcctgg ggggtccctg 2580

agactctcct gtgcagcctc tggattcacc tttagcacta gtggaatgcg tgtgagctgg 2640

gtccgccagg ctccagggaa ggggctggag tgggtctcac gcattgattg ggatgatgat 2700

aaattctaca gcacatctct gaagacccgg ttcaccatct ccagagacaa ttccaagaac 2760

acgctgtatc tgcaaatgaa cagcctgaga gccgaggaca cggccgtata ttactgtgcg 2820

aaagatgacg gtggcacccc gcccggggaa ttctttgact actggggcca gggcaccctg 2880

gtcaccgtct cctcagtgcc tggtgactct tccgcaatca gtcctgggac agaaacctca 2940

gggacagcct ttatgagttt tgatcacatc taacagagag gatgggccag tcgcactagt 3000

tctagcgact tctatactat gaggtgcagc tgttggagtc tgggggaggc ttggtacagc 3060

ctggggggtc cctgagactc tcctgtgcag cctctggatt cacctttagc gattatgcca 3120

tgcactgggt ccgccaggct ccagggaagg ggctggagtg ggtctcaggt attagttgga 3180

atagtggtag cataggctat gcggactctg tgaagggccg gttcaccatc tccagagaca 3240

attccaagaa cacgctgtat ctgcaaatga acagcctgag agccgaggac acggccgtat 3300

attactgtgc gaaaatagtg ggagctactt acaactggtt cgacccctgg ggccagggca 3360

ccctggtcac cgtctcctca gtgcctggtg actcttccgc aatcagtcct gggacagaaa 3420

cctcagggac agcctttatg agttttgatc acatctaaca gagaggatgg gccagacgcg 3480

atcgaactag cagtaagctc acgtagaggt gcagctgttg gagtctgggg gaggcttggt 3540

acagcctggg gggtccctga gactctcctg tgcagcctct ggattcacct ttagcaacgc 3600

ctggatgagc tgggtccgcc aggctccagg gaaggggctg gagtgggtct cacgtattaa 3660

aagcaaaact gatggtggga caacagacta cgctgcaccc gtgaaaggcc ggttcaccat 3720

ctccagagac aattccaaga acacgctgta tctgcaaatg aacagcctga gagccgagga 3780

cacggccgta tattactgtg cgaaagaaag gggtagcagc tcgtcctttg actactgggg 3840

ccagggcacc ctggtcaccg tctcctcagt gcctggtgac tcttccgcaa tcagtcctgg 3900

gacagaaacc tcagggacag cctttatgag ttttgatcac atctaacaga gaggatgggc 3960

cagcgatcgt atacgcagta cgacgcgtat acagaggtgc agctgttgga gtctggggga 4020

ggcttggtac agcctggggg gtccctgaga ctctcctgtg cagcctctgg attcaccttt 4080

agcagctatg gcatgcactg ggtccgccag gctccaggga aggggctgga gtgggtctca 4140

gttatatggt atgatggaag taataaatac tatgcagact ccgtgaaggg ccggttcacc 4200

atctccagag acaattccaa gaacacgctg tatctgcaaa tgaacagcct gagagccgag 4260

gacacggccg tatattactg tgcgaaagat cttgtcttcg ggccagctgc tactcctttt 4320

gactactggg gccagggcac cctggtcacc gtctcctcag tgcctggtga ctcttccgca 4380

atcagtcctg ggacagaaac ctcagggaca gcctttatga gttttgatca catctaacag 4440

agaggatggg ccagctatag cgtatacgtc atcatagtcg catagaggtg cagctgttgg 4500

agtctggggg aggcttggta cagcctgggg ggtccctgag actctcctgt gcagcctctg 4560

gattcacctt tagcagcaac tacatgagct gggtccgcca ggctccaggg aaggggctgg 4620

agtgggtctc agttatttat agcggtggta gcacatacta cgcagactcc gtgaagggcc 4680

ggttcaccat ctccagagac aattccaaga acacgctgta tctgcaaatg aacagcctga 4740

gagccgagga cacggccgta tattactgtg cgaaagcgaa gttctcctat ggttcaggga 4800

gttattactt tgactactgg ggccagggca ccctggtcac cgtctcctca gtgcctggtg 4860

actcttccgc aatcagtcct gggacagaaa cctcagggac agcctttatg agttttgatc 4920

acatctaaca gagaggatgg gccagtcgat agtgatcgct gcgtatctga cgtcagaggt 4980

gcagctgttg gagtctgggg gaggcttggt acagcctggg gggtccctga gactctcctg 5040

tgcagcctct ggattcacct ttagcagcta ctggatgcac tgggtccgcc aggctccagg 5100

gaaggggctg gagtgggtct cacgtattaa tagtgatggg agtagcacaa gctacgcgga 5160

ctccgtgaag ggccggttca ccatctccag agacaattcc aagaacacgc tgtatctgca 5220

aatgaacagc ctgagagccg aggacacggc cgtatattac tgtgcgaaag gatattgtag 5280

tagtaccagc tgctatgctg atgcttttga tatctggggc cagggcaccc tggtcaccgt 5340

ctcctcagtg cctggtgact cttccgcaat cagtcctggg acagaaacct cagggacagc 5400

ctttatgagt tttgatcaca tctaacagag aggatgggcc agtgtcgtcg caacacatac 5460

gcacagtcgt gcgaggtgca gctgttggag tctgggggag gcttggtaca gcctgggggg 5520

tccctgagac tctcctgtgc agcctctgga ttcaccttta gcagtagtaa ctggtggggc 5580

tgggtccgcc aggctccagg gaaggggctg gagtgggtct catacatcta ttatagtggg 5640

agcacctact acaacccgtc cctcaagagt cggttcacca tctccagaga caattccaag 5700

aacacgctgt atctgcaaat gaacagcctg agagccgagg acacggccgt atattactgt 5760

gcgaaagatc gaagtagagg gggttttgac tactggggcc agggcaccct ggtcaccgtc 5820

tcctcagtgc ctggtgactc ttccgcaatc agtcctggga cagaaacctc agggacagcc 5880

tttatgagtt ttgatcacat ctaacagaga ggatgggcca gactcatcta cactcgcgca 5940

cagagcgtca cgaggtgcag ctgttggagt ctgggggagg cttggtacag cctggggggt 6000

ccctgagact ctcctgtgca gcctctggat tcacctttag cggttactac tggagctggg 6060

tccgccaggc tccagggaag gggctggagt gggtctcaga aatcaatcat agtggaagca 6120

ccaactacaa cccgtccctc aagagtcggt tcaccatctc cagagacaat tccaagaaca 6180

cgctgtatct gcaaatgaac agcctgagag ccgaggacac ggccgtatat tactgtgcga 6240

aagcccgtac cagcgcgggc ccgggtggtg cttttgattt ctggggccag ggcaccctgg 6300

tcaccgtctc ctcagtgcct ggtgactctt ccgcaatcag tcctgggaca gaaacctcag 6360

ggacagcctt tatgagtttt gatcacatct aacagagagg atgggccaga gtgtatgtca 6420

tagatagaca tatagtcgcg aggtgcagct gttggagtct gggggaggct tggtacagcc 6480

tggggggtcc ctgagactct cctgtgcagc ctctggattc acctttagca gctactggat 6540

cggctgggtc cgccaggctc cagggaaggg gctggagtgg gtctcaatca tctatcctgg 6600

tgactctgat accagataca gcccgtcctt ccaaggccgg ttcaccatct ccagagacaa 6660

ttccaagaac acgctgtatc tgcaaatgaa cagcctgaga gccgaggaca cggccgtata 6720

ttactgtgcg aaaggggttt gtagtaccag ctcgtacgcc ttttactact actactacgg 6780

tatggacgtc tggggccagg gcaccctggt caccgtctcc tcagtgcctg gtgactcttc 6840

cgcaatcagt cctgggacag aaacctcagg gacagccttt atgagttttg atcacatcta 6900

acagagagga tgggccagca tactctaccg acactatccg agacgcgcga ggtgcagctg 6960

ttggagtctg ggggaggctt ggtacagcct ggggggtccc tgagactctc ctgtgcagcc 7020

tctggattca cctttagcag ctatgctatg aattgggtcc gccaggctcc agggaagggg 7080

ctggagtggg tctcatggat caacaccaac actgggaacc caacgtatgc ccagggcttc 7140

acaggacggt tcaccatctc cagagacaat tccaagaaca cgctgtatct gcaaatgaac 7200

agcctgagag ccgaggacac ggccgtatat tactgtgcga aagactacgg tggtaactcc 7260

tactggtact tcgatctctg gggccagggc accctggtca ccgtctcctc agtgcctggt 7320

gactcttccg caatcagtcc tgggacagaa acctcaggga cagcctttat gagttttgat 7380

cacatctaac agagaggatg ggccaggttt aaacggtacc 7420

<210> 145
<211> 11635
<212> DNA
<213> Artificial Sequence

<220>
<223> Synthetic DNA

<400> 145
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt catatgtagc ttacgacact 420
   gataacttcg tatatggtat tatatacgaa gttataagct tcaccatgat tgaacaagat 480
   ggattgcacg caggttctcc ggccgcttgg gtggagaggc tattcggcta tgactgggca 540
   caacagacaa tcggctgctc tgatgccgcc gtgttccggc tgtcagcgca ggggcgcccg 600
   gttctttttg tcaagaccga cctgtccggt gccctgaatg aactgcagga cgaggcagcg 660
   cggctatcgt ggctggccac gacgggcgtt ccttgcgcag ctgtgctcga cgttgtcact 720
   gaagcgggaa gggactggct gctattgggc gaagtgccgg ggcaggatct cctgtcatct 780
   caccttgctc ctgccgagaa agtatccatc atggctgatg caatgcggcg gctgcatacg 840
   cttgatccgg ctacctgccc attcgaccac caagcgaaac atcgcatcga gcgagcacgt 900
   actcggatgg aagccggtct tgtcgatcag gatgatctgg acgaagagca tcaggggctc 960
   gcgccagccg aactgttcgc caggctcaag gcgcgcatgc ccgacggcga ggatctcgtc 1020
   gtgacccatg gcgatgcctg cttgccgaat atcatggtgg aaaatggccg cttttctgga 1080
   ttcatcgact gtggccggct gggtgtggcg gaccgctatc aggacatagc gttggctacc 1140
   cgtgatattg ctgaagagct tggcggcgaa tgggctgacc gcttcctcgt gctttacggt 1200
   atcgccgctc ccgattcgca gcgcatcgcc ttctatcgcc ttcttgacga gttcttctga 1260
   actagtgcgg gactctgggg ttcgaaatga ccgaccaagc gacgcccaac ctgccatcac 1320
   gagatttcga ttccaccgcc gccttctatg aaaggttggg cttcggaatc gttttccggg 1380
   acgccggctg gatgatcctc cagcgcgggg atctcatgct ggagttcttc gcccacccca 1440
   acttgtttat tgcagcttat aatggttaca aataaagcaa tagcatcaca aatttcacaa 1500
   ataaagcatt tttttcactg cattctagtt gtggtttgtc caaactcatc aatgtatctt 1560
   atcatgtctg ttataccgtc gatctctagc tagagcttgg cgtaatcatg gtcatagctg 1620
   tttcctgtgt gcccacccca acttgtttat tgcagcttat aatggttaca aataaagcaa 1680
   tagcatcaca aatttcacaa ataaagcatt tttttcactg cattctagtt gtggtttgtc 1740
   caaactcatc aatgtatctt atcatgtctg ataacttcgt ataatgtatg ctatacgaac 1800
   ggtactcgag agctcgttta aacagcactg tagatcagac acgatatcgc gaggaggtgc 1860
   agctgttgga gtctggggga ggcttggtac agcctggggg gtccctgaga ctctcctgtg 1920
   cagcctctgg attcaccttt agcagctatg ctatgcattg ggtccgccag gctccaggga 1980
   aggggctgga gtgggtctca tggatcaacg ctggcaatgg taacacaaaa tattcacaga 2040
   agttccaggg ccggttcacc atctccagag acaattccaa gaacacgctg tatctgcaaa 2100
   tgaacagcct gagagccgag gacacggccg tatattactg tgcgaaagcg tctagtagtg 2160
   gttattacta cgtccacttt gactactggg gccagggcac cctggtcacc gtctcctcag 2220
   tgcctggtga ctcttccgca atcagtcctg ggacagaaac ctcaggacag gcctttatga 2280
   gttttgatca catctaacag agaggatggg ccagtgatac gtctcatagt agtgcgagag 2340
   atacgaggtg cagctgttgg agtctggggg aggcttggta cagcctgggg ggtccctgag 2400
   actctcctgt gcagcctctg gattcacctt tagcagctat ggtatcagct gggtccgcca 2460
   ggctccaggg aaggggctgg agtgggtctc atggatcagc gcttacaatg gtaacacaaa 2520
   ctatgcacag aagctccagg gccggttcac catctccaga gacaattcca agaacacgct 2580
   gtatctgcaa atgaacagcc tgagagccga ggacacggcc gtatattact gtgcgaaaga 2640
   tttaaggtta tcctattact actttgacta ctggggccag ggcaccctgg tcaccgtctc 2700
   ctcagtgcct ggtgactctt ccgcaatcag tcctgggaca gaaacctcag ggacagcctt 2760
   tatgagtttt gatcacatct aacagagagg atgggccagt ctacgtagct gtactactca 2820
   cgactacagg aggtgcagct gttggagtct gggggaggct tggtacagcc tggggggtcc 2880
   ctgagactct cctgtgcagc ctctggattc acctttagcg gctttgccat gaactgggtc 2940
   cgccaggctc cagggaaggg gctggagtgg gtctcaacta ttagtaatag tgctggtagc 3000
   acatattacg cagactccgt gaagggccgg ttcaccatct ccagagacaa ttccaagaac 3060
   acgctgtatc tgcaaatgaa cagcctgaga gccgaggaca cggccgtata ttactgtgcg 3120
   aaatatgggg aggatattgt cgttgtgcca gctcgctacg gtgtggacgt ctggggccag 3180
   ggcaccctgg tcaccgtctc ctcagtgcct ggtgactctt ccgcaatcag tcctgggaca 3240
   gaaacctcag ggacagcctt tatgagtttt gatcacatct aacagagagg atgggccagt 3300
   agagacgagt cgtcgctcga catacgcgtg aggtgcagct gttggagtct gggggaggct 3360
   tggtacagcc tggggggtcc ctgagactct cctgtgcagc ctctggattc acctttagca 3420
   gctctgctgt gcagtgggtc cgccaggctc cagggaaggg gctggagtgg gtctcatgga 3480
   tcgtcgttgg cagtggtaac acaaactacg cacagaagtt ccaggaacgg ttcaccatct 3540
   ccagagacaa ttccaagaac acgctgtatc tgcaaatgaa cagcctgaga gccgaggaca 3600
   cggccgtata ttactgtgcg aaagacgtcc ttgtagtacc agctgccccc tactactact 3660
   acggtatgga cgtctggggc cagggcaccc tggtcaccgt ctcctcagtg cctggtgact 3720
   cttccgcaat cagtcctggg acagaaacct cagggacagc ctttatgagt tttgatcaca 3780
   tctaacagag aggatgggcc agagactata ctagcgtcgt ctagtacgct atgaggtgca 3840
   gctgttggag tctgggggag gcttggtaca gcctgggggg tccctgagac tctcctgtgc 3900
   agcctctgga ttcaccttta gcactagtgg agtgggtgtg ggctgggtcc gccaggctcc 3960
   agggaagggg ctggagtggg tctcactcat ttattggaat gatgataagc gctacagccc 4020
   atctctgaag agccggttca ccatctccag agacaattcc aagaacacgc tgtatctgca 4080
   aatgaacagc ctgagagccg aggacacggc cgtatattac tgtgcgaaag acgatggggc 4140
   gtatgggccg tatagcagcg cctggacctt tgactactgg ggccagggca ccctggtcac 4200
   cgtctcctca gtgcctggtg actcttccgc aatcagtcct gggacagaaa cctcagggac 4260
   agcctttatg agttttgatc acatctaaca gagaggatgg gccagcgacg tgacttacac 4320
   acacttacag atcgtgaggt gcagctgttg gagtctgggg gaggcttggt acagcctggg 4380
   gggtccctga gactctcctg tgcagcctct ggattcacct ttagcactag tggaatgcgt 4440
   gtgagctggg tccgccaggc tccagggaag gggctggagt gggtctcacg cattgattgg 4500
   gatgatgata aattctacag cacatctctg aagacccggt tcaccatctc cagagacaat 4560
   tccaagaaca cgctgtatct gcaaatgaac agcctgagag ccgaggacac ggccgtatat 4620
   tactgtgcga aagatgacgg tggcaccccg cccggggaat tctttgacta ctggggccag 4680
   ggcaccctgg tcaccgtctc ctcagtgcct ggtgactctt ccgcaatcag tcctgggaca 4740
   gaaacctcag ggacagcctt tatgagtttt gatcacatct aacagagagg atgggccagt 4800
   cgcactagtt ctagcgactt ctatactatg aggtgcagct gttggagtct gggggaggct 4860
   tggtacagcc tggggggtcc ctgagactct cctgtgcagc ctctggattc acctttagcg 4920
   attatgccat gcactgggtc cgccaggctc cagggaaggg gctggagtgg gtctcaggta 4980
   ttagttggaa tagtggtagc ataggctatg cggactctgt gaagggccgg ttcaccatct 5040
   ccagagacaa ttccaagaac acgctgtatc tgcaaatgaa cagcctgaga gccgaggaca 5100
   cggccgtata ttactgtgcg aaaatagtgg gagctactta caactggttc gacccctggg 5160
   gccagggcac cctggtcacc gtctcctcag tgcctggtga ctcttccgca atcagtcctg 5220
   ggacagaaac ctcagggaca gcctttatga gttttgatca catctaacag agaggatggg 5280
   ccagacgcga tcgaactagc agtaagctca cgtagaggtg cagctgttgg agtctggggg 5340
   aggcttggta cagcctgggg ggtccctgag actctcctgt gcagcctctg gattcacctt 5400
   tagcaacgcc tggatgagct gggtccgcca ggctccaggg aaggggctgg agtgggtctc 5460
   acgtattaaa agcaaaactg atggtgggac aacagactac gctgcacccg tgaaaggccg 5520
   gttcaccatc tccagagaca attccaagaa cacgctgtat ctgcaaatga acagcctgag 5580
   agccgaggac acggccgtat attactgtgc gaaagaaagg ggtagcagct cgtcctttga 5640
   ctactggggc cagggcaccc tggtcaccgt ctcctcagtg cctggtgact cttccgcaat 5700
   cagtcctggg acagaaacct cagggacagc ctttatgagt tttgatcaca tctaacagag 5760
   aggatgggcc agcgatcgta tacgcagtac gacgcgtata cagaggtgca gctgttggag 5820
   tctgggggag gcttggtaca gcctgggggg tccctgagac tctcctgtgc agcctctgga 5880
   ttcaccttta gcagctatgg catgcactgg gtccgccagg ctccagggaa ggggctggag 5940
   tgggtctcag ttatatggta tgatggaagt aataaatact atgcagactc cgtgaagggc 6000
   cggttcacca tctccagaga caattccaag aacacgctgt atctgcaaat gaacagcctg 6060
   agagccgagg acacggccgt atattactgt gcgaaagatc ttgtcttcgg gccagctgct 6120
   actccttttg actactgggg ccagggcacc ctggtcaccg tctcctcagt gcctggtgac 6180
   tcttccgcaa tcagtcctgg gacagaaacc tcagggacag cctttatgag ttttgatcac 6240
   atctaacaga gaggatgggc cagctatagc gtatacgtca tcatagtcgc atagaggtgc 6300
   agctgttgga gtctggggga ggcttggtac agcctggggg gtccctgaga ctctcctgtg 6360
   cagcctctgg attcaccttt agcagcaact acatgagctg ggtccgccag gctccaggga 6420
   aggggctgga gtgggtctca gttatttata gcggtggtag cacatactac gcagactccg 6480
   tgaagggccg gttcaccatc tccagagaca attccaagaa cacgctgtat ctgcaaatga 6540
   acagcctgag agccgaggac acggccgtat attactgtgc gaaagcgaag ttctcctatg 6600
   gttcagggag ttattacttt gactactggg gccagggcac cctggtcacc gtctcctcag 6660
   tgcctggtga ctcttccgca atcagtcctg ggacagaaac ctcagggaca gcctttatga 6720
   gttttgatca catctaacag agaggatggg ccagtcgata gtgatcgctg cgtatctgac 6780
   gtcagaggtg cagctgttgg agtctggggg aggcttggta cagcctgggg ggtccctgag 6840
   actctcctgt gcagcctctg gattcacctt tagcagctac tggatgcact gggtccgcca 6900
   ggctccaggg aaggggctgg agtgggtctc acgtattaat agtgatggga gtagcacaag 6960
   ctacgcggac tccgtgaagg gccggttcac catctccaga gacaattcca agaacacgct 7020
   gtatctgcaa atgaacagcc tgagagccga ggacacggcc gtatattact gtgcgaaagg 7080
   atattgtagt agtaccagct gctatgctga tgcttttgat atctggggcc agggcaccct 7140
   ggtcaccgtc tcctcagtgc ctggtgactc ttccgcaatc agtcctggga cagaaacctc 7200
   agggacagcc tttatgagtt ttgatcacat ctaacagaga ggatgggcca gtgtcgtcgc 7260
   aacacatacg cacagtcgtg cgaggtgcag ctgttggagt ctgggggagg cttggtacag 7320
   cctggggggt ccctgagact ctcctgtgca gcctctggat tcacctttag cagtagtaac 7380
   tggtggggct gggtccgcca ggctccaggg aaggggctgg agtgggtctc atacatctat 7440
   tatagtggga gcacctacta caacccgtcc ctcaagagtc ggttcaccat ctccagagac 7500
   aattccaaga acacgctgta tctgcaaatg aacagcctga gagccgagga cacggccgta 7560
   tattactgtg cgaaagatcg aagtagaggg ggttttgact actggggcca gggcaccctg 7620
   gtcaccgtct cctcagtgcc tggtgactct tccgcaatca gtcctgggac agaaacctca 7680
   gggacagcct ttatgagttt tgatcacatc taacagagag gatgggccag actcatctac 7740
   actcgcgcac agagcgtcac gaggtgcagc tgttggagtc tgggggaggc ttggtacagc 7800
   ctggggggtc cctgagactc tcctgtgcag cctctggatt cacctttagc ggttactact 7860
   ggagctgggt ccgccaggct ccagggaagg ggctggagtg ggtctcagaa atcaatcata 7920
   gtggaagcac caactacaac ccgtccctca agagtcggtt caccatctcc agagacaatt 7980
   ccaagaacac gctgtatctg caaatgaaca gcctgagagc cgaggacacg gccgtatatt 8040
   actgtgcgaa agcccgtacc agcgcggccc cgggtggtgc ttttgatttc tggggccagg 8100
   gcaccctggt caccgtctcc tcagtgcctg gtgactcttc cgcaatcagt cctgggacag 8160
   aaacctcagg gacagccttt atgagttttg atcacatcta acagagagga tgggccagag 8220
   tgtatgtcat agatagacat atagtcgcga ggtgcagctg ttggagtctg ggggaggctt 8280
   ggtacagcct ggggggtccc tgagactctc ctgtgcagcc tctggattca cctttagcag 8340
   ctactggatc ggctgggtcc gccaggctcc agggaagggg ctggagtggg tctcaatcat 8400
   ctatcctggt gactctgata ccagatacag cccgtccttc caaggccggt tcaccatctc 8460
   cagagacaat tccaagaaca cgctgtatct gcaaatgaac agcctgagag ccgaggacac 8520
   ggccgtatat tactgtgcga aaggggtttg tagtaccagc tcgtacgcct tttactacta 8580
   ctactacggt atggacgtct ggggccaggg caccctggtc accgtctcct cagtgcctgg 8640
   tgactcttcc gcaatcagtc ctgggacaga aacctcaggg acagccttta tgagttttga 8700
   tcacatctaa cagagaggat gggccagcat actctaccga cactatccga gacgcgcgag 8760
   gtgcagctgt tggagtctgg gggaggcttg gtacagcctg gggggtccct gagactctcc 8820
   tgtgcagcct ctggattcac ctttagcagc tatgctatga attgggtccg ccaggctcca 8880
   gggaaggggc tggagtgggt ctcatggatc aacaccaaca ctgggaaccc aacgtatgcc 8940
   cagggcttca caggacggtt caccatctcc agagacaatt ccaagaacac gctgtatctg 9000
   caaatgaaca gcctgagagc cgaggacacg gccgtatatt actgtgcgaa agactacggt 9060
   ggtaactcct actggtactt cgatctctgg ggccagggca ccctggtcac cgtctcctca 9120
   gtgcctggtg actcttccgc aatcagtcct gggacagaaa cctcagggac agcctttatg 9180
   agttttgatc acatctaaca gagaggatgg gccaggttta aacggtaccg ctagcatgct 9240
   ggccaggatc catgcccatg gccgcggatg cggcgcgcca tcgatatgcg tcgacattta 9300
   aatgggcccc ccggtgatat ccccgggtct agagcggccg ctaccgttcg tatattctat 9360
   cttatacgaa gttatatcga attcctgcag agatctagct tggcgtaatc atggtcatag 9420
   ctgtttcctg tgtgaaattg ttatccgctc acaattccac acaacatacg agccggaagc 9480
   ataaagtgta aagcctgggg tgcctaatga gtgagctaac tcacattaat tgcgttgcgc 9540
   tcactgcccg ctttccagtc gggaaacctg tcgtgccagc tgcattaatg aatcggccaa 9600
   cgcgcgggga gaggcggttt gcgtattggg cgctcttccg cttcctcgct cactgactcg 9660
   ctgcgctcgg tcgttcggct gcggcgagcg gtatcagctc actcaaaggc ggtaatacgg 9720
   ttatccacag aatcagggga taacgcagga aagaacatgt gagcaaaagg ccagcaaaag 9780
   gccaggaacc gtaaaaaggc cgcgttgctg gcgtttttcc ataggctccg cccccctgac 9840
   gagcatcaca aaaatcgacg ctcaagtcag aggtggcgaa acccgacagg actataaaga 9900
   taccaggcgt ttccccctgg aagctccctc gtgcgctctc ctgttccgac cctgccgctt 9960
   accggatacc tgtccgcctt tctcccttcg ggaagcgtgg cgctttctca tagctcacgc 10020
   tgtaggtatc tcagttcggt gtaggtcgtt cgctccaagc tgggctgtgt gcacgaaccc 10080
   cccgttcagc ccgaccgctg cgccttatcc ggtaactatc gtcttgagtc caacccggta 10140
   agacacgact tatcgccact ggcagcagcc actggtaaca ggattagcag agcgaggtat 10200
   gtaggcggtg ctacagagtt cttgaagtgg tggcctaact acggctacac tagaagaaca 10260
   gtatttggta tctgcgctct gctgaagcca gttaccttcg gaaaaagagt tggtagctct 10320
   tgatccggca aacaaaccac cgctggtagc ggtggttttt ttgtttgcaa gcagcagatt 10380
   acgcgcagaa aaaaaggatc tcaagaagat cctttgatct tttctacggg gtctgacgct 10440
   cagtggaacg aaaactcacg ttaagggatt ttggtcatga gattatcaaa aaggatcttc 10500
   acctagatcc ttttaaatta aaaatgaagt tttaaatcaa tctaaagtat atatgagtaa 10560
   acttggtctg acagttacca atgcttaatc agtgaggcac ctatctcagc gatctgtcta 10620
   tttcgttcat ccatagttgc ctgactcccc gtcgtgtaga taactacgat acgggagggc 10680
   ttaccatctg gccccagtgc tgcaatgata ccgcgagacc cacgctcacc ggctccagat 10740
   ttatcagcaa taaaccagcc agccggaagg gccgagcgca gaagtggtcc tgcaacttta 10800
   tccgcctcca tccagtctat taattgttgc cgggaagcta gagtaagtag ttcgccagtt 10860
   aatagtttgc gcaacgttgt tgccattgct acaggcatcg tggtgtcacg ctcgtcgttt 10920
   ggtatggctt cattcagctc cggttcccaa cgatcaaggc gagttacatg atcccccatg 10980
   ttgtgcaaaa aagcggttag ctccttcggt cctccgatcg ttgtcagaag taagttggcc 11040
   gcagtgttat cactcatggt tatggcagca ctgcataatt ctcttactgt catgccatcc 11100
   gtaagatgct tttctgtgac tggtgagtac tcaaccaagt cattctgaga atagtgtatg 11160
   cggcgaccga gttgctcttg cccggcgtca atacgggata ataccgcgcc acatagcaga 11220
   actttaaaag tgctcatcat tggaaaacgt tcttcggggc gaaaactctc aaggatctta 11280
   ccgctgttga gatccagttc gatgtaaccc actcgtgcac ccaactgatc ttcagcatct 11340
   tttactttca ccagcgtttc tgggtgagca aaaacaggaa ggcaaaatgc cgcaaaaaag 11400
   ggaataaggg cgacacggaa atgttgaata ctcatactct tcctttttca atattattga 11460
   agcatttatc agggttattg tctcatgagc ggatacatat ttgaatgtat ttagaaaaat 11520
   aaacaaatag gggttccgcg cacatttccc cgaaaagtgc cacctgacgt ctaagaaacc 11580
   attattatca tgacattaac ctataaaaat aggcgtatca cgaggccctt tcgtc 11635

<210> 146
<211> 11624
<212> DNA
<213> Artificial Sequence

<220>
<223> Synthetic DNA

<400> 146
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt catatgtagc ttacgacact 420
   gataacttcg tatatggtat tatatacgaa gttataagct tcaccatgat tgaacaagat 480
   ggattgcacg caggttctcc ggccgcttgg gtggagaggc tattcggcta tgactgggca 540
   caacagacaa tcggctgctc tgatgccgcc gtgttccggc tgtcagcgca ggggcgcccg 600
   gttctttttg tcaagaccga cctgtccggt gccctgaatg aactgcagga cgaggcagcg 660
   cggctatcgt ggctggccac gacgggcgtt ccttgcgcag ctgtgctcga cgttgtcact 720
   gaagcgggaa gggactggct gctattgggc gaagtgccgg ggcaggatct cctgtcatct 780
   caccttgctc ctgccgagaa agtatccatc atggctgatg caatgcggcg gctgcatacg 840
   cttgatccgg ctacctgccc attcgaccac caagcgaaac atcgcatcga gcgagcacgt 900
   actcggatgg aagccggtct tgtcgatcag gatgatctgg acgaagagca tcaggggctc 960
   gcgccagccg aactgttcgc caggctcaag gcgcgcatgc ccgacggcga ggatctcgtc 1020
   gtgacccatg gcgatgcctg cttgccgaat atcatggtgg aaaatggccg cttttctgga 1080
   ttcatcgact gtggccggct gggtgtggcg gaccgctatc aggacatagc gttggctacc 1140
   cgtgatattg ctgaagagct tggcggcgaa tgggctgacc gcttcctcgt gctttacggt 1200
   atcgccgctc ccgattcgca gcgcatcgcc ttctatcgcc ttcttgacga gttcttctga 1260
   actagtgcgg gactctgggg ttcgaaatga ccgaccaagc gacgcccaac ctgccatcac 1320
   gagatttcga ttccaccgcc gccttctatg aaaggttggg cttcggaatc gttttccggg 1380
   acgccggctg gatgatcctc cagcgcgggg atctcatgct ggagttcttc gcccacccca 1440
   acttgtttat tgcagcttat aatggttaca aataaagcaa tagcatcaca aatttcacaa 1500
   ataaagcatt tttttcactg cattctagtt gtggtttgtc caaactcatc aatgtatctt 1560
   atcatgtctg ttataccgtc gatctctagc tagagcttgg cgtaatcatg gtcatagctg 1620
   tttcctgtgt gcccacccca acttgtttat tgcagcttat aatggttaca aataaagcaa 1680
   tagcatcaca aatttcacaa ataaagcatt tttttcactg cattctagtt gtggtttgtc 1740
   caaactcatc aatgtatctt atcatgtctg ataacttcgt ataatgtatg ctatacgaac 1800
   ggtactcgag agctcatgcg gtaccgttta aacctggccc atcctctctg ttagatgtga 1860
   tcaaaactca taaaggctgt ccctgaggtt tctgtcccag gactgattgc ggaagagtca 1920
   ccaggcactg aggagacggt gaccagggtg ccctggcccc agagatcgaa gtaccagtag 1980
   gagttaccac cgtagtcttt cgcacagtaa tatacggccg tgtcctcggc tctcaggctg 2040
   ttcatttgca gatacagcgt gttcttggaa ttgtctctgg agatggtgaa ccgtcctgtg 2100
   aagccctggg catacgttgg gttcccagtg ttggtgttga tccatgagac ccactccagc 2160
   cccttccctg gagcctggcg gacccaattc atagcatagc tgctaaaggt gaatccagag 2220
   gctgcacagg agagtctcag ggacccccca ggctgtacca agcctccccc agactccaac 2280
   agctgcacct cgcgcgtctc ggatagtgtc ggtagagtat gctggcccat cctctctgtt 2340
   agatgtgatc aaaactcata aaggctgtcc ctgaggtttc tgtcccagga ctgattgcgg 2400
   aagagtcacc aggcactgag gagacggtga ccagggtgcc ctggccccag acgtccatac 2460
   cgtagtagta gtagtaaaag gcgtacgagc tggtactaca aacccctttc gcacagtaat 2520
   atacggccgt gtcctcggct ctcaggctgt tcatttgcag atacagcgtg ttcttggaat 2580
   tgtctctgga gatggtgaac cggccttgga aggacgggct gtatctggta tcagagtcac 2640
   caggatagat gattgagacc cactccagcc ccttccctgg agcctggcgg acccagccga 2700
   tccagtagct gctaaaggtg aatccagagg ctgcacagga gagtctcagg gaccccccag 2760
   gctgtaccaa gcctccccca gactccaaca gctgcacctc gcgactatat gtctatctat 2820
   gacatacact ctggcccatc ctctctgtta gatgtgatca aaactcataa aggctgtccc 2880
   tgaggtttct gtcccaggac tgattgcgga agagtcacca ggcactgagg agacggtgac 2940
   cagggtgccc tggccccaga aatcaaaagc accacccggg cccgcgctgg tacgggcttt 3000
   cgcacagtaa tatacggccg tgtcctcggc tctcaggctg ttcatttgca gatacagcgt 3060
   gttcttggaa ttgtctctgg agatggtgaa ccgactcttg agggacgggt tgtagttggt 3120
   gcttccacta tgattgattt ctgagaccca ctccagcccc ttccctggag cctggcggac 3180
   ccagctccag tagtaaccgc taaaggtgaa tccagaggct gcacaggaga gtctcaggga 3240
   ccccccaggc tgtaccaagc ctcccccaga ctccaacagc tgcacctcgt gacgctctgt 3300
   gcgcgagtgt agatgagtct ggcccatcct ctctgttaga tgtgatcaaa actcataaag 3360
   gctgtccctg aggtttctgt cccaggactg attgcggaag agtcaccagg cactgaggag 3420
   acggtgacca gggtgccctg gccccagtag tcaaaacccc ctctacttcg atctttcgca 3480
   cagtaatata cggccgtgtc ctcggctctc aggctgttca tttgcagata cagcgtgttc 3540
   ttggaattgt ctctggagat ggtgaaccga ctcttgaggg acgggttgta gtaggtgctc 3600
   ccactataat agatgtatga gacccactcc agccccttcc ctggagcctg gcggacccag 3660
   ccccaccagt tactactgct aaaggtgaat ccagaggctg cacaggagag tctcagggac 3720
   cccccaggct gtaccaagcc tcccccagac tccaacagct gcacctcgca cgactgtgcg 3780
   tatgtgttgc gacgacactg gcccatcctc tctgttagat gtgatcaaaa ctcataaagg 3840
   ctgtccctga ggtttctgtc ccaggactga ttgcggaaga gtcaccaggc actgaggaga 3900
   cggtgaccag ggtgccctgg ccccagatat caaaagcatc agcatagcag ctggtactac 3960
   tacaatatcc tttcgcacag taatatacgg ccgtgtcctc ggctctcagg ctgttcattt 4020
   gcagatacag cgtgttcttg gaattgtctc tggagatggt gaaccggccc ttcacggagt 4080
   ccgcgtagct tgtgctactc ccatcactat taatacgtga gacccactcc agccccttcc 4140
   ctggagcctg gcggacccag tgcatccagt agctgctaaa ggtgaatcca gaggctgcac 4200
   aggagagtct cagggacccc ccaggctgta ccaagcctcc cccagactcc aacagctgca 4260
   cctctgacgt cagatacgca gcgatcacta tcgactggcc catcctctct gttagatgtg 4320
   atcaaaactc ataaaggctg tccctgaggt ttctgtccca ggactgattg cggaagagtc 4380
   accaggcact gaggagacgg tgaccagggt gccctggccc cagtagtcaa agtaataact 4440
   ccctgaacca taggagaact tcgctttcgc acagtaatat acggccgtgt cctcggctct 4500
   caggctgttc atttgcagat acagcgtgtt cttggaattg tctctggaga tggtgaaccg 4560
   gcccttcacg gagtctgcgt agtatgtgct accaccgcta taaataactg agacccactc 4620
   cagccccttc cctggagcct ggcggaccca gctcatgtag ttgctgctaa aggtgaatcc 4680
   agaggctgca caggagagtc tcagggaccc cccaggctgt accaagcctc ccccagactc 4740
   caacagctgc acctctatgc gactatgatg acgtatacgc tatagctggc ccatcctctc 4800
   tgttagatgt gatcaaaact cataaaggct gtccctgagg tttctgtccc aggactgatt 4860
   gcggaagagt caccaggcac tgaggagacg gtgaccaggg tgccctggcc ccagtagtca 4920
   aaaggagtag cagctggccc gaagacaaga tctttcgcac agtaatatac ggccgtgtcc 4980
   tcggctctca ggctgttcat ttgcagatac agcgtgttct tggaattgtc tctggagatg 5040
   gtgaaccggc ccttcacgga gtctgcatag tatttattac ttccatcata ccatataact 5100
   gagacccact ccagcccctt ccctggagcc tggcggaccc agtgcatgcc atagctgcta 5160
   aaggtgaatc cagaggctgc acaggagagt ctcaggaccc ccccaggctg taccaagcct 5220
   cccccagact ccaacagctg cacctctgta tacgcgtcgt actgcgtata cgatcgctgg 5280
   cccatcctct ctgttagatg tgatcaaaac tcataaaggc tgtccctgag gtttctgtcc 5340
   caggactgat tgcggaagag tcaccaggca ctgaggagac ggtgaccagg gtgccctggc 5400
   cccagtagtc aaaggacgag ctgctacccc tttctttcgc acagtaatat acggccgtgt 5460
   cctcggctct caggctgttc atttgcagat acagcgtgtt cttggaattg tctctggaga 5520
   tggtgaaccg gcctttcacg ggtgcagcgt agtctgttgt cccaccatca gttttgcttt 5580
   taatacgtga gacccactcc agccccttcc ctggagcctg gcggacccag ctcatccagg 5640
   cgttgctaaa ggtgaatcca gaggctgcac aggagagtct cagggacccc ccaggctgta 5700
   ccaagcctcc cccagactcc aacagctgca cctctacgtg agcttactgc tagttcgatc 5760
   gcgtctggcc catcctctct gttagatgtg atcaaaactc ataaaggctg tccctgaggt 5820
   ttctgtccca ggactgattg cggaagagtc accaggcact gaggagacgg tgaccagggt 5880
   gccctggccc caggggtcga accagttgta agtagctccc actattttcg cacagtaata 5940
   tacggccgtg tcctcggctc tcaggctgtt catttgcaga tacagcgtgt tcttggaatt 6000
   gtctctggag atggtgaacc ggcccttcac agagtccgca tagcctatgc taccactatt 6060
   ccaactaata cctgagaccc actccagccc cttccctgga gcctggcgga cccagtgcat 6120
   ggcataatcg ctaaaggtga atccagaggc tgcacaggag agtctcaggg accccccagg 6180
   ctgtaccaag cctcccccag actccaacag ctgcacctca tagtatagaa gtcgctagaa 6240
   ctagtgcgac tggcccatcc tctctgttag atgtgatcaa aactcataaa ggctgtccct 6300
   gaggtttctg tcccaggact gattgcggaa gagtcaccag gcactgagga gacggtgacc 6360
   agggtgccct ggccccagta gtcaaagaat tccccggcgg ggtgccaccg tcatctttcg 6420
   gcacagtaat atacggccgt gtcctcggct ctcaggctgt tcatttgcag atacagcgtg 6480
   ttcttggaat tgtctctgga gatggtgaac cgggtcttca gagatgtgct gtagaattta 6540
   tcatcatccc aatcaatgcg tgagacccac tccagcccct tccctggagc ctggcggacc 6600
   cagctcacac gcattccact agtgctaaag gtgaatccag aggctgcaca ggagagtctc 6660
   agggaccccc caggctgtac caagcctccc ccagactcca acagctgcac ctcacgatct 6720
   gtaagtgtgt gtaagtcacg tcgctggccc atcctctctg ttagatgtga tcaaaactca 6780
   taaaggctgt ccctgaggtt tctgtcccag gactgattgc ggaagagtca ccaggcactg 6840
   aggagacggt gaccagggtg ccctggcccc agtagtcaaa ggtccaggcg ctgctatacg 6900
   gcccatacgc ccatcgtctt ttcgcacagt aatatacggc cgtgtcctcg gctctcaggc 6960
   tgttcatttg cagatacagc gtgttcttgg aattgtctct ggagatggtg aaccggctct 7020
   tcagagatgg gctgtagcgc ttatcatcat tccaataaat gagtgagacc cactccagcc 7080
   ccttccctgg agcctggcgg acccagccca cacccactcc actagtgcta aaggtgaatc 7140
   cagaggctgc acaggagagt ctcaggaccc cccaggctgg taccaagcct cccccagact 7200
   ccaacagctg cacctcatag cgtactagac gacgctagta tagtctctgg cccatcctct 7260
   ctgttagatg tgatcaaaac tcataaaggc tgtccctgag gtttctgtcc caggactgat 7320
   tgcggaagag tcaccaggca ctgaggagac ggtgaccagg gtgccctggc cccagacgtc 7380
   cataccgtag tagtagtagg gggcagctgg tactacaagg acgtctttcg cacagtaata 7440
   tacggccgtg tcctcggctc tcaggctgtt catttgcaga tacagcgtgt tcttggaatt 7500
   gtctctggag atggtgaacc gttcctggaa cttctgtgcg tagtttgtgt taccactgcc 7560
   aacgacgatc catgagaccc actccagccc cttccctgga gcctggcgga cccactgcac 7620
   agcagagctg ctaaaggtga atccagaggc tgcacaggag agtctcaggg accccccagg 7680
   ctgtaccaag cctcccccag actccaacag ctgcacctca cgcgtatgtc gagcgacgac 7740
   tcgtctctac tggcccatcc tctctgttag atgtgatcaa aactcataaa ggctgtccct 7800
   gaggtttctg tcccaggact gattgcggaa gagtcaccag gcactgagga gacggtgacc 7860
   agggtgccct ggccccagac gtccacaccg tagcgagctg gcacaacgac aatatcctcc 7920
   ccatatttcg cacagtaata tacggccgtg tcctcggctc tcaggctgtt catttgcaga 7980
   tacagcgtgt tcttggaatt gtctctggag atggtgaacc ggcccttcac ggagtctgcg 8040
   taatatgtgc taccagcact attactaata gttgagaccc actccagccc cttccctgga 8100
   gcctggcgga cccagttcat ggcaaagccg ctaaaggtga atccagaggc tgcacaggag 8160
   agtctcaggg accccccagg ctgtaccaag cctcccccag actccaacag ctgcacctcc 8220
   tgtagtcgtg agtagtacag ctacgtagac tggcccatcc tctctgttag atgtgatcaa 8280
   aactcataaa ggctgtccct gaggtttctg tcccaggact gattgcggaa gagtcaccag 8340
   gcactgagga gacggtgacc agggtgccct ggccccagta gtcaaagtag taataggata 8400
   accttaaatc tttcgcacag taatatacgg ccgtgtcctc ggctctcagg ctgttcattt 8460
   gcagatacag cgtgttcttg gaattgtctc tggagatggt gaaccggccc tggagcttct 8520
   gtgcatagtt tgtgttacca ttgtaagcgc tgatccatga gacccactcc agccccttcc 8580
   ctggagcctg gcggacccag ctgataccat agctgctaaa ggtgaatcca gaggctgcac 8640
   aggagagtct cagggacccc ccaggctgta ccaagcctcc cccagactcc aacagctgca 8700
   cctcgtatct ctcgcactac tatgagacgt atcactggcc catcctctct gttagatgtg 8760
   atcaaaactc ataaaggctg tccctgaggt ttctgtccca ggactgattg cggaagagtc 8820
   accaggcact gaggagacgg tgaccagggt gccctggccc cagtagtcaa agtggacgta 8880
   gtaataacca ctactagacg ctttcgcaca gtaatatacg gccgtgtcct cggctctcag 8940
   gctgttcatt tgcagataca gcgtgttctt ggaattgtct ctggagatgg tgaaccggcc 9000
   ctggaacttc tgtgaatatt ttgtgttacc attgccagcg ttgatccatg agacccactc 9060
   cagccccttc cctggagcct ggcggaccca atgcatagca tagctgctaa aggtgaatcc 9120
   agaggctgca caggagagtc tcagggaccc cccaggctgt accaagcctc ccccagactc 9180
   caacagctgc acctcctcgc gatatcgtgt ctgatctaca gtgctgttta aacgagctcg 9240
   ctagcggatc ccgcggatgc ggcgcgccat cgatatgcgt cgacatttaa atgggccccc 9300
   cggtgatatc cccgggtcta gagcggccgc taccgttcgt atattctatc ttatacgaag 9360
   ttatatcgaa ttcctgcaga gatctagctt ggcgtaatca tggtcatagc tgtttcctgt 9420
   gtgaaattgt tatccgctca caattccaca caacatacga gccggaagca taaagtgtaa 9480
   agcctggggt gcctaatgag tgagctaact cacattaatt gcgttgcgct cactgcccgc 9540
   tttccagtcg ggaaacctgt cgtgccagct gcattaatga atcggccaac gcgcggggag 9600
   aggcggtttg cgtattgggc gctcttccgc ttcctcgctc actgactcgc tgcgctcggt 9660
   cgttcggctg cggcgagcgg tatcagctca ctcaaaggcg gtaatacggt tatccacaga 9720
   atcaggggat aacgcaggaa agaacatgtg agcaaaaggc cagcaaaagg ccaggaaccg 9780
   taaaaaggcc gcgttgctgg cgtttttcca taggctccgc ccccctgacg agcatcacaa 9840
   aaatcgacgc tcaagtcaga ggtggcgaaa cccgacagga ctataaagat accaggcgtt 9900
   tccccctgga agctccctcg tgcgctctcc tgttccgacc ctgccgctta ccggatacct 9960
   gtccgccttt ctcccttcgg gaagcgtggc gctttctcat agctcacgct gtaggtatct 10020
   cagttcggtg taggtcgttc gctccaagct gggctgtgtg cacgaacccc ccgttcagcc 10080
   cgaccgctgc gccttatccg gtaactatcg tcttgagtcc aacccggtaa gacacgactt 10140
   atcgccactg gcagcagcca ctggtaacag gattagcaga gcgaggtatg taggcggtgc 10200
   tacagagttc ttgaagtggt ggcctaacta cggctacact agaagaacag tatttggtat 10260
   ctgcgctctg ctgaagccag ttaccttcgg aaaaagagtt ggtagctctt gatccggcaa 10320
   acaaaccacc gctggtagcg gtggtttttt tgtttgcaag cagcagatta cgcgcagaaa 10380
   aaaaggatct caagaagatc ctttgatctt ttctacgggg tctgacgctc agtggaacga 10440
   aaactcacgt taagggattt tggtcatgag attatcaaaa aggatcttca cctagatcct 10500
   tttaaattaa aaatgaagtt ttaaatcaat ctaaagtata tatgagtaaa cttggtctga 10560
   cagttaccaa tgcttaatca gtgaggcacc tatctcagcg atctgtctat ttcgttcatc 10620
   catagttgcc tgactccccg tcgtgtagat aactacgata cgggagggct taccatctgg 10680
   ccccagtgct gcaatgatac cgcgagaccc acgctcaccg gctccagatt tatcagcaat 10740
   aaaccagcca gccggaaggg ccgagcgcag aagtggtcct gcaactttat ccgcctccat 10800
   ccagtctatt aattgttgcc gggaagctag agtaagtagt tcgccagtta atagtttgcg 10860
   caacgttgtt gccattgcta caggcatcgt ggtgtcacgc tcgtcgtttg gtatggcttc 10920
   attcagctcc ggttcccaac gatcaaggcg agttacatga tcccccatgt tgtgcaaaaa 10980
   agcggttagc tccttcggtc ctccgatcgt tgtcagaagt aagttggccg cagtgttatc 11040
   actcatggtt atggcagcac tgcataattc tcttactgtc atgccatccg taagatgctt 11100
   ttctgtgact ggtgagtact caaccaagtc attctgagaa tagtgtatgc ggcgaccgag 11160
   ttgctcttgc ccggcgtcaa tacgggataa taccgcgcca catagcagaa ctttaaaagt 11220
   gctcatcatt ggaaaacgtt cttcggggcg aaaactctca aggatcttac cgctgttgag 11280
   atccagttcg atgtaaccca ctcgtgcacc caactgatct tcagcatctt ttactttcac 11340
   cagcgtttct gggtgagcaa aaacaggaag gcaaaatgcc gcaaaaaagg gaataagggc 11400
   gacacggaaa tgttgaatac tcatactctt cctttttcaa tattattgaa gcatttatca 11460
   gggttattgt ctcatgagcg gatacatatt tgaatgtatt tagaaaaata aacaaatagg 11520
   ggttccgcgc acatttcccc gaaaagtgcc acctgacgtc taagaaacca ttattatcat 11580
   gacattaacc tataaaaata ggcgtatcac gaggcccttt cgtc 11624

<210> 147
<211> 339
<212> DNA
<213> Artificial Sequence

<220>
<223> Synthetic DNA

<400> 147
gaggtgcagc tgttggagtc tgggggaggc ttggtacagc ctggggggtc cctgagactc 60

tcctgtgcag cctctggatt cacctttagc atctatgtca tgagctgggt ccgccaggct 120

ccagggaagg ggctggagtg ggtctcagct attagtgcta gtggtgataa cacatactac 180

gcagactccg tgaagggccg gttcaccatc tccagagaca attccaagaa cacgctgtat 240

ctgcaaatga acagcctgag agccgaggac acggccgtat attactgtgc gaaaacccct 300

acttactggg gccagggcac cctggtcacc gtctcctca 339

<210> 148
<211> 354
<212> DNA
<213> Artificial Sequence

<220>
<223> Synthetic DNA

<400> 148
gaggtgcagc tgttggagtc tgggggaggc ttggtacagc ctggggggtc cctgagactc 60

tcctgtgcag cctctggatt cacctttagc agctttttca tgaactgggt ccgccaggct 120

ccagggaagg ggctggagtg ggtctcaact attagtgcca gtgatggtag gacacactac 180

gcagactccg tgaagggccg gttcaccatc tccagagaca attccaagaa cacgctgtat 240

ctgcaaatga acagcctgag agccgaggac acggccgtat attactgtgc gaaagatctc 300

cactggtacg gtatggacgc ctggggccag ggcaccctgg tcaccgtctc ctca 354

<210> 149
<211> 375
<212> DNA
<213> Artificial Sequence

<220>
<223> Synthetic DNA

<400> 149

gaggtgcagc tgttggagtc tgggggaggc ttggtacagc ctggggggtc cctgagactc 60

tcctgtgcag cctctggatt cacctttagc agctttgaca tgagctgggt ccgccaggct 120

ccagggaagg ggctggagtg ggtctcaagt attagtggta gtggtattag aacatattac 180

gcagactccg tgaagggccg gttcaccatc tccagagaca attccaagaa cacgctgtat 240

ctgcaaatga acagcctgag agccgaggac acggccgtat attactgtgc gaaagatggg 300

gtgtatagca gtggccagta ctaccattac ggtatggacg tctggggcca gggcaccctg 360

gtcaccgtct cctca 375

<210> 150
<211> 387
<212> DNA
<213> Artificial Sequence

<220>
<223> Synthetic DNA

<400> 150
gaggtgcagc tgttggagtc tgggggaggc ttggtacagc ctggggggtc cctgagactc 60

tcctgtgcag cctctggatt cacctttagc acctttgtca tgacgtgggt ccgccaggct 120

ccagggaagg ggctggagtg ggtctcagct attactggta gtgggggtga cacatactac 180

gcagactccg tgaagggccg gttcaccatc tccagagaca attccaagaa cacgctgtat 240

ctgcaaatga acagcctgag agccgaggac acggccgtat attactgtgc gaaagatctc 300

cacgtagaag atgttttggc tggagaagat gttttggctg ggggtctgga cgtctggggc 360

cagggcaccc tggtcaccgt ctcctca 387

<210> 151
<211> 363
<212> DNA
<213> Artificial Sequence

<220>
<223> Synthetic DNA

<400> 151
gaggtgcagc tgttggagtc tgggggaggc ttggtacagc ctggggggtc cctgagactc 60

tcctgtgcag cctctggatt cacctttagc aagtacctta tgaggtgggt ccgccaggct 120

ccagggaagg ggctggagtg ggtctcaagt attagtgaca gtggtgaagg cccaacctat 180

tcagactccg tgaagggccg gttcaccatc tccagagaca attccaagaa cacgctgtat 240

ctgcaaatga acagcctgag agccgaggac acggccgtat attactgtgc gaaatgcgat 300

ggttcggggc gtgactgcgg tatggacgtc tggggccagg gcaccctggt caccgtctcc 360

tca 363

<210>  152
<211>  339
<212>  DNA
<213>  Artificial Sequence

<220>
<223>  Synthetic DNA

<400>  152
gaggtgcagc tgttggagtc tgggggaggc ttggtacagc ctggggggtc cctgagactc      60

tcctgtgcag cctctggatt cacctttagc aactatgcca tgacctgggt ccgccaggct     120

ccagggaagg ggctggagtg ggtctcagct attagtaatt atggtggtag cacatactac     180

tcagactccg tgaagggccg gttcaccatc tccagagaca attccaagaa cacgctgtat     240

ctgcaaatga acagcctgag agccgaggac acggccgtat attactgtgc gaaatacttt     300

gacttctggg gccagggcac cctggtcacc gtctcctca                            339


<210>  153
<211>  381
<212>  DNA
<213>  Artificial Sequence

<220>
<223>  Synthetic DNA

<400>  153
gaggtgcagc tgttggagtc tgggggaggc ttggtacagc ctggggggtc cctgagactc      60

tcctgtgcag cctctggatt cacctttagc acctatgtca tgggctgggt ccgccaggct     120

ccagggaagg ggctggagtg ggtctcaggt attagtggtc gtggtggtag cacatactac     180

gcagactccg tgaagggccg gttcaccatc tccagagaca attccaagaa cacgctgtat     240

ctgcaaatga acagcctgag agccgaggac acggccgtat attactgtgc gaaagacaac     300

tcttacgatt tttggagagg ttactactac tactacggta tggacgtctg gggccagggc     360

accctggtca ccgtctcctc a                                               381


<210>  154
<211>  369
<212>  DNA
<213>  Artificial Sequence

<220>
<223>  Synthetic DNA

<400>  154
gaggtgcagc tgttggagtc tgggggaggc ttggtacagc ctggggggtc cctgagactc      60

tcctgtgcag cctctggatt cacctttagc agctattcca tgagctgggt ccgccaggct     120

ccagggaagg ggctggagtg ggtctcagct attagtggca ctggtggtgc catatactac     180

gcagactccg tgaagggccg gttcaccatc tccagagaca attccaagaa cacgctgtat     240

ctgcaaatga acagcctgag agccgaggac acggccgtat attactgtgc gaaagggggt 300

ctcaccaaat attgtagtgg tgattactgt cactattggg gccagggcac cctggtcacc 360

gtctcctca 369

<210> 155
<211> 351
<212> DNA
<213> Artificial Sequence

<220>
<223> Synthetic DNA

<400> 155
gaggtgcagc tgttggagtc tgggggaggc ttggtacagc ctggggggtc cctgagactc 60

tcctgtgcag cctctggatt cacctttagc aactttgcca tgacctgggt ccgccaggct 120

ccagggaagg ggctggagtg ggtctcaact gttactagta gtggtgctgg cacatactac 180

gcagactccg tgaagggccg gttcaccatc tccagagaca attccaagaa cacgctgtat 240

ctgcaaatga acagcctgag agccgaggac acggccgtat attactgtgc gaaatctctt 300

ggcagcagcc ttgactactg gggccagggc accctggtca ccgtctcctc a 351

<210> 156
<211> 360
<212> DNA
<213> Artificial Sequence

<220>
<223> Synthetic DNA

<400> 156
gaggtgcagc tgttggagtc tgggggaggc ttggtacagc ctggggggtc cctgagactc 60

tcctgtgcag cctctggatt cacctttagc aactttgacc tcagctgggt ccgccaggct 120

ccagggaagg ggctggagtg ggtctcagct attcgtggta gcggtagtag cgctttctac 180

gcagactccg tgcagggccg gttcaccatc tccagagaca attccaagaa cacgctgtat 240

ctgcaaatga acagcctgag agccgaggac acggccgtat attactgtgc gaaattatcc 300

tggcattact ctggttgggg gaatccttgg ggccagggca ccctggtcac cgtctcctca 360

<210> 157
<211> 357
<212> DNA
<213> Artificial Sequence

<220>
<223> Synthetic DNA

<400> 157
gaggtgcagc tgttggagtc tgggggaggc ttggtacagc ctggggggtc cctgagactc 60

tcctgtgcag cctctggatt cacctttagc aactttggca tggactgggt ccgccaggct 120

ccagggaagg ggctggagtg ggtctcaact attactgtca gtggtgaaaa cacatattac 180

gcagactccg tgaagggccg gttcaccatc tccagagaca attccaagaa cacgctgtat 240

ctgcaaatga acagcctgag agccgaggac acggccgtat attactgtgc gaaaggcttt 300

agcagtggct ggtcatttga ctactggggc cagggcaccc tggtcaccgt ctcctca 357

<210> 158
<211> 363
<212> DNA
<213> Artificial Sequence

<220>
<223> Synthetic DNA

<400> 158
gaggtgcagc tgttggagtc tgggggaggc ttggtacagc ctggggggtc cctgagactc 60

tcctgtgcag cctctggatt cacctttagc agttatgccc tgagctgggt ccgccaggct 120

ccagggaagg ggctggagtg ggtctcactt gttagtggta gtgctattag tacatactac 180

gcagactccg tgaagggccg gttcaccatc tccagagaca attccaagaa cacgctgtat 240

ctgcaaatga acagcctgag agccgaggac acggccgtat attactgtgc gaaaggggct 300

cgagcccacc tctactacga tatggacgtc tggggccagg gcaccctggt caccgtctcc 360

tca 363

<210> 159
<211> 360
<212> DNA
<213> Artificial Sequence

<220>
<223> Synthetic DNA

<400> 159
gaggtgcagc tgttggagtc tgggggaggc ttggtacagc ctggggggtc cctgagactc 60

tcctgtgcag cctctggatt cacctttagc acctttttca tgagctgggt ccgccaggct 120

ccagggaagg ggctggagtg ggtctcaact aatactggtg atggtggtag tacatactac 180

tcagactccg tgaagggccg gttcaccatc tccagagaca attccaagaa cacgctgtat 240

ctgcaaatga acagcctgag agccgaggac acggccgtat attactgtgc gaaaactact 300

atggttcggg gagttatgct tgactactgg ggccagggca ccctggtcac cgtctcctca 360

<210> 160
<211> 375
<212> DNA
<213> Artificial Sequence

<220>
<223> Synthetic DNA

<400>  160
   gaggtgcagc tgttggagtc tgggggaggc ttggtacagc ctggggggtc cctgagactc      60
   tcctgtgcag cctctggatt cacctttagc agatatgcca tgggctgggt ccgccaggct     120
   ccagggaagg ggctggagtg ggtctcaagt atgagtgcta gtgctggcag cacatactac     180
   gcagactccg tgaggggccg gttcaccatc tccagagaca attccaagaa cacgctgtat     240
   ctgcaaatga acagcctgag agccgaggac acggccgtat attactgtgc gaaagatctg     300
   gttcacgcgg atatagtggc cctggggtgt ggccctgact actggggcca gggcaccctg     360
   gtcaccgtct cctca                                                      375


<210>  161
<211>  381
<212>  DNA
<213>  Artificial Sequence

<220>
<223>  Synthetic DNA

<400>  161
gaggtgcagc tgttggagtc tgggggaggc ttggtacagc ctggggggtc cctgagactc      60

tcctgtgcag cctctggatt cacctttagc aactttgcca taagctgggt ccgccaggct     120

ccagggaagg ggctggagtg ggtctcaggt atcagtggta ctggtggtag gacatactac     180

gcagactccg tgaagggccg gttcaccatc tccagagaca attccaagaa cacgctgtat     240

ctgcaaatga acagcctgag agccgaggac acggccgtat attactgtgc gaaaggggtc     300

gatgctacat atagtgccac ctggtacgtg gggggctact atgagtattg gggccagggc     360

accctggtca ccgtctcctc a                                               381


<210>  162
<211>  6974
<212>  DNA
<213>  Artificial Sequence

<220>
<223>  Synthetic DNA

<400>  162
ccgcggggcg cgccgaggtg cagctgttgg agtctggggg aggcttggta cagcctgggg      60

ggtccctgag actctcctgt gcagcctctg gattcacctt tagcatctat gtcatgagct     120

gggtccgcca ggctccaggg aaggggctgg agtgggtctc agctattagt gctagtggtg     180

ataacacata ctacgcagac tccgtgaagg gccggttcac catctccaga gacaattcca     240

agaacacgct gtatctgcaa atgaacagcc tgagagccga ggacacggcc gtatattact     300

gtgcgaaaac ccctacttac tggggccagg gcaccctggt caccgtctcc tcagatgaga     360

tttgatagaa acctcattga gaccccgctg atgaccccat gagaccacat agagacccta     420

cagagaccca gagaggtcag agcctgcctg gaggaggtgc agctgttgga gtctggggga 480

ggcttggtac agcctggggg gtccctgaga ctctcctgtg cagcctctgg attcaccttt 540

agcagctttt tcatgaactg ggtccgccag gctccaggga aggggctgga gtgggtctca 600

actattagtg ccagtgatgg taggacacac tacgcagact ccgtgaaggg ccggttcacc 660

atctccagag acaattccaa gaacacgctg tatctgcaaa tgaacagcct gagagccgag 720

gacacggccg tatattactg tgcgaaagat ctccactggt acggtatgga cgcctggggc 780

cagggcaccc tggtcaccgt ctcctcatgg gctctgtcac cgtcatcagc accactgcca 840

tagcctgaga tgtcatagag agccatagag acaccactga aaccccataa aggccctgta 900

gggacccgag gtgcagctgt tggagtctgg gggaggcttg gtacagcctg gggggtccct 960

gagactctcc tgtgcagcct ctggattcac ctttagcagc tttgacatga gctgggtccg 1020

ccaggctcca gggaaggggc tggagtgggt ctcaagtatt agtggtagtg gtattagaac 1080

atattacgca gactccgtga agggccggtt caccatctcc agagacaatt ccaagaacac 1140

gctgtatctg caaatgaaca gcctgagagc cgaggacacg gccgtatatt actgtgcgaa 1200

agatggggtg tatagcagtg gccagtacta ccattacggt atggacgtct ggggccaggg 1260

caccctggtc accgtctcct cacgccatca gccctgtggg gaaggacaga gagcgctgac 1320

ggagccgccg ccacacaacc tcgtggagct gggcctcata aaacccccag agacccccag 1380

aggaggtgca gctgttggag tctgggggag gcttggtaca gcctgggggg tccctgagac 1440

tctcctgtgc agcctctgga ttcaccttta gcacctttgt catgacgtgg gtccgccagg 1500

ctccagggaa ggggctggag tgggtctcag ctattactgg tagtgggggt gacacatact 1560

acgcagactc cgtgaagggc cggttcacca tctccagaga caattccaag aacacgctgt 1620

atctgcaaat gaacagcctg agagccgagg acacggccgt atattactgt gcgaaagatc 1680

tccacgtaga agatgttttg gctggagaag atgttttggc tgggggtctg gacgtctggg 1740

gccagggcac cctggtcacc gtctcctcag cggggttcgg acacgaccga aaggactcgt 1800

ggtttcctgc cccgaaatag ctctgatgtg ggacagctgg gaggtgttct gcagctgggc 1860

tgtggtcgcg aggtgcagct gttggagtct gggggaggct tggtacagcc tggggggtcc 1920

ctgagactct cctgtgcagc ctctggattc acctttagca agtaccttat gaggtgggtc 1980

cgccaggctc cagggaaggg gctggagtgg gtctcaagta ttagtgacag tggtgaaggc 2040

ccaacctatt cagactccgt gaagggccgg ttcaccatct ccagagacaa ttccaagaac 2100

acgctgtatc tgcaaatgaa cagcctgaga gccgaggaca cggccgtata ttactgtgcg 2160

aaatgcgatg gttcgggcgg tgactgcggt atggacgtct ggggccaggg caccctggtc 2220

accgtctcct cagagcacga aaataggatg gaaaggggag aaaaaaataa aacatcgact 2280

gaaaggggcc aaagagagca tcggccgcag ctgctccatc tgctggcacg gcgaggtgca 2340

gctgttggag tctgggggag gcttggtaca gcctgggggg tccctgagac tctcctgtgc 2400

agcctctgga ttcaccttta gcaactatgc catgacctgg gtccgccagg ctccagggaa 2460

ggggctggag tgggtctcag ctattagtaa ttatggtggt agcacatact actcagactc 2520

cgtgaagggc cggttcacca tctccagaga caattccaag aacacgctgt atctgcaaat 2580

gaacagcctg agagccgagg acacggccgt atattactgt gcgaaatact ttgacttctg 2640

gggccagggc accctggtca ccgtctcctc aggggtctgc acgtctcaga agaggcacaa 2700

cccgctccac agctcgctgc aggggtggtg gcatcccctg ctgaccccac gcagctgcac 2760

tggctgcgaa agaggtgcag ctgttggagt ctgggggagg cttggtacag cctggggggt 2820

ccctgagact ctcctgtgca gcctctggat tcacctttag cacctatgtc atgggctggg 2880

tccgccaggc tccagggaag gggctggagt gggtctcagg tattagtggt cgtggtggta 2940

gcacatacta cgcagactcc gtgaagggcc ggttcaccat ctccagagac aattccaaga 3000

acacgctgta tctgcaaatg aacagcctga gagccgagga cacggccgta tattactgtg 3060

cgaaagacaa ctcttacgat ttttggagag gttactacta ctactacggt atggacgtct 3120

ggggccaggg caccctggtc accgtctcct caggtctctc ttatgaaccg ggcctgaaga 3180

ccacccatag gacgcacatg ctgtgatggt cacgtgtagg aatgctgccc cgcaccaaca 3240

gggtgtcctt atgaggtgca gctgttggag tctgggggag gcttggtaca gcctgggggg 3300

tccctgagac tctcctgtgc agcctctgga ttcaccttta gcagctattc catgagctgg 3360

gtccgccagg ctccagggaa ggggctggag tgggtctcag ctattagtgg cactggtggt 3420

gccatatact acgcagactc cgtgaagggc cggttcacca tctccagaga caattccaag 3480

aacacgctgt atctgcaaat gaacagcctg agagccgagg acacggccgt atattactgt 3540

gcgaaagggg gtctcaccaa atattgtagt ggtgattact gtcactattg gggccagggc 3600

accctggtca ccgtctcctc atctaaatgg tctttacgtg gtttcagtca catctctatg 3660

gctctctatg acatctcagt ggggtctctt tgtggtcgga gtgtggtccg gctccgcgag 3720

ggaggtgcag ctgttggagt ctgggggagg cttggtacag cctggggggt ccctgagact 3780

ctcctgtgca gcctctggat tcacctttag caactttgcc atgacctggg tccgccaggc 3840

tccagggaag gggctggagt gggtctcaac tgttactagt agtggtgctg gcacatacta 3900

cgcagactcc gtgaagggcc ggttcaccat ctccagagac aattccaaga acacgctgta 3960

tctgcaaatg aacagcctga gagccgagga cacggccgta tattactgtg cgaaatctct 4020

tggcagcagc cttgactact ggggccaggg caccctggtc accgtctcct cactctatga 4080

gatctcagtg atgtctcgaa gggaattcaa cgcaatctat atgagatctc ttcggagtcg 4140

caatgaggtc tctattagaa ctctatgggg tcgaggtgca gctgttggag tctgggggag 4200

gcttggtaca gcctgggggg tccctgagac tctcctgtgc agcctctgga ttcaccttta 4260

gcaactttga cctcagctgg gtccgccagg ctccagggaa ggggctggag tgggtctcag 4320

ctattcgtgg tagcggtagt agcgctttct acgcagactc cgtgcagggc cggttcacca 4380

tctccagaga caattccaag aacacgctgt atctgcaaat gaacagcctg agagccgagg 4440

acacggccgt atattactgt gcgaaattat cctggcatta ctctggttgg gggaatcctt 4500

ggggccaggg caccctggtc accgtctcct caggtgggct tcggcccttt ccggggtctt 4560

tagggcatct acggggtctc actgtgttct cagtggggtc tctatgaggt gttgactgaa 4620

cctcatcggg atgaggtgca gctgttggag tctgggggag gcttggtaca gcctgggggg 4680

tccctgagac tctcctgtgc agcctctgga ttcaccttta gcaactttgg catggactgg 4740

gtccgccagg ctccagggaa ggggctggag tgggtctcaa ctattactgt cagtggtgaa 4800

aacacatatt acgcagactc cgtgaagggc cggttcacca tctccagaga caattccaag 4860

aacacgctgt atctgcaaat gaacagcctg agagccgagg acacggccgt atattactgt 4920

gcgaaaggct ttagcagtgg ctggtcattt gactactggg gccagggcac cctggtcacc 4980

gtctcctcag ctctcctaag aacagaacaa gccctgatct ccttcccccc gtgatttcgc 5040

caccatcagc cctcatagct cccatctgct cctcttggct gccccgccag aggtgcagct 5100

gttggagtct gggggaggct tggtacagcc tggggggtcc ctgagactct cctgtgcagc 5160

ctctggattc acctttagca gttatgccct gagctgggtc cgccaggctc cagggaaggg 5220

gctggagtgg gtctcacttg ttagtggtag tgctattagt acatactacg cagactccgt 5280

gaagggccgg ttcaccatct ccagagacaa ttccaagaac acgctgtatc tgcaaatgaa 5340

cagcctgaga gccgaggaca cggccgtata ttactgtgcg aaaggggctc gagcccacct 5400

ctactacgat atggacgtct ggggccaggg caccctggtc accgtctcct cagctgagag 5460

aggggatggg ctccgtgccc acattggggc tcagctccac gctgctgctg tatggagccg 5520

tgcgatgtgg ggctgtgctg ggcacggaca cggaggtgca gctgttggag tctgggggag 5580

gcttggtaca gcctgggggg tccctgagac tctcctgtgc agcctctgga ttcaccttta 5640

gcaccttttt catgagctgg gtccgccagg ctccagggaa ggggctggag tgggtctcaa 5700

ctaatactgg tgatggtggt agtacatact actcagactc cgtgaagggc cggttcacca 5760

tctccagaga caattccaag aacacgctgt atctgcaaat gaacagcctg agagccgagg 5820

acacggccgt atattactgt gcgaaaacta ctatggttcg ggagttatgc cttgactact 5880

ggggccaggg caccctggtc accgtctcct caatgcagag ctgtgtgctg ggacgaagaa 5940

gtggagctgt gcgtggaagc acacaacggc tgagttctga agagaccctc tgtcctctcg 6000

tccccccgct gtgaggtgca gctgttggag tctgggggag gcttggtaca gcctgggggg 6060

tccctgagac tctcctgtgc agcctctgga ttcaccttta gcagatatgc catgggctgg 6120

gtccgccagg ctccagggaa ggggctggag tgggtctcaa gtatgagtgc tagtgctggc 6180

agcacatact acgcagactc cgtgaggggc cggttcacca tctccagaga caattccaag 6240

aacacgctgt atctgcaaat gaacagcctg agagccgagg acacggccgt atattactgt 6300

gcgaaagatc tggttcacgc ggatatagtg gccctggggt gtggccctga ctactggggc 6360

cagggcaccc tggtcaccgt ctcctcagac cccattgatg tcccattgat accccagacg 6420

gactccactg aagctcaagc gaggccgggg cgcccctgga ggggcagcca agatctcctt 6480

ccctcccgag gtgcagctgt tggagtctgg gggaggcttg gtacagcctg gggggtccct 6540

gagactctcc tgtgcagcct ctggattcac ctttagcaac tttgccataa gctgggtccg 6600

ccaggctcca gggaaggggc tggagtgggt ctcaggtatc agtggtactg gtggtaggac 6660

atactacgca gactccgtga agggccggtt caccatctcc agagacaatt ccaagaacac 6720

gctgtatctg caaatgaaca gcctgagagc cgaggacacg gccgtatatt actgtgcgaa 6780

aggggtcgat gctacatata gtgccacctg gtacgtgggg ggctactatg agtattgggg 6840

ccagggcacc ctggtcaccg tctcctcaac accctttact tacatcggtc agatctaata 6900

aagaccacat tcagatccca tgcaaagcgc attgatgccc ctcagagacc ccacagagtc 6960

ccctcagaat cgat 6974

<210> 163
<211> 10804
<212> DNA
<213> Artificial Sequence

<220>
<223> Synthetic DNA

<400> 163
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgaatt gacgcgtatt gggatggaac 420

catatgtagc ttacgacact gataacttcg tatatggtat tatatacgaa gttataagct 480

tcaccatggc caagcctttg tctcaagaag aatccaccct cattgaaaga gcaacggcta 540

caatcaacag catccccatc tctgaagact acagcgtcgc cagcgcagct ctctctagcg 600

acggccgcat cttcactggt gtcaatgtat atcattttac tgggggacct tgtgcagaac 660

tcgtggtgct gggcactgct gctgctgcgg cagctggcaa cctgacttgt atcgtcgcga 720

tcggaaatga gaacaggggc atcttgagcc cctgcggacg gtgtcgacag gtgcttctcg 780

atctgcatcc tgggatcaaa gcgatagtga aggacagtga tggacagccg acggcagttg 840

ggattcgtga attgctgccc tctggttatg tgtgggaggg ctaaactagt gcacttcgtg 900

gccgaggagc aggactgaca cgtgctacga gatttcgatt ccaccgccgc cttctatgaa 960

aggttgggct tcggaatcgt tttccgggac gccggctgga tgatcctcca gcgcggggat 1020

ctcatgctgg agttcttcgc ccaccccaac ttgtttattg cagcttataa tggttacaaa 1080

taaagcaata gcatcacaaa tttcacaaat aaagcatttt tttcactgca ttctagttgt 1140

ggtttgtcca aactcatcaa tgtatcttat catgtctgta taccgtcgac ctctagctag 1200

agcttggcgt aatcatggtc atagctgttt cctgtgtgcc caccccaact tgtttattgc 1260

agcttataat ggttacaaat aaagcaatag catcacaaat ttcacaaata aagcattttt 1320

ttcactgcat tctagttgtg gtttgtccaa actcatcaat gtatcttatc atgtctgata 1380

acttcgtata ttctatctta tacgaacggt actcgagagc tcatgcggta ccgctagcat 1440

gctggccagg atccatgccc atggccgcgg ggcgcgccga ggtgcagctg ttggagtctg 1500

ggggaggctt ggtacagcct ggggggtccc tgagactctc ctgtgcagcc tctggattca 1560

cctttagcat ctatgtcatg agctgggtcc gccaggctcc agggaagggg ctggagtggg 1620

tctcagctat tagtgctagt ggtgataaca catactacgc agactccgtg aagggccggt 1680

tcaccatctc cagagacaat tccaagaaca cgctgtatct gcaaatgaac agcctgagag 1740

ccgaggacac ggccgtatat tactgtgcga aaacccctac ttactggggc cagggcaccc 1800

tggtcaccgt ctcctcagat gagatttgat agaaacctca ttgagacccc gctgatgacc 1860

ccatgagacc acatagagac cctacagaga cccagagagg tcagagcctg cctggaggag 1920

gtgcagctgt tggagtctgg gggaggcttg gtacagcctg gggggtccct gagactctcc 1980

tgtgcagcct ctggattcac ctttagcagc tttttcatga actgggtccg ccaggctcca 2040

gggaaggggc tggagtgggt ctcaactatt agtgccagtg atggtaggac acactacgca 2100

gactccgtga agggccggtt caccatctcc agagacaatt ccaagaacac gctgtatctg 2160

caaatgaaca gcctgagagc cgaggacacg gccgtatatt actgtgcgaa agatctccac 2220

tggtacggta tggacgcctg gggccagggc accctggtca ccgtctcctc atgggctctg 2280

tcaccgtcat cagcaccact gccatagcct gagatgtcat agagagccat agagacacca 2340

ctgaaacccc ataaaggccc tgtagggacc cgaggtgcag ctgttggagt ctgggggagg 2400

cttggtacag cctggggggt ccctgagact ctcctgtgca gcctctggat tcacctttag 2460

cagctttgac atgagctggg tccgccaggc tccagggaag gggctggagt gggtctcaag 2520

tattagtggt agtggtatta gaacatatta cgcagactcc gtgaagggcc ggttcaccat 2580
ctccagagac aattccaaga acacgctgta tctgcaaatg aacagcctga gagccgagga 2640
cacggccgta tattactgtg cgaaagatgg ggtgtatagc agtggccagt actaccatta 2700
cggtatggac gtctggggcc agggcaccct ggtcaccgtc tcctcacgcc atcagccctg 2760
tggggaagga cagagagcgc tgacggagcc gccgccacac aacctcgtgg agctgggcct 2820
cataaaaccc ccagagaccc ccagaggagg tgcagctgtt ggagtctggg ggaggcttgg 2880
tacagcctgg ggggtccctg agactctcct gtgcagcctc tggattcacc tttagcacct 2940
ttgtcatgac gtgggtccgc caggctccag ggaaggggct ggagtgggtc tcagctatta 3000
ctggtagtgg gggtgacaca tactacgcag actccgtgaa gggccggttc accatctcca 3060
gagacaattc caagaacacg ctgtatctgc aaatgaacag cctgagagcc gaggacacgg 3120
ccgtatatta ctgtgcgaaa gatctccacg tagaagatgt tttggctgga gaagatgttt 3180
tggctggggg tctggacgtc tggggccagg gcaccctggt caccgtctcc tcagcggggt 3240
tcggacacga ccgaaaggac tcgtggtttc ctgccccgaa atagctctga tgtggggaca 3300
gctggaggtg ttctgcagct gggctgtggt cgcgaggtgc agctgttgga gtctggggga 3360
ggcttggtac agcctggggg gtccctgaga ctctcctgtg cagcctctgg attcaccttt 3420
agcaagtacc ttatgaggtg ggtccgccag gctccaggga aggggctgga gtgggtctca 3480
agtattagtg acagtggtga aggcccaacc tattcagact ccgtgaaggg ccggttcacc 3540
atctccagag acaattccaa gaacacgctg tatctgcaaa tgaacagcct gagagccgag 3600
gacacggccg tatattactg tgcgaaatgc gatggttcgg ggcgtgactg cggtatggac 3660
gtctggggcc agggcaccct ggtcaccgtc tcctcagagc acgaaaatag gatggaaagg 3720
ggagaaaaaa ataaaacatc gactgaaagg ggccaaagag agcatcggcc gcagctgctc 3780
catctgctgg cacggcgagg tgcagctgtt ggagtctggg ggaggcttgg tacagcctgg 3840
ggggtccctg agactctcct gtgcagcctc tggattcacc tttagcaact atgccatgac 3900
ctgggtccgc caggctccag ggaaggggct ggagtgggtc tcagctatta gtaattatgg 3960
tggtagcaca tactactcag actccgtgaa gggccggttc accatctcca gagacaattc 4020
caagaacacg ctgtatctgc aaatgaacag cctgagagcc gaggacacgg ccgtatatta 4080
ctgtgcgaaa tactttgact tctggggcca gggcaccctg gtcaccgtct cctcaggggt 4140
ctgcacgtct cagaagaggc acaacccgct ccacagctcg ctgcaggggt ggtggcatcc 4200
cctgctgacc ccacgcagct gcactggctg cgaaagaggt gcagctgttg gagtctgggg 4260
gaggcttggt acagcctggg gggtccctga gactctcctg tgcagcctct ggattcacct 4320
ttagcaccta tgtcatgggc tgggtccgcc aggctccagg gaaggggctg gagtgggtct 4380
caggtattag tggtcgtggt ggtagcacat actacgcaga ctccgtgaag ggccggttca 4440

ccatctccag agacaattcc aagaacacgc tgtatctgca aatgaacagc ctgagagccg 4500

aggacacggc cgtatattac tgtgcgaaag acaactctta cgatttttgg agaggttact 4560

actactacta cggtatggac gtctggggcc agggcaccct ggtcaccgtc tcctcaggtc 4620

tctcttatga accgggcctg aagaccaccc ataggacgca catgctgtga tggtcacgtg 4680

taggaatgct gccccgcacc aacagggtgt ccttatgagg tgcagctgtt ggagtctggg 4740

ggaggcttgg tacagcctgg ggggtccctg agactctcct gtgcagcctc tggattcacc 4800

tttagcagct attccatgag ctgggtccgc caggctccag ggaaggggct ggagtgggtc 4860

tcagctatta gtggcactgg tggtgccata tactacgcag actccgtgaa gggccggttc 4920

accatctcca gagacaattc caagaacacg ctgtatctgc aaatgaacag cctgagagcc 4980

gaggacacgg ccgtatatta ctgtgcgaaa gggggtctca ccaaatattg tagtggtgat 5040

tactgtcact attggggcca gggcaccctg gtcaccgtct cctcatctaa atggtcttta 5100

cgtggtttca gtcacatctc tatggctctc tatgacatct cagtggggtc tctttgtggt 5160

cggagtgtgg tccggctccg cgagggaggt gcagctgttg gagtctgggg gaggcttggt 5220

acagcctggg gggtccctga gactctcctg tgcagcctct ggattcacct ttagcaactt 5280

tgccatgacc tgggtccgcc aggctccagg gaaggggctg gagtgggtct caactgttac 5340

tagtagtggt gctggcacat actacgcaga ctccgtgaag ggccggttca ccatctccag 5400

agacaattcc aagaacacgc tgtatctgca aatgaacagc ctgagagccg aggacacggc 5460

cgtatattac tgtgcgaaat ctcttggcag cagccttgac tactggggcc agggcaccct 5520

ggtcaccgtc tcctcactct atgagatctc agtgatgtct cgaagggaat tcaacgcaat 5580

ctatatgaga tctcttcgga gtcgcaatga ggtctctatt agaactctat ggggtcgagg 5640

tgcagctgtt ggagtctggg ggaggcttgg tacagcctgg ggggtccctg agactctcct 5700

gtgcagcctc tggattcacc tttagcaact ttgacctcag ctgggtccgc caggctccag 5760

ggaaggggct ggagtgggtc tcagctattc gtggtagcgg tagtagcgct ttctacgcag 5820

actccgtgca gggccggttc accatctcca gagacaattc caagaacacg ctgtatctgc 5880

aaatgaacag cctgagagcc gaggacacgg ccgtatatta ctgtgcgaaa ttatcctggc 5940

attactctgg ttgggggaat ccttggggcc agggcaccct ggtcaccgtc tcctcaggtg 6000

ggcttcggcc ctttccgggg tctttagggc atctacgggg tctcactgtg ttctcagtgg 6060

ggtctctatg aggtgttgac tgaacctcat cgggatgagg tgcagctgtt ggagtctggg 6120

ggaggcttgg tacagcctgg ggggtccctg agactctcct gtgcagcctc tggattcacc 6180

tttagcaact ttggcatgga ctgggtccgc caggctccag ggaaggggct ggagtgggtc 6240

tcaactatta ctgtcagtgg tgaaaacaca tattacgcag actccgtgaa gggccggttc 6300

accatctcca gagacaattc caagaacacg ctgtatctgc aaatgaacag cctgagagcc 6360

gaggacacgg ccgtatatta ctgtgcgaaa ggctttagca gtggctggtc atttgactac 6420

tggggccagg gcaccctggt caccgtctcc tcagctctcc taagaacaga acaagccctg 6480

atctccttcc ccccgtgatt tcgccaccat cagccctcat agctcccatc tgctcctctt 6540

ggctgccccg ccagaggtgc agctgttgga gtctggggga ggcttggtac agcctggggg 6600

gtccctgaga ctctcctgtg cagcctctgg attcaccttt agcagttatg ccctgagctg 6660

ggtccgccag gctccaggga aggggctgga gtgggtctca cttgttagtg gtagtgctat 6720

tagtacatac tacgcagact ccgtgaaggg ccggttcacc atctccagag acaattccaa 6780

gaacacgctg tatctgcaaa tgaacagcct gagagccgag gacacggccg tatattactg 6840

tgcgaaaggg gctcgagccc acctctacta cgatatggac gtctggggcc agggcaccct 6900

ggtcaccgtc tcctcagctg agagagggga tgggctccgt gcccacattg gggctcagct 6960

ccacgctgct gctgtatgga gccgtgcgat gtggggctgt gctgggcacg gacacggagg 7020

tgcagctgtt ggagtctggg ggaggcttgg tacagcctgg ggggtccctg agactctcct 7080

gtgcagcctc tggattcacc tttagcacct ttttcatgag ctgggtccgc caggctccag 7140

ggaaggggct ggagtgggtc tcaactaata ctggtgatgg tggtagtaca tactactcag 7200

actccgtgaa gggccggttc accatctcca gagacaattc caagaacacg ctgtatctgc 7260

aaatgaacag cctgagagcc gaggacacgg ccgtatatta ctgtgcgaaa actactatgg 7320

ttcggggagt tatgcttgac tactggggcc agggcaccct ggtcaccgtc tcctcaatgc 7380

agagctgtgt gctgggacga agaagtggag ctgtgcgtgg aagcacacaa cggctgagtt 7440

ctgaagagac cctctgtcct ctcgtccccc cgctgtgagg tgcagctgtt ggagtctggg 7500

ggaggcttgg tacagcctgg ggggtccctg agactctcct gtgcagcctc tggattcacc 7560

tttagcagat atgccatggg ctgggtccgc caggctccag ggaaggggct ggagtgggtc 7620

tcaagtatga gtgctagtgc tggcagcaca tactacgcag actccgtgag gggccggttc 7680

accatctcca gagacaattc caagaacacg ctgtatctgc aaatgaacag cctgagagcc 7740

gaggacacgg ccgtatatta ctgtgcgaaa gatctggttc acgcggatat agtggccctg 7800

gggtgtggcc ctgactactg gggccagggc accctggtca ccgtctcctc agaccccatt 7860

gatgtcccat tgatacccca gacggactcc actgaagctc aagcgaggcc ggggcgcccc 7920

tggaggggca gccaagatct ccttccctcc cgaggtgcag ctgttggagt ctgggggagg 7980

cttggtacag cctggggggt ccctgagact ctcctgtgca gcctctggat tcacctttag 8040

caactttgcc ataagctggg tccgccaggc tccagggaag gggctggagt gggtctcagg 8100

tatcagtggt actggtggta ggacatacta cgcagactcc gtgaagggcc ggttcaccat 8160

ctccagagac aattccaaga acacgctgta tctgcaaatg aacagcctga gagccgagga 8220

cacggccgta tattactgtg cgaaaggggt cgatgctaca tatagtgcca cctggtacgt 8280

ggggggctac tatgagtatt ggggccaggg caccctggtc accgtctcct caacaccctt 8340

tacttacatc ggtcagatct aataaagacc acattcagat cccatgcaaa gcgcattgat 8400

gcccctcaga gaccccacag agtcccctca gaatcgatat gcatttaaat gggccccccg 8460

gtgatatccc cgggtctaga gcggccgcta ccgttcgtat aatgtatgct atacgaagtt 8520

atatcgaatt cctgcagaga tctgttccat cccaatggcg cgccgagctt ggcgtaatca 8580

tggtcatagc tgtttcctgt gtgaaattgt tatccgctca caattccaca caacatacga 8640

gccggaagca taaagtgtaa agcctggggt gcctaatgag tgagctaact cacattaatt 8700

gcgttgcgct cactgcccgc tttccagtcg ggaaacctgt cgtgccagct gcattaatga 8760

atcggccaac gcgcggggag aggcggtttg cgtattgggc gctcttccgc ttcctcgctc 8820

actgactcgc tgcgctcggt cgttcggctg cggcgagcgg tatcagctca ctcaaaggcg 8880

gtaatacggt tatccacaga atcaggggat aacgcaggaa agaacatgtg agcaaaaggc 8940

cagcaaaagg ccaggaaccg taaaaaggcc gcgttgctgg cgtttttcca taggctccgc 9000

ccccctgacg agcatcacaa aaatcgacgc tcaagtcaga ggtggcgaaa cccgacagga 9060

ctataaagat accaggcgtt tccccctgga agctccctcg tgcgctctcc tgttccgacc 9120

ctgccgctta ccggatacct gtccgccttt ctcccttcgg gaagcgtggc gctttctcat 9180

agctcacgct gtaggtatct cagttcggtg taggtcgttc gctccaagct gggctgtgtg 9240

cacgaacccc ccgttcagcc cgaccgctgc gccttatccg gtaactatcg tcttgagtcc 9300

aacccggtaa gacacgactt atcgccactg gcagcagcca ctggtaacag gattagcaga 9360

gcgaggtatg taggcggtgc tacagagttc ttgaagtggt ggcctaacta cggctacact 9420

agaagaacag tatttggtat ctgcgctctg ctgaagccag ttaccttcgg aaaaagagtt 9480

ggtagctctt gatccggcaa acaaaccacc gctggtagcg gtggtttttt tgtttgcaag 9540

cagcagatta cgcgcagaaa aaaaggatct caagaagatc ctttgatctt ttctacgggg 9600

tctgacgctc agtggaacga aaactcacgt taagggattt tggtcatgag attatcaaaa 9660

aggatcttca cctagatcct tttaaattaa aaatgaagtt ttaaatcaat ctaaagtata 9720

tatgagtaaa cttggtctga cagttaccaa tgcttaatca gtgaggcacc tatctcagcg 9780

atctgtctat ttcgttcatc catagttgcc tgactccccg tcgtgtagat aactacgata 9840

cgggagggct taccatctgg ccccagtgct gcaatgatac cgcgagaccc acgctcaccg 9900

gctccagatt tatcagcaat aaaccagcca gccggaaggg ccgagcgcag aagtggtcct 9960

gcaactttat ccgcctccat ccagtctatt aattgttgcc gggaagctag agtaagtagt 10020

tcgccagtta atagtttgcg caacgttgtt gccattgcta caggcatcgt ggtgtcacgc 10080

tcgtcgtttg gtatggcttc attcagctcc ggttcccaac gatcaaggcg agttacatga 10140

tcccccatgt tgtgcaaaaa agcggttagc tccttcggtc ctccgatcgt tgtcagaagt 10200

aagttggccg cagtgttatc actcatggtt atggcagcac tgcataattc tcttactgtc 10260

atgccatccg taagatgctt ttctgtgact ggtgagtact caaccaagtc attctgagaa 10320

tagtgtatgc ggcgaccgag ttgctcttgc ccggcgtcaa tacgggataa taccgcgcca 10380

catagcagaa ctttaaaagt gctcatcatt ggaaaacgtt cttcggggcg aaaactctca 10440

aggatcttac cgctgttgag atccagttcg atgtaaccca ctcgtgcacc caactgatct 10500

tcagcatctt ttactttcac cagcgtttct gggtgagcaa aaacaggaag gcaaaatgcc 10560

gcaaaaaagg gaataagggc gacacggaaa tgttgaatac tcatactctt cctttttcaa 10620

tattattgaa gcatttatca gggttattgt ctcatgagcg gatacatatt tgaatgtatt 10680

tagaaaaata aacaaatagg ggttccgcgc acatttcccc gaaaagtgcc acctgacgtc 10740

taagaaacca ttattatcat gacattaacc tataaaaata ggcgtatcac gaggcccttt 10800

cgtc 10804

<210> 164
<211> 10827
<212> DNA
<213> Artificial Sequence

<220>
<223> Synthetic DNA

<400> 164
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgaatt gacgcgtatt gggatggaac 420

catatgtagc ttacgacact gataacttcg tatatggtat tatatacgaa gttataagct 480

tcaccatggc caagcctttg tctcaagaag aatccaccct cattgaaaga gcaacggcta 540

caatcaacag catccccatc tctgaagact acagcgtcgc cagcgcagct ctctctagcg 600

acggccgcat cttcactggt gtcaatgtat atcattttac tgggggacct tgtgcagaac 660

tcgtggtgct gggcactgct gctgctgcgg cagctggcaa cctgacttgt atcgtcgcga 720

tcggaaatga gaacaggggc atcttgagcc cctgcggacg gtgtcgacag gtgcttctcg 780

atctgcatcc tgggatcaaa gcgatagtga aggacagtga tggacagccg acggcagttg 840

ggattcgtga attgctgccc tctggttatg tgtgggaggg ctaaactagt gcacttcgtg 900

gccgaggagc aggactgaca cgtgctacga gatttcgatt ccaccgccgc cttctatgaa 960

aggttgggct tcggaatcgt tttccgggac gccggctgga tgatcctcca gcgcggggat 1020

ctcatgctgg agttcttcgc ccaccccaac ttgtttattg cagcttataa tggttacaaa 1080

taaagcaata gcatcacaaa tttcacaaat aaagcatttt tttcactgca ttctagttgt 1140

ggtttgtcca aactcatcaa tgtatcttat catgtctgta taccgtcgac ctctagctag 1200

agcttggcgt aatcatggtc atagctgttt cctgtgtgcc caccccaact tgtttattgc 1260

agcttataat ggttacaaat aaagcaatag catcacaaat ttcacaaata aagcattttt 1320

ttcactgcat tctagttgtg gtttgtccaa actcatcaat gtatcttatc atgtctgata 1380

acttcgtata ttctatctta tacgaacggt actcgagagc tcatgcggta ccgctagcat 1440

gctggccagg atccatgccc atggccgcgg atgcggcgcg ccatcgattc tgaggggact 1500

ctgtggggtc tctgaggggc atcaatgcgc tttgcatggg atctgaatgt ggtctttatt 1560

agatctgacc gatgtaagta aagggtgttg aggagacggt gaccagggtg ccctggcccc 1620

aatactcata gtagcccccc acgtaccagg tggcactata tgtagcatcg acccctttcg 1680

cacagtaata tacggccgtg tcctcggctc tcaggctgtt catttgcaga tacagcgtgt 1740

tcttggaatt gtctctggag atggtgaacc ggcccttcac ggagtctgcg tagtatgtcc 1800

taccaccagt accactgata cctgagaccc actccagccc cttccctgga gcctggcgga 1860

cccagcttat ggcaaagttg ctaaaggtga atccagaggc tgcacaggag agtctcaggg 1920

accccccagg ctgtaccaag cctcccccag actccaacag ctgcacctcg ggagggaagg 1980

agatcttggc tgcccctcca ggggcgcccc ggcctcgctt gagcttcagt ggagtccgtc 2040

tggggtatca atgggacatc aatggggtct gaggagacgg tgaccagggt gccctggccc 2100

cagtagtcag ggccacaccc cagggccact atatccgcgt gaaccagatc tttcgcacag 2160

taatatacgg ccgtgtcctc ggctctcagg ctgttcattt gcagatacag cgtgttcttg 2220

gaattgtctc tggagatggt gaaccggccc ctcacggagt ctgcgtagta tgtgctgcca 2280

gcactagcac tcatacttga gacccactcc agccccttcc ctggagcctg gcggacccag 2340

cccatggcat atctgctaaa ggtgaatcca gaggctgcac aggagagtct cagggacccc 2400

ccaggctgta ccaagcctcc cccagactcc aacagctgca cctcacagcg gggggacgag 2460

aggacagagg gtctcttcag aactcagccg ttgtgtgctt ccacgcacag ctccacttct 2520

tcgtcccagc acacagctct gcattgagga gacggtgacc agggtgccct ggccccagta 2580

gtcaagcata actccccgaa ccatagtagt tttcgcacag taatatacgg ccgtgtcctc 2640

ggctctcagg ctgttcattt gcagatacag cgtgttcttg gaattgtctc tggagatggt 2700

gaaccggccc ttcacggagt ctgagtagta tgtactacca ccatcaccag tattagttga 2760

gacccactcc agccccttcc ctggagcctg gcggacccag ctcatgaaaa aggtgctaaa 2820

ggtgaatcca gaggctgcac aggagagtct cagggacccc ccaggctgta ccaagcctcc 2880

cccagactcc aacagctgca cctccgtgtc cgtgcccagc acagccccac atcgcacggc 2940

tccatacagc agcagcgtgg agctgagccc caatgtgggc acggagccca tcccctctct 3000

cagctgagga gacggtgacc agggtgccct ggccccagac gtccatatcg tagtagaggt 3060

gggctcgagc ccctttcgca cagtaatata cggccgtgtc ctcggctctc aggctgttca 3120

tttgcagata cagcgtgttc ttggaattgt ctctggagat ggtgaaccgg cccttcacgg 3180

agtctgcgta gtatgtacta atagcactac cactaacaag tgagacccac tccagcccct 3240

tccctggagc ctggcggacc cagctcaggg cataactgct aaaggtgaat ccagaggctg 3300

cacaggagag tctcagggac cccccaggct gtaccaagcc tcccccagac tccaacagct 3360

gcacctctgg cggggcagcc aagaggagca gatgggagct atgagggctg atggtggcga 3420

aatcacgggg ggaaggagat cagggcttgt tctgttctta ggagagctga ggagacggtg 3480

accagggtgc cctggcccca gtagtcaaat gaccagccac tgctaaagcc tttcgcacag 3540

taatatacgg ccgtgtcctc ggctctcagg ctgttcattt gcagatacag cgtgttcttg 3600

gaattgtctc tggagatggt gaaccggccc ttcacggagt ctgcgtaata tgtgttttca 3660

ccactgacag taatagttga gacccactcc agccccttcc ctggagcctg gcggacccag 3720

tccatgccaa agttgctaaa ggtgaatcca gaggctgcac aggagagtct cagggacccc 3780

ccaggctgta ccaagcctcc cccagactcc aacagctgca cctcatcccg atgaggttca 3840

gtcaacacct catagagacc ccactgagaa cacagtgaga ccccgtagat gccctaaaga 3900

ccccggaaag ggccgaagcc cacctgagga gacggtgacc agggtgccct ggccccaagg 3960

attcccccaa ccagagtaat gccaggataa tttcgcacag taatatacgg ccgtgtcctc 4020

ggctctcagg ctgttcattt gcagatacag cgtgttcttg gaattgtctc tggagatggt 4080

gaaccggccc tgcacggagt ctgcgtagaa agcgctacta ccgctaccac gaatagctga 4140

gacccactcc agccccttcc ctggagcctg gcggacccag ctgaggtcaa agttgctaaa 4200

ggtgaatcca gaggctgcac aggagagtct cagggacccc ccaggctgta ccaagcctcc 4260

cccagactcc aacagctgca cctcgacccc atagagttct aatagagacc tcattgcgac 4320

tccgaagaga tctcatatag attgcgttga attcccttcg agacatcact gagatctcat 4380

agagtgagga gacggtgacc agggtgccct ggccccagta gtcaaggctg ctgccaagag 4440

atttcgcaca gtaatatacg gccgtgtcct cggctctcag gctgttcatt tgcagataca 4500

gcgtgttctt ggaattgtct ctggagatgg tgaaccggcc cttcacggag tctgcgtagt 4560

atgtgccagc accactacta gtaacagttg agacccactc cagccccttc cctggagcct 4620

ggcggaccca ggtcatggca aagttgctaa aggtgaatcc agaggctgca caggagagtc 4680

tcagggaccc cccaggctgt accaagcctc ccccagactc caacagctgc acctccctcg 4740

cggagccgga ccacactccg accacaaaga gaccccactg agatgtcata gagagccata 4800

gagatgtgac tgaaaccacg taaagaccat ttagatgagg agacggtgac cagggtgccc 4860

tggccccaat agtgacagta atcaccacta caatatttgg tgagaccccc tttcgcacag 4920

taatatacgg ccgtgtcctc ggctctcagg ctgttcattt gcagatacag cgtgttcttg 4980

gaattgtctc tggagatggt gaaccggccc ttcacggagt ctgcgtagta tatggcacca 5040

ccagtgccac taatagctga gacccactcc agccccttcc ctggagcctg gcggacccag 5100

ctcatggaat agctgctaaa ggtgaatcca gaggctgcac aggagagtct cagggacccc 5160

ccaggctgta ccaagcctcc cccagactcc aacagctgca cctcataagg acaccctgtt 5220

ggtgcggggc agcattccta cacgtgacca tcacagcatg tgcgtcctat gggtggtctt 5280

caggcccggt tcataagaga gacctgagga gacggtgacc agggtgccct ggccccagac 5340

gtccataccg tagtagtagt agtaacctct ccaaaaatcg taagagttgt ctttcgcaca 5400

gtaatatacg gccgtgtcct cggctctcag gctgttcatt tgcagataca gcgtgttctt 5460

ggaattgtct ctggagatgg tgaaccggcc cttcacggag tctgcgtagt atgtgctacc 5520

accacgacca ctaatacctg agacccactc cagccccttc cctggagcct ggcggaccca 5580

gcccatgaca taggtgctaa aggtgaatcc agaggctgca caggagagtc tcagggaccc 5640

cccaggctgt accaagcctc ccccagactc caacagctgc acctctttcg cagccagtgc 5700

agctgcgtgg ggtcagcagg ggatgccacc acccctgcag cgagctgtgg agcgggttgt 5760

gcctcttctg agacgtgcag acccctgagg agacggtgac cagggtgccc tggccccaga 5820

agtcaaagta tttcgcacag taatatacgg ccgtgtcctc ggctctcagg ctgttcattt 5880

gcagatacag cgtgttcttg gaattgtctc tggagatggt gaaccggccc ttcacggagt 5940

ctgagtagta tgtgctacca ccataattac taatagctga gacccactcc agccccttcc 6000

ctggagcctg gcggacccag gtcatggcat agttgctaaa ggtgaatcca gaggctgcac 6060

aggagagtct cagggacccc ccaggctgta ccaagcctcc cccagactcc aacagctgca 6120

cctcgccgtg ccagcagatg gagcagctgc ggccgatgct ctctttggcc cctttcagtc 6180

gatgttttat tttttctccc cctttccatc ctattttcgt gctctgagga gacggtgacc 6240

agggtgccct ggccccagac gtccataccg cagtcacgcc ccgaaccatc gcatttcgca 6300

cagtaatata cggccgtgtc ctcggctctc aggctgttca tttgcagata cagcgtgttc 6360

ttggaattgt ctctggagat ggtgaaccgg cccttcacgg agtctgaata ggttgggcct 6420

tcaccactgt cactaatact tgagacccac tccagcccct tccctggagc ctggcggacc 6480

cacctcataa ggtacttgct aaaggtgaat ccagaggctg cacaggagag tctcagggac 6540

cccccaggct gtaccaagcc tcccccagac tccaacagct gcacctcgcg accacagccc 6600

agctgcagaa cacctccagc tgtccccaca tcagagctat ttcggggcag gaaaccacga 6660

gtcctttcgg tcgtgtccga accccgctga ggagacggtg accagggtgc cctggcccca 6720

gacgtccaga cccccagcca aaacatcttc tccagccaaa acatcttcta cgtggagatc 6780

tttcgcacag taatatacgg ccgtgtcctc ggctctcagg ctgttcattt gcagatacag 6840

cgtgttcttg gaattgtctc tggagatggt gaaccggccc ttcacggagt ctgcgtagta 6900

tgtgtcaccc ccactaccag taatagctga gacccactcc agccccttcc ctggagcctg 6960

gcggacccac gtcatgacaa aggtgctaaa ggtgaatcca gaggctgcac aggagagtct 7020

cagggacccc ccaggctgta ccaagcctcc cccagactcc aacagctgca cctcctctgg 7080

gggtctctgg gggttttatg aggcccagct ccacgaggtt gtgtggcggc ggctccgtca 7140

gcgctctctg tccttcccca cagggctgat ggcgtgagga gacggtgacc agggtgccct 7200

ggccccagac gtccataccg taatggtagt actggccact gctatacacc ccatctttcg 7260

cacagtaata tacggccgtg tcctcggctc tcaggctgtt catttgcaga tacagcgtgt 7320

tcttggaatt gtctctggag atggtgaacc ggcccttcac ggagtctgcg taatatgttc 7380

taataccact accactaata cttgagaccc actccagccc cttccctgga gcctggcgga 7440

cccagctcat gtcaaagctg ctaaaggtga atccagaggc tgcacaggag agtctcaggg 7500

accccccagg ctgtaccaag cctcccccag actccaacag ctgcacctcg ggtccctaca 7560

gggcctttat ggggtttcag tggtgtctct atggctctct atgacatctc aggctatggc 7620

agtggtgctg atgacggtga cagagcccat gaggagacgg tgaccagggt gccctggccc 7680

caggcgtcca taccgtacca gtggagatct ttcgcacagt aatatacggc cgtgtcctcg 7740

gctctcaggc tgttcatttg cagatacagc gtgttcttgg aattgtctct ggagatggtg 7800

aaccggccct tcacggagtc tgcgtagtgt gtcctaccat cactggcact aatagttgag 7860

acccactcca gccccttccc tggagcctgg cggacccagt tcatgaaaaa gctgctaaag 7920

gtgaatccag aggctgcaca ggagagtctc agggaccccc caggctgtac caagcctccc 7980

ccagactcca acagctgcac ctcctccagg caggctctga cctctctggg tctctgtagg 8040

gtctctatgt ggtctcatgg ggtcatcagc ggggtctcaa tgaggtttct atcaaatctc 8100

atctgaggag acggtgacca gggtgccctg gccccagtaa gtaggggttt tcgcacagta 8160

atatacggcc gtgtcctcgg ctctcaggct gttcatttgc agatacagcg tgttcttgga 8220

attgtctctg gagatggtga accggccctt cacggagtct gcgtagtatg tgttatcacc 8280

actagcacta atagctgaga cccactccag ccccttccct ggagcctggc ggacccagct 8340

catgacatag atgctaaagg tgaatccaga ggctgcacag gagagtctca gggacccccc 8400

aggctgtacc aagcctcccc cagactccaa cagctgcacc tcggcgcgcc ccgcggatgt 8460

cgacgattta aatgggcccc ccggtgatat ccccgggtct agagcggccg ctaccgttcg 8520

tataatgtat gctatacgaa gttatatcga attcctgcag agatctgttc catcccaatg 8580

gcgcgccgag cttggcgtaa tcatggtcat agctgtttcc tgtgtgaaat tgttatccgc 8640

tcacaattcc acacaacata cgagccggaa gcataaagtg taaagcctgg ggtgcctaat 8700

gagtgagcta actcacatta attgcgttgc gctcactgcc cgctttccag tcgggaaacc 8760

tgtcgtgcca gctgcattaa tgaatcggcc aacgcgcggg gagaggcggt ttgcgtattg 8820

ggcgctcttc cgcttcctcg ctcactgact cgctgcgctc ggtcgttcgg ctgcggcgag 8880

cggtatcagc tcactcaaag gcggtaatac ggttatccac agaatcaggg gataacgcag 8940

gaaagaacat gtgagcaaaa ggccagcaaa aggccaggaa ccgtaaaaag gccgcgttgc 9000

tggcgttttt ccataggctc cgcccccctg acgagcatca caaaaatcga cgctcaagtc 9060

agaggtggcg aaacccgaca ggactataaa gataccaggc gtttccccct ggaagctccc 9120

tcgtgcgctc tcctgttccg accctgccgc ttaccggata cctgtccgcc tttctccctt 9180

cgggaagcgt ggcgctttct catagctcac gctgtaggta tctcagttcg gtgtaggtcg 9240

ttcgctccaa gctgggctgt gtgcacgaac cccccgttca gcccgaccgc tgcgccttat 9300

ccggtaacta tcgtcttgag tccaacccgg taagacacga cttatcgcca ctggcagcag 9360

ccactggtaa caggattagc agagcgaggt atgtaggcgg tgctacagag ttcttgaagt 9420

ggtggcctaa ctacggctac actagaagaa cagtatttgg tatctgcgct ctgctgaagc 9480

cagttacctt cggaaaaaga gttggtagct cttgatccgg caaacaaacc accgctggta 9540

gcggtggttt ttttgtttgc aagcagcaga ttacgcgcag aaaaaaagga tctcaagaag 9600

atcctttgat cttttctacg gggtctgacg ctcagtggaa cgaaaactca cgttaaggga 9660

ttttggtcat gagattatca aaaaggatct tcacctagat ccttttaaat taaaaatgaa 9720

gttttaaatc aatctaaagt atatatgagt aaacttggtc tgacagttac caatgcttaa 9780

tcagtgaggc acctatctca gcgatctgtc tatttcgttc atccatagtt gcctgactcc 9840

ccgtcgtgta gataactacg atacgggagg gcttaccatc tggccccagt gctgcaatga 9900

taccgcgaga cccacgctca ccggctccag atttatcagc aataaaccag ccagccggaa 9960

gggccgagcg cagaagtggt cctgcaactt tatccgcctc catccagtct attaattgtt 10020

gccgggaagc tagagtaagt agttcgccag ttaatagttt gcgcaacgtt gttgccattg 10080

ctacaggcat cgtggtgtca cgctcgtcgt ttggtatggc ttcattcagc tccggttccc 10140

aacgatcaag gcgagttaca tgatccccca tgttgtgcaa aaaagcggtt agctccttcg 10200

gtcctccgat cgttgtcaga agtaagttgg ccgcagtgtt atcactcatg gttatggcag 10260

cactgcataa ttctcttact gtcatgccat ccgtaagatg cttttctgtg actggtgagt 10320

actcaaccaa gtcattctga gaatagtgta tgcggcgacc gagttgctct tgcccggcgt 10380

caatacggga taataccgcg ccacatagca gaactttaaa agtgctcatc attggaaaac 10440

gttcttcggg gcgaaaactc tcaaggatct taccgctgtt gagatccagt tcgatgtaac 10500

ccactcgtgc acccaactga tcttcagcat cttttacttt caccagcgtt tctgggtgag 10560

caaaaacagg aaggcaaaat gccgcaaaaa agggaataag ggcgacacgg aaatgttgaa 10620

tactcatact cttccttttt caatattatt gaagcattta tcagggttat tgtctcatga 10680

gcggatacat atttgaatgt atttagaaaa ataaacaaat aggggttccg cgcacatttc 10740

cccgaaaagt gccacctgac gtctaagaaa ccattattat catgacatta acctataaaa 10800

ataggcgtat cacgaggccc tttcgtc 10827

<210> 165
<211> 375
<212> DNA
<213> Artificial Sequence

<220>
<223> Synthetic DNA

<400> 165
gaggtgcagc tgttggagtc tgggggaggc ttggtacagc ctggggggtc cctgagactc 60

tcctgtgcag cctctggatt cacctttagc ggctttgcca tgaactgggt ccgccaggct 120

ccagggaagg ggctggagtg ggtctcaact attagtaata gtgctggtag cacatattac 180

gcagactccg tgaagggccg gttcaccatc tccagagaca attccaagaa cacgctgtat 240

ctgcaaatga acagcctgag agccgaggac acggccgtat attactgtgc gaaatatggg 300

gaggatattg tcgttgtgcc agctcgctac ggtgtggacg tctggggcca gggcaccctg 360

gtcaccgtct cctca 375

<210> 166
<211> 363
<212> DNA
<213> Artificial Sequence

<220>
<223> Synthetic DNA

<400> 166
gaggtgcagc tgttggagtc tgggggaggc ttggtacagc ctggggggtc cctgagactc 60

tcctgtgcag cctctggatt cacctttagc agttttgccc tgaattgggt ccgccaggct 120

ccagggaagg ggctggagtg ggtctcaact attactggta gtgctagtaa cacatactac 180

gcagactccg tgaagggccg gttcaccatc tccagagaca attccaagaa cacgctgtat 240

ctgcaaatga acagcctgag agccgaggac acggccgtat attactgtgc gaaaggggca 300

catagtcgca gcctaacttg gttcggcccc tggggccagg gcaccctggt caccgtctcc 360

tca 363

<210> 167
<211> 372
<212> DNA
<213> Artificial Sequence

<220>
<223> Synthetic DNA

<400> 167
gaggtgcagc tgttggagtc tgggggaggc ttggtacagc ctggggggtc cctgagactc 60

tcctgtgcag cctctggatt cacctttagc aactatgcca tgagctgggt ccgccaggct 120

ccagggaagg ggctggagtg ggtctcaact tttagtaaga ctggtgatag aacattttat 180

gcagactccg tgaagggccg gttcaccatc tccagagaca attccaagaa cacgctgtat 240

ctgcaaatga acagcctgag agccgaggac acggccgtat attactgtgc gaaaaatggg 300

ccctttggtt gtggtggtga ctgcgcagac tttgactact ggggccaggg caccctggtc 360

accgtctcct ca 372

<210> 168
<211> 369
<212> DNA
<213> Artificial Sequence

<220>
<223> Synthetic DNA

<400> 168
gaggtgcagc tgttggagtc tgggggaggc ttggtacagc ctggggggtc cctgagactc 60

tcctgtgcag cctctggatt cacctttagc aactttccca tgagctgggt ccgccaggct 120

ccagggaagg ggctggagtg ggtctcagtt attagtggta gtggtggaag cacactctac 180

acagactccg tgaagggccg gttcaccatc tccagagaca attccaagaa cacgctgtat 240

ctgcaaatga acagcctgag agccgaggac acggccgtat attactgtgc gaaagatcag 300

gacgattttt ggagtgtttc aaattggttc gacccctggg gccagggcac cctggtcacc 360

gtctcctca 369

<210> 169
<211> 357
<212> DNA
<213> Artificial Sequence

<220>
<223> Synthetic DNA

<400> 169

gaggtgcagc tgttggagtc tgggggaggc ttggtacagc ctggggggtc cctgagactc 60

tcctgtgcag cctctggatt cacctttagc agctttgcca tgacctgggt ccgccaggct 120

ccagggaagg ggctggagtg ggtctcagct attagtggta gtgttggtag cacatactac 180

gcagactccg tgcagggccg gttcaccatc tccagagaca attccaagaa cacgctgtat 240

ctgcaaatga acagcctgag agccgaggac acggccgtat attactgtgc gaaagacggt 300

ggggcctact acggaacggt caactggggc cagggcaccc tggtcaccgt ctcctca 357

<210> 170
<211> 378
<212> DNA
<213> Artificial Sequence

<220>
<223> Synthetic DNA

<400> 170
gaggtgcagc tgttggagtc tgggggaggc ttggtacagc ctggggggtc cctgagactc 60

tcctgtgcag cctctggatt cacctttagc atctttgccg tgagctgggt ccgccaggct 120

ccagggaagg ggctggagtg ggtctcagct attagtggtc atggtcttag cacatactac 180

ggagactccg tgaagggccg gttcaccatc tccagagaca attccaagaa cacgctgtat 240

ctgcaaatga acagcctgag agccgaggac acggccgtat attactgtgc gaaagccgga 300

agtgctggta gcagctaccc gttctactac tacggtatgg acgtctgggg ccagggcacc 360

ctggtcaccg tctcctca 378

<210> 171
<211> 387
<212> DNA
<213> Artificial Sequence

<220>
<223> Synthetic DNA

<400> 171
gaggtgcagc tgttggagtc tgggggaggc ttggtacagc ctggggggtc cctgagactc 60

tcctgtgcag cctctggatt cacctttagc agctatgcca tgatctgggt ccgccaggct 120

ccagggaagg ggctggagtg ggtctcaggt attactgtta gtggcggtag cacagactac 180

gcagactccg tgaagggccg gttcaccatc tccagagaca attccaagaa cacgctgtat 240

ctgcaaatga acagcctgag agccgaggac acggccgtat attactgtgc gaaagggggg 300

ccccaactta tagcagcagc tgggacttat tatcactact acggtatgga cgtctggggc 360

cagggcaccc tggtcaccgt ctcctca 387

<210> 172
<211> 360

<212>  DNA
<213>  Artificial Sequence

<220>
<223>  Synthetic DNA

<400>  172
gaggtgcagc tgttggagtc tgggggaggc ttggtacagc ctggggggtc cctgagactc      60

tcctgtgcag cctctggatt cacctttagc agctatacca tgaactgggt ccgccaggct     120

ccagggaagg ggctggagtg ggtctcatac attagtagtg gtagtggtag catatactac     180

gcagactctg tgaagggccg gttcaccatc tccagagaca attccaagaa cacgctgtat     240

ctgcaaatga acagcctgag agccgaggac acggccgtat attactgtgc gaaacgaggt     300

gatagtagtg catacgccct tgactactgg ggccagggca ccctggtcac cgtctcctca     360


<210>  173
<211>  372
<212>  DNA
<213>  Artificial Sequence

<220>
<223>  Synthetic DNA

<400>  173
gaggtgcagc tgttggagtc tgggggaggc ttggtacagc ctggggggtc cctgagactc      60

tcctgtgcag cctctggatt cacctttagc agccatagca tgaactgggt ccgccaggct     120

ccagggaagg ggctggagtg ggtctcatac attagaagta gtagtagtac catatactac     180

gcagactctg tgaagggtcg gttcaccatc tccagagaca attccaagaa cacgctgtat     240

ctgcaaatga acagcctgag agccgaggac acggccgtat attactgtgc gaaagtcccc     300

aattactatg gttcatttcc tcgagatggt atggacgtct ggggccaggg caccctggtc     360

accgtctcct ca                                                         372


<210>  174
<211>  369
<212>  DNA
<213>  Artificial Sequence

<220>
<223>  Synthetic DNA

<400>  174
gaggtgcagc tgttggagtc tgggggaggc ttggtacagc ctggggggtc cctgagactc      60

tcctgtgcag cctctggatt cacctttagc agctataaca tgaactgggt ccgccaggct     120

ccagggaagg ggctggagtg ggtctcatac gttagtagtg attctagtac catatactac     180

gcagactttg tggagggccg gttcaccatc tccagagaca attccaagaa cacgctgtat     240

ctgcaaatga acagcctgag agccgaggac acggccgtat attactgtgc gaaaggcctg     300

gcgttctgcg ccagctgcta tggatacttc cagctctggg gccagggcac cctggtcacc 360

gtctcctca 369

<210> 175
<211> 357
<212> DNA
<213> Artificial Sequence

<220>
<223> Synthetic DNA

<400> 175
gaggtgcagc tgttggagtc tgggggaggc ttggtacagc ctggggggtc cctgagactc 60

tcctgtgcag cctctggatt cacctttagc agcaactcca tgagctgggt ccgccaggct 120

ccagggaagg ggctggagtg ggtctcactt atttatagca gtggtggcac atactacgca 180

gactccgtga agggccggtt caccatctcc agagacaatt ccaagaacac gctgtatctg 240

caaatgaaca gcctgagagc cgaggacacg gccgtatatt actgtgcgaa agcgtgggtg 300

tatgatagta gtgtttttga ctattggggc cagggcaccc tggtcaccgt ctcctca 357

<210> 176
<211> 360
<212> DNA
<213> Artificial Sequence

<220>
<223> Synthetic DNA

<400> 176
gaggtgcagc tgttggagtc tgggggaggc ttggtacagc ctggggggtc cctgagactc 60

tcctgtgcag cctctggatt cacctttagc tatgccataa gctgggtccg ccaggctcca 120

gggaaggggc tggagtgggt ctcaggtatt agtggcggcg gtggtagcac attctacgct 180

gactccgtgc agggccggtt caccatctcc agagacaatt ccaagaacac gctgtatctg 240

caaatgaaca gcctgagagc cgaggacacg gccgtatatt actgtgcgaa acccgagaga 300

gggatagtgg gggctgcatt tgactcctgg ggccagggca ccctggtcac cgtctcctca 360

<210> 177
<211> 354
<212> DNA
<213> Artificial Sequence

<220>
<223> Synthetic DNA

<400> 177
gaggtgcagc tgttggagtc tgggggaggc ttggtacagc ctggggggtc cctgagactc 60

tcctgtgcag cctctggatt cacctttagc agccattgga tgaggtgggt ccgccaggct 120

ccagggaagg ggctggagtg ggtctcaaac ataaagcaag gtggaagtga agaatattat 180

gtggactctg tgaagggccg gttcaccatc tccagagaca attccaagaa cacgctgtat 240

ctgcaaatga acagcctgag agccgaggac acggccgtat attactgtgc gaaaggaaat 300

tataaggcag gttgtgacta ctggggccag ggcaccctgg tcaccgtctc ctca 354

<210> 178
<211> 348
<212> DNA
<213> Artificial Sequence

<220>
<223> Synthetic DNA

<400> 178
gaggtgcagc tgttggagtc tgggggaggc ttggtacagc ctggggggtc cctgagactc 60

tcctgtgcag cctctggatt cacctttagc acctatagca tgaactgggt ccgccaggct 120

ccagggaagg ggctggagtg ggtctcatcc attactagta gtggtagtta catatactgc 180

gcagactcag tgaagggccg gttcaccatc tccagagaca attccaagaa cacgctgtat 240

ctgcaaatga acagcctgag agccgaggac acggccgtat attactgtgc gaaagatggc 300

tccggtatgg acgtctgggg ccagggcacc ctggtcaccg tctcctca 348

<210> 179
<211> 357
<212> DNA
<213> Artificial Sequence

<220>
<223> Synthetic DNA

<400> 179
gaggtgcagc tgttggagtc tgggggaggc ttggtacagc ctggggggtc cctgagactc 60

tcctgtgcag cctctggatt cacctttagc tcctatagca tgacctgggt ccgccaggct 120

ccagggaagg ggctggagtg ggtctcaaac attagcagta gtagtaatac cgcattctac 180

gcagactccg tgaagggccg gttcaccatc tccagagaca attccaagaa cacgctgtat 240

ctgcaaatga acagcctgag agccgaggac acggccgtat attactgtgc gaaagatcga 300

aatggctaca atttctttga ctactggggc cagggcaccc tggtcaccgt ctcctca 357

<210> 180
<211> 7155
<212> DNA
<213> Artificial Sequence

<220>
<223> Synthetic DNA

<400> 180
   ggtaccgagg tgcagctgtt ggagtctggg ggaggcttgg tacagcctgg ggggtccctg 60
   agactctcct gtgcagcctc tggattcacc tttagcagtt ttgccctgaa ttgggtccgc 120
   caggctccag ggaaggggct ggagtgggtc tcaactatta ctggtagtgc tagtaacaca 180
   tactacgcag actccgtgaa gggccggttc accatctcca gagacaattc caagaacacg 240
   ctgtatctgc aaatgaacag cctgagagcc gaggacacgg ccgtatatta ctgtgcgaaa 300
   ggggcacata gtcgcagcct aacttggttc ggcccctggg gccagggcac cctggtcacc 360
   gtctcctcag tgcctggtga ctcttccgca atcagtcctg ggacagaaac ctcagggaca 420
   gcctttatga gttttgatca catctaacag agaggatggg ccagtgagct agagtgatag 480
   agcgtgcgtg tgcggaggtg cagctgttgg agtctggggg aggcttggta cagcctgggg 540
   ggtccctgag actctcctgt gcagcctctg gattcacctt tagcaactat gccatgagct 600
   gggtccgcca ggctccaggg aaggggctgg agtgggtctc aacttttagt aagactggtg 660
   atagaacatt ttatgcagac tccgtgaagg gccggttcac catctccaga gacaattcca 720
   agaacacgct gtatctgcaa atgaacagcc tgagagccga ggacacggcc gtatattact 780
   gtgcgaaaaa tgggcccttt ggttgtggtg gtgactgcgc agactttgac tactggggcc 840
   agggcaccct ggtcaccgtc tcctcagtgc ctggtgactc ttccgcaatc agtcctggga 900
   cagaaacctc agggacagcc tttatgagtt ttgatcacat ctaacagaga ggatgggcca 960
   ggaggtgcag ctgttggagt ctgggggagg cttggtacag cctggggggt ccctgagact 1020
   ctcctgtgca gcctctggat tcacctttag caactttccc atgagctggg tccgccaggc 1080
   tccagggaag gggctggagt gggtctcagt tattagtggt agtggtggaa gcacactcta 1140
   cacagactcc gtgaagggcc ggttcaccat ctccagagac aattccaaga acacgctgta 1200
   tctgcaaatg aacagcctga gagccgagga cacggccgta tattactgtg cgaaagatca 1260
   ggacgatttt tggagtgttt caaattggtt cgacccctgg ggccagggca ccctggtcac 1320
   cgtctcctca gtgcctggtg actcttccgc aatcagtcct gggacagaaa cctcagggac 1380
   agcctttatg agttttgatc acatctaaca gagaggatgg gccagtatag acatctatga 1440
   tacgctcact catacgaggt gcagctgttg gagtctgggg gaggcttggt acagcctggg 1500
   gggtccctga gactctcctg tgcagcctct ggattcacct ttagcagctt tgccatgacc 1560
   tgggtccgcc aggctccagg gaaggggctg gagtgggtct cagctattag tggtagtgtt 1620
   ggtagcacat actacgcaga ctccgtgcag ggccggttca ccatctccag agacaattcc 1680
   aagaacacgc tgtatctgca aatgaacagc ctgagagccg aggacacggc cgtatattac 1740
   tgtgcgaaag acggtggggc ctactacgga acggtcaact ggggccaggg caccctggtc 1800
   accgtctcct cagtgcctgg tgactcttcc gcaatcagtc ctgggacaga aacctcaggg 1860
   acagccttta tgagttttga tcacatctaa cagagaggat gggccaggag gtgcagctgt 1920
   tggagtctgg gggaggcttg gtacagcctg gggggtccct gagactctcc tgtgcagcct 1980
   ctggattcac ctttagcatc tttgccgtga gctgggtccg ccaggctcca gggaaggggc 2040
   tggagtgggt ctcagctatt agtggtcatg gtcttagcac atactacgga gactccgtga 2100
   agggccggtt caccatctcc agagacaatt ccaagaacac gctgtatctg caaatgaaca 2160
   gcctgagagc cgaggacacg gccgtatatt actgtgcgaa agccggaagt gctggtagca 2220
   gctacccgtt ctactactac ggtatggacg tctggggcca gggcaccctg gtcaccgtct 2280
   cctcagtgcc tggtgactct tccgcaatca gtcctgggac agaaacctca gggacagcct 2340
   ttatgagttt tgatcacatc taacagagag gatgggccag tcatcgagtc tcgagctctc 2400
   tcgcagacac gaggtgcagc tgttggagtc tgggggaggc ttggtacagc ctggggggtc 2460
   cctgagactc tcctgtgcag cctctggatt cacctttagc agctatgcca tgatctgggt 2520
   ccgccaggct ccagggaagg ggctggagtg ggtctcaggt attactgtta gtggcggtag 2580
   cacagactac gcagactccg tgaagggccg gttcaccatc tccagagaca attccaagaa 2640
   cacgctgtat ctgcaaatga acagcctgag agccgaggac acggccgtat attactgtgc 2700
   gaaagggggg ccccaactta tagcagcagc tgggacttat tatcactact acggtatgga 2760
   cgtctggggc cagggcaccc tggtcaccgt ctcctcagtg cctggtgact cttccgcaat 2820
   cagtcctggg acagaaacct cagggacagc ctttatgagt tttgatcaca tctaacagag 2880
   aggatgggcc aggaggtgca gctgttggag tctgggggag gcttggtaca gcctgggggg 2940
   tccctgagac tctcctgtgc agcctctgga ttcaccttta gcagctatac catgaactgg 3000
   gtccgccagg ctccagggaa ggggctggag tgggtctcat acattagtag tggtagtggt 3060
   agcatatact acgcagactc tgtgaagggc cggttcacca tctccagaga caattccaag 3120
   aacacgctgt atctgcaaat gaacagcctg agagccgagg acacggccgt atattactgt 3180
   gcgaaacgag gtgatagtag tgcatacgcc cttgactact ggggccaggg caccctggtc 3240
   accgtctcct cagtgcctgg tgactcttcc gcaatcagtc ctgggacaga aacctcaggg 3300
   acagccttta tgagttttga tcacatctaa cagagaggat gggccagtct gtctcgctga 3360
   gtgacgctga tgtgtacgag gtgcagctgt tggagtctgg gggaggcttg gtacagcctg 3420
   gggggtccct gagactctcc tgtgcagcct ctggattcac ctttagcagc catagcatga 3480
   actgggtccg ccaggctcca gggaaggggc tggagtgggt ctcatacatt agaagtagta 3540
   gtagtaccat atactacgca gactctgtga agggtcggtt caccatctcc agagacaatt 3600
   ccaagaacac gctgtatctg caaatgaaca gcctgagagc cgaggacacg gccgtatatt 3660
   actgtgcgaa agtccccaat tactatggtt catttcctcg agatggtatg gacgtctggg 3720
   gccagggcac cctggtcacc gtctcctcag tgcctggtga ctcttccgca atcagtcctg 3780
   ggacagaaac ctcagggaca gcctttatga gttttgatca catctaacag agaggatggg 3840
   ccagtgctat agactgctcg ctacacgtgc agcggaggtg cagctgttgg agtctggggg 3900
   aggcttggta cagcctgggg ggtccctgag actctcctgt gcagcctctg gattcacctt 3960
   tagcagctat aacatgaact gggtccgcca ggctccaggg aaggggctgg agtgggtctc 4020
   atacgttagt agtgattcta gtaccatata ctacgcagac tttgtggagg gccggttcac 4080
   catctccaga gacaattcca agaacacgct gtatctgcaa atgaacagcc tgagagccga 4140
   ggacacggcc gtatattact gtgcgaaagg cctggcgttc tgcgccagct gctatggata 4200
   cttccagctc tggggccagg gcaccctggt caccgtctcc tcagtgcctg gtgactcttc 4260
   cgcaatcagt cctgggacag aaacctcagg gacagccttt atgagttttg atcacatcta 4320
   acagagagga tgggccagga ggtgcagctg ttggagtctg ggggaggctt ggtacagcct 4380
   ggggggtccc tgagactctc ctgtgcagcc tctggattca cctttagcag caactccatg 4440
   agctgggtcc gccaggctcc agggaagggg ctggagtggg tctcacttat ttatagcagt 4500
   ggtggcacat actacgcaga ctccgtgaag ggccggttca ccatctccag agacaattcc 4560
   aagaacacgc tgtatctgca aatgaacagc ctgagagccg aggacacggc cgtatattac 4620
   tgtgcgaaag cgtgggtgta tgatagtagt gtttttgact attggggcca gggcaccctg 4680
   gtcaccgtct cctcagtgcc tggtgactct tccgcaatca gtcctgggac agaaacctca 4740
   gggacagcct ttatgagttt tgatcacatc taacagagag gatgggccag actcacagag 4800
   agactcagcg agagagtgtg gaggtgcagc tgttggagtc tgggggaggc ttggtacagc 4860
   ctggggggtc cctgagactc tcctgtgcag cctctggatt cacctttagc tatgccataa 4920
   gctgggtccg ccaggctcca gggaaggggc tggagtgggt ctcaggtatt agtggcggcg 4980
   gtggtagcac attctacgct gactccgtgc agggccggtt caccatctcc agagacaatt 5040
   ccaagaacac gctgtatctg caaatgaaca gcctgagagc cgaggacacg gccgtatatt 5100
   actgtgcgaa acccgagaga gggatagtgg ggctgcattt tgactcctgg ggccagggca 5160
   ccctggtcac cgtctcctca gtgcctggtg actcttccgc aatcagtcct gggacagaaa 5220
   cctcagggac agcctttatg agttttgatc acatctaaca gagaggatgg gccaggaggt 5280
   gcagctgttg gagtctgggg gaggcttggt acagcctggg gggtccctga gactctcctg 5340
   tgcagcctct ggattcacct ttagcagcca ttggatgagg tgggtccgcc aggctccagg 5400
   gaaggggctg gagtgggtct caaacataaa gcaaggtgga agtgaagaat attatgtgga 5460
   ctctgtgaag ggccggttca ccatctccag agacaattcc aagaacacgc tgtatctgca 5520
   aatgaacagc ctgagagccg aggacacggc cgtatattac tgtgcgaaag gaaattataa 5580
   ggcaggttgt gactactggg gccagggcac cctggtcacc gtctcctcag tgcctggtga 5640
   ctcttccgca atcagtcctg ggacagaaac ctcagggaca gcctttatga gttttgatca 5700
   catctaacag agaggatggg ccagagctat cgcgagtctg actgagtgag ctcggaggtg 5760
   cagctgttgg agtctggggg aggcttggta cagcctgggg ggtccctgag actctcctgt 5820
   gcagcctctg gattcacctt tagcacctat agcatgaact gggtccgcca ggctccaggg 5880
   aaggggctgg agtgggtctc atccattact agtagtggta gttacatata ctgcgcagac 5940
   tcagtgaagg gccggttcac catctccaga gacaattcca agaacacgct gtatctgcaa 6000
   atgaacagcc tgagagccga ggacacggcc gtatattact gtgcgaaaga tggctccggt 6060
   atggacgtct ggggccaggg caccctggtc accgtctcct cagtgcctgg tgactcttcc 6120
   gcaatcagtc ctgggacaga aacctcaggg acagccttta tgagttttga tcacatctaa 6180
   cagagaggat gggccaggag gtgcagctgt tggagtctgg gggaggcttg gtacagcctg 6240
   gggggtccct gagactctcc tgtgcagcct ctggattcac ctttagctcc tatagcatga 6300
   cctgggtccg ccaggctcca gggaaggggc tggagtgggt ctcaaacatt agcagtagta 6360
   gtaataccgc attctacgca gactccgtga agggccggtt caccatctcc agagacaatt 6420
   ccaagaacac gctgtatctg caaatgaaca gcctgagagc cgaggacacg gccgtatatt 6480
   actgtgcgaa agatcgaaat ggctacaatt tctttgacta ctggggccag ggcaccctgg 6540
   tcaccgtctc ctcagtgcct ggtgactctt ccgcaatcag tcctgggaca gaaacctcag 6600
   ggacagcctt tatgagtttt gatcacatct aacagagagg atgggccaga tagctctcga 6660
   tcacgtgcga tcgtagcagg aggtgcagct gttggagtct gggggaggct tggtacagcc 6720
   tggggggtcc ctgagactct cctgtgcagc ctctggattc acctttagcg gctttgccat 6780
   gaactgggtc cgccaggctc cagggaaggg gctggagtgg gtctcaacta ttagtaatag 6840
   tgctggtagc acatattacg cagactccgt gaagggccgg ttcaccatct ccagagacaa 6900
   ttccaagaac acgctgtatc tgcaaatgaa cagcctgaga gccgaggaca cggccgtata 6960
   ttactgtgcg aaatatgggg aggatattgt cgttgtgcca gctcgctacg gtgtggacgt 7020
   ctggggccag ggcaccctgg tcaccgtctc ctcagtgcct ggtgactctt ccgcaatcag 7080
   tcctgggaca gaaacctcag ggacagcctt tatgagtttt gatcacatct aacagagagg 7140
   atgggccagg ctagc 7155

<210> 181
<211> 11374
<212> DNA
<213> Artificial Sequence

<220>
<223> Synthetic DNA

<400> 181
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180
accatatgcg gtgtgaaata ccgcacagat gcgtaaggag aaaataccgc atcaggcgcc 240
attcgccatt caggctgcgc aactgttggg aagggcgatc ggtgcgggcc tcttcgctat 300
tacgccagct ggcgaaaggg ggatgtgctg caaggcgatt aagttgggta acgccagggt 360
tttcccagtc acgacgttgt aaaacgacgg ccagtgaatt catatgtagc ttacgacact 420
gataacttcg tatatggtat tatatacgaa gttataagct tcaccatgat tgaacaagat 480
ggattgcacg caggttctcc ggccgcttgg gtggagaggc tattcggcta tgactgggca 540
caacagacaa tcggctgctc tgatgccgcc gtgttccggc tgtcagcgca ggggcgcccg 600
gttctttttg tcaagaccga cctgtccggt gccctgaatg aactgcagga cgaggcagcg 660
cggctatcgt ggctggccac gacgggcgtt ccttgcgcag ctgtgctcga cgttgtcact 720
gaagcgggaa gggactggct gctattgggc gaagtgccgg ggcaggatct cctgtcatct 780
caccttgctc ctgccgagaa agtatccatc atggctgatg caatgcggcg gctgcatacg 840
cttgatccgg ctacctgccc attcgaccac caagcgaaac atcgcatcga gcgagcacgt 900
actcggatgg aagccggtct tgtcgatcag gatgatctgg acgaagagca tcaggggctc 960
gcgccagccg aactgttcgc caggctcaag gcgcgcatgc ccgacggcga ggatctcgtc 1020
gtgacccatg gcgatgcctg cttgccgaat atcatggtgg aaaatggccg cttttctgga 1080
ttcatcgact gtggccggct gggtgtggcg gaccgctatc aggacatagc gttggctacc 1140
cgtgatattg ctgaagagct tggcggcgaa tgggctgacc gcttcctcgt gctttacggt 1200
atcgccgctc ccgattcgca gcgcatcgcc ttctatcgcc ttcttgacga gttcttctga 1260
actagtgcgg gactctgggg ttcgaaatga ccgaccaagc gacgcccaac ctgccatcac 1320
gagatttcga ttccaccgcc gccttctatg aaaggttggg cttcggaatc gttttccggg 1380
acgccggctg gatgatcctc cagcgcgggg atctcatgct ggagttcttc gcccacccca 1440
acttgtttat tgcagcttat aatggttaca aataaagcaa tagcatcaca aatttcacaa 1500
ataaagcatt tttttcactg cattctagtt gtggtttgtc caaactcatc aatgtatctt 1560
atcatgtctg ttataccgtc gatctctagc tagagcttgg cgtaatcatg gtcatagctg 1620
tttcctgtgt gcccacccca acttgtttat tgcagcttat aatggttaca aataaagcaa 1680
tagcatcaca aatttcacaa ataaagcatt tttttcactg cattctagtt gtggtttgtc 1740
caaactcatc aatgtatctt atcatgtctg ataacttcgt ataatgtatg ctatacgaac 1800
ggtactcgag agctcatgcg gtaccgaggt gcagctgttg gagtctgggg gaggcttggt 1860
acagcctggg gggtccctga gactctcctg tgcagcctct ggattcacct ttagcagttt 1920
tgccctgaat tgggtccgcc aggctccagg gaaggggctg gagtgggtct caactattac 1980
tggtagtgct agtaacacat actacgcaga ctccgtgaag ggccggttca ccatctccag 2040

agacaattcc aagaacacgc tgtatctgca aatgaacagc ctgagagccg aggacacggc 2100

cgtatattac tgtgcgaaag gggcacatag tcgcagccta acttggttcg gcccctgggg 2160

ccagggcacc ctggtcaccg tctcctcagt gcctggtgac tcttccgcaa tcagtcctgg 2220

gacagaaacc tcagggacag cctttatgag ttttgatcac atctaacaga gaggatgggc 2280

cagtgagcta gagtgataga gcgtgcgtgt gcggaggtgc agctgttgga gtctggggga 2340

ggcttggtac agcctggggg gtccctgaga ctctcctgtg cagcctctgg attcaccttt 2400

agcaactatg ccatgagctg ggtccgccag gctccaggga aggggctgga gtgggtctca 2460

acttttagta agactggtga tagaacattt tatgcagact ccgtgaaggg ccggttcacc 2520

atctccagag acaattccaa gaacacgctg tatctgcaaa tgaacagcct gagagccgag 2580

gacacggccg tatattactg tgcgaaaaat gggccctttg gttgtggtgg tgactgcgca 2640

gactttgact actggggcca gggcaccctg gtcaccgtct cctcagtgcc tggtgactct 2700

tccgcaatca gtcctgggac agaaacctca gggacagcct ttatgagttt tgatcacatc 2760

taacagagag gatgggccag gaggtgcagc tgttggagtc tgggggaggc ttggtacagc 2820

ctggggggtc cctgagactc tcctgtgcag cctctggatt cacctttagc aactttccca 2880

tgagctgggt ccgccaggct ccagggaagg ggctggagtg ggtctcagtt attagtggta 2940

gtggtggaag cacactctac acagactccg tgaagggccg gttcaccatc tccagagaca 3000

attccaagaa cacgctgtat ctgcaaatga acagcctgag agccgaggac acggccgtat 3060

attactgtgc gaaagatcag gacgattttt ggagtgtttc aaattggttc gacccctggg 3120

gccagggcac cctggtcacc gtctcctcag tgcctggtga ctcttccgca atcagtcctg 3180

ggacagaaac ctcagggaca gcctttatga gttttgatca catctaacag agaggatggg 3240

ccagtataga catctatgat acgctcactc atacgaggtg cagctgttgg agtctggggg 3300

aggcttggta cagcctgggg ggtccctgag actctcctgt gcagcctctg gattcacctt 3360

tagcagcttt gccatgacct gggtccgcca ggctccaggg aaggggctgg agtgggtctc 3420

agctattagt ggtagtgttg gtagcacata ctacgcagac tccgtgcagg gccggttcac 3480

catctccaga gacaattcca agaacacgct gtatctgcaa atgaacagcc tgagagccga 3540

ggacacggcc gtatattact gtgcgaaaga cggtggggcc tactacggaa cggtcaactg 3600

gggccagggc accctggtca ccgtctcctc agtgcctggt gactcttccg caatcagtcc 3660

tgggacagaa acctcaggga cagcctttat gagttttgat cacatctaac agagaggatg 3720

ggccaggagg tgcagctgtt ggagtctggg ggaggcttgg tacagcctgg ggggtccctg 3780

agactctcct gtgcagcctc tggattcacc tttagcatct ttgccgtgag ctgggtccgc 3840

caggctccag ggaaggggct ggagtgggtc tcagctatta gtggtcatgg tcttagcaca 3900

tactacggag actccgtgaa gggccggttc accatctcca gagacaattc caagaacacg 3960

ctgtatctgc aaatgaacag cctgagagcc gaggacacgg ccgtatatta ctgtgcgaaa 4020

gccggaagtg ctggtagcag ctacccgttc tactactacg gtatggacgt ctggggccag 4080

ggcaccctgg tcaccgtctc ctcagtgcct ggtgactctt ccgcaatcag tcctgggaca 4140

gaaacctcag ggacagcctt tatgagtttt gatcacatct aacagagagg atgggccagt 4200

catcgagtct cgagctctct cgcagacacg aggtgcagct gttggagtct gggggaggct 4260

tggtacagcc tggggggtcc ctgagactct cctgtgcagc ctctggattc acctttagca 4320

gctatgccat gatctgggtc cgccaggctc cagggaaggg gctggagtgg gtctcaggta 4380

ttactgttag tggcggtagc acagactacg cagactccgt gaagggccgg ttcaccatct 4440

ccagagacaa ttccaagaac acgctgtatc tgcaaatgaa cagcctgaga gccgaggaca 4500

cggccgtata ttactgtgcg aaaggggggc cccaacttat agcagcagct gggacttatt 4560

atcactacta cggtatggac gtctggggcc agggcaccct ggtcaccgtc tcctcagtgc 4620

ctggtgactc ttccgcaatc agtcctggga cagaaacctc agggacagcc tttatgagtt 4680

ttgatcacat ctaacagaga ggatgggcca ggaggtgcag ctgttggagt ctgggggagg 4740

cttggtacag cctggggggt ccctgagact ctcctgtgca gcctctggat tcacctttag 4800

cagctatacc atgaactggg tccgccaggc tccagggaag gggctggagt gggtctcata 4860

cattagtagt ggtagtggta gcatatacta cgcagactct gtgaagggcc ggttcaccat 4920

ctccagagac aattccaaga acacgctgta tctgcaaatg aacagcctga gagccgagga 4980

cacggccgta tattactgtg cgaaacgagg tgatagtagt gcatacgccc ttgactactg 5040

gggccagggc accctggtca ccgtctcctc agtgcctggt gactcttccg caatcagtcc 5100

tgggacagaa acctcaggga cagcctttat gagttttgat cacatctaac agagaggatg 5160

ggccagtctg tctcgctgag tgacgctgat gtgtacgagg tgcagctgtt ggagtctggg 5220

ggaggcttgg tacagcctgg ggggtccctg agactctcct gtgcagcctc tggattcacc 5280

tttagcagcc atagcatgaa ctgggtccgc caggctccag ggaaggggct ggagtgggtc 5340

tcatacatta gaagtagtag tagtaccata tactacgcag actctgtgaa gggtcggttc 5400

accatctcca gagacaattc caagaacacg ctgtatctgc aaatgaacag cctgagagcc 5460

gaggacacgg ccgtatatta ctgtgcgaaa gtccccaatt actatggttc atttcctcga 5520

gatggtatgg acgtctgggg ccagggcacc ctggtcaccg tctcctcagt gcctggtgac 5580

tcttccgcaa tcagtcctgg gacagaaacc tcagggacag cctttatgag ttttgatcac 5640

atctaacaga gaggatgggc cagtgctata gactgctcgc tacacgtgca gcggaggtgc 5700

agctgttgga gtctggggga ggcttggtac agcctggggg gtccctgaga ctctcctgtg 5760

cagcctctgg attcaccttt agcagctata acatgaactg ggtccgccag gctccaggga 5820

aggggctgga gtgggtctca tacgttagta gtgattctag taccatatac tacgcagact 5880

ttgtggaggg ccggttcacc atctccagag acaattccaa gaacacgctg tatctgcaaa 5940

tgaacagcct gagagccgag gacacggccg tatattactg tgcgaaaggc ctggcgttct 6000

gcgccagctg ctatggatac ttccagctct ggggccaggg caccctggtc accgtctcct 6060

cagtgcctgg tgactcttcc gcaatcagtc ctgggacaga aacctcaggg acagccttta 6120

tgagttttga tcacatctaa cagagaggat gggccaggag gtgcagctgt tggagtctgg 6180

gggaggcttg gtacagcctg gggggtccct gagactctcc tgtgcagcct ctggattcac 6240

ctttagcagc aactccatga gctgggtccg ccaggctcca gggaaggggc tggagtgggt 6300

ctcacttatt tatagcagtg gtggcacata ctacgcagac tccgtgaagg gccggttcac 6360

catctccaga gacaattcca agaacacgct gtatctgcaa atgaacagcc tgagagccga 6420

ggacacggcc gtatattact gtgcgaaagc gtgggtgtat gatagtagtg tttttgacta 6480

ttggggccag ggcaccctgg tcaccgtctc ctcagtgcct ggtgactctt ccgcaatcag 6540

tcctgggaca gaaacctcag ggacagcctt tatgagtttt gatcacatct aacagagagg 6600

atgggccaga ctcacagaga gactcagcga gagagtgtgg aggtgcagct gttggagtct 6660

gggggaggct tggtacagcc tggggggtcc ctgagactct cctgtgcagc ctctggattc 6720

acctttagct atgccataag ctgggtccgc caggctccag ggaaggggct ggagtgggtc 6780

tcaggtatta gtggcggcgg tggtagcaca ttctacgctg actccgtgca gggccggttc 6840

accatctcca gagacaattc caagaacacg ctgtatctgc aaatgaacag cctgagagcc 6900

gaggacacgg ccgtatatta ctgtgcgaaa cccgagagag ggatagtggg ggctgcattt 6960

gactcctggg gccagggcac cctggtcacc gtctcctcag tgcctggtga ctcttccgca 7020

atcagtcctg ggacagaaac ctcagggaca gcctttatga gttttgatca catctaacag 7080

agaggatggg ccaggaggtg cagctgttgg agtctggggg aggcttggta cagcctgggg 7140

ggtccctgag actctcctgt gcagcctctg gattcacctt tagcagccat tggatgaggt 7200

gggtccgcca ggctccaggg aaggggctgg agtgggtctc aaacataaag caaggtggaa 7260

gtgaagaata ttatgtggac tctgtgaagg gccggttcac catctccaga gacaattcca 7320

agaacacgct gtatctgcaa atgaacagcc tgagagccga ggacacggcc gtatattact 7380

gtgcgaaagg aaattataag gcaggttgtg actactgggg ccagggcacc ctggtcaccg 7440

tctcctcagt gcctggtgac tcttccgcaa tcagtcctgg gacagaaacc tcagggacag 7500

cctttatgag ttttgatcac atctaacaga gaggatgggc cagagctatc gcgagtctga 7560

ctgagtgagc tcggaggtgc agctgttgga gtctggggga ggcttggtac agcctggggg 7620

gtccctgaga ctctcctgtg cagcctctgg attcaccttt agcacctata gcatgaactg 7680

ggtccgccag gctccaggga aggggctgga gtgggtctca tccattacta gtagtggtag 7740

ttacatatac tgcgcagact cagtgaaggg ccggttcacc atctccagag acaattccaa 7800

gaacacgctg tatctgcaaa tgaacagcct gagagccgag gacacggccg tatattactg 7860

tgcgaaagat ggctccggta tggacgtctg gggccagggc accctggtca ccgtctcctc 7920

agtgcctggt gactcttccg caatcagtcc tgggacagaa acctcaggga cagcctttat 7980

gagttttgat cacatctaac agagaggatg ggccaggagg tgcagctgtt ggagtctggg 8040

ggaggcttgg tacagcctgg ggggtccctg agactctcct gtgcagcctc tggattcacc 8100

tttagctcct atagcatgac ctgggtccgc caggctccag ggaaggggct ggagtgggtc 8160

tcaaacatta gcagtagtag taataccgca ttctacgcag actccgtgaa gggccggttc 8220

accatctcca gagacaattc caagaacacg ctgtatctgc aaatgaacag cctgagagcc 8280

gaggacacgg ccgtatatta ctgtgcgaaa gatcgaaatg gctacaattt ctttgactac 8340

tggggccagg gcaccctggt caccgtctcc tcagtgcctg gtgactcttc cgcaatcagt 8400

cctgggacag aaacctcagg gacagccttt atgagttttg atcacatcta acagagagga 8460

tgggccagat agctctcgat cacgtgcgat cgtagcagga ggtgcagctg ttggagtctg 8520

ggggaggctt ggtacagcct ggggggtccc tgagactctc ctgtgcagcc tctggattca 8580

cctttagcgg ctttgccatg aactgggtcc gccaggctcc agggaagggg ctggagtggg 8640

tctcaactat tagtaatagt gctggtagca catattacgc agactccgtg aagggccggt 8700

tcaccatctc cagagacaat tccaagaaca cgctgtatct gcaaatgaac agcctgagag 8760

ccgaggacac ggccgtatat tactgtgcga aatatgggga ggatattgtc gttgtgccag 8820

ctcgctacgg tgtggacgtc tggggccagg gcaccctggt caccgtctcc tcagtgcctg 8880

gtgactcttc cgcaatcagt cctgggacag aaacctcagg gacagccttt atgagttttg 8940

atcacatcta acagagagga tgggccaggc tagcatgctg gccaggatcc atgcccatgg 9000

ccgcggatgc ggcgcgccat cgatatgcgt cgacatttaa atggcccccc cggtgatatc 9060

cccgggtcta gagcggccgc taccgttcgt atattctatc ttatacgaag ttatatcgaa 9120

ttcctgcaga gatctagctt ggcgtaatca tggtcatagc tgtttcctgt gtgaaattgt 9180

tatccgctca caattccaca caacatacga gccggaagca taaagtgtaa agcctggggt 9240

gcctaatgag tgagctaact cacattaatt gcgttgcgct cactgcccgc tttccagtcg 9300

ggaaacctgt cgtgccagct gcattaatga atcggccaac gcgcggggag aggcggtttg 9360

cgtattgggc gctcttccgc ttcctcgctc actgactcgc tgcgctcggt cgttcggctg 9420

cggcgagcgg tatcagctca ctcaaaggcg gtaatacggt tatccacaga atcaggggat 9480

aacgcaggaa agaacatgtg agcaaaaggc cagcaaaagg ccaggaaccg taaaaaggcc 9540

gcgttgctgg cgtttttcca taggctccgc ccccctgacg agcatcacaa aaatcgacgc 9600

tcaagtcaga ggtggcgaaa cccgacagga ctataaagat accaggcgtt tccccctgga 9660

agctccctcg tgcgctctcc tgttccgacc ctgccgctta ccggatacct gtccgccttt 9720

ctcccttcgg gaagcgtggc gctttctcat agctcacgct gtaggtatct cagttcggtg 9780

taggtcgttc gctccaagct gggctgtgtg cacgaacccc ccgttcagcc cgaccgctgc 9840

gccttatccg gtaactatcg tcttgagtcc aacccggtaa gacacgactt atcgccactg 9900

gcagcagcca ctggtaacag gattagcaga gcgaggtatg taggcggtgc tacagagttc 9960

ttgaagtggt ggcctaacta cggctacact agaagaacag tatttggtat ctgcgctctg 10020

ctgaagccag ttaccttcgg aaaaagagtt ggtagctctt gatccggcaa acaaaccacc 10080

gctggtagcg gtggtttttt tgtttgcaag cagcagatta cgcgcagaaa aaaaggatct 10140

caagaagatc ctttgatctt ttctacgggg tctgacgctc agtggaacga aaactcacgt 10200

taagggattt tggtcatgag attatcaaaa aggatcttca cctagatcct tttaaattaa 10260

aaatgaagtt ttaaatcaat ctaaagtata tatgagtaaa cttggtctga cagttaccaa 10320

tgcttaatca gtgaggcacc tatctcagcg atctgtctat ttcgttcatc catagttgcc 10380

tgactccccg tcgtgtagat aactacgata cgggagggct taccatctgg ccccagtgct 10440

gcaatgatac cgcgagaccc acgctcaccg gctccagatt tatcagcaat aaaccagcca 10500

gccggaaggg ccgagcgcag aagtggtcct gcaactttat ccgcctccat ccagtctatt 10560

aattgttgcc gggaagctag agtaagtagt tcgccagtta atagtttgcg caacgttgtt 10620

gccattgcta caggcatcgt ggtgtcacgc tcgtcgtttg gtatggcttc attcagctcc 10680

ggttcccaac gatcaaggcg agttacatga tcccccatgt tgtgcaaaaa agcggttagc 10740

tccttcggtc ctccgatcgt tgtcagaagt aagttggccg cagtgttatc actcatggtt 10800

atggcagcac tgcataattc tcttactgtc atgccatccg taagatgctt ttctgtgact 10860

ggtgagtact caaccaagtc attctgagaa tagtgtatgc ggcgaccgag ttgctcttgc 10920

ccggcgtcaa tacgggataa taccgcgcca catagcagaa ctttaaaagt gctcatcatt 10980

ggaaaacgtt cttcggggcg aaaactctca aggatcttac cgctgttgag atccagttcg 11040

atgtaaccca ctcgtgcacc caactgatct tcagcatctt ttactttcac cagcgtttct 11100

gggtgagcaa aaacaggaag gcaaaatgcc gcaaaaaagg gaataagggc gacacggaaa 11160

tgttgaatac tcatactctt cctttttcaa tattattgaa gcatttatca gggttattgt 11220

ctcatgagcg gatacatatt tgaatgtatt tagaaaaata aacaaatagg ggttccgcgc 11280

acatttcccc gaaaagtgcc acctgacgtc taagaaacca ttattatcat gacattaacc 11340

tataaaaata ggcgtatcac gaggcccttt cgtc 11374

<210> 182

<211>  11392
<212>  DNA
<213>  Artificial Sequence

<220>
<223>  Synthetic DNA

<400>  182
tcgcgcgttt cggtgatgac ggtgaaaacc tctgacacat gcagctcccg gagacggtca      60

cagcttgtct gtaagcggat gccgggagca gacaagcccg tcagggcgcg tcagcgggtg     120

ttggcgggtg tcggggctgg cttaactatg cggcatcaga gcagattgta ctgagagtgc     180

accatatgcg gtgtgaaata ccgcacagat gcgtaaggag aaaataccgc atcaggcgcc     240

attcgccatt caggctgcgc aactgttggg aagggcgatc ggtgcgggcc tcttcgctat     300

tacgccagct ggcgaaaggg ggatgtgctg caaggcgatt aagttgggta acgccagggt     360

tttcccagtc acgacgttgt aaaacgacgg ccagtgaatt catatgtagc ttacgacact     420

gataacttcg tatatggtat tatatacgaa gttataagct tcaccatgat tgaacaagat     480

ggattgcacg caggttctcc ggccgcttgg gtggagaggc tattcggcta tgactgggca     540

caacagacaa tcggctgctc tgatgccgcc gtgttccggc tgtcagcgca ggggcgcccg     600

gttctttttg tcaagaccga cctgtccggt gccctgaatg aactgcagga cgaggcagcg     660

cggctatcgt ggctggccac gacgggcgtt ccttgcgcag ctgtgctcga cgttgtcact     720

gaagcgggaa gggactggct gctattgggc gaagtgccgg ggcaggatct cctgtcatct     780

caccttgctc ctgccgagaa agtatccatc atggctgatg caatgcggcg gctgcatacg     840

cttgatccgg ctacctgccc attcgaccac caagcgaaac atcgcatcga gcgagcacgt     900

actcggatgg aagccggtct tgtcgatcag gatgatctgg acgaagagca tcaggggctc     960

gcgccagccg aactgttcgc caggctcaag gcgcgcatgc ccgacggcga ggatctcgtc    1020

gtgacccatg gcgatgcctg cttgccgaat atcatggtgg aaaatggccg cttttctgga    1080

ttcatcgact gtggccggct gggtgtggcg gaccgctatc aggacatagc gttggctacc    1140

cgtgatattg ctgaagagct tggcggcgaa tgggctgacc gcttcctcgt gctttacggt    1200

atcgccgctc ccgattcgca gcgcatcgcc ttctatcgcc ttcttgacga gttcttctga    1260

actagtgcgg gactctgggg ttcgaaatga ccgaccaagc gacgcccaac ctgccatcac    1320

gagatttcga ttccaccgcc gccttctatg aaaggttggg cttcggaatc gttttccggg    1380

acgccggctg gatgatcctc cagcgcgggg atctcatgct ggagttcttc gcccacccca    1440

acttgtttat tgcagcttat aatggttaca aataaagcaa tagcatcaca aatttcacaa    1500

ataaagcatt tttttcactg cattctagtt gtggtttgtc caaactcatc aatgtatctt    1560

atcatgtctg ttataccgtc gatctctagc tagagcttgg cgtaatcatg gtcatagctg    1620

tttcctgtgt gcccacccca acttgtttat tgcagcttat aatggttaca aataaagcaa    1680

tagcatcaca aatttcacaa ataaagcatt tttttcactg cattctagtt gtggtttgtc 1740

caaactcatc aatgtatctt atcatgtctg ataacttcgt ataatgtatg ctatacgaac 1800

ggtactcgag agctcatgcg gtaccgctag catgctggcc aggatccatg cccatggccg 1860

cggatgcggc gcgccatcga tatgcgtcga catttaaatg ggccccccgg tgatatcccc 1920

ggggctagcc tggcccatcc tctctgttag atgtgatcaa aactcataaa ggctgtccct 1980

gaggtttctg tcccaggact gattgcggaa gagtcaccag gcactgagga gacggtgacc 2040

agggtgccct ggccccagac gtccacaccg tagcgagctg gcacaacgac aatatcctcc 2100

ccatatttcg cacagtaata tacggccgtg tcctcggctc tcaggctgtt catttgcaga 2160

tacagcgtgt tcttggaatt gtctctggag atggtgaacc ggcccttcac ggagtctgcg 2220

taatatgtgc taccagcact attactaata gttgagaccc actccagccc cttccctgga 2280

gcctggcgga cccagttcat ggcaaagccg ctaaaggtga atccagaggc tgcacaggag 2340

agtctcaggg accccccagg ctgtaccaag cctcccccag actccaacag ctgcacctcc 2400

tgctacgatc gcacgtgatc gagagctatc tggcccatcc tctctgttag atgtgatcaa 2460

aactcataaa ggctgtccct gaggtttctg tcccaggact gattgcggaa gagtcaccag 2520

gcactgagga gacggtgacc agggtgccct ggccccagta gtcaaagaaa ttgtagccat 2580

ttcgatcttt cgcacagtaa tatacggccg tgtcctcggc tctcaggctg ttcatttgca 2640

gatacagcgt gttcttggaa ttgtctctgg agatggtgaa ccggcccttc acggagtctg 2700

cgtagaatgc ggtattacta ctactgctaa tgtttgagac ccactccagc cccttccctg 2760

gagcctggcg gacccaggtc atgctatagg agctaaaggt gaatccagag gctgcacagg 2820

agagtctcag ggacccccca ggctgtacca agcctccccc agactccaac agctgcacct 2880

cctggcccat cctctctgtt agatgtgatc aaaactcata aaggctgtcc ctgaggtttc 2940

tgtcccagga ctgattgcgg aagagtcacc aggcactgag gagacggtga ccagggtgcc 3000

ctggccccag acgtccatac cggagccatc tttcgcacag taatatacgg ccgtgtcctc 3060

ggctctcagg ctgttcattt gcagatacag cgtgttcttg gaattgtctc tggagatggt 3120

gaaccggccc ttcactgagt ctgcgcagta tatgtaacta ccactactag taatggatga 3180

gacccactcc agccccttcc ctggagcctg gcggacccag ttcatgctat aggtgctaaa 3240

ggtgaatcca gaggctgcac aggagagtct cagggacccc ccaggctgta ccaagcctcc 3300

cccagactcc aacagctgca cctccgagct cactcagtca gactcgcgat agctctggcc 3360

catcctctct gttagatgtg atcaaaactc ataaaggctg tccctgaggt ttctgtccca 3420

ggactgattg cggaagagtc accaggcact gaggagacgg tgaccagggt gccctggccc 3480

cagtagtcac aacctgcctt ataatttcct ttcgcacagt aatatacggc cgtgtcctcg 3540

gctctcaggc tgttcatttg cagatacagc gtgttcttgg aattgtctct ggagatggtg 3600

aaccggccct tcacagagtc cacataatat tcttcacttc caccttgctt tatgtttgag 3660

acccactcca gccccttccc tggagcctgg cggacccacc tcatccaatg gctgctaaag 3720

gtgaatccag aggctgcaca ggagagtctc agggaccccc caggctgtac caagcctccc 3780

ccagactcca acagctgcac ctcctggccc atcctctctg ttagatgtga tcaaaactca 3840

taaaggctgt ccctgaggtt tctgtcccag gactgattgc ggaagagtca ccaggcactg 3900

aggagacggt gaccagggtg ccctggcccc aggagtcaaa tgcagccccc actatccctc 3960

tctcgggttt cgcacagtaa tatacggccg tgtcctcggc tctcaggctg ttcatttgca 4020

gatacagcgt gttcttggaa ttgtctctgg agatggtgaa ccggccctgc acggagtcag 4080

cgtagaatgt gctaccaccg ccgccactaa tacctgagac ccactccagc cccttccctg 4140

gagcctggcg gacccagctt atggcatagc taaaggtgaa tccagaggct gcacaggaga 4200

gtctcaggga ccccccaggc tgtaccaagc ctcccccaga ctccaacagc tgcacctcca 4260

cactctctcg ctgagtctct ctgtgagtct ggcccatcct ctctgttaga tgtgatcaaa 4320

actcataaag gctgtccctg aggtttctgt cccaggactg attgcggaag agtcaccagg 4380

cactgaggag acggtgacca gggtgccctg gccccaatag tcaaaaacac tactatcata 4440

cacccacgct ttcgcacagt aatatacggc cgtgtcctcg gctctcaggc tgttcatttg 4500

cagatacagc gtgttcttgg aattgtctct ggagatggtg aaccggccct tcacggagtc 4560

tgcgtagtat gtgccaccac tgctataaat aagtgagacc cactccagcc ccttccctgg 4620

agcctggcgg acccagctca tggagttgct gctaaaggtg aatccagagg ctgcacagga 4680

gagtctcagg gaccccccag gctgtaccaa gcctccccca gactccaaca gctgcacctc 4740

ctggcccatc ctctctgtta gatgtgatca aaactcataa aggctgtccc tgaggtttct 4800

gtcccaggac tgattgcgga agagtcacca ggcactgagg agacggtgac cagggtgccc 4860

tggccccaga gctggaagta tccatagcag ctggcgcaga acgccaggcc tttcgcacag 4920

taatatacgg ccgtgtcctc ggctctcagg ctgttcattt gcagatacag cgtgttcttg 4980

gaattgtctc tggagatggt gaaccggccc tccacaaagt ctgcgtagta tatggtacta 5040

gaatcactac taacgtatga gacccactcc agccccttcc ctggagcctg gcggacccag 5100

ttcatgttat agctgctaaa ggtgaatcca gaggctgcac aggagagtct cagggacccc 5160

ccaggctgta ccaagcctcc cccagactcc aacagctgca cctccgctgc acgtgtagcg 5220

agcagtctat agcactggcc catcctctct gttagatgtg atcaaaactc ataaaggctg 5280

tccctgaggt ttctgtccca ggactgattg cggaagagtc accaggcact gaggagacgg 5340

tgaccagggt gccctggccc cagacgtcca taccatctcg aggaaatgaa ccatagtaat 5400

tggggacttt cgcacagtaa tatacggccg tgtcctcggc tctcaggctg ttcatttgca 5460

gatacagcgt gttcttggaa ttgtctctgg agatggtgaa ccgacccttc acagagtctg 5520

cgtagtatat ggtactacta ctacttctaa tgtatgagac ccactccagc cccttccctg 5580

gagcctggcg gacccagttc atgctatggc tgctaaaggt gaatccagag gctgcacagg 5640

agagtctcag ggacccccca ggctgtacca agcctccccc agactccaac agctgcacct 5700

cgtacacatc agcgtcactc agcgagacag actggcccat cctctctgtt agatgtgatc 5760

aaaactcata aaggctgtcc ctgaggtttc tgtcccagga ctgattgcgg aagagtcacc 5820

aggcactgag gagacggtga ccagggtgcc ctggccccag tagtcaaggg cgtatgcact 5880

actatcacct cgtttcgcac agtaatatac ggccgtgtcc tcggctctca ggctgttcat 5940

ttgcagatac agcgtgttct tggaattgtc tctggagatg gtgaaccggc ccttcacaga 6000

gtctgcgtag tatatgctac cactaccact actaatgtat gagacccact ccagcccctt 6060

ccctggagcc tggcggaccc agttcatggt atagctgcta aaggtgaatc cagaggctgc 6120

acaggagagt ctcagggacc ccccaggctg taccaagcct cccccagact ccaacagctg 6180

cacctcctgg cccatcctct ctgttagatg tgatcaaaac tcataaaggc tgtccctgag 6240

gtttctgtcc caggactgat tgcggaagag tcaccaggca ctgaggagac ggtgaccagg 6300

gtgccctggc cccagacgtc cataccgtag tagtgataat aagtcccagc tgctgctata 6360

agttggggcc cccctttcgc acagtaatat acggccgtgt cctcggctct caggctgttc 6420

atttgcagat acagcgtgtt cttggaattg tctctggaga tggtgaaccg gcccttcacg 6480

gagtctgcgt agtctgtgct accgccacta acagtaatac ctgagaccca ctccagcccc 6540

ttccctggag cctggcggac ccagatcatg gcatagctgc taaaggtgaa tccagaggct 6600

gcacaggaga gtctcaggga ccccccaggc tgtaccaagc ctcccccaga ctccaacagc 6660

tgcacctcgt gtctgcgaga gagctcgaga ctcgatgact ggcccatcct ctctgttaga 6720

tgtgatcaaa actcataaag gctgtccctg aggtttctgt cccaggactg attgcggaag 6780

agtcaccagg cactgaggag acggtgacca gggtgccctg gccccagacg tccataccgt 6840

agtagtagaa cgggtagctg ctaccagcac ttccggcttt cgcacagtaa tatacggccg 6900

tgtcctcggc tctcaggctg ttcatttgca gatacagcgt gttcttggaa ttgtctctgg 6960

agatggtgaa ccggcccttc acggagtctc cgtagtatgt gctaagacca tgaccactaa 7020

tagctgagac ccactccagc cccttccctg gagcctggcg gacccagctc acggcaaaga 7080

tgctaaaggt gaatccagag gctgcacagg agagtctcag ggacccccca ggctgtacca 7140

agcctccccc agactccaac agctgcacct cctggcccat cctctctgtt agatgtgatc 7200

aaaactcata aaggctgtcc ctgaggtttc tgtcccagga ctgattgcgg aagagtcacc 7260

aggcactgag gagacggtga ccagggtgcc ctggccccag ttgaccgttc cgtagtaggc 7320

cccaccgtct ttcgcacagt aatatacggc cgtgtcctcg gctctcaggc tgttcatttg 7380

cagatacagc gtgttcttgg aattgtctct ggagatggtg aaccggccct gcacggagtc 7440

tgcgtagtat gtgctaccaa cactaccact aatagctgag acccactcca gccccttccc 7500

tggagcctgg cggacccagg tcatggcaaa gctgctaaag gtgaatccag aggctgcaca 7560

ggagagtctc agggaccccc caggctgtac caagcctccc ccagactcca acagctgcac 7620

ctcgtatgag tgagcgtatc atagatgtct atactggccc atcctctctg ttagatgtga 7680

tcaaaactca taaaggctgt ccctgaggtt tctgtcccag gactgattgc ggaagagtca 7740

ccaggcactg aggagacggt gaccagggtg ccctggcccc aggggtcgaa ccaatttgaa 7800

acactccaaa aatcgtcctg atctttcgca cagtaatata cggccgtgtc ctcggctctc 7860

aggctgttca tttgcagata cagcgtgttc ttggaattgt ctctggagat ggtgaaccgg 7920

cccttcacgg agtctgtgta gagtgtgctt ccaccactac cactaataac tgagacccac 7980

tccagcccct tccctggagc ctggcggacc cagctcatgg gaaagttgct aaaggtgaat 8040

ccagaggctg cacaggagag tctcagggac cccccaggct gtaccaagcc tcccccagac 8100

tccaacagct gcacctcctg gcccatcctc tctgttagat gtgatcaaaa ctcataaagg 8160

ctgtccctga ggtttctgtc ccaggactga ttgcggaaga gtcaccaggc actgaggaga 8220

cggtgaccag ggtgccctgg ccccagtagt caaagtctgc gcagtcacca ccacaaccaa 8280

agggcccatt tttcgcacag taatatacgg ccgtgtcctc ggctctcagg ctgttcattt 8340

gcagatacag cgtgttcttg gaattgtctc tggagatggt gaaccggccc ttcacggagt 8400

ctgcataaaa tgttctatca ccagtcttac taaaagttga gacccactcc agccccttcc 8460

ctggagcctg gcggacccag ctcatggcat agttgctaaa ggtgaatcca gaggctgcac 8520

aggagagtct cagggacccc ccaggctgta ccaagcctcc cccagactcc aacagctgca 8580

cctccgcaca cgcacgctct atcactctag ctcactggcc catcctctct gttagatgtg 8640

atcaaaactc ataaaggctg tccctgaggt ttctgtccca ggactgattg cggaagagtc 8700

accaggcact gaggagacgg tgaccagggt gccctggccc caggggccga accaagttag 8760

gctgcgacta tgtgcccctt tcgcacagta atatacggcc gtgtcctcgg ctctcaggct 8820

gttcatttgc agatacagcg tgttcttgga attgtctctg gagatggtga accggccctt 8880

cacggagtct gcgtagtatg tgttactagc actaccagta atagttgaga cccactccag 8940

ccccttccct ggagcctggc ggacccaatt cagggcaaaa ctgctaaagg tgaatccaga 9000

ggctgcacag gagagtctca gggacccccc aggctgtacc aagcctcccc cagactccaa 9060

cagctgcacc tcggtaccgg atcctctaga gcggccgcta ccgttcgtat attctatctt 9120

atacgaagtt atatcgaatt cctgcagaga tctagcttgg cgtaatcatg gtcatagctg 9180

tttcctgtgt gaaattgtta tccgctcaca attccacaca acatacgagc cggaagcata 9240

aagtgtaaag cctggggtgc ctaatgagtg agctaactca cattaattgc gttgcgctca 9300

ctgcccgctt tccagtcggg aaacctgtcg tgccagctgc attaatgaat cggccaacgc 9360

gcggggagag gcggtttgcg tattgggcgc tcttccgctt cctcgctcac tgactcgctg 9420

cgctcggtcg ttcggctgcg gcgagcggta tcagctcact caaaggcggt aatacggtta 9480

tccacagaat caggggataa cgcaggaaag aacatgtgag caaaaggcca gcaaaaggcc 9540

aggaaccgta aaaaggccgc gttgctggcg tttttccata ggctccgccc ccctgacgag 9600

catcacaaaa atcgacgctc aagtcagagg tggcgaaacc cgacaggact ataaagatac 9660

caggcgtttc cccctggaag ctccctcgtg cgctctcctg ttccgaccct gccgcttacc 9720

ggatacctgt ccgcctttct cccttcggga agcgtggcgc tttctcatag ctcacgctgt 9780

aggtatctca gttcggtgta ggtcgttcgc tccaagctgg gctgtgtgca cgaacccccc 9840

gttcagcccg accgctgcgc cttatccggt aactatcgtc ttgagtccaa cccggtaaga 9900

cacgacttat cgccactggc agcagccact ggtaacagga ttagcagagc gaggtatgta 9960

ggcggtgcta cagagttctt gaagtggtgg cctaactacg gctacactag aagaacagta 10020

tttggtatct gcgctctgct gaagccagtt accttcggaa aaagagttgg tagctcttga 10080

tccggcaaac aaaccaccgc tggtagcggt ggtttttttg tttgcaagca gcagattacg 10140

cgcagaaaaa aaggatctca agaagatcct ttgatctttt ctacggggtc tgacgctcag 10200

tggaacgaaa actcacgtta agggattttg gtcatgagat tatcaaaaag gatcttcacc 10260

tagatccttt taaattaaaa atgaagtttt aaatcaatct aaagtatata tgagtaaact 10320

tggtctgaca gttaccaatg cttaatcagt gaggcaccta tctcagcgat ctgtctattt 10380

cgttcatcca tagttgcctg actccccgtc gtgtagataa ctacgatacg ggagggctta 10440

ccatctggcc ccagtgctgc aatgataccg cgagacccac gctcaccggc tccagattta 10500

tcagcaataa accagccagc cggaagggcc gagcgcagaa gtggtcctgc aactttatcc 10560

gcctccatcc agtctattaa ttgttgccgg gaagctagag taagtagttc gccagttaat 10620

agtttgcgca acgttgttgc cattgctaca ggcatcgtgg tgtcacgctc gtcgtttggt 10680

atggcttcat tcagctccgg ttcccaacga tcaaggcgag ttacatgatc ccccatgttg 10740

tgcaaaaaag cggttagctc cttcggtcct ccgatcgttg tcagaagtaa gttggccgca 10800

gtgttatcac tcatggttat ggcagcactg cataattctc ttactgtcat gccatccgta 10860

agatgctttt ctgtgactgg tgagtactca accaagtcat tctgagaata gtgtatgcgg 10920

cgaccgagtt gctcttgccc ggcgtcaata cgggataata ccgcgccaca tagcagaact 10980

ttaaaagtgc tcatcattgg aaaacgttct tcggggcgaa aactctcaag gatcttaccg 11040

ctgttgagat ccagttcgat gtaacccact cgtgcaccca actgatcttc agcatctttt 11100

actttcacca gcgtttctgg gtgagcaaaa acaggaaggc aaaatgccgc aaaaaaggga 11160

ataagggcga cacggaaatg ttgaatactc atactcttcc tttttcaata ttattgaagc 11220

atttatcagg gttattgtct catgagcgga tacatatttg aatgtattta gaaaaataaa 11280

caaatagggg ttccgcgcac atttccccga aaagtgccac ctgacgtcta agaaaccatt 11340

attatcatga cattaaccta taaaaatagg cgtatcacga ggccctttcg tc 11392

<210> 183
<211> 1251
<212> PRT
<213> Homo sapiens

<400> 183

Ile Ile Pro Val Asn His His His His His His Glu Glu Glu Asn Pro
1               5                   10                  15

Asp Phe Trp Asn Arg Glu Ala Ala Glu Ala Leu Gly Ala Ala Lys Lys
            20                  25                  30

Leu Gln Pro Ala Gln Thr Ala Ala Lys Asn Leu Ile Ile Phe Leu Gly
        35                  40                  45

Asp Gly Met Gly Val Ser Thr Val Thr Ala Ala Arg Ile Leu Lys Gly
    50                  55                  60

Gln Lys Lys Asp Lys Leu Gly Pro Glu Ile Pro Leu Ala Met Asp Arg
65                  70                  75                  80

Phe Pro Tyr Val Ala Leu Ser Lys Thr Tyr Asn Val Asp Lys His Val
                85                  90                  95

Pro Asp Ser Gly Ala Thr Ala Thr Ala Tyr Leu Cys Gly Val Lys Gly
            100                 105                 110

Asn Phe Gln Thr Ile Gly Leu Ser Ala Ala Ala Arg Phe Asn Gln Cys
        115                 120                 125

Asn Thr Thr Arg Gly Asn Glu Val Ile Ser Val Met Asn Arg Ala Lys
    130                 135                 140

Lys Ala Gly Lys Ser Val Gly Val Val Thr Thr Thr Arg Val Gln His
145                 150                 155                 160

Ala Ser Pro Ala Gly Thr Tyr Ala His Thr Val Asn Arg Asn Trp Tyr
                165                 170                 175

Ser Asp Ala Asp Val Pro Ala Ser Ala Arg Gln Glu Gly Cys Gln Asp

            180                 185                 190

Ile Ala Thr Gln Leu Ile Ser Asn Met Asp Ile Asp Val Ile Leu Gly
        195                 200                 205

Gly Gly Arg Lys Tyr Met Phe Pro Met Gly Thr Pro Asp Pro Glu Tyr
    210                 215                 220

Pro Asp Asp Tyr Ser Gln Gly Gly Thr Arg Leu Asp Gly Lys Asn Leu
225                 230                 235                 240

Val Gln Glu Trp Leu Ala Lys Arg Gln Gly Ala Arg Tyr Val Trp Asn
                245                 250                 255

Arg Thr Glu Leu Met Gln Ala Ser Leu Asp Pro Ser Val Thr His Leu
            260                 265                 270

Met Gly Leu Phe Glu Pro Gly Asp Met Lys Tyr Glu Ile His Arg Asp
        275                 280                 285

Ser Thr Leu Asp Pro Ser Leu Met Glu Met Thr Glu Ala Ala Leu Arg
    290                 295                 300

Leu Leu Ser Arg Asn Pro Arg Gly Phe Phe Leu Phe Val Glu Gly Gly
305                 310                 315                 320

Arg Ile Asp His Gly His His Glu Ser Arg Ala Tyr Arg Ala Leu Thr
                325                 330                 335

Glu Thr Ile Met Phe Asp Asp Ala Ile Glu Arg Ala Gly Gln Leu Thr
            340                 345                 350

Ser Glu Glu Asp Thr Leu Ser Leu Val Thr Ala Asp His Ser His Val
        355                 360                 365

Phe Ser Phe Gly Gly Tyr Pro Leu Arg Gly Ser Ser Ile Phe Gly Leu
    370                 375                 380

Ala Pro Gly Lys Ala Arg Asp Arg Lys Ala Tyr Thr Val Leu Leu Tyr
385                 390                 395                 400

Gly Asn Gly Pro Gly Tyr Val Leu Lys Asp Gly Ala Arg Pro Asp Val
                405                 410                 415

Thr Glu Ser Glu Ser Gly Ser Pro Glu Tyr Arg Gln Gln Ser Ala Val
            420                 425                 430

Pro Leu Asp Glu Glu Thr His Ala Gly Glu Asp Val Ala Val Phe Ala
        435                 440                 445

Arg Gly Pro Gln Ala His Leu Val His Gly Val Gln Glu Gln Thr Phe
    450                 455                 460

Ile Ala His Val Met Ala Phe Ala Ala Cys Leu Glu Pro Tyr Thr Ala
465                 470                 475                 480

Cys Asp Leu Ala Pro Pro Ala Gly Thr Thr Asp Ala Ala His Pro Gly
                485                 490                 495

Glu Phe Arg Ala Asn Tyr Gln Asn Gly Lys Asn Asn Val Pro Arg Leu
            500                 505                 510

Lys Leu Ser Tyr Lys Glu Met Leu Glu Ser Asn Asn Val Ile Thr Phe
        515                 520                 525

Asn Gly Leu Ala Asn Ser Ser Ser Tyr His Thr Phe Leu Leu Asp Glu
    530                 535                 540

Glu Arg Ser Arg Leu Tyr Val Gly Ala Lys Asp His Ile Phe Ser Phe
545                 550                 555                 560

Asp Leu Val Asn Ile Lys Asp Phe Gln Lys Ile Val Trp Pro Val Ser
                565                 570                 575

Tyr Thr Arg Arg Asp Glu Cys Lys Trp Ala Gly Lys Asp Ile Leu Lys
            580                 585                 590

Glu Cys Ala Asn Phe Ile Lys Val Leu Lys Ala Tyr Asn Gln Thr His
        595                 600                 605

Leu Tyr Ala Cys Gly Thr Gly Ala Phe His Pro Ile Cys Thr Tyr Ile
    610                 615                 620

Glu Ile Gly His His Pro Glu Asp Asn Ile Phe Lys Leu Glu Asn Ser
625                 630                 635                 640

His Phe Glu Asn Gly Arg Gly Lys Ser Pro Tyr Asp Pro Lys Leu Leu
                645                 650                 655

Thr Ala Ser Leu Leu Ile Asp Gly Glu Leu Tyr Ser Gly Thr Ala Ala
            660                 665                 670

Asp Phe Met Gly Arg Asp Phe Ala Ile Phe Arg Thr Leu Gly His His
        675                 680                 685

His Pro Ile Arg Thr Glu Gln His Asp Ser Arg Trp Leu Asn Asp Pro
    690                 695                 700

Lys Phe Ile Ser Ala His Leu Ile Ser Glu Ser Asp Asn Pro Glu Asp
705                 710                 715                 720

Asp Lys Val Tyr Phe Phe Phe Arg Glu Asn Ala Ile Asp Gly Glu His
                725                 730                 735

Ser Gly Lys Ala Thr His Ala Arg Ile Gly Gln Ile Cys Lys Asn Asp
            740                 745                 750

Phe Gly Gly His Arg Ser Leu Val Asn Lys Trp Thr Thr Phe Leu Lys
        755                 760                 765

Ala Arg Leu Ile Cys Ser Val Pro Gly Pro Asn Gly Ile Asp Thr His
    770                 775                 780

Phe Asp Glu Leu Gln Asp Val Phe Leu Met Asn Phe Lys Asp Pro Lys
785                 790                 795                 800

Asn Pro Val Val Tyr Gly Val Phe Thr Thr Ser Ser Asn Ile Phe Lys
                805                 810                 815

Gly Ser Ala Val Cys Met Tyr Ser Met Ser Asp Val Arg Arg Val Phe
            820                 825                 830

Leu Gly Pro Tyr Ala His Arg Asp Gly Pro Asn Tyr Gln Trp Val Pro
        835                 840                 845

Tyr Gln Gly Arg Val Pro Tyr Pro Arg Pro Gly Thr Cys Pro Ser Lys
    850                 855                 860

Thr Phe Gly Gly Phe Asp Ser Thr Lys Asp Leu Pro Asp Asp Val Ile
865                 870                 875                 880

Thr Phe Ala Arg Ser His Pro Ala Met Tyr Asn Pro Val Phe Pro Met
                885                 890                 895

Asn Asn Arg Pro Ile Val Ile Lys Thr Asp Val Asn Tyr Gln Phe Thr
            900                 905                 910

Gln Ile Val Val Asp Arg Val Asp Ala Glu Asp Gly Gln Tyr Asp Val
        915                 920                 925

Met Phe Ile Gly Thr Asp Val Gly Thr Val Leu Lys Val Val Ser Ile
    930                 935                 940

Pro Lys Glu Thr Trp Tyr Asp Leu Glu Glu Val Leu Leu Glu Glu Met
945                 950                 955                 960

Thr Val Phe Arg Glu Pro Thr Ala Ile Ser Ala Met Glu Leu Ser Thr
                965                 970                 975

Lys Gln Gln Gln Leu Tyr Ile Gly Ser Thr Ala Gly Val Ala Gln Leu
            980                 985                 990

Pro Leu His Arg Cys Asp Ile Tyr Gly Lys Ala Cys Ala Glu Cys Cys
        995                 1000                1005

Leu Ala Arg Asp Pro Tyr Cys Ala Trp Asp Gly Ser Ala Cys Ser
    1010                1015                1020

Arg Tyr Phe Pro Thr Ala Lys Arg Arg Thr Arg Arg Gln Asp Ile
    1025                1030                1035

Arg Asn Gly Asp Pro Leu Thr His Cys Ser Asp Leu His His Asp
    1040                1045                1050

Asn His His Gly His Ser Pro Glu Glu Arg Ile Ile Tyr Gly Val
    1055                1060                1065

Glu Asn Ser Ser Thr Phe Leu Glu Cys Ser Pro Lys Ser Gln Arg
    1070                1075                1080

Ala Leu Val Tyr Trp Gln Phe Gln Arg Arg Asn Glu Glu Arg Lys
    1085                1090                1095

Glu Glu Ile Arg Val Asp Asp His Ile Ile Arg Thr Asp Gln Gly
    1100                1105                1110

Leu Leu Leu Arg Ser Leu Gln Gln Lys Asp Ser Gly Asn Tyr Leu
    1115                1120                1125

Cys His Ala Val Glu His Gly Phe Ile Gln Thr Leu Leu Lys Val
    1130                1135                1140

Thr Leu Glu Val Ile Asp Thr Glu His Leu Glu Glu Leu Leu His
    1145                1150                1155

Lys Asp Asp Asp Gly Asp Gly Ser Lys Thr Lys Glu Met Ser Asn
    1160                1165                1170

Ser Met Thr Pro Ser Gln Lys Val Trp Tyr Arg Asp Phe Met Gln

1175                    1180                    1185

Leu Ile  Asn His Pro Asn Leu  Asn Thr Met Asp Glu  Phe Cys Glu
    1190                    1195                    1200

Gln Val  Trp Lys Arg Asp Arg  Lys Gln Arg Arg Gln  Arg Pro Gly
    1205                    1210                    1215

His Thr  Pro Gly Asn Ser Asn  Lys Trp Lys His Leu  Gln Glu Asn
    1220                    1225                    1230

Lys Lys  Gly Arg Asn Arg Arg  Thr His Glu Phe Glu  Arg Ala Pro
    1235                    1240                    1245

Arg Ser  Val
    1250

## Description

### Technical Field

The present invention relates to a cell that produces a human antibody. More specifically, the present invention relates to a chicken B cell that produces a human antibody.

### Background Art

An antibody is useful for performing identification of biological substances, the function analysis thereof, and the like, and it also plays an important role in the treatment of diseases. Such an antibody binds to a specific antigen in a living body and provokes various *in vivo* protective immune reactions, such as antibody-dependent cell-mediated cytotoxicity (ADCC) or complement-dependent cytotoxicity (CDC), so that the antibody recognizes a "foreign substance" to a living body and removes it. Paying attention on the ability of such an antibody, in recent years, antibody drugs have been vigorously developed. In order to treat a wide variety of diseases, it is necessary to promptly and simply supply antibodies that recognize various target antigens.

To date, as a means for preparing an antibody group comprising desired physical and physiological characteristics, a technique of producing a monoclonal antibody has been used. In a case where such a monoclonal antibody is produced, a hybridoma method comprising fusing B cells generated as a result of *in vivo* immunity with myelomas has been generally applied. However, this method has many problems in that, for example, immunological tolerance is produced due to the use of *in vivo* immunity, and it takes time and effort to finally obtain a desired antibody.

As a technique of overcoming the problem regarding immunological tolerance, a method that does not utilize *in vivo* immunity has been developed. This method comprises embedding a single chain valuable fragment (scFv) gene consisting of the variable regions of various antibodies into a phage particle, then allowing the phage to present a single chain antibody gene product on the surface thereof, and then obtaining a clone having an affinity for an antigen of interest from a single chain antibody library displayed by this phage. This method is referred to as a phage display method. This technique is excellent in terms of avoidance of immunological tolerance. However, since the clone obtained from the phage library is a single chain clone, it is necessary to prepare a complete antibody consisting of two chains according to a recombinant DNA technique or the like. Moreover, a change may be generated in affinity during the process of converting a single chain antibody to a complete antibody, and thus, there may be a considerable number of cases where a variable region sequence needs to be adjusted. Accordingly, in terms of time and effort, it cannot be said that the phage display method has been significantly improved in comparison to the method involving *in vivo* immunity.

As methods for overcoming the problem regarding the aforementioned existing method for producing a monoclonal antibody, an ADLib system (Patent Literature 1 and Non Patent Literature 1) and a method of further modifying such an ADLib system (Patent Literature 2) have been reported. The ADLib system is a technique capable of simply preparing a variety of antibodies with desired binding properties to all types of antigens. This method is a technique of selectively obtaining a desired antibody from an antibody library constructed from a chicken B cell-derived cell line DT40, in which antibody genes have become diversified autonomously. The ADLib system is superior to prior arts, in that it enables the avoidance of immunological tolerance, which is an advantage of an *in vitro* system antibody production technique, and in that a complete IgM antibody can be promptly obtained.

In general, in a case where an antibody is administered as a pharmaceutical product to an animal or a human, the antibody is required to be compatible with the type of the animal species, while considering minimization of immunogenicity *in vivo.* Hence, utilizing a DNA recombination technique, a chimeric antibody has been produced by replacing the constant region of a monoclonal antibody produced in mice or the like with a humanized constant region, and a humanized antibody has also been produced by transplanting the complementarity determining region (CDR) of the antibody into a human antibody. However, there may be a case where a change would occur in affinity or function in the process of such chimerization or humanization therefore it cannot be said that the probability of producing a humanized antibody that retains the original functions is high. Moreover, such a chimeric antibody or a humanized antibody only has a small region derived from other animal species therefore its complementarity determining region has immunogenicity, although its immunogenicity is lower than that of the original monoclonal antibody. Hence, such a chimeric antibody or a humanized antibody has been problematic in terms of the appearance of anti-antibody.

Thus, it has been attempted to produce an animal generating a human antibody by introducing a human antibody gene into a mouse. In Patent Literature 3, a microcell of a chromosomal fragment comprising a human antibody gene has been transplanted into mouse ES cells, and then, a chimeric mouse has been produced from the ES cells, thereby producing a chimeric mouse that expresses a human antibody. Moreover, in Non Patent Literature 2, genomic DNA in a mouse antibody gene locus has been replaced with the corresponding genomic DNA in a human antibody gene locus, so as to produce a chimeric mouse that expresses a human antibody. By both of the methods, it is possible to obtain an antibody having complementarity determining regions that are completely derived from human antibody genes. However, since *in vivo* immunity is utilized in both of the methods, the problems regarding time and effort required until a desired antibody is finally obtained have still remained.

Under the aforementioned circumstances, even in the case of using the ADLib system, a method of producing an antibody having an amino acid sequence derived from a human from chicken B cells is necessary to prepare an antibody drug. In addition, in order to treat various diseases, a technique of simply producing an antibody group having various antigen-recognizing properties is necessary.

Nevertheless, differing from a case where a human antibody gene is introduced into a mouse to "humanize" it, in a case where chicken B cells are "humanized," a difference in the system of diversifying an antibody becomes problematic. In the case of animals such as a chicken, a rabbit, a bovine or sheep, there is a region in which "pseudogenes" are gathered, upstream of an antibody variable region. The sequence of the respective pseudogenes are similar to that of antibody variable region and are not translated by themselves. All or a part of variable region sequences are rewritten by pseudogene sequences by a phenomenon referred to as gene conversion, so that a variety of sequences are generated. Diversification of antibodies occurs as a result of V(D)J recombination in animals such as a human or a mouse. In contrast, in animals such as a chicken, a rabbit, a bovine or sheep, such diversification of antibodies occurs by a mechanism referred to as gene conversion that is completely different from the recombination. Moreover, since the pseudogene region has a structure comprising an extremely large number of repeating sequences, it is significantly difficult to analyze the sequence of this region. Thus, even at the time point of March 2015, the pseudogene region of a chicken antibody heavy chain has not yet been analyzed. Due to such a difference in mechanisms or a problem regarding the structure of an antibody gene locus, it has been considered extremely difficult to construct a system of producing a desired human antibody from chicken B cells.

Under such circumstances, it has been reported that a cell line was produced by replacing a variable region and a pseudogene region present in the antibody gene locus of DT40 cells with human-derived sequences (Non Patent Literature 3). In this publication, a chicken antibody variable region has been replaced with a gene sequence comprising fluorescent proteins, namely, a green fluorescent protein (GFP) and a cyan fluorescent protein (CFP), and an attP sequence as a recombinase-recognizing site, and a DT40 cell, into which a human antibody variable region and the after-mentioned pseudogene sequence had been inserted utilizing the recombinase, has been then produced, and thereafter, the occurrence of gene conversion has been confirmed (Non Patent Literatures 3 and 4). However, since a chicken-derived constant region sequence has been used, the expressed antibody molecule is a human-chicken chimeric antibody, and thus, the immunogenicity of the antibody is extremely high. Furthermore, since the light chain is a fusion protein of a human-derived κ chain and a chicken-derived λ chain, the antibody molecule is largely different from a native human antibody. Hence, in order to produce an antibody used for the treatment of humans, it is necessary to further carry out a chimerization operation, and it is still likely that affinity or functions will be changed in the process of chimerization or humanization. Further, with regard to the sequence of the pseudogene region used herein, in the case of a light chain, a minimalist library that is a cluster of artificial sequences, in which one amino acid in each of the CDR sequences of the inserted human antibody variable region has been substituted with Tyr or Trp, and the sequences in which mutations have been introduced into the framework region of a variant of a naturally existing human antibody variable region, have been utilized. On the other hand, a heavy chain thereof is composed of an artificial sequence in which each of the CDR sequences of the inserted human antibody variable region has been substituted with Tyr or Trp, and mutant sequences thereof. These sequences have been designed for the purpose of allowing a small number of pseudogenes to correspond to a variety of antigens. A method of obtaining an antigen-specific antibody in the cells has not been reported.

### Citation List

### Patent Literature

Patent Literature 1: Japanese Patent No.4214234
Patent Literature 2: WO 2008/047480
Patent Literature 3: WO 97/07671

### Non Patent Literature

Non Patent Literature 1: Seo et al., Nature Biotech, Volume 23, pp. 731-735, 2005
Non Patent Literature 2: Recombinant Antibodies for Immunotherapy, pp. 100-108, 2009, Cambridge Press
Non Patent Literature 3: Schusser et al., PLOS ONE, Volume 8 issue 11 e80108, 2013
Non Patent Literature 4: Leighton et al., Frontiers in Immunology, Volume 6 Article 126, 2015

### Summary of Invention

### Technical Problem

Under the aforementioned circumstances, it is an object of the present invention to provide chicken B cells, each of which produces each of various human antibodies.

It is another object of the present invention to provide a method for producing antibodies from the chicken B cells.

### Solution to Problem

The present inventors have conducted intensive studies regarding production of cells that produce a variety of human antibodies. As a result, the inventors have inserted or replaced the genes in the variable region and constant region of the light chain and heavy chain of a human antibody into chicken B cell antibody light chain and heavy chain gene loci, so that they have succeeded in obtaining transformed cells that each express a human antibody. Moreover, the present inventors have further produced transformed cells, in which a plurality of human-derived antibody variable region sequences have been inserted as pseudogenes into the light chain and heavy chain, and thereafter, the inventors have treated these cells with an HDAC inhibitor, so that they have confirmed that gene conversion has occurred in the transformed cells, as in the case of the original chicken B cells. Furthermore, the present inventors have also succeeded in producing an antigen-specific human antibody from the transformed cells.

That is to say, the present inventors have confirmed that the aforementioned cells are prepared, and from each of the prepared cell, antibody in which the variable region has been modified in various ways can be obtained, and they have then succeeded in producing a variety of human antibodies, thereby completing the present invention.

Specifically, the present invention includes the following (1) to (21):
(1) A chicken B cell, in which, in an antibody light chain gene locus thereof, all or a part of a DNA sequence derived from a human antibody light chain variable region and a human antibody light chain constant region are inserted, or the antibody light chain gene locus is replaced with all or a part of a DNA sequence derived from a human antibody light chain variable region and a human antibody light chain constant region, and in an antibody heavy chain gene locus thereof, all or a part of a DNA sequence derived from a human antibody heavy chain variable region and a human antibody heavy chain constant region are inserted, or the antibody heavy chain gene locus is replaced with all or a part of a DNA sequence derived from a human antibody heavy chain variable region and a human antibody heavy chain constant region, and in an antibody light chain pseudogene locus thereof, two or more DNA sequences derived from human antibody light chain variable regions are inserted, or the antibody light chain pseudogene locus is replaced with two or more DNA sequences derived from human antibody light chain variable regions, and/or in an antibody heavy chain pseudogene locus thereof, two or more DNA sequences derived from human antibody heavy chain variable regions are inserted, or the antibody heavy chain pseudogene locus is replaced with two or more DNA sequences derived from human antibody heavy chain variable regions.
(2) The chicken B cell according to (1) above, wherein the number of the DNA sequences derived from the human antibody light chain variable regions to be inserted into the antibody light chain pseudogene locus, or to be replaced with the sequences in the antibody light chain pseudogene locus is 5 or more.
(3) The chicken B cell according to (1) above, wherein the number of the DNA sequences derived from the human antibody heavy chain variable regions to be inserted into the antibody heavy chain pseudogene locus, or to be replaced with the sequences in the antibody heavy chain pseudogene locus is 5 or more.
(4) The chicken B cell according to (1) above, wherein the number of the DNA sequences derived from the human antibody light chain variable regions to be inserted into the antibody light chain pseudogene locus, or to be replaced with the sequences in the antibody light chain pseudogene locus is 15 or more.
(5) The chicken B cell according to (1) above, wherein the number of the DNA sequences derived from the human antibody heavy chain variable regions to be inserted into the antibody heavy chain pseudogene locus, or to be replaced with the sequences in the antibody heavy chain pseudogene locus is 15 or more.
(6) The chicken B cell according to any one of (1) to (5) above, wherein the position of the DNA sequence derived from the human antibody light chain variable region inserted into the antibody light chain gene locus is upstream of the position of the DNA sequence derived from the human antibody light chain constant region, and the position of the DNA sequence derived from the human antibody heavy chain variable region inserted into the antibody heavy chain gene locus is upstream of the position of the DNA sequence derived from the human antibody heavy chain constant region.
(7) The chicken B cell according to (6) above, wherein the positions of the DNA sequence derived from the human antibody light chain variable region and the DNA sequence derived from the human antibody light chain constant region inserted into the antibody light chain gene locus are upstream of the positions of a chicken antibody light chain variable region and a constant region thereof, and the positions of the human antibody heavy chain variable region and constant region inserted into the antibody heavy chain gene locus are upstream of the positions of a chicken antibody heavy chain variable region and a constant region thereof.
(8) The chicken B cell according to any one of (1) to (7) above, wherein the human antibody heavy chain is a γ chain.
(9) The chicken B cell according to any one of (1) to (8) above, wherein the human antibody light chain is a γ chain or a κ chain.
(10) The chicken B cell according to any one of (1) to (9) above, which has an ability to express a human antibody on the cell surface thereof and also to secrete the human antibody into a culture solution.
(11) The chicken B cell according to any one of (1) to (10) above, which is a DT40 cell.
(12) The chicken B cell according to any one of (1) to (11), which has undergone to a treatment of relaxing chromatin.
(13) The chicken B cell according to (12) above, wherein the treatment of relaxing chromatin is reduction or deletion of the function of histone deacetylase in the chicken B cell.
(14) The chicken B cell according to (13) above, wherein the method for reducing or deleting the function of histone deacetylase is reduction or deletion of the expression of a histone deacetylase gene in the chicken B cell.
(15) The chicken B cell according to (14) above, wherein the histone deacetylase is HDAC2.
(16) The chicken B cell according to (13) above, wherein the method for reducing or deleting the function of histone deacetylase is a treatment with an HDAC inhibitor.
(17) An antibody-producing cell library consisting of the chicken B cells according to any one of (12) to (16) above.
(18) A method for producing an antibody from the chicken B cells according to any one of (1) to (16) above.
(19) An antibody obtained by the method according to (18) above.
(20) A kit for producing the chicken B cells according to any one of (1) to (16) above.
(21) A kit for producing an antibody by the method according to (18) above.

### Advantageous Effects of Invention

It becomes possible to simply and promptly obtain human antibodies that recognize a variety of antigens from the cells of the present invention.

### Brief Description of Drawings

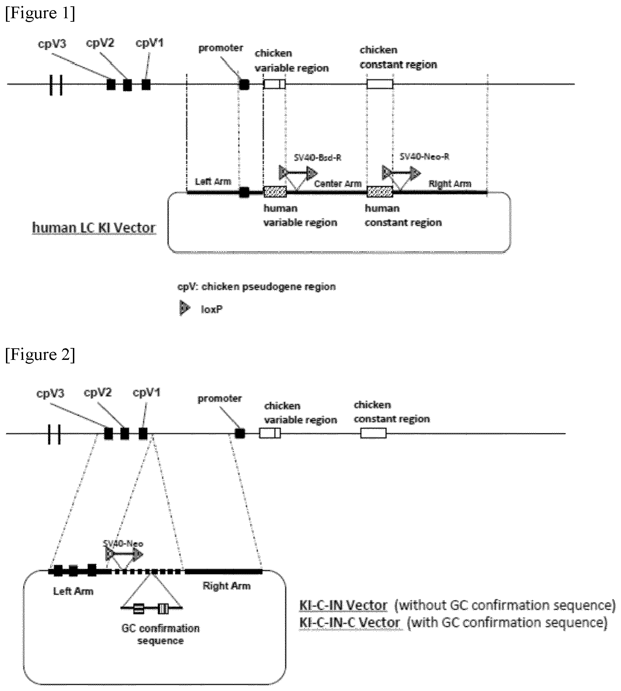

[Figure 1] Figure 1 is a view schematically showing the structure of a targeting vector for replacing the antibody light chain constant region and antibody light chain variable region of a DT40 cell with the light chain constant region and light chain variable region of a human-derived antibody (IgG). cpV1 to cpV3: chicken pseudogenes.
[Figure 2] Figure 2 is a view schematically showing the structure of a targeting vector for inserting a sequence for confirming gene conversion (GC confirmation sequence; a human pseudogene consisting of a sequence derived from a human antibody variable region, the same applied below) downstream of the antibody light chain pseudogene region of a DT40 cell. cpV1 to cpV3: chicken pseudogenes.

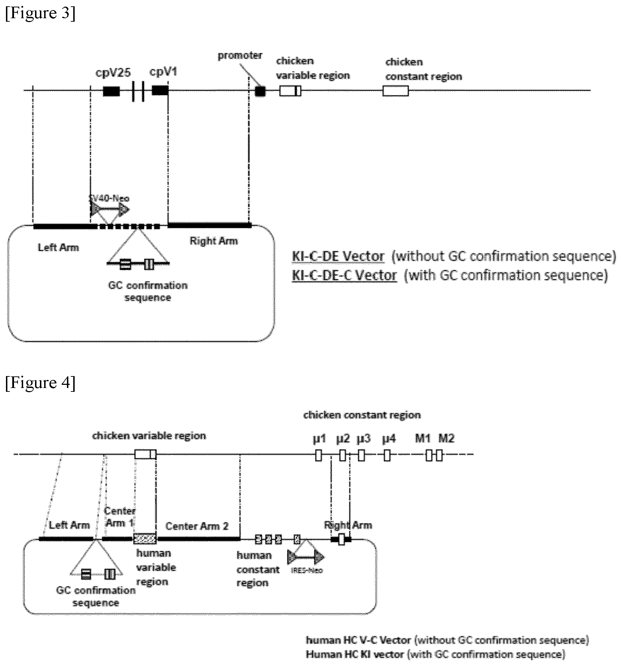

[Figure 3] Figure 3 is a view schematically showing the structure of a targeting vector for deleting the antibody light chain pseudogene region of a DT40 cell, and replacing the antibody light chain pseudogene region of the DT40 cell with a GC confirmation sequence. cpV25, cpV1: chicken pseudogenes.
[Figure 4] Figure 4 is a view schematically showing the structure of a targeting vector for replacing the antibody heavy chain constant region and antibody heavy chain variable region of a DT40 cell with the heavy chain constant region and heavy chain variable region of a human-derived antibody (IgG) and also for inserting a GC confirmation sequence therein.

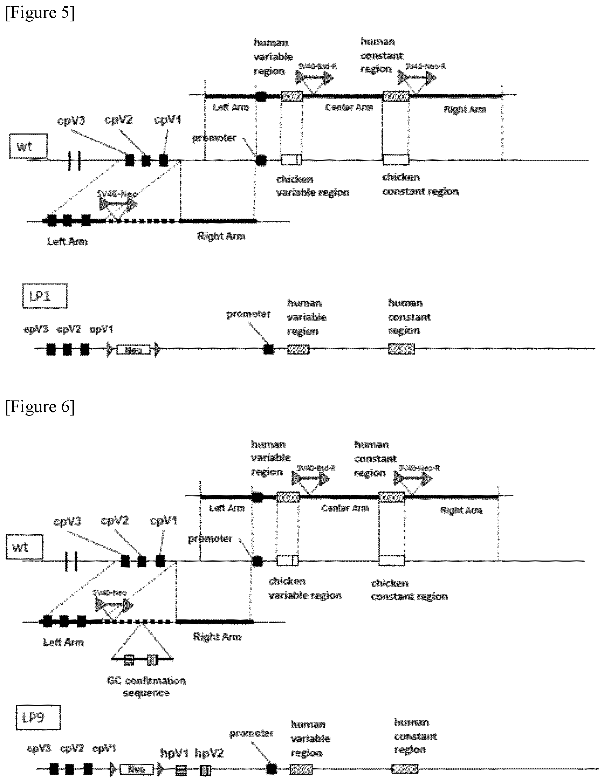

[Figure 5] Figure 5 is a view schematically showing the procedures for replacing the antibody light chain gene regions of a DT40 cell with the light chain variable region and light chain constant region of a human-derived antibody, and the thus obtained antibody light chain gene region of the DT40 cell. cpV1 to cpV3: chicken pseudogenes.
[Figure 6] Figure 6 is a view schematically showing the procedures for replacing the antibody light chain gene regions of a DT40 cell with human-derived antibody light chain variable region and light chain constant region genes, and further inserting a GC confirmation sequence downstream of the antibody light chain pseudogene region of the DT40 cell, and the obtained DT40 cell antibody light chain gene region. cpV1 to cpV3: chicken pseudogenes; and hpV1 and hpV2: human pseudogenes.

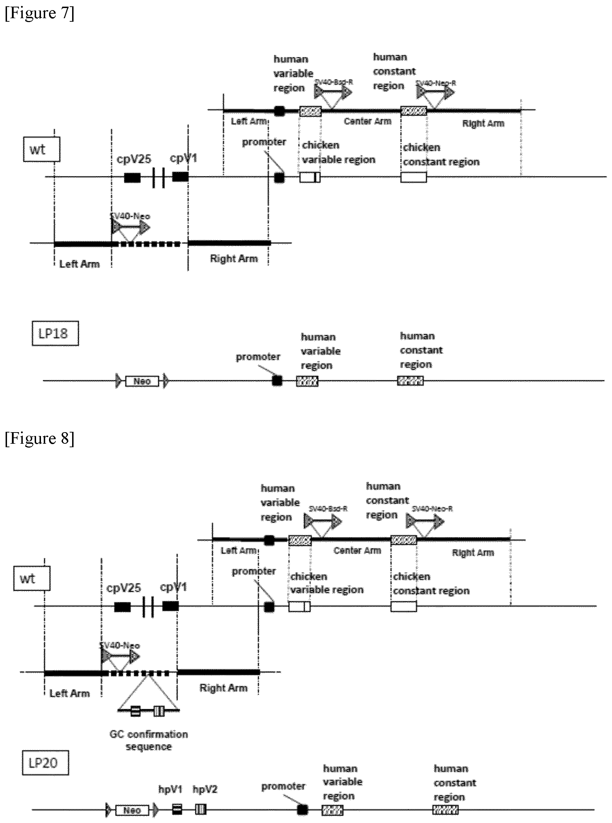

[Figure 7] Figure 7 is a view schematically showing the procedures for replacing the antibody light chain gene regions of a DT40 cell with human-derived antibody light chain variable region and light chain constant region genes, and further deleting the antibody light chain pseudogene region of the DT40 cell, and the thus obtained antibody light chain gene region of the DT40 cell. cpV25 and cpV1: chicken pseudogenes.
[Figure 8] Figure 8 is a view schematically showing the procedures for replacing the antibody light chain gene regions of a DT40 cell with human-derived antibody light chain variable region and light chain constant region genes, and further replacing the pseudogene region of the DT40 cell with a GC confirmation sequence, and the obtained DT40 cell antibody light chain gene region. cpV25 and cpV1: chicken pseudogenes; and hpV1 and hpV2: human pseudogenes.

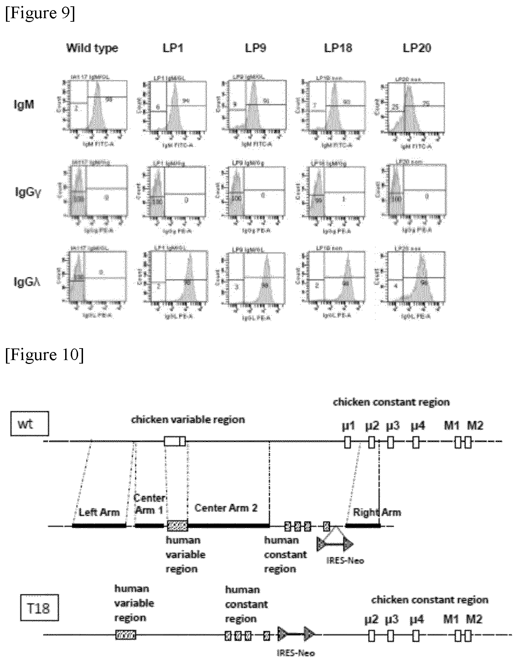

[Figure 9] Figure 9 shows the results obtained by analyzing by flow cytometry an antibody produced by a cell line in which a human-derived antibody light chain gene region has been inserted into the antibody light chain gene region of a DT40 cell, or a cell line in which the antibody light chain gene region of a DT40 cell has been replaced with a human antibody light chain gene.
[Figure 10] Figure 10 is a view schematically showing the procedures for converting the antibody heavy chain gene regions of a DT40 cell to the heavy chain variable region and heavy chain constant region of a human-derived antibody, and the thus obtained antibody heavy chain gene region of the DT40 cell.

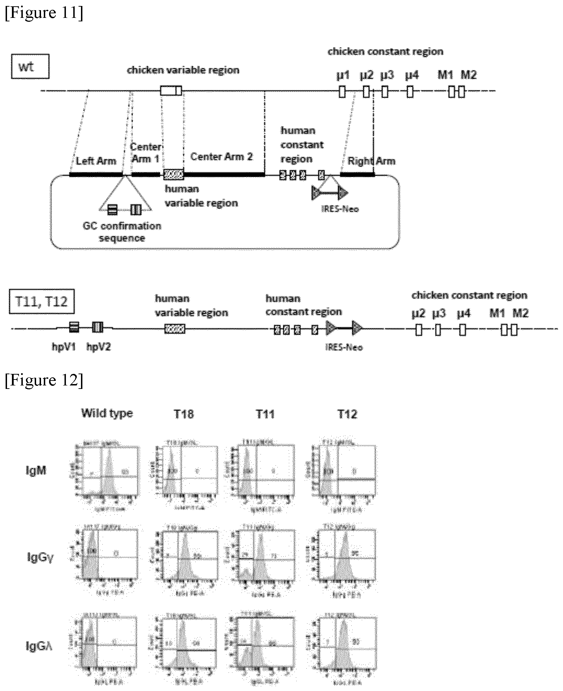

[Figure 11] Figure 11 is a view schematically showing the procedures for converting the antibody heavy chain gene regions of a DT40 cell to the heavy chain variable region and heavy chain constant region gene of a human-derived antibody, and further inserting a GC confirmation sequence downstream of the heavy chain pseudogene region of the DT40 cell, the thus obtained antibody light chain gene region of the DT40 cell, and the structure of the used targeting vector. hpV1 and hpV2: human pseudogenes.
[Figure 12] Figure 12 shows the results obtained by analyzing by flow cytometry an antibody produced by a cell line, in which the antibody heavy chain gene regions of a DT40 cell have been replaced with human-derived antibody heavy chain variable region and heavy chain constant region genes, and further, a GC confirmation sequence has been inserted downstream of the heavy chain pseudogene region of the DT40 cell.

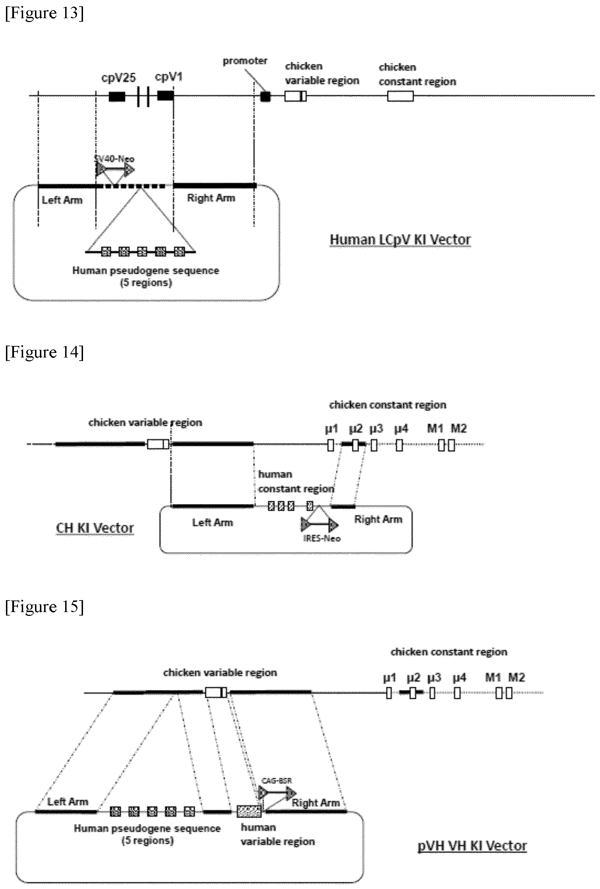

[Figure 13] Figure 13 is a view schematically showing the procedures for replacing the antibody light chain pseudogene region of a DT40 cell with a pseudogene consisting of a sequence derived from a human Igγ variable region (human light chain pseudogene), the thus obtained antibody light chain region of the DT40 cell, and the structure of the used targeting vector.
[Figure 14] Figure 14 is a view schematically showing the structure of a targeting vector for replacing the antibody heavy chain constant region of a DT40 cell with the heavy chain gene region of human IgG₁.
[Figure 15] Figure 15 is a view schematically showing the structure of a targeting vector for introducing the heavy chain variable region of a human-derived antibody and five pseudogenes consisting of human Igγ variable region-derived sequences (human heavy chain pseudogenes) into the antibody heavy chain variable region of a DT40 cell.

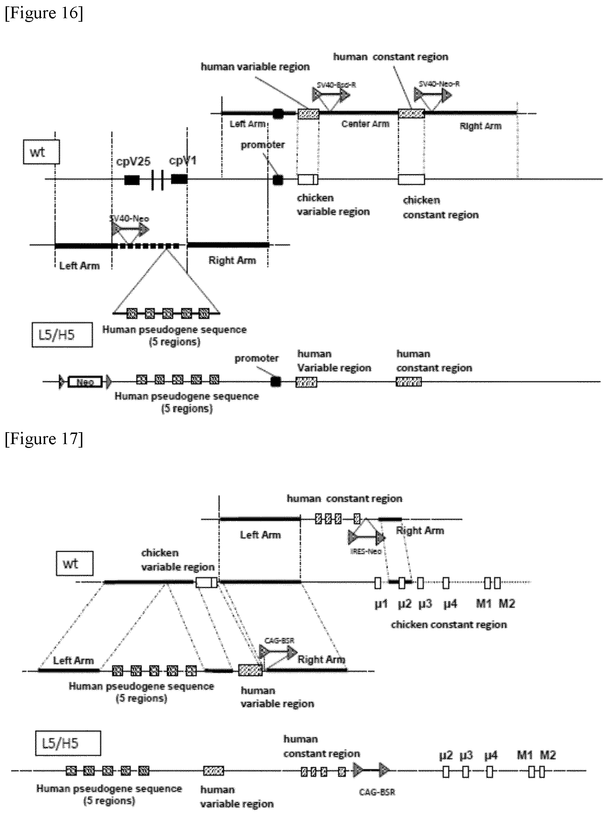

[Figure 16] Figure 16 is a view schematically showing the procedures for replacing the antibody light chain gene regions of a DT40 cell with the light chain variable region and light chain constant region of a human-derived antibody, and then inserting human light chain pseudogene sequences (five sequences) therein, and the thus obtained antibody light chain gene region of the DT40 cell. cpV25 and cpV1: chicken pseudogenes.
[Figure 17] Figure 17 is a view schematically showing the procedures for introducing the heavy chain variable region and heavy chain constant region of a human-derived antibody into the antibody heavy chain gene regions of a DT40 cell, and then inserting human heavy chain pseudogene sequences (five sequences) therein, and the thus obtained antibody heavy chain gene region of the DT40 cell.

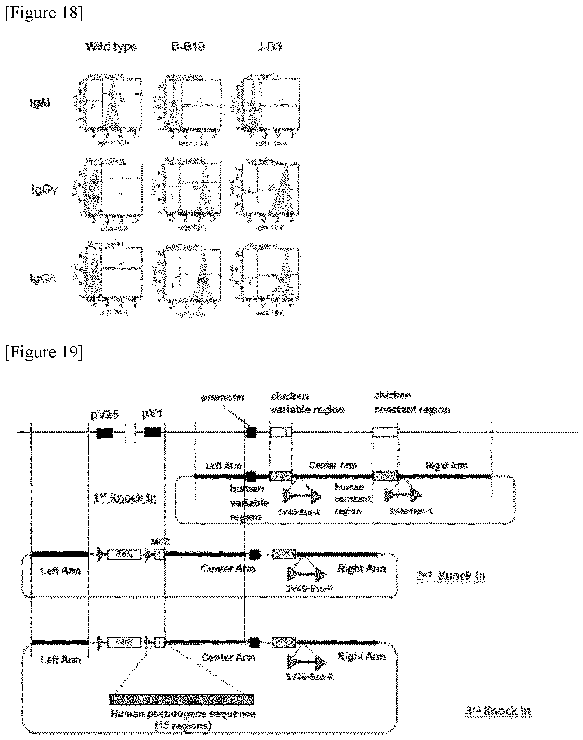

[Figure 18] Figure 18 shows the results obtained by analyzing by flow cytometry an antibody produced by a cell line, in which the antibody light chain gene region and antibody heavy chain gene region of a DT40 cell have been replaced with human-derived antibody genes, and human light chain pseudogene sequences (five sequences) have been then inserted therein.
[Figure 19] Figure 19 is a view schematically showing the procedures for replacing the antibody light chain gene regions of a DT40 cell with the light chain variable region and light chain constant region of a human-derived antibody, and then inserting human heavy chain pseudogene sequences (15 sequences) therein, and the structure of the used targeting vector. pV25 and pV1: chicken pseudogenes.

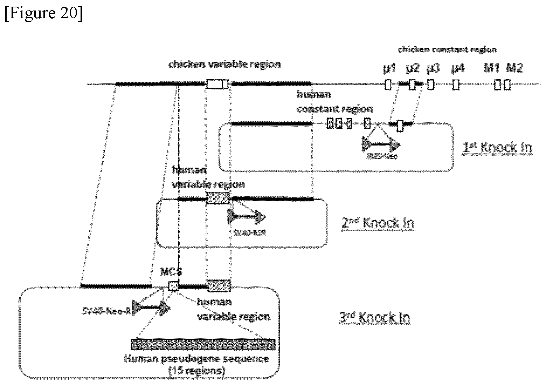

[Figure 20] Figure 20 is a view schematically showing the procedures for introducing the heavy chain variable region and heavy chain constant region of a human-derived antibody into the antibody heavy chain gene regions of a DT40 cell, and then inserting human heavy chain pseudogene sequences (15 sequences) therein, and the structure of the used targeting vector.

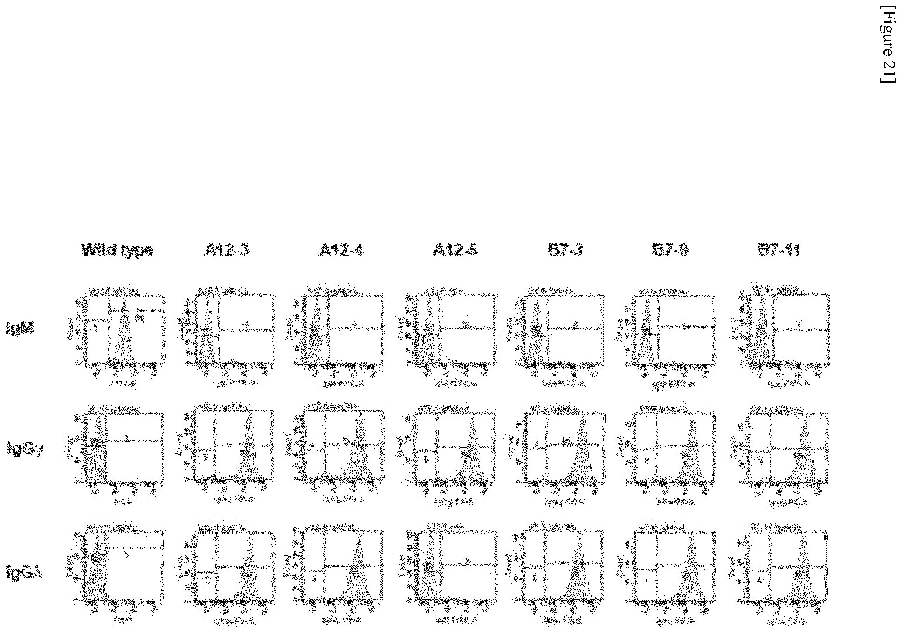

[Figure 21] Figure 21 shows the results obtained by analyzing by flow cytometry an antibody produced by an antibody produced by an L15/H15 cell line, in which the antibody light chain gene region and antibody heavy chain gene region of a DT40 cell have been replaced with the gene regions of a human-derived antibody, and human light chain pseudogene sequences (15 sequences) have been then inserted therein.

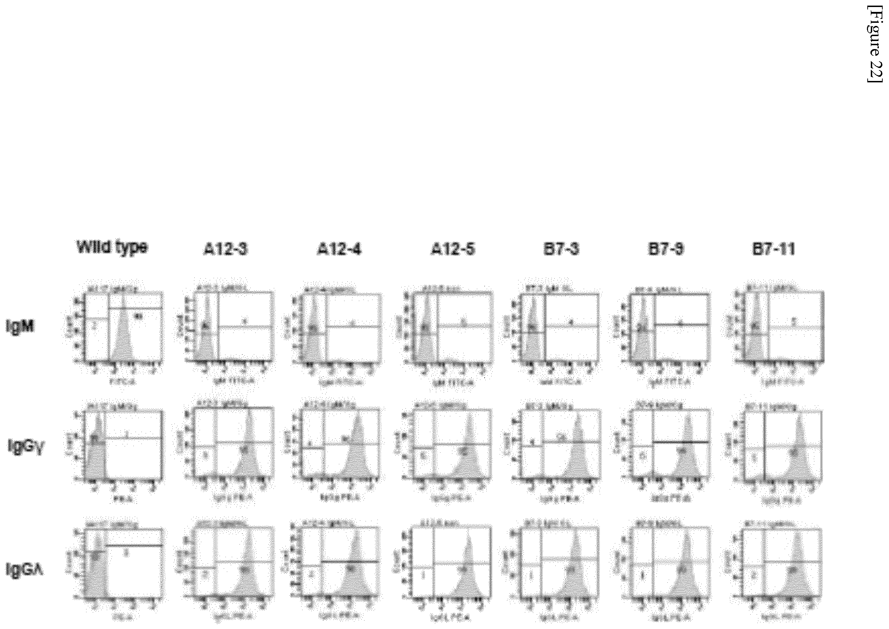

[Figure 22] Figure 22 shows the results shown in Figure 21, in which incorrect data have been corrected based on the data before the priority date.

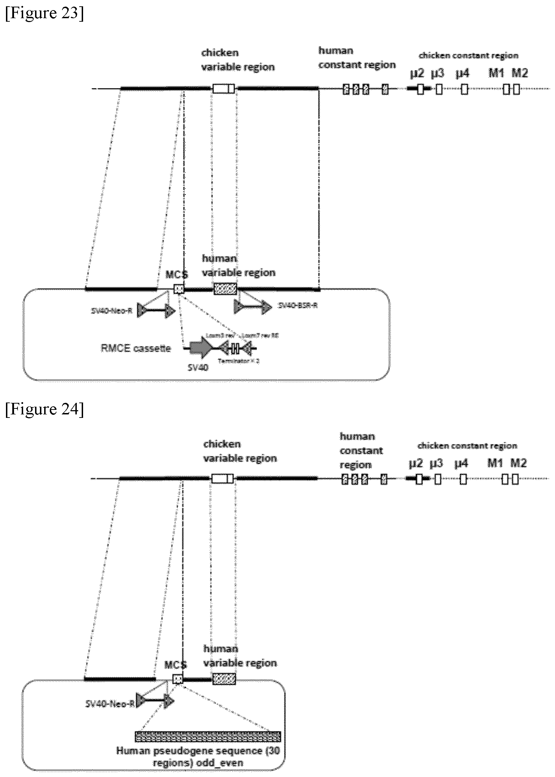

[Figure 23] Figure 23 is a view schematically showing the procedures for replacing the antibody heavy chain variable region of a DT40 cell with the heavy chain variable region of a human-derived antibody, and at the same time, inserting a cassette sequence for RMCE into the antibody heavy chain variable region of the DT40 cell, and the structure of the used targeting vector.
[Figure 24] Figure 24 is a view schematically showing the procedures for inserting 30 human heavy chain pseudogene sequences into the antibody heavy chain gene region of a DT40 cell, and the structure of the used targeting vector.

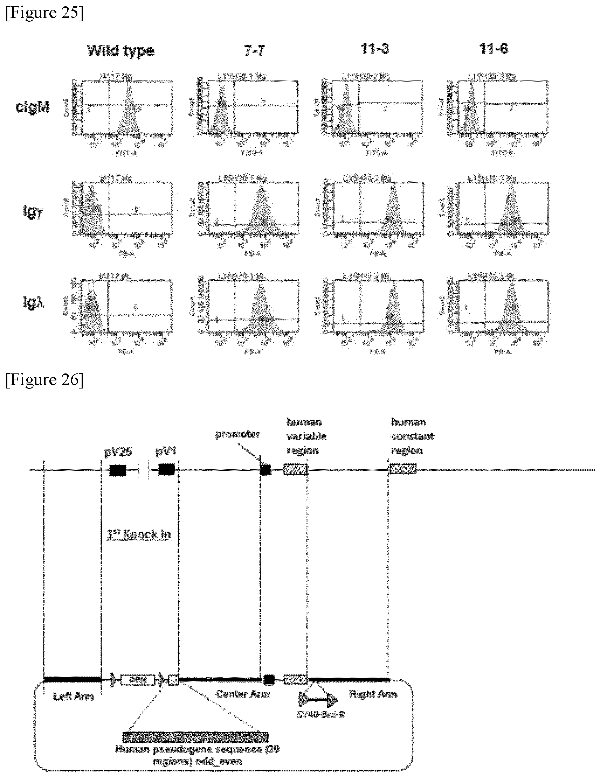

[Figure 25] Figure 25 shows the results obtained by analyzing by flow cytometry an antibody produced by an L15/H30 cell line, in which the antibody light chain gene region and antibody heavy chain gene region of a DT40 cell have been replaced with the gene regions of a human-derived antibody, and thereafter, 15 human light chain pseudogene sequences have been inserted into the light chain and 30 human heavy chain pseudogene sequences have been inserted into the heavy chain.
[Figure 26] Figure 26 is a view schematically showing the procedures for inserting 30 human light chain pseudogene sequences into the antibody light chain gene region of a DT40 cell, and the structure of the used targeting vector.

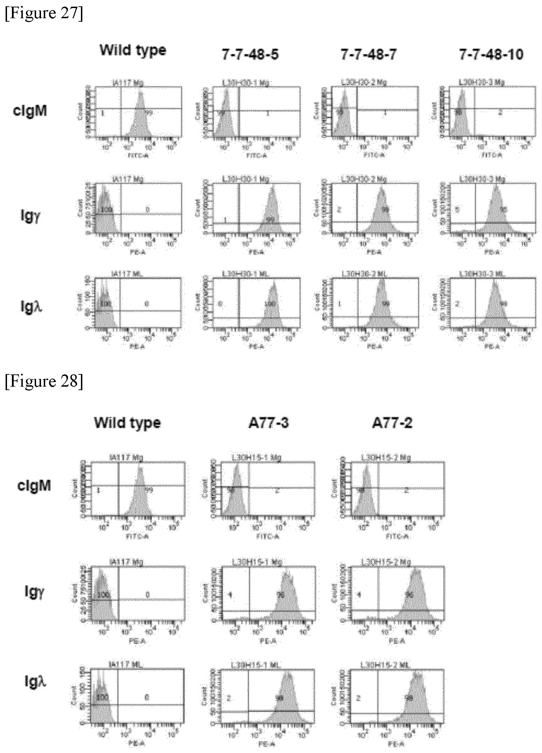

[Figure 27] Figure 27 shows the results obtained by analyzing by flow cytometry an antibody produced by an L30/H30 cell line, in which the antibody light chain gene region and antibody heavy chain gene region of a DT40 cell have been replaced with the gene regions of a human-derived antibody, and thereafter, 30 human light chain pseudogene sequences have been inserted into the light chain and 30 human heavy chain pseudogene sequences have been inserted into the heavy chain.
[Figure 28] Figure 28 shows the results obtained by analyzing by flow cytometry an antibody produced by an L30/H15 cell line, in which the antibody light chain gene region and antibody heavy chain gene region of a DT40 cell have been replaced with the gene regions of a human-derived antibody, and thereafter, 30 human light chain pseudogene sequences have been inserted into the light chain and 15 human heavy chain pseudogene sequences have been inserted into the heavy chain.

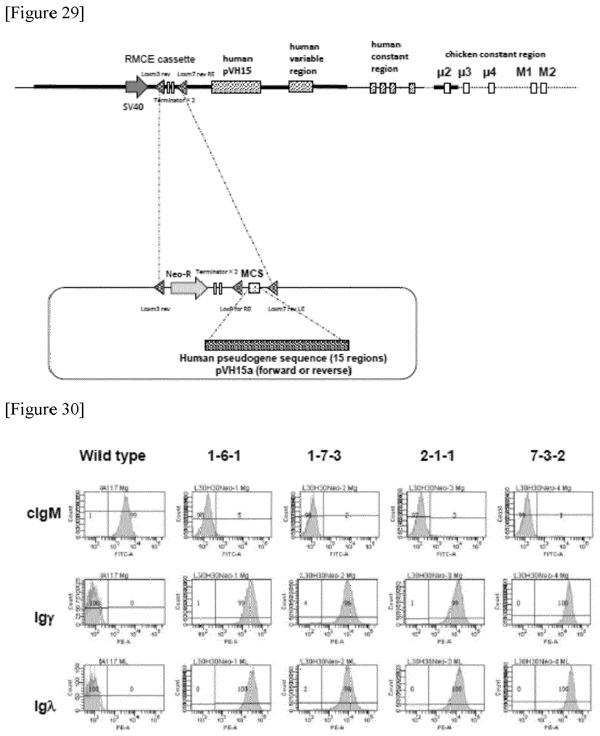

[Figure 29] Figure 29 is a view schematically showing the procedures for additionally inserting 15 human heavy chain pseudogene sequences into the antibody heavy chain gene region of the L30/H15 cell line in the forward or reverse direction according to an RMCE method, so as to have 30 human pseudogene sequences in total, and the structure of the used targeting vector. In the figure, the symbols "for" and "rev" indicate the forward direction and the reverse direction, respectively. The same is applied to other figures below.
[Figure 30] Figure 30 shows the results obtained by analyzing by flow cytometry an antibody produced by a cell line, in which 15 human heavy chain pseudogene sequences have been additionally inserted into the L30/H15 cell line in the forward or reverse direction according to the RMCE method (which is an L30/H15f15f cell line in the case of the forward direction, and an L30/H15r15f cell line in the case of the reverse direction).

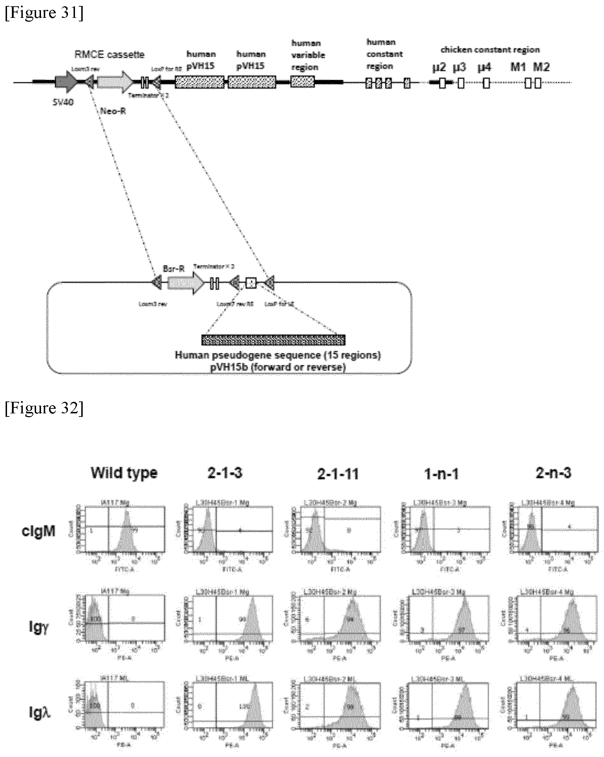

[Figure 31] Figure 31 is a view schematically showing the procedures for additionally inserting 15 human heavy chain pseudogene sequences to each of the antibody heavy chain gene regions of the L30/H15f15f cell line and the L30/H15r15f cell line in the forward or reverse direction, so as to have 45 human pseudogene sequences in total, and the structure of the used targeting vector.
[Figure 32] Figure 32 shows the results obtained by analyzing by flow cytometry an antibody produced by a cell line (L30/H15f15f15f cell line), in which 15 human heavy chain pseudogene sequences have been additionally inserted into the antibody heavy chain gene region of the L30/H15f15f cell line in the forward direction.

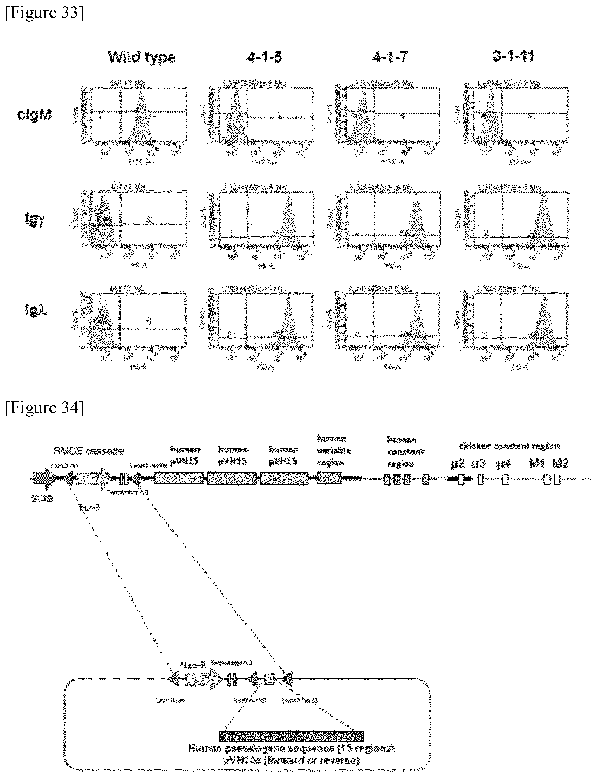

[Figure 33] Figure 33 shows the results obtained by analyzing by flow cytometry an antibody produced by a cell line (L30/H15r15r15f cell line), in which 15 human heavy chain pseudogene sequences have been additionally inserted into the antibody heavy chain gene region of the L30/H15r15f cell line in the reverse direction.
[Figure 34] Figure 34 is a view schematically showing the procedures for additionally inserting 15 human heavy chain pseudogene sequences into each of the antibody heavy chain gene regions of the L30/H15f15f15f cell line and the L30/H15r15r15f cell line in the forward or reverse direction according to the RMCE method, so as to have 60 human pseudogene sequences in total, and the structure of the used targeting vector.

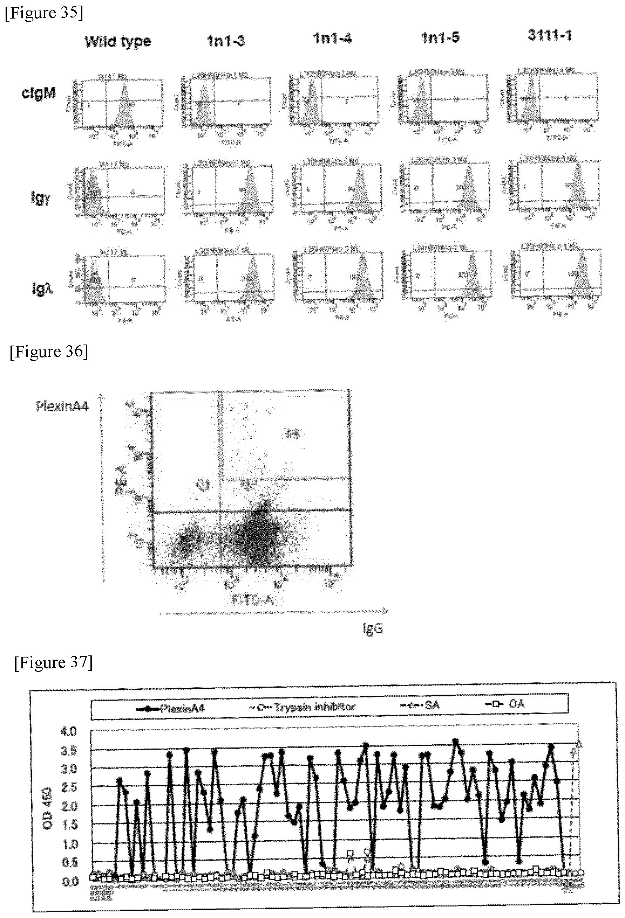

[Figure 35] Figure 35 shows the results obtained by analyzing by flow cytometry an antibody produced by a cell line, in which 30 human light chain pseudogene sequences have been inserted into the antibody light chain gene region of a DT40 cell, and 60 human heavy chain pseudogene sequences have been inserted into the antibody heavy chain gene region thereof.
[Figure 36] Figure 36 shows the results obtained by separating a cell population binding to a Plexin A4 protein from a TSA-treated L15/H15 cell line using an ADLib system, and then analyzing the cell population by flow cytometry.
[Figure 37] Figure 37 shows the results obtained by performing single cell sorting on a cell population in the P5 area of Figure 36, plating the obtained cells on a 96-well plate to culture them, and carrying out ELISA analysis on the cell supernatant in each well. The horizontal axis indicates the number of each well fraction of the 96-well plate, from which the cell supernatant used as a measurement sample is derived. Trypsin inhibitor: trypsin inhibitor; SA: streptavidin; OA: ovalbumin.

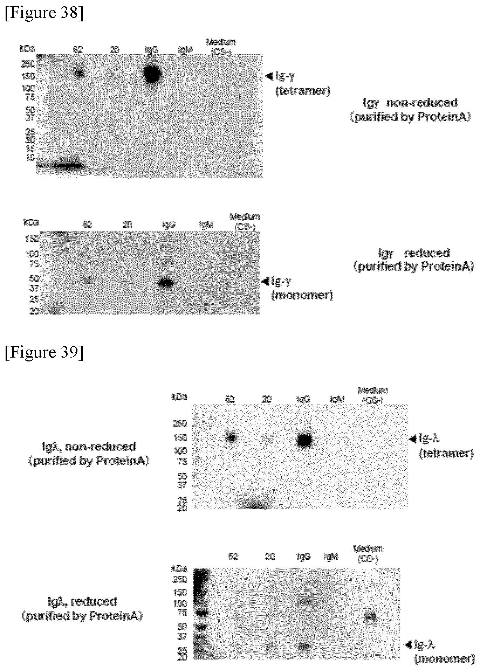

[Figure 38] Figure 38 shows the results obtained by analyzing antibodies secreted from positive clones #62 and #20 according to Western blotting. The samples were treated under reduction and non-reduction conditions, and Western blotting was then carried out on the samples, using a goat anti-human IgGy antibody.
[Figure 39] Figure 39 shows the results obtained by analyzing antibodies secreted from positive clones #62 and #20 according to Western blotting. The samples were treated under reduction and non-reduction conditions, and Western blotting was then carried out on the samples, using a goat anti-human IgGy antibody.

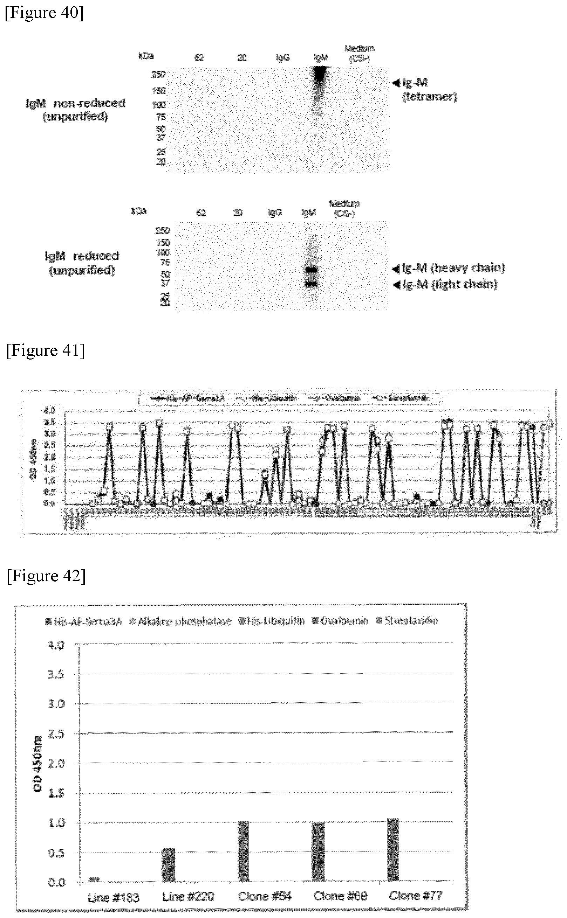

[Figure 40] Figure 40 shows the results obtained by analyzing antibodies secreted from positive clones #62 and #20 according to Western blotting. The samples were treated under reduction and non-reduction conditions, and Western blotting was then carried out on the samples, using a goat anti-chicken IgM antibody.
[Figure 41] Figure 41 shows the results of a primary screening of a cell population binding to His-AP-hSema3A from the TSA-treated L15/H15 cell line, which has been carried out using an ADLib system.
[Figure 42] Figure 42 shows the results of a secondary screening, which has been carried out after the monocloning of the cell population binding to His-AP-hSema3A.

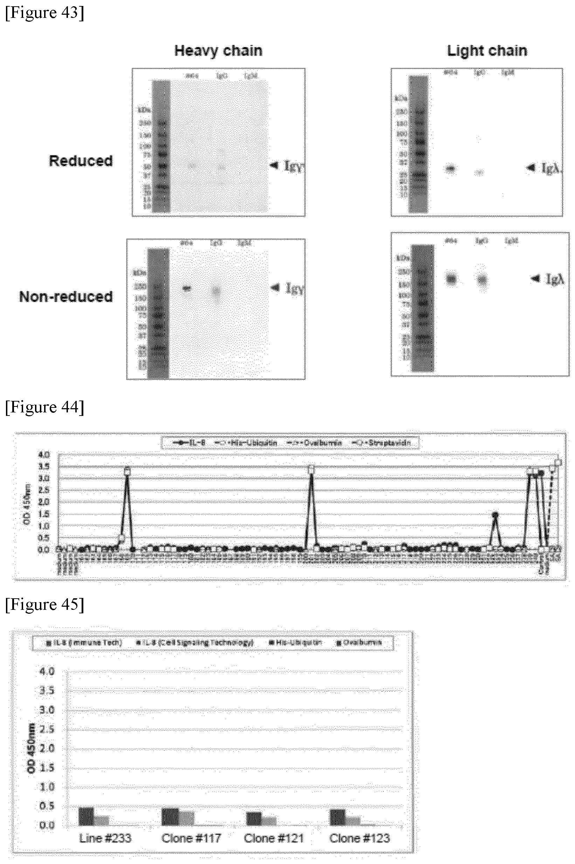

[Figure 43] Figure 43 shows the results obtained by analyzing by Western blotting a His-AP-hSema3A antibody secreted from the positive clones after completion of the secondary screening. The samples were treated under reduction and non-reduction conditions, and Western blotting was then carried out on the samples, using a goat anti-human IgGy antibody or a goat anti-human IgGλ antibody.
[Figure 44] Figure 44 shows the results of a primary screening of a cell population binding to IL-8 from a TSA-treated L30/H45 cell line, which has been carried out using an ADLib system.
[Figure 45] Figure 45 shows the results of a secondary screening, which has been carried out after the monocloning of the cell population binding to IL-8.

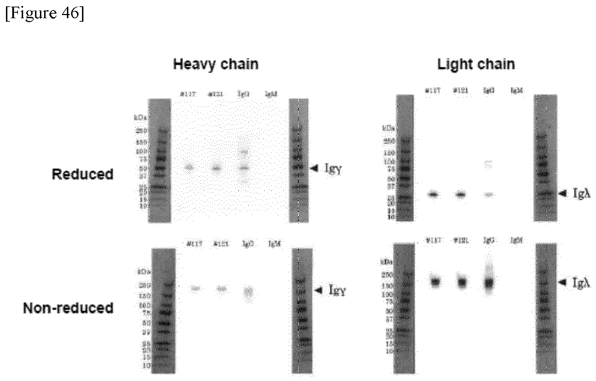

[Figure 46] Figure 46 shows the results obtained by analyzing by Western blotting an IL-8 antibody secreted from the positive clones after completion of the secondary screening. The samples were treated under reduction and non-reduction conditions, and Western blotting was then carried out on the samples, using a goat anti-human IgGy antibody or a goat anti-human IgGλ antibody.

### Description of Embodiments

One embodiment of the present invention is a chicken B cell, in which, in an antibody light chain gene locus thereof, all or a part of a DNA sequence derived from a human antibody light chain variable region and a human antibody light chain constant region are inserted, or the antibody light chain gene locus is replaced with all or a part of a DNA sequence derived from a human antibody light chain variable region and a human antibody light chain constant region, and in an antibody heavy chain gene locus thereof, all or a part of a DNA sequence derived from a human antibody heavy chain variable region and a human antibody heavy chain constant region are inserted, or the antibody heavy chain gene locus is replaced with all or a part of a DNA sequence derived from a human antibody heavy chain variable region and a human antibody heavy chain constant region, and in an antibody light chain pseudogene locus thereof, two or more DNA sequences derived from human antibody light chain variable regions are inserted, or the antibody light chain pseudogene locus is replaced with two or more DNA sequences derived from human antibody light chain variable regions, and/or in an antibody heavy chain pseudogene locus thereof, two or more DNA sequences derived from human antibody heavy chain variable regions are inserted, or the antibody heavy chain pseudogene locus is replaced with two or more DNA sequences derived from human antibody heavy chain variable regions.

The B cell of the present invention is an antibody-producing B cell derived from a chicken, and an example of the B cell of the present invention can be a DT 40 cell from a chicken. Other than such DT40 cells, B cells from a bovine, sheep, a rabbit and the like, which have a system for diversifying antibody sequences according to gene conversion, can also be used.

Moreover, in addition to the aforementioned configuration such that, in an antibody light chain gene locus thereof, all or a part of a DNA sequence derived from a human antibody light chain variable region and a human antibody light chain constant region are inserted, or the antibody light chain gene locus is replaced with all or a part of a DNA sequence derived from a human antibody light chain variable region and a human antibody light chain constant region, and in an antibody heavy chain gene locus thereof, all or a part of a DNA sequence derived from a human antibody heavy chain variable region and a human antibody heavy chain constant region are inserted, or the antibody heavy chain gene locus is replaced with all or a part of a DNA sequence derived from a human antibody heavy chain variable region and a human antibody heavy chain constant region, and in an antibody light chain pseudogene locus thereof, two or more DNA sequences derived from human antibody light chain variable regions are inserted, or the antibody light chain pseudogene locus is replaced with two or more DNA sequences derived from human antibody light chain variable regions, and/or in an antibody heavy chain pseudogene locus thereof, two or more DNA sequences derived from human antibody heavy chain variable regions are inserted, or the antibody heavy chain pseudogene locus is replaced with two or more DNA sequences derived from human antibody heavy chain variable regions, the chicken B cell of the present invention may also comprise mutations or modifications in other gene regions and the like.

The term "antibody light chain gene locus" is used herein to mean a gene locus in which a gene encoding the light chain variable region and light chain constant region of an antibody is present, and the term "antibody heavy chain gene locus" is a gene locus in which a gene encoding the heavy chain variable region and heavy chain constant region of an antibody is present.

In addition, the term "pseudogene" is used herein to mean a DNA sequence that is similar to a functional gene but does not function as an expressing gene. The chicken antibody light chain pseudogene locus and the chicken heavy chain pseudogene locus are each located upstream of the antibody light chain gene locus and heavy chain gene locus, in which the functional gene is present, and contribute to generate the diversity as a result of gene conversion. A pseudogene is not present in an antibody gene locus in human genome, but in the present description, a DNA sequence having a sequence similar to a human antibody variable region, which has been introduced into a chicken antibody gene locus for the purpose of causing gene conversion with the inserted human antibody variable region, is collectively referred to as a "human pseudogene."

A DNA sequence derived from a human antibody light chain variable region and a human antibody light chain constant region, which have been inserted into a chicken antibody light chain gene locus or have been replaced with a sequence in the chicken antibody light chain gene locus, may be all of the sequence of the human antibody light chain variable region and the human antibody light chain constant region, or may also be a part of the sequence. Moreover, the position of the DNA sequence derived from the human antibody light chain variable region inserted into the chicken antibody light chain gene locus is desirably upstream of a position, into which the DNA sequence derived from the human antibody light chain constant region has been inserted.

Likewise, a DNA sequence derived from a human antibody heavy chain variable region and a human antibody heavy chain constant region, which have been inserted into a chicken antibody heavy chain gene locus or have been replaced with a sequence in the chicken antibody heavy chain gene locus, may be all of the sequence of the human heavy chain variable region and the human heavy chain constant region, or may also be a part of the sequence. Moreover, the position of the DNA sequence derived from the human antibody heavy chain variable region inserted into the chicken antibody heavy chain gene locus is desirably upstream of a position, into which the DNA sequence derived from the human antibody heavy chain constant region has been inserted.

Furthermore, the positions of the DNA sequences derived from the human antibody heavy chain variable region and the human antibody constant region inserted into the chicken antibody light chain gene locus are desirably upstream of the positions of DNA sequence derived from a chicken antibody light chain variable region and a chicken antibody light chain constant region. Likewise, the positions of the DNA sequences derived from the human antibody heavy chain variable region and the human antibody heavy chain constant region inserted into the chicken antibody heavy chain gene locus are desirably upstream of the positions of the chicken antibody heavy chain variable region and the chicken antibody heavy chain constant region.

In the present embodiment, the DNA sequences derived from the human antibody light chain variable region, which have been inserted into the chicken antibody light chain pseudogene locus or have been replaced with the DNA sequences in the chicken antibody light chain pseudogene locus (which are also referred to as "human light chain pseudogene sequences" in the present description and drawings) can be the sequences of known human antibody light chain variable regions. Such sequence information can also be obtained from websites such as V Base (http://www2.mrc-lmb.cam.ac.uk/vbase/list2.php). As such human light chain pseudogene sequences, DNA sequences comprising all or a part of the CDR1, CDR2 and CDR3 of the human antibody light chain variable region can be used. It is desirable that each CDR be not identical to the DNA sequence of the human antibody light chain variable region that has been inserted into the chicken antibody light chain gene locus or has been replaced with the sequence in the chicken antibody light chain gene locus, or to the CDRs of other human light chain pseudogene sequences. Moreover, it is desirable that the framework (FW) region of the human light chain pseudogene sequence be identical to that of the human antibody light chain variable region that has been inserted into the chicken antibody light chain gene locus or has been replaced with the sequence in the chicken antibody light chain gene locus. For instance, when the DNA sequence of SEQ ID NO: 5 is selected as an antibody light chain variable region, the sequences shown in SEQ ID NO: 69 to SEQ ID NO: 88 and SEQ ID NO: 127 to SEQ ID NO: 141 can be used in combination as a light chain pseudogene.

Likewise, the DNA sequences derived from the human antibody heavy chain variable regions, which have been inserted into the chicken antibody heavy chain pseudogene locus or have been replaced with the DNA sequences in the chicken antibody heavy chain pseudogene locus (which are also referred to as "human heavy chain pseudogene sequences" in the present description and drawings) can be the sequences of known human antibody heavy chain variable regions. Such sequence information can also be obtained from websites such as V Base (http://www2.mrc-lmb.cam.ac.uk/vbase/list2.php). As such human heavy chain pseudogene sequences, DNA sequences comprising all or a part of the CDR1, CDR2 and CDR3 of the human antibody heavy chain variable regions can be used. It is desirable that each CDR be not identical to the DNA sequence of the human antibody heavy chain variable region that has been inserted into the chicken antibody heavy chain gene locus or has been replaced with the sequence in the chicken antibody heavy chain gene locus, or to the CDRs of other human heavy chain pseudogene sequences. Moreover, it is desirable that the framework (FW) regions of the human heavy chain pseudogene sequences be identical to that of the human antibody heavy chain variable region that has been inserted into the chicken antibody heavy chain gene locus or has been replaced with the sequence in the chicken antibody heavy chain gene locus. For instance, when the DNA sequence of SEQ ID NO: 30 is selected as an antibody heavy chain variable region, the sequences shown in SEQ ID NO: 89 to SEQ ID NO: 108, SEQ ID NO: 127 to SEQ ID NO: 141, SEQ ID NO: 147 to SEQ ID NO: 161, and SEQ ID NO: 165 to SEQ ID NO: 179 can be used in combination as a heavy chain pseudogene.

In the present embodiment, the position of the DNA sequences derived from the human antibody light chain variable regions inserted into the chicken antibody light chain pseudogene locus is desirably upstream of the position of the human antibody light chain variable region inserted into the chicken antibody light chain gene locus. In addition, when a chicken antibody light chain pseudogene sequence remains, the DNA sequences derived from the human antibody light chain variable regions is desirably located downstream of the chicken antibody light chain pseudogene region. Likewise, the position of the DNA sequences derived from the human antibody heavy chain variable regions inserted into the chicken antibody heavy chain pseudogene locus is desirably upstream of the position of the human antibody heavy chain variable region inserted into the chicken antibody heavy chain gene locus. In addition, when a chicken antibody heavy chain pseudogene sequence remains, the DNA sequence derived from the human antibody heavy chain variable regions is desirably located downstream of the chicken antibody heavy chain pseudogene region.

Moreover, in the present embodiment, the DNA sequences derived from the human antibody variable regions, which are inserted or substituted as a pseudogene upstream of the chicken antibody variable region, may be used either in the same direction as, or in the direction opposite to, the direction of the human antibody variable region sequence inserted or substituted into the chicken antibody variable region.

The number of the DNA sequences derived from the human antibody light chain variable regions, which are inserted into the chicken antibody light chain pseudogene locus, or are substituted (replaced) with pseudogenes in the chicken antibody light chain pseudogene locus, is preferably 2 or more, more preferably 5 or more, 7 or more, 9 or more, 11 or more, 13 or more, further preferably 15 or more, 20 or more, or 25 or more. Likewise, the number of DNA sequences derived from the human antibody heavy chain variable regions, which are inserted into the chicken antibody heavy chain pseudogene locus, or are substituted (replaced) with pseudogenes in the chicken antibody heavy chain pseudogene locus, is preferably 2 or more, more preferably 5 or more, 7 or more, 9 or more, 11 or more, 13 or more, further preferably 15 or more, 20 or more, 25 or more, and most preferably 30 or more.

It is to be noted that the DNA sequence derived from the human antibody light chain variable region or the DNA sequence derived from the human antibody heavy chain variable region, which is used herein, is considered to be one DNA sequence, when it is a series of continuous variable region homologous sequences contained in a region over a variable region, and comprises any one of FW1, CDR1, FW2, CDR2, FW3, CDR3 and FW4.

In the present embodiment, the type of a human antibody heavy chain, which is inserted into the chicken antibody heavy chain gene locus, may be any one of a γ chain, an s chain, an α chain, a δ chain and a ε chain. It is preferably a γ chain. On the other hand, the type of a human antibody light chain, which is inserted into the chicken antibody light chain gene locus and the chicken antibody light chain pseudogene locus may be either a λ chain or a κ chain.

In the present embodiment, in order to use the transgenic chicken B cell for preparing an antibody library, the chicken B cell preferably does not only express a human antibody on the cell membrane thereof, but it also has an ability to secrete the human antibody into a culture solution. Therefore, when the introduced human antibody gene is transcribed, alternative splicing similar to an *in vivo* expression control mechanism needs to appropriately take place. Thus, with regard to introduction of the constant region, it is ideal to use a genomic structure that is completely identical to the constant region of a chicken antibody heavy chain constant region. However, since the nucleotide sequence of the genomic region of a chicken has not yet been analyzed at the time point of March 2015, this cannot be used. In the present embodiment, an intron sequence in the genomic sequence of a mouse antibody heavy chain has been used, and as a result, a human antibody on the surface of a cell membrane and a secretory human antibody have been successfully expressed at the same time. Accordingly, it is also possible to use, as a substitute, an intron sequence derived from known mammalian animals such as a mouse or a human.

In the present embodiment, a knocking-in method involving homologous recombination is used as a method of inserting a human antibody gene into a chicken antibody gene locus and/or replacing the gene in the chicken antibody gene locus with a human antibody gene. Moreover, as methods of inserting human antibody variable region-derived sequences into the pseudogene region in the chicken antibody chain gene locus and/or replacing the gene in the chicken antibody chain gene locus with a human antibody gene, a knocking-in method involving homologous recombination and a recombinase mediated cassette exchange (RMCE) method involving site-specific recombination can be preferably used.

As described above, a chicken B cell produced in the present embodiment means a chicken B cell, into which DNA sequences derived from a human antibody gene locus have been inserted, or which have been replaced with the DNA sequences derived from the human antibody gene locus; and a chicken B cell, in which a wide variety of mutations have been further introduced into the human-derived antibody light chain or heavy chain variable region of the aforementioned cell. Diversification of antibodies can be achieved by reorganization of the variable region that will cause generation of various variable region sequences. Therefore, a chicken B cell produced in the present embodiment also means a chicken B cell on which a treatment of introducing various mutations necessary for the reorganization of the variable region has been performed. Herein, examples of the method of introducing a mutation necessary for the reorganization of the variable region, which can be used herein, include methods known in the present technical field, such as a method of using B cells in which XRCC2 and XRCC3 have been deleted (e.g., Cumber et al., Nature Biotech. 204: 1129-1134 2002, JP Patent Publication (Kokai) No. 2003-503750 A, etc.), a method of controlling the expression of an AID gene (e.g., Kanayama et al., Nucleic Acids Res. 34: e10., 2006, JP Patent Publication (Kokai) No. 2004-298072 A, etc.), and a method of relaxing chromatin to promote gene conversion (e.g., Patent Literature 1, Patent Literature 2, Non Patent Literature 1, etc.). Among others, a method of relaxing chromatin to promote gene conversion is particularly preferable.

An example of the method of relaxing chromatin in a chicken B cell can be a method, which comprises inhibiting histone deacetylase (HDAC) activity existing in the chicken B cell by a certain method, and thereby significantly promoting gene conversion in the cell (for details, see the above-mentioned Patent Literature 1, Patent Literature 2, Non Patent Literature 1, etc.). As methods of inhibiting the activity of HDAC existing in cells, a method of treating the B cell of the present invention with an HDAC inhibitor (see Patent Literature 1 or Non Patent Literature 1), a method of reducing or deleting the function of an HDAC gene in a chicken B cell (see Patent Literature 2), and the like can be used. The HDAC inhibitor is not particularly limited, as long as it is able to inhibit the activity of HDAC. Examples of the HDAC inhibitor that can be used herein include trichostatin A (TSA), butyric acid, valproic acid, suberoylanilide hydroxamic acid (SAHA), CBHA (m-carboxycinnamic acid bis-hydroxamide), PTACH, Apicidin, Scriptaid, M344, APHA compound 8, BML-210, Oxamflatin, and MS-275. Other than these substances, an inactive-type protein of the HDAC as a target of activity inhibition (a dominant negative) may also be used as an inhibitory substance, if possible.

When the function of an HDAC gene is reduced or deleted, isoforms of the HDAC as a target whose gene function is to be reduced or deleted vary depending on an antibody-producing B cell used. The isoform is preferably an HDAC2 gene (for details, see Patent Literature 2).

The embodiment of the present invention that is relevant to the above-described embodiment includes an antibody-producing cell library consisting of the chicken B cells of the present invention. The present cell library means a population of the chicken B cells of the present invention that produce antibodies reacting with various antigens. The chicken B cell according to the above-described embodiment of the present invention is a cell, in which variation is generated in the variable region sequence of an antibody produced by the cell, by insertion of DNA sequences derived from a human-derived antibody variable regions into a chicken antibody pseudogene locus, etc., and the sequence of the antibody variable region is further diversified by a treatment of relaxing chromatin, so that the cell can have an ability to produce antibodies reacting with various antigens.

Therefore, a population comprising a plurality of the chicken B cells of the present invention can be a cell population producing antibodies reacting with various antigens.

Other embodiments of the present invention relate to a method for producing an antibody from the chicken B cell of the present invention, and a produced antibody.

The antibody produced from the chicken B cell of the present invention is a humanized antibody, and more preferably a human antibody. The humanized antibody means an antibody, in which a part of a sequence derived from a chicken gene is comprised in the amino acid sequence of the heavy chain or light chain of a produced antibody, and the human antibody means an antibody, in which the entire amino acid sequence of the heavy chain or light chain of a produced antibody is a sequence derived from a human gene.

When the chicken B cell of the present invention is cultured under culture conditions suitable for the cell, it produces a humanized antibody or a human antibody. It has been known that, in a cell line derived from chicken B cells such as DT40 cells, gene conversion occurs in an antibody gene locus thereof, although it does not occur with high efficiency. Thus, such gene conversion occurs also in the chicken B cell of the present invention, and primary variation in the produced antibodies is achieved by gene conversion occurring in an antibody gene locus, into which a human-derived antibody gene has been inserted. Moreover, by relaxing chromatin in the chicken B cell of the present invention, the gene conversion efficiency in the antibody gene locus is further increased, so that a population of chicken B cells capable of producing more various antibodies can be prepared. In order to select chicken B cells producing antibodies exhibiting desired antigen specificity by utilizing the relaxing of chromatin, an ADLib system can be used. The above-mentioned antibody-producing cell library can also be prepared by utilizing the ADLib system (see Patent Literature 1 or Non Patent Literature 1).

Furthermore, in particular, as a method of obtaining a desired antibody reacting with a membrane-bound protein, an ADLib axCELL (antigen expressing cell) method can be utilized (WO2010/064454).

Another embodiment of the present invention includes a kit for producing the chicken B cell of the present invention or producing an antibody.

The kit for producing the chicken B cell comprises: a medium, supplements and the like that are necessary for the culture of the chicken B cell; a vector used for inserting a DNA sequence derived from a human antibody gene into the antibody gene locus of the chicken B cell, or replacing the chicken B cell antibody gene locus with the DNA sequence derived from the human antibody gene; and reagents. In addition, the kit for producing the chicken B cell may also comprise: elements necessary for preparing an antibody-producing cell library, such as an HDAC inhibitor; and reagents necessary for reducing or deleting the function of the HDAC genes.

On the other hand, the kit for producing an antibody may comprise, as reagents for selecting an antibody having desired antigen specificity, all types of items that are considered necessary for production of the antibody. Examples of such reagents include various antigens, magnetic beads, a reagent for preparing such magnetic beads, a labeled antibody used to select an antibody, and a plate necessary for examinations such as ELISA, which are.

Hereinafter, the present invention will be described more in detail in the following examples. However, these examples are not intended to limit the scope of the present invention.

### Examples

### Confirmation of gene conversion

### 1. Preparation of vectors

### 1-1. Replacement of constant region and variable region of chicken antibody light chain (Igλ) (human LC KI vector)

For replacement of the constant region and variable region of chicken Igλ, a targeting vector as shown in Figure 1 was prepared. Methods for preparing individual parts will be described below. (1) Variable region and constant region of human Igλ

cDNA prepared from a human Burkitt's lymphoma cell line Ramos was used as a template, and PCR was carried out using a sense primer (TCCACCATGGCCTGGGCTCTG) (SEQ ID NO: 1) and an antisense primer (GTTGAGAACCTATGAACATTCTGTAGGGGCCAC) (SEQ ID NO: 2), so that the variable region and constant region of a human antibody light chain (Igλ) were amplified. The amplified regions were cloned into pGEM-T-Easy (Promega) to obtain pGEM-hIgG1-LC. This plasmid pGEM-hIgG1-LC was used as a template, and PCR was carried out using a sense primer (ATTGGCGCGCCTCTCCAGGTTCCCTGGTGCAGGCACAGTCTGCCCTGACTC AGC) (SEQ ID NO: 3) and an antisense primer (TTCCATATGAGCGACTCACCTAGGACGGTCAGCTTGGTCC) (SEQ ID NO: 4), so as to obtain the variable region (SEQ ID NO: 5) of human Igλ.

Moreover, the pGEM-hIgG1-LC was used as a template, and PCR was carried out using a sense primer (ATTGGCGCGCCTCTGCCTCTCTCTTGCAGGTCAGCCCAAGGCTGCCCCCTC) (SEQ ID NO: 6) and an antisense primer (GGAATTCCATATGGAGTGGGACTACTATGAACATTCTGTAGGGG) (SEQ ID NO: 7), so as to obtain the constant region (SEQ ID NO: 8) of human Igλ.

### (2) Left Arm

The genomic DNA of a chicken B cell line DT40 was used as a template, and PCR was carried out using a sense primer (GAGATCTCCTCCTCCCATCC) (SEQ ID NO: 9) and an antisense primer (CAAAGGACACGACAGAGCAA) (SEQ ID NO: 10), so that a region ranging from upstream of the variable region of chicken Igλ to downstream of the constant region thereof was amplified. A functional allele was separated from a non-functional allele by electrophoresis. The functional allele with a size of approximately 8.0 kbp was cut out, and it was then cloned into pGEM-T-Easy to obtain pGEM-cIgM-LCgenome. Using the pGEM-cIgM-LCgenome as a template, PCR was carried out with a sense primer (TGTCTCGAGTGAAGGTCACCAAGGATGG) (SEQ ID NO: 11) and an antisense primer (TTAAGCTTGGAGAGGAGAGAGGGGAGAA) (SEQ ID NO: 12), so as to obtain Left Arm shown in SEQ ID NO: 13.

### (3) Center Arm

Based on the sequence analysis of the pGEM-cIgM-LCgenome, the sequence of an intron region located between the variable region and the constant region of chicken Igλ was determined, and Center Arm shown in SEQ ID NO: 14 (wherein a blasticidin resistance gene having loxP sequences at both ends was inserted into the midstream thereof) was then synthesized.

### (4) Right Arm

The sequence of a region downstream of the constant region of chicken Igλ was determined using the pGEM-cIgM-LCgenome, and Right Arm shown in SEQ ID NO: 15 (wherein a neomycin resistance gene having lox2272 sequences at both ends was inserted into the midstream thereof) was then synthesized.

### (5) Construction of targeting vector

The above-described Left Arm, human Igλ variable region, Center Arm, human Igλ constant region, and Right Arm were incorporated into pBluescript to have the structure shown in Figure 1, so as to obtain a human LC KI vector. The gene sequence of this vector is shown in SEQ ID NO: 16.

### 1-2. Knocking-in into downstream of chicken antibody light chain (Igλ) pseudogene region (KI-C-IN vector)

In order to insert a sequence for confirming gene conversion (a GC confirmation sequence; a human pseudogene consisting of a sequence derived from the variable region of human Igλ) (SEQ ID NO: 17) downstream of the pseudogene region of chicken Igλ, a targeting vector as shown in Figure 2 was first produced. Methods for preparing individual parts will be described below.

### (1) Left Arm

The genomic DNA of DT40 was used as a template, and a 4.3-kb region (pseudogenes (pV) 1 to 3) located upstream of the promoter in the variable region was amplified by PCR using a sense primer (TTCTGTGAGCTGAGAAAAGGAGTGTA) (SEQ ID NO: 18) and an antisense primer (CCTGCATTGTGGCACAGCGGGGTT) (SEQ ID NO: 19), and it was then cloned into pCR4blunt-TOPO (Life Technologies) to obtain pCR4-cIgM-LC pV1 genome. Based on this sequence, a region comprising pV1-3 (SEQ ID NO: 20) was cloned, so as to obtain Left Arm.

### (2) Right Arm

Based on the sequence of the pCR4-cIgM-LCpV1 genome obtained in the above section, a portion that is a region between pV1 and the promoter (SEQ ID NO: 21) was cloned, so as to obtain Right Arm. (3) Construction of targeting vector

The above-described Left Arm, neomycin resistance gene, and Right Arm were incorporated into the pCR4blunt-TOPO to have the structure shown in Figure 2, so as to obtain a KI-C-IN vector. The gene sequence of this vector is shown in SEQ ID NO: 22.

### 1-3. Insertion of GC confirmation sequence downstream of pseudogene region of chicken antibody light chain (Igλ) (KI-C-IN-C vector)

In order to insert a GC confirmation sequence downstream of the pseudogene region of chicken Igλ, a targeting vector as shown in Figure 2 was produced. Methods for preparing individual parts will be described below.

### (1) Sequence for confirmation of GC

Based on the variable region of human Igλ cloned in 1-1 above, a mutant sequence, in which 3 nucleotides were inserted into each CDR, and a mutant sequence, in which 3 nucleotides were deleted from each CDR, were designed, and these mutant sequences were then ligated to each other using the untranslated region sequence of a chicken Igλ pseudogene region to synthesize a sequence for confirmation of GC (SEQ ID NO: 17).

### (2) Construction of targeting vector

The sequence for confirmation of GC was incorporated into the KI-C-IN vector prepared in 1-2 above to have the structure shown in Figure 2, so as to obtain a KI-C-IN-C vector. The gene sequence of this vector is shown in SEQ ID NO: 23.

### 1-4. Deletion of pseudogene region of chicken antibody light chain (Igλ) (KI-C-DE vector)

In order to delete the pseudogene region of chicken Igλ, a targeting vector as shown in Figure 3 was produced. Methods for preparing individual parts will be described below.

### (1) Left Arm

The genomic DNA of DT40 was used as a template, and an approximately 3.0-kb region located upstream of pV25 was amplified by PCR using a sense primer (CGCTTTGTACGAACGTTGTCACGT) (SEQ ID NO: 24) and an antisense primer (TACCTGAAGGTCTCTTTGTGTTTTG) (SEQ ID NO: 25), and it was then cloned into the pCR4blunt-TOPO to obtain Left Arm shown in SEQ ID NO: 26.

### (2) Right Arm

The same Right Arm as that prepared in 1-2 above was used.

### (3) Construction of targeting vector

The above-described Left Arm, neomycin resistance gene, and Right Arm were incorporated into the pCR4blunt-TOPO to have the structure shown in Figure 3, so as to obtain a KI-C-DE vector. The gene sequence of this vector is shown in SEQ ID NO: 27.

### 1-5. Replacement of pseudogene region of chicken antibody light chain (Igλ) with sequence for confirmation of GC (KI-C-DE-C vector)

In order to replace the pseudogene region of chicken Igλ with a sequence for confirmation of GC, a targeting vector as shown in Figure 3 was produced. Methods for preparing individual parts will be described below.

### (1) GC confirmation sequence

The GC confirmation sequence prepared in 1-2 above was used.

### (2) Construction of targeting vector

The above-described GC confirmation sequence was incorporated into the KI-C-DE vector produced in 1-3 above to have the structure shown in Figure 3, so as to obtain a KI-C-DE-C vector. The gene sequence of this vector is shown in SEQ ID NO: 28.

### 1-6. Knocking-in into constant region and variable region of chicken antibody heavy chain (IgM)

In order to substitute a region ranging from the variable region of chicken IgM (IgM) to the constant region µ1 thereof with a human antibody heavy chain (IgG₁), a targeting vector as shown in Figure 4 was produced. Methods for preparing individual parts will be described below.

### (1) Human IgG₁ constant region

Among sequences obtained by subjecting human genome chromosome 14 32.33 comprising human IgG₁ to shotgun sequencing, a sequence corresponding to IgG₁ was obtained (Accession No.: NW_001838121.1). The sequences of CHI corresponding to the "C region" of "IGH1," a hinge, CH2, CH3 and a transmembrane domain were extracted from a sequence between the nucleotide 41520 and the nucleotide 43117, and a sequence was then designed by replacing the intron with a mouse IgG₂ₐ-derived sequence. Thereafter, a human IgG₁ constant region sequence (SEQ ID NO: 29) was obtained by synthesis.

### (2) Human IgG₁ variable region

Based on the sequence of a human germ cell-derived IgG₁ variable region gene, a sequence was designed by ligating VH3-23, D5-12, and JH1 sequences to one another, and a chicken-derived secretory signal sequence or splicing signal sequence was then added to the sequence to prepare a human IgG₁ variable region (SEQ ID NO: 30).

### (3) Left Arm

The genomic DNA of DT40 was used as a template, and a region ranging from a region upstream of a chicken IgM variable region to the variable region was amplified by PCR using a sense primer (TTCCCGAAGCGAAAGCCGCGT) (SEQ ID NO: 31) and an antisense primer (ACTCACCGGAGGAGACGATGA) (SEQ ID NO: 32), and an approximately 4.1-kbp fragment was then cloned, so as to obtain pCR4 Blunt-TOPO-cIgM-HC pV1 genome. Based on this sequence, Left Arm (SEQ ID NO: 33), to the 3' terminal side of which a Vlox sequence was added, was designed and synthesized.

### (4) Center Arm 1

Based on the approximately 4.1-kbp sequence ranging from the region upstream of the chicken IgM variable region to the variable region, which had been cloned upon the above-described cloning of the Left Arm, Center Arm 1 corresponding to an approximately 1.4-kbp region upstream of the chicken IgM variable region was synthesized (SEQ ID NO: 34).

### (5) Center Arm 2

The genomic DNA of DT40 was used as a template, and an approximately 4.3-kbp region located downstream of the chicken IgM variable region was amplified by PCR using a sense primer (GGGGATCCTGGGTCAGTCGAAGGGGGCG) (SEQ ID NO: 35) and an antisense primer (GTGCGGCCGCCAAAAGCGGTAAAATCCACCC) (SEQ ID NO: 36), and it was then cloned, so as to obtain Center Arm 2 shown in SEQ ID NO: 37.

### (6) Right Arm

The genomic DNA of DT40 was used as a template, and an approximately 0.9-kbp region comprising a chicken IgM constant region µ2 was amplified by PCR using a sense primer (CCAAACCACCTCCTGGTGTCC) (SEQ ID NO: 38) and an antisense primer (CAAACCAAAACTACGGATTCTCTGACC) (SEQ ID NO: 39), and it was then cloned, so as to obtain Right Arm shown in SEQ ID NO: 40. (7) Construction of targeting vector

The above-described Left Arm, multi-cloning site, Center Arm1, human IgG₁ variable region, blasticidin resistance gene, Center Arm 2, human IgG₁ constant region, neomycin resistance gene, and Right Arm were incorporated into pBluescript to have the structure shown in Figure 4, so as to obtain a human HC V-C vector. The gene sequence of this vector is shown in SEQ ID NO: 41.

### 1-7. Knocking-in into constant region, variable region, and pseudogene region of chicken antibody heavy chain (IgM) (human HC KI vector)

In order to insert a sequence for confirming gene conversion (a GC confirmation sequence; a human pseudogene consisting of a human Igγ variable region-derived sequence), a human IgG₁ variable region, and a human IgG₁ constant region into a region ranging from a region downstream of the pseudogene region of chicken IgM to a constant region µ1 thereof, a targeting vector as shown in Figure 4 was produced. Methods for preparing individual parts will be described below.

### (1) Human IgG₁ constant region

The human IgG₁ constant region sequence described in 1-6 above was used.

### (2) Human IgG₁ variable region

The human IgG₁ variable region described in 1-6 above was used.

### (3) Sequence for confirming gene conversion

Based on the variable region of human Igγ designed in 1-6 above, a mutant sequence was designed by deleting one nucleotide from CDR1, another mutant sequence was designed by deleting two nucleotides from CDR2, and another mutant sequence was designed by inserting one nucleotide into CDR3. These mutant sequences were ligated to one another using the untranslated region sequence of a chicken Igγ pseudogene region to synthesize a GC confirmation sequence (SEQ ID NO: 42).

### (4) Left Arm

The Left Arm described in 1-6 above was used.

### (5) Center Arm 1

The Center Arm 1 described in 1-6 above was used.

### (6) Center Arm 2

The Center Arm 2 described in 1-6 above was used.

### (7) Right Arm

The Right Arm described in 1-6 above was used.

### (8) Construction of targeting vector

The above-described Left Arm, GC confirmation sequence, Center Arm 1, human IgG₁ variable region, blasticidin resistance gene, Center Arm 2, human IgG₁ constant region, neomycin resistance gene, and Right Arm were incorporated into pBluescript to have the structure shown in Figure 4, so as to obtain a human HC KI vector. The gene sequence of this vector is shown in SEQ ID NO: 43.

### 1-8. Construction of vector for expressing Cre recombinase

Since a drug resistance gene has been incorporated into the above-produced vector in such a form that loxP sequences have been added to both ends of the gene, Cre recombinase is allowed to act on the vector after completion of the gene transfer, so that this gene can be removed. Hence, a Cre recombinase gene shown in SEQ ID NO: 44 was incorporated into pEGFP-C1 (BD Bioscience, #6084-1) to produce Cre_EGFP-C1 shown in SEQ ID NO: 45.

### 2. Production of cell lines into light chain of which human sequence has been inserted or substituted

As a host, DT40, a cell line derived from chicken B cells, was used. The DT40 was cultured by the following method.

Using a CO₂ thermostatic chamber, the cells were cultured at 39.5°C in the presence of 5 % CO₂. IMDM (Life Technologies) was used as a medium, and 9 % FBS, 1 % chicken serum, 100 units/mL penicillin, 100 µg/mL streptomycin, and 50 µM monothioglycerol were added to the medium, and the thus obtained medium was then used. Hereinafter, unless otherwise specified, the "medium" indicates a medium having the aforementioned composition.

In order to evaluate gene conversion in a light chain, five types of cell lines shown in the following Table 1 were prepared.

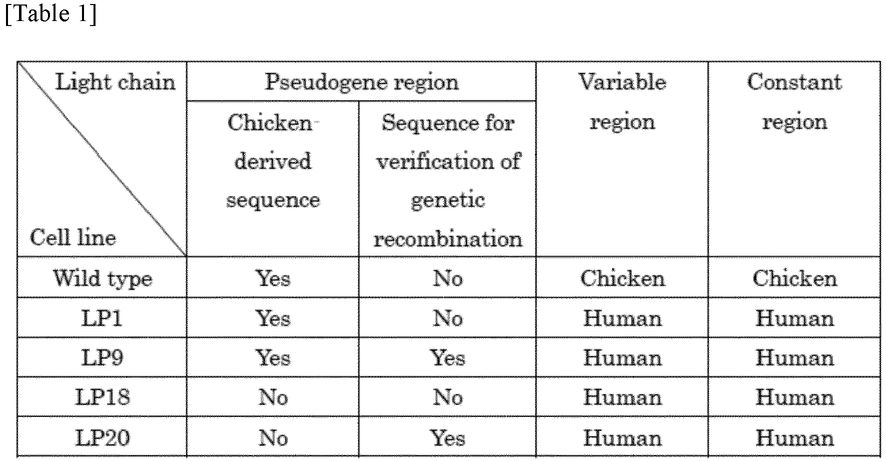

Methods for producing these cell lines will be described below.

### 2-1. Production of cell lines in which variable region and constant region have been replaced with human-type regions

The human LC KI vector produced in 1-1 above was linearized with the restriction enzyme NotI, and it was then introduced into DT40 cells by electroporation. The resulting cells were cultured in a medium, to which 2 mg/mL G418 and 10 µg/mL blasticidin had been added, for 7 to 10 days. Thereafter, the growing cells were subjected to genotyping by PCR, and a cell line in which gene substitution had occurred in a gene locus of interest was selected. The Cre_pEGFP-C1 produced in 1-8 above was introduced into the selected cell line, and the resulting cell line was then subjected to single cell sorting by flow cytometry, so as to select GFP-positive cells. Thereafter, the GFP-positive cells were subjected to genotyping by PCR, so as to obtain a cell line 37-3, in which the removal of the drug resistance genes could be confirmed. Thereafter, the KI-C-IN vector produced in 1-2 above was linearized with the restriction enzyme NotI, and it was then introduced into the cell line 37-3. The cells were cultured in a medium, to which 2 mg/mL G418 had been added, for 7 to 10 days. Thereafter, the growing cells were subjected to genotyping by PCR, so as to obtain a cell line LP1, in which gene insertion had occurred in a gene locus of interest (Figure 5).

### 2-2. Production of cell line in which variable region and constant region have been replaced with human-type regions, and further, in which sequence for confirming gene conversion (GC sequence) has been inserted downstream of chicken pseudogene region

The KI-C-IN-C vector produced in 1-3 above was linearized with the restriction enzyme NotI, and it was then introduced into the cell line 37-3 obtained in the above section. The resulting cells were cultured in a medium, to which 2 mg/mL G418 had been added, for 7 to 10 days. Thereafter, the growing cells were subjected to genotyping by PCR, so as to obtain a cell line LP9, in which gene insertion had occurred in a gene locus of interest (Figure 6).

### 2-3. Production of cell line in which variable region and constant region have been replaced with human-type regions, and further, in which chicken pseudogene region has been deleted

The KI-C-DE vector produced in 1-4 above was linearized with the restriction enzyme NotI, and it was then introduced into the cell line 37-3 obtained in 2-1 above. The resulting cells were cultured in a medium, to which 2 mg/mL G418 had been added, for 7 to 10 days. Thereafter, the growing cells were subjected to genotyping by PCR, so as to obtain a cell line LP18, in which gene insertion had occurred in a gene locus of interest (Figure 7).

### 2-4. Production of cell line in which variable region and constant region have been replaced with human-type regions, and further, in which chicken pseudogene region has been replaced with sequence for confirming gene conversion (GC sequence)

The KI-C-DE-C vector produced in 1-5 above was linearized with the restriction enzyme NotI, and it was then introduced into the cell line 37-3 obtained in 2-1 above. The resulting cells were cultured in a medium, to which 2 mg/mL G418 had been added, for 7 to 10 days. Thereafter, the growing cells were subjected to genotyping by PCR, so as to obtain a cell line LP20, in which gene substitution had occurred in a gene locus of interest (Figure 8).

### 3. Confirmation of antibody expression (flow cytometry and ELISA)

### 3-1. Analysis by flow cytometry

An antibody expressing on the surface of cells of each of the cell lines produced in 2 above was analyzed. Each cell line was cultured, and the cultured cells were then dispensed on a 96-well plate (Nunc, 249662) in an amount of 5 × 10⁵ cells/well. The cells were washed with 150 µL of FACS buffer (PBS containing 0.3 % bovine serum albumin (BSA)) twice, and 50 µL of a primary antibody solution, which had been prepared by diluting Goat anti-Chicken IgM-FITC conjugate (Bethyl, A30-102F-20), Goat Anti-Human IgG (Gamma chain specific) R-PE conjugate (Southern biotech, A2040-09) or Goat Anti-human lambda PE conjugate (Southern biotech, 2070-09) with FACS buffer, 1000 times, 500 times, and 500 times, respectively, was added to the resulting cells. The mixture was incubated on ice in a dark place for 30 minutes, and it was then washed with 150 µL of FACS buffer twice. Thereafter, 200 µL of a PI solution, which had been 1000 times diluted with FACS buffer, was added to the reaction mixture, and was then suspended therein. This suspension was subjected to a flow cytometric analysis, and the expression of chicken IgM and human Igγ (heavy chain) or human Igλ (light chain) was analyzed. As a result, it was confirmed that, as shown in Figure 9, only chicken IgM was expressed in wild-type DT40, whereas chicken IgM and human Igλ were expressed in the transformed cell lines LP1, LP9, LP18, and LP20. In all of the cell lines, the expression of Igγ that had not undergone gene transfer was not observed.

### 3-2. Analysis by ELISA

The concentrations of chicken IgM and human IgG₁, which were secreted from the cells produced in 2 above into culture supernatants, were measured. Individual cell lines were suspended in a medium containing no chicken serum to a concentration of 4 × 10⁵ cells/mL, and 2 mL each of the suspension was then plated on each well of a 24-well plate, followed by performing a culture for 3 days. Thereafter, the culture solution was recovered, was then filtrated through a 0.22-µm filter, and was then used in the measurement.

The method of measuring IgG is as follows. 20 µL of 1.0 µg/mL Goat anti-Human IgG-Fc affinity purified (Bethyl, A80-104A) was dispensed into an immuno 384-well plate Maxisorp (Nunc, 464718), and it was then reacted at room temperature for 1 hour or more, so that it was immobilized on the plate. Thereafter, the resultant on the plate was washed with a washing solution (PBS containing 0.05% Tween 20) five times, 50 µL of a blocking solution (PBS containing 1% BSA) was then added thereto, and the mixture was then reacted at room temperature for 30 minutes. Thereafter, the reaction mixture was washed with a washing solution five times, 20 µL of a measurement sample or Human IgG1 Lambda-UNLB (Southern Biotech, 0151L-01) serving as a standard substance was then added thereto, and the obtained mixture was then reacted at room temperature for 1 hour. Thereafter, the reaction mixture was washed with a washing solution five times, 20 µL of Goat anti-Human IgG-Fc HRP conjugated (Bethyl, A80-104P), which had been 1000 times diluted with PBS containing 1 % BSA and 0.05% Tween 20, was then added thereto, and the obtained mixture was then reacted at room temperature for 1 hour. Thereafter, the reaction mixture was washed with a washing solution five times, 20 µL of TMB+ (Dako, S159985) was then added thereto, and the obtained mixture was subjected to a coloring reaction at room temperature for 3 minutes. Subsequently, 20 µL of 1 N sulfuric acid was added to the reaction mixture to termination the reaction. Using Infinite M1000 (TECAN), the absorbance at 450 nm was measured.

For the measurement of IgM, the system of AlphaLISA Immunoassay (Perkin Elmer) was used. Goat anti-chicken IgM antibody (Bethyl, A30-102A) was labeled with biotin, employing Chromalink™ Biotin Labeling Reagent (SoluLink Inc., #B1001-105), so that a biotinylated antibody was prepared. Subsequently, Goat anti-chicken IgM antibody (Bethyl, A30-102A) was allowed to bind to Unconjugated AlphaLISA Acceptor Beads (Perkin Elmer), so that antibody-bound Alpha Screen beads were prepared. These biotin-labeled antibody and antibody-bound Alpha Screen beads were each diluted with an assay buffer (PBS containing 1% BSA) to concentrations of 4.5 nM and 50 µg/mL, respectively. Thereafter, they were mixed with each other in equal amounts, so as to prepare a chicken IgM concentration measurement solution. 20 µL of the chicken IgM concentration measurement solution was mixed with 5 µL of a measurement sample or the chicken serum (GIBCO, 16110) serving as a standard substance on a 96-well plate (Nunc), and the mixed solution was then dispensed into Alpha Plate-384 (Perkin Elmer) in an amount of 12.5 µL each. The mixed solution was reacted at room temperature for 60 minutes, and thereafter, 12.5 µL of AlphaScreen Streptavidin Donor Beads (Perkin Elmer), which had been adjusted to a concentration of 80 µg/mL with an assay buffer, was added to the reaction solution, and the obtained mixture was then reacted at room temperature under light shielding conditions for 30 minutes. Thereafter, using EnSpire (Perkin Elmer), an analysis was carried out.

The measurement results are shown in Table 2. It was confirmed that, as with the wild-type DT40, chicken IgM was secreted also in the transformed cell lines LP1, LP9, LP18 and LP20.

**[Table 2]**

| Cell line | IgG (µg/mL) | IgM (µg/mL) |
|---|---|---|
| Wild type | <0.01 | 2.30 |
| LP1 | <0.01 | 0.09 |
| LP9 | <0.01 | 0.10 |
| LP18 | <0.01 | 0.09 |
| LP20 | <0.01 | 0.03 |

### 4. Confirmation of gene conversion

The cell lines, in which the expression of human Igλ (light chain) had been confirmed in 3 above, were cultured in a medium containing 2.5 ng/mL TSA for 20 days, and genomic DNA was then extracted from 1 × 10⁶ cells. This genomic DNA was used as a template, and a light chain variable region was amplified by PCR using a sense primer to which a recognition sequence had been added (CGTATCGCCTCCCTCGCGCCATCAGNNNNNNNNNNCAGGTTCCCTGGTGC AGGC) (SEQ ID NO: 46), and an antisense primer (CTATGCGCCTTGCCAGCCCGCTCAGGCTTGGTCCCTCCGCCGAA) (SEQ ID NO: 47). Thereafter, the resultant was purified by agarose gel extraction, and was then concentrated using QIAGEN PCR Purification Kit (QIAGEN). Using the Next-Generation Sequencing Analysis System of Roche Diagnostics, the sequence of this sample was analyzed, and the presence or absence of gene conversion was evaluated based on whether or not a gene sequence identical to the inserted human pseudogene sequence would be present in the variable region. The results are shown in Table 3. It was confirmed that gene conversion had not occurred in LP1 and LP18 that did not comprise the human pseudogene sequence, whereas such gene conversion had occurred in LP9 and LP20 having the human pseudogene sequence.

**[Table 3]**

| Cell line | Number of sequences | Number of sequences with genetic recombination | Frequency |
|---|---|---|---|
| LP1 | 785 | 0 | 0.0% |
| LP9 | 344 | 18 | 5.2% |
| LP18 | 378 | 0 | 0.0% |
| LP20 | 634 | 31 | 4.9% |

### 5. Production of cell line, in which human sequence has been inserted and/or substituted into heavy chain

In order to evaluate gene conversion in a heavy chain, the three types of cell lines shown in Table 4 were prepared.

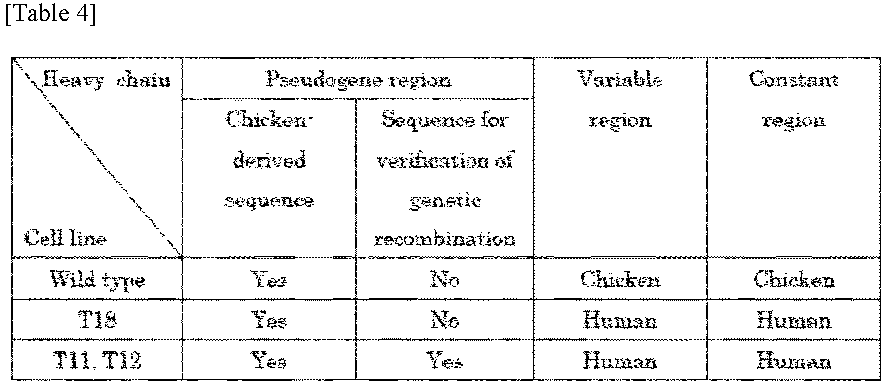

Methods for producing these cell lines will be described below.

### 5-1. Production of cell line in which variable region and constant region of heavy chain have been replaced with human-type regions

The human HC V-C vector produced in 1-6 above was linearized with the restriction enzyme SalI, and it was then introduced into the cell line 37-3 obtained in 2-2 above. The resulting cells were cultured in a medium, to which 2 mg/mL G418 and 10 µg/mL blasticidin had been added, for 7 to 10 days. Thereafter, the growing cells were subjected to genotyping by PCR, and a cell line T18 in which gene substitution had occurred in a gene locus of interest was obtained (Figure 10).

### 5-2. Production of cell line in which variable region and constant region of heavy chain have been replaced with human-type regions, and sequence for confirming gene conversion (GC confirmation sequence) has been inserted downstream of pseudogene region

The human HC KI vector produced in 1-7 above was linearized with the restriction enzyme SalI, and it was then introduced into the cell line 37-3 obtained in 2-2 above. The resulting cells were cultured in a medium, to which 2 mg/mL G418 and 10 µg/mL blasticidin had been added, for 7 to 10 days. Thereafter, the growing cells were subjected to genotyping by PCR, and cell lines T11 and T12, in which gene substitution had occurred in a gene locus of interest was obtained (Figure 11).

### 6. Confirmation of antibody expression (flow cytometry and ELISA)

### 6-1. Analysis by flow cytometry

Based on the method described in 3-1 above, the expression of an antibody in the cells produced in 5 above was confirmed. The results are shown in Figure 12. It was confirmed that only chicken IgM was expressed in wild-type DT40, whereas the expression of chicken IgM disappeared and instead, human Igγ and Igλ were expressed in the transformed cell lines T18, T11 and T12.

### 6-2. Analysis by ELISA

Based on the method described in 3-2 above, the antibodies secreted from the cells produced in 5 above into a culture supernatant were measured. The results are shown in Table 5. As with 6-1 above, it was confirmed that only chicken IgM was secreted from wild-type DT40, whereas human IgG₁ was secreted from T18, T11 and T12, into which human IgG₁ had been introduced.

**[Table 5]**

| Cell line | IgG (µg/mL) | IgM (µg/mL) |
|---|---|---|
| Wild type | <0.01 | 2.30 |
| T18 | 1.00 | 0.03 |
| T11 | 0.40 | <0.01 |
| T12 | 0.69 | <0.01 |

### 7. Confirmation of gene conversion

Using the cell lines in which the expression of an antibody had been confirmed in 6 above, gene conversion was confirmed. The genomic DNA extracted by the method described in 4 above was used as a template, and a heavy chain variable region was amplified by PCR using a sense primer (CTATGCGCCTTGCCAGCCCGCTCAGTCCGTCAGCGCTCTCT) (SEQ ID NO: 48) and an antisense primer to which an identification tag had been added (CGTATCGCCTCCCTCGCGCC ATCAGNNNNNNNNNNCGGTGACCAGGGTGCCCTG) (SEQ ID NO: 49). Thereafter, a sequence analysis was carried out based on the method described in 4 above. The results are shown in Table 6. It was confirmed that gene conversion had not occurred at all in T18 containing no human pseudogenes, whereas such gene conversion had occurred in T11 and T12 having such a human pseudogene sequence.

**[Table 6]**

| Cell line | Number of sequences | Number of sequences with genetic recombination | Frequency |
|---|---|---|---|
| T18 | 1125 | 0 | 0.0% |
| T11 | 3038 | 415 | 13.4% |
| T12 | 4108 | 305 | 7.4% |

### Production of L5/H5 cell line, in which pseudogene region consisting of five human variable region-derived sequences has been inserted into each of light chain and heavy chain

### 8. Production of vectors

### 8-1. Knocking-in into chicken antibody (Igλ) pseudogene region

In order to substitute the pseudogene region of chicken Igλ with a pseudogene consisting of a sequence derived from the variable region of human Igλ (a human light chain pseudogene), a targeting vector as shown in Figure 13 was produced. The production method will be described below.

### (1) Designing and production of human antibody light chain pseudogene sequence (pVL5)

Based on the gene sequences of immunoglobulin λ light chain variable regions, which had not caused V(D)J recombination in the human genome in the database, five sequences were selected from each of CDR1, CDR2 and CDR3, and the framework sequence of the Igλ variable region cloned in 1-1 above was then combined therewith, so as to design five sequences, namely, pVL001 (SEQ ID NO: 69), pVL002 (SEQ ID NO: 70), pVL003 (SEQ ID NO: 71), pVL004 (SEQ ID NO: 72) and pVL005 (SEQ ID NO: 73). Thereafter, a human light chain pseudogene sequence pVL5 (SEQ ID NO: 50) comprising these sequences was designed, and was then subjected to gene synthesis.

### (2) Production of targeting vector

On the basis of the KI-C-DE vector produced in 1-4 above, the GC confirmation sequence was replaced with the human antibody light chain pseudogene sequence pVL5 described in the above section to produce a human LCpV KI vector. The gene sequence of this vector is shown in SEQ ID NO: 51.

### 8-2. Substitution of chicken antibody heavy chain (IgM) constant region

In order to substitute the heavy chain constant region of chicken IgM with human IgG₁, a targeting vector as shown in Figure 14 was produced. The production method will be described below.

### (1) Human IgG₁ constant region

The human IgG₁ constant region produced in 1-6 above was used.

### (2) Left Arm

The Center Arm 2 produced in 1-6 above was used.

### (3) Right Arm

The Right Arm produced in 1-6 above was used.

### (4) Production of targeting vector

The above-described Left Arm, human IgG₁ constant region sequence, neomycin resistance gene, and Right Arm were incorporated into pBluescript to produce a human CH KI vector. The gene sequence of this vector is shown in SEQ ID NO: 52.

### 8-3. Insertion into chicken IgM variable and pseudogene regions (1) Human IgG₁ variable region

The human IgG₁ variable region produced in 1-6 above was used.

### (2) Designing and production of human antibody heavy chain pseudogene sequence (pVH5)

Based on the sequences of human immunoglobulin heavy chain variable regions in the database, five sequences were selected from each of CDR1, CDR2 and CDR3, and the framework sequence of the variable region cloned in 1-6 above was then combined therewith, so as to design five sequences, namely, pVH001 (SEQ ID NO: 89), pVH002 (SEQ ID NO: 90), pVH003 (SEQ ID NO: 91), pVH004 (SEQ ID NO: 92) and pVH005 (SEQ ID NO: 93). Thereafter, a human antibody heavy chain pseudogene sequence pVH5 (SEQ ID NO: 53) comprising these sequences was designed, and was then subjected to gene synthesis.

### (3) Left Arm

The Left Arm produced in 1-6 above was used.

### (4) Center Arm

The Center Arm 1 produced in 1-6 above was used.

### (5) Right arm

The Center Arm 2 produced in 1-6 above was used. The confirmed sequence of the Right Arm is shown in SEQ ID NO: 54. (6) Production of targeting vector

The above-described Left Arm, human antibody heavy chain pseudogene sequence pVH5, Center Arm, human IgG₁ variable region sequence, blasticidin resistance gene, and Right Arm were incorporated into pBluescript to obtain a human pVH VH KI vector. The gene sequence of this vector is shown in SEQ ID NO: 55.

### 9. Production of L5/H5 cell line, in which five human pseudogenes have been introduced into each of gene loci of chicken antibody light chain and heavy chain

The human CH KI vector produced in 8-2 above, the human pVH VH KI vector produced in 8-3 above, and the human LC pV KI vector produced in 8-1 above were introduced into the cell line 37-3 obtained in 2-2 above, so as to produce a L5/H5 cell line (Figure 15).

The human CH KI vector, which had been linearized with the restriction enzyme SalI, was introduced into the cell line 37-3, and the cells were then cultured in a medium containing 2 mg/mL G418. The growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance gene had been removed.

Subsequently, the human pVH VH KI vector, which had been linearized with the restriction enzyme SalI, was introduced into the cell line 37-3, and the cells were then cultured in a medium containing 10 µg/mL blasticidin. The growing cells were subjected to genotyping by PCR, so as to confirm that substitution had occurred in a gene locus of interest. Thereafter, Cre-pEGFP-C1 was introduced into the cells, and the resulting cells were then subjected to cell sorting to select GFP-positive cells. Thereafter, the selected cells were subjected to genotyping by PCR, so as to confirm that the drug resistance gene had been removed.

Finally, the human LC pV KI vector, which had been linearized with the restriction enzyme NotI, was introduced into the cell line 37-3, and the cells were then cultured in a medium containing 2 mg/mL G418. The growing cells were subjected to genotyping by PCR, so as to obtain cell lines B-B10 and J-D3, in each of which substitution had occurred in a gene locus of interest (Figure 16 and Figure 17).

### 10. Confirmation of antibody expression (flow cytometry and ELISA)

### 10-1. Analysis by flow cytometry

Based on the method described in 3-1 above, the expression of an antibody in the cells produced in 9 above was confirmed. The results are shown in Figure 18. It was confirmed that only chicken IgM was expressed in wild-type DT40, whereas the expression of chicken IgM disappeared and instead, human Igγ and Igλ were expressed in the transformed cell lines B-B10 and J-D3.

### 10-2. Analysis of ELISA

Based on the method described in 3-2 above, the antibodies secreted from the cells produced in 9 above were measured. The results are shown in Table 7. As with 10-1 above, it was confirmed that only chicken IgM was secreted from wild-type DT40, whereas human IgG₁ was secreted from the transformed cell lines B-B10 and J-D3.

**[Table 7]**

| Cell line | IgG (µg/mL) | IgM (µg/mL) |
|---|---|---|
| Wild type | <0.01 | 2.55 |
| B-B10 | 2.19 | 0.01 |
| J-D3 | 3.45 | <0.01 |

### 11. Confirmation of gene conversion

Using the cell lines in which the expression of an antibody had been confirmed in 10 above, gene conversion was confirmed. The genomic DNA extracted from the cells, which had been cultured in the presence of TSA for 42 days, according to the method described in 4 above was used as a template, and a light chain variable region was amplified by PCR using a sense primer to which a recognition sequence had been added (CGTATCGCCTCCCTCGCGCCATCAGNNNNNNNNNNCAGGTTCCCTGGTGC AGGC) (SEQ ID NO: 46), and an antisense primer (CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56) and an antisense primer to which an identification tag had been added (CGTATCGCCTCCCTCGCGCCATCAGNNNNNNNNNNTGGGGGGGGTTCATA TGAAG) (SEQ ID NO: 57). Thereafter, sequence analyses were carried out according to the method described in 4 above. The results obtained by analyzing the sequences of the light chain and heavy chain variable regions in the cell line B-B10 are shown in Table 8 and Table 9, respectively. A clone comprising the sequence derived from the inserted human pseudogene was confirmed in both of the light chain and heavy chain variable regions, and thus, it was demonstrated that a variety of sequences could be generated as a result of gene conversion. Moreover, it was also confirmed that a variety of sequences could be generated as a result of gene conversion even in the cell line J-D3.

**[Table 8]**

| # | % | CDR1 | CDR2 | CDR3 |
|---|---|---|---|---|
| 1 | 0.183 | VL | pVL003 | VL |
| 2 | 0.046 | Not matched | VL | pVL003 |
| 3 | 0.046 | VL | VL | pVL004 |

| | | | | |
|---|---|---|---|---|
| VL: sequence inserted into variable region pVL001-005: sequences inserted into pseudogene region Not matched: sequence that is not completely matched with either VL or pVL (which is probably caused by partial genetic recombination or mutation) | | | | |

**[Table 9]**

| # | % | CDR1 | CDR2 | CDR3 |
|---|---|---|---|---|
| 1 | 11.176 | pVH004 | VH | VH |
| 2 | 0.131 | pVH001 | VH | VH |
| 3 | 0.131 | pVH004 | Not matched | VH |
| 4 | 0.065 | pVH003 | VH | VH |

| | | | | |
|---|---|---|---|---|
| VH: sequence inserted into variable region pVH001-005: sequences inserted into pseudogene region Not matched: sequence that is not completely matched with either VL or pVL (which is probably caused by partial genetic recombination or mutation) | | | | |

### Production of L15/H15 cell line, in which 15 human variable region-derived sequences have been inserted into each of chicken antibody light chain and heavy chain gene loci

### 12. Production of vectors

### 12-1. Knocking-in into chicken antibody light chain (Igλ,) variable and constant regions

Based on the method described in 1-1 above, a targeting vector, Lambda-VL-CL KI vector 2.0, shown in Figure 19 was produced. (1) Variable region and constant region of human Igλ

The variable region and the constant region produced in 1-1 above were used.

### (2) Left Arm

The Left Arm produced in 1-1 above was used.

### (3) Center Arm

The Center Arm produced in 1-1 above was used.

### (4) Right Arm

Based on the sequence of a region downstream of the constant region of a chicken antibody light chain, which was determined in 1-1 above, Right Arm was synthesized. (5) Construction of targeting vector

The above-described Left Arm, human Igλ variable region, Center Arm, human Igλ constant region, and Right Arm were incorporated into pBluescript to have the structure shown in Figure 19, so as to obtain Lambda-VL-CL KI vector 2.0 (SEQ ID NO: 58).

12-2. Knock-out of chicken antibody light chain (Igλ) pseudogene (1) Human Igλ variable region

The variable region sequence described in 1-1 above was used.

### (2) Left Arm

The Left Arm described in 1-2 above was used.

### (3) Center Arm

The Left Arm described in 1-1 above was used as Center Arm.

### (4) Right Arm

The Center Arm described in 1-1 above was used as Right Arm.

### (5) Multi-cloning site

For insertion of a human antibody light chain pseudogene sequence pVL15, a multi-cloning site shown in SEQ ID NO: 59 was designed. (6) Construction of targeting vector

The above-described Left Arm, neomycin resistance gene, multi-cloning site, Center Arm, human Igλ variable region, blasticidin resistance gene, and Right Arm were incorporated into pUC19, so as to construct a targeting vector pVL KI_pUC19_step5 _BrRev (SEQ ID NO: 60), as shown in Figure 19.

### 12-3. Knocking-in into chicken antibody light chain (Igλ) pseudogene and variable regions

### (1) Human antibody light chain pseudogene sequence (pVL15)

Based on the sequences of human immunoglobulin λ variable regions in the database, 15 CDR1, CDR2 and CDR3 sequences were selected, and the framework sequence of the human Igλ variable region cloned in 1-1 above were then combined therewith, so as to design 15 sequences, namely, pVL101 (SEQ ID NO: 74), pVL102 (SEQ ID NO: 75), pVL103 (SEQ ID NO: 76), pVL104 (SEQ ID NO: 77), pVL105 (SEQ ID NO: 78), pVL106 (SEQ ID NO: 79), pVL107 (SEQ ID NO: 80), pVL108 (SEQ ID NO: 81), pVL109 (SEQ ID NO: 82), pVL110 (SEQ ID NO: 83), pVL111 (SEQ ID NO: 84), pVL112 (SEQ ID NO: 85), pVL113 (SEQ ID NO: 86), pVL114 (SEQ ID NO: 87), and pVL115 (SEQ ID NO: 88). A human antibody light chain pseudogene sequence pVL15 (SEQ ID NO: 61) comprising these sequences was designed, and was then subjected to gene synthesis.

### (2) Construction of targeting vector

The above-described human antibody light chain pseudogene sequence pVL15 was incorporated into the multi-cloning site of the pVL KI_pUC19_step6 _BrRev produced in 12-2 above, so as to construct a targeting vector pVL KI_pUC19_step6 _BrRev_pVL#odd (SEQ ID NO: 62) as shown in Figure 19.

### 12-4. Knocking-in into chicken antibody heavy chain (IgM) constant region

The human CH KI vector produced in 8-2 above was used.

### 12-5. Knocking-in into chicken antibody heavy chain (IgM) variable region (1) Human IgG₁ variable region

The variable region sequence described in 1-6 above was used.

### (2) Left Arm

The Center Arm described in 1-6 above was used.

### (3) Right Arm

The Right Arm described in 1-6 above was used.

### (4) Construction of targeting vector

The above-described Left Arm, human IgG₁ variable region, blasticidin resistance gene, and Right Arm were incorporated into pUC19, so as to produce a targeting vector H(X)0 · hV(B)_074-009/No4 (SEQ ID NO: 63) as shown in Figure 20.

### 12-6. Knocking-in into chicken antibody heavy chain (IgM) pseudogene region (1) Human antibody heavy chain pseudogene sequence (pVH15)

Based on the sequences of human immunoglobulin heavy chain variable regions in the database, 15 CDR1, CDR2 and CDR3 sequences were selected, and the framework sequence of the human IgG₁ variable region cloned in 1-6 above was then combined therewith, so as to design 15 sequences, namely, pVH101 (SEQ ID NO: 94), pVH102 (SEQ ID NO: 95), pVH103 (SEQ ID NO: 96), pVH104 (SEQ ID NO: 97), pVH105 (SEQ ID NO: 98), pVH106 (SEQ ID NO: 99), pVH107 (SEQ ID NO: 100), pVH108 (SEQ ID NO: 101), pVH109 (SEQ ID NO: 102), pVH110 (SEQ ID NO: 103), pVH111 (SEQ ID NO: 104), pVH112 (SEQ ID NO: 105), pVH113 (SEQ ID NO: 106), pVH114 (SEQ ID NO: 107), and pVH115 (SEQ ID NO: 108). Thereafter, a human antibody heavy chain pseudogene sequence pVH15 (SEQ ID NO: 64) was designed by ligating these sequences to one another, and was then subjected to gene synthesis.

### (2) Left Arm

The Left Arm described in 1-6 above was used.

### (3) Right Arm

The Center Arm described in 1-6 above was used.

### (4) Multi-cloning site

For insertion of the human antibody heavy chain pseudogene sequence, a multi-cloning site shown in SEQ ID NO: 65 was designed.

### (5) Site-specific recombination using Cre recombinase (RMCE method)

It has been known that if a pseudogene sequence region is prolonged, the transduction efficiency of homologous recombination is reduced. Thus, in order to easily insert a pseudogene sequence using Cre recombinase, an RMCE method was applied. First, a cassette sequence for RMCE comprising a loxm3 rev sequence and a loxm7 rev RE sequence was designed. (6) Construction of targeting vector

The Left Arm, neomycin resistance gene, cassette sequence for RMCE, multi-cloning site, Right Arm, and human IgG₁ variable region were incorporated into pUC19, and thereafter, the human antibody heavy chain pseudogene region pVH15 was incorporated into the multi-cloning site, so as to produce a targeting vector pVH KI_pUC19_step3_pVH#odd_15pVH_RMCE (SEQ ID NO: 66) as shown in Figure 20.

### 13. Production of cell lines

The Lambda-VL-CL KI vector 2.0 prepared in 12-1 above, the pVL KI_pUC19_step5 _BrRev prepared in 12-2 above, the pVL KI_pUC19_step6 _BrRev_pVL#odd prepared in 12-3 above, the human CH KI vector prepared in 12-4 above, the H(X)0 · hV(B)_074-009/No4 prepared in 12-5 above, and the pVH KI_pUC19_step3_pVH#odd_15pVH_RMCE prepared in 12-6 above were each introduced into DT40 cells, so as to produce cell lines.

First, the human CH KI vector, which had been linearized with the restriction enzyme SalI, was introduced into the cells, and the obtained mixture was then cultured in a medium containing 2 mg/mL G418. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance gene had been removed.

Subsequently, the Lambda-VL-CL KI vector 2.0, which had been linearized with the restriction enzyme NotI was introduced into DT40, and the obtained mixture was then culture in a medium containing 10 µg/mL blasticidin. Thereafter, the growing cells were subjected to genotyping by PCR, so as to confirm that substitution had occurred in a gene locus of interest. Thereafter, Cre_EGFP-C1 was introduced into the cells, and the resulting cells were then subjected to cell sorting to select GFP-positive cells. Thereafter, the selected cells were subjected to genotyping by PCR, so as to confirm that the drug resistance gene had been removed.

Thereafter, the pVL KI_pUC19_step5_BrRev, which had been linearized with the restriction enzyme NotI, was introduced into the cells, and the obtained mixture was then cultured in a medium containing 2 mg/mL G418 and 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance genes had been removed.

Thereafter, the pVL KI_pUC19_step6_BrRev_pVL#odd, which had been linearized with the restriction enzyme NotI, was introduced into the cells, and the obtained mixture was then cultured in a medium containing 2 mg/mL G418 and 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance genes had been removed.

Finally, the H(X)0 · hV(B)_074-009/No4 linearized with the restriction enzyme ScaI and pVH KI_pUC19_step3_pVH#odd_15pVH_RMCE linearized with the restriction enzyme NotI were introduced into the cells, and the obtained mixture was then cultured in a medium containing 2 mg/mL G418 and 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to obtain cell lines A12-3, A12-4, A12-5, B7-3, B7-9 and B7-11, from each of which the drug resistance genes had been removed.

### 14. Confirmation of antibody expression (flow cytometry and ELISA)

### 14-1. Analysis by flow cytometry

Based on the method described in 3 above, the expression of an antibody in the cells produced in 13 above was confirmed. The results are shown in Figure 21 and

Figure 22. It was confirmed that only chicken IgM was expressed in wild-type DT40, whereas the expression of chicken IgM disappeared and instead, human Igγ and Igλ were expressed in the transformed cell lines A12-3, A12-4, A12-5, B7-3, B7-9 and B7-11 (Figures 21 and 22). It is to be noted that since the histogram of Igλ in A12-5 was incorrect in Figure 21, Figure 22 including correct data obtained before the priority date was attached herewith.

### 14-2. Analysis by ELISA

Based on the method described in 3-2 above, the antibodies secreted from the cells produced in 13 above were measured. The results are shown in Table 10. As with 10-1 above, it was confirmed that only chicken IgM was secreted from wild-type DT40, whereas human IgG₁ was secreted from the transformed cell lines A12-3, A12-4, A12-5, B7-3, B7-9 and B7-11.

**[Table 10]**

| Cell line | IgG (µg/mL) | IgM (µg/mL) |
|---|---|---|
| Wild type | <0.01 | 2.54 |
| A12 3 | 0.52 | <0.01 |
| A124 | 0.51 | <0.01 |
| A12 5 | 0.65 | 0.01 |
| B7 - 3 | 0.62 | 0.01 |
| B7 9 | 0.76 | 0.02 |
| B7-11 | 0.77 | 0.02 |

### 15. Confirmation of gene conversion

Using the cell lines in which the expression of an antibody had been confirmed in 10 above, gene conversion was confirmed. Using, as a template, the genomic DNA extracted from the cells, which had been cultured for 21 days or 42 days in the presence of TSA, by the method described in 4 above, a light chain variable region was amplified by PCR using a sense primer to which a recognition sequence had been added (CGTATCGCCTCCCTCGCGCCATCAGNNNNNNNNNNCAGGTTCCCTGGTGC AGGC) (SEQ ID NO: 46), and an antisense primer (CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56) and an antisense primer to which an identification tag had been added (CGTATCGCCTCCCTCGCGCCATCAGNNNNNNNNNNTGGGGGGGGTTCATA TGAAG) (SEQ ID NO: 57). Thereafter, sequence analyses were carried out based on the method described in 4 above. The results of the sequence analysis performed on the variable regions of the light chain and heavy chain in the cell line B7-11 cultured in the presence of TSA for 21 days are shown in Table 11 and Table 12, respectively. The results (the top 10) of the sequence analysis performed on the variable regions of the light chain and heavy chain in the cell line B7-11 cultured in the presence of TSA for 42 days are shown in Table 13 and Table 14, respectively. A clone comprising a sequence derived from a human pseudogene was found in the variable regions of both of the light chain and the heavy chain, and it was demonstrated that a variety of sequences were generated as a result of gene conversion. In addition, it was also confirmed that a variety of sequences were generated as a result of gene conversion even in other cell lines.

**[Table 11]**

| # | % | CDR1 | CDR2 | CDR3 |
|---|---|---|---|---|
| 1 | 0.327 | VL | pVL103 | VL |
| 2 | 0.327 | VL | pVL102 | VL |
| 3 | 0.196 | VL | VL | pVL114 |
| 4 | 0.196 | VL | pVL105 | VL |
| 5 | 0.131 | VL | pVL104 | VL |
| 6 | 0.131 | VL | VL | pVL112 |
| 7 | 0.131 | pVL105 | VL | VL |
| 8 | 0.065 | VL | Not matched | pVL106 |
| 9 | 0.065 | VL | pVL109 | VL |
| 10 | 0.065 | VL | VL | pVL110 |
| 11 | 0.065 | VL | pNL107 | VL |

| | | | | |
|---|---|---|---|---|
| VL: sequence inserted into variable region pVL101-115: sequences inserted into pseudogene region Not matched: sequence that is not completely matched with either VL or pVL (which is probably caused by partial genetic recombination or mutation) | | | | |

**[Table 12]**

| # | % | CDR1 | CDR2 | CDR3 |
|---|---|---|---|---|
| 1 | 0.46 | pVH105 | VH | VH |
| 2 | 0.345 | pVH108 | VH | VH |
| 3 | 0.345 | VH | pVH107 | Not matched |
| 4 | 0.345 | pVH107 | VH | VH |
| 5 | 0.345 | pVH111 | pVH111 | VH |
| 6 | 0.46 | pVH112 | Not matched | VH |
| 7 | 0.23 | pVH109 | VH | VH |
| 8 | 0.23 | VH | pVH112 | VH |
| 9 | 0.23 | pVH105 | Not matched | VH |
| 10 | 0.115 | pVH108 | pVH108 | VH |
| 11 | 0.115 | VH | VH | pVH113 |
| 12 | 0.115 | VH | VH | pVH109 |
| 13 | 0.115 | pVH107 | VH | Not matched |
| 14 | 0.115 | pVH103 | Not matched | VH |
| 15 | 0.23 | pVH111 | Not matched | VH |
| 16 | 0.23 | pVH111 | Not matched | Not matched |
| 17 | 0.115 | pVH111 | VH | VH |

| | | | | |
|---|---|---|---|---|
| VL: sequence inserted into variable region pVL101-115: sequences inserted into pseudogene region Not matched: sequence that is not completely matched with either VL or pVL (which is probably caused by partial genetic recombination or mutation) | | | | |

**[Table 13]**

| # | % | CDR1 | CDR2 | CDR3 |
|---|---|---|---|---|
| 1 | 0.175 | VL | pVL109 | VL |
| 2 | 0.175 | pVL105 | VL | VL |
| 3 | 0.175 | pVL105 | pVL105 | VL |
| 4 | 0.116* | VL | pVL105 | VL |
| 5 | 0.116* | VL | pVL105 | VL |
| 6 | 0.116 | Not matched | pVL105 | VL |
| 7 | 0.058 | VL | pVL109 | Not matched |
| 8 | 0.058 | VL | VL | pVL114 |
| 9 | 0.058 | VL | pVL102 | VL |
| 10 | 0.058 | VL | pVL108 | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) *: Sequences of portions other than CDRs are different, respectively. | | | | |

**[Table 14]**

| # | % | CDR1 | CDR2 | CDR3 |
|---|---|---|---|---|
| 1 | 1.789 | pVH108 | VH | VH |
| 2 | 1.022 | pVH 112 | VH | VH |
| 3 | 0.681 | pVH109 | VH | VH |
| 4 | 0.596 | VH | pVH112 | VH |
| 5 | 0.596 | pVH111 | Not matched | VH |
| 6 | 0.511 | pVH107 | VH | VH |
| 7 | 0.426 | pVH111 | pVH111 | VH |
| 8 | 0.341 | pVH111 | VH | VH |
| 9 | 0.256 | pVH108 | pVH108 | VH |
| 10 | 0.256 | VH | pVH107 | Not matched |

| | | | | |
|---|---|---|---|---|
| VH: sequence inserted into variable region pVH sequence inserted into pseudogene region Not matched: sequence that is not completely matched with either VH or pVH (winch is probably caused by partial genetic recombination or mutation) | | | | |

### Production of L15/H30 cell line, in which 15 pseudogene regions each consisting of human variable region-derived sequence have been inserted into light chain and 30 such pseudogene regions have been inserted into heavy chain

### 16. Production of vectors

### 16-1. Knocking-in into chicken antibody light chain (Igλ) variable and constant regions

The Lambda-VL-CL KI vector 2.0 produced in 12-1 above was used.

### 16-2. Knock-out of chicken antibody light chain (Igλ) pseudogene

The pVL KI_pUC19_step5_BrRev produced in 12-2 above was used.

### 16-3. Knocking-in into chicken antibody light chain (Igλ) pseudogene and variable regions

The pVL KI_pUC19_step6 _BrRev_pVL#odd produced in 12-3 above was used.

### 16-4. Knocking-in into chicken antibody heavy chain (IgM) constant region

The human CH KI vector produced in 8-2 above was used.

### 16-5. Knocking-in into chicken antibody heavy chain (IgM) variable region (1) Human IgG₁ variable region

The variable region sequence described in 1-6 above was used.

### (2) Left Arm

The Left Arm described in 1-6 above was used.

### (3) Center Arm

The Center Arm described in 1-6 above was used.

### (4) Right Arm

The Right Arm described in 1-6 above was used.

### (5) Cassette sequence for RMCE

The cassette sequence for RMCE designed in 12-6 above was used.

### (6) Construction of targeting vector

The above-described Left Arm, neomycin resistance gene, cassette sequence for RMCE, Center Arm, human IgG₁ variable region, and Right Arm were incorporated into pUC19, so as to produce a targeting vector pVH KI_pUC19_step5_BsrRev_RMCE_cassette (SEQ ID NO: 109) as shown in Figure 23.

### 16-6. Knocking-in into chicken antibody heavy chain (IgM) pseudogene region (1) Human antibody heavy chain pseudogene sequence (pVH30)

Based on the sequences of human immunoglobulin heavy chain variable regions in the database, 30 CDR1, CDR2 and CDR3 sequences were selected, and the framework sequence of the human IgG₁ variable region cloned in 1-6 above was then combined therewith, so as to design 30 sequences, namely, pVH101 (SEQ ID NO: 94), pVH102 (SEQ ID NO: 95), pVH103 (SEQ ID NO: 96), pVH104 (SEQ ID NO: 97), pVH105 (SEQ ID NO: 98), pVH106 (SEQ ID NO: 99), pVH107 (SEQ ID NO: 100), pVH108 (SEQ ID NO: 101), pVH109 (SEQ ID NO: 102), pVH110 (SEQ ID NO: 103), pVH111 (SEQ ID NO: 104), pVH112 (SEQ ID NO: 105), pVH113 (SEQ ID NO: 106), pVH114 (SEQ ID NO: 107), pVH115 (SEQ ID NO: 108), pVH116 (SEQ ID NO: 110), pVH117 (SEQ ID NO: 111), pVH118 (SEQ ID NO: 112), pVH119 (SEQ ID NO: 113), pVH120 (SEQ ID NO: 114), pVH121 (SEQ ID NO: 115), pVH122 (SEQ ID NO: 116), pVH123 (SEQ ID NO: 117), pVH124 (SEQ ID NO: 118), pVH125 (SEQ ID NO: 119), pVH126 (SEQ ID NO: 120), pVH127 (SEQ ID NO: 121), pVH128 (SEQ ID NO: 122), pVH129 (SEQ ID NO: 123), and pVH130 (SEQ ID NO: 124). Thereafter, a human antibody heavy chain pseudogene sequence pVH30 (SEQ ID NO: 125) was designed by ligating these sequences to one another, and was then subjected to gene synthesis.

### (2) Left Arm

The Left Arm described in 1-6 above was used.

### (3) Right Arm

The Right Arm described in 1-6 above was used.

### (4) Multi-cloning site

For insertion of the human antibody heavy chain pseudogene sequence, a multi-cloning site shown in SEQ ID NO: 65 was designed. (5) Construction of targeting vector

The Left Arm, neomycin resistance gene, multi-cloning site, Right Arm, human IgG₁ variable region were incorporated into pUC19, and thereafter, the human antibody heavy chain pseudogene region pVH30 was incorporated into the multi-cloning site, so as to produce a targeting vector pVH KI_pUC19_step3_pVH#odd_even (SEQ ID NO: 126) as shown in Figure 24.

### 17. Production of cell lines

The Lambda-VL-CL KI vector 2.0 produced in 12-1 above, the pVL KI_pUC19_step5_BrRev produced in 12-2 above, the pVL KI_pUC19_step6_BrRev_pVL#odd produced in 12-3 above, the human CH KI vector produced in 12-4 above, the pVH KI_pUC19_step5_BsrRev_RMCE_cassette produced in 16-5 above, and the pVH KI_pUC19_step3_pVH#odd_even produced in 16-6 above were each introduced into DT40 cells, so as to produce cell lines.

First, the human CH KI vector, which had been linearized with the restriction enzyme SalI, was introduced into the cells, and the obtained mixture was then cultured in a medium containing 2 mg/mL G418. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance gene had been removed.

Subsequently, the Lambda-VL-CL KI vector 2.0, which had been linearized with the restriction enzyme NotI, was introduced into DT40 cells, and the obtained mixture was then cultured in a medium containing 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance gene had been removed.

Thereafter, the pVL KI_pUC19_step5_BrRev, which had been linearized with the restriction enzyme NotI, was introduced into the cells, and the obtained mixture was then cultured in a medium containing 2 mg/mL G418 and 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance genes had been removed.

Thereafter, the pVL KI_pUC19_step6_BrRev_pVL#odd, which had been linearized with the restriction enzyme NotI, was introduced into the cells, and the obtained mixture was then cultured in a medium containing 2 mg/mL G418 and 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance genes had been removed.

Further, the pVH KI_pUC19_step5_BsrRev_RMCE_cassette, which had been linearized with the restriction enzyme NotI, was introduced into the cells, and the obtained mixture was then cultured in a medium containing 2 mg/mL G418 and 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance genes had been removed.

Finally, the pVH KI_pUC19_step3_pVH#odd_even, which had been linearized with the restriction enzyme NotI, was introduced into the cells, and the obtained mixture was then cultured in a medium containing 2 mg/mL G418. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to obtain cell lines #7-7, #11-3 and #11-6, from each of which the drug resistance gene had been removed.

### 18. Confirmation of antibody expression (flow cytometry and ELISA)

### 18-1. Analysis by flow cytometry

Based on the method described in 3 above, the expression of an antibody in the cells produced in 16 above was confirmed. The results are shown in Figure 25. It was confirmed that only chicken IgM was expressed in wild-type DT40, whereas the expression of chicken IgM disappeared and instead, human Igγ and Igλ were expressed in the transformed cell lines #7-7, #11-3 and #11-6 (Figure 25).

### 18-2. Analysis by ELISA

The antibodies secreted from the cells produced in 17 above were measured. The measurement of human IgG was carried out based on the method described in 3-2 above. The method for measuring chicken IgM is as follows.

That is, 20 µL of 1.0 µg/mL Goat anti-chicken IgM (Bethyl, A30-102A) was dispensed on an immuno 384-well plate Maxisorp (Nunc, 464718), and it was then reacted at room temperature for 1 hour or more, so that it was immobilized on the plate. Thereafter, the resultant was washed with a washing solution (PBS containing 0.05% Tween 20) five times, and 50 µL of a blocking solution (PBS containing 1% BSA) was then added thereto. The obtained mixture was reacted at room temperature for 30 minutes. Thereafter, the resultant was washed with a washing solution five times, and 20 µL of a measurement sample or chicken serum (GIBCO, 16110) serving as a standard substance was then added thereto. The obtained mixture was reacted at room temperature for 1 hour. The resultant was washed with a washing solution five times, and 20 µL of Goat anti-chicken IgM HRP conjugated (Bethyl, A30-102P), which had been 5000 times diluted with PBS containing 1 % BSA and 0.05% Tween 20, was then added thereto. The obtained mixture was reacted at room temperature for 1 hour. Thereafter, the reaction mixture was washed with a washing solution five times, 20 µL of TMB+ (Dako, S159985) was then added thereto, and the obtained mixture was subjected to a coloring reaction at room temperature for 3 minutes. Subsequently, 20 µL of 1 N sulfuric acid was added to the reaction mixture to termination the reaction. Using Infinite M1000 (TECAN), the absorbance at 450 nm was measured.

The results are shown in Table 15. It was confirmed that, as with 13-1 above, only chicken IgM was secreted from wild-type DT40, whereas human IgG₁ was secreted from the transformed cell lines #7-7, #11-3 and #11-6.

**[Table 15]**

| | Human IgG1 (µg/mL) | Chicken IgM (µg/mL) |
|---|---|---|
| Wild type | <0.01 | 2.33 |
| #7-7 | 0.13 | <0.01 |
| #11-3 | 0.24 | <0.01 |
| #11-6 | 0.16 | <0.01 |

### 19. Confirmation of gene conversion

Using the cell lines in which the expression of an antibody had been confirmed in 10 above, gene conversion was confirmed. Using, as a template, the genomic DNA extracted from the cells, which had been cultured for 42 days in the presence of TSA, by the method described in 4 above, a light chain variable region was amplified by PCR using a sense primer to which a recognition sequence had been added (CGTATCGCCTCCCTCGCGCCATCAGNNNNNNNNNNCAGGTTCCCTGGTGC AGGC) (SEQ ID NO: 46), and an antisense primer (CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56) and an antisense primer to which an identification tag had been added (CGTATCGCCTCCCTCGCGCCATCAGNNNNNNNNNNTGGGGGGGGTTCATA TGAAG) (SEQ ID NO: 57). Thereafter, sequence analyses were carried out based on the method described in 4 above. The results of the sequence analysis performed on the variable regions of the light chain and heavy chain in the cell line #11-6 (the top 10) are shown in Table 16 and Table 17, respectively. A clone comprising a the additionally inserted human pseudogene-derived sequence was found in the variable regions of both of the light chain and the heavy chain, and it was demonstrated that more various sequences than in the case of L15/H15 cell line were generated as a result of gene conversion. In addition, it was also confirmed that a variety of sequences were generated as a result of gene conversion even in other cell lines.

**[Table 16]**

| # | % | CDR1 | CDR2 | CDR3 |
|---|---|---|---|---|
| 1 | 0.166 | VL | pVL105 | VL |
| 2 | 0.166 | pVL105 | VL | VL |
| 3 | 0.125 | pVL113 | pVL113 | pVL113 |
| 4 | 0.125 | VL | pVL109 | VL |
| 5 | 0.083 | Not matched | pVL105 | VL |
| 6 | 0.083 | VL | pVL104 | pVL104 |
| 7 | 0.083 | VL | pVL113 | VL |
| 8 | 0.083 | pVL114 | pVL114 | pVL114 |
| 9 | 0.042 | pVL109 | pVL109 | pVL109 |
| 10 | 0.042 | pVL115 | pVL115 | pVL115 |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) | | | | |

**[Table 17]**

| # | % | CDR1 | CDR2 | CDR3 |
|---|---|---|---|---|
| 1 | 1.077 | pVH123 | VH | VH |
| 2 | 0.824 | VH | pVH129 | VH |
| 3 | 0.760 | pVH104 | Not matched | VH |
| 4 | 0.507 | pVH123 | Not matched | VH |
| 5 | 0.444 | pVH117 | Not matched | VH |
| 6 | 0.380 | pVH129 | pVH129 | VH |
| 7 | 0 . 380 | pVH126 | pVH112 | VH |
| 8 | 0.380 | VH | pVH111 | Not matched |
| 9 | 0.317 | pVH102 | VH | Not matched |
| 10 | 0.253 | pVH112 | Not matched | VH |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) | | | | |

### Production of L30/H30 cell line, in which 30 pseudogene regions each consisting of human variable region-derived sequence have been inserted into light chain, and 30 such pseudogene regions have been inserted into heavy chain

### 20. Knocking-in into chicken antibody light chain (Igλ) pseudogene and variable regions

### (1) Human antibody light chain pseudogene sequence (pVL30)

Based on the sequences of human immunoglobulin λ variable regions in the database, 30 CDR1, CDR2 and CDR3 sequences were selected, and the framework sequence of the human Igλ variable region cloned in 1-1 above was then combined therewith, so as to design 30 sequences, namely, pVL101 (SEQ ID NO: 74), pVL102 (SEQ ID NO: 75), pVL103 (SEQ ID NO: 76), pVL104 (SEQ ID NO: 77), pVL105 (SEQ ID NO: 78), pVL106 (SEQ ID NO: 79), pVL107 (SEQ ID NO: 80), pVL108 (SEQ ID NO: 81), pVL109 (SEQ ID NO: 82), pVL110 (SEQ ID NO: 83), pVL111 (SEQ ID NO: 84), pVL112 (SEQ ID NO: 85), pVL113 (SEQ ID NO: 86), pVL114 (SEQ ID NO: 87), pVL115 (SEQ ID NO: 88), pVL116 (SEQ ID NO: 127), pVL117 (SEQ ID NO: 128), pVL118 (SEQ ID NO: 129), pVL119 (SEQ ID NO: 130), pVL120 (SEQ ID NO: 131), pVL121 (SEQ ID NO: 132), pVL122 (SEQ ID NO: 133), pVL123 (SEQ ID NO: 134), pVL124 (SEQ ID NO: 135), pVL125 (SEQ ID NO: 136), pVL126 (SEQ ID NO: 137), pVL127 (SEQ ID NO: 138), pVL128 (SEQ ID NO: 139), pVL129 (SEQ ID NO: 140), and pVL130 (SEQ ID NO: 141). Thereafter, a human antibody light chain pseudogene sequence pVL30 (SEQ ID NO: 142) comprising these sequences was designed, and was then subjected to gene synthesis. (2) Construction of targeting vector

The human pseudogene sequence pVL30 synthesized in the above section was incorporated into the multi-cloning site of the pVL KI_pUC19_step6_BrRev in 16-2 above, so as to construct a targeting vector pVL KI_pUC19_step6_BrRev_#odd+even (SEQ ID NO: 143) as shown in Figure 26.

### 21. Production of cell lines

The pVL KI_pUC19_step6_BrRev_#odd+even prepared in 20 above was introduced into the L15/H30 cell line produced in 17 above to produce cell lines.

The pVL KI_pUC19_step6_BrRev_#odd+even, which had been linearized with the restriction enzyme NotI, was introduced into the aforementioned cell line, and the obtained mixture was then cultured in a medium containing 2 mg/mL G418 and 10µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to obtain cell lines #7-7-48-5, #7-7-48-7 and #7-7-48-10, from each of which the drug resistance genes had been removed.

### 22. Confirmation of antibody expression (flow cytometry and ELISA)

### 22-1. Analysis by flow cytometry

Based on the method described in 3 above, the expression of an antibody in the cells produced in 21 above was confirmed. The results are shown in Figure 27. It was confirmed that only chicken IgM was expressed in wild-type DT40, whereas the expression of chicken IgM disappeared and instead, human Igγ and Igλ were expressed in the transformed cell lines #7-7-48-5, #7-7-48-7 and #7-7-48-10 (Figure 27).

### 22-2. Analysis by ELISA

Based on the method described in 18-2 above, the antibodies secreted from the cells produced in 21 above were measured. The results are shown in Table 18. As with 13-1 above, it was confirmed that only chicken IgM was secreted from wild-type DT40, whereas human IgG₁ was secreted from the transformed cell lines #7-7-48-5, #7-7-48-7 and #7-7-48-10.

**[Table 18]**

| | Human IgG1 (µg/mL) | Chicken IgM (µg/mL) |
|---|---|---|
| Wild type | <0.01 | 2.33 |
| #7-7-48-5 | 0.26 | <0.01 |
| #7-7-48-7 | 0.16 | <001 |
| #7-7-48-10 | 0.13 | <0.01 |

### 23. Confirmation of gene conversion

Using the cell lines in which the expression of an antibody had been confirmed in 22 above, gene conversion was confirmed. Using, as a template, the genomic DNA extracted from the cells, which had been cultured for 42 days in the presence of TSA, by the method described in 4 above, a light chain variable region was amplified by PCR using a sense primer to which a recognition sequence had been added (CGTATCGCCTCCCTCGCGCCATCAGNNNNNNNNNNCAGGTTCCCTGGTGC AGGC) (SEQ ID NO: 46), and an antisense primer (CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56), and an antisense primer to which an identification tag had been added (CGTATCGCCTCCCTCGCGCCATCAGNNNNNNNNNNTGGGGGGGGTTCATA TGAAG) (SEQ ID NO: 57). Thereafter, sequence analyses were carried out based on the method described in 4 above. The results of the sequence analysis performed on the variable regions of the light chain and heavy chain in the cell line #7-7-48-10 (the top 10) are shown in Table 19 and Table 20, respectively. A clone comprising the additionally inserted human pseudogene-derived sequence was found in the variable regions of both of the light chain and the heavy chain, and it was demonstrated that more various sequences were generated by increasing the number of pseudogenes. In addition, it was also confirmed that a variety of sequences were generated as a result of gene conversion even in other cell lines.

**[Table 19]**

| # | % | CDR1 | CDR2 | CDR3 |
|---|---|---|---|---|
| 1 | 0.347* | VL | pVL118 | VL |
| 2 | 0.270 | pVL119 | VL | VL |
| 3 | 0.154 | VL | pVL123 | pVL118 |
| 4 | 0.116 | pVL130 | pVL130 | pVL130 |
| 5 | 0.116 | VL | VL | pVL127 |
| 6 | 0.077 | pVL116 | pVL116 | pVL116 |
| 7 | 0.077 | pVL119 | pVL119 | VL |
| 8 | 0.077 | VL | pVL129 | pVL129 |
| 9 | 0.077 | VL | pVL105 | VL |
| 10 | 0.077* | VL | pVL118 | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) *: Sequences of portions other than CDRs are different, respectively. | | | | |

**[Table 20]**

| # | % | CDR1 | CDR2 | CDR3 |
|---|---|---|---|---|
| 1 | 33.191* | pVH104 | Not matched | VH |
| 2 | 8.085 | pVH129 | Not matched | VH |
| 3 | 6.241 | pVH108 | Not matched | VH |
| 4 | 5.106 | pVH111 | Not matched | VH |
| 5 | 3.688** | pVH126 | Not matched | VH |
| 6 | 1.418 | pVH126 | pVH126 | VH |
| 7 | 1.418 | pVH101 | Not matched | VH |
| 8 | 1.277* | pVH104 | Not matched | VH |
| 9 | 1.277** | pVH126 | Not matched | VH |
| 10 | 0.993* | pVH104 | Not matched | VH |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) *: Sequences of CDR2 are different, respectively. **: Sequences of CDR2 are different, respectively. | | | | |

### Production of L30/H15 cell line, in which 30 pseudogene regions each consisting of human variable region-derived sequence have been inserted into light chain, and 15 such pseudogene regions have been inserted into heavy chain

### 24. Production of cell lines

The pVL KI_pUC19_step6_BrRev_#odd+even prepared in 20 above was introduced into the L15/H15 cell line A12-4 produced in 17 above to produce cell lines.

The pVL KI_pUC19_step6_BrRev_pVL#odd+even, which had been linearized with the restriction enzyme NotI, was introduced into the aforementioned cell line, and the obtained mixture was then cultured in a medium containing 2 mg/mL G418 and 10µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to obtain cell lines A77-3 and A77-2, from each of which the drug resistance genes had been removed.

### 25. Confirmation of antibody expression (flow cytometry and ELISA)

### 25-1. Analysis by flow cytometry

Based on the method described in 3 above, the expression of an antibody in the cells produced in 24 above was confirmed. The results are shown in Figure 28. It was confirmed that only chicken IgM was expressed in wild-type DT40, whereas the expression of chicken IgM disappeared and instead, human Igγ and Igλ were expressed in the transformed cell lines A77-3 and A77-2 (Figure 28).

### 25-2. Analysis by ELISA

Based on the method described in 3-2 above, the antibodies secreted from the cells produced in 24 above were measured. The results are shown in Table 21. As with 13-1 above, it was confirmed that only chicken IgM was secreted from wild-type DT40, whereas human IgG₁ was secreted from the transformed cell lines A77-3 and A77-2.

**[Table 21]**

| | Human IgG1 (µg/mL) | Chicken IgM (µg/mL) |
|---|---|---|
| Wild type | <0.01 | 2.33 |
| A77-3 | 0.43 | <0.01 |
| A77-2 | 0.48 | <0.01 |

### Production of L30/H15f15f and L30/H15r15f cell lines, in which 30 pseudogene regions each consisting of human variable region-derived sequence have been inserted into light chain and 30 such pseudogene regions have been inserted into heavy chain according to RMCE method

### 26. Production of vectors

### 26-1. Further insertion of 15 pseudogenes constructed in forward direction into chicken antibody heavy chain (IgM) pseudogene region

### (1) Human antibody heavy chain pseudogene sequence (pVH15a)

The pVH116, pVH117, pVH118, pVH119, pVH120, pVH121, pVH122, pVH123, pVH124, pVH125, pVH126, pVH127, pVH128, pVH129 and pVH130, which had been designed in 16 above, were ligated to one another in the forward direction, so as to produce pVH15a (SEQ ID NO: 144). (2) Construction of vector

Loxm3 rev sequence, Neomycin resistance gene, SV40 early poly A terminator, SV40 polyadenylation region, loxP for RE sequence, pVH15a (forward direction), and loxm7 rev LE sequence were inserted into a pUC57-Amp vector, so as to produce a vector RMCE_pVH1stKI_pVH15evenFor (SEQ ID NO: 145) as shown in Figure 29.

### 26-2. Further insertion of 15 pseudogenes constructed in reverse direction into chicken antibody heavy chain (IgM) pseudogene region

Loxm3 rev sequence, Neomycin resistance gene, SV40 early poly A terminator, SV40 polyadenylation region, loxP for RE sequence, pVH15a (reverse direction), and loxm7 rev LE sequence were inserted into a pUC57-Amp vector, so as to produce a vector RMCE_pVH1stKI_pVH15evenRev (SEQ ID NO: 146) as shown in Figure 29.

### 27. Production of cell lines

An L30/H15f15f cell line was produced by introducing the RMCE_pVH1stKI_pVH15evenFor produced in 26-1 above into the L30/H15 cell line produced in 24 above.

The RMCE_pVH1stKI_pVH15evenFor, together with Cre_EGFP-C1, was introduced into the aforementioned cell line to induce site-specific recombination, and the obtained mixture was then cultured in a medium containing 2 mg/mL G418. Thereafter, the growing cells were subjected to genotyping by PCR, and cell lines 1-6-1 and 1-7-3 were obtained, in which substitution was confirmed to have occurred in a gene locus of interest.

An L30/H15r15f cell line was produced by introducing the RMCE_pVH1stKI_pVH15evenRev produced in 26-2 into the L30/H15 cell line produced in 24 above.

The RMCE_pVH1stKI_pVH15evenRev, together with Cre_pEGFP-C1, was introduced into the aforementioned cell line to induce site-specific recombination, and the obtained mixture was then cultured in a medium containing 2 mg/mL G418. Thereafter, the growing cells were subjected to genotyping by PCR, and cell lines 2-1-1 and 7-3-2 were obtained, in which substitution was confirmed to have occurred in a gene locus of interest.

### 28. Confirmation of antibody expression (flow cytometry and ELISA)

### 28-1. Analysis by flow cytometry

Based on the method described in 3 above, the expression of an antibody in the cells produced in 27 above was confirmed. The results are shown in Figure 30. It was confirmed that only chicken IgM was expressed in wild-type DT40, whereas the expression of chicken IgM disappeared and instead, human Igγ and Igλ were expressed in the transformed cell lines 1-6-1, 1-7-3, 2-1-1 and 7-3-2 (Figure 30).

### 28-2. Analysis by ELISA

Based on the method described in 3-2 above, the antibodies secreted from the cells produced in 27 above were measured. The results are shown in Table 22. As with 13-1 above, it was confirmed that only chicken IgM was secreted from wild-type DT40, whereas human IgG₁ was secreted from the transformed cell lines 1-6-1, 1-7-3, 2-1-1 and 7-3-2.

**[Table 22]**

| | Human IgG1 (µg/mL) | Chicken IgM (µg/mL) |
|---|---|---|
| Wild type | <0.01 | 2.33 |
| 1-6-1 | 0.18 | <001 |
| 1-7-3 | 0.07 | <0.01 |
| 2-1-1 | 0.12 | <0.01 |
| 7-3-2 | 0.18 | <0.01 |

### 29. Confirmation of gene conversion

Using the cell lines in which the expression of an antibody had been confirmed in 10 above, gene conversion was confirmed. Using, as a template, the genomic DNA extracted from the cells, which had been cultured for 42 days in the presence of TSA, by the method described in 4 above, a light chain variable region was amplified by PCR using a sense primer to which a recognition sequence had been added (CGTATCGCCTCCCTCGCGCCATCAGNNNNNNNNNNCAGGTTCCCTGGTGC AGGC) (SEQ ID NO: 46), and an antisense primer (CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56), and an antisense primer to which an identification tag had been added (CGTATCGCCTCCCTCGCGCCATCAGNNNNNNNNNNTGGGGGGGGTTCATA TGAAG) (SEQ ID NO: 57). Thereafter, sequence analyses were carried out based on the method described in 4 above. The results of the sequence analysis performed on the variable regions of the light chain and heavy chain in the cell line 1-6-1 (the top 10) are shown in Table 23 and Table 24, respectively, and the results of the sequence analysis performed on the variable regions of the light chain and heavy chain in the cell line 7-3-2 (the top 10) are shown in Table 25 and Table 26, respectively. A clone comprising the additionally inserted human pseudogene-derived sequence was found in the variable regions of both of the light chain and the heavy chain, and it was demonstrated that a variety of sequences were generated as in the case of the L30/H30 cell line. In addition, it was also confirmed that a variety of sequences were generated as a result of gene conversion even in other cell lines.

**[Table 23]**

| # | % | CDR1 | CDR2 | CDR3 |
|---|---|---|---|---|
| 1 | 0.935 | VL | pVL102 | VL |
| 2 | 0.468 | VL | pVL118 | VL |
| 3 | 0.271 | Not matched | pVL102 | VL |
| 4 | 0.271* | pVL119 | VL | VL |
| 5 | 0.222 | VL | VL | pVL129 |
| 6 | 0.172* | pVL119 | VL | VL |
| 7 | 0.172 | VL | pVL105 | VL |
| 8 | 0.123 | VL | pVL130 | VL |
| 9 | 0.098 | Not matched | pVL122 | VL |
| 10 | 0.098 | VL | pVL109 | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) *: Sequences of portions other than CDRs are different, respectively. | | | | |

**[Table 24]**

| # | % | CDR1 | CDR2 | CDR3 |
|---|---|---|---|---|
| 1 | 6.393* | pVH112 | Not matched | VH |
| 2 | 0.586 | pVH109 | Not matched | VH |
| 3 | 0.479 | pVH108 | Not matched | VH |
| 4 | 0.479 | pVH117 | Not matched | VH |
| 5 | 0.426 | pVH107 | Not matched | VH |
| 6 | 0.320 | pVH124 | Not matched | VH |
| 7 | 0.266* | pVH112 | Not matched | VH |
| 8 | 0.266 | VH | Not matched | pVH109 |
| 9 | 0.160 | Not matched | pVH125 | VH |
| 10 | 0.160* | pVH112 | Not matched | VH |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) *: Sequences of CDR2 are different, respectively. | | | | |

**[Table 25]**

| # | % | CDR1 | CDR2 | CDR3 |
|---|---|---|---|---|
| 1 | 0.293 | VL | pVL109 | VL |
| 2 | 0.267 | pVL119 | VL | VL |
| 3 | 0.187* | pVL105 | VL | VL |
| 4 | 0.160 | VL | VL | pVL129 |
| 5 | 0.133 | VL | pVL101 | VL |
| 6 | 0.133 | VL | pVL118 | VL |
| 7 | 0.133 | VL | VL | pVL127 |
| 8 | 0.107 | pVL119 | pVL119 | VL |
| 9 | 0.080 | VL | VL | pVL123 |
| 10 | 0.080* | pVL105 | VL | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) *: Sequences of portions other than CDRs are different, respectively. | | | | |

**[Table 26]**

| # | % | CDR1 | CDR2 | CDR3 |
|---|---|---|---|---|
| 1 | 0.867* | VH | Not matched | pVH109 |
| 2 | 0.819* | VH | Not matched | pVH109 |
| 3 | 0.819 | VH | Not matched | pVH116 |
| 4 | 0.337 | pVH109 | Not matched | VH |
| 5 | 0.241* | VH | Not matched | pVH109 |
| 6 | 0.193 | pVH105 | Not matched | VH |
| 7 | 0.193 | pVH117 | Not matched | VH |
| 8 | 0.145 | pVH112 | Not matched | VH |
| 9 | 0.145 | pVH126 | Not matched | pVH116 |
| 10 | 0.096 | pVH107 | Not matched | pVH109 |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) *: Sequences of CDR2 are different, respectively. | | | | |

### Production of L30/H15f15f15f and L30/H15r15r15f cell lines, in which 30 pseudogene regions each consisting of human variable region-derived sequence have been inserted into light chain and 45 such pseudogene regions have been inserted into heavy chain according to RMCE method

### 30. Production of vector

### 30-1. Further insertion of 15 pseudogenes constructed in forward direction into chicken antibody heavy chain (IgM) pseudogene region

### (1) Human antibody heavy chain pseudogene sequence (pVH15b)

Based on the sequences of a human immunoglobulin heavy chain variable regions in the database, novel 15 CDR1, CDR2 and CDR 3 sequences were selected, and pVH131 (SEQ ID NO: 147), pVH132 (SEQ ID NO: 148), pVH133 (SEQ ID NO: 149), pVH134 (SEQ ID NO: 150), pVH135 (SEQ ID NO: 151), pVH136 (SEQ ID NO: 152), pVH137 (SEQ ID NO: 153), pVH138 (SEQ ID NO: 154), pVH139 (SEQ ID NO: 155), pVH140 (SEQ ID NO: 156), pVH141 (SEQ ID NO: 157), pVH142 (SEQ ID NO: 158), pVH143 (SEQ ID NO: 159), pVH144 (SEQ ID NO: 160), and pVH145 (SEQ ID NO: 161) were ligated to one another in the forward direction to produce pVH15b (SEQ ID NO: 162).

### (2) Construction of vector

Loxm3 rev sequence, blasticidin resistance gene, SV40 early poly A terminator, SV40 polyadenylation region, loxm7 rev RE sequence, pVH15b (forward direction), and loxP for LE sequence were inserted into a pUC57-Amp vector, so as to produce a vector pVH15B-ver3_RMCE2nd_For (SEQ ID NO: 163) as shown in Figure 31.

### 30-2. Further insertion of 15 pseudogenes constructed in reverse direction into chicken antibody heavy chain (IgM) pseudogene region

Loxm3 rev sequence, blasticidin resistance gene, SV40 early poly A terminator, SV40 polyadenylation region, loxm7 rev RE sequence, pVH15b (reverse direction), and loxP for LE sequence were inserted into a pUC57-Amp vector, so as to produce a vector pVH15B-ver3_RMCE2nd_Rev (SEQ ID NO: 164) as shown in Figure 31.

### 31. Production of cell lines

An L30/H15f15f15f cell line was produced by introducing the pVH15B-ver3_RMCE2nd_For produced in 30-1 above into the L30/H15f15f cell line produced in 2 above.

The pVH15B-ver3_RMCE2nd_For, together with Cre_pEGFP-C1, was introduced into the aforementioned cell line to induce site-specific recombination, and the obtained mixture was then cultured in a medium containing 10 µg/mL blasticidin. Thereafter, the growing cells were subjected to genotyping by PCR, and cell lines 2-1-3, 2-1-11, 1-n-1 and 2-n-3 were obtained, in which substitution was confirmed to have occurred in a gene locus of interest.

An L30/H15r15r15f cell line was produced by introducing the pVH15B-ver3_RMCE2nd_Rev produced in 27-2 above into the L30/H15r15f cell line produced in 27 above.

The pVH15B-ver3_RMCE2nd_Rev, together with Cre_pEGFP-C1, was introduced into the aforementioned cell line to induce site-specific recombination, and the obtained mixture was then cultured in a medium containing 10 µg/mL blasticidin. Thereafter, the growing cells were subjected to genotyping by PCR, and cell lines 4-1-5, 4-1-7 and 3-1-11 were obtained, in which substitution was confirmed to have occurred in a gene locus of interest.

### 32. Confirmation of antibody expression (flow cytometry and ELISA)

### 32-1. Analysis by flow cytometry

Based on the method described in 3 above, the expression of an antibody in the cells produced in 31 above was confirmed. The results are shown in Figure 32 and Figure 33. It was confirmed that only chicken IgM was expressed in wild-type DT40, whereas the expression of chicken IgM disappeared and instead, human Igγ and Igλ were expressed in the transformed cell lines 2-1-3, 2-1-11, 1-n-1 and 2-n-3 (which are shown in Figure 32), and in the transformed cell lines 4-1-5, 4-1-7 and 3-1-11 (which are shown in Figure 33).

### 32-2. Analysis by ELISA

Based on the method described in 3-2 above, the antibodies secreted from the cells produced in 31 above were measured. The results are shown in Table 27. As with 13-1 above, it was confirmed that only chicken IgM was secreted from wild-type DT40, whereas human IgG₁ was secreted from the transformed cell lines 2-1-3, 2-1-11, 1-n-1, 2-n-3, 4-1-5, 4-1-7 and 3-1-11.

**[Table 27]**

| | Human IgG1 (µg/mL) | Chicken IgM (µg/mL) |
|---|---|---|
| Wild type | <0.01 | 2.33 |
| 2-1-3 | 0.36 | <0.01 |
| 2-1-11 | 0.16 | <0.01 |
| 1-n-1 | 0.18 | <0.01 |
| 2-n-3 | 0.16 | <0.01 |
| 4-1-5 | 0.37 | <0.0 1 |
| 4-1-7 | 0.94 | <0.01 |
| 3-1-11 | 1.31 | <0.01 |

### 33. Confirmation of gene conversion

Using the cell lines in which the expression of an antibody had been confirmed in 10 above, gene conversion was confirmed. Using, as a template, the genomic DNA extracted from the cells, which had been cultured for 42 days in the presence of TSA, by the method described in 4 above, a light chain variable region was amplified by PCR using a sense primer to which a recognition sequence had been added (CGTATCGCCTCCCTCGCGCCATCAGNNNNNNNNNNCAGGTTCCCTGGTGC AGGC) (SEQ ID NO: 46), d, and an antisense primer (CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56), and an antisense primer to which an identification tag had been added (CGTATCGCCTCCCTCGCGCCATCAGNNNNNNNNNNTGGGGGGGGTTCATA TGAAG) (SEQ ID NO: 57). Thereafter, sequence analyses were carried out based on the method described in 4 above. The results of the sequence analysis performed on the variable regions of the light chain and heavy chain in the cell line 2-1-3 (the top 10) are shown in Table 28 and Table 29, and the results of the sequence analysis performed on the variable regions of the light chain and heavy chain in the cell line 3-1-11 (the top 10) are shown in Table 30 and Table 31, respectively. A clone comprising the additionally inserted human pseudogene-derived sequence was found in the variable regions of both of the light chain and the heavy chain, and it was demonstrated that a variety of sequences were generated as a result of gene conversion. In addition, it was also confirmed that a variety of sequences were generated as a result of gene conversion even in other cell lines.

**[Table 28]**

| # | % | CDR1 | CDR2 | CDR3 |
|---|---|---|---|---|
| 1 | 0.882* | pVL119 | VL | VL |
| 2 | 0.428** | VL | pVL118 | VL |
| 3 | 0.241 | pVL119 | pVL119 | VL |
| 4 | 0.187** | VL | pVL118 | VL |
| 5 | 0.187** | VL | pVL118 | VL |
| 6 | 0.134 | VL | pVL119 | VL |
| 7 | 0.134 | VL | pVL109 | VL |
| 8 | 0.134 | pVL105 | VL | VL |
| 9 | 0.107* | pVL119 | VL | VL |
| 10 | 0.080 | VL | pVL105 | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) *, **: Sequences of portions other than CDRs are different, respectively. | | | | |

**[Table 29]**

| # | % | CDR1 | CDR2 | CDR3 |
|---|---|---|---|---|
| 1 | 2.226 | pVH112 | Not matched | VH |
| 2 | 1.272 | pVH109 | Not matched | VH |
| 3 | 0.636 | VH | pVH112 | VH |
| 4 | 0.477 | pVH144 | Not matched | VH |
| 5 | 0.477 | pVH105 | Not matched | VH |
| 6 | 0.318 | pVH145 | Not matched | VH |
| 7 | 0.318 | pVH137 | Not matched | VH |
| 8 | 0.318* | pVH108 | Not matched | VH |
| 9 | 0.318* | pVH108 | Not matched | VH |
| 10 | 0.318 | VH | Not matched | pVH116 |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) *: Sequences of CDR2 are different, respectively. | | | | |

**[Table 30]**

| # | % | CDR1 | CDR2 | CDR3 |
|---|---|---|---|---|
| 1 | 1.000 | pVL105 | VL | VL |
| 2 | 0.788 | pVL119 | VL | VL |
| 3 | 0.515* | VL | pVL118 | VL |
| 4 | 0.455* | VL | pVL118 | VL |
| 5 | 0.182 | VL | pVL105 | VL |
| 6 | 0.152** | pVL119 | pVL119 | VL |
| 7 | 0.152 | VL | pVL109 | VL |
| 8 | 0.121 | VL | pVL104 | VL |
| 9 | 0.091** | pVL119 | pVL119 | VL |
| 10 | 0.091 | VL | pVL107 | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) *, **: Sequences of portions other than CDRs are different, respectively. | | | | |

**[Table 31]**

| # | % | CDR1 | CDR2 | CDR3 |
|---|---|---|---|---|
| 1 | 1.524 | pVH109 | Not matched | VH |
| 2 | 1.220 | pVH112 | Not matched | VH |
| 3 | 0.915 | pVH117 | Not matched | VH |
| 4 | 0.915 | pVH142 | Not matched | VH |
| 5 | 0.610 | pVH108 | Not matched | VH |
| 6 | 0.610 | pVH138 | Not matched | VH |
| 7 | 0.610 | pVH107 | Not matched | VH |
| 8 | 0.457 | pVH139 | Not matched | VH |
| 9 | 0.457 | pVH102 | Not matched | VH |
| 10 | 0.457 | pVH142 | Not matched | Not matched |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) | | | | |

### Production of L30/H15f15f15f15f and L30/H15r15r15r15f cell lines, in which 30 pseudogene regions each consisting of human variable region-derived sequence have been inserted into light chain and 60 such pseudogene regions have been inserted into heavy chain according to RMCE method

### 34. Production of vector

### 34-1. Further insertion of 15 pseudogenes constructed in forward direction into chicken antibody heavy chain (IgM) pseudogene region

### (1) Human antibody heavy chain pseudogene sequence (pVH15c)

Based on the sequences of human immunoglobulin heavy chain variable regions in the database, novel 15 CDR1, CDR2 and CDR 3 sequences were selected, and pVH146 (SEQ ID NO: 165), pVH147 (SEQ ID NO: 166), pVH148 (SEQ ID NO: 167), pVH149 (SEQ ID NO: 168), pVH150 (SEQ ID NO: 169), pVH151 (SEQ ID NO: 170), pVH152 (SEQ ID NO: 171), pVH153 (SEQ ID NO: 172), pVH154 (SEQ ID NO: 173), pVH155 (SEQ ID NO: 174), pVH156 (SEQ ID NO: 175), pVH157 (SEQ ID NO: 176), pVH158 (SEQ ID NO: 177), pVH159 (SEQ ID NO: 178), and pVH160 (SEQ ID NO: 179) were ligated to one another in the forward direction to produce pVH15c (SEQ ID NO: 180).

### (2) Construction of vector

Loxm3 rev sequence, neomycin resistance gene, SV40 early poly A terminator, SV40 polyadenylation region, loxP for RE sequence, pVH15c (forward direction), and loxm7 rev LE sequence were inserted into a pUC57-Amp vector, so as to produce a vector pVH15A_RMCE1st_Fw (SEQ ID NO: 181) as shown in Figure 34.

### 34-2. Further insertion of 15 pseudogenes constructed in reverse direction into chicken antibody heavy chain (IgM) pseudogene region

Loxm3 rev sequence, neomycin resistance gene, SV40 early poly A terminator, SV40 polyadenylation region, loxP for RE sequence, pVH15c (reverse direction), and loxm7 rev LE sequence were inserted into a pUC57-Amp vector, so as to produce a vector pVH15A_RMCE1st_Rv (SEQ ID NO: 182) as shown in Figure 34.

### 35. Production of cell lines

An L30/H15f15f15f15f cell line was produced by introducing the pVH15A _RMCE1st_Fw produced in 34-1 above into the L30/H15f15f15f cell line produced in 31 above.

The pVH15A_RMCE1st_Fw, together with Cre_pEGFP-C1, was introduced into the aforementioned cell line to induce site-specific recombination, and the obtained mixture was then cultured in a medium containing 2 mg/mL G418. Thereafter, the growing cells were subjected to genotyping by PCR, and cell lines 1n1-3, lnl-4, and 1n1-5 were obtained, in which substitution was confirmed to have occurred in a gene locus of interest.

An L30/H15r15r15r15f cell line was produced by introducing the pVH15A _RMCE1st_Rv produced in 34-2 above into the L30/H15r15r15f cell line produced in 31 above.

The pVH15A RMCElst Rv, together with Cre_pEGFP-C1, was introduced into the aforementioned cell line to induce site-specific recombination, and the obtained mixture was then cultured in a medium containing 2 mg/mL G418. Thereafter, the growing cells were subjected to genotyping by PCR, and a cell line 3111-1 was obtained, in which substitution was confirmed to have occurred in a gene locus of interest.

### 36. Confirmation of antibody expression (flow cytometry and ELISA)

### 36-1. Analysis by flow cytometry

Based on the method described in 3 above, the expression of an antibody in the cells produced in 35 above was confirmed. The results are shown in Figure 35. It was confirmed that only chicken IgM was expressed in wild-type DT40, whereas the expression of chicken IgM disappeared and instead, human Igγ and Igλ were expressed in the transformed cell lines 1n1-3, 1n1-4, 1n1-5 and 3111-1 (Figure 35).

### 36-2. Analysis by ELISA

Based on the method described in 3-2 above, the antibodies secreted from the cells produced in 35 above were measured. The results are shown in Table 32. As with 13-1 above, it was confirmed that only chicken IgM was secreted from wild-type DT40, whereas human IgG₁ was secreted from the transformed cell lines 1n1-3, 1n1-4, 1n1-5 and 3111-1.

**[Table 32]**

| | Human IgG1 (µg/mL) | Chicken IgM (µg/mL) |
|---|---|---|
| Wild type | <0.01 | 2.33 |
| 1n1-3 | 0.32 | <0.01 |
| 1n1-4 | 0.34 | <0.01 |
| 1n1-5 | 0.22 | <0.01 |
| 3111-1 | 0.49 | <0.01 |

### 37. Confirmation of gene conversion

Using the cell lines in which the expression of an antibody had been confirmed in 10 above, gene conversion was confirmed. Using, as a template, the genomic DNA extracted from the cells, which had been cultured for 42 days in the presence of TSA, by the method described in 4 above, a light chain variable region was amplified by PCR using a sense primer to which a recognition sequence had been added (GTATCGCCTCCCTCGCGCCATCAGNNNNNNNNNNCAGGTTCCCTGGTGCA GGC) (SEQ ID NO: 46), and an antisense primer (CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56), and an antisense primer to which an identification tag had been added (CGTATCGCCTCCCTCGCGCCATCAGNNNNNNNNNNTGGGGGGGGTTCATA TGAAG) (SEQ ID NO: 57). Thereafter, sequence analyses were carried out based on the method described in 4 above. The results of the sequence analysis performed on the variable regions of the light chain and heavy chain in the cell line 1n1-3 (the top 10) are shown in Table 33 and Table 34, and the results of the sequence analysis performed on the variable regions of the light chain and heavy chain in the cell line 3111-1 (the top 10) are shown in Table 35 and Table 36, respectively. A clone comprising the additionally inserted human pseudogene-derived sequence was found in the variable regions of both of the light chain and the heavy chain, and it was demonstrated that more various sequences were generated as a result of gene conversion. In addition, it was also confirmed that a variety of sequences were generated as a result of gene conversion even in other cell lines.

**[Table 33]**

| # | % | CDR1 | CDR2 | CDR3 |
|---|---|---|---|---|
| 1 | 1.217 | Not matched | pVL105 | VL |
| 2 | 0.466* | Not matched | pVL118 | VL |
| 3 | 0.304 | pVL119 | VL | VL |
| 4 | 0.223** | pVL119 | pVL119 | VL |
| 5 | 0.223* | Not matched | pVL118 | VL |
| 6 | 0.162 | Not matched | pVL109 | VL |
| 7 | 0.162 | pVL105 | VL | VL |
| 8 | 0.142 | Not matched | pVL119 | VL |
| 9 | 0.081 | Not matched | VL | pVL129 |
| 10 | 0.061** | pVL119 | pVL119 | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) *: Sequences of CDR2 are different, respectively **: Sequences of portions other than CDRs are different, respectively. | | | | |

**[Table 34]**

| # | % | CDR1 | CDR2 | CDR3 |
|---|---|---|---|---|
| 1 | 63.115* | pVH105 | Not matched | VH |
| 2 | 2.186 | pVH138 | pVH138 | VH |
| 3 | 0.929 | pVH124 | Not matched | VH |
| 4 | 0.820 | pVH112 | Not matched | VH |
| 5 | 0.765* | pVH105 | Not matched | VH |
| 6 | 0.656* | pVH105 | Not matched | VH |
| 7 | 0.601 | pVH155 | Not matched | VH |
| 8 | 0.601* | pVH105 | Not matched | VH |
| 9 | 0.546* | pVH105 | Not matched | VH |
| 10 | 0.492 | pVH156 | Not matched | VH |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) *: Sequences of CDR2 are different, respectively. | | | | |

**[Table 35]**

| # | % | CDR1 | CDR2 | CDR3 |
|---|---|---|---|---|
| 1 | 0.142 | VL | VL | pVL129 |
| 2 | 0.094 | VL | VL | pVL128 |
| 3 | 0.071* | pVL119 | VL | VL |
| 4 | 0.047* | pVL119 | VL | VL |
| 5 | 0.024 | Not matched | pVL107 | pVL107 |
| 6 | 0.024 | pVL108 | pVL123 | pVL123 |
| 7 | 0.024 | pVL108 | pVL108 | pVL123 |
| 8 | 0.024* | pVL119 | VL | VL |
| 9 | 0.024 | VL | VL | pVL125 |
| 10 | 0.024 | VL | VL | pVL130 |

| | | | | |
|---|---|---|---|---|
| VL: sequence inserted into variable region pVL: sequence inserted into pseudogene region Not matched sequence that is not completely matched with either VL or pVL (which is probably caused by partial genetic recombination or mutation) *: Sequences of portions other than CDRs are different, respectively. | | | | |

**[Table 36]**

| # | % | CDR1 | CDR2 | CDR3 |
|---|---|---|---|---|
| 1 | 51.762* | pVH126 | Not matched | VH |
| 2 | 23.429 | pVH108 | Not matched | VH |
| 3 | 1.053 | pVH129 | Not matched | VH |
| 4 | 0.545* | pVH126 | Not matched | VH |
| 5 | 0.436* | pVH126 | Not matched | VH |
| 6 | 0.291 | pVH104 | Not matched | VH |
| 7 | 0.291* | pVH126 | Not matched | VH |
| 8 | 0.291** | pVH126 | Not matched | Not matched |
| 9 | 0.254** | pVH126 | Not matched | Not matched |
| 10 | 0.254* | pVH126 | Not matched | VH |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) *: Sequences of CDR2 are different, respectively. *: Sequences of CDR2 and CDR3 are different, respectively. | | | | |

### Antibody selection 1

### 38. Preparation of antigen

Plexin A4 was selected as an antigen protein. Plexin A4 is a molecule belonging to Plexin A type, and has been known as a factor for controlling axon extension in the sensory nerve or the sympathetic nerve.

As an antigen, Plexin A4, in which FLAG tags were added to the C-termini of a Sema domain and a PSI (Plexin-Semaphorin-Integrin) domain, was used (the amino acid sequence is shown in SEQ ID NO: 68). Based on Accession No. NM_020911 and Accession No. EAW83796, the nucleotide sequence shown in SEQ ID NO: 67 was synthesized, and it was then inserted into an expression vector in which the EBNA-1 gene and Hygromycin resistance gene of pCEP4 (Life Technologies, V044-50) had been deleted, thereby constructing a Plexin A4 expression vector.

The obtained Plexin A4 expression vector was introduced into FreeStyle 293 F cells (Life Technologies, R790-07), using PEI (polyethylenimine), so that it was allowed to express therein. At that time, an expression vector comprising an EBNA-1 gene was also introduced into the cells, separately. The expression was carried out at 3 L-scale, and the culture was carried out for 7 days.

Utilizing FLAG tags, a protein of interest in the culture supernatant was purified. An XK16/20 column (GE Healthcare, 28-9889-37) was filled with 2.5 mL of anti-M2-agarose resin (Sigma, A2220), and it was then equilibrated with D-PBS(-) (Wako Pure Chemical Industries, Ltd., 045-29795). Thereafter, the culture supernatant was loaded thereon, it was washed with D-PBS(-), and it was then eluted with D-PBS(-) containing 0.1 mg/mL FLAG peptide (Sigma, F3290). The resultant was monitored with the absorbance at 280 nm, and a fraction corresponding to the peak was then recovered. The molecular weight of the recovered protein was measured by SDS-PAGE and CBB staining, and it was then confirmed that the obtained molecular weight corresponded to the molecular weight of the protein of interest.

Using an ultrafiltration membrane (Amicon, UFC0801008), approximately 24 mL of the recovered fraction was concentrated to a volume of 2 mL, and then, using Hi Load 16/600 Superdex 200 pg (GE Healthcare, 28-9893-35), the fraction was purified by gel filtration chromatography. The column was equilibrated with D-PBS(-), and the recovered fraction was then loaded thereon, followed by elution with D-PBS(-). The resultant was monitored with the absorbance at 280 nm, and a fraction corresponding to the peak was then recovered. The molecular weight of the recovered protein was measured by SDS-PAGE and CBB staining.

### 39. Selection of antibody

### 39-1. Production of antigen magnetic beads

Using Dynabeads M-270 Carboxylic Acid (Life Technologies, 14305D) and Dynabeads M-270 Epoxy (Life Technologies, 14301) as magnetic beads, these beads were allowed to bind to the antigen in accordance with the instruction manual. At this time, MPC-S (Life Technologies, DBA13346) was used as a magnetic stand. Regarding Dynabeads M-270 Carboxylic Acid, 10 µL of beads were washed with 20 µL of 25 mM MES (pH 5.0) three times, and 10 µL of 50 mg/mL NHS solution and 10 µL of 50 mg/mL EDC solution were added to the resulting solution. The obtained mixture was reacted at room temperature for 30 minutes, and the reaction solution was then washed with 20 µL of 25 mM MES (pH 5.0) twice. A supernatant was removed from this magnetic beads suspension, and 10 µL of Plexin A4 protein solution, which had been adjusted to 0.6 mg/mL with 25 mM MES (pH 5.0), was then added to the suspension. The obtained mixture was reacted at 4°C overnight, while being subjected to rotary stirring. Thereafter, a supernatant was removed from the reaction solution, and 20 µL of quenching buffer (50 mM Tris-HCl) was then added thereto, followed by the reaction of the mixture at room temperature for 15 minutes while being subjected to rotary stirring. Regarding Dynabeads M-270 Epoxy, 10 µL of beads were washed with 20 µL of 100 mM Na-phosphate buffer three times, and thereafter, 13.3 µL of Plexin A4 protein solution adjusted to 0.45 mg/ml with 100 mM Na-phosphate buffer and 6.7 µL of 100 mM Na-phosphate buffer containing 3 M ammonium sulfate were added and suspended therein. The obtained mixture was reacted at 37°C overnight, while being subjected to rotary stirring. After the removal of a supernatant, the two types of beads were collectively suspended in 1 mL of selection buffer (PBS containing 1% BSA), and the obtained suspension was then washed with the same buffer as mentioned above three times. Thereafter, the resultant was suspended in 200 µL of selection buffer, and was then subjected to selection.

### 39-2. Selection by antigen magnetic beads

An L15/H15 cell line, B7-3, was cultured in a medium containing 2.5 ng/mL TSA for 3 weeks. Thereafter, approximately 1 × 10⁸ cells were washed with 10 mL of selection buffer, and a supernatant was then removed from the reaction solution. The remaining solution was washed with 1 mL of selection buffer, and it was then suspended in 950 µL of selection buffer. To this cell suspension, 50 µL of the antigen magnetic beads prepared in 17-1 above was added, and the obtained mixture was then reacted at 4°C for 30 minutes, while being stirred by rotation. Thereafter, using KingFisher mL (Thermo, 5400050), the reaction solution was washed with 0.5 mL of selection buffer three times. Thereafter, the recovered cells were suspended in 20 mL of medium, the suspension was then plated on a 9-cm dish, and it was then cultured in a CO₂ incubator for 7 days.

### 39-3. Screening by flow cytometry

The cell culture solution obtained in 39-2 above was recovered, and 3 × 10⁶ cells were then fractionated into a 1.5-mL tube. The cells were centrifuged to remove a supernatant, and the resulting cells were then washed with 1.5 mL of PBS to remove a supernatant. Using EZ-Link (registered trademark) NHS-PEG4-Biotinylation Kit (PIERCE, 21455), a biotin-labeled Plexin A4 protein was prepared, and 300 µL of primary staining solution (2.5 µg/mL biotin-labeled Plexin A4 protein solution was then added to the protein. The obtained mixture was left at rest at 4°C under light-shielded conditions for 60 minutes. Thereafter, a supernatant was removed by centrifugation, and the resultant was then washed with 500 µL of PBS twice. After that, 300 µL of secondary staining solution, in which Goat Anti-Human IgG gamma chain specific R-FITC conjugated (Southern biotech, A2040-02) and Streptavidin PE conjugated (eBioscience, 12-4317-87) had been each diluted with buffer 1000 times and 500 times, respectively, was added to the resultant, and the obtained mixture was then left at rest at 4°C under light-shielded conditions for 60 minutes. Thereafter, a supernatant was removed by centrifugation, and the resultant was then washed with 500 µL of PBS twice. The resultant was suspended in 500 µL of FACS buffer (containing PI solution (Invitrogen, P3566), which had been 1000 times diluted with PBS). This sample was subjected to a flow cytometric analysis. The results are shown in Figure 36. A cell population, in which the signals of both Plexin A4 protein and IgGγ were high, was discovered in the P5 area.

### 39-4. Screening by antigen-immobilized ELISA method

The cell culture supernatant obtained in 39-3 above was recovered, and a clone reacting with a Plexin A4 antigen was screened by an antigen-immobilized ELISA method using such a Plexin A4 antigen.

First, 20 µL of solution comprising a 2.5 µg/mL Plexin A4 antigen protein and a negative control that was a trypsin inhibitor (Sigma, T6522), streptavidin (Nacalai Tesque Inc., 32243) or ovalbumin (Sigma, A5503) was dispensed in an immuno 384-well plate Maxisorp (Nunc, 464718), and it was then reacted at 4°C overnight, so that it was immobilized on the plate. Thereafter, the reaction mixture was washed with a washing solution (PBS containing 0.05% Tween 20) three times, and 45 µL of blocking solution (PBS containing 1% BSA) was then added thereto. The obtained mixture was reacted at room temperature for 1 hour. The resultant was washed with a washing solution three times, 25 µL of a measurement sample was then added thereto, and the obtained mixture was then reacted at room temperature for 1 hour. The resultant was washed with a washing solution three times, 25 µL of Goat anti-Human IgG-Fc HRP-conjugated (Bethyl, A80-104P), which had been 2000 times diluted with PBS buffer, was then added thereto, and the obtained mixture was then reacted for 1 hour. The resultant was washed with a washing solution three times, 25 µL of TMB+ (Dako, S159985) was then added thereto, and the obtained mixture was then subjected to a coloring reaction for 5 minutes. Thereafter, 25 µL of 1 M sulfuric acid was added to the reaction mixture, so as to terminate the reaction. Using Infinite M1000 (TECAN), the absorbance at 450 nm was measured. The results are shown in Figure 37. It was demonstrated that an antibody specifically reacting with Plexin A4 was generated in almost all of the clones.

According to screening in which the flow cytometry described in 39-3 above was combined with the antigen-immobilized ELISA method described in 39-4 above, a positive clone specifically binding to Plexin A4 could be identified. In addition to the case of B7-3, even in selections in which the L15/H30 cell line #11-6 and the L30/H30 cell line 7-7-48-10 were used, a positive clone specifically binding to Plexin A4 could be identified.

### 40. Confirmation of expression of anti-Plexin A4 antibody

### 40-1. Analysis by ELISA

Among the positive clones identified in 39-5above, regarding the positive clones #62 and #20, the concentrations of chicken IgM and human IgG₁ secreted into the culture supernatant were measured. Each cell line was suspended in a medium containing no chicken serum to result in 4 × 10⁵ cells/mL, and 1 mL each of the suspension was plated on a 24-well plate. The cells were cultured for 3 days, and the culture solution was recovered and was then filtrated through a 0.22-µm filter. Thereafter, the resulting cells were used in the measurement.

The method for measuring IgG is as follows. 100 µL of Goat anti-Human IgG-Fc affinity purified (Bethyl, A80-104A), which had been adjusted to 1.0 µg/mL by dilution with PBS, was dispensed in F96 Maxisorp Nunc Immunoplate (Nunc, 464718), and it was then reacted at 4°C for 1 hour, so that it was immobilized on the plate. Thereafter, the reaction mixture was washed with a washing solution (PBS containing 0.05% Tween 20) five times, and 200 µL of blocking solution (PBS containing 1% BSA) was then added thereto. The obtained mixture was reacted at room temperature for 95 minutes. The resultant was washed with a washing solution five times, and 100 µL of a measurement sample or Human IgG1 Lambda-UNLB (Southern Biotech, 0151L-01) serving as a standard substance was then added thereto. The obtained mixture was reacted at room temperature for 1hour. The resultant was washed with a washing solution five times, and 100 µL of Goat anti-Human IgG-Fc HRP conjugated (Bethyl, A80-104P), which had been 1000 times diluted with PBS, was then added thereto. The obtained mixture was reacted at room temperature for 1 hour. The resultant was washed with a washing solution three times, and 100 µL of TMB+ (Dako, S159985) was then added thereto. The obtained mixture was subjected to a coloring reaction at room temperature for 3 minutes, and 20 µL of 1 M sulfuric acid was then added thereto, so as to terminate the reaction. Using Infinite M1000 (TECAN), the absorbance at 450 nm was measured.

The method for measuring IgM is as follows. 100 µL of Goat anti-chicken IgM (Bethyl, A30-102A), which had been diluted with PBS to 1.0 µg/mL, was dispensed in F96 Maxisorp Nunc Immunoplate (Nunc, 439454), and it was then reacted at 4°C overnight, so that it was immobilized on the plate. Thereafter, the reaction mixture was washed with a washing solution (PBS containing 0.05% Tween 20) five times, and 200 µL of blocking solution (PBS containing 1% BSA) was then added thereto. The obtained mixture was reacted at room temperature for 95 minutes. The resultant was washed with a washing solution five times, and 100 µL of chicken serum (GIBCO, 16110) serving as a measurement sample or a standard substance was added thereto. The obtained mixture was reacted at room temperature for 1 hour. The resultant was washed with a washing solution five times, and 100 µL of Goat anti-chicken IgM HRP conjugated (Bethyl, A30-102P), which had been 5000 times diluted with PBS containing 1% BSA and 0.05% Tween 20, was then added thereto. The obtained mixture was reacted at room temperature for 1 hour. The resultant was washed with a washing solution five times, and 100 µL of TMB+ (Dako, S159985) was then added thereto. The obtained mixture was subjected to a coloring reaction at room temperature for 3 minutes, and 100 µL of 1 N sulfuric acid was then added thereto, so as to terminate the reaction. Using Infinite M1000 (TECAN), the absorbance at 450 nm was measured.

The measurement results are shown in Table 37. It was confirmed that human IgG₁ was secreted from the clone #20 and the clone #62, and that the secretion level of chicken IgM was extremely small.

**[Table 37]**

| Clone name | IgG (µg/mL) | IgM (µg/mL) |
|---|---|---|
| #62 | 5.79 | 0.186 |
| #20 | 1.54 | 0.231 |

### 40-2. Analysis by Western blotting

Antibodies secreted from the positive clones #62 and #20, which produce the antigen-reactive antibody obtained in 17-4 above, were analyzed according to Western blotting.

Each cell line was suspended in a medium containing no chicken serum to result in 4 × 10⁵ cells/mL, and 1 mL each of the suspension was plated on a 24-well plate. The cells were cultured for 3 days, and the culture solution containing the cell line was recovered and was then filtrated through a 0.22-µm filter (Millipore, SLGV J13 SL). For detection of Igγ and Igλ, a sample purified with a Protein A column was used, and for detection of IgM, a sample before purification was used. To a sample used in detection under reduction conditions (reduction sample), Tris-SDS-β-mercaptoethanol sample treatment solution (COSMO BIO Co., Ltd., 423437) was added in an equal amount of the aforementioned sample. On the other hand, to a sample used in detection under non-reduction conditions (non-reduction sample), Tris-SDS sample treatment solution (COSMO BIO Co., Ltd., 423420) was added in an equal amount of the aforementioned sample. Thereafter, the reduction sample was reacted at 98°C for 3 minutes, and the non-reduction sample was reacted at 37°C for 30 minutes. Thereafter, the sample was electrophoresed on a 4% to 20% polyacrylamide gel (COSMO BIO Co., Ltd., 414879), and it was then transcribed on a nylon membrane. After that, it was blocked with a blocking buffer (PBS containing 1% BSA), and was then reacted with a primary antibody (Goat Anti-Human IgG-Fc Fragment Ab-HRP (Bethyl, A80-104P), Goat Anti-Human IgG Lambda-HRP (Southern Biotech, 2070-05), and Goat Anti-chicken IgM-HRP (Bethyl, A30-102P). The resultant was washed with PBS containing 0.1 % Tween 20 three times, and then, chicken IgM, human Igγ (heavy chain), and human Igλ (light chain) were detected by chemiluminescence using ECL plus (GE Healthcare, RPN2132). The results are shown in Figure 38 to Figure 40. According to Western blotting using an anti-Igy antibody, a band of 55 kDa was detected under reduction conditions, and a band of approximately 160 kDa was detected under non-reduction conditions (Figure 38). According to Western blotting using an anti-Igλ antibody, a band of 25kDa was detected under reduction conditions, and a band of approximately 160 kDa was detected under non-reduction conditions (Figure 39). Moreover, according to Western blotting using an anti-IgM antibody, no signals were detected (Figure 40).

From these results, it was confirmed that the antibody produced by the obtained positive clone is a full-length human antibody.

### Antibody selection 2

### 41. Preparation of antigen

Semaphorin 3A (Sema3A) was selected as an antigen protein. Sema3A is a molecule belonging to the Sema3 family, and has been known as a factor for controlling axon extension in the sensory nerve or the sympathetic nerve.

As an antigen, His-AP-hSema3A (SEQ ID NO: 183), in which a His tag and human alkaline phosphatase (AP) were added to the N-terminus of Sema3A, was used. A cell line HEK293 stably expressing this protein was provided by Department of Molecular Pharmacology & Neurobiology, Yokohama City University, Graduate School of Medicine.

The expression was carried out at 4 L-scale, and the culture was carried out for 4 days. Utilizing His tags, a protein of interest in the culture supernatant was purified. 5 mL of HisTrap EXCEL (GE Healthcare, 17-3712-06) was equilibrated with Solution A (20 mM Na-phosphate, 150 mM NaCl, 20 mM Imidazole (pH 7.5)), a culture supernatant was then loaded thereon, and it was then washed with Solution A. Thereafter, elution was carried out by gradient elution in which Solution A was linearly replaced with Solution B (20 mM Na-phosphate, 150 mM NaCl, 500 mM Imidazole (pH 7.5)) at 25 column volumes. The resultant was monitored with the absorbance at 280 nm, and a fraction corresponding to the peak was then recovered. The molecular weight of the recovered protein was measured by SDS-PAGE and CBB staining, and it was then confirmed that the obtained molecular weight corresponded to the molecular weight of the protein of interest.

Using an ultrafiltration membrane (Amicon, UFC0801008), approximately 80 mL of the recovered fraction was concentrated to a volume of 1.5 mL, and then, using Hi Load 16/600 Superdex 200 pg (GE Healthcare, 28-9893-35), the fraction was purified by gel filtration chromatography. The column was equilibrated with an elution buffer (50 mM Na-phosphate, 300 mM NaCl, 400 mM Arginine, pH 7.0), and the recovered fraction was then loaded thereon, followed by elution with the elution buffer. The resultant was monitored with the absorbance at 280 nm, and a fraction corresponding to the peak was then recovered. The molecular weight of the recovered protein was measured by SDS-PAGE and CBB staining. Subsequently, in order to remove arginine from the eluant, the second gel filtration chromatography was carried out with D-PBS(-). Similarly, the resultant was monitored with the absorbance at 280 nm, and a fraction corresponding to the peak was then recovered. The molecular weight of the recovered protein was measured by SDS-PAGE and CBB staining.

### 42. Selection of antibody

### 42-1. Production of antigen magnetic beads

Using Dynabeads M-270 Carboxylic Acid (Life Technologies, 14305D), Dynabeads M-270 Epoxy (Life Technologies, 14301), and Dynabeads His-Tag Isolation & Pulldown (Life Technologies, 10103D) as magnetic beads, these beads were allowed to bind to the antigen in accordance with the instruction manual. At this time, MPC-S (Life Technologies, DBA13346) was used as a magnetic stand. Regarding Dynabeads M-270 Carboxylic Acid, 40 µL of beads were washed with 80 µL of 25 mM MES (pH 5.0) three times, and 40 µL of 50 mg/mL NHS solution and 40 µL of 50 mg/mL EDC solution were added to the resulting beads. The obtained mixture was reacted at room temperature for 30 minutes, and the reaction solution was then washed with 80 µL of 25 mM MES (pH 5.0) twice. A supernatant was removed from this magnetic beads suspension. For positive selection, 50.6 µL of His-AP-hSema3A protein solution, which had been adjusted to 0.475 mg/ml with 25 mM MES (pH 5.0), was added to the above-obtained solution to make a suspension. For negative selection, PBS was used instead of the antigen solution, and 50.6 µL of the solution diluted with 25 mM MES (pH 5.0) was added to the above-obtained solution to make a suspension. The obtained suspension was reacted at 4°C overnight, while being subjected to rotary stirring. Thereafter, a supernatant was removed from the reaction solution, and 80 µL of quenching buffer (50 mM Tris-HCl) was then added thereto, followed by the reaction of the mixture at room temperature for 15 minutes while being subjected to rotary stirring. Regarding Dynabeads M-270 Epoxy, 40 µL of beads were washed with 80 µL of 100 mM Na-phosphate buffer three times. Thereafter, for positive selection, 53.3 µL of His-AP-hSema3A protein solution adjusted to 0.45 mg/ml with a 100 mM Na-phosphate buffer and 26.7 µL of 100 mM Na-phosphate buffer containing 3 M ammonium sulfate were added to the above-obtained beads to make a suspension, and for negative selection, PBS was used instead of the antigen solution, and 53.3 µL of the solution diluted with 25 mM MES (pH 5.0) and 26.7 µL of 100 mM Na-phosphate buffer containing 3 M ammonium sulfate were added to the above-obtained beads to make a suspension. The obtained suspension was reacted at 37°C overnight, while being subjected to rotary stirring. Regarding Dynabeads His-Tag Isolation & Pulldown, 8 µL of beads were washed with 80 µL of PBS three times. Thereafter, for positive selection, 101.9 µL of His-AP-hSema3A protein solution having an antigen concentration of 4.49 µM was added to the above-obtained beads to make a suspension, and for negative selection, 101.9 µL of PBS was used instead of the antigen solution, and it was then added to the above-obtained beads to make a suspension. The obtained suspension was reacted at 4°C for 10 minutes, while being subjected to rotary stirring. After the removal of a supernatant, the three types of beads were collectively suspended in 1 mL of selection buffer (PBS containing 1% BSA), and the obtained suspension was then washed with the same buffer as mentioned above three times. Thereafter, the resultant was suspended in 200 µL of selection buffer, and was then subjected to selection.

### 42-2. Selection by antigen magnetic beads

An L15/H15 cell line, B7-3, was cultured in a medium containing 2.5 ng/mL TSA for 86 days. Thereafter, approximately 1.5 × 10⁷ cells were washed with 10 mL of selection buffer, and a supernatant was then removed from the reaction solution. The remaining solution was washed with 1 mL of selection buffer, and it was then suspended in 950 µL of selection buffer. To this cell suspension, 50 µL of the antigen magnetic beads for negative selection prepared in 39-1 above was added, and the obtained mixture was then reacted at 4°C for 30 minutes, while being stirred by rotation. Thereafter, using KingFisher mL (Thermo, 5400050), the magnetic beads were removed. Subsequently, to this cell suspension, 50 µL of the antigen magnetic beads for positive selection was added, and the obtained mixture was then reacted at 4°C for 30 minutes, while being stirred by rotation. Thereafter, using KingFisher mL (Thermo, 5400050), the reaction solution was washed with 1.7 mL of selection buffer three times. Thereafter, the recovered cells were suspended in 20 mL of medium, the suspension was then plated on a 96-well plate, and it was then cultured in a CO₂ incubator.

### 42-3. Primary screening

The cell culture solution obtained in 42-2 above was recovered, and a clone specifically reacting with a target antigen was screened based on the antigen reactivity of secretory IgG in antigen-immobilized ELISA. 20 µL of antigen protein and negative control antigen solution (His-Ubiquitin, Ovalbumin, Streptavidin), which had been adjusted to 2.5 µg/mL with PBS, was dispensed on an immuno 384-well plate Maxisorp (Nunc, 464718), and it was then reacted at 4°C overnight, so that it was immobilized thereon. The resultant was washed with a washing solution (PBS containing 0.05% Tween 20) five times, and 45 µL of a blocking solution (PBS containing 1% BSA) was then added thereto. The obtained mixture was reacted at room temperature for 115 minutes. The reaction mixture was washed with a washing solution five times, and 25 µL of culture supernatant was then added thereto. The obtained mixture was reacted at room temperature 130 minutes. Thereafter, the resultant was washed with a washing solution five times, and 25 µL of Goat anti-Human IgG-Fc HRP-conjugated, which had been 2000 times diluted with a blocking solution, was added thereto. The obtained mixture was reacted at room temperature 47 minutes. The resultant was washed with a washing solution five times, 25 µL of TMB+ (Dako, S159985) was then added thereto, and the obtained mixture was then reacted for 5 minutes. Thereafter, 25 µL of 1 N sulfuric acid was added to the reaction mixture, so as to terminate the reaction, and then, using a microplate reader, the absorbance at 450 nm was measured (Figure 41). The cell having an absorbance of 0.1 or more and an S/N ratio of 5 or more was determined to be a positive cell, and it was then subjected to secondary screening.

### 42-4. Secondary screening

The cell culture supernatant obtained in 42-3 above was recovered, and it was then subjected to the secondary screening according to antigen-immobilized ELISA based on the method described in 42-3 above. The cell having an absorbance of 0.5 or more and an S/N ratio of 5 or more was determined to be a positive cell, and it was then subjected to monocloning

### 42-5. Primary screening after monocloning

The cell culture supernatant obtained in 42-4 above was recovered, and it was then subjected to screening according to antigen-immobilized ELISA based on the method described in 42-3 above. The cell having an absorbance of 0.2 or more and an S/N ratio of 5 or more was determined to be a positive cell, and it was then subjected to secondary screening.

### 42-6. Secondary screening after monocloning

The cell culture supernatant obtained in 42-5 above was recovered, and it was then subjected to screening according to antigen-immobilized ELISA based on the method described in 42-3 above. Positive clones #64, #69 and #77, which had an absorbance of 0.5 or more and an S/N ratio of 5 or more, were selected (Figure 42).

### 43. Confirmation of expression of anti-His-AP-hSema3A antibody

### 43-1. Analysis by ELISA

Among the positive clones selected in 42-6 above, regarding the positive clone #64, the concentrations of chicken IgM and human IgG₁ secreted into the culture supernatant were measured. The measurement method was as described in 40-1 above.

The measurement results are shown in Table 38. It was confirmed that, as with wild-type DT40, human IgG was secreted from the positive clone #64, and that secretion of chicken IgM was not observed.

**[Table 38]**

| Clone name | IgG (µg/mL) | Chicken IgM (µg/mL) |
|---|---|---|
| #64 | 2.66 | <0.0005 |

### 43-2. Analysis by Western blotting

According to Western blotting, an antibody secreted from the positive clone #64 producing an antigen-reactive antibody obtained in 42-6 above was analyzed.

Each cell line was suspended in a medium containing no chicken serum to result in 4 × 10⁵ cells/mL, and 20 mL of the suspension was plated on a 9-cm dish. The cells were cultured for 4 days, and the culture solution containing the cell line was recovered and was then filtrated through a 0.22-µm filter (Millipore, SLGV J13 SL). For detection of Igγ and Igλ, a sample purified with a Protein A column was used, and for detection of IgM, a sample before purification was used. To a sample used in detection under reduction conditions (reduction sample), Tris-SDS-β-mercaptoethanol sample treatment solution (COSMO BIO Co., Ltd., 423437) was added in an equal amount of the aforementioned sample. On the other hand, to a sample used in detection under non-reduction conditions (non-reduction sample), Tris-SDS sample treatment solution (COSMO BIO Co., Ltd., 423420) was added in an equal amount of the aforementioned sample. Thereafter, the reduction sample was reacted at 98°C for 3 minutes, and the non-reduction sample was reacted at 37°C for 30 minutes. Thereafter, the sample was electrophoresed on XV PANTERA 5-20% T-HCL 10W (DRC, NXV-275HP), and it was then transcribed on a PVDF membrane. After that, it was blocked with a blocking buffer (TBS containing 5 % skimmed milk and 0.1 % Tween 20), and was then reacted with a primary antibody (Goat Anti-Human IgG-Fc Fragment Ab-HRP (Bethyl, A80-104P) and Goat Anti-Human IgG Lambda-HRP (Southern Biotech, 2070-05)). The resultant was washed with TBS containing 0.1 % Tween 20 three times, and then, chicken IgM, human Igγ (heavy chain), and human Igλ, (light chain) were detected by chemiluminescence using Luminata Forte Western HRP substrate (Millipore, WBLUF0100). The results are shown in Figure 43. According to Western blotting using an anti-Igy antibody, a band of 55 kDa was detected under reduction conditions (Figure 43A), and a band of approximately 160 kDa was detected under non-reduction conditions (Figure 43B). According to Western blotting using an anti-Igλ, antibody, a band of 25kDa was detected under reduction conditions (Figure 43C), and a band of approximately 160 kDa was detected under non-reduction conditions (Figure 43D).

From these results, it was confirmed that the antibody produced by the obtained positive clone is a full-length human antibody.

### Antibody selection 3

### 44. Preparation of antigen

IL-8 was selected as an antigen protein. IL-8 is one type of chemokine. IL-8 exhibits chemotaxis to neutrophils and T lymphocytes, and has an activity of promoting adhesion of leucocytes to vascular endothelial cells or the functions of neutrophils. Hence, it is considered that IL-8 is associated with inflammatory disease, rheumatoid arthritis and other diseases, which are attended with infiltration of neutrophils.

As antigens, human IL-8 (Immune TECH, IT-401-003P) was used in selection, and two types of human IL-8 (Immune TECH, IT-401-003P, and CELL Signaling Technology, 8921LF) were used in screening.

### 45. Selection of antibody

### 45-1. Production of antigen magnetic beads

Using Dynabeads M-270 Carboxylic Acid (Life Technologies, 14305D), Dynabeads M-270 Epoxy (Life Technologies, 14301) and Dynabeads His-Tag Isolation & Pulldown (Life Technologies, 10103D) as magnetic beads, these beads were allowed to bind to the antigen in accordance with the instruction manual. At this time, MPC-S (Life Technologies, DBA13346) was used as a magnetic stand. Regarding Dynabeads M-270 Carboxylic Acid, 35 µL of beads were washed with 70 µL of 25 mM MES (pH 5.0) three times, and 35 µL of 50 mg/mL NHS solution and 35 µL of 50 mg/mL EDC solution were added to the resulting beads. The obtained mixture was reacted at room temperature for 30 minutes, and the reaction solution was then washed with 70 µL of 25 mM MES (pH 5.0) twice. A supernatant was removed from this magnetic beads suspension. For positive selection, 35 µL of IL-8 (Immune TECH, IT-401-003P) protein solution adjusted to 0.6 mg/ml with 25 mM MES (pH 5.0) was added to the above-obtained solution to make a suspension. For negative selection, PBS was used instead of the antigen solution, and 35 µL of the solution diluted with 25 mM MES (pH 5.0) was added to the above-obtained solution to make a suspension. The obtained suspension was reacted at 4°C overnight, while being subjected to rotary stirring. Thereafter, a supernatant was removed from the reaction solution, and 70 µL of quenching buffer (50 mM Tris-HCl) was then added thereto, followed by the reaction of the mixture at room temperature for 15 minutes while being subjected to rotary stirring. Regarding Dynabeads M-270 Epoxy, 35 µL of beads were washed with 70 µL of 100 mM Na-phosphate buffer three times. Thereafter, for positive selection, 46.7 µL of IL-8 protein solution adjusted to 0.45 mg/ml with a 100 mM Na-phosphate buffer and 23.3 µL of 100 mM Na-phosphate buffer containing 3 M ammonium sulfate were added to the above-obtained solution to make a suspension, and for negative selection, PBS was used instead of the antigen solution, and 46.7 µL of the solution diluted with 100 mM Na-phosphate buffer and 23.3 µL of 100 mM Na-phosphate buffer containing 3 M ammonium sulfate were added to the above-obtained solution to make a suspension. The obtained suspension was reacted at 37°C overnight, while being subjected to rotary stirring. Regarding Dynabeads His-Tag Isolation & Pulldown, 7 µL of beads were washed with 70 µL of PBS three times. Thereafter, for positive selection, 70 µL of IL-8 protein solution having an antigen concentration of 5.75 µM was added to the above-obtained solution to make a suspension, and for negative selection, 70 µL of PBS was used instead of the antigen solution, and it was added to the above-obtained solution to make a suspension. The obtained suspension was reacted at 4°C for 210 minutes, while being subjected to rotary stirring. After the removal of a supernatant, the three types of beads were collectively suspended in 1 mL of a selection buffer (PBS containing 1% BSA), and the obtained suspension was then washed with the same buffer as mentioned above three times. Thereafter, the resultant was suspended in 700 µL of selection buffer, and was then subjected to selection.

### 45-2. Selection by antigen magnetic beads

An L30/H15f15f15f cell line, 2-1-3, was cultured in a medium containing 2.5 ng/mL TSA for 45 days. Thereafter, approximately 1.5 × 10⁷ cells were washed with 10 mL of selection buffer, and a supernatant was then removed from the reaction solution. The remaining cells were washed with 10 mL of selection buffer, and it was then suspended in 950 µL of selection buffer. To this cell suspension, 50 µL of the antigen magnetic beads for negative selection prepared in 39-1 above was added, and the obtained mixture was then reacted at 4°C for 30 minutes, while being stirred by rotation. Thereafter, using KingFisher mL (Thermo, 5400050), the magnetic beads were removed. Subsequently, to this cell suspension, 50 µL of the antigen magnetic beads for positive selection was added, and the obtained mixture was then reacted at 4°C for 30 minutes, while being stirred by rotation. Thereafter, using KingFisher mL (Thermo, 5400050), the reaction solution was washed with 1.7 mL of selection buffer three times. Thereafter, the recovered cells were suspended in 20 mL of medium, the suspension was then plated on a 96-well plate, and it was then cultured in a CO₂ incubator.

### 45-3. Screening and identification of positive clones

In the same matter as described in 42-3 to 42-6 above, two times of screening, monocloning, and two times of screening were carried out to obtain positive clone #117, #121 and #123. The results of the primary screening after completion of the selection are shown in Figure 44, and the results of the secondary screening after completion of the monocloning are shown in Figure 45.

### 46. Confirmation of expression of anti-IL-8 antibody

### 46-1. Analysis by ELISA

Regarding the positive clones #117 and #121 generating antigen-reactive antibodies obtained in 45-3 above, the concentrations of chicken IgM and human IgG₁ secreted into the culture supernatant were measured. The measurement method was as described in 40-1 above.

The measurement results are shown in Table 39. It was confirmed that, as with wild-type DT40, human IgG was secreted from the positive clones #117 and #121, and that secretion of chicken IgM was not observed.

**[Table 39]**

| Clone name | IgG (µg/mL) | IgM (µg/mL) |
|---|---|---|
| #117 | 3.65 | <0.041 |
| #121 | 3.7 | <0.041 |

### 46-2. Analysis by Western blotting

According to Western blotting, antibodies secreted from the positive clones #117 and #121 were analyzed. The method was as described in 40-2 above.

The results are shown in Figure 46. According to Western blotting using an anti-Igy antibody, a band of 55 kDa was detected under reduction conditions (Figure 46A), and a band of approximately 160 kDa was detected under non-reduction conditions (Figure 46B). According to Western blotting using an anti-Igλ antibody, a band of 25 kDa was detected under reduction conditions (Figure 46C), and a band of approximately 160 kDa was detected under non-reduction conditions (Figure 46D).

From these results, it was confirmed that the antibody produced by the obtained positive clone is a full-length human antibody.

### Industrial Applicability

The present invention relates to a method for promptly producing a variety of human antibodies. Considering the importance of antibody drugs, it is anticipated that the technique provided by the present invention will play an extremely important role in the development of biotechnology-based pharmaceutical products exhibiting desired drug effects, in particular, antibody drugs, in the field of future drug discovery and medical services.

### Sequence Listing

### TPC0130CBS-sequenching ST25.txt

Furthermore, the present invention relates to the following items:
[Item 1] A chicken B cell, in which, in an antibody light chain gene locus thereof, all or a part of a DNA sequence derived from a human antibody light chain variable region and a human antibody light chain constant region are inserted, or the antibody light chain gene locus is replaced with all or a part of a DNA sequence derived from a human antibody light chain variable region and a human antibody light chain constant region, and in an antibody heavy chain gene locus thereof, all or a part of a DNA sequence derived from a human antibody heavy chain variable region and a human antibody heavy chain constant region are inserted, or the antibody heavy chain gene locus is replaced with all or a part of a DNA sequence derived from a human antibody heavy chain variable region and a human antibody heavy chain constant region, and in an antibody light chain pseudogene locus thereof, two or more DNA sequences derived from human antibody light chain variable regions are inserted, or the antibody light chain pseudogene locus is replaced with two or more DNA sequences derived from human antibody light chain variable regions, and/or in an antibody heavy chain pseudogene locus thereof, two or more DNA sequences derived from human antibody heavy chain variable regions are inserted, or the antibody heavy chain pseudogene locus is replaced with two or more DNA sequences derived from human antibody heavy chain variable regions.
[Item 2] The chicken B cell according to item 1, wherein the number of the DNA sequences derived from the human antibody light chain variable regions to be inserted into the antibody light chain pseudogene locus, or to be replaced with the sequences in the antibody light chain pseudogene locus is 5 or more.
[Item 3] The chicken B cell according to item 1, wherein the number of the DNA sequences derived from the human antibody heavy chain variable regions to be inserted into the antibody heavy chain pseudogene locus, or to be replaced with the sequences in the antibody heavy chain pseudogene locus is 5 or more.
[Item 4] The chicken B cell according to item 1, wherein the number of the DNA sequences derived from the human antibody light chain variable regions to be inserted into the antibody light chain pseudogene locus, or to be replaced with the sequences in the antibody light chain pseudogene locus is 15 or more.
[Item 5] The chicken B cell according to item 1, wherein the number of the DNA sequences derived from the human antibody heavy chain variable regions to be inserted into the antibody heavy chain pseudogene locus, or to be replaced with the sequences in the antibody heavy chain pseudogene locus is 15 or more.
[Item 6] The chicken B cell according to any one of items 1 to 5, wherein the position of the DNA sequence derived from the human antibody light chain variable region inserted into the antibody light chain gene locus is upstream of the position of the DNA sequence derived from the human antibody light chain constant region, and the position of the DNA sequence derived from the human antibody heavy chain variable region inserted into the antibody heavy chain gene locus is upstream of the position of the DNA sequence derived from the human antibody heavy chain constant region.
[Item 7] The chicken B cell according to item 6, wherein the positions of the DNA sequence derived from the human antibody light chain variable region and the DNA sequence derived from the human antibody light chain constant region inserted into the antibody light chain gene locus are upstream of the positions of a chicken antibody light chain variable region and a constant region thereof, and the position of the human antibody heavy chain variable region and constant region inserted into the antibody heavy chain gene locus is upstream of the positions of a chicken antibody heavy chain variable region and a constant region thereof.
[Item 8] The chicken B cell according to any one of items 1 to 7, wherein the human antibody heavy chain is a γ chain.
[Item 9] The chicken B cell according to any one of items 1 to 8, wherein the human antibody light chain is a γ chain or a κ chain.
[Item 10] The chicken B cell according to any one of items 1 to 9, which has an ability to express a human antibody on the cell surface thereof and also to secrete the human antibody into a culture solution.
[Item 11] The chicken B cell according to any one of items 1 to 10, which is a DT40 cell.
[Item 12] The chicken B cell according to any one of items 1 to 11, which has undergone to a treatment of relaxing chromatin.
[Item 13] The chicken B cell according to item 12, wherein the treatment of relaxing chromatin is reduction or deletion of the function of histone deacetylase in the chicken B cell.
[Item 14] The chicken B cell according to item 13, wherein the method for reducing or deleting the function of histone deacetylase is reduction or deletion of the expression of a histone deacetylase gene in the chicken B cell.
[Item 15] The chicken B cell according to item 14, wherein the histone deacetylase is HDAC2.
[Item 16] The chicken B cell according to item 13, wherein the method for reducing or deleting the function of histone deacetylase is a treatment with an HDAC inhibitor.
[Item 17] An antibody-producing cell library consisting of the chicken B cells according to any one of items 12 to 16.
[Item 18] A method for producing an antibody from the chicken B cells according to any one of items 1 to 16.
[Item 19] An antibody obtained by the method according to item 18.
[Item 20] A kit for producing the chicken B cells according to any one of items 1 to 16.
[Item 21] A kit for producing an antibody by the method according to item 18.

## Claims

1. A chicken B cell, in which the chicken antibody light chain gene locus is replaced by the DNA sequences of a human antibody light chain variable region and by the DNA sequences of a human antibody light chain constant region,
and in which in the chicken antibody heavy chain gene locus the DNA sequences of a human antibody heavy chain variable region and of a human antibody heavy chain constant region are inserted,
and in which the chicken antibody light chain pseudogene locus is replaced by 5 or more DNA sequences of human antibody light chain variable regions,
and in which in the chicken antibody heavy chain pseudogene locus 5 or more DNA sequences of human antibody heavy chain variable regions are inserted,
wherein the chicken B cell has the ability to express a human antibody on the cell surface and also to secrete the human antibody into the culture solution.

2. The chicken B cell according to claim 1, wherein the position of the DNA sequence of the human antibody light chain variable region inserted into the antibody light chain gene locus is upstream of the position of the DNA sequence of the human antibody light chain constant region, and the position of the DNA sequence of the human antibody heavy chain variable region inserted into the antibody heavy chain gene locus is upstream of the position of the DNA sequence of the human antibody heavy chain constant region.

3. The chicken B cell according to claim 1 or 2, wherein the human antibody heavy chain is a γ chain.

4. The chicken B cell according to any one of claims 1 to 3, wherein the human antibody light chain is a λ chain or a κ chain.

5. The chicken B cell according to any one of claims 1 to 4, which is a DT40 cell.

6. The chicken B cell according to any one of claims 1 to 5, which has undergone a treatment for relaxing chromatin.

7. The chicken B cell according to claim 6, wherein the treatment for relaxing chromatin is reduction or deletion of the function of histone deacetylase in the chicken B cell.

8. The chicken B cell according to claim 7, wherein the method for reducing or deleting the function of histone deacetylase is reduction or deletion of the expression of a histone deacetylase gene in the chicken B cell.

9. The chicken B cell according to claim 8, wherein the histone deacetylase is HDAC2.

10. The chicken B cell according to claim 7, wherein the method for reducing or deleting the function of histone deacetylase is a treatment with an HDAC inhibitor.

11. An antibody-producing cell library consisting of chicken B cells according to any one of claims 6 to 10.

12. A method for producing a human antibody from the chicken B cell according to any one of claims 1 to 10, comprising culturing said chicken B cell under culturing conditions suitable for the cell to produce a human antibody and obtaining said antibody.
